(19) Europäisches Patentamt
European Patent Office
Office européen des brevets

(11) **EP 4 139 287 B1**

(12) **EUROPEAN PATENT SPECIFICATION**

(45) Date of publication and mention of the grant of the patent:
**26.02.2025 Bulletin 2025/09**

(21) Application number: **21720251.4**

(22) Date of filing: **20.04.2021**

(51) International Patent Classification (IPC):
***C07D 277/38*** *(2006.01)* ***C07D 417/06*** *(2006.01)*
***C07D 417/10*** *(2006.01)* ***A61K 31/426*** *(2006.01)*
***A61K 31/4439*** *(2006.01)* ***A61P 37/06*** *(2006.01)*

(52) Cooperative Patent Classification (CPC):
(C-Sets available)
**C07D 277/38; A61K 31/426; A61K 31/427; A61K 31/4439; A61K 31/454; A61K 31/4709; A61K 31/4725; A61K 31/496; A61K 31/519; A61K 31/5377; A61K 31/55; A61K 45/06; C07D 417/06; C07D 417/10** (Cont.)

(86) International application number:
**PCT/EP2021/060170**

(87) International publication number:
**WO 2021/214020 (28.10.2021 Gazette 2021/43)**

(54) **SUBSTITUTED AMINOTHIAZOLES AS DGKZETA INHIBITORS FOR IMMUNE ACTIVATION**

SUBSTITUIERTE AMINOTHIAZOLE ALS DGKZETA-INHIBITOREN ZUR IMMUNAKTIVIERUNG

AMINOTHIAZOLES SUBSTITUÉS EN TANT QU'INHIBITEURS DE DGKZETA POUR L'ACTIVATION IMMUNITAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.04.2020 EP 20171280**

(43) Date of publication of application:
**01.03.2023 Bulletin 2023/09**

(73) Proprietors:
- **Bayer Aktiengesellschaft**
  **51373 Leverkusen (DE)**
- **Deutsches Krebsforschungszentrum**
  **69120 Heidelberg (DE)**

(72) Inventors:
- **SCHMEES, Norbert**
  **13469 Berlin (DE)**
- **ROEHN, Ulrike**
  **10115 Berlin (DE)**
- **KIRCHHOFF, Dennis**
  **13086 Berlin (DE)**
- **PETERSEN, Kirstin**
  **13465 Berlin (DE)**
- **NGUYEN, Thi, Thanh, Uyen**
  **10967 Berlin (DE)**
- **GREES, Mareike**
  **13465 Berlin (DE)**
- **WERBECK, Nicolas**
  **79111 Freiburg (DE)**
- **BOEMER, Ulf**
  **16548 Glienicke (DE)**
- **BADER, Benjamin**
  **13467 Berlin (DE)**
- **STOECKIGT, Detlef**
  **14467 Potsdam (DE)**
- **OFFRINGA, Rienk**
  **69120 Heidelberg (DE)**
- **LINK, Corinna**
  **69120 Heidelberg (DE)**
- **TESTOLIN, Giambattista**
  **14480 Potsdam (DE)**
- **NOWAK-REPPEL, Katrin**
  **13187 Berlin (DE)**
- **ROSENTRETER, Ulrich**
  **31619 Binnen (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(74) Representative: **BIP Patents**
**c/o Bayer Intellectual Property GmbH**
**Alfred-Nobel-Straße 50**
**40789 Monheim am Rhein (DE)**

(56) References cited:
**WO-A1-2019/133445** **WO-A1-2020/006016**
**WO-A1-2020/006018**

Remarks:
The complete document including Reference Table(s) and the Sequence Listing(s) can be downloaded from the EPO website

(52) Cooperative Patent Classification (CPC): (Cont.)

C-Sets
**A61K 31/426, A61K 2300/00;**
**A61K 31/427, A61K 2300/00;**
**A61K 31/4439, A61K 2300/00;**
**A61K 31/454, A61K 2300/00;**
**A61K 31/4709, A61K 2300/00;**
**A61K 31/4725, A61K 2300/00;**
**A61K 31/496, A61K 2300/00;**
**A61K 31/519, A61K 2300/00;**
**A61K 31/5377, A61K 2300/00;**
**A61K 31/55, A61K 2300/00**

## Description

The present invention covers substituted aminothiazole compounds of general formula (I) as described and defined herein, methods of preparing said compounds, intermediate compounds useful for preparing said compounds, pharmaceutical compositions and combinations comprising said compounds, and the use of said compounds for manufacturing pharmaceutical compositions for the treatment or prophylaxis of diseases, in particular of diacylglycerol kinase zeta (DGKzeta, DGKζ) regulated disorders, as a sole agent or in combination with other active ingredients.

The compounds of general formula (I) inhibit DGKζ and, by this, enhance T cell mediated immune response. This is a new strategy to use the patient's own immune system to overcome immunoevasive strategies utilized by many neoplastic disorders, respectively cancer and by this enhancing anti-tumor immunity. Furthermore, said compounds are used in particular to treat disorders such as viral infections or conditions with dysregulated immune responses or other disorders associated with aberrant DGKζ signaling.

The present invention further relates to the use of the compounds of general formula (I) for manufacturing pharmaceutical compositions for enhancement of T cell mediated immune response.

The present invention further relates to the use of the compounds of general formula (I) for manufacturing pharmaceutical compositions for the treatment of cancer.

The present invention further relates to the use of the compounds of general formula (I) for manufacturing pharmaceutical compositions for the treatment or prophylaxis of virus infections, lymphoproliferative disorders, asthma, eye diseases, and type 2 diabetes/ insulin resistance.

## Background

Diacylglycerol kinases (DGKs) represent a family of enzymes that catalyze phosphorylation of the membrane lipid sn-1,2 diacylglycerol (DAG) to form phosphatidic acid (PA) (T.O. Eichmann and A. Lass, Cell. Mol. Life Sci. 2015, 72, 3931-3952). In T cells, DAG is formed downstream of the T cell receptor (TCR) after activation of the gamma 1 isoform of phospholipase C (PLCγ1) and cleavage of phosphatidylinositol 4,5-biphosphate (PIP2) into DAG and an additional second messenger, inositol 1,4,5-triphosphate (IP3) (S. Krishna and X.-P. Zhong, Front. Immunol. 2013, 4, 178). Whereas, IP3 is important in facilitating release of calcium from the endoplasmic reticulum, DAG interacts with other proteins important in TCR signal transduction, such as Protein kinase Cθ (E. J. Quann et al., Nat. Immunol. 2011, 12 (7), 647-654) and the Ras activating protein RasGRP1 (S. Krishna and X.-P. Zhong, Front. Immunol. 2013, 4, 178). Although, three isoforms of DGK are known to be present within T cells [DGKα (DGKalpha), DGKδ (DGKdelta), and DGKζ (DGKzeta)], only two, DGKα and DGKζ, are thought to play an important role in facilitating DAG metabolism downstream of the TCR (R. P. Joshi and G. A. Koretzky, Int. J. Mol. Sci. 2013, 14 (4), 6649-6673).

Targeting the activity of DGKζ in T cells, either by germline deletion, or with chemical inhibitors, results in enhanced and sustained signaling downstream of T cells, as assessed by prolonged phosphorylation of downstream molecules, such as extracellular signal-related kinases 1/2 (ERK1/2) and NFκB (X.-P.-Zhong et al., Nat. Immunol. 2003, 4, 882-890; B. A. Olenchock et al., Nat. Immunol. 2006, 7 (11), 1174-1181; M. J. Riese et al., J. Biol. Chem. 2011, 286, 5254-5265; E. M. Wesley et al., ImmunoHorizons 2018, 2 (4), 107-118).

Deletion of DGKζ in T cells leads to enhanced production of effector cytokines, such as IL2, IFNγ and enhanced proliferation (X.-P. Zhong et al., Nat. Immunol. 2003, 4, 882-890; B. A. Olenchock et al., Nat. Immunol. 2006, 7 (11), 1174-1181, E. M. Riese et al., J. Biol. Chem. 2011, 286, 5254-5264).

Adoptive transfer of DGKζ deficient T cell reduced leukaemia burden after inoculation of C1498.SIY leukaemia cells compared to control. Also, DGKζ deficient T cells are at least partially resistant to PD1 mediated inhibitory signals (W. Jing et al., Cancer Res. 2017, 77 (20), 5676-5686). In addition, DGKζ deficient mice have reduced tumor sizes compared to control after orthotopic tumor injection of a pancreatic tumor model (E. M. Wesley et al., ImmunoHorizons, 2018, 2 (4), 107-118). Also, S. Wee et al. inoculated C57BL/6 mice with a variety of syngeneic tumor cell lines - MC38 colon carcinoma, B16F1 melanoma, and C1498 leukemia - and analysed survival and tumor growth between mice deficient in DGKζ in the presence or absence of anti-PD1 treatment. DGKζ-/- mice suppressed growth of subcutaneously implanted tumor cells in the three model systems and the combination of DGKζ-deficiency and anti-PD1 was additive in tumor control (S. Wee et al., Proceedings of the American Association for Cancer Research Annual Meeting 2019; Cancer Res. 2019, 79 (13 Suppl): Abstract nr 936).

These findings suggest that DGKζ might serve as a useful target for enhancing T cell anti-tumor activity.

Additionally, the adoptive transfer of CAR (chimeric antigen receptor)-T cells deficient in DGKζ demonstrated increased efficacy compared to wild type CAR T cells in the treatment of murine mesothelioma (M. J. Riese et al., Cancer Res. 2013, 73 (12), 3566-3577) and a glioblastoma xenograft mouse model in combination with DGKα knockout (I.-Y. Jung et al., Cancer Res. 2018, 78 (16), 4692-4703).

Also, DGKζ-deficient mice mounted a more robust immune response to lymphocytic choriomeningitis virus infection than did wild-type mice (X.-P. Zhong et al., Nat. Immunol. 2003, 4, 882-890).

DGKζ is also relevant in natural killer (NK) cells. Upon stimulation through multiple activating receptors, NK cells from mice lacking DGKζ display increased cytokine production and degranulation in an ERK-dependent manner. Additionally, they have improved cytotoxic functions against tumor cell lines. (E. Yang et al. J. Immunol. 2016, 197(3), 934-41.)

Apart from immune-cell regulation, DGKζ also plays a role in cancer, mediating numerous aspects of cancer cell progression including proliferation, apoptosis, survival, invasion and tumorigenicity, e.g. in osteosarcoma, colon cancer, breast cancer, prostate cancer, glioma and leukemia models (W. Yu et al., Front. Oncol. 2019, 8:655; K. Cai et al., BMC Cancer 2014 ,14:208; J. Diao et al., Mol. Neurobiol. 2016; 53, 5425-35; H. Li et al. Pharmazie 2019, 74(7): 418-422).

In addition, DGKζ knock-out diminished both airway inflammation and airway hyperresponsiveness in mice and also reduced bronchoconstriction of human airway samples in vitro by blocking T helper 2 (TH2) differentiation (B. A. Singh et al., Sci. Signal. 2019, 12: eaax3332).

Taken together, the findings from these studies argue that restraining DGKζ activity in T cells and tumor cells may prove valuable in generating more vigorous immune responses against pathogens and tumors and in ameliorating Th2 driven (auto-) immune diseases (in re-balancing the immune-system).

Furthermore, inhibition of DGKα has the potential to reverse the life-threatening Epstein-Barr virus (EBV) -associated immunopathology that occurs in X-linked lymphoproliferative disease (XLP-1) patients (E. Ruffo et al., Sci. Transl. Med. 2016, 8: (321):321ra7; S. Velnati et al., Eur. J. Med. Chem. 2019, 164, 378-390). Based on the underlying mode of action, it can be assumed that inhibition of DGKζ would have a similar effect.

The DGKα-inhibitor II (R59949) could suppress retinal neovascularization and protect retinal astrocytes in an Oxygen-Induced Retinopathy Model (L. Yang et al., J. Mol. Neurosci. 2015, 56, 78-88). Also, based on the underlying mode of action, it can be assumed that inhibition of DGKζ would have a similar effect.

In a DGKζ knock-out mouse it was shown that DGKζ deficiency increases protection against insulin resistance (B. Benziane et al., J. Lipid Res. 2017, 58 (12), 2324-2333).

In summary, inhibiting DGKζ activity has a therapeutic potential in targeting tumors directly as well as addressing virus infections, lymphoproliferative disorders, asthma, eye diseases, and type 2 diabetes/ insulin resistance.

**Prior Art**

WO2020/006016 and WO2020/006018 describe Naphthyridinone compounds as T cell activators, which inhibit the activity of DGKα and/or DGKζ, for treatment of viral infections and proliferative disorders, such as cancer. WO2021/041588 describes pyridopyrimidinonyl compounds as T cell activators, which inhibit the activity of DGKα and/or DGKζ, for treatment of viral infections and proliferative disorders, such as cancer.

2,4,5-trisubstituted triazole derivatives featuring a substituted amino group attached to C-2 of the thiazole core have been disclosed in published patent applications in various technical contexts but not in the context of DGK inhibition.

WO 2014/181287 discloses heterocyclyl compounds as inhibitors of Interleukin 17 and Tumour Necrosis Factor alpha.

WO 2014/173904 discloses compounds having antibacterial activity.

WO 2009/149054 discloses small molecule inhibitors for the treatment or prevention of Dengue fever infection.

WO 2007/130075 discloses aminothiazole derivatives as human stearoyl-CoA desaturase inhibitors.

WO 2012/064715 discloses compositions and methods relating to heat shock transcription factor activating compounds and targets thereof.

WO 2005/103022 discloses substituted thiazole and pyrimidine derivatives as melanocortin receptor modulators.

CN 106109467 discloses the medical application of aromatic compounds, including thiazoles, in treating pyrazinamide-resistant tuberculosis.

WO 2015/199206 discloses compounds derived from a six-membered ring as TRPV4 inhibitors.

WO 2015/046193 discloses aromatic heterocyclic amine derivatives as TRPV4 inhibitors.

CN 103159695 discloses thiazole compounds capable of restraining human immunodeficiency (HIV) virus replication and effective against drug-resistant HIV virus strains.

WO 2013/056684 discloses thiazole derivatives as dihydroorotate dehydrogenase (DHODH) inhibitors.

WO 2013/033037 discloses compounds of various chemotypes, *inter alia* thiazole derivatives, as antiprion compounds.

WO 2012/075393 discloses compounds of various chemotypes, *inter alia* thiazole derivatives, as activators of proteasomal degradation.

JP 2011032254 discloses compounds of various chemotypes, *inter alia* thiazole derivatives, as pest controlling agents.

WO 2009/041790 discloses 2,4,5-trisubstituted thiazole derivatives as inhibitors of the sphingosylphosphorylcholine (SPC) receptor for treatment of inflammatory diseases.

WO 2008/090382 discloses thiazole and oxazole derivatives for use in the treatment of prion diseases, cancer, and conditions of the central nervous system as well as in the regulation of stem cells.

WO 2007/022415 discloses substituted 2-aminothiazoles for treating neurodegenerative diseases.

WO 2006/122011 discloses thiazole compounds and methods of use in treating viral infections, particularly hepatitis C virus infections.

WO 2006/078287 discloses derivatives of various 5-membered heteroarenes, *inter alia* thiazoles, as inhibitors of phosphodiesterase 4B.

WO 2005/102318, WO 2005/102325, WO 2005/102326, WO 2005/102346, WO 2005/102455, WO 2005/112920, WO 2005/115304, WO 2005/115385 all deal with c-Kit inhibitors of various chemotypes, including 2-aminothiazole derivatives, and various uses thereof.

EP 1543824 and US 2005/0137239 disclose thiazole derivatives to counter glycation.

WO 2004/014884 discloses thiazole derivatives as neuropeptide Y receptor ligands.

WO 2019/133445 discloses aminothiazole derivatives as inhibitors of Vanin-1.

WO 2021/043966 discloses substituted five-membered nitrogen containing heteroaryl compounds as inhibitors of the NOD-like receptor (NLR) family, pyrin domain-containing protein 3 (NLRP3).

2,4,5-trisubstituted triazole derivatives structurally related to the compounds of the present invention but yet structurally distinct have also been disclosed in several scientific publications.

D. Kikelj and U. Urleb, Science of Synthesis (2002), 11, 627-833, is a general review on the synthesis of thiazoles. By far most of the specific compounds disclosed are, besides having the thiazole core in common with the compounds of the present invention, structurally distant from these. Several indivdual compounds disclosed therein, namely those disclosed on pp 651, 681-683, and 719, and including {4-methyl-2-[methyl(phenyl)amino]-1,3-thiazol-5-yl}(phenyl) methanone, are somewhat structurally related to the compounds of the present invention but yet structurally distinct.

2,4,5-trisubstituted triazole derivatives structurally related to the compounds of the present invention but yet structurally distinct have also been disclosed in several journal articles.

None of the journal articles listed below, which also disclose some compounds which are somewhat structurally related to the compounds of the present invention but yet structurally distinct, disclose a therapeutic of pharmaceutical application of the compounds disclosed therein.

S. Titus et al., Tetrahedron Lett. 2014, 55, 5465-5467, disclose a four-component synthesis of 4-hydrazinothiazole derivatives.

T. N. Birkinshaw et al., J. Chem. Soc. Perkin Trans. 1, 1988, 2209-2212, disclose spectrometric and chemical studies on 5-acyl and 5-nitroso-2-(N,N-disubstituted amino)thiazoles.

R. A. Funnell et al., J. Chem. Soc. Perkin Trans. 1, 1987, 2311-2315 disclose a study on the formation of isomeric 2-(N,N-disubstituted amino) thiazol-5-yl ketones.

J. C. Brindley et al., J. Chem. Soc. Perkin Trans. 1, 1987, 1153-1158 disclose the conversion of N'-substituted N-acyl and N-imidoyl thioureas into 2-(N,N-disubstituted amino) thiazol-5-yl ketones.

G. D. Meakins et al., J. Chem. Soc. Chem. Comm. 1984, 837-838, disclose a chemical reaction unexpectedly yieding 5-benzoyl-4-methyl-2-(N-methyl-N-phenylamino)thiazole.

K. Akiba et al., Tetrahedron Lett. 1975, 7, 459-462, disclose the synthesis of certain 2-arylaminothiazoles by 1,3-cycloaddition of 4-aryl-3-arylimino-5-imino-1,2-4-thiadiazolidine (also known as Hector's base) and acetylenes.

Finally, three structures which are somewhat structurally related to the compounds of the present invention but yet structurally distinct, are disclosed in the database Chemical Abstracts Registry (CAS Registry®), all without a reference and without a technical application, having the CAS Registry numbers 1349635-19-3, 1349215-57-1, and 1348293-02-6.

However, the state of the art does not describe:

- the specific substituted aminothiazole compounds of general formula (I) of the present invention as described and defined herein, i.e. compounds having a 2-aminothiazole core bearing:
    - an optionally substituted 5-membered heteroaroyl group attached to C-5,
    - a $-NH_2$ or methyl group attached to C-4,
    - and (i) an optionally substituted phenyl or 5- or 6-membered heteroaryl group and (ii) an alkyl group laterally substituted with a group $-S(=O)_2-NH_2$, or, particularly, $-C(=O)-NH_2$, both (i) and (ii) being attached to the nitrogen atom attached to C-2, said laterally substituted alkyl group being essential for potent inhibition of DGKζ as shown in comparative experiments, *infra,*

    or stereoisomers, tautomers, N-oxides, hydrates, solvates, salts thereof, or mixtures of same, as described and defined herein, and as hereinafter referred to as "compounds of general formula (I)" or "compounds of the present invention",

- or their pharmacological activity.

It is desirable to provide novel compounds having prophylactic and therapeutic properties.

Accordingly, it is an object of the present invention to provide compounds and pharmaceutical compositions comprising these compounds used for prophylactic and therapeutic use in DGKζ regulated disorders in a T cell immune-stimulatory or immune-modifing manner. DGKζ regulated disorders comprise conditions with dysregulated immune responses, particularly in an immunologically supressed tumor microenvironment in cancer, autoimmune diseases, viral infections as well as other disorders associated with aberrant DGKζ signalling. Said compounds can be used as sole agent or in combination with other active ingredients.

It has now been found, and this constitutes the basis of the present invention, that the compounds of the present invention have surprising and advantageous properties.

In particular, the compounds of the present invention have surprisingly been found to effectively inhibit the DGKζ protein and, by this, enhance T-cell mediated immunity. Accordingly, they provide novel structures for the treatment diseases of mammals, including humans, in particular of cancers, and may therefore be used for the treatment or prophylaxis of hyperproliferative disorders, such as cancer, for example.

## DESCRIPTION of the INVENTION

In accordance with a first aspect, the present invention covers compounds of general formula (I):

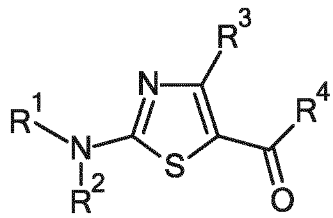

(I)

in which :

$R^1$ represents a phenyl or 6-membered heteroaryl group optionally substituted, one, two, or three times, each substituent independently selected from a halogen atom or a group selected from hydroxy, cyano, nitro, $C_1$-$C_6$-alkyl, (phenyl)-($C_1$-$C_3$-alkyl)-, $C_1$-$C_6$-haloalkyl, $C_1$-$C_6$-alkoxy, (phenyl)-($C_1$-$C_3$-alkoxy)-, $C_1$-$C_6$-haloalkoxy, -N($R^5$)($R^6$),

wherein the phenyl groups in said (phenyl)-($C_1$-$C_3$-alkyl)- and (phenyl)-($C_1$-$C_3$-alkoxy)-groups are optionally substituted one or two times, each substituent independently selected from a halogen atom or a group selected from cyano, methyl, ethyl, trifluoromethyl, methoxy, ethoxy, dimethylamino and trifluoromethoxy,

or two substituents attached to adjacent carbon atoms of said phenyl or 6-membered heteroaryl group together form a bivalent group selected from $-(CH_2)_3-$, $-(CH_2)_4-$, - $(CH_2)_2$-O-, $-(CH_2)_3$-O-, $-CH_2$-O-$CH_2-$, $-(CH_2)_2$-O-$CH_2-$, -O-$CH_2$-O-, -O-$CH_2$-$CH_2$-O-, -O-$CF_2$-O-, -O-$CH_2$-$CF_2$-O-, and -O-$CF_2$-$CF_2$-O-,
or

$R^1$ represents a 5-membered heteroaryl group optionally substituted one or two times, each substituent independently selected from a halogen atom or a group selected from cyano, $C_1$-$C_3$-alkyl, and $C_1$-$C_3$-alkoxy;

$R^2$ represents a group

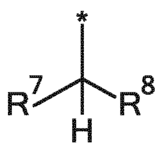

,

wherein "*" indicates the point of attachment to the nitrogen atom to which $R^2$ is attached;

$R^3$ represents a group selected from methyl and $-NH_2$;

$R^4$ represents a 5-membered heteroaryl group optionally substituted one or two times, each substituent independently selected from a halogen atom or a group selected from $C_1$-$C_6$-alkyl, (($R^9$)O)-($C_1$-$C_3$-alkyl)-, (($R^{10}$)($R^{11}$)N)-($C_1$-$C_3$-alkyl)-, ($C_3$-$C_7$-cycloalkyl)-($C_1$-$C_3$-alkyl)-, $C_3$-$C_7$-cycloalkyl, -O$R^9$, -N($R^{10}$)($R^{11}$), -C(=O)-N($R^{15}$)($R^{16}$), -C(=O)-O$R^{17}$, phenyl and a 5- or 6-membered heteroaryl group,
wherein the phenyl group and the 5- or 6-membered heteroaryl group are optionally substituted one or two times, each substituent independently selected from a halogen atom or a group selected from cyano, $C_1$-$C_3$-alkyl, $C_1$-$C_3$-haloalkyl, (($R^{22}$)($R^{23}$)N)-$C_1$-$C_3$-alkyl, -O$R^9$, -N($R^{10}$)($R^{11}$) and -C(=O)-N($R^{15}$)($R^{16}$);

$R^5$ and $R^6$ represent, independently from each occurrence, a hydrogen atom or a group selected from $C_1$-$C_4$-alkyl, ($C_1$-$C_4$-alkyl)-C(=O)-, $C_3$-$C_4$-cycloalkyl and (phenyl)-($C_1$-$C_3$-alkyl)-,
or

$R^5$ and $R^6$, together with the nitrogen atom to which they are attached, represent a monocyclic nitrogen containing 4- to 7-membered heterocycloalkyl group which is optionally substituted one, two or three times, each substituent independently selected from a halogen atom or a group selected from oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy;

$R^7$ represents a hydrogen atom or a $C_1$-$C_2$-alkyl group;

$R^8$ represents a group selected from -C(=O)-$NH_2$ and -S$(=O)_2$-$NH_2$;

$R^9$ represents a hydrogen atom or a group selected from $C_1$-$C_6$-alkyl, ($C_3$-$C_7$-cycloalkyl)-($C_1$-$C_3$-alkyl)-, (phenyl)-($C_1$-$C_3$-alkyl)-, (5- or 6-membered heteroaryl)-($C_1$-$C_3$-alkyl)-, $C_1$-$C_6$-haloalkyl, $C_2$-$C_4$-hydroxyalkyl, ($C_1$-$C_3$-alkoxy)-$C_2$-$C_3$-alkyl-, (($C_1$-$C_3$-alkyl)-C(=O)-O)-$C_2$-$C_3$-alkyl-, -C($R^{18}$)($R^{19}$)-C(=O)-O$R^{17}$, -C($R^{18}$)($R^{19}$)-C(=O)-N($R^{20}$)($R^{21}$), -C(=O)-N($R^{20}$)($R^{21}$), $C_3$-$C_7$-cycloalkyl, phenyl and a 5- or 6-membered heteroaryl group,

wherein $C_3$-$C_7$-cycloalkyl, and the $C_3$-$C_7$-cycloalkyl within said ($C_3$-$C_7$-cycloalkyl)-($C_1$-$C_3$-alkyl)- group is optionally substituted one or two times, each substituent independently selected from a fluorine atom or a group selected from oxo, cyano, $C_1$-$C_2$-alkyl and $C_1$-$C_2$-haloalkyl,

and wherein the phenyl group within said (phenyl)-($C_1$-$C_3$-alkyl)- group and said phenyl group itself, and the 5- or 6-membered heteroaryl group within said (5- or 6-membered heteroaryl)-($C_1$-$C_3$-alkyl)- group and said 5- or 6-membered heteroaryl group itself, are optionally substituted one or two times, each substituent independently selected from a halogen atom or a group selected from cyano, methyl, ethyl, trifluoromethyl, methoxy, ethoxy, dimethylamino and trifluoromethoxy;

$R^{10}$ and $R^{11}$ represent, independently from each occurrence, a hydrogen atom or a group selected from $C_1$-$C_4$-alkyl, $C_1$-$C_4$-haloalkyl, $C_2$-$C_4$-hydroxyalkyl, ($C_1$-$C_3$-alkoxy)-$C_2$-$C_3$-alkyl-, (($R^{22}$)($R^{23}$)N)-$C_2$-$C_3$-alkyl, ($C_3$-$C_7$-cycloalkyl)-($C_1$-$C_3$-alkyl)-, ($C_1$-$C_4$-alkyl)-C(=O)-, $C_3$-$C_7$-cycloalkyl, ($C_3$-$C_7$-cycloalkyl)-C(=O)-, (phenyl)-($C_1$-$C_3$-alkyl)-, (phenyl)-($C_1$-$C_3$-alkyl)-C(=O)-, (phenyl)-($C_1$-$C_3$-alkyl)-O-C(=O)-, phenyl and a 5- or 6-membered heteroaryl group,

wherein $C_3$-$C_7$-cycloalkyl, and the $C_3$-$C_7$-cycloalkyl within said ($C_3$-$C_7$-cycloalkyl)-($C_1$-$C_3$-alkyl)- and ($C_3$-$C_7$-cycloalkyl)-C(=O)- groups are optionally substituted one or two times, each substituent independently selected from a fluorine atom or a group selected from cyano, $C_1$-$C_2$-alkyl and $C_1$-$C_2$-haloalkyl,

and wherein said phenyl and said 5- or 6-membered heteroaryl group, and the phenyl groups within said (phenyl)-($C_1$-$C_3$-alkyl)-, (phenyl)-($C_1$-$C_3$-alkyl)-C(=O)- and (phenyl)-($C_1$-$C_3$-alkyl)-O-C(=O)- groups, are optionally substituted one or two times, each substituent independently selected from a halogen atom or a group selected from cyano, methyl, ethyl, trifluoromethyl, methoxy, ethoxy, dimethylamino and trifluoromethoxy,
or

$R^{10}$ and $R^{11}$, together with the nitrogen atom to which they are attached, represent a monocyclic nitrogen containing 4- to 7-membered heterocycloalkyl group, or a bicyclic nitrogen containing 5- to 11-membered heterocycloalkyl group, which are optionally substituted one, two or three times, each substituent independently selected from a halogen atom or a group selected from cyano, oxo, hydroxy, $C_1$-$C_4$-alkyl, $C_1$-$C_4$-haloalkyl, ($C_1$-$C_4$-alkyl)-C(=O)-, $C_3$-$C_7$-cycloalkyl, $C_1$-$C_4$-alkoxy, -N($R^{22}$)($R^{23}$), and a monocyclic 4- to 7-membered heterocycloalkyl group;

$R^{15}$ and $R^{16}$ represent, independently from each occurrence, a hydrogen atom or a group selected from $C_1$-$C_4$-alkyl,

$C_1$-$C_4$-haloalkyl, $C_1$-$C_4$-hydroxyalkyl, (cyano)-$C_1$-$C_4$-alkyl-, ($C_1$-$C_4$-alkoxy)-$C_2$-$C_3$-alkyl-, ($C_1$-$C_4$-haloalkoxy)-$C_2$-$C_3$-alkyl-, (phenoxy)-$C_2$-$C_3$-alkyl-, ($C_3$-$C_7$-cycloalkyl)-($C_1$-$C_3$-alkyl)-, $C_3$-$C_7$-cycloalkyl, bicyclic $C_5$-$C_{11}$-cycloalkyl, monocyclic 4- to 7-membered heterocycloalkyl, (phenyl)-($C_1$-$C_3$-alkyl)-, phenyl and a 5- or 6-membered heteroaryl group,

wherein $C_3$-$C_7$-cycloalkyl, the $C_3$-$C_7$-cycloalkyl within said ($C_3$-$C_7$-cycloalkyl)-($C_1$-$C_3$-alkyl)- group, the bicyclic $C_5$-$C_{11}$-cycloalkyl and the monocyclic 4- to 7-membered heterocycloalkyl groups are optionally substituted one, two or three times, each substituent independently selected from a halogen atom or a group selected from oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy,

and wherein the phenyl and the 5- or 6-membered heteroaryl groups, including the phenyl groups within said (phenoxy)-$C_2$-$C_3$-alkyl- and (phenyl)-($C_1$-$C_3$-alkyl)- groups, are optionally substituted one or two times, each substituent independently selected from a halogen atom or a group selected from cyano, methyl, ethyl, trifluoromethyl, methoxy, ethoxy, dimethylamino and trifluoromethoxy,
or

$R^{15}$ and $R^{16}$, together with the nitrogen atom to which they are attached, represent a monocyclic nitrogen containing 4- to 7-membered heterocycloalkyl group which is optionally substituted one, two or three times, each substituent independently selected from a halogen atom or a group selected from oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy;

$R^{17}$ represents a $C_1$-$C_4$-alkyl group;

$R^{18}$ and $R^{19}$ represent, independently from each occurrence, a hydrogen atom or a $C_1$-$C_4$-alkyl group;

$R^{20}$ represents a hydrogen atom or a group selected from $C_1$-$C_6$-alkyl, $C_3$-$C_4$-alkenyl, $C_3$-$C_4$-alkynyl, $C_1$-$C_3$-alkoxy, $C_3$-$C_7$-cycloalkyl, bicyclic $C_5$-$C_{11}$-cycloalkyl, adamantyl, monocyclic 4- to 7-membered heterocycloalkyl, bicyclic 5- to 11-membered heterocycloalkyl, phenyl, naphthyl, and 5- to 10-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, bicyclic $C_5$-$C_{11}$-cycloalkyl, adamantyl, monocyclic 4- to 7-membered heterocycloalkyl, bicyclic 5- to 11-membered heterocycloalkyl, phenyl, and 5- to 10-membered heteroaryl, said phenyl and 5- to 10-membered heteroaryl substituents themselves being optionally substituted one or two times, each substituent independently selected from a halogen atom or a group selected from cyano, methyl, ethyl, trifluoromethyl, methoxy, ethoxy, dimethylamino and trifluoromethoxy,

and wherein $C_3$-$C_7$-cycloalkyl, bicyclic $C_5$-$C_{11}$-cycloalkyl, adamantyl, monocyclic 4-to 7-membered heterocycloalkyl and bicyclic 5- to 11-membered heterocycloalkyl are optionally substituted one, two or three times, each substituent independently selected from a halogen atom or a group selected from cyano, oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy,

and wherein said phenyl, naphthyl and 5- to 10-membered heteroaryl groups are optionally substituted one, two or three times, each substituent independently selected from a halogen atom or a group selected from cyano, $C_1$-$C_4$-alkyl, $C_1$-$C_4$-haloalkyl, $C_1$-$C_4$-alkoxy, $C_1$-$C_4$-haloalkoxy, -N($R^{22}$)($R^{23}$) and -C(=O)-N($R^{24}$)($R^{25}$),

$R^{21}$ represents a hydrogen atom or a $C_1$-$C_4$-alkyl group,
or

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_4$-alkyl, $C_1$-$C_4$-haloalkyl, (phenyl)-($C_1$-$C_3$-alkyl)-, ($C_1$-$C_4$-alkyl)-C(=O)-, $C_3$-$C_4$-cycloalkyl, $C_1$-$C_4$-alkoxy, $C_1$-$C_3$-haloalkoxy, -N($R^{22}$)($R^{23}$) and -C(=O)-N($R^{24}$)($R^{25}$);

$R^{22}$ and $R^{23}$ represent, independently from each occurrence, a hydrogen atom or a group selected from $C_1$-$C_2$-alkyl and ($C_1$-$C_2$-alkyl)-C(=O)-, and

$R^{24}$ and $R^{25}$ represent, independently from each occurrence, a hydrogen atom or a $C_1$-$C_4$-alkyl group,

and stereoisomers, tautomers, N-oxides, hydrates, solvates, and salts thereof, and mixtures of same.

## DEFINITIONS

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero. Unless otherwise indicated, it is possible that optionally substituted groups are substituted with as many optional substituents as can be accommodated by replacing a hydrogen atom with a non-hydrogen substituent on any available carbon or nitrogen atom. Commonly, it is possible for the number of optional substituents, when present, to be 1, 2, 3 or 4, in particular 1, 2 or 3.

When groups in the compounds according to the invention are substituted, it is possible for said groups to be mono-substituted or poly-substituted with substituent(s), unless otherwise specified. Within the scope of the present invention, the meanings of all groups which occur repeatedly are independent from one another. It is possible that groups in the compounds according to the invention are substituted with one, two or three identical or different substituents, particularly with one substituent.

As used herein, an oxo substituent represents an oxygen atom, which is bound to a carbon atom or to a sulfur atom via a double bond.

Should a composite substituent be composed of more than one part, *e.g.* ($C_1$-$C_2$-alkoxy)-($C_1$-$C_6$-alkyl)-, it is possible for a given part to be attached at any suitable position of said composite substituent, e.g. it is possible for the $C_1$-$C_2$-alkoxy part to be attached to any suitable carbon atom of the $C_1$-$C_6$-alkyl part of said ($C_1$-$C_2$-alkoxy)-($C_1$-$C_6$-alkyl)-group. A hyphen at the beginning or at the end of such a composite substituent indicates the point of attachment of said composite substituent to the rest of the molecule. Should a ring, comprising carbon atoms and optionally one or more heteroatoms, such as nitrogen, oxygen or sulfur atoms for example, be substituted with a substituent, it is possible for said substituent to be bound at any suitable position of said ring, be it bound to a suitable carbon atom and/or to a suitable heteroatom.

The term "comprising" when used in the specification includes "consisting of".

If within the present text any item is referred to as "as mentioned herein", it means that it may be mentioned anywhere in the present text.

The terms as mentioned in the present text have the following meanings:
The term "halogen atom" means a fluorine, chlorine, bromine or iodine atom, particularly a fluorine, chlorine or bromine atom.

The term "$C_1$-$C_6$-alkyl" means a linear or branched, saturated, monovalent hydrocarbon group having 1, 2, 3, 4, 5 or 6 carbon atoms, e.g. a methyl, ethyl, propyl, isopropyl, butyl, sec-butyl, isobutyl, *tert*-butyl, pentyl, isopentyl, 2-methylbutyl, 1-methylbutyl, 1-ethylpropyl, 1,2-dimethylpropyl, neo-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Particularly, said group has 1, 2, 3 or 4 carbon atoms ("$C_1$-$C_4$-alkyl"), e.g. a methyl, ethyl, propyl, isopropyl, butyl, sec-butyl isobutyl, or *tert*-butyl group, more particularly 1, 2 or 3 carbon atoms ("$C_1$-$C_3$-alkyl"), e.g. a methyl, ethyl, n-propyl or isopropyl group, more particularly 1 or 2 carbon atoms ("$C_1$-$C_2$-alkyl"), e.g. a methyl or ethyl group.

The term "$C_1$-$C_4$-hydroxyalkyl" means a linear or branched, saturated, monovalent hydrocarbon group in which the term "$C_1$-$C_4$-alkyl" is defined *supra,* and in which 1 or 2 hydrogen atoms are replaced with a hydroxy group, *e.g.* a hydroxymethyl, 1-hydroxyethyl, 2-hydroxyethyl, 1,2-dihydroxyethyl, 3-hydroxypropyl, 2-hydroxypropyl, 1-hydroxypropyl, 1-hydroxypropan-2-yl, 2-hydroxypropan-2-yl, 2,3-dihydroxypropyl, 1,3-dihydroxypropan-2-yl, 3-hydroxy-2-methyl-propyl, 2-hydroxy-2-methyl-propyl, 1-hydroxy-2-methyl-propyl, 1-hydroxybutyl, 2-hydroxybutyl, 3-hydroxybutyl, 4-hydroxybutyl group, or an isomer thereof.

The term "$C_1$-$C_6$-haloalkyl" means a linear or branched, saturated, monovalent hydrocarbon group in which the term "$C_1$-$C_6$-alkyl" is as defined *supra,* and in which one or more of the hydrogen atoms are replaced, identically or differently, with a halogen atom. Particularly, said halogen atom is a fluorine atom. Said $C_1$-$C_6$-haloalkyl group is, for example, fluoromethyl, difluoromethyl, trifluoromethyl, 2-fluoroethyl, 2,2-difluoroethyl, 2,2,2-trifluoroethyl, pentafluoroethyl, 3,3,3-trifluoropropyl or 1,3-difluoropropan-2-yl.

The term "$C_1$-$C_6$-alkoxy" means a linear or branched, saturated, monovalent group of formula ($C_1$-$C_6$-alkyl)-O-, in which the term "$C_1$-$C_6$-alkyl" is as defined *supra, e.g.* a methoxy, ethoxy, *n*-propoxy, isopropoxy, n-butoxy, sec-butoxy, isobutoxy, *tert*-butoxy, pentyloxy, isopentyloxy or *n*-hexyloxy group, or an isomer thereof.

The term "$C_1$-$C_6$-haloalkoxy" means a linear or branched, saturated, monovalent $C_1$-$C_6$-alkoxy group, as defined *supra,* in which one or more of the hydrogen atoms is replaced, identically or differently, with a halogen atom. Particularly, said halogen atom is a fluorine atom. Said $C_1$-$C_6$-haloalkoxy group is, for example, fluoromethoxy, difluoromethoxy,

trifluoromethoxy, 2,2,2-trifluoroethoxy or pentafluoroethoxy.

The term "$C_3$-$C_4$-alkenyl" means a linear or branched, monovalent hydrocarbon group, which contains one or two double bonds, and which has 3 or 4 carbon atoms. Said alkenyl group is, for example, a prop-2-en-1-yl (or "allyl"), prop-1-en-1-yl, but-3-enyl, but-2-enyl or but-1-enyl group.

The term "$C_3$-$C_4$-alkynyl" means a linear or branched, monovalent hydrocarbon group which contains one triple bond, and which contains 3 or 4 carbon atoms. Said $C_3$-$C_4$-alkynyl group is, for example, a prop-1-ynyl, prop-2-ynyl (or "propargyl"), but-1-ynyl, but-2-ynyl or but-3-ynyl group.

The term "$C_3$-$C_7$-cycloalkyl" means a saturated, monovalent, monocyclic hydrocarbon ring which contains 3, 4, 5, 6 or 7 carbon ring atoms ("$C_3$-$C_7$-cycloalkyl"). Said $C_3$-$C_7$-cycloalkyl group is for example, a cyclopropyl, cyclobutyl, cyclopentyl, cyclohexyl or cycloheptyl group.

The term "bicyclic $C_6$-$C_{11}$-cycloalkyl" means a spirocycloalkyl, fused $C_6$-$C_{10}$-cycloalkyl or bridged $C_7$-$C_{10}$-cycloalkyl group as defined below:

The term "spirocycloalkyl" means a bicyclic, saturated, monovalent $C_5$-$C_{11}$ hydrocarbon group in which the two rings share one common ring carbon atom, and wherein said bicyclic hydrocarbon group contains 5, 6, 7, 8, 9, 10 or 11 carbon atoms, it being possible for said spirocycloalkyl group to be attached to the rest of the molecule via any one of the carbon atoms except the spiro carbon atom. Said spirocycloalkyl group is, for spiro[2.6]nonyl, spiro[3.3]heptyl, spiro[3.4]octyl, spiro[3.5]nonyl, spiro[3.6]decyl, spiro[4.4]nonyl, spiro[4.5]decyl, spiro[4.6]undecyl or spiro[5.5]undecyl.

The term "fused $C_6$-$C_{10}$-cycloalkyl" means a bicyclic, saturated, monovalent hydrocarbon group, in which the two rings share two adjacent ring atoms, such as bicyclo[4.2.0]octyl, octahydropentalenyl or decalinyl.

The term "bridged $C_7$-$C_{10}$-cycloalkyl" means a bicyclic, saturated, monovalent hydrocarbon group which the two rings share two common ring atoms which are not adjacent, *e.g.* bicyclo[2.2.1]heptyl (also known as norbornyl).

The term "bicyclic $C_5$-$C_{11}$-cycloalkyl" means a spirocycloalkyl, fused $C_5$-$C_{10}$-cycloalkyl or bridged $C_5$-$C_{10}$-cycloalkyl group as defined below:

The term "spirocycloalkyl" means a bicyclic, saturated, monovalent $C_5$-$C_{11}$ hydrocarbon group in which the two rings share one common ring carbon atom, and wherein said bicyclic hydrocarbon group contains 5, 6, 7, 8, 9, 10 or 11 carbon atoms, it being possible for said spirocycloalkyl group to be attached to the rest of the molecule via any one of the carbon atoms except the spiro carbon atom. Said spirocycloalkyl group is, for spiro[2.6]nonyl, spiro[3.3]heptyl, spiro[3.4]octyl, spiro[3.5]nonyl, spiro[3.6]decyl, spiro[4.4]nonyl, spiro[4.5]decyl, spiro[4.6]undecyl or spiro[5.5]undecyl.

The term "fused $C_5$-$C_{10}$-cycloalkyl" means a bicyclic, saturated, monovalent hydrocarbon group, in which the two rings share two adjacent ring atoms, such as bicyclo[4.2.0]octyl, octahydropentalenyl or decalinyl.

The term "bridged $C_5$-$C_{10}$-cycloalkyl" means a bicyclic, saturated, monovalent hydrocarbon group which the two rings share two common ring atoms which are not adjacent, *e.g.* bicyclo[1.1.1]pentyl or bicyclo[2.2.1]heptyl (also known as norbornyl).

The term "monocyclic 4- to 7-membered heterocycloalkyl" means a monocyclic, saturated heterocycle with 4, 5, 6 or 7 ring atoms in total, which contains one or two identical or different ring heteroatoms from the series N, O and S.

Said monocyclic heterocycloalkyl group, without being limited thereto, can be a 4-membered ring, such as azetidinyl, oxetanyl or thietanyl, for example; or a 5-membered ring, such as tetrahydrofuranyl, 1,3-dioxolanyl, thiolanyl, pyrrolidinyl, imidazolidinyl, pyrazolidinyl, 1,1-dioxidothiolanyl, 1,2-oxazolidinyl, 1,3-oxazolidinyl or 1,3-thiazolidinyl, for example; or a 6-membered ring, such as tetrahydropyranyl, tetrahydrothiopyranyl, piperidinyl, morpholinyl, dithianyl, thiomorpholinyl, piperazinyl, 1,3-dioxanyl, 1,4-dioxanyl or 1,2-oxazinanyl, for example, or a 7-membered ring, such as azepanyl, 1,4-diazepanyl or 1,4-oxazepanyl, for example.

The term "monocyclic nitrogen containing 4- to 7-membered heterocycloalkyl group" means a monocyclic, saturated heterocycle with 4, 5, 6 or 7 ring atoms in total, which contains one ring nitrogen atom and optionally one further ring heteroatom from the series N, O and S.

Said monocyclic nitrogen containing 4- to 7-membered heterocycloalkyl group, without being limited thereto, can be a 4-membered ring, such as azetidinyl, for example; or a 5-membered ring, such as pyrrolidinyl, imidazolidinyl, pyrazolidinyl, 1,2-oxazolidinyl, 1,3-oxazolidinyl or 1,3-thiazolidinyl, for example; or a 6-membered ring, such as piperidinyl, morpholinyl, thiomorpholinyl, piperazinyl, or 1,2-oxazinanyl, for example, or a 7-membered ring, such as azepanyl, 1,4-diazepanyl or 1,4-oxazepanyl, for example.

The term "monocyclic nitrogen containing 4- to 7-membered heterocycloalkyl group which is optionally benzocondensed" means a monocyclic, saturated heterocycle with 4, 5, 6 or 7 ring atoms in total, which contains one ring nitrogen atom and optionally one further ring heteroatom from the series N, O and S, in which two adjacent ring carbon atoms may be shared with a benzene ring optionally fused thereto, such group being one of the aforementioned monocyclic nitrogen containing 4- to 7-membered heterocycloalkyl groups, such as pyrrolidinyl, piperidinyl, and the like, or benzocondensed groups *e.g.* 3,4-dihydroquinolin-1(2H)-yl, 3,4-dihydroisoquinolin-2(1H)-yl, 1,3-dihydro-2H-isoindol-2-yl or 2,3-dihydro-1H-indol-1-yl.

The term "bicyclic 6-11 membered heterocycloalkyl" means a 6- to 11-membered heterospirocycloalkyl, a 6- to 10-membered fused heterocycloalkyl or a 7- to 10-membered bridged heterocycloalkyl group as defined below:

The term "6- to 11-membered heterospirocycloalkyl" means a bicyclic, saturated heterocycle with 6, 7, 8, 9, 10 or 11 ring atoms in total, in which the two rings share one common ring carbon atom, which "heterospirocycloalkyl" contains one or two identical or different ring heteroatoms from the series: N, O, S; it being possible for said heterospirocycloalkyl group to be attached to the rest of the molecule via any one of the carbon atoms, except the spiro carbon atom, or, if present, a nitrogen atom.

Said heterospirocycloalkyl group is, for example, azaspiro[2.3]hexyl, azaspiro[3.3]heptyl, oxaazaspiro[3.3]heptyl, thiaazaspiro[3.3]heptyl, oxaspiro[3.3]heptyl, oxazaspiro[5.3]nonyl, oxazaspiro[4.3]octyl, azaspiro[4,5]decyl, oxazaspiro [5.5]undecyl, diazaspiro[3.3]heptyl, thiazaspiro[3.3]heptyl, thiazaspiro[4.3]octyl, azaspiro[5.5]undecyl, or one of the further homologous scaffolds such as spiro[3.4]-, spiro[4.4]-, spiro[2.4]-, spiro[2.5]-, spiro[2.6]-, spiro[3.5]-, spiro [3.6]-, spiro[4.5]- and spiro[4.6]-.

The term "6- to 10-membered fused heterocycloalkyl" means a bicyclic, saturated heterocycle with 6, 7, 8, 9 or 10 ring atoms in total, in which the two rings share two adjacent ring atoms, which "fused heterocycloalkyl" contains one or two identical or different ring heteroatoms from the series: N, O, S; it being possible for said fused heterocycloalkyl group to be attached to the rest of the molecule via any one of the carbon atoms or, if present, a nitrogen atom.

Said fused heterocycloalkyl group is, for example, azabicyclo[3.3.0]octyl, azabicyclo[4.3.0]nonyl, diazabicyclo[4.3.0] nonyl, oxazabicyclo[4.3.0]nonyl, thiazabicyclo[4.3.0]nonyl or azabicyclo[4.4.0]decyl.

The term "7- to 10-membered bridged heterocycloalkyl" means a bicyclic, saturated heterocycle with 7, 8, 9 or 10 ring atoms in total, in which the two rings share two common ring atoms which are not adjacent, which "bridged heterocycloalkyl" contains one or two identical or different ring heteroatoms from the series: N, O, S; it being possible for said bridged heterocycloalkyl group to be attached to the rest of the molecule via any one of the carbon atoms, except the spiro carbon atom, or, if present, a nitrogen atom.

Said bridged heterocycloalkyl group is, for example, azabicyclo[2.2.1]heptyl, oxazabicyclo[2.2.1]heptyl, thiazabicyclo [2.2.1]heptyl, diazabicyclo[2.2.1]heptyl, azabicyclo[2.2.2]octyl, diazabicyclo[2.2.2]octyl, oxazabicyclo[2.2.2]octyl, thiazabicyclo[2.2.2]octyl, azabicyclo[3.2.1]octyl, diazabicyclo[3.2.1]octyl, oxazabicyclo[3.2.1]octyl, thiazabicyclo [3.2.1]octyl, azabicyclo[3.3.1]nonyl, diazabicyclo[3.3.1]nonyl, oxazabicyclo[3.3.1]nonyl, thiazabicyclo[3.3.1]nonyl, azabicyclo[4.2.1]nonyl, diazabicyclo[4.2.1]nonyl, oxazabicyclo[4.2.1]nonyl, thiaza-bicyclo[4.2.1]nonyl, azabicyclo [3.3.2]decyl, diazabicyclo[3.3.2]decyl, oxazabicyclo[3.3.2]decyl, thiazabicyclo[3.3.2]decyl or azabicyclo[4.2.2]decyl.

The term "bicyclic nitrogen containing 6-11 membered heterocycloalkyl" means a heterospirocycloalkyl, fused heterocycloalkyl or bridged heterocycloalkyl group as defined *supra,* however containing one ring nitrogen atom and optionally one or two further ring heteroatoms from the series N, O and S; it being possible for said bicyclic nitrogen containing 6-11 membered heterocycloalkyl group to be attached to the rest of the molecule via a nitrogen atom or any one of the carbon atoms, except a spiro carbon atom.

The term "bicyclic 5-11 membered heterocycloalkyl" means a 5-11 membered heterospirocycloalkyl, a 5-11 membered fused heterocycloalkyl or a 5-11 membered bridged heterocycloalkyl group as defined below:

The term "5-11 membered heterospirocycloalkyl" means a bicyclic, saturated heterocycle with 5, 6, 7, 8, 9, 10 or 11 ring atoms in total, in which the two rings share one common ring carbon atom, which "heterospirocycloalkyl" contains one or two identical or different ring heteroatoms from the series: N, O, S; it being possible for said heterospirocycloalkyl group to be attached to the rest of the molecule via any one of the carbon atoms, except the spiro carbon atom, or, if present, a nitrogen atom.

Said heterospirocycloalkyl group is, for example, azaspiro[2.2]pentyl, azaspiro[2.3]hexyl, azaspiro[3.3]heptyl, oxaazaspiro[3.3]heptyl, thiaazaspiro[3.3]heptyl, oxaspiro[3.3]heptyl, oxazaspiro[5.3]nonyl, oxazaspiro[4.3]octyl, azaspiro[4,5]decyl, oxazaspiro [5.5]undecyl, diazaspiro[3.3]heptyl, thiazaspiro[3.3]heptyl, thiazaspiro[4.3]octyl, azaspiro [5.5]undecyl, or one of the further homologous scaffolds such as spiro[3.4]-, spiro[4.4]-, spiro[2.4]-, spiro[2.5]-, spiro [2.6]-, spiro[3.5]-, spiro[3.6]-, spiro[4.5]- and spiro[4.6]-.

The term "5-11 membered fused heterocycloalkyl" means a bicyclic, saturated heterocycle with 5, 6, 7, 8, 9 or 10 ring atoms in total, in which the two rings share two adjacent ring atoms, which "fused heterocycloalkyl" contains one or two identical or different ring heteroatoms from the series: N, O, S; it being possible for said fused heterocycloalkyl group to be attached to the rest of the molecule via any one of the carbon atoms or, if present, a nitrogen atom.

Said fused heterocycloalkyl group is, for example, azabicyclo[3.1.0]hexyl, azabicyclo[3.3.0]octyl, azabicyclo[4.3.0] nonyl, diazabicyclo[4.3.0]nonyl, oxazabicyclo[4.3.0]nonyl, thiazabicyclo[4.3.0]nonyl or azabicyclo[4.4.0]decyl.

The term "5-11 membered bridged heterocycloalkyl" means a bicyclic, saturated heterocycle with 5, 6, 7, 8, 9 or 10 ring atoms in total, in which the two rings share two common ring atoms which are not adjacent, which "bridged heterocycloalkyl" contains one or two identical or different ring heteroatoms from the series: N, O, S; it being possible for said bridged heterocycloalkyl group to be attached to the rest of the molecule via any one of the carbon atoms, except the spiro carbon atom, or, if present, a nitrogen atom.

Said bridged heterocycloalkyl group is, for example, azabicyclo[2.2.1]heptyl, oxazabicyclo[2.2.1]heptyl, thiazabicyclo [2.2.1]heptyl, diazabicyclo[2.2.1]heptyl, azabicyclo[2.2.2]octyl, diazabicyclo[2.2.2]octyl, oxazabicyclo[2.2.2]octyl, thiazabicyclo[2.2.2]octyl, azabicyclo[3.2.1]octyl, diazabicyclo[3.2.1]octyl, oxazabicyclo[3.2.1]octyl, thiazabicyclo [3.2.1]octyl, azabicyclo[3.3.1]nonyl, diazabicyclo[3.3.1]nonyl, oxazabicyclo[3.3.1]nonyl, thiazabicyclo[3.3.1]nonyl, azabicyclo[4.2.1]nonyl, diazabicyclo[4.2.1]nonyl, oxazabicyclo[4.2.1]nonyl, thiaza-bicyclo[4.2.1]nonyl, azabicyclo [3.3.2]decyl, diazabicyclo[3.3.2]decyl, oxazabicyclo[3.3.2]decyl, thiazabicyclo[3.3.2]decyl or azabicyclo[4.2.2]decyl.

The term "bicyclic nitrogen containing 5-11 membered heterocycloalkyl" means a 5-11 membered heterospirocycloalkyl, 5-11 membered fused heterocycloalkyl or 5-11 membered bridged heterocycloalkyl group as defined *supra,* however containing one ring nitrogen atom and optionally one or two further ring heteroatoms from the series N, O and S; it being possible for said bicyclic nitrogen containing 5-11 membered heterocycloalkyl group to be attached to the rest of the molecule via a nitrogen atom or any one of the carbon atoms, except a spiro carbon atom.

The term "heteroaryl" means a monovalent, monocyclic or bicyclic aromatic ring having 5, 6, 8, 9 or 10 ring atoms (a "5- to 10-membered heteroaryl" group), which contains at least one ring heteroatom and optionally one, two or three further ring heteroatoms from the series: N, O and/or S, and which is bound via a ring carbon atom, or, if valency allows as *e.g.* in pyrrol-1-yl, a nitrogen atom.

Said heteroaryl group can be a 5-membered heteroaryl group, such as, for example, thienyl, furanyl, pyrrolyl, oxazolyl, thiazolyl, imidazolyl, pyrazolyl, isoxazolyl, isothiazolyl, oxadiazolyl, triazolyl, thiadiazolyl or tetrazolyl; or a 6-membered heteroaryl group, such as, for example, pyridinyl (herein also referred to as pyridyl), pyridazinyl, pyrimidinyl, pyrazinyl or triazinyl; or a 9-membered heteroaryl group, such as, for example, benzofuranyl, benzothienyl, benzoxazolyl, benzisoxazolyl, benzimidazolyl, benzothiazolyl, benzotriazolyl, thiazolopyridinyl, indazolyl, indolyl, isoindolyl, indolizinyl or purinyl; or a 10-membered heteroaryl group, such as, for example, quinolinyl, quinazolinyl, isoquinolinyl, cinnolinyl, phthalazinyl, quinoxalinyl or pteridinyl.

In general, and unless otherwise mentioned, the heteroaryl or heteroarylene groups include all possible isomeric forms thereof, *e.g.*: tautomers and positional isomers with respect to the point of linkage to the rest of the molecule. Thus, for some illustrative non-restricting examples, the term pyridinyl includes pyridin-2-yl, pyridin-3-yl and pyridin-4-yl; or the term thienyl includes thien-2-yl and thien-3-yl.

The term "$C_1$-$C_6$", as used in the present text, e.g. in the context of the definition of "$C_1$-$C_6$-alkyl", "$C_1$-$C_6$-haloalkyl", "$C_1$-$C_6$-hydroxyalkyl", "$C_1$-$C_6$-alkoxy" or "$C_1$-$C_6$-haloalkoxy" means an alkyl group having a finite number of carbon atoms of 1 to 6, *i.e.* 1, 2, 3, 4, 5 or 6 carbon atoms.

Further, as used herein, the term "$C_3$-$C_7$", as used in the present text, e.g. in the context of the definition of "$C_3$-$C_7$-cycloalkyl", means a cycloalkyl group having a finite number of carbon atoms of 3 to 7, *i.e.* 3, 4, 5, 6 or 7 carbon atoms.

When a range of values is given, said range encompasses each value and sub-range within said range.

For example:

"$C_1$-$C_6$" encompasses $C_1$, $C_2$, $C_3$, $C_4$, $C_5$, $C_6$, $C_1$-$C_6$, $C_1$-$C_5$, $C_1$-$C_4$, $C_1$-$C_3$, $C_1$-$C_2$, $C_2$-$C_6$, $C_2$-$C_5$, $C_2$-$C_4$, $C_2$-$C_3$, $C_3$-$C_6$, $C_3$-$C_5$, $C_3$-$C_4$, $C_4$-$C_6$, $C_4$-$C_5$, and $C_5$-$C_6$;

"$C_2$-$C_6$" encompasses $C_2$, $C_3$, $C_4$, $C_5$, $C_6$, $C_2$-$C_6$, $C_2$-$C_5$, $C_2$-$C_4$, $C_2$-$C_3$, $C_3$-$C_6$, $C_3$-$C_5$, $C_3$-$C_4$, $C_4$-$C_6$, $C_4$-$C_5$, and $C_5$-$C_6$;

"$C_3$-$C_6$" encompasses $C_3$, $C_4$, $C_5$, $C_6$, $C_3$-$C_6$, $C_3$-$C_5$, $C_3$-$C_4$, $C_4$-$C_6$, $C_4$-$C_5$, and $C_5$-$C_6$.

As used herein, the term "leaving group" means an atom or a group of atoms that is displaced in a chemical reaction as stable species taking with it the bonding electrons. In particular, such a leaving group is selected from the group comprising: a halogen atom, in particular a fluorine atom, a chlorine atom, a bromine atom or an iodide atom, being displaced as halide, in particular fluoride, chloride, bromide or iodide; (methylsulfonyl)oxy, [(trifluoromethyl)sulfonyl]oxy, [(nonafluorobutyl)sulfonyl]oxy, (phenylsulfonyl)oxy, [(4-methylphenyl)sulfonyl]oxy, [(4-bromophenyl)sulfonyl]oxy, [(4-nitrophenyl)sulfonyl]oxy, [(2-nitrophenyl)sulfonyl]oxy, [(4-isopropylphenyl)sulfonyl]oxy, [(2,4,6-triisopropylphenyl)sulfonyl]oxy, [(2,4,6-trimethylphenyl)sulfonyl]oxy, [(4-tert-butylphenyl)sulfonyl]oxy and [(4-methoxyphenyl)sulfonyl]oxy.

As used herein, the term "dipolar aprotic solvent" means a solvent selected from acetone, acetonitrile, priopionitrile, dimethylsulfoxide, diethylsulfoxide, N,N-dimethylformamide, N,N-dimethylacetamide, N,N-diethylformamide, N,N-diethylacetamide, 1-methyl-2-pyrrolidinone, 1-ethyl-2-pyrrolidinone, 1-methyl-2-piperidinone and 1-ethyl-2-piperidinone, or mixtures thereof. Particularly, said dipolar aprotic solvent is acetonitrile, dimethylsulfoxide, N,N-dimethylformamide, N,N-dimethylacetamide or 1-methyl-2-pyrrolidinone.

As used herein, the term "room temperature" means a temperature in the range from 15 °C to 25 °C.

It is possible for the compounds of general formula (I) to exist as isotopic variants. The invention therefore includes one or more isotopic variant(s) of the compounds of general formula (I), particularly deuterium-containing compounds of general formula (I).

The term "Isotopic variant" of a compound or a reagent is defined as a compound exhibiting an unnatural proportion of one or more of the isotopes that constitute such a compound.

The term "Isotopic variant of the compound of general formula (I)" is defined as a compound of general formula (I) exhibiting an unnatural proportion of one or more of the isotopes that constitute such a compound.

The expression "unnatural proportion" means a proportion of such isotope which is higher than its natural abundance. The natural abundances of isotopes to be applied in this context are described in "Isotopic Compositions of the Elements 1997", Pure Appl. Chem., 70(1), 217-235, 1998.

Examples of such isotopes include stable and radioactive isotopes of hydrogen, carbon, nitrogen, oxygen, phosphorus, sulfur, fluorine, chlorine, bromine and iodine, such as $^{2}H$ (deuterium), $^{3}H$ (tritium), $^{11}C$, $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$ and $^{131}I$, respectively.

With respect to the treatment and/or prophylaxis of the disorders specified herein the isotopic variant(s) of the compounds of general formula (I) preferably contain deuterium ("deuterium-containing compounds of general formula (I)"). Isotopic variants of the compounds of general formula (I) in which one or more radioactive isotopes, such as $^{3}H$ or $^{14}C$, are incorporated are useful e.g. in drug and/or substrate tissue distribution studies. These isotopes are particularly preferred for the ease of their incorporation and detectability. Positron emitting isotopes such as $^{18}F$ or $^{11}C$ may be incorporated into a compound of general formula (I). These isotopic variants of the compounds of general formula (I) are useful for in vivo imaging applications. Deuterium-containing and $^{13}C$-containing compounds of general formula (I) can be used in mass spectrometry analyses in the context of preclinical or clinical studies.

Isotopic variants of the compounds of general formula (I) can generally be prepared by methods known to a person skilled in the art, such as those described in the schemes and/or examples herein, by substituting a reagent for an isotopic variant of said reagent, preferably for a deuterium-containing reagent. Depending on the desired sites of deuteration, in some cases deuterium from $D_2O$ can be incorporated either directly into the compounds or into reagents that are useful for synthesizing such compounds. Deuterium gas is also a useful reagent for incorporating deuterium into molecules. Catalytic deuteration of olefinic bonds and acetylenic bonds is a rapid route for incorporation of deuterium. Metal catalysts (i.e. Pd, Pt, and Rh) in the presence of deuterium gas can be used to directly exchange deuterium for hydrogen in functional groups containing hydrocarbons. A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA.

The term "deuterium-containing compound of general formula (I)" is defined as a compound of general formula (I), in which one or more hydrogen atom(s) is/are replaced by one or more deuterium atom(s) and in which the abundance of deuterium at each deuterated position of the compound of general formula (I) is higher than the natural abundance of deuterium, which is about 0.015%. Particularly, in a deuterium-containing compound of general formula (I) the abundance of deuterium at each deuterated position of the compound of general formula (I) is higher than 10%, 20%, 30%, 40%, 50%, 60%, 70% or 80%, preferably higher than 90%, 95%, 96% or 97%, even more preferably higher than 98% or 99% at said position(s). It is understood that the abundance of deuterium at each deuterated position is independent of the abundance of deuterium at other deuterated position(s).

The selective incorporation of one or more deuterium atom(s) into a compound of general formula (I) may alter the

physicochemical properties (such as for example acidity [C. L. Perrin, et al., J. Am. Chem. Soc., 2007, 129, 4490], basicity [C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of general formula (I). In some cases deuterium substitution reduces or eliminates the formation of an undesired or toxic metabolite and enhances the formation of a desired metabolite (e.g. Nevirapine: A. M. Sharma et al., Chem. Res. Toxicol., 2013, 26, 410; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels. This could result in lower side effects and enhanced efficacy, depending on the particular compound's pharmacokinetic/ pharmacodynamic relationship. ML-337 (C. J. Wenthur et al., J. Med. Chem., 2013, 56, 5208) and Odanacatib (K. Kassahun et al., WO2012/112363) are examples for this deuterium effect. Still other cases have been reported in which reduced rates of metabolism result in an increase in exposure of the drug without changing the rate of systemic clearance (e.g. Rofecoxib: F. Schneider et al., Arzneim. Forsch. / Drug. Res., 2006, 56, 295; Telaprevir: F. Maltais et al., J. Med. Chem., 2009, 52, 7993). Deuterated drugs showing this effect may have reduced dosing requirements (e.g. lower number of doses or lower dosage to achieve the desired effect) and/or may produce lower metabolite loads.

A compound of general formula (I) may have multiple potential sites of attack for metabolism. To optimize the above-described effects on physicochemical properties and metabolic profile, deuterium-containing compounds of general formula (I) having a certain pattern of one or more deuterium-hydrogen exchange(s) can be selected. Particularly, the deuterium atom(s) of deuterium-containing compound(s) of general formula (I) is/are attached to a carbon atom and/or is/are located at those positions of the compound of general formula (I), which are sites of attack for metabolizing enzymes such as e.g. cytochrome $P_{450}$.

In another embodiment the present invention concerns a deuterium-containing compound of general formula (I) having 1, 2, 3 or 4 deuterium atoms, particularly with 1, 2 or 3 deuterium atoms.

Where the plural form of the word compounds, salts, polymorphs, hydrates, solvates and the like, is used herein, this is taken to mean also a single compound, salt, polymorph, isomer, hydrate, solvate or the like.

By "stable compound' or "stable structure" is meant a compound that is sufficiently robust to survive isolation to a useful degree of purity from a reaction mixture, and formulation into an efficacious therapeutic agent.

The compounds of the present invention optionally contain one or more asymmetric centres, depending upon the location and nature of the various substituents desired. It is possible that one or more asymmetric carbon atoms are present in the (R) or (S) configuration, which can result in racemic mixtures in the case of a single asymmetric centre, and in diastereomeric mixtures in the case of multiple asymmetric centres. In certain instances, it is possible that asymmetry also be present due to restricted rotation about a given bond, for example, the central bond adjoining two substituted aromatic rings of the specified compounds.

Preferred isomers are those which produce the more desirable biological activity. These separated, pure or partially purified isomers or racemic mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g.*, HPLC columns using a chiral phase), with or without conventional derivatisation, optimally chosen to maximise the separation of the enantiomers. Suitable HPLC columns using a chiral phase are commercially available, such as those manufactured by Daicel, *e.g.*, Chiracel OD and Chiracel OJ, for example, among many others, which are all routinely selectable. Enzymatic separations, with or without derivatisation, are also useful. The optically active compounds of the present invention can likewise be obtained by chiral syntheses utilizing optically active starting materials.

In order to distinguish different types of isomers from each other reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e.g.* (R)- or (S)-isomers, in any ratio. Isolation of a single

stereoisomer, *e.g.* a single enantiomer or a single diastereomer, of a compound of the present invention is achieved by any suitable state of the art method, such as chromatography, especially chiral chromatography, for example.

Further, it is possible for some of the compounds of the present invention to exist as tautomers. For example, the compounds of the present invention may contain a pyridone moiety and can exist as a pyridone, or as an hydroxypyridine, or even a mixture in any amount of the two tautomers, namely :

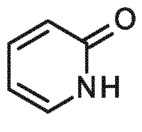

pyridone

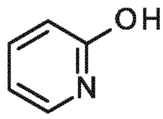

hydroxypyridine

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

The present invention also covers useful forms of the compounds of the present invention, such as hydrates, solvates, salts, in particular pharmaceutically acceptable salts, and/or co-precipitates.

The compounds of the present invention can exist as a hydrate, or as a solvate, wherein the compounds of the present invention contain polar solvents, in particular water, methanol or ethanol for example, as structural element of the crystal lattice of the compounds. It is possible for the amount of polar solvents, in particular water, to exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.

Further, it is possible for the compounds of the present invention to exist in free form, *e.g.* as a free base, or as a free acid, or as a zwitterion, or to exist in the form of a salt. Said salt may be any salt, either an organic or inorganic addition salt, particularly any pharmaceutically acceptable organic or inorganic addition salt, which is customarily used in pharmacy, or which is used, for example, for isolating or purifying the compounds of the present invention.

The term "pharmaceutically acceptable salt" refers to an inorganic or organic acid addition salt of a compound of the present invention. For example, see S. M. Berge et al., "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, or "mineral acid", such as hydrochloric, hydrobromic, hydroiodic, sulfuric, sulfamic, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, 3-phenylpropionic, pivalic, 2-hydroxyethanesulfonic, itaconic, trifluoromethanesulfonic, dodecylsulfuric, ethanesulfonic, benzenesulfonic, para-toluenesulfonic, methanesulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium, magnesium or strontium salt, or an aluminium or a zinc salt, or an ammonium salt derived from ammonia or from an organic primary, secondary or tertiary amine having 1 to 20 carbon atoms, such as ethylamine, diethylamine, triethylamine, ethyldiisopropylamine, monoethanolamine, diethanolamine, triethanolamine, dicyclohexylamine, dimethylaminoethanol, diethylaminoethanol, tris(hydroxymethyl)aminomethane, procaine, dibenzylamine, *N*-methylmorpholine, arginine, lysine, 1,2-ethylenediamine, *N*-methylpiperidine, *N*-methyl-glucamine, *N,N*-dimethyl-glucamine, *N*-ethyl-glucamine, 1,6-hexanediamine, glucosamine, sarcosine, serinol, 2-amino-1,3-propanediol, 3-amino-1,2-propanediol, 4-amino-1,2,3-butanetriol, or a salt with a quarternary ammonium ion having 1 to 20 carbon atoms, such as tetramethylammonium, tetraethylammonium, tetra(*n*-propyl)ammonium, tetra(*n*-butyl)ammonium, *N*-benzyl-*N,N,N*-trimethylammonium, choline or benzalkonium.

Those skilled in the art will further recognise that it is possible for acid addition salts of the claimed compounds to be prepared by reaction of the compounds with the appropriate inorganic or organic acid via any of a number of known methods. Alternatively, alkali and alkaline earth metal salts of acidic compounds of the present invention are prepared by reacting the compounds of the present invention with the appropriate base via a variety of known methods. The present invention includes all possible salts of the compounds of the present invention as single salts, or as any mixture of said

salts, in any ratio.

In the present text, in particular in the "Experimental Section", for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown. Unless specified otherwise, suffixes to chemical names or structural formulae relating to salts, such as "hydrochloride", "trifluoroacetate", "sodium salt", or "x HCl", "x $CF_3COOH$", "x $Na^+$", for example, mean a salt form, the stoichiometry of which salt form not being specified.

This applies analogously to cases in which synthesis intermediates or example compounds or salts thereof have been obtained, by the preparation and/or purification processes described, as solvates, such as hydrates, with (if defined) unknown stoichiometric composition.

Furthermore, the present invention includes all possible crystalline forms, or polymorphs, of the compounds of the present invention, either as single polymorph, or as a mixture of more than one polymorph, in any ratio.

The term "prodrugs" here designates compounds which themselves can be biologically active or inactive, but are converted (for example metabolically or hydrolytically) into compounds according to the invention during their residence time in the body.

The invention further includes all possible cyclodextrin clathrates, *i.e.* alpha-, beta-, or gamma-cyclodextrins, hydroxypropyl-beta-cyclodextrins, methylbetacyclodextrins.

In accordance with a second embodiment of the first aspect, the present invention covers compounds of general formula (I), *supra,* in which:

$R^1$ represents a phenyl or pyridinyl group optionally substituted, one, two, or three times, each substituent independently selected from a halogen atom or a group selected from hydroxy, cyano, nitro, $C_1$-$C_4$-alkyl, (phenyl)-($C_1$-$C_2$-alkyl)-, $C_1$-$C_4$-haloalkyl, $C_1$-$C_4$-alkoxy, (phenyl)-($C_1$-$C_2$-alkoxy)-, $C_1$-$C_4$-haloalkoxy, -N($R^5$)($R^6$),

wherein the phenyl groups in said (phenyl)-($C_1$-$C_2$-alkyl)- and (phenyl)-($C_1$-$C_2$-alkoxy)-groups are optionally substituted one or two times, each substituent independently selected from a fluorine atom, a chlorine atom and a bromine atom, or a group selected from methyl, trifluoromethyl and methoxy,

or two substituents attached to adjacent carbon atoms of said phenyl or pyridinyl group together form a bivalent group selected from $-(CH_2)_3-$, $-(CH_2)_4-$, $-(CH_2)_2-O-$, $-(CH_2)_3-O-$, $-CH_2-O-CH_2-$, $-O-CH_2-O-$, $-O-CH_2-CH_2-O-$ and $-O-CF_2-O-$,

or

$R^1$ represents a pyrazolyl group optionally substituted one or two times, each substituent independently selected from a halogen atom or a group selected from cyano, $C_1$-$C_2$-alkyl, and $C_1$-$C_2$-alkoxy;

$R^2$ represents a group

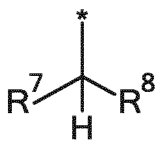

,

wherein "*" indicates the point of attachment to the nitrogen atom to which $R^2$ is attached;

$R^3$ represents a group selected from methyl and $-NH_2$;

$R^4$ represents a 5-membered heteroaryl group selected from oxazolyl, isoxazolyl, thiazolyl, isothiazolyl and oxadiazolyl, optionally substituted one or two times, each substituent independently selected from a fluorine atom, a chlorine atom and a bromine atom, or a group selected from $C_1$-$C_4$-alkyl, (($R^9$)O)-($C_1$-$C_3$-alkyl)-, (($R^{10}$)($R^{11}$)N)-($C_1$-$C_3$-alkyl)-, ($C_3$-$C_7$-cycloalkyl)-($C_1$-$C_3$-alkyl)-, $C_3$-$C_7$-cycloalkyl, $-OR^9$, -N($R^{10}$)($R^{11}$), -C(=O)-N($R^{15}$)($R^{16}$), - C(=O)-$OR^{17}$, phenyl and a 5- or 6-membered

heteroaryl group,
wherein the phenyl group and the 5- or 6-membered heteroaryl group are optionally substituted one or two times, each substituent independently selected from a fluorine atom, a chlorine atom and a bromine atom, or a group selected from cyano, $C_1$-$C_2$-alkyl, $C_1$-$C_2$-fluoroalkyl, -$OR^9$, -$N(R^{10})(R^{11})$ and -C(=O)-$N(R^{15})(R^{16})$;

$R^5$ and $R^6$ represent, independently from each occurrence, a hydrogen atom or a group selected from $C_1$-$C_2$-alkyl and ($C_1$-$C_2$-alkyl)-C(=O)-,
or

$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom, a chlorine atom and a bromine atom, or a group selected from oxo, hydroxy, $C_1$-$C_2$-alkyl and ($C_1$-$C_2$-alkyl)-C(=O)-;

$R^7$ represents a hydrogen atom or a $C_1$-$C_2$-alkyl group;

$R^8$ represents a -C(=O)-$NH_2$ group;

$R^9$ represents a hydrogen atom or a group selected from $C_1$-$C_4$-alkyl, ($C_3$-$C_7$-cycloalkyl)-($C_1$-$C_2$-alkyl)-, (phenyl)-($C_1$-$C_2$-alkyl)-, $C_1$-$C_4$-haloalkyl, $C_2$-$C_3$-hydroxyalkyl, ($C_1$-$C_2$-alkoxy)-$C_2$-alkyl-, (($C_1$-$C_2$-alkyl)-C(=O)-O)-$C_2$-alkyl-, -C(=O)-$N(R^{20})(R^{21})$, $C_3$-$C_7$-cycloalkyl and phenyl,

wherein $C_3$-$C_7$-cycloalkyl, and the $C_3$-$C_7$-cycloalkyl within said ($C_3$-$C_7$-cycloalkyl)-($C_1$-$C_2$-alkyl)- group is optionally substituted one or two times, each substituent independently selected from a fluorine atom or a group selected from oxo and methyl,

and wherein the phenyl group within said (phenyl)-($C_1$-$C_2$-alkyl)- group and said phenyl group itself are optionally substituted one or two times, each substituent independently selected from a fluorine atom, a chlorine atom and a bromine atom, or a group selected from cyano, methyl, trifluoromethyl and methoxy;

$R^{10}$ and $R^{11}$ represent, independently from each occurrence, a hydrogen atom or a group selected from $C_1$-$C_2$-alkyl, $C_1$-$C_2$-haloalkyl, $C_2$-$C_3$-hydroxyalkyl, ($C_1$-$C_2$-alkoxy)-$C_2$-alkyl-, (($R^{22}$)($R^{23}$) N)-$C_2$-alkyl, ($C_3$-$C_7$-cycloalkyl)-($C_1$-$C_2$-alkyl)-, ($C_1$-$C_2$-alkyl)-C(=O)-, $C_3$-$C_7$-cycloalkyl, ($C_3$-$C_7$-cycloalkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,

wherein $C_3$-$C_7$-cycloalkyl, and the $C_3$-$C_7$-cycloalkyl within said ($C_3$-$C_7$-cycloalkyl)-($C_1$-$C_2$-alkyl)- and ($C_3$-$C_7$-cycloalkyl)-C(=O)- groups are optionally substituted one or two times, each substituent independently selected from a fluorine atom or a group selected from cyano, $C_1$-$C_2$-alkyl and $C_1$-$C_2$-haloalkyl,

and wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a bromine atom, or a group selected from methyl, trifluoromethyl and methoxy,

or

$R^{10}$ orand $R^{11}$, together with the nitrogen atom to which they are attached, represent a monocyclic nitrogen containing 4- to 7-membered heterocycloalkyl group, or a bicyclic nitrogen containing 5- to 10-membered heterocycloalkyl group, which are optionally substituted one, two or three times, each substituent independently selected from a halogen atom or a group selected from cyano, oxo, hydroxy, $C_1$-$C_2$-alkyl, $C_1$-$C_2$-haloalkyl, ($C_1$-$C_2$-alkyl)-C(=O)-, $C_1$-$C_2$-alkoxy, -$N(R^{22})(R^{23})$, and a monocyclic 4- to 7-membered heterocycloalkyl group;

$R^{15}$ and $R^{16}$ represent, independently from each occurrence, a hydrogen atom or a group selected from $C_1$-$C_4$-alkyl, $C_1$-$C_4$-haloalkyl, $C_1$-$C_4$-hydroxyalkyl, (cyano)-$C_1$-$C_4$-alkyl-, ($C_1$-$C_4$-alkoxy)-$C_2$-$C_3$-alkyl-,

($C_1$-$C_4$-haloalkoxy)-$C_2$-$C_3$-alkyl-, ($C_3$-$C_7$-cycloalkyl)-($C_1$-$C_3$-alkyl)-, $C_3$-$C_7$-cycloalkyl, bicyclic $C_5$-$C_{11}$-cycloalkyl, monocyclic 4- to 7-membered heterocycloalkyl, (phenyl)-($C_1$-$C_3$-alkyl)-, phenyl and a 5- or 6-membered heteroaryl group,

wherein $C_3$-$C_7$-cycloalkyl, the $C_3$-$C_7$-cycloalkyl within said ($C_3$-$C_7$-cycloalkyl)-($C_1$-$C_3$-alkyl)- group, the bicyclic $C_5$-$C_{11}$-cycloalkyl and the monocyclic 4- to 7-membered heterocycloalkyl groups are optionally substituted one or two times, each substituent independently selected from a halogen atom or a group selected from oxo, $C_1$-$C_2$-alkyl, ($C_1$-$C_2$-alkyl)-C(=O)- and cyclopropyl,

and wherein the phenyl and the 5- or 6-membered heteroaryl groups, including the phenyl group within said (phenyl)-($C_1$-$C_3$-alkyl)- group, are optionally substituted one or two times, each substituent independently selected from a fluorine atom, a chlorine atom and a bromine atom, or a group selected from methyl, trifluoromethyl and methoxy,

or

$R^{15}$ and $R^{16}$, together with the nitrogen atom to which they are attached, represent a monocyclic nitrogen containing 4- to 7-membered heterocycloalkyl group which is optionally substituted one or two times, each substituent independently selected from a halogen atom or a group selected from oxo, $C_1$-$C_2$-alkyl and ($C_1$-$C_2$-alkyl)-C(=O)-;

$R^{17}$ represents a $C_1$-$C_4$-alkyl group;

$R^{20}$ represents a hydrogen atom or a group selected from $C_1$-$C_6$-alkyl, $C_3$-$C_7$-cycloalkyl, monocyclic 4- to 7-membered heterocycloalkyl, phenyl, and 5- to 6-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, monocyclic 4- to 7-membered heterocycloalkyl, phenyl, and 5- to 6-membered heteroaryl, said phenyl and 5- to 6-membered heteroaryl substituents themselves being optionally substituted one or two times, each substituent independently selected from a fluorine atom, a chlorine atom and a bromine atom, or a group selected from methyl, trifluoromethyl and methoxy,

and wherein $C_3$-$C_7$-cycloalkyl and monocyclic 4- to 7-membered heterocycloalkyl are optionally substituted one or two or three times, each substituent independently selected from a halogen atom or a group selected from cyano, oxo, hydroxy, $C_1$-$C_2$-alkyl and ($C_1$-$C_2$-alkyl)-C(=O)-,

and wherein said phenyl and 5- to 6-membered heteroaryl groups are optionally substituted one, two or three times, each substituent independently selected from a halogen atom or a group selected from cyano, $C_1$-$C_2$-alkyl, $C_1$-$C_2$-haloalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group, or

$R^{20}$ and $R^{21}$, together with the nitrogen atom to which they are attached, represent a monocyclic nitrogen containing 4- to 7-membered heterocycloalkyl group which is substituted one, two or three times, each substituent independently selected from a halogen atom or a group selected from cyano, oxo, hydroxy, $C_1$-$C_2$-alkyl, $C_1$-$C_2$-haloalkyl, (phenyl)-($C_1$-$C_2$-alkyl)-, ($C_1$-$C_2$-alkyl)-C(=O)-, $C_3$-$C_4$-cycloalkyl, $C_1$-$C_2$-alkoxy, $C_1$-$C_2$-haloalkoxy, - N($R^{22}$)($R^{23}$) and -C(=O)-N($R^{24}$)($R^{25}$)

$R^{22}$ and $R^{23}$ represent, independently from each occurrence, a hydrogen atom or a group selected from $C_1$-$C_2$-alkyl and ($C_1$-$C_2$-alkyl)-C(=O)-, and
$R^{24}$ and $R^{25}$ represent, independently from each occurrence, a hydrogen atom or a $C_1$-$C_2$-alkyl group,
and stereoisomers, tautomers, N-oxides, hydrates, solvates, and salts thereof, and mixtures of same.

In accordance with a third embodiment of the first aspect, the present invention covers compounds of general formula (I), *supra,* in which:

$R^1$ represents a phenyl or pyridinyl group optionally substituted, one, two, or three times, each substituent independently selected from a fluorine atom, a chlorine atom and a bromine atom, or a group selected from hydroxy, cyano, $C_1$-$C_4$-alkyl, $C_1$-$C_2$-fluoroalkyl, $C_1$-$C_2$-alkoxy, (phenyl)-($C_1$-$C_2$-alkoxy)-, $C_1$-$C_2$-fluoroalkoxy and -N($R^5$)($R^6$),
or two substituents attached to adjacent carbon atoms of said phenyl or pyridinyl group together form a bivalent group selected from $-(CH_2)_3-$, $-O-CH_2-O-$ and $-O-CF_2-O-$, or

$R^1$ represents a pyrazolyl group optionally substituted with one methyl group,

$R^2$ represents a group

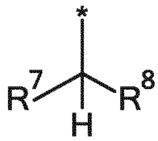

,

wherein "*" indicates the point of attachment to the nitrogen atom to which $R^2$ is attached;

$R^3$ represents a group selected from methyl and $-NH_2$;

$R^4$ represents a group selected from

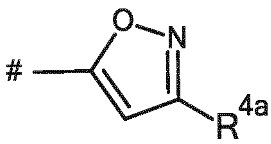

and

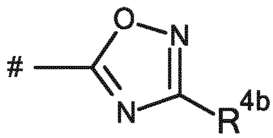

,

wherein "#" indicates the point of attachment to the carbonyl group to which $R^4$ is attached, and wherein

$R^{4a}$ and $R^{4b}$ represent, independently from each other, a fluorine atom, a chlorine atom or a bromine atom, or a group selected from $C_1$-$C_4$-alkyl, (($R^9$)O)-($C_1$-$C_2$-alkyl)-, (($R^{10}$)($R^{11}$)N)-($C_1$-$C_2$-alkyl)-, ($C_3$-$C_7$-cycloalkyl)-($C_1$-$C_2$-alkyl)-, $C_3$-$C_7$-cycloalkyl, -O$R^9$, -N($R^{10}$)($R^{11}$), - C(=O)-N($R^{15}$)($R^{16}$), -C(=O)-O$R^{17}$, phenyl and a 5- or 6-membered heteroaryl group selected from oxazolyl, isoxazolyl, thiazolyl, isothiazolyl, pyrazolyl, pyridinyl, pyrazinyl, pyrimidinyl and pyridazinyl,
wherein the phenyl group and the 5- or 6-membered heteroaryl group selected from oxazolyl, isoxazolyl, thiazolyl, isothiazolyl, pyrazolyl, pyridinyl, pyrazinyl, pyrimidinyl and pyridazinyl are optionally substituted one or two times, each substituent independently selected from a fluorine atom and a chlorine atom, or a group selected from cyano, $C_1$-$C_2$-alkyl, -O$R^9$, -N($R^{10}$)($R^{11}$) and -C(=O)-N($R^{15}$)($R^{16}$);

$R^5$ and $R^6$ represent, independently from each occurrence, a hydrogen atom or a $C_1$-$C_2$-alkyl group, or

$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom or a group selected from hydroxy and $C_1$-$C_2$-alkyl;

$R^7$ represents a hydrogen atom or a $C_1$-$C_2$-alkyl group;

$R^8$ represents a -C(=O)-$NH_2$ group;

$R^9$ represents a hydrogen atom or a group selected from $C_1$-$C_2$-alkyl, benzyl, $C_1$-$C_2$-fluoroalkyl, $C_2$-hydroxyalkyl, ($C_1$-$C_2$-alkoxy)-$C_2$-alkyl-, (($C_1$-$C_2$-alkyl)-C(=O)-O)-$C_2$-alkyl-, - C(=O)-N($R^{20}$)($R^{21}$) and phenyl,

wherein the phenyl group within said benzyl group and said phenyl group itself are optionally substituted one or two times, each substituent independently selected from a fluorine atom and a chlorine atom, or a group selected from cyano and methyl;

$R^{10}$ and $R^{11}$ represent, independently from each occurrence, a hydrogen atom or a group selected from $C_1$-$C_2$-alkyl, $C_1$-$C_2$-fluoroalkyl, ($C_3$-$C_5$-cycloalkyl)-($C_1$-$C_2$-alkyl)- ($C_1$-$C_2$-alkyl)-C(=O)-, $C_3$-$C_7$-cycloalkyl, $C_3$-$C_7$-cycloalkyl-(C=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,

wherein $C_3$-$C_7$-cycloalkyl, and the $C_3$-$C_5$-cycloalkyl within said ($C_3$-$C_5$-cycloalkyl)-($C_1$-$C_2$-alkyl)- and the $C_3$-$C_7$-cycloalkyl within the $C_3$-$C_7$-cycloalkyl-(C=O)- groups are optionally substituted one or two times, each substituent independently selected from a fluorine atom or a group selected from cyano, $C_1$-$C_2$-alkyl and $C_1$-$C_2$-fluoroalkyl,

and wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a methyl group,

or

$R^{10}$ and $R^{11}$, together with the nitrogen atom to which they are attached, represent a monocyclic nitrogen containing 4- to 7-membered heterocycloalkyl group which is optionally substituted one, two or three times, each substituent independently selected from a fluorine atom or a group selected from cyano, oxo, $C_1$-$C_2$-alkyl, $C_1$-$C_2$-fluoroalkyl and ($C_1$-$C_2$-alkyl)-C(=O)-;

$R^{15}$ and $R^{16}$ represent, independently from each occurrence, a hydrogen atom or a group selected from $C_1$-$C_4$-alkyl, $C_1$-$C_3$-fluoroalkyl, (cyano)-$C_1$-$C_2$-alkyl-, ($C_1$-$C_4$-alkoxy)-$C_2$-$C_3$-alkyl-, ($C_1$-$C_2$-fluoroalkoxy)-$C_2$-$C_3$-alkyl-, ($C_3$-$C_5$-cycloalkyl)-($C_1$-$C_2$-alkyl)-, $C_3$-$C_5$-cycloalkyl, phenyl and a 5- or 6-membered heteroaryl group selected from pyrazolyl, pyridinyl and pyrimidinyl,

wherein $C_3$-$C_5$-cycloalkyl, and the $C_3$-$C_5$-cycloalkyl within said ($C_3$-$C_5$-cycloalkyl)-($C_1$-$C_2$-alkyl)- group are optionally substituted one or two times, each substituent independently selected from a fluorine atom, or a group selected from methyl and cyclopropyl,

and wherein the phenyl and the 5- or 6-membered heteroaryl groups selected from pyrazolyl, pyridinyl and pyrimidinyl are optionally substituted one or two times, each substituent independently selected from a fluorine atom and a chlorine atom, or a group selected from methyl, trifluoromethyl and methoxy,

or

$R^{15}$ and $R^{16}$, together with the nitrogen atom to which they are attached, represent a monocyclic nitrogen containing 4- to 7-membered heterocycloalkyl group which is optionally substituted once with a fluorine atom or a group selected from oxo, $C_1$-$C_2$-alkyl and $CH_3$-C(=O)-;

$R^{17}$ represents a $C_1$-$C_2$-alkyl group;

$R^{20}$ represents a hydrogen atom or a group selected from $C_1$-$C_3$-alkyl and phenyl,

wherein said $C_1$-$C_3$-alkyl group is optionally substituted one or two times, each substituent independently selected from a fluorine atom or a group selected from hydroxy, $C_1$-$C_3$-alkoxy and phenyl, said phenyl itself being optionally substituted one or two times, each substituent independently selected from a fluorine atom, a chlorine atom and a methyl group,

and wherein said phenyl group is optionally substituted one, two or three times, each substituent independently selected from a fluorine atom and a chlorine atom or a group selected from methyl, trifluoromethyl, methoxy and trifluoromethoxy;

$R^{21}$ represents a hydrogen atom or a $C_1$-$C_2$-alkyl group;
or

$R^{20}$ and $R^{21}$, together with the nitrogen atom to which they are attached, represent a monocyclic nitrogen containing 4- to 7-membered heterocycloalkyl group which is optionally substituted one or two times, each substituent independently selected from a halogen atom or a group selected from cyano, oxo, hydroxy, $C_1$-$C_2$-alkyl, $C_1$-$C_2$-fluoroalkyl, benzyl, ($C_1$-$C_2$-alkyl)-C(=O)- and $C_3$-$C_4$-cycloalkyl,

and stereoisomers, tautomers, N-oxides, hydrates, solvates, and salts thereof, and mixtures of same.

In accordance with a fourth embodiment of the first aspect, the present invention covers compounds of general formula (I), *supra,* in which:

$R^1$ represents a phenyl or pyridinyl group optionally substituted, one or two times, each substituent independently selected from a fluorine atom, a chlorine atom and a bromine atom, or a group selected from methyl, difluoromethyl, trifluoromethyl, methoxy, benzyloxy, difluoromethoxy and trifluoromethoxy,
or two substituents attached to adjacent carbon atoms of said phenyl or pyridinyl group together form a bivalent group

$$-O-CF_2-O-;$$

$R^2$ represents a group

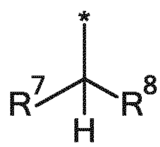

,

wherein "*" indicates the point of attachment to the nitrogen atom to which $R^2$ is attached;

$R^3$ represents a group selected from methyl and $-NH_2$;

$R^4$ represents a group

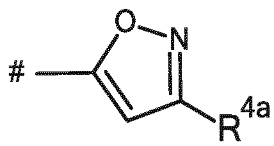

,

wherein "#" indicates the point of attachment to the carbonyl group to which $R^4$ is attached,

and wherein

$R^{4a}$ represents a chlorine atom or a bromine atom, or a group selected from (($R^9$)O)-($C_1$-$C_2$-alkyl)-, (($R^{10}$)($R^{11}$)N)-($C_1$-$C_2$-alkyl)-, ($C_3$-$C_5$-cycloalkyl)-($C_1$-$C_2$-alkyl)-, $C_3$-$C_5$-cycloalkyl, - $OR^9$, -C(=O)-N($R^{15}$)($R^{16}$) and phenyl,
wherein the phenyl group is optionally substituted one or two times, each substituent independently selected from a fluorine atom and a chlorine atom and a methyl group,
or

$R^4$ represents a group

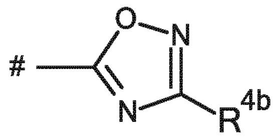

,

wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl, -C(=O)-N($R^{15}$)($R^{16}$) and phenyl, wherein the phenyl group is optionally substituted one or two times, each substituent independently selected from a fluorine atom and a chlorine atom, or a group selected from cyano, $C_1$-$C_2$-alkyl and -O$R^9$;

$R^7$ represents a hydrogen atom or a $C_1$-$C_2$-alkyl group;

$R^8$ represents a -C(=O)-$NH_2$ group;

$R^9$ represents a hydrogen atom or a group selected from $C_1$-$C_2$-alkyl, benzyl, $C_1$-$C_2$-fluoroalkyl, ($C_1$-$C_2$-alkoxy)-$C_2$-alkyl-, (($C_1$-$C_2$-alkyl)-C(=O)-O)-$C_2$-alkyl-, -C(=O)-N($R^{20}$)($R^{21}$) and phenyl,
wherein the phenyl group within said benzyl group and said phenyl group itself are optionally substituted one or two times, each substituent independently selected from a fluorine atom and a chlorine atom, or a group selected from cyano and methyl;

$R^{10}$ and $R^{11}$ represent, independently from each occurrence, a hydrogen atom or a group selected from $C_1$-$C_2$-alkyl, ($C_3$-$C_5$-cycloalkyl)-($C_1$-$C_2$-alkyl)-, $C_3$-$C_7$-cycloalkyl and (phenyl)-($C_1$-$C_2$-alkyl)-O-C(=O)-,

wherein $C_3$-$C_7$-cycloalkyl, and the $C_3$-$C_5$-cycloalkyl within said ($C_3$-$C_5$-cycloalkyl)-($C_1$-$C_2$-alkyl)-group are optionally substituted one or two times, each substituent independently selected from a fluorine atom or a group selected from cyano, methyl and $C_1$-fluoroalkyl,

nd wherein the phenyl group within said (phenyl)-($C_1$-$C_2$-alkyl)-O-C(=O)- group is optionally substituted one or two times, each substituent independently selected from a fluorine atom, a chlorine atom and a methyl group,

or

$R^{10}$ and $R^{11}$, together with the nitrogen atom to which they are attached, represent a monocyclic nitrogen containing 4- to 7-membered heterocycloalkyl group which is optionally substituted one, two or three times, each substituent independently selected from a fluorine atom or a group selected from cyano, methyl and $C_1$-fluoroalkyl;

$R^{15}$ and $R^{16}$ represent, independently from each occurrence, a hydrogen atom or a group selected from $C_1$-$C_4$-alkyl, $C_1$-$C_2$-fluoroalkyl, ($C_1$-$C_4$-alkoxy)-$C_2$-$C_3$-alkyl-, ($C_1$-$C_2$-fluoroalkoxy)-$C_2$-$C_3$-alkyl-, ($C_3$-$C_5$-cycloalkyl)-($C_1$-$C_2$-alkyl)-, $C_3$-$C_5$-cycloalkyl, phenyl and pyridinyl,

wherein $C_3$-$C_5$-cycloalkyl, and the $C_3$-$C_5$-cycloalkyl within said ($C_3$-$C_5$-cycloalkyl)-($C_1$-$C_2$-alkyl)-group are optionally substituted one or two times, each substituent independently selected from a fluorine atom and a methyl group,

and wherein the phenyl and pyridinyl groups are optionally substituted one or two times, each substituent independently selected from a fluorine atom and a chlorine atom, or a group selected from methyl, trifluoromethyl and methoxy;

$R^{20}$ represents a group selected from benzyl and phenyl,
wherein said phenyl group, and the phenyl group within said benzyl group, is optionally substituted one or two times, each substituent independently selected from a fluorine atom, a chlorine atom and a methyl group, and

$R^{21}$ represents a hydrogen atom or a methyl group,

and stereoisomers, tautomers, N-oxides, hydrates, solvates, and salts thereof, and mixtures of same.

In accordance with a fifth embodiment of the first aspect, the present invention covers compounds of general formula (I), *supra,* in which:

$R^1$ represents a group

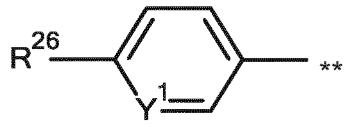

,

wherein "**" indicates the point of attachment to the nitrogen atom to which R' is attached;

$R^2$ represents a group

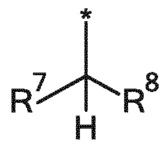

,

wherein "*" indicates the point of attachment to the nitrogen atom to which $R^2$ is attached;

$R^3$ represents a group selected from methyl and $-NH_2$;

$R^4$ represents a group

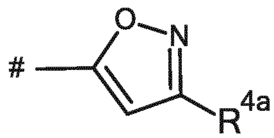

,

wherein "#" indicates the point of attachment to the carbonyl group to which $R^4$ is attached, and wherein

$R^{4a}$ represents a chlorine atom or a bromine atom, or a group selected from $((R^9)O)$-($C_1$-alkyl)-, $((R^{10})(R^{11})N)$-($C_1$-alkyl)-, ($C_3$-$C_5$-cycloalkyl)-($C_1$-alkyl)-, $C_3$-$C_5$-cycloalkyl, $-OR^9$, - C(=O)-N($R^{15}$)($R^{16}$) and phenyl,
wherein the phenyl group is optionally substituted one or two times, each substituent independently selected from a fluorine atom and a chlorine atom and a methyl group,
or

$R^4$ represents a group

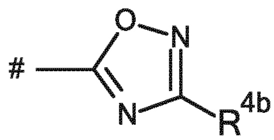

,

wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl and phenyl,

wherein the phenyl group is optionally substituted one or two times, each substituent independently selected from a fluorine atom and a chlorine atom, or a group selected from cyano, $C_1$-$C_2$-alkyl and -$OR^9$;

$R^7$ represents a hydrogen atom or a $C_1$-$C_2$-alkyl group;

$R^8$ represents a -C(=O)-$NH_2$ group;

$R^9$ represents a hydrogen atom or a group selected from $C_1$-$C_2$-alkyl, benzyl, $C_1$-$C_2$-fluoroalkyl, ($C_1$-$C_2$-alkoxy)-$C_2$-alkyl-, (($C_1$-$C_2$-alkyl)-C(=O)-O)-$C_2$-alkyl- and phenyl,
wherein the phenyl group within said benzyl group and said phenyl group itself are optionally substituted one or two times, each substituent independently selected from a fluorine atom and a chlorine atom, or a group selected from cyano and methyl;

$R^{10}$ and $R^{11}$ represent, independently from each occurrence, a hydrogen atom or a group selected from $C_1$-$C_2$-alkyl, $C_3$-$C_7$-cycloalkyl and (benzyl)-O-C(=O)-,

wherein $C_3$-$C_7$-cycloalkyl is optionally substituted one or two times, each substituent independently selected from a fluorine atom or a group selected from methyl and trifluoromethyl,

and wherein the phenyl group within said (benzyl)-O-C(=O)- group is optionally substituted one or two times, each substituent independently selected from a fluorine atom, a chlorine atom and a methyl group,

or

$R^{10}$ and $R^{11}$, together with the nitrogen atom to which they are attached, represent a monocyclic nitrogen containing 4- to 7-membered heterocycloalkyl group which is optionally substituted one, two or three times, each substituent independently selected from a fluorine atom or a group selected from cyano, methyl and trifluoromethyl;

$R^{15}$ and $R^{16}$ represent, independently from each occurrence, a hydrogen atom or a group selected from $C_1$-$C_4$-alkyl, $C_1$-$C_2$-fluoroalkyl, ($C_1$-$C_4$-alkoxy)-$C_2$-alkyl-, ($C_1$-$C_2$-fluoroalkoxy)-$C_2$-alkyl-, $C_3$-$C_5$-cycloalkyl, phenyl and pyridinyl,
wherein $C_3$-$C_5$-cycloalkyl is optionally substituted one or two times, each substituent independently selected from a fluorine atom and a methyl group;

$Y^1$ represents -C(H)=, -C(F)=, -C(Cl)=, -C(CN)= or -N=, and

$R^{26}$ represents a fluorine atom, a chlorine atom or a bromine atom, or a group selected from methyl, difluoromethyl, trifluoromethyl, methoxy, benzyloxy, difluoromethoxy and trifluoromethoxy,
and stereoisomers, tautomers, N-oxides, hydrates, solvates, and salts thereof, and mixtures of same.

In accordance with a sixth embodiment of the first aspect, the present invention covers compounds of general formula (I), *supra,* in which:

$R^1$ represents a group

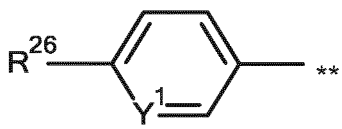

,

wherein "**" indicates the point of attachment to the nitrogen atom to which $R^1$ is attached;

$R^2$ represents a group

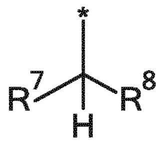

,

wherein "*" indicates the point of attachment to the nitrogen atom to which $R^2$ is attached;

$R^3$ represents a group selected from methyl and $-NH_2$;

$R^4$ represents a group

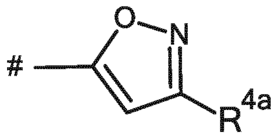

,

wherein "#" indicates the point of attachment to the carbonyl group to which $R^4$ is attached, and wherein

$R^{4a}$ represents a chlorine atom or a bromine atom, or a group selected from $((R^9)O)$-($C_1$-alkyl)-, $C_3$-$C_5$-cycloalkyl, $-OR^9$ and $-C(=O)-N(R^{15})(R^{16})$, or

$R^4$ represents a group

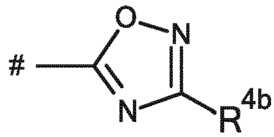

,

wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl and phenyl, wherein the phenyl group is optionally substituted one or two times, each substituent independently selected from a fluorine atom and a chlorine atom and a methyl group;

$R^7$ represents a hydrogen atom or a $C_1$-$C_2$-alkyl group;

$R^8$ represents a $-C(=O)-NH_2$ group;

$R^9$ represents a hydrogen atom or a group selected from $C_1$-$C_2$-alkyl, benzyl, $C_1$-$C_2$-fluoroalkyl and phenyl,
wherein the phenyl group within said benzyl group and said phenyl group itself are optionally substituted one or two times, each substituent independently selected from a fluorine atom and a chlorine atom, or a group selected from cyano and methyl;

$R^{15}$ and $R^{16}$ represent, independently from each occurrence, a hydrogen atom or a group selected from $C_1$-$C_2$-alkyl, $C_3$-$C_5$-cycloalkyl, phenyl and pyridinyl,
wherein $C_3$-$C_5$-cycloalkyl is optionally substituted one or two times, each substituent independently selected from a fluorine atom and a methyl group;

$Y^1$ represents -C(H)=, -C(F)=, -C(Cl)= or -N=, and

$R^{26}$ represents a fluorine atom, a chlorine atom or a bromine atom, or a group selected from difluoromethyl, methoxy, benzyloxy, difluoromethoxy and trifluoromethoxy,

and stereoisomers, tautomers, N-oxides, hydrates, solvates, and salts thereof, and mixtures of same.

In accordance with a seventh embodiment of the first aspect, the present invention covers compounds of general formula (I), *supra,* in which:

$R^1$ represents a group

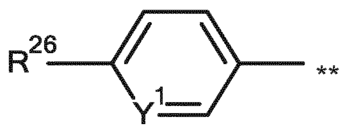

,

wherein "**" indicates the point of attachment to the nitrogen atom to which $R^1$ is attached;

$R^2$ represents a group

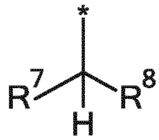

,

wherein "*" indicates the point of attachment to the nitrogen atom to which $R^2$ is attached;

$R^3$ represents a group $-NH_2$;

$R^4$ represents a group

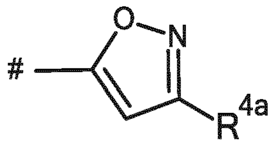

,

wherein "#" indicates the point of attachment to the carbonyl group to which $R^4$ is attached, and wherein

$R^{4a}$ represents a chlorine atom or a bromine atom, or a group selected from $((R^9)O)-(C_1\text{-alkyl})-$, $C_3$-$C_5$-cycloalkyl, $-OR^9$ and $-C(=O)-N(R^{15})(R^{16})$, or

$R^4$ represents a group

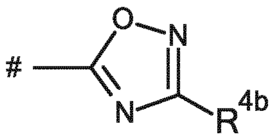

,

wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl and phenyl, wherein the phenyl group is optionally substituted one or two times, each substituent independently selected from a fluorine atom and a chlorine atom and a methyl group;

$R^7$ represents a methyl group;

$R^8$ represents a $-C(=O)-NH_2$ group;

$R^9$ represents a hydrogen atom or a group selected from $C_1$-$C_2$-alkyl, benzyl, $C_1$-$C_2$-fluoroalkyl and phenyl, wherein the phenyl group within said benzyl group and said phenyl group itself are optionally substituted one or two times, each substituent independently selected from a fluorine atom and a chlorine atom, or a group selected from cyano and methyl;

$R^{15}$ and $R^{16}$ represent, independently from each occurrence, a hydrogen atom or a group selected from $C_1$-$C_2$-alkyl, $C_3$-$C_5$-cycloalkyl, phenyl and pyridinyl, wherein $C_3$-$C_5$-cycloalkyl is optionally substituted one or two times, each substituent independently selected from a fluorine atom and a methyl group;

$Y^1$ represents -C(H)=, -C(F)=, -C(Cl)= or -N=, and

$R^{26}$ represents a fluorine atom, a chlorine atom or a bromine atom, or a group selected from difluoromethyl, methoxy, benzyloxy, difluoromethoxy and trifluoromethoxy,

and stereoisomers, tautomers, N-oxides, hydrates, solvates, and salts thereof, and mixtures of same.

In accordance with an eigth embodiment of the first aspect, the present invention covers compounds of general formula (I), *supra,* in which:
in which:

$R^1$ represents a phenyl or 6-membered heteroaryl group optionally substituted, one, two, or three times, each substituent independently selected from a halogen atom or a group selected from cyano, nitro, $C_1$-$C_6$-alkyl, (phenyl)-($C_1$-$C_3$-alkyl)-, $C_1$-$C_6$-haloalkyl, $C_1$-$C_6$-alkoxy, (phenyl)-($C_1$-$C_3$-alkoxy)-, $C_1$-$C_6$-haloalkoxy, -N($R^5$)($R^6$),

wherein the phenyl groups in said (phenyl)-($C_1$-$C_3$-alkyl)- and (phenyl)-($C_1$-$C_3$-alkoxy)-groups are optionally substituted one or two times, each substituent independently selected from a halogen atom or a group selected from cyano, methyl, ethyl, trifluoromethyl, methoxy, ethoxy, dimethylamino and trifluoromethoxy,

or two substituents attached to adjacent carbon atoms of said phenyl or 6-membered heteroaryl group together form a bivalent group selected from $-(CH_2)_3-$, $-(CH_2)_4-$, - $(CH_2)_2$-O-, $-(CH_2)_3$-O-, $-CH_2$-O-$CH_2$-, $-(CH_2)_2$-O-$CH_2$-, -O-$CH_2$-O-, -O-$CH_2$-$CH_2$-O-, -O-$CF_2$-O-, -O-$CH_2$-$CF_2$-O-, and -O-$CF_2$-$CF_2$-O-,

or

$R^1$ represents a 5-membered heteroaryl group optionally substituted one or two times, each substituent independently selected from a halogen atom or a group selected from cyano, $C_1$-$C_3$-alkyl, and $C_1$-$C_3$-alkoxy;

$R^2$ represents a group

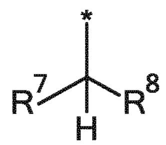

,

wherein "*" indicates the point of attachment to the nitrogen atom to which $R^2$ is attached;

$R^3$ represents a group selected from methyl and $-NH_2$;

$R^4$ represents a 5-membered heteroaryl group optionally substituted one or two times, each substituent independently selected from a halogen atom or a group selected from $C_1$-$C_3$-alkyl, -N($R^{10}$)($R^{11}$), -C(=O)-N($R^{15}$)($R^{16}$), -C(=O)-O$R^{17}$, phenyl and a 5- or 6-membered heteroaryl group,
wherein the phenyl group and the 5- or 6-membered heteroaryl group are optionally substituted one or two times, each substituent independently selected from a halogen atom or a group selected from cyano, $C_1$-$C_3$-alkyl, $C_1$-$C_3$-haloalkyl, (($R^{22}$)($R^{23}$)N)-$C_1$-$C_3$-alkyl, -O$R^9$, -N($R^{10}$)($R^{11}$) and -C(=O)-N($R^{15}$)($R^{16}$);

$R^5$ and $R^6$ represent, independently from each occurrence, a hydrogen atom or a group selected from $C_1$-$C_4$-alkyl, ($C_1$-$C_4$-alkyl)-C(=O)-, $C_3$-$C_4$-cycloalkyl and (phenyl)-($C_1$-$C_3$-alkyl)-, or

$R^5$ and $R^6$, together with the nitrogen atom to which they are attached, represent a monocyclic nitrogen containing 4- to 7-membered heterocycloalkyl group which is optionally substituted one, two or three times, each substituent independently selected from a halogen atom or a group selected from oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy;

$R^7$ represents a hydrogen atom or a $C_1$-$C_2$-alkyl group;

$R^8$ represents a group selected from -C(=O)-$NH_2$ and -S(=O)$_2$-$NH_2$;

$R^9$ represents a hydrogen atom or a group selected from $C_1$-$C_6$-alkyl, (phenyl)-($C_1$-$C_3$-alkyl)-, $C_1$-$C_6$-haloalkyl, $C_2$-$C_4$-hydroxyalkyl, ($C_1$-$C_3$-alkoxy)-$C_2$-$C_3$-alkyl-, (($C_1$-$C_3$-alkyl)-C(=O)-O)-$C_2$-$C_3$-alkyl-, -C($R^{18}$)($R^{19}$)-C(=O)-O$R^{17}$, -C($R^{18}$)($R^{19}$)-C(=O)-N($R^{20}$)($R^{21}$) and phenyl,
wherein the phenyl group within said (phenyl)-($C_1$-$C_3$-alkyl)- group and said phenyl group itself are optionally substituted one or two times, each substituent independently selected from a halogen atom or a group selected from cyano, methyl, ethyl, trifluoromethyl, methoxy, ethoxy, dimethylamino and trifluoromethoxy;

$R^{10}$ and $R^{11}$ represent, independently from each occurrence, a hydrogen atom or a group selected from $C_1$-$C_4$-alkyl, $C_1$-$C_4$-haloalkyl, $C_2$-$C_4$-hydroxyalkyl, ($C_1$-$C_3$-alkoxy)-$C_2$-$C_3$-alkyl-, (($R^{22}$)($R^{23}$)N)-$C_2$-$C_3$-alkyl, ($C_1$-$C_4$-alkyl)-C(=O)-, $C_3$-$C_7$-cycloalkyl, ($C_3$-$C_7$-cycloalkyl)-C(=O)-, (phenyl)-($C_1$-$C_3$-alkyl)-, (phenyl)-($C_1$-$C_3$-alkyl)-C(=O)- and (phenyl)-($C_1$-$C_3$-alkyl)-O-C(=O)-,
wherein the phenyl groups within said (phenyl)-($C_1$-$C_3$-alkyl)-, (phenyl)-($C_1$-$C_3$-alkyl)-C(=O)- and (phenyl)-($C_1$-$C_3$-alkyl)-O-C(=O)- groups are optionally substituted one or two times, each substituent independently selected from a halogen atom or a group selected from cyano, methyl, ethyl, trifluoromethyl, methoxy, ethoxy, dimethylamino and trifluoromethoxy,
or

$R^{10}$ and $R^{11}$, together with the nitrogen atom to which they are attached, represent a monocyclic nitrogen containing 4- to 7-membered heterocycloalkyl group, or a bicyclic nitrogen containing 6- to 11-membered heterocycloalkyl group, which are optionally substituted one, two or three times, each substituent independently selected from a halogen atom or a group selected from cyano, oxo, hydroxy, $C_1$-$C_4$-alkyl, $C_1$-$C_4$-haloalkyl, ($C_1$-$C_4$-alkyl)-C(=O)-, $C_3$-$C_7$-cycloalkyl, $C_1$-$C_4$-alkoxy, -N($R^{22}$)($R^{23}$), and a monocyclic 4- to 7-membered heterocycloalkyl group;

$R^{15}$ and $R^{16}$ represent, independently from each occurrence, a hydrogen atom or a group selected from $C_1$-$C_4$-alkyl, $C_1$-$C_4$-haloalkyl, $C_1$-$C_4$-hydroxyalkyl, ($C_1$-$C_3$-alkoxy)-$C_2$-$C_3$-alkyl-, $C_3$-$C_7$-cycloalkyl, monocyclic 4- to 7-membered heterocycloalkyl and (phenyl)-($C_1$-$C_3$-alkyl)-,
wherein $C_3$-$C_7$-cycloalkyl and monocyclic 4- to 7-membered heterocycloalkyl are optionally substituted one, two or three times, each substituent independently selected from a halogen atom or a group selected from oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy, or

$R^{15}$ and $R^{16}$, together with the nitrogen atom to which they are attached, represent a monocyclic nitrogen containing 4- to 7-membered heterocycloalkyl group which is optionally substituted one, two or three times, each substituent independently selected from a halogen atom or a group selected from oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy;

$R^{17}$ represents a $C_1$-$C_4$-alkyl group;

$R^{18}$ and $R^{19}$ represent, independently from each occurrence, a hydrogen atom or a $C_1$-$C_4$-alkyl group;

$R^{20}$ represents a hydrogen atom or a group selected from $C_1$-$C_6$-alkyl, $C_3$-$C_4$-alkenyl, $C_3$-$C_4$-alkynyl, $C_1$-$C_3$-alkoxy, $C_3$-$C_7$-cycloalkyl, bicyclic $C_6$-$C_{11}$-cycloalkyl, adamantyl, monocyclic 4- to 7-membered heterocycloalkyl, bicyclic 6- to 11-membered heterocycloalkyl, phenyl, naphthyl, and 5- to 10-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, bicyclic $C_6$-$C_{11}$-cycloalkyl, adamantyl, monocyclic 4- to 7-membered heterocycloalkyl, bicyclic 6- to 11-membered heterocycloalkyl, phenyl, and 5- to 10-membered heteroaryl, said phenyl and 5- to 10-membered heteroaryl substituents themselves being optionally substituted one or two times, each substituent independently selected from a halogen atom or a group selected from cyano, methyl, ethyl, trifluoromethyl, methoxy, ethoxy, dimethylamino and trifluoromethoxy,

wherein $C_3$-$C_7$-cycloalkyl, bicyclic $C_6$-$C_{11}$-cycloalkyl, adamantyl, monocyclic 4- to 7-membered heterocycloalkyl and bicyclic 6- to 11-membered heterocycloalkyl are optionally substituted one, two or three times, each substituent independently selected from a halogen atom or a group selected from cyano, oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy,

and wherein said phenyl, naphthyl and 5- to 10-membered heteroaryl groups are optionally substituted one, two or three times, each substituent independently selected from a halogen atom or a group selected from cyano, $C_1$-$C_4$-alkyl, $C_1$-$C_4$-haloalkyl, $C_1$-$C_4$-alkoxy, $C_1$-$C_4$-haloalkoxy, -N($R^{22}$)($R^{23}$) and -C(=O)-N($R^{24}$)($R^{25}$),

$R^{21}$ represents a hydrogen atom or a $C_1$-$C_4$-alkyl group, or

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_4$-alkyl, $C_1$-$C_4$-haloalkyl, (phenyl)-($C_1$-$C_3$-alkyl)-, ($C_1$-$C_4$-alkyl)-C(=O)-, $C_3$-$C_4$-cycloalkyl, $C_1$-$C_4$-alkoxy, $C_1$-$C_3$-haloalkoxy, -N($R^{22}$)($R^{23}$) and -C(=O)-N($R^{24}$)($R^{25}$);

$R^{22}$ and $R^{23}$ represent, independently from each occurrence, a hydrogen atom or a group selected from $C_1$-$C_2$-alkyl and ($C_1$-$C_2$-alkyl)-C(=O)-, and

$R^{24}$ and $R^{25}$ represent, independently from each occurrence, a hydrogen atom or a $C_1$-$C_4$-alkyl group,

and stereoisomers, tautomers, N-oxides, hydrates, solvates, and salts thereof, and mixtures of same.

In accordance with a ninth embodiment of the first aspect, the present invention covers compounds of general formula (I), *supra,* in which:

$R^1$ represents a phenyl or pyridinyl group optionally substituted, one, two, or three times, each substituent independently selected from a halogen atom or a group selected from cyano, nitro, $C_1$-$C_4$-alkyl, (phenyl)-($C_1$-$C_2$-alkyl)-, $C_1$-$C_4$-haloalkyl, $C_1$-$C_4$-alkoxy, (phenyl)-($C_1$-$C_2$-alkoxy)-, $C_1$-$C_4$-haloalkoxy, -N($R^5$)($R^6$),

wherein the phenyl groups in said (phenyl)-($C_1$-$C_2$-alkyl)- and (phenyl)-($C_1$-$C_2$-alkoxy)-groups are optionally substituted one or two times, each substituent independently selected from a fluorine atom, a chlorine atom and a bromine atom, or a group selected from methyl, trifluoromethyl and methoxy,

or two substituents attached to adjacent carbon atoms of said phenyl or pyridinyl group together form a bivalent group selected from $-(CH_2)_3-$, $-(CH_2)_4-$, $-(CH_2)_2-O-$, $-(CH_2)_3-O-$, $-CH_2-O-CH_2-$, $-O-CH_2-O-$, $-O-CH_2-CH_2-O-$ and $-O-CF_2-O-$,

or

$R^1$ represents a pyrazolyl group optionally substituted one or two times, each substituent independently selected from a halogen atom or a group selected from cyano, $C_1$-$C_2$-alkyl, and $C_1$-$C_2$-alkoxy;

$R^2$ represents a group

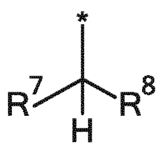

,

wherein "*" indicates the point of attachment to the nitrogen atom to which $R^2$ is attached;

$R^3$ represents a group selected from methyl and $-NH_2$;

$R^4$ represents a 5-membered heteroaryl group selected from oxazolyl, isoxazolyl, thiazolyl and isothiazolyl, optionally substituted one or two times, each substituent independently selected from a fluorine atom, a chlorine atom and a bromine atom, or a group selected from $C_1$-$C_3$-alkyl, $-N(R^{10})(R^{11})$, $-C(=O)-N(R^{15})(R^{16})$, $-C(=O)-OR^{17}$, phenyl and a 5- or 6-membered heteroaryl group,
wherein the phenyl group and the 5- or 6-membered heteroaryl group are optionally substituted one or two times, each substituent independently selected from a fluorine atom, a chlorine atom and a bromine atom, or a group selected from cyano, $C_1$-$C_2$-alkyl, $C_1$-$C_2$-fluoroalkyl, $-OR^9$, $-N(R^{10})(R^{11})$ and $-C(=O)-N(R^{15})(R^{16})$;

$R^5$ and $R^6$ represent, independently from each occurrence, a hydrogen atom or a group selected from $C_1$-$C_2$-alkyl and ($C_1$-$C_2$-alkyl)-C(=O)-,
or

$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom, a chlorine atom and a bromine atom, or a group selected from oxo, hydroxy, $C_1$-$C_2$-alkyl and ($C_1$-$C_2$-alkyl)-C(=O)-;

$R^7$ represents a hydrogen atom or a $C_1$-$C_2$-alkyl group;

$R^8$ represents a $-C(=O)-NH_2$ group;

$R^9$ represents a hydrogen atom or a group selected from $C_1$-$C_4$-alkyl, (phenyl)-($C_1$-$C_2$-alkyl)-, $C_1$-$C_4$-haloalkyl, $C_2$-$C_3$-hydroxyalkyl, ($C_1$-$C_2$-alkoxy)-$C_2$-alkyl-, (($C_1$-$C_2$-alkyl)-C(=O)-O)-$C_2$-alkyl-, $-C(R^{18})(R^{19})-C(=O)-OR^{17}$, $-C(R^{18})(R^{19})-C(=O)-N(R^{20})(R^{21})$ and phenyl,
wherein the phenyl group within said (phenyl)-($C_1$-$C_2$-alkyl)- group and said phenyl group itself are optionally substituted one or two times, each substituent independently selected from a fluorine atom, a chlorine atom and a bromine atom, or a group selected from methyl, trifluoromethyl and methoxy;

$R^{10}$ and $R^{11}$ represent, independently from each occurrence, a hydrogen atom or a group selected from $C_1$-$C_2$-alkyl, $C_1$-$C_2$-haloalkyl, $C_2$-$C_3$-hydroxyalkyl, ($C_1$-$C_2$-alkoxy)-$C_2$-alkyl-, (($R^{22}$)($R^{23}$)N)-$C_2$-alkyl, ($C_1$-$C_2$-alkyl)-C(=O)-, $C_3$-$C_5$-cycloalkyl, ($C_3$-$C_5$-cycloalkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,
wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a bromine atom, or a group selected from methyl, trifluoromethyl and methoxy,
or

$R^{10}$ and $R^{11}$, together with the nitrogen atom to which they are attached, represent a monocyclic nitrogen containing 4- to 7-membered heterocycloalkyl group, or a bicyclic nitrogen containing 6- to 10-membered heterocycloalkyl group, which are optionally substituted one, two or three times, each substituent independently selected from a halogen atom or a group selected from cyano, oxo, hydroxy, $C_1$-$C_2$-alkyl, $C_1$-$C_2$-haloalkyl, ($C_1$-$C_2$-alkyl)-C(=O)-, $C_1$-$C_2$-alkoxy, $-N(R^{22})(R^{23})$, and a monocyclic 4- to 7-membered heterocycloalkyl group;

$R^{15}$ and $R^{16}$ represent, independently from each occurrence, a hydrogen atom or a group selected from $C_1$-$C_4$-alkyl, $C_1$-$C_4$-haloalkyl, $C_1$-$C_4$-hydroxyalkyl, ($C_1$-$C_2$-alkoxy)-$C_2$-$C_3$-alkyl-, $C_3$-$C_7$-cycloalkyl, monocyclic 4- to 7-membered heterocycloalkyl and (phenyl)-($C_1$-$C_3$-alkyl)-,
wherein $C_3$-$C_7$-cycloalkyl and monocyclic 4- to 7-membered heterocycloalkyl are optionally substitueted one or two times, each substituent independently selected from a halogen atom or a group selected from oxo, $C_1$-$C_2$-alkyl and ($C_1$-$C_2$-alkyl)-C(=O)-, or

$R^{15}$ and $R^{16}$, together with the nitrogen atom to which they are attached, represent a monocyclic nitrogen containing 4- to 7-membered heterocycloalkyl group which is optionally substituted one or two times, each substituent independently selected from a halogen atom or a group selected from oxo, $C_1$-$C_2$-alkyl

and ($C_1$-$C_2$-alkyl)-C(=O)-;

$R^{17}$ represents a $C_1$-$C_4$-alkyl group;

$R^{18}$ and $R^{19}$ represent, independently from each occurrence, a hydrogen atom or a $C_1$-$C_2$-alkyl group;

$R^{20}$ represents a hydrogen atom or a group selected from $C_1$-$C_6$-alkyl, $C_3$-$C_4$-alkenyl, $C_3$-$C_4$-alkynyl, $C_1$-$C_3$-alkoxy, $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, naphthyl, and 5- to 10-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a bromine atom, or a group selected from methyl, trifluoromethyl and methoxy,

wherein $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl are optionally substituted one or two or three times, each substituent independently selected from a halogen atom or a group selected from cyano, oxo, hydroxy, $C_1$-$C_2$-alkyl and ($C_1$-$C_2$-alkyl)-C(=O)-,

and wherein said phenyl, naphthyl and 5- to 10-membered heteroaryl groups are optionally substituted one, two or three times, each substituent independently selected from a halogen atom or a group selected from cyano, $C_1$-$C_2$-alkyl, $C_1$-$C_2$-haloalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group,
or

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-haloalkyl, (phenyl)-($C_1$-$C_2$-alkyl)-, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$);

$R^{22}$ and $R^{23}$ represent, independently from each occurrence, a hydrogen atom or a group selected from $C_1$-$C_2$-alkyl and ($C_1$-$C_2$-alkyl)-C(=O)-, and

$R^{24}$ and $R^{25}$ represent, independently from each occurrence, a hydrogen atom or a $C_1$-$C_2$-alkyl group,

and stereoisomers, tautomers, N-oxides, hydrates, solvates, and salts thereof, and mixtures of same.

In accordance with a tenth embodiment of the first aspect, the present invention covers compounds of general formula (I), *supra,* in which:

$R^1$ represents a phenyl or pyridinyl group optionally substituted, one, two, or three times, each substituent independently selected from a fluorine atom, a chlorine atom and a bromine atom, or a group selected from cyano, $C_1$-$C_4$-alkyl, $C_1$-$C_2$-fluoroalkyl, $C_1$-$C_2$-alkoxy, $C_1$-$C_2$-fluoroalkoxy, -N($R^5$)($R^6$), or two substituents attached to adjacent carbon atoms of said phenyl or pyridinyl group together form a bivalent group selected from $-(CH_2)_3-$, $-O-CH_2-O-$ and $-O-CF_2-O-$, or

$R^1$ represents a pyrazolyl group optionally substituted with one methyl group,

$R^2$ represents a group

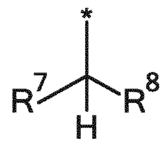

,

wherein "*" indicates the point of attachment to the nitrogen atom to which $R^2$ is attached;

$R^3$ represents a group selected from methyl and $-NH_2$;

$R^4$ represents a group

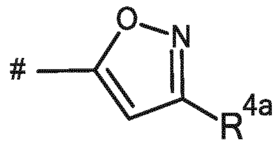

,

wherein "#" indicates the point of attachment to the carbonyl group to which $R^4$ is attached, and wherein

$R^{4a}$ represents a group selected from $-N(R^{10})(R^{11})$, $-C(=O)-N(R^{15})(R^{16})$, $-C(=O)-OR^{17}$, phenyl and a 5- or 6-membered heteroaryl group selected from oxazolyl, isoxazolyl, thiazolyl, isothiazolyl, pyrazolyl, pyridinyl, pyrazinyl, pyrimidinyl and pyridazinyl,
wherein the phenyl group and the 5- or 6-membered heteroaryl group selected from oxazolyl, isoxazolyl, thiazolyl, isothiazolyl, pyrazolyl, pyridinyl, pyrazinyl, pyrimidinyl and pyridazinyl are optionally substituted one or two times, each substituent independently selected from a fluorine atom and a chlorine atom, or a group selected from cyano, $C_1$-$C_2$-alkyl, $-OR^9$, $-N(R^{10})(R^{11})$ and $-C(=O)-N(R^{15})(R^{16})$;

$R^5$ and $R^6$ represent, independently from each occurrence, a hydrogen atom or a $C_1$-$C_2$-alkyl group, or

$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom or a group selected from hydroxy and $C_1$-$C_2$-alkyl;

$R^7$ represents a hydrogen atom or a $C_1$-$C_2$-alkyl group;

$R^8$ represents a $-C(=O)-NH_2$ group;

$R^9$ represents a hydrogen atom or a group selected from $C_1$-$C_2$-alkyl, benzyl, $C_1$-$C_2$-fluoroalkyl, ($C_1$-$C_2$-alkoxy)-$C_2$-alkyl-, (($C_1$-$C_2$-alkyl)-C(=O)-O)-$C_2$-alkyl-, $-C(R^{18})(R^{19})-C(=O)-OR^{17}$, $-C(R^{18})(R^{19})-C(=O)-N(R^{20})(R^{21})$ and phenyl, wherein the phenyl group within said benzyl group and said phenyl group itself are optionally substituted one or two times, each substituent independently selected from a fluorine atom, a chlorine atom and a methyl group;

$R^{10}$ and $R^{11}$ represent, independently from each occurrence, a hydrogen atom or a group selected from $C_1$-$C_2$-alkyl, $C_1$-$C_2$-fluoroalkyl, ($C_1$-$C_2$-alkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-, wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a methyl group, or

$R^{10}$ and $R^{11}$, together with the nitrogen atom to which they are attached, represent a monocyclic nitrogen containing 4- to 7-membered heterocycloalkyl group which is optionally substituted one or two times, each substituent independently selected from a fluorine atom or a group selected from oxo, $C_1$-$C_2$-alkyl,

$C_1$-$C_2$-fluoroalkyl and ($C_1$-$C_2$-alkyl)-C(=O)-;

$R^{15}$ and $R^{16}$ represent, independently from each occurrence, a hydrogen atom or a group selected from $C_1$-$C_3$-alkyl, $C_1$-$C_3$-fluoroalkyl and $C_3$-$C_5$-cycloalkyl, or

$R^{15}$ and $R^{16}$, together with the nitrogen atom to which they are attached, represent a monocyclic nitrogen containing 4- to 7-membered heterocycloalkyl group which is optionally substituted once with a fluorine atom or a group selected from oxo, $C_1$-$C_2$-alkyl and $CH_3$-C(=O)-;

$R^{17}$ represents a $C_1$-$C_2$-alkyl group;

$R^{18}$ and $R^{19}$ represent, independently from each occurrence, a hydrogen atom or a methyl group;

$R^{20}$ represents a hydrogen atom or a group selected from optionally substituted $C_1$-$C_3$-alkyl, unsubstituted $C_4$-$C_6$-alkyl, prop-2-ynyl, methoxy, $C_3$-$C_6$-cycloalkyl, adamantyl, monocyclic 4- to 7-membered heterocycloalkyl, phenyl, and 5- to 10-membered heteroaryl, wherein said $C_1$-$C_3$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_6$-cycloalkyl, adamantyl, monocyclic 4- to 7-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a methyl group, wherein said $C_3$-$C_6$-cycloalkyl, adamantyl and monocyclic 4- to 7-membered heterocycloalkyl groups are optionally substituted one or two or three times, each substituent independently selected from a fluorine atom or a group selected from oxo, $C_1$-$C_2$-alkyl and ($C_1$-$C_2$-alkyl)-C(=O)-, and wherein said phenyl and 5- to 10-membered heteroaryl groups are optionally substituted one, two or three times, each substituent independently selected from a fluorine atom and a chlorine atom or a group selected from cyano, $C_1$-$C_2$-alkyl, $C_1$-$C_2$-fluoroalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group, or

$R^{26}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-fluoroalkyl, benzyl, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$);

$R^{22}$ and $R^{23}$ represent, independently from each occurrence, a hydrogen atom or a group selected from $C_1$-$C_2$-alkyl and ($C_1$-$C_2$-alkyl)-C(=O)-;

$R^{24}$ and $R^{25}$ represent, independently from each occurrence, a hydrogen atom or a $C_1$-$C_2$-alkyl group, and

and stereoisomers, tautomers, N-oxides, hydrates, solvates, and salts thereof, and mixtures of same.

## FURTHER EMBODIMENTS OF THE FIRST ASPECT OF THE PRESENT INVENTION

In a further embodiment of the first aspect, the present invention covers compounds of formula (I), *supra,* in which:

$R^1$ represents a phenyl or pyridinyl group optionally substituted, one, two, or three times, each substituent independently selected from a halogen atom or a group selected from hydroxy, cyano, nitro, $C_1$-$C_4$-alkyl, (phenyl)-($C_1$-$C_2$-alkyl)-, $C_1$-$C_4$-haloalkyl, $C_1$-$C_4$-alkoxy, (phenyl)-($C_1$-$C_2$-alkoxy)-, $C_1$-$C_4$-haloalkoxy, -N($R^5$)($R^6$), wherein the phenyl groups in said (phenyl)-($C_1$-$C_2$-alkyl)- and (phenyl)-($C_1$-$C_2$-alkoxy)-groups are optionally substituted one or two times, each substituent independently selected from a fluorine atom, a chlorine atom and a bromine atom, or a group selected from methyl, trifluoromethyl and methoxy, or two substituents attached to adjacent carbon atoms of said phenyl or pyridinyl group together form a bivalent group selected from $-(CH_2)_3-$, $-(CH_2)_4-$, $-(CH_2)_2-O-$, $-(CH_2)_3-O-$, $-CH_2-O-CH_2-$, $-O-CH_2-O-$, $-O-CH_2-CH_2-O-$ and $-O-CF_2-O-$, or

$R^1$ represents a pyrazolyl group optionally substituted one or two times, each substituent independently selected

from a halogen atom or a group selected from cyano, $C_1$-$C_2$-alkyl, and $C_1$-$C_2$-alkoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl or pyridinyl group optionally substituted, one, two, or three times, each substituent independently selected from a halogen atom or a group selected from hydroxy, cyano, nitro, $C_1$-$C_4$-alkyl, (phenyl)-($C_1$-$C_2$-alkyl)-, $C_1$-$C_4$-haloalkyl, $C_1$-$C_4$-alkoxy, (phenyl)-($C_1$-$C_2$-alkoxy)-, $C_1$-$C_4$-haloalkoxy, -N($R^5$)($R^6$),

wherein the phenyl groups in said (phenyl)-($C_1$-$C_2$-alkyl)- and (phenyl)-($C_1$-$C_2$-alkoxy)-groups are optionally substituted one or two times, each substituent independently selected from a fluorine atom, a chlorine atom and a bromine atom, or a group selected from methyl, trifluoromethyl and methoxy,

or two substituents attached to adjacent carbon atoms of said phenyl or pyridinyl group together form a bivalent group selected from $-(CH_2)_3-$, $-(CH_2)_4-$, $-(CH_2)_2-O-$, $-(CH_2)_3-O-$, $-CH_2-O-CH_2-$, $-O-CH_2-O-$, $-O-CH_2-CH_2-O-$ and $-O-CF_2-O-$,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl or pyridinyl group optionally substituted, one, two, or three times, each substituent independently selected from a halogen atom or a group selected from cyano, nitro, $C_1$-$C_4$-alkyl, (phenyl)-($C_1$-$C_2$-alkyl)-, $C_1$-$C_4$-haloalkyl, $C_1$-$C_4$-alkoxy, (phenyl)-($C_1$-$C_2$-alkoxy)-, $C_1$-$C_4$-haloalkoxy, -N($R^5$)($R^6$), wherein the phenyl groups in said (phenyl)-($C_1$-$C_2$-alkyl)- and (phenyl)-($C_1$-$C_2$-alkoxy)-groups are optionally substituted one or two times, each substituent independently selected from a fluorine atom, a chlorine atom and a bromine atom, or a group selected from methyl, trifluoromethyl and methoxy, or two substituents attached to adjacent carbon atoms of said phenyl or pyridinyl group together form a bivalent group selected from $-(CH_2)_3-$, $-(CH_2)_4-$, $-(CH_2)_2-O-$, $-(CH_2)_3-O-$, $-CH_2-O-CH_2-$, $-O-CH_2-O-$, $-O-CH_2-CH_2-O-$ and $-O-CF_2-O-$, or

$R^1$ represents a pyrazolyl group optionally substituted one or two times, each substituent independently selected from a halogen atom or a group selected from cyano, $C_1$-$C_2$-alkyl, and $C_1$-$C_2$-alkoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl or pyridinyl group optionally substituted, one, two, or three times, each substituent independently selected from a halogen atom or a group selected from cyano, nitro, $C_1$-$C_4$-alkyl, (phenyl)-($C_1$-$C_2$-alkyl)-, $C_1$-$C_4$-haloalkyl, $C_1$-$C_4$-alkoxy, (phenyl)-($C_1$-$C_2$-alkoxy)-, $C_1$-$C_4$-haloalkoxy, -N($R^5$)($R^6$),

wherein the phenyl groups in said (phenyl)-($C_1$-$C_2$-alkyl)- and (phenyl)-($C_1$-$C_2$-alkoxy)-groups are optionally substituted one or two times, each substituent independently selected from a fluorine atom, a chlorine atom and a bromine atom, or a group selected from methyl, trifluoromethyl and methoxy,

or two substituents attached to adjacent carbon atoms of said phenyl or pyridinyl group together form a bivalent group selected from $-(CH_2)_3-$, $-(CH_2)_4-$, $-(CH_2)_2-O-$, $-(CH_2)_3-O-$, $-CH_2-O-CH_2-$, $-O-CH_2-O-$, $-O-CH_2-CH_2-O-$ and $-O-CF_2-O-$,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl or pyridinyl group optionally substituted, one, two, or three times, each substituent independently selected from a fluorine atom, a chlorine atom and a bromine atom, or a group selected from hydroxy, cyano, $C_1$-$C_4$-alkyl, $C_1$-$C_2$-fluoroalkyl, $C_1$-$C_2$-alkoxy, (phenyl)-($C_1$-$C_2$-alkoxy)-, $C_1$-$C_2$-fluoroalkoxy and -N($R^5$)($R^6$), or two substituents attached to adjacent carbon atoms of said phenyl or pyridinyl group together form a bivalent group selected from $-(CH_2)_3-$, $-O-CH_2-O-$ and $-O-CF_2-O-$, or

$R^1$ represents a pyrazolyl group optionally substituted with one methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl or pyridinyl group optionally substituted, one, two, or three times, each substituent independently selected from a fluorine atom, a chlorine atom and a bromine atom, or a group selected from hydroxy, cyano, $C_1$-$C_4$-alkyl, $C_1$-$C_2$-fluoroalkyl, $C_1$-$C_2$-alkoxy, (phenyl)-($C_1$-$C_2$-alkoxy)-, $C_1$-$C_2$-fluoroalkoxy and -N($R^5$)($R^6$), or two substituents attached to adjacent carbon atoms of said phenyl or pyridinyl group together form a bivalent group selected from $-(CH_2)_3-$, $-O-CH_2-O-$ and $-O-CF_2-O-$,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl or pyridinyl group optionally substituted, one, two, or three times, each substituent independently selected from a fluorine atom, a chlorine atom and a bromine atom, or a group selected from cyano, $C_1$-$C_4$-alkyl, $C_1$-$C_2$-fluoroalkyl, $C_1$-$C_2$-alkoxy, $C_1$-$C_2$-fluoroalkoxy, -N($R^5$)($R^6$),
or two substituents attached to adjacent carbon atoms of said phenyl or pyridinyl group together form a bivalent group selected from $-(CH_2)_3-$, $-O-CH_2-O-$ and $-O-CF_2-O-$,
or

$R^1$ represents a pyrazolyl group optionally substituted with one methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl or pyridinyl group optionally substituted, one, two, or three times, each substituent independently selected from a fluorine atom, a chlorine atom and a bromine atom, or a group selected from cyano, $C_1$-$C_4$-alkyl, $C_1$-$C_2$-fluoroalkyl, $C_1$-$C_2$-alkoxy, $C_1$-$C_2$-fluoroalkoxy, -N($R^5$)($R^6$),
or two substituents attached to adjacent carbon atoms of said phenyl or pyridinyl group together form a bivalent group selected from $-(CH_2)_3-$, $-O-CH_2-O-$ and $-O-CF_2-O-$,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl group optionally substituted, one, two, or three times, each substituent independently selected from a fluorine atom, a chlorine atom and a bromine atom, or a group selected from hydroxy, cyano, $C_1$-$C_4$-alkyl, $C_1$-$C_2$-fluoroalkyl, $C_1$-$C_2$-alkoxy, (phenyl)-($C_1$-$C_2$-alkoxy)-, $C_1$-$C_2$-fluoroalkoxy and -N($R^5$)($R^6$),
or two substituents attached to adjacent carbon atoms of said phenyl group together form a bivalent group selected from $-(CH_2)_3-$, $-O-CH_2-O-$ and $-O-CF_2-O-$,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a pyridinyl group optionally substituted, one, two, or three times, each substituent independently selected from a fluorine atom, a chlorine atom and a bromine atom, or a group selected from hydroxy, cyano, $C_1$-$C_4$-alkyl, $C_1$-$C_2$-fluoroalkyl, $C_1$-$C_2$-alkoxy, (phenyl)-($C_1$-$C_2$-alkoxy)-, $C_1$-$C_2$-fluoroalkoxy and -N($R^5$)($R^6$),
or two substituents attached to adjacent carbon atoms of said pyridinyl group together form a bivalent group selected from $-(CH_2)_3-$, $-O-CH_2-O-$ and $-O-CF_2-O-$,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl group optionally substituted, one, two, or three times, each substituent independently selected from a fluorine atom, a chlorine atom and a bromine atom, or a group selected from cyano, $C_1$-$C_4$-alkyl, $C_1$-$C_2$-fluoroalkyl, $C_1$-$C_2$-alkoxy, $C_1$-$C_2$-fluoroalkoxy, -N($R^5$)($R^6$),
or two substituents attached to adjacent carbon atoms of said phenyl or pyridinyl group together form a bivalent group selected from $-(CH_2)_3-$, $-O-CH_2-O-$ and $-O-CF_2-O-$,

and stereoisomers, tautomers, N-oxides, hydrates, solvates, and salts thereof, and mixtures of same.
In a further embodiment of the first aspect, the present invention covers compounds of formula (I), *supra,* in which:

$R^1$ represents a pyridinyl group optionally substituted, one, two, or three times, each substituent independently selected from a fluorine atom, a chlorine atom and a bromine atom, or a group selected from cyano, $C_1$-$C_4$-alkyl, $C_1$-$C_2$-fluoroalkyl, $C_1$-$C_2$-alkoxy, $C_1$-$C_2$-fluoroalkoxy, -N($R^5$)($R^6$),
or two substituents attached to adjacent carbon atoms of said phenyl or pyridinyl group together form a bivalent group selected from $-(CH_2)_3-$, $-O-CH_2-O-$ and $-O-CF_2-O-$,

and stereoisomers, tautomers, N-oxides, hydrates, solvates, and salts thereof, and mixtures of same.
In a further embodiment of the first aspect, the present invention covers compounds of formula (I), *supra,* in which:

$R^1$ represents a phenyl or pyridinyl group optionally substituted, one or two times, each substituent independently selected from a fluorine atom, a chlorine atom and a bromine atom, or a group selected from methyl, difluoromethyl, trifluoromethyl, methoxy, benzyloxy, difluoromethoxy and trifluoromethoxy,
or two substituents attached to adjacent carbon atoms of said phenyl or pyridinyl group together form a bivalent group $-O-CF_2-O-$,

and stereoisomers, tautomers, N-oxides, hydrates, solvates, and salts thereof, and mixtures of same.
In a further embodiment of the first aspect, the present invention covers compounds of formula (I), *supra,* in which:

$R^1$ represents a phenyl group optionally substituted, one or two times, each substituent independently selected from a fluorine atom, a chlorine atom and a bromine atom, or a group selected from methyl, difluoromethyl, trifluoromethyl, methoxy, benzyloxy, difluoromethoxy and trifluoromethoxy,
or two substituents attached to adjacent carbon atoms of said phenyl group together form a bivalent group $-O-CF_2-O-$,

and stereoisomers, tautomers, N-oxides, hydrates, solvates, and salts thereof, and mixtures of same.
In a further embodiment of the first aspect, the present invention covers compounds of formula (I), *supra,* in which:

$R^1$ represents a pyridinyl group optionally substituted, one or two times, each substituent independently selected from a fluorine atom, a chlorine atom and a bromine atom, or a group selected from methyl, difluoromethyl, trifluoromethyl, methoxy, benzyloxy, difluoromethoxy and trifluoromethoxy,
or two substituents attached to adjacent carbon atoms of said pyridinyl group together form a bivalent group $-O-CF_2-O-$,

and stereoisomers, tautomers, N-oxides, hydrates, solvates, and salts thereof, and mixtures of same.
In a further embodiment of the first aspect, the present invention covers compounds of formula (I), *supra,* in which:

$R^1$ represents a phenyl or pyridinyl group optionally substituted, one or two times, each substituent independently selected from a fluorine atom and a chlorine atom, or a group selected from $C_1$-$C_2$-alkyl, $C_1$-$C_2$-fluoroalkyl, $C_1$-$C_2$-alkoxy, $C_1$-$C_2$-fluoroalkoxy,

and stereoisomers, tautomers, N-oxides, hydrates, solvates, and salts thereof, and mixtures of same.
In a further embodiment of the first aspect, the present invention covers compounds of formula (I), *supra,* in which:

$R^1$ represents a phenyl group optionally substituted, one or two times, each substituent independently selected from a fluorine atom and a chlorine atom, or a group selected from cyano, $C_1$-$C_2$-alkyl, $C_1$-$C_2$-fluoroalkyl, $C_1$-$C_2$-alkoxy, $C_1$-$C_2$-fluoroalkoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a phenyl group optionally substituted, one or two times, each substituent independently selected from a fluorine atom and a chlorine atom, or a group selected from $C_1$-$C_2$-alkyl, $C_1$-$C_2$-fluoroalkyl, $C_1$-$C_2$-alkoxy, $C_1$-$C_2$-fluoroalkoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a pyridinyl group optionally substituted, one or two times, each substituent independently selected from a fluorine atom and a chlorine atom, or a group selected from $C_1$-$C_2$-alkyl, $C_1$-$C_2$-fluoroalkyl, $C_1$-$C_2$-alkoxy, $C_1$-$C_2$-fluoroalkoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a group

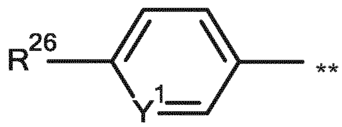

wherein "**" indicates the point of attachment to the nitrogen atom to which $R^1$ is attached, and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a group

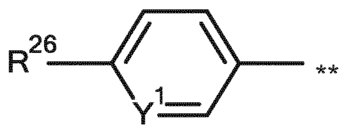

wherein "**" indicates the point of attachment to the nitrogen atom to which $R^1$ is attached;

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a group

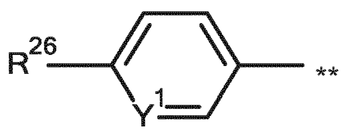

wherein "**" indicates the point of attachment to the nitrogen atom to which $R^1$ is attached;

$Y^1$ represents -C(H)=, -C(F)=, -C(Cl)=, -C(CN)= or -N=, and

$R^{26}$ represents a fluorine atom, a chlorine atom or a bromine atom, or a group selected from methyl, difluoromethyl, trifluoromethyl, methoxy, benzyloxy, difluoromethoxy and trifluoromethoxy,
and stereoisomers, tautomers, N-oxides, hydrates, solvates, and salts thereof, and mixtures of same.

In a further embodiment of the first aspect, the present invention covers compounds of formula *(I), supra,* in which:

$R^1$ represents a group

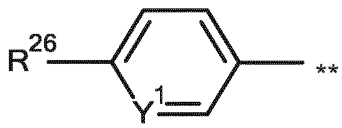

wherein "**" indicates the point of attachment to the nitrogen atom to which $R^1$ is attached;
$Y^1$ represents -C(H)=, -C(F)=, -C(Cl)= or -N=, and

$R^{26}$ represents a fluorine atom, a chlorine atom or a bromine atom, or a group selected from difluoromethyl, methoxy, benzyloxy, difluoromethoxy and trifluoromethoxy,
and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a group

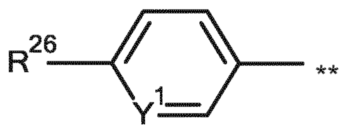

wherein "**" indicates the point of attachment to the nitrogen atom to which $R^1$ is attached;
$Y^1$ represents -C(H)=, -C(F)= or -N=;

$R^{26}$ represents a fluorine atom, a chlorine atom, or a group selected from $C_1$-$C_2$-alkyl, $C_1$-$C_2$-fluoroalkyl, $C_1$-$C_2$-alkoxy and $C_1$-$C_2$-fluoroalkoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a group

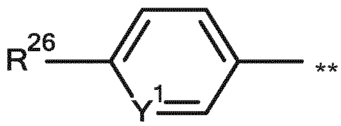

,

wherein "**" indicates the point of attachment to the nitrogen atom to which $R^1$ is attached,
$Y^1$ represents -C(H)= or-C(F)=, and

$R^{26}$ represents a fluorine atom, a chlorine atom, or a group selected from difluoromethyl, trifluoromethyl, methoxy, difluoromethoxy and trifluoromethoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a group

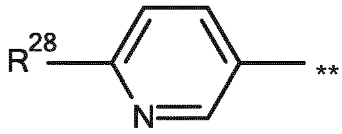

,

wherein "**" indicates the point of attachment to the nitrogen atom to which $R^1$ is attached, and

$R^{28}$ represents a group selected from methyl, difluoromethyl, trifluoromethyl, methoxy, difluoromethoxy and trifluoromethoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a group selected from

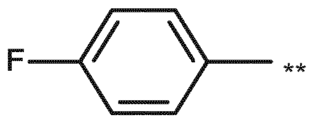 , 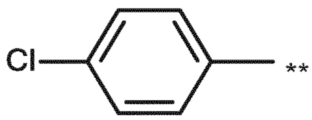 , 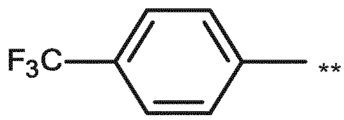 ,

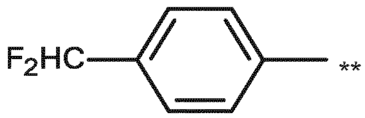 , 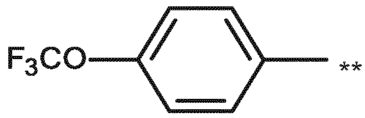 ,

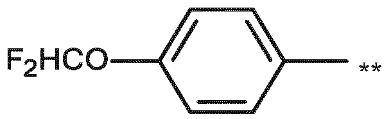 , 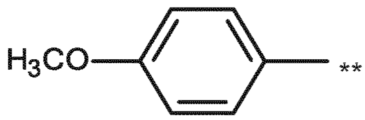 ,

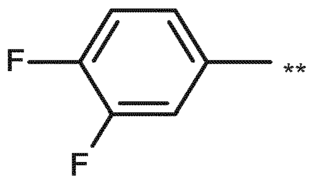 , 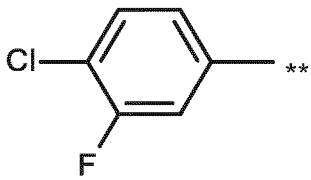 , 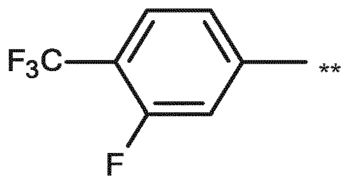 ,

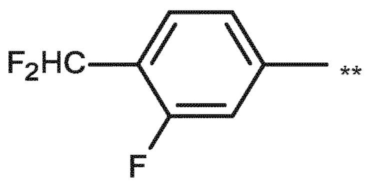 , 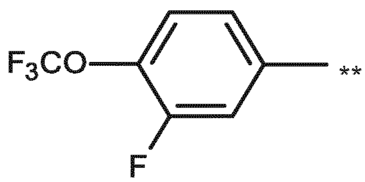 ,

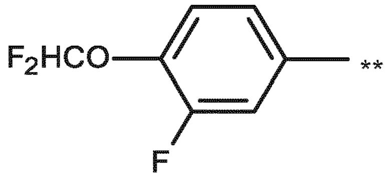,

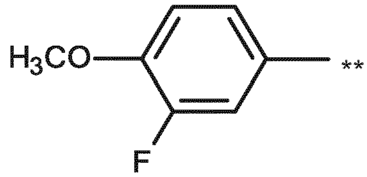,

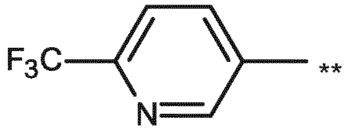,

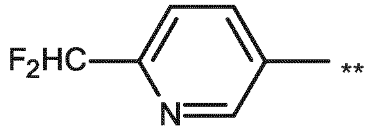,

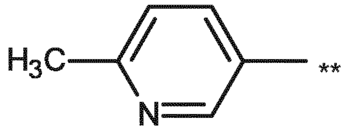,

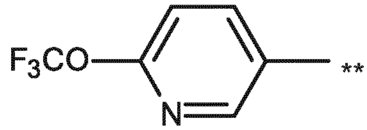,

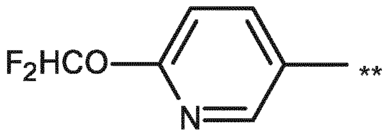, 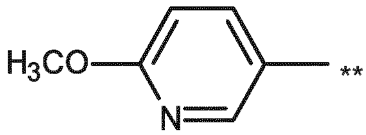,

wherein "**" indicates the point of attachment to the nitrogen atom to which $R^1$ is attached,
and stereoisomers, tautomers, N-oxides, hydrates, solvates, and salts thereof, and mixtures of same.

In a further embodiment of the first aspect, the present invention covers compounds of formula (I), *supra,* in which:

$R^1$ represents a group selected from

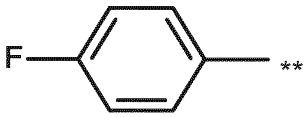 and 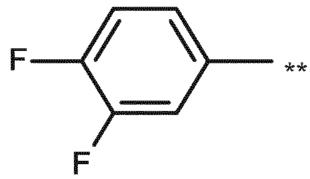,

wherein "**" indicates the point of attachment to the nitrogen atom to which $R^1$ is attached, and stereoisomers, tautomers, N-oxides, hydrates, solvates, and salts thereof, and mixtures of same.

In a further embodiment of the first aspect, the present invention covers compounds of formula (I), *supra,* in which:

$R^2$ represents a group

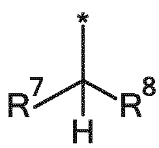,

wherein "*" indicates the point of attachment to the nitrogen atom to which $R^2$ is attached;

$R^7$ represents a hydrogen atom or a $C_1$-$C_2$-alkyl group;

$R^8$ represents a -C(=O)-$NH_2$ group,

and stereoisomers, tautomers, N-oxides, hydrates, solvates, and salts thereof, and mixtures of same.

In a further embodiment of the first aspect, the present invention covers compounds of formula (I), *supra,* in which:

$R^2$ represents a group

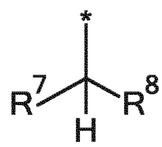

,

wherein "*" indicates the point of attachment to the nitrogen atom to which $R^2$ is attached;

$R^7$ represents a $C_1$-$C_2$-alkyl group;

$R^8$ represents a -C(=O)-$NH_2$ group,

and stereoisomers, tautomers, N-oxides, hydrates, solvates, and salts thereof, and mixtures of same.

In a further embodiment of the first aspect, the present invention covers compounds of formula (I), *supra,* in which:

$R^2$ represents a group

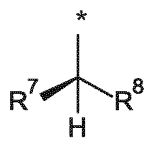

,

wherein "*" indicates the point of attachment to the nitrogen atom to which $R^2$ is attached;
$R^7$ represents a $C_1$-$C_2$-alkyl group;
$R^8$ represents a -C(=O)-$NH_2$ group,

and stereoisomers, tautomers, N-oxides, hydrates, solvates, and salts thereof, and mixtures of same.

In a further embodiment of the first aspect, the present invention covers compounds of formula (I), *supra,* in which:

$R^2$ represents a group

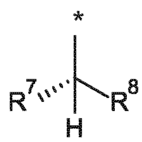

,

wherein "*" indicates the point of attachment to the nitrogen atom to which $R^2$ is attached;
$R^7$ represents a $C_1$-$C_2$-alkyl group;
$R^8$ represents a -C(=O)-$NH_2$ group,

and stereoisomers, tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group in a ratio of about 99:1 and higher,

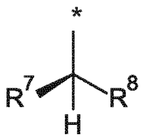 and 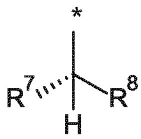 ,

in favour of

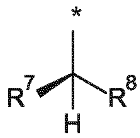 ,

wherein "*" indicates the point of attachment to the nitrogen atom to which $R^2$ is attached;

$R^7$ represents a $C_1$-$C_2$-alkyl group;

$R^8$ represents a -C(=O)-$NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

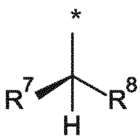 and 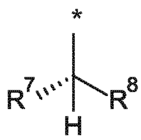 ,

in a ratio of about 98:2 and higher, in favour of

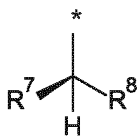 ,

wherein "*" indicates the point of attachment to the nitrogen atom to which $R^2$ is attached;

$R^7$ represents a $C_1$-$C_2$-alkyl group;

$R^8$ represents a -C(=O)-$NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

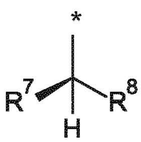 and 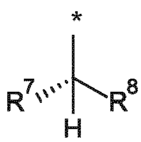 ,

in a ratio of about 95:5 and higher, in favour of

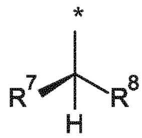

,

wherein "*" indicates the point of attachment to the nitrogen atom to which $R^2$ is attached;

$R^7$ represents a $C_1$-$C_2$-alkyl group;

$R^8$ represents a $-C(=O)-NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

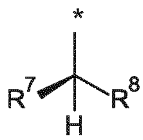

and

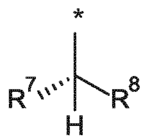

, i

in a ratio of about 90:10 and higher, in favour of

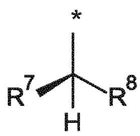

,

wherein "*" indicates the point of attachment to the nitrogen atom to which $R^2$ is attached;

$R^7$ represents a $C_1$-$C_2$-alkyl group;

$R^8$ represents a $-C(=O)-NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

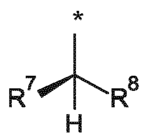

and

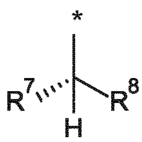

,

in a ratio of about 80:20 and higher, in favour of

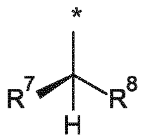

,

wherein "*" indicates the point of attachment to the nitrogen atom to which $R^2$ is attached;

$R^7$ represents a $C_1$-$C_2$-alkyl group;

$R^8$ represents a -C(=O)-$NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

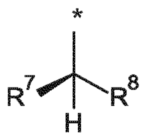

and

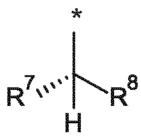

, in a ratio of about 50:50, that is, racemic mixtures in case of compounds featuring no further element of chirality, wherein "*" indicates the point of attachment to the nitrogen atom to which $R^2$ is attached;

$R^7$ represents a $C_1$-$C_2$-alkyl group;

$R^8$ represents a -C(=O)-$NH_2$ group,

and tautomers, N-oxides, hydrates, solvates, and salts thereof, and mixtures of same.

In a further embodiment of the first aspect, the present invention covers compounds of formula (I), *supra,* in which:

$R^2$ represents a group

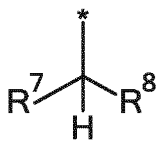

,

wherein "*" indicates the point of attachment to the nitrogen atom to which $R^2$ is attached;

$R^7$ represents a methyl group;

$R^8$ represents a -C(=O)-$NH_2$ group,

and stereoisomers, tautomers, N-oxides, hydrates, solvates, and salts thereof, and mixtures of same.

In a further embodiment of the first aspect, the present invention covers compounds of formula (I), *supra,* in which:

$R^2$ represents a group

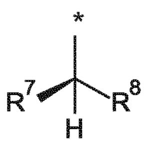

,

wherein "*" indicates the point of attachment to the nitrogen atom to which $R^2$ is attached;

$R^7$ represents a methyl group;

$R^8$ represents a $-C(=O)-NH_2$ group,

and stereoisomers, tautomers, N-oxides, hydrates, solvates, and salts thereof, and mixtures of same.

In a further embodiment of the first aspect, the present invention covers compounds of formula (I), *supra,* in which:

$R^2$ represents a group

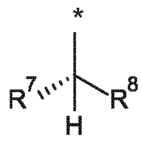

,

wherein "*" indicates the point of attachment to the nitrogen atom to which $R^2$ is attached;

$R^7$ represents a methyl group;

$R^8$ represents a $-C(=O)-NH_2$ group,

and stereoisomers, tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

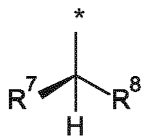

and

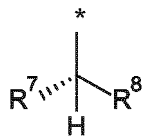

,

in a ratio of about 99:1 and higher, in favour of

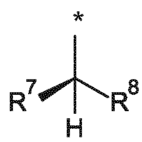

,

wherein "*" indicates the point of attachment to the nitrogen atom to which $R^2$ is attached;

$R^7$ represents a methyl group;

$R^8$ represents a $-C(=O)-NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

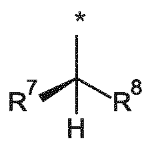

and

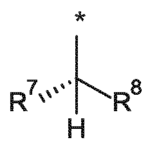

,

in a ratio of about 98:2 and higher, in favour of

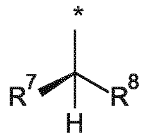

,

wherein "*" indicates the point of attachment to the nitrogen atom to which $R^2$ is attached;

$R^7$ represents a methyl group;

$R^8$ represents a $-C(=O)-NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

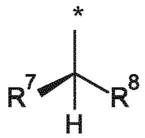

and

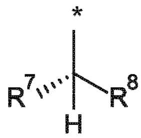

, i

in a ratio of about 95:5 and higher, in favour of

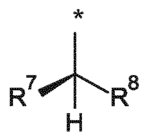

,

wherein "*" indicates the point of attachment to the nitrogen atom to which $R^2$ is attached;

$R^7$ represents a methyl group;

$R^8$ represents a $-C(=O)-NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

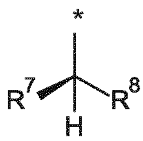

and

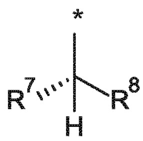

,

in a ratio of about 90:10 and higher, in favour of

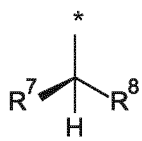

,

wherein "*" indicates the point of attachment to the nitrogen atom to which $R^2$ is attached;

$R^7$ represents a methyl group;

$R^8$ represents a $-C(=O)-NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

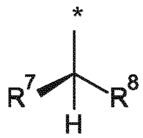

and

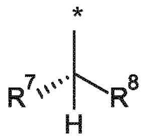

,

in a ratio of about 80:20 and higher, in favour of

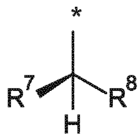

,

wherein "*" indicates the point of attachment to the nitrogen atom to which $R^2$ is attached;

$R^7$ represents a methyl group;

$R^8$ represents a $-C(=O)-NH_2$ group,

and tautomers, N-oxides, hydrates, solvates, and salts thereof, and mixtures of same.

In a further embodiment of the first aspect, the present invention covers isomeric mixtures of compounds of formula (I), *supra,* in which:

$R^2$ represents a group

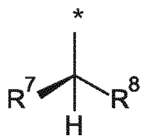

and

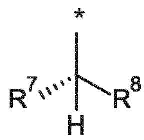

,

in a ratio of about 50:50, that is, racemic mixtures in case of compounds featuring no further element of chirality, wherein "*" indicates the point of attachment to the nitrogen atom to which $R^2$ is attached;

$R^7$ represents a methyl group;

$R^8$ represents a $-C(=O)-NH_2$ group,

and tautomers, N-oxides, hydrates, solvates, and salts thereof, and mixtures of same.

In a further embodiment of the first aspect, the present invention covers compounds of formula (I), *supra,* in which:

$R^3$ represents a group selected from methyl and $-NH_2$,

and stereoisomers, tautomers, N-oxides, hydrates, solvates, and salts thereof, and mixtures of same.

In a further embodiment of the first aspect, the present invention covers compounds of formula (I), *supra,* in which:

$R^3$ represents a $-NH_2$ group,

and stereoisomers, tautomers, N-oxides, hydrates, solvates, and salts thereof, and mixtures of same.

In a further embodiment of the first aspect, the present invention covers compounds of formula (I), *supra,* in which:

$R^3$ represents a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a 5-membered heteroaryl group selected from oxazolyl, isoxazolyl, thiazolyl, isothiazolyl and oxadiazolyl, optionally substituted one or two times, each substituent independently selected from a fluorine atom, a chlorine atom and a bromine atom, or a group selected from $C_1$-$C_4$-alkyl, $((R^9)O)$-($C_1$-$C_3$-alkyl)-, $((R^{10})(R^{11})N)$-($C_1$-$C_3$-alkyl)-, ($C_3$-$C_7$-cycloalkyl)-($C_1$-$C_3$-alkyl)-, $C_3$-$C_7$-cycloalkyl, $-OR^9$, $-N(R^{10})(R^{11})$, $-C(=O)-N(R^{15})(R^{16})$, $-C(=O)-OR^{17}$, phenyl and a 5- or 6-membered heteroaryl group,
wherein the phenyl group and the 5- or 6-membered heteroaryl group are optionally substituted one or two times, each substituent independently selected from a fluorine atom, a chlorine atom and a bromine atom, or a group selected from cyano, $C_1$-$C_2$-alkyl, $C_1$-$C_2$-fluoroalkyl, $-OR^9$, $-N(R^{10})(R^{11})$ and $-C(=O)-N(R^{15})(R^{16})$,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a 5-membered heteroaryl group selected from oxazolyl, isoxazolyl, thiazolyl and isothiazolyl, optionally substituted one or two times, each substituent independently selected from a fluorine atom, a chlorine atom and a bromine atom, or a group selected from $C_1$-$C_3$-alkyl, $-N(R^{10})(R^{11})$, $-C(=O)-N(R^{15})(R^{16})$, $-C(=O)-OR^{17}$, phenyl and a 5- or 6-membered heteroaryl group,
wherein the phenyl group and the 5- or 6-membered heteroaryl group are optionally substituted one or two times, each substituent independently selected from a fluorine atom, a chlorine atom and a bromine atom, or a group selected from cyano, $C_1$-$C_2$-alkyl, $C_1$-$C_2$-fluoroalkyl, $-OR^9$, $-N(R^{10})(R^{11})$ and $-C(=O)-N(R^{15})(R^{16})$,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group selected from

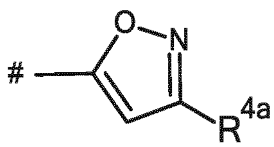
and
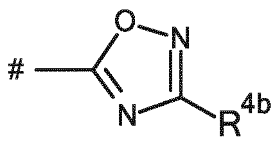
,

wherein "#" indicates the point of attachment to the carbonyl group to which $R^4$ is attached, and wherein

$R^{4a}$ and $R^{4b}$ represent, independently from each other, a fluorine atom, a chlorine atom or a bromine atom, or a group selected from $C_1$-$C_4$-alkyl, $((R^9)O)$-($C_1$-$C_2$-alkyl)-, $((R^{10})(R^{11})N)$-($C_1$-$C_2$-alkyl)-, ($C_3$-$C_7$-cycloalkyl)-($C_1$-$C_2$-alkyl)-, $C_3$-$C_7$-cycloalkyl, $-OR^9$, $-N(R^{10})(R^{11})$, $-C(=O)-N(R^{15})(R^{16})$, $-C(=O)-OR^{17}$, phenyl and a 5- or 6-membered heteroaryl group selected from oxazolyl, isoxazolyl, thiazolyl, isothiazolyl, pyrazolyl, pyridinyl, pyrazinyl, pyrimidinyl and pyridazinyl, wherein the phenyl group and the 5- or 6-membered heteroaryl group selected from oxazolyl, isoxazolyl, thiazolyl, isothiazolyl, pyrazolyl, pyridinyl, pyrazinyl, pyrimidinyl and pyridazinyl are optionally substituted one or two times, each substituent independently selected from a fluorine atom and a chlorine atom, or a group selected from cyano, $C_1$-$C_2$-alkyl, $-OR^9$, $-N(R^{10})(R^{11})$ and $-C(=O)-N(R^{15})(R^{16})$,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

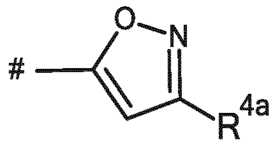

,

wherein "#" indicates the point of attachment to the carbonyl group to which $R^4$ is attached, and wherein

$R^{4a}$ represents a fluorine atom, a chlorine atom or a bromine atom, or a group selected from $C_1$-$C_4$-alkyl, (($R^9$)O)-($C_1$-$C_2$-alkyl)-, (($R^{10}$)($R^{11}$)N)-($C_1$-$C_2$-alkyl)-, ($C_3$-$C_7$-cycloalkyl)-($C_1$-$C_2$-alkyl)-, $C_3$-$C_7$-cycloalkyl, -$OR^9$, -N($R^{10}$)($R^{11}$), -C(=O)-N($R^{15}$)($R^{16}$), -C(=O)-$OR^{17}$, phenyl and a 5- or 6-membered heteroaryl group selected from oxazolyl, isoxazolyl, thiazolyl, isothiazolyl, pyrazolyl, pyridinyl, pyrazinyl, pyrimidinyl and pyridazinyl,

wherein the phenyl group and the 5- or 6-membered heteroaryl group selected from oxazolyl, isoxazolyl, thiazolyl, isothiazolyl, pyrazolyl, pyridinyl, pyrazinyl, pyrimidinyl and pyridazinyl are optionally substituted one or two times, each substituent independently selected from a fluorine atom and a chlorine atom, or a group selected from cyano, $C_1$-$C_2$-alkyl, -$OR^9$, -N($R^{10}$)($R^{11}$) and -C(=O)-N($R^{15}$)($R^{16}$),
and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

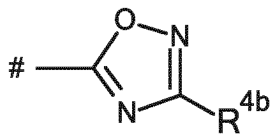

,

wherein "#" indicates the point of attachment to the carbonyl group to which $R^4$ is attached, and wherein

$R^{4b}$ represents a fluorine atom, a chlorine atom or a bromine atom, or a group selected from $C_1$-$C_4$-alkyl, (($R^9$)O)-($C_1$-$C_2$-alkyl)-, (($R^{10}$)($R^{11}$)N)-($C_1$-$C_2$-alkyl)-, ($C_3$-$C_7$-cycloalkyl)-($C_1$-$C_2$-alkyl)-, $C_3$-$C_7$-cycloalkyl, -$OR^9$, -N($R^{10}$)($R^{11}$), -C(=O)-N($R^{15}$)($R^{16}$), -C(=O)-$OR^{17}$, phenyl and a 5- or 6-membered heteroaryl group selected from oxazolyl, isoxazolyl, thiazolyl, isothiazolyl, pyrazolyl, pyridinyl, pyrazinyl, pyrimidinyl and pyridazinyl, wherein the phenyl group and the 5- or 6-membered heteroaryl group selected from oxazolyl, isoxazolyl, thiazolyl, isothiazolyl, pyrazolyl, pyridinyl, pyrazinyl, pyrimidinyl and pyridazinyl are optionally substituted one or two times, each substituent independently selected from a fluorine atom and a chlorine atom, or a group selected from cyano, $C_1$-$C_2$-alkyl, -$OR^9$, -N($R^{10}$)($R^{11}$) and -C(=O)-N($R^{15}$)($R^{16}$),

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

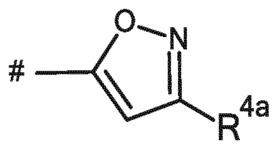

,

wherein "#" indicates the point of attachment to the carbonyl group to which $R^4$ is attached, and wherein

$R^{4a}$ represents a group selected from -N($R^{10}$)($R^{11}$), -C(=O)-N($R^{15}$)($R^{16}$), -C(=O)-$OR^{17}$, phenyl and a 5- or 6-membered heteroaryl group selected from oxazolyl, isoxazolyl, thiazolyl, isothiazolyl, pyrazolyl, pyridinyl, pyrazinyl, pyrimidinyl and pyridazinyl,

wherein the phenyl group and the 5- or 6-membered heteroaryl group selected from oxazolyl, isoxazolyl, thiazolyl, isothiazolyl, pyrazolyl, pyridinyl, pyrazinyl, pyrimidinyl and pyridazinyl are optionally substituted one or two times, each substituent independently selected from a fluorine atom and a chlorine atom, or a group selected from cyano, $C_1$-$C_2$-alkyl, -$OR^9$, -$N(R^{10})(R^{11})$ and -C(=O)-$N(R^{15})(R^{16})$;
and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents an isoxazolyl group substituted with one phenyl group,

wherein the phenyl group is optionally substituted one or two times, each substituent independently selected from a fluorine atom, a chlorine atom or a methyl group, and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

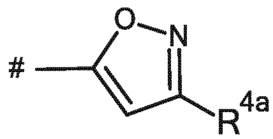

,

wherein "#" indicates the point of attachment to the carbonyl group to which $R^4$ is attached, and wherein

$R^{4a}$ represents a chlorine atom or a bromine atom, or a group selected from $((R^9)O)$-($C_1$-$C_2$-alkyl)-, $((R^{10})(R^{11})N)$-($C_1$-$C_2$-alkyl)-, ($C_3$-$C_5$-cycloalkyl)-($C_1$-$C_2$-alkyl)-, $C_3$-$C_5$-cycloalkyl, - $OR^9$, -C(=O)-$N(R^{15})(R^{16})$ and phenyl, wherein the phenyl group is optionally substituted one or two times, each substituent independently selected from a fluorine atom and a chlorine atom and a methyl group, or

$R^4$ represents a group

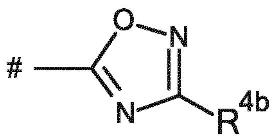

,

wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl, -C(=O)-$N(R^{15})(R^{16})$ and phenyl, wherein the phenyl group is optionally substituted one or two times, each substituent independently selected from a fluorine atom and a chlorine atom, or a group selected from cyano, $C_1$-$C_2$-alkyl and -$OR^9$,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

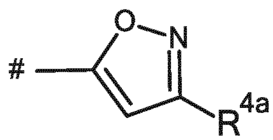

,

wherein "#" indicates the point of attachment to the carbonyl group to which $R^4$ is attached, and wherein

$R^{4a}$ represents a chlorine atom or a bromine atom, or a group selected from $((R^9)O)$-($C_1$-$C_2$-alkyl)-, $((R^{10})(R^{11})N)$-($C_1$-$C_2$-alkyl)-, ($C_3$-$C_5$-cycloalkyl)-($C_1$-$C_2$-alkyl)-, $C_3$-$C_5$-cycloalkyl, - $OR^9$, -C(=O)-N($R^{15}$)($R^{16}$) and phenyl, wherein the phenyl group is optionally substituted one or two times, each substituent independently selected from a fluorine atom and a chlorine atom and a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

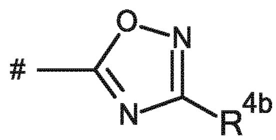

,

wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl, -C(=O)-N($R^{15}$)($R^{16}$) and phenyl, wherein the phenyl group is optionally substituted one or two times, each substituent independently selected from a fluorine atom and a chlorine atom, or a group selected from cyano, $C_1$-$C_2$-alkyl and -$OR^9$,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

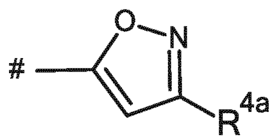

,

wherein "#" indicates the point of attachment to the carbonyl group to which $R^4$ is attached, and wherein

$R^{4a}$ represents a chlorine atom or a bromine atom, or a group selected from $((R^9)O)$-($C_1$-alkyl)-, $((R^{10})(R^{11})N)$-($C_1$-alkyl)-, ($C_3$-$C_5$-cycloalkyl)-($C_1$-alkyl)-, $C_3$-$C_5$-cycloalkyl, -$OR^9$, - C(=O)-N($R^{15}$)($R^{16}$) and phenyl, wherein the phenyl group is optionally substituted one or two times, each substituent independently selected from a fluorine atom and a chlorine atom and a methyl group, or

$R^4$ represents a group

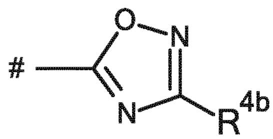

,

wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl and phenyl, wherein the phenyl group is optionally substituted one or two times, each substituent independently selected from a fluorine atom and a chlorine

atom, or a group selected from cyano, $C_1$-$C_2$-alkyl and -$OR^9$,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

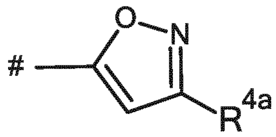

,

wherein "#" indicates the point of attachment to the carbonyl group to which $R^4$ is attached, and wherein

$R^{4a}$ represents a chlorine atom or a bromine atom, or a group selected from (($R^9$)O)-($C_1$-alkyl)-, (($R^{10}$)($R^{11}$)N)-($C_1$-alkyl)-, ($C_3$-$C_5$-cycloalkyl)-($C_1$-alkyl)-, $C_3$-$C_5$-cycloalkyl, -$OR^9$, - C(=O)-N($R^{15}$)($R^{16}$) and phenyl, wherein the phenyl group is optionally substituted one or two times, each substituent independently selected from a fluorine atom and a chlorine atom and a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

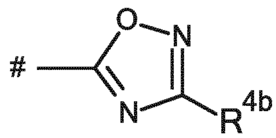

,

wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl and phenyl, wherein the phenyl group is optionally substituted one or two times, each substituent independently selected from a fluorine atom and a chlorine atom, or a group selected from cyano, $C_1$-$C_2$-alkyl and -$OR^9$,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

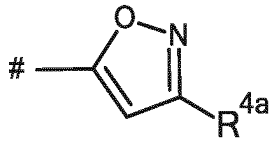

,

wherein "#" indicates the point of attachment to the carbonyl group to which $R^4$ is attached, and wherein

$R^{4a}$ represents a chlorine atom or a bromine atom, or a group selected from (($R^9$)O)-($C_1$-alkyl)-, $C_3$-$C_5$-cycloalkyl, -$OR^9$ and -C(=O)-N($R^{15}$)($R^{16}$), or

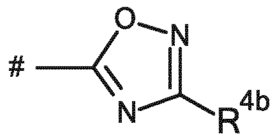

,

$R^4$ represents a group wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl and phenyl, wherein the phenyl group is optionally substituted one or two times, each substituent independently selected from a fluorine atom and a chlorine atom and a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

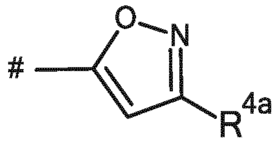

,

wherein "#" indicates the point of attachment to the carbonyl group to which $R^4$ is attached, and wherein

$R^{4a}$ represents a chlorine atom or a bromine atom, or a group selected from $((R^9)O)$-($C_1$-alkyl)-, $C_3$-$C_5$-cycloalkyl, $-OR^9$ and $-C(=O)-N(R^{15})(R^{16})$,

and stereoisomers, tautomers, N-oxides, hydrates, solvates, and salts thereof, and mixtures of same.

In a further embodiment of the first aspect, the present invention covers compounds of formula (I), *supra,* in which:

$R^4$ represents a group

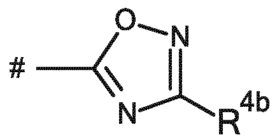

,

wherein "#" indicates the point of attachment to the carbonyl group to which $R^4$ is attached, and wherein

$R^{4b}$ represents a group selected from $C_1$-$C_4$-alkyl, $C_3$-$C_5$-cycloalkyl and phenyl, wherein the phenyl group is optionally substituted one or two times, each substituent independently selected from a fluorine atom and a chlorine atom and a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^5$ and $R^6$ represent, independently from each occurrence, a hydrogen atom or a group selected from $C_1$-$C_2$-alkyl and ($C_1$-$C_2$-alkyl)-C(=O)-, or

$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom, a chlorine atom and a bromine atom, or a group selected from oxo, hydroxy, $C_1$-$C_2$-alkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I), *supra,* in

which:

$R^5$ and $R^6$ represent, independently from each occurrence, a hydrogen atom or a group selected from $C_1$-$C_2$-alkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom, a chlorine atom and a bromine atom, or a group selected from oxo, hydroxy, $C_1$-$C_2$-alkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^5$ and $R^6$ represent, independently from each occurrence, a hydrogen atom or a $C_1$-$C_2$-alkyl group, or

$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom or a group selected from hydroxy and $C_1$-$C_2$-alkyl,

and stereoisomers, tautomers, N-oxides, hydrates, solvates, and salts thereof, and mixtures of same.

In a further embodiment of the first aspect, the present invention covers compounds of formula (I), *supra,* in which:

$R^5$ and $R^6$ represent, independently from each occurrence, a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom or a group selected from hydroxy and $C_1$-$C_2$-alkyl,

and stereoisomers, tautomers, N-oxides, hydrates, solvates, and salts thereof, and mixtures of same.

In a further embodiment of the first aspect, the present invention covers compounds of formula (I), *supra,* in which:

$R^7$ represents a hydrogen atom,

and stereoisomers, tautomers, N-oxides, hydrates, solvates, and salts thereof, and mixtures of same.

In a further embodiment of the first aspect, the present invention covers compounds of formula (I), *supra,* in which:

$R^7$ represents a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^7$ represents an ethyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^7$ represents a hydrogen atom or a $C_1$-$C_2$-alkyl group;

and stereoisomers, tautomers, N-oxides, hydrates, solvates, and salts thereof, and mixtures of same.

In a further embodiment of the first aspect, the present invention covers compounds of formula (I), *supra,* in which:

$R^9$ represents a hydrogen atom or a group selected from $C_1$-$C_4$-alkyl, ($C_3$-$C_7$-cycloalkyl)-($C_1$-$C_2$-alkyl)-, (phenyl)-($C_1$-$C_2$-alkyl)-, $C_1$-$C_4$-haloalkyl, $C_2$-$C_3$-hydroxyalkyl, ($C_1$-$C_2$-alkoxy)-$C_2$-alkyl-, (($C_1$-$C_2$-alkyl)-C(=O)-O)-$C_2$-alkyl-, -C(=O)-N($R^{20}$)($R^{21}$), $C_3$-$C_7$-cycloalkyl and phenyl, wherein $C_3$-$C_7$-cycloalkyl, and the $C_3$-$C_7$-cycloalkyl within said ($C_3$-$C_7$-cycloalkyl)-($C_1$-$C_2$-alkyl)- group is optionally substituted one or two times, each substituent independently selected from a fluorine atom or a group selected from oxo and methyl, and wherein the phenyl group within said (phenyl)-($C_1$-$C_2$-alkyl)- group and said phenyl group itself are optionally substituted one or two times, each substituent independently selected from a fluorine atom, a chlorine atom and a bromine atom, or a group selected from cyano, methyl, trifluoromethyl and methoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^9$ represents a hydrogen atom or a group selected from $C_1$-$C_4$-alkyl, (phenyl)-($C_1$-$C_2$-alkyl)-, $C_1$-$C_4$-haloalkyl, $C_2$-$C_3$-hydroxyalkyl, ($C_1$-$C_2$-alkoxy)-$C_2$-alkyl-, (($C_1$-$C_2$-alkyl)-C(=O)-O)-$C_2$-alkyl-, -C($R^{18}$)($R^{19}$)-C(=O)-O$R^{17}$, -C($R^{18}$)($R^{19}$)-C(=O)-N($R^{20}$)($R^{21}$) and phenyl, wherein the phenyl group within said (phenyl)-($C_1$-$C_2$-alkyl)- group and said phenyl group itself are optionally substituted one or two times, each substituent independently selected from a fluorine atom, a chlorine atom and a bromine atom, or a group selected from methyl, trifluoromethyl and methoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^9$ represents a hydrogen atom or a group selected from $C_1$-$C_2$-alkyl, benzyl, $C_1$-$C_2$-fluoroalkyl, $C_2$-hydroxyalkyl, ($C_1$-$C_2$-alkoxy)-$C_2$-alkyl-, (($C_1$-$C_2$-alkyl)-C(=O)-O)-$C_2$-alkyl-, - C(=O)-N($R^{20}$)($R^{21}$) and phenyl, wherein the phenyl group within said benzyl group and said phenyl group itself are optionally substituted one or two times, each substituent independently selected from a fluorine atom and a chlorine atom, or a group selected from cyano and methyl,

and stereoisomers, tautomers, N-oxides, hydrates, solvates, and salts thereof, and mixtures of same.

In a further embodiment of the first aspect, the present invention covers compounds of formula (I), *supra,* in which:

$R^9$ represents a hydrogen atom or a group selected from $C_1$-$C_2$-alkyl, benzyl, $C_1$-$C_2$-fluoroalkyl, ($C_1$-$C_2$-alkoxy)-$C_2$-alkyl-, (($C_1$-$C_2$-alkyl)-C(=O)-O)-$C_2$-alkyl-, -C($R^{18}$)($R^{19}$)-C(=O)-O$R^{17}$, -C($R^{18}$)($R^{19}$)-C(=O)-N($R^{20}$)($R^{21}$) and phenyl,
wherein the phenyl group within said benzyl group and said phenyl group itself are optionally substituted one or two times, each substituent independently selected from a fluorine atom, a chlorine atom and a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^9$ represents a hydrogen atom or a group selected from $C_1$-$C_2$-alkyl, benzyl, $C_1$-$C_2$-fluoroalkyl, ($C_1$-$C_2$-alkoxy)-$C_2$-alkyl-, (($C_1$-$C_2$-alkyl)-C(=O)-O)-$C_2$-alkyl-, -C(=O)-N($R^{20}$)($R^{21}$) and phenyl,
wherein the phenyl group within said benzyl group and said phenyl group itself are optionally substituted one or two times, each substituent independently selected from a fluorine atom and a chlorine atom, or a group selected from cyano and methyl,

and stereoisomers, tautomers, N-oxides, hydrates, solvates, and salts thereof, and mixtures of same.

In a further embodiment of the first aspect, the present invention covers compounds of formula (I), *supra,* in

which:

$R^9$ represents a group selected from $C_1$-$C_2$-alkyl, benzyl, $C_1$-$C_2$-fluoroalkyl, ($C_1$-$C_2$-alkoxy)-$C_2$-alkyl- and phenyl, wherein the phenyl group within said benzyl group and said phenyl group itself are optionally substituted one or two times, each substituent independently selected from a fluorine atom, a chlorine atom and a methyl group;

and stereoisomers, tautomers, N-oxides, hydrates, solvates, and salts thereof, and mixtures of same.

In a further embodiment of the first aspect, the present invention covers compounds of formula (I), *supra,* in which:

$R^9$ represents a hydrogen atom or a group selected from $C_1$-$C_2$-alkyl, benzyl, $C_1$-$C_2$-fluoroalkyl, ($C_1$-$C_2$-alkoxy)-$C_2$-alkyl-, (($C_1$-$C_2$-alkyl)-C(=O)-O)-$C_2$-alkyl- and phenyl,
wherein the phenyl group within said benzyl group and said phenyl group itself are optionally substituted one or two times, each substituent independently selected from a fluorine atom and a chlorine atom, or a group selected from cyano and methyl,

and stereoisomers, tautomers, N-oxides, hydrates, solvates, and salts thereof, and mixtures of same.

In a further embodiment of the first aspect, the present invention covers compounds of formula (I), *supra,* in which:

$R^9$ represents a hydrogen atom or a group selected from $C_1$-$C_2$-alkyl, benzyl, $C_1$-$C_2$-fluoroalkyl and phenyl,
wherein the phenyl group within said benzyl group and said phenyl group itself are optionally substituted one or two times, each substituent independently selected from a fluorine atom and a chlorine atom, or a group selected from cyano and methyl,

and stereoisomers, tautomers, N-oxides, hydrates, solvates, and salts thereof, and mixtures of same.

In a further embodiment of the first aspect, the present invention covers compounds of formula (I), *supra,* in which:

$R^{10}$ and $R^{11}$ represent, independently from each occurrence, a hydrogen atom or a group selected from $C_1$-$C_2$-alkyl, $C_1$-$C_2$-haloalkyl, $C_2$-$C_3$-hydroxyalkyl, ($C_1$-$C_2$-alkoxy)-$C_2$-alkyl-, (($R^{22}$)($R^{23}$)N)-$C_2$-alkyl, ($C_3$-$C_7$-cycloalkyl)-($C_1$-$C_2$-alkyl)-, ($C_1$-$C_2$-alkyl)-C(=O)-, $C_3$-$C_7$-cycloalkyl, ($C_3$-$C_7$-cycloalkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,

wherein $C_3$-$C_7$-cycloalkyl, and the $C_3$-$C_7$-cycloalkyl within said ($C_3$-$C_7$-cycloalkyl)-($C_1$-$C_2$-alkyl)- and ($C_3$-$C_7$-cycloalkyl)-C(=O)- groups are optionally substituted one or two times, each substituent independently selected from a fluorine atom or a group selected from cyano, $C_1$-$C_2$-alkyl and $C_1$-$C_2$-haloalkyl,

and wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a bromine atom, or a group selected from methyl, trifluoromethyl and methoxy,

or

$R^{10}$ and $R^{11}$, together with the nitrogen atom to which they are attached, represent a monocyclic nitrogen containing 4- to 7-membered heterocycloalkyl group, or a bicyclic nitrogen containing 5- to 10-membered heterocycloalkyl group, which are optionally substituted one, two or three times, each substituent independently selected from a halogen atom or a group selected from cyano, oxo, hydroxy, $C_1$-$C_2$-alkyl, $C_1$-$C_2$-haloalkyl, ($C_1$-$C_2$-alkyl)-C(=O)-, $C_1$-$C_2$-alkoxy, -N($R^{22}$)($R^{23}$), and a monocyclic 4- to 7-membered heterocycloalkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{10}$ and $R^{11}$ represent, independently from each occurrence, a hydrogen atom or a group selected from $C_1$-$C_2$-alkyl, $C_1$-$C_2$-haloalkyl, $C_2$-$C_3$-hydroxyalkyl, ($C_1$-$C_2$-alkoxy)-$C_2$-alkyl-, (($R^{22}$)($R^{23}$)N)-$C_2$-alkyl, ($C_3$-$C_7$-cy-

cloalkyl)-($C_1$-$C_2$-alkyl)-, ($C_1$-$C_2$-alkyl)-C(=O)-, $C_3$-$C_7$-cycloalkyl, ($C_3$-$C_7$-cycloalkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,

wherein $C_3$-$C_7$-cycloalkyl, and the $C_3$-$C_7$-cycloalkyl within said ($C_3$-$C_7$-cycloalkyl)-($C_1$-$C_2$-alkyl)- and ($C_3$-$C_7$-cycloalkyl)-C(=O)- groups are optionally substituted one or two times, each substituent independently selected from a fluorine atom or a group selected from cyano, $C_1$-$C_2$-alkyl and $C_1$-$C_2$-haloalkyl,

nd wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a bromine atom, or a group selected from methyl, trifluoromethyl and methoxy,

a

and stereoisomers, tautomers, N-oxides, hydrates, solvates, and salts thereof, and mixtures of same.

In a further embodiment of the first aspect, the present invention covers compounds of formula (I), *supra,* in which:

$R^{10}$ and $R^{11}$, together with the nitrogen atom to which they are attached, represent a monocyclic nitrogen containing 4- to 7-membered heterocycloalkyl group, or a bicyclic nitrogen containing 5- to 10-membered heterocycloalkyl group, which are optionally substituted one, two or three times, each substituent independently selected from a halogen atom or a group selected from cyano, oxo, hydroxy, $C_1$-$C_2$-alkyl, $C_1$-$C_2$-haloalkyl, ($C_1$-$C_2$-alkyl)-C(=O)-, $C_1$-$C_2$-alkoxy, -N($R^{22}$)($R^{23}$), and a monocyclic 4- to 7-membered heterocycloalkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{10}$ and $R^{11}$ represent, independently from each occurrence, a hydrogen atom or a group selected from $C_1$-$C_2$-alkyl, $C_1$-$C_2$-haloalkyl, $C_2$-$C_3$-hydroxyalkyl, ($C_1$-$C_2$-alkoxy)-$C_2$-alkyl-, (($R^{22}$)($R^{23}$) N)-$C_2$-alkyl, ($C_1$-$C_2$-alkyl)-C(=O)-, $C_3$-$C_5$-cycloalkyl, ($C_3$-$C_5$-cycloalkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,
wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a bromine atom, or a group selected from methyl, trifluoromethyl and methoxy, or

$R^{10}$ and $R^{11}$, together with the nitrogen atom to which they are attached, represent a monocyclic nitrogen containing 4- to 7-membered heterocycloalkyl group, or a bicyclic nitrogen containing 6- to 10-membered heterocycloalkyl group, which are optionally substituted one, two or three times, each substituent independently selected from a halogen atom or a group selected from cyano, oxo, hydroxy, $C_1$-$C_2$-alkyl, $C_1$-$C_2$-haloalkyl, ($C_1$-$C_2$-alkyl)-C(=O)-, $C_1$-$C_2$-alkoxy, -N($R^{22}$)($R^{23}$), and a monocyclic 4- to 7-membered heterocycloalkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{10}$ and $R^{11}$ represent, independently from each occurrence, a hydrogen atom or a group selected from $C_1$-$C_2$-alkyl, $C_1$-$C_2$-haloalkyl, $C_2$-$C_3$-hydroxyalkyl, ($C_1$-$C_2$-alkoxy)-$C_2$-alkyl-, (($R^{22}$)($R^{23}$)N)-$C_2$-alkyl, ($C_1$-$C_2$-alkyl)-C(=O)-, $C_3$-$C_5$-cycloalkyl, ($C_3$-$C_5$-cycloalkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,
wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a bromine atom, or a group selected from methyl, trifluoromethyl and methoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^{10}$ and $R^{11}$, together with the nitrogen atom to which they are attached, represent a monocyclic nitrogen containing 4- to 7-membered heterocycloalkyl group, or a bicyclic nitrogen containing 6- to 10-membered heterocycloalkyl group, which are optionally substituted one, two or three times, each substituent independently selected from a halogen atom or a group selected from cyano, oxo, hydroxy, $C_1$-$C_2$-alkyl, $C_1$-$C_2$-haloalkyl, ($C_1$-$C_2$-alkyl)-C(=O)-, $C_1$-$C_2$-alkoxy, -N($R^{22}$)($R^{23}$), and a monocyclic 4- to 7-membered heterocycloalkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{10}$ and $R^{11}$ represent, independently from each occurrence, a hydrogen atom or a group selected from $C_1$-$C_2$-alkyl, $C_1$-$C_2$-fluoroalkyl, ($C_3$-$C_5$-cycloalkyl)-($C_1$-$C_2$-alkyl)- ($C_1$-$C_2$-alkyl)-C(=O)-, $C_3$-$C_7$-cycloalkyl, $C_3$-$C_7$-cycloalkyl-(C=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,

wherein $C_3$-$C_7$-cycloalkyl, and the $C_3$-$C_5$-cycloalkyl within said ($C_3$-$C_5$-cycloalkyl)-($C_1$-$C_2$-alkyl)- and the $C_3$-$C_7$-cycloalkyl within the $C_3$-$C_7$-cycloalkyl-(C=O)- groups are optionally substituted one or two times, each substituent independently selected from a fluorine atom or a group selected from cyano, $C_1$-$C_2$-alkyl and $C_1$-$C_2$-fluoroalkyl,

and wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a methyl group,

or

$R^{10}$ and $R^{11}$, together with the nitrogen atom to which they are attached, represent a monocyclic nitrogen containing 4- to 7-membered heterocycloalkyl group which is optionally substituted one, two or three times, each substituent independently selected from a fluorine atom or a group selected from cyano, oxo, $C_1$-$C_2$-alkyl, $C_1$-$C_2$-fluoroalkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^{10}$ and $R^{11}$ represent, independently from each occurrence, a hydrogen atom or a group selected from $C_1$-$C_2$-alkyl, $C_1$-$C_2$-fluoroalkyl, ($C_3$-$C_5$-cycloalkyl)-($C_1$-$C_2$-alkyl)- ($C_1$-$C_2$-alkyl)-C(=O)-, $C_3$-$C_7$-cycloalkyl, $C_3$-$C_7$-cycloalkyl-(C=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,

wherein $C_3$-$C_7$-cycloalkyl, and the $C_3$-$C_5$-cycloalkyl within said ($C_3$-$C_5$-cycloalkyl)-($C_1$-$C_2$-alkyl)- and the $C_3$-$C_7$-cycloalkyl within the $C_3$-$C_7$-cycloalkyl-(C=O)- groups are optionally substituted one or two times, each substituent independently selected from a fluorine atom or a group selected from cyano, $C_1$-$C_2$-alkyl and $C_1$-$C_2$-fluoroalkyl,

and wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{10}$ and $R^{11}$, together with the nitrogen atom to which they are attached, represent a monocyclic nitrogen containing 4- to 7-membered heterocycloalkyl group which is optionally substituted one, two or three times, each substituent independently selected from a fluorine atom or a group selected from cyano, oxo, $C_1$-$C_2$-alkyl, $C_1$-$C_2$-fluoroalkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I)*, supra,* in which:

$R^{10}$ and $R^{11}$ represent, independently from each occurrence, a hydrogen atom or a group selected from $C_1$-$C_2$-alkyl, $C_1$-$C_2$-fluoroalkyl, ($C_1$-$C_2$-alkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-, wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a methyl group, or

$R^{10}$ and $R^{11}$, together with the nitrogen atom to which they are attached, represent a monocyclic nitrogen containing 4- to 7-membered heterocycloalkyl group which is optionally substituted one or two times, each substituent independently selected from a fluorine atom or a group selected from oxo, $C_1$-$C_2$-alkyl, $C_1$-$C_2$-fluoroalkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I)*, supra,* in which:

$R^{10}$ and $R^{11}$ represent, independently from each occurrence, a hydrogen atom or a group selected from $C_1$-$C_2$-alkyl, $C_1$-$C_2$-fluoroalkyl, ($C_1$-$C_2$-alkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,
wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a methyl group,

and stereoisomers, tautomers, N-oxides, hydrates, solvates, and salts thereof, and mixtures of same.

In a further embodiment of the first aspect, the present invention covers compounds of formula (I)*, supra,* in which:

$R^{10}$ and $R^{11}$, together with the nitrogen atom to which they are attached, represent a monocyclic nitrogen containing 4- to 7-membered heterocycloalkyl group which is optionally substituted one or two times, each substituent independently selected from a fluorine atom or a group selected from oxo, $C_1$-$C_2$-alkyl, $C_1$-$C_2$-fluoroalkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I)*, supra,* in which:

$R^{10}$ and $R^{11}$ represent, independently from each occurrence, a hydrogen atom or a group selected from $C_1$-$C_2$-alkyl, ($C_3$-$C_5$-cycloalkyl)-($C_1$-$C_2$-alkyl)-, $C_3$-$C_7$-cycloalkyl and (phenyl)-($C_1$-$C_2$-alkyl)-O-C(=O)-, wherein $C_3$-$C_7$-cycloalkyl, and the $C_3$-$C_5$-cycloalkyl within said ($C_3$-$C_5$-cycloalkyl)-($C_1$-$C_2$-alkyl)- group are optionally substituted one or two times, each substituent independently selected from a fluorine atom or a group selected from cyano, methyl and $C_1$-fluoroalkyl, and wherein the phenyl group within said (phenyl)-($C_1$-$C_2$-alkyl)-O-C(=O)- group is optionally substituted one or two times, each substituent independently selected from a fluorine atom, a chlorine atom and a methyl group, or

$R^{10}$ and $R^{11}$, together with the nitrogen atom to which they are attached, represent a monocyclic nitrogen containing 4- to 7-membered heterocycloalkyl group which is optionally substituted one, two or threetimes, each substituent independently selected from a fluorine atom or a group selected from cyano, methyl and $C_1$-fluoroalkyl,

and stereoisomers, tautomers, N-oxides, hydrates, solvates, and salts thereof, and mixtures of same.

In a further embodiment of the first aspect, the present invention covers compounds of formula (I)*, supra,* in which:

$R^{10}$ and $R^{11}$ represent, independently from each occurrence, a hydrogen atom or a group selected from $C_1$-$C_2$-alkyl, ($C_3$-$C_5$-cycloalkyl)-($C_1$-$C_2$-alkyl)-, $C_3$-$C_7$-cycloalkyl and (phenyl)-($C_1$-$C_2$-alkyl)-O-C(=O)-, wherein $C_3$-$C_7$-cycloalkyl, and the $C_3$-$C_5$-cycloalkyl within said ($C_3$-$C_5$-cycloalkyl)-($C_1$-$C_2$-alkyl)- group are optionally substituted one or two times, each substituent independently selected from a fluorine atom or a group selected from cyano, methyl and $C_1$-fluoroalkyl, and wherein the phenyl group within said (phenyl)-($C_1$-$C_2$-alkyl)-O-C(=O)- group is optionally substituted one or two times, each substituent independently selected from a fluorine atom, a chlorine atom and a methyl group,

and stereoisomers, tautomers, N-oxides, hydrates, solvates, and salts thereof, and mixtures of same.

In a further embodiment of the first aspect, the present invention covers compounds of formula (I)*, supra,* in which:

$R^{10}$ and $R^{11}$, together with the nitrogen atom to which they are attached, represent a monocyclic nitrogen containing 4- to 7-membered heterocycloalkyl group which is optionally substituted one, two or threetimes, each substituent independently selected from a fluorine atom or a group selected from cyano, methyl and $C_1$-fluoroalkyl,

and stereoisomers, tautomers, N-oxides, hydrates, solvates, and salts thereof, and mixtures of same.

In a further embodiment of the first aspect, the present invention covers compounds of formula (I)*, supra,* in which:

$R^{10}$ and $R^{11}$ represent, independently from each occurrence, a hydrogen atom or a group selected from $C_1$-$C_2$-alkyl, $C_3$-$C_7$-cycloalkyl and (benzyl)-O-C(=O)-,

wherein $C_3$-$C_7$-cycloalkyl is optionally substituted one or two times, each substituent independently selected from a fluorine atom or a group selected from methyl and trifluoromethyl,

and wherein the phenyl group within said (benzyl)-O-C(=O)- group is optionally substituted one or two times, each substituent independently selected from a fluorine atom, a chlorine atom and a methyl group,

or

$R^{10}$ and $R^{11}$, together with the nitrogen atom to which they are attached, represent a monocyclic nitrogen containing 4- to 7-membered heterocycloalkyl group which is optionally substituted one, two or threetimes, each substituent independently selected from a fluorine atom or a group selected from cyano, methyl and trifluoromethyl,

and stereoisomers, tautomers, N-oxides, hydrates, solvates, and salts thereof, and mixtures of same.

In a further embodiment of the first aspect, the present invention covers compounds of formula (I)*, supra,* in which:

$R^{10}$ and $R^{11}$ represent, independently from each occurrence, a hydrogen atom or a group selected from $C_1$-$C_2$-alkyl, $C_3$-$C_7$-cycloalkyl and (benzyl)-O-C(=O)-,

wherein $C_3$-$C_7$-cycloalkyl is optionally substituted one or two times, each substituent independently selected from a fluorine atom or a group selected from methyl and trifluoromethyl,

and wherein the phenyl group within said (benzyl)-O-C(=O)- group is optionally substituted one or two times, each substituent independently selected from a fluorine atom, a chlorine atom and a methyl group,

and stereoisomers, tautomers, N-oxides, hydrates, solvates, and salts thereof, and mixtures of same.

In a further embodiment of the first aspect, the present invention covers compounds of formula (I)*, supra,* in which:

$R^{10}$ and $R^{11}$, together with the nitrogen atom to which they are attached, represent a monocyclic nitrogen containing 4- to 7-membered heterocycloalkyl group which is optionally substituted one, two or threetimes, each substituent independently selected from a fluorine atom or a group selected from cyano, methyl

and trifluoromethyl,

and stereoisomers, tautomers, N-oxides, hydrates, solvates, and salts thereof, and mixtures of same.

In a further embodiment of the first aspect, the present invention covers compounds of formula (I)*, supra,* in which:

$R^{15}$ and $R^{16}$ represent, independently from each occurrence, a hydrogen atom or a group selected from $C_1$-$C_4$-alkyl, $C_1$-$C_4$-haloalkyl, $C_1$-$C_4$-hydroxyalkyl, (cyano)-$C_1$-$C_4$-alkyl-, ($C_1$-$C_4$-alkoxy)-$C_2$-$C_3$-alkyl-, ($C_1$-$C_4$-haloalkoxy)-$C_2$-$C_3$-alkyl-, ($C_3$-$C_7$-cycloalkyl)-($C_1$-$C_3$-alkyl)-, $C_3$-$C_7$-cycloalkyl, bicyclic $C_5$-$C_{11}$-cycloalkyl, monocyclic 4- to 7-membered heterocycloalkyl, (phenyl)-($C_1$-$C_3$-alkyl)-, phenyl and a 5- or 6-membered heteroaryl group, wherein $C_3$-$C_7$-cycloalkyl, the $C_3$-$C_7$-cycloalkyl within said ($C_3$-$C_7$-cycloalkyl)-($C_1$-$C_3$-alkyl)- group, the bicyclic $C_5$-$C_{11}$-cycloalkyl and the monocyclic 4- to 7-membered heterocycloalkyl groups are optionally substituted one or two times, each substituent independently selected from a halogen atom or a group selected from oxo, $C_1$-$C_2$-alkyl, ($C_1$-$C_2$-alkyl)-C(=O)- and cyclopropyl, and wherein the phenyl and the 5- or 6-membered heteroaryl groups, including the phenyl group within said (phenyl)-($C_1$-$C_3$-alkyl)- group, are optionally substituted one or two times, each substituent independently selected from a fluorine atom, a chlorine atom and a bromine atom, or a group selected from methyl, trifluoromethyl and methoxy, or

$R^{15}$ and $R^{16}$, together with the nitrogen atom to which they are attached, represent a monocyclic nitrogen containing 4- to 7-membered heterocycloalkyl group which is optionally substituted one or two times, each substituent independently selected from a halogen atom or a group selected from oxo, $C_1$-$C_2$-alkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I)*, supra,* in which:

$R^{1S}$ and $R^{16}$ represent, independently from each occurrence, a hydrogen atom or a group selected from $C_1$-$C_4$-alkyl, $C_1$-$C_4$-haloalkyl, $C_1$-$C_4$-hydroxyalkyl, (cyano)-$C_1$-$C_4$-alkyl-, ($C_1$-$C_4$-alkoxy)-$C_2$-$C_3$-alkyl-, ($C_1$-$C_4$-haloalkoxy)-$C_2$-$C_3$-alkyl-, ($C_3$-$C_7$-cycloal kyl)-($C_1$-$C_3$-alkyl)-, $C_3$-$C_7$-cycloalkyl, bicyclic $C_3$-$C_{11}$-cycloalkyl, monocyclic 4- to 7-membered heterocycloalkyl, (phenyl)-($C_1$-$C_3$-alkyl)-, phenyl and a 5- or 6-membered heteroaryl group, wherein $C_3$-$C_7$-cycloalkyl, the $C_3$-$C_7$-cycloalkyl within said ($C_3$-$C_7$-cycloalkyl)-($C_1$-$C_3$-alkyl)- group, the bicyclic $C_3$-$C_{11}$-cycloalkyl and the monocyclic 4- to 7-membered heterocycloalkyl groups are optionally substituted one or two times, each substituent independently selected from a halogen atom or a group selected from oxo, $C_1$-$C_2$-alkyl, ($C_1$-$C_2$-alkyl)-C(=O)- and cyclopropyl, and wherein the phenyl and the 5- or 6-membered heteroaryl groups, including the phenyl group within said (phenyl)-($C_1$-$C_3$-alkyl)- group, are optionally substituted one or two times, each substituent independently selected from a fluorine atom, a chlorine atom and a bromine atom, or a group selected from methyl, trifluoromethyl and methoxy,

and stereoisomers, tautomers, N-oxides, hydrates, solvates, and salts thereof, and mixtures of same.

In a further embodiment of the first aspect, the present invention covers compounds of formula *(I), supra,* in which:

$R^{15}$ and $R^{16}$, together with the nitrogen atom to which they are attached, represent a monocyclic nitrogen containing 4- to 7-membered heterocycloalkyl group which is optionally substituted one or two times, each substituent independently selected from a halogen atom or a group selected from oxo, $C_1$-$C_2$-alkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I)*, supra,* in which:

$R^{15}$ and $R^{16}$ represent, independently from each occurrence, a hydrogen atom or a group selected from $C_1$-$C_4$-alkyl, $C_1$-$C_4$-haloalkyl, $C_1$-$C_4$-hydroxyalkyl, ($C_1$-$C_2$-alkoxy)-$C_2$-$C_3$-alkyl-, $C_3$-$C_7$-cycloalkyl, monocyclic 4- to 7-membered heterocycloalkyl and (phenyl)-($C_1$-$C_3$-alkyl)-, wherein $C_3$-$C_7$-cycloalkyl and monocyclic 4- to 7-membered heterocycloalkyl are optionally substitueted one or two times, each substituent independently selected from a halogen atom or a group selected from oxo, $C_1$-$C_2$-alkyl and ($C_1$-$C_2$-alkyl)-C(=O)-, or

$R^{15}$ and $R^{16}$, together with the nitrogen atom to which they are attached, represent a monocyclic nitrogen containing 4- to 7-membered heterocycloalkyl group which is optionally substituted one or two times, each substituent independently selected from a halogen atom or a group selected from oxo, $C_1$-$C_2$-alkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$ represent, independently from each occurrence, a hydrogen atom or a group selected from $C_1$-$C_4$-alkyl, $C_1$-$C_4$-haloalkyl, $C_1$-$C_4$-hydroxyalkyl, ($C_1$-$C_2$-alkoxy)-$C_2$-$C_3$-alkyl-, $C_3$-$C_7$-cycloalkyl, monocyclic 4- to 7-membered heterocycloalkyl and (phenyl)-($C_1$-$C_3$-alkyl)-, wherein $C_3$-$C_7$-cycloalkyl and monocyclic 4- to 7-membered heterocycloalkyl are optionally substitueted one or two times, each substituent independently selected from a halogen atom or a group selected from oxo, $C_1$-$C_2$-alkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$, together with the nitrogen atom to which they are attached, represent a monocyclic nitrogen containing 4- to 7-membered heterocycloalkyl group which is optionally substituted one or two times, each substituent independently selected from a halogen atom or a group selected from oxo, $C_1$-$C_2$-alkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$ represent, independently from each occurrence, a hydrogen atom or a group selected from $C_1$-$C_4$-alkyl, $C_1$-$C_3$-fluoroalkyl, (cyano)-$C_1$-$C_2$-alkyl-, ($C_1$-$C_4$-alkoxy)-$C_2$-$C_3$-alkyl-, ($C_1$-$C_2$-fluoroalkoxy)-$C_2$-$C_3$-alkyl-, ($C_3$-$C_5$-cycloalkyl)-($C_1$-$C_2$-alkyl)-, $C_3$-$C_5$-cycloalkyl, phenyl and a 5- or 6-membered heteroaryl group selected from pyrazolyl, pyridinyl and pyrimidinyl, wherein $C_3$-$C_5$-cycloalkyl, and the $C_3$-$C_5$-cycloalkyl within said ($C_3$-$C_5$-cycloalkyl)-($C_1$-$C_2$-alkyl)- group are optionally substituted one or two times, each substituent independently selected from a fluorine atom, or a group selected from methyl and cyclopropyl, and wherein the phenyl and the 5- or 6-membered heteroaryl groups selected from pyrazolyl, pyridinyl and pyrimidinyl are optionally substituted one or two times, each substituent independently selected from a fluorine atom and a chlorine atom, or a group selected from methyl, trifluoromethyl and methoxy, or

$R^{15}$ and $R^{16}$, together with the nitrogen atom to which they are attached, represent a monocyclic nitrogen containing 4- to 7-membered heterocycloalkyl group which is optionally substituted once with a fluorine atom or a group selected from oxo, $C_1$-$C_2$-alkyl and $CH_3$-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$ represent, independently from each occurrence, a hydrogen atom or a group selected from $C_1$-$C_4$-alkyl, $C_1$-$C_3$-fluoroalkyl, (cyano)-$C_1$-$C_2$-alkyl-, ($C_1$-$C_4$-alkoxy)-$C_2$-$C_3$-alkyl-, ($C_1$-$C_2$-fluoroalkoxy)-$C_2$-$C_3$-alkyl-, ($C_3$-$C_5$-cycloalkyl)-($C_1$-$C_2$-alkyl)-, $C_3$-$C_5$-cycloalkyl, phenyl and a 5- or 6-membered heteroaryl group selected from pyrazolyl, pyridinyl and pyrimidinyl, wherein $C_3$-$C_5$-cycloalkyl, and the $C_3$-$C_5$-cycloalkyl within said ($C_3$-$C_5$-cycloalkyl)-($C_1$-$C_2$-alkyl)- group are optionally substituted one or two times, each substituent independently selected from a fluorine atom, or a group selected from methyl and cyclopropyl, and wherein the phenyl and the 5- or 6-membered heteroaryl groups selected from pyrazolyl, pyridinyl and pyrimidinyl are optionally substituted one or two times, each substituent independently selected from a fluorine atom and a chlorine atom, or a group selected from methyl, trifluoromethyl and methoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$, together with the nitrogen atom to which they are attached, represent a monocyclic nitrogen containing 4- to 7-membered heterocycloalkyl group which is optionally substituted once with a fluorine atom or a group selected from oxo, $C_1$-$C_2$-alkyl and $CH_3$-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$ represent, independently from each occurrence, a hydrogen atom or a group selected from $C_1$-$C_3$-alkyl, $C_1$-$C_3$-fluoroalkyl and $C_3$-$C_5$-cycloalkyl, or

$R^{15}$ and $R^{16}$, together with the nitrogen atom to which they are attached, represent a monocyclic nitrogen containing 4- to 7-membered heterocycloalkyl group which is optionally substituted once with a fluorine atom or a group selected from oxo, $C_1$-$C_2$-alkyl and $CH_3$-C(=O)-;

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$ represent, independently from each occurrence, a hydrogen atom or a group selected from $C_1$-$C_3$-alkyl, $C_1$-$C_3$-fluoroalkyl and $C_3$-$C_5$-cycloalkyl,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$, together with the nitrogen atom to which they are attached, represent a monocyclic nitrogen containing 4- to 7-membered heterocycloalkyl group which is optionally substituted once with a fluorine atom or a group selected from oxo, $C_1$-$C_2$-alkyl and $CH_3$-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$, together with the nitrogen atom to which they are attached, represent a monocyclic nitrogen containing 4- to 7-membered heterocycloalkyl group which is optionally substituted once with a group selected from oxo, methyl and $CH_3$-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$ represent, independently from each occurrence, a hydrogen atom or a group selected from $C_1$-$C_4$-alkyl, $C_1$-$C_2$-fluoroalkyl, ($C_1$-$C_4$-alkoxy)-$C_2$-$C_3$-alkyl-, ($C_1$-$C_2$-fluoroalkoxy)-$C_2$-$C_3$-alkyl-, ($C_3$-$C_5$-cycloalkyl)-($C_1$-$C_2$-alkyl)-, $C_3$-$C_5$-cycloalkyl, phenyl and pyridinyl, wherein $C_3$-$C_5$-cycloalkyl, and the $C_3$-$C_5$-cycloalkyl within said ($C_3$-$C_5$-cycloalkyl)-($C_1$-$C_2$-alkyl)- group are optionally substituted one or two times, each substituent independently selected from a fluorine atom and a methyl group, and wherein the phenyl and pyridinyl groups are optionally substituted one or two times, each substituent independently selected from a fluorine atom and a chlorine atom, or a group selected from methyl, trifluoromethyl and methoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$ represent, independently from each occurrence, a hydrogen atom or a group selected from $C_1$-$C_4$-alkyl, $C_1$-$C_2$-fluoroalkyl, ($C_1$-$C_4$-alkoxy)-$C_2$-alkyl-, ($C_1$-$C_2$-fluoroalkoxy)-$C_2$-alkyl-, $C_3$-$C_5$-cycloalkyl, phenyl and pyridinyl, wherein $C_3$-$C_5$-cycloalkyl is optionally substituted one or two times, each substituent independently selected from a fluorine atom and a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{15}$ and $R^{16}$ represent, independently from each occurrence, a hydrogen atom or a group selected from $C_1$-$C_2$-alkyl, $C_3$-$C_5$-cycloalkyl, phenyl and pyridinyl, wherein $C_3$-$C_5$-cycloalkyl is optionally substituted one or two times, each substituent independently selected from a fluorine atom and a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{17}$ represents a $C_1$-$C_4$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{17}$ represents a $C_1$-$C_3$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{17}$ represents a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{17}$ represents a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{17}$ represents an ethyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{18}$ and $R^{19}$ represent, independently from each occurrence, a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:
$R^{18}$ and $R^{19}$ represent, independently from each occurrence, a hydrogen atom or a methyl group, and stereoisomers, tautomers, N-oxides, hydrates, solvates, and salts thereof, and mixtures of same.

In a further embodiment of the first aspect, the present invention covers compounds of formula (I), *supra,* in

which:

$R^{18}$ and $R^{19}$ both represent a methyl group,
and stereoisomers, tautomers, N-oxides, hydrates, solvates, and salts thereof, and mixtures of same.

In a further embodiment of the first aspect, the present invention covers compounds of formula (I), *supra,* in which:

$R^{18}$ and $R^{19}$ both represent a hydrogen atom,

and stereoisomers, tautomers, N-oxides, hydrates, solvates, and salts thereof, and mixtures of same.

In a further embodiment of the first aspect, the present invention covers compounds of formula (I), *supra,* in which:

$R^{18}$ represents a hydrogen atom and $R^{19}$ represents a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from $C_1$-$C_6$-alkyl, $C_3$-$C_4$-alkenyl, $C_3$-$C_4$-alkynyl, $C_1$-$C_3$-alkoxy, $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, naphthyl, and 5- to 10-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a bromine atom, or a group selected from methyl, trifluoromethyl and methoxy,

wherein $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl are optionally substituted one or two or three times, each substituent independently selected from a halogen atom or a group selected from cyano, oxo, hydroxy, $C_1$-$C_2$-alkyl and ($C_1$-$C_2$-alkyl)-C(=O)-,

and wherein said phenyl, naphthyl and 5- to 10-membered heteroaryl groups are optionally substituted one, two or three times, each substituent independently selected from a halogen atom or a group selected from cyano, $C_1$-$C_2$-alkyl, $C_1$-$C_2$-haloalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group,
or

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-haloalkyl, (phenyl)-($C_1$-$C_2$-alkyl)-, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$);

$R^{22}$ and $R^{23}$ represent, independently from each occurrence, a hydrogen atom or a group selected from $C_1$-$C_2$-alkyl and ($C_1$-$C_2$-alkyl)-C(=O)-;

$R^{24}$ and $R^{25}$ represent, independently from each occurrence, a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from $C_1$-$C_6$-alkyl, $C_3$-$C_4$-alkenyl, $C_3$-$C_4$-alkynyl, $C_1$-$C_3$-alkoxy, $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, naphthyl, and 5- to 10-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a bromine atom, or a group selected from methyl, trifluoromethyl and methoxy,

wherein $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl are optionally substituted one or two or three times, each substituent independently selected from a halogen atom or a group selected from cyano, oxo, hydroxy, $C_1$-$C_2$-alkyl and ($C_1$-$C_2$-alkyl)-C(=O)-,

and wherein said phenyl, naphthyl and 5- to 10-membered heteroaryl groups are optionally substituted one, two or three times, each substituent independently selected from a halogen atom or a group selected from cyano, $C_1$-$C_2$-alkyl, $C_1$-$C_2$-haloalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group,

$R^{22}$ and $R^{23}$ represent, independently from each occurrence, a hydrogen atom or a group selected from $C_1$-$C_2$-alkyl and ($C_1$-$C_2$-alkyl)-C(=O)-;

$R^{24}$ and $R^{25}$ represent, independently from each occurrence, a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-haloalkyl, (phenyl)-($C_1$-$C_2$-alkyl)-, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$);

$R^{22}$ and $R^{23}$ represent, independently from each occurrence, a hydrogen atom or a group selected from $C_1$-$C_2$-alkyl and ($C_1$-$C_2$-alkyl)-C(=O)-;

$R^{24}$ and $R^{25}$ represent, independently from each occurrence, a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from $C_1$-$C_6$-alkyl, $C_3$-$C_7$-cycloalkyl, monocyclic 4- to 7-membered heterocycloalkyl, phenyl, and 5- to 6-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, monocyclic 4- to 7-membered heterocycloalkyl, phenyl, and 5- to 6-membered heteroaryl, said phenyl and 5- to 6-membered heteroaryl substituents themselves being optionally substituted one or two times, each substituent independently selected from a fluorine atom, a chlorine atom and a bromine atom, or a group selected from methyl, trifluoromethyl and methoxy,

and wherein $C_3$-$C_7$-cycloalkyl and monocyclic 4- to 7-membered heterocycloalkyl, are optionally substituted one or two or three times, each substituent independently selected from a halogen atom or a group selected from cyano, oxo, hydroxy, $C_1$-$C_2$-alkyl and ($C_1$-$C_2$-alkyl)-C(=O)-,

and wherein said phenyl and 5- to 6-membered heteroaryl groups are optionally substituted one, two or three times, each substituent independently selected from a halogen atom or a group selected from cyano, $C_1$-$C_2$-alkyl, $C_1$-$C_2$-haloalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group,

or

$R^{20}$ and $R^{21}$, together with the nitrogen atom to which they are attached, represent a monocyclic nitrogen containing 4- to 7-membered heterocycloalkyl group which is substituted one, two or three times, each substituent independently selected from a halogen atom or a group selected from cyano, oxo, hydroxy, $C_1$-$C_2$-alkyl, $C_1$-$C_2$-haloalkyl, (phenyl)-($C_1$-$C_2$-alkyl)-, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$),

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from $C_1$-$C_6$-alkyl, $C_3$-$C_7$-cycloalkyl, monocyclic 4- to 7-membered heterocycloalkyl, phenyl, and 5- to 6-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, monocyclic 4- to 7-membered heterocycloalkyl, phenyl, and 5- to 6-membered heteroaryl, said phenyl and 5- to 6-membered heteroaryl substituents themselves being optionally substituted one or two times, each substituent independently selected from a fluorine atom, a chlorine atom and a bromine atom, or a group selected from methyl, trifluoromethyl and methoxy,

and wherein $C_3$-$C_7$-cycloalkyl and monocyclic 4- to 7-membered heterocycloalkyl, are optionally substituted one or two or three times, each substituent independently selected from a halogen atom or a group selected from cyano, oxo, hydroxy, $C_1$-$C_2$-alkyl and ($C_1$-$C_2$-alkyl)-C(=O)-,

and wherein said phenyl and 5- to 6-membered heteroaryl groups are optionally substituted one, two or three times, each substituent independently selected from a halogen atom or a group selected from cyano, $C_1$-$C_2$-alkyl, $C_1$-$C_2$-haloalkyl, $C_1$-$C_2$-alkoxy, $C_1$-$C_2$-haloalkoxy, -N($R^{22}$)($R^{23}$) and -C(=O)-N($R^{24}$)($R^{25}$), and in which

$R^{21}$ represents a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ and $R^{21}$, together with the nitrogen atom to which they are attached, represent a monocyclic nitrogen containing 4- to 7-membered heterocycloalkyl group which is substituted one, two or three times, each substituent independently selected from a halogen atom or a group selected from cyano, oxo, hydroxy, $C_1$-$C_2$-alkyl, $C_1$-$C_2$-haloalkyl, (phenyl)-($C_1$-$C_2$-alkyl)-, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$),

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from $C_1$-$C_6$-alkyl, $C_3$-$C_4$-alkenyl, $C_3$-$C_4$-alkynyl, $C_1$-$C_3$-alkoxy, $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, naphthyl, and 5- to 10-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a bromine atom, or a group selected from methyl, trifluoromethyl and methoxy,

wherein $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl are optionally substituted one or two or three times, each substituent independently selected from a halogen atom or a group selected from cyano, oxo, hydroxy, $C_1$-$C_2$-alkyl and ($C_1$-$C_2$-alkyl)-C(=O)-,

and wherein said phenyl, naphthyl and 5- to 10-membered heteroaryl groups are optionally substituted one, two or three times, each substituent independently selected from a halogen atom or a group selected from cyano, $C_1$-$C_2$-alkyl, $C_1$-$C_2$-haloalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group,
or

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-haloalkyl, (phenyl)-($C_1$-$C_2$-alkyl)-, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$);

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from $C_1$-$C_6$-alkyl, $C_3$-$C_4$-alkenyl, $C_3$-$C_4$-alkynyl, $C_1$-$C_3$-alkoxy, $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, naphthyl, and 5- to 10-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a bromine atom, or a group selected from methyl, trifluoromethyl and methoxy,

wherein $C_3$-$C_7$-cycloalkyl, bicyclic $C_6$-$C_{11}$-cycloalkyl, adamantyl, monocyclic 4- to 7-membered heterocycloalkyl, bicyclic 6- to 10-membered heterocycloalkyl are optionally substituted one or two or three times, each substituent independently selected from a halogen atom or a group selected from cyano, oxo, hydroxy, $C_1$-$C_2$-

alkyl and $(C_1$-$C_2$-alkyl)-C(=O)-,

and wherein said phenyl, naphthyl and 5- to 10-membered heteroaryl groups are optionally substituted one, two or three times, each substituent independently selected from a halogen atom or a group selected from cyano, $C_1$-$C_2$-alkyl, $C_1$-$C_2$-haloalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-haloalkyl, (phenyl)-($C_1$-$C_2$-alkyl)-, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$);

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from $C_1$-$C_3$-alkyl and phenyl, wherein said $C_1$-$C_3$-alkyl group is optionally substituted one or two times, each substituent independently selected from a fluorine atom or a group selected from hydroxy, $C_1$-$C_3$-alkoxy and phenyl, said phenyl itself being optionally substituted one or two times, each substituent independently selected from a fluorine atom, a chlorine atom and a methyl group, and wherein said phenyl group is optionally substituted one, two or three times, each substituent independently selected from a fluorine atom and a chlorine atom or a group selected from methyl, trifluoromethyl, methoxy and trifluoromethoxy,

$R^{21}$ represents a hydrogen atom or a $C_1$-$C_2$-alkyl group; or

$R^{20}$ and $R^{21}$, together with the nitrogen atom to which they are attached, represent a monocyclic nitrogen containing 4- to 7-membered heterocycloalkyl group which is optionally substituted one or two times, each substituent independently selected from a halogen atom or a group selected from cyano, oxo, hydroxy, $C_1$-$C_2$-alkyl, $C_1$-$C_2$-fluoroalkyl, benzyl, ($C_1$-$C_2$-alkyl)-C(=O)- and $C_3$-$C_4$-cycloalkyl,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from $C_1$-$C_3$-alkyl and phenyl, wherein said $C_1$-$C_3$-alkyl group is optionally substituted one or two times, each substituent independently selected from a fluorine atom or a group selected from hydroxy, $C_1$-$C_3$-alkoxy and phenyl, said phenyl itself being optionally substituted one or two times, each substituent independently selected from a fluorine atom, a chlorine atom and a methyl group, and wherein said phenyl group is optionally substituted one, two or three times, each substituent independently selected from a fluorine atom and a chlorine atom or a group selected from methyl, trifluoromethyl, methoxy and trifluoromethoxy, and in which

$R^{21}$ represents a hydrogen atom or a $C_1$-$C_2$-alkyl group;

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ and $R^{21}$, together with the nitrogen atom to which they are attached, represent a monocyclic nitrogen containing 4- to 7-membered heterocycloalkyl group which is optionally substituted one or two times, each substituent independently selected from a halogen atom or a group selected from cyano, oxo, hydro-

xy, $C_1$-$C_2$-alkyl, $C_1$-$C_2$-fluoroalkyl, benzyl, ($C_1$-$C_2$-alkyl)-C(=O)- and $C_3$-$C_4$-cycloalkyl,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a group selected from benzyl and phenyl, wherein said phenyl group, and the phenyl group within said benzyl group, is optionally substituted one or two times, each substituent independently selected from a fluorine atom, a chlorine atom and a methyl group, and in which

$R^{21}$ represents a hydrogen atom or a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from optionally substituted $C_1$-$C_3$-alkyl, unsubstituted $C_4$-$C_6$-alkyl, prop-2-ynyl, methoxy, $C_3$-$C_6$-cycloalkyl, adamantyl, monocyclic 4- to 7-membered heterocycloalkyl, phenyl, and 5- to 10-membered heteroaryl,

wherein said $C_1$-$C_3$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_6$-cycloalkyl, adamantyl, monocyclic 4- to 7-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a methyl group,

wherein said $C_3$-$C_6$-cycloalkyl, adamantyl and monocyclic 4- to 7-membered heterocycloalkyl groups are optionally substituted one or two or three times, each substituent independently selected from a fluorine atom or a group selected from oxo, $C_1$-$C_2$-alkyl and ($C_1$-$C_2$-alkyl)-C(=O)-,

and wherein said phenyl and 5- to 10-membered heteroaryl groups are optionally substituted one, two or three times, each substituent independently selected from a fluorine atom and a chlorine atom or a group selected from cyano, $C_1$-$C_2$-alkyl, $C_1$-$C_2$-fluoroalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group, or

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-fluoroalkyl, benzyl, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$);

$R^{22}$ and $R^{23}$ represent, independently from each occurrence, a hydrogen atom or a group selected from $C_1$-$C_2$-alkyl and ($C_1$-$C_2$-alkyl)-C(=O)-;
$R^{24}$ and $R^{25}$ represent, independently from each occurrence, a hydrogen atom or a $C_1$-$C_2$-alkyl group,
and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from optionally substituted $C_1$-$C_3$-alkyl, unsubstituted $C_4$-$C_6$-alkyl, prop-2-ynyl, methoxy, $C_3$-$C_6$-cycloalkyl, adamantyl, monocyclic 4- to 7-membered heterocycloalkyl, phenyl, and 5- to 10-membered heteroaryl, wherein said $C_1$-$C_3$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_6$-cycloalkyl, adamantyl, monocyclic 4- to 7-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a methyl group, wherein said $C_3$-$C_6$-cycloalkyl, adamantyl and monocyclic 4- to 7-membered heterocycloalkyl groups are optionally substituted one or two or three times, each substituent independently selected from a fluorine atom or a group selected from oxo, $C_1$-$C_2$-alkyl and ($C_1$-$C_2$-alkyl)-C(=O)-, and wherein said phenyl and 5- to 10-membered heteroaryl groups are optionally substituted one, two or three times, each substituent independently selected from a fluorine atom and a chlorine atom or a group selected from cyano, $C_1$-$C_2$-alkyl, $C_1$-$C_2$-fluoroalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group,

$R^{22}$ and $R^{23}$ represent, independently from each occurrence, a hydrogen atom or a group selected from $C_1$-$C_2$-alkyl and ($C_1$-$C_2$-alkyl)-C(=O)-;

$R^{24}$ and $R^{25}$ represent, independently from each occurrence, a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I)*, supra,* in which:

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-fluoroalkyl, benzyl, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$);

$R^{22}$ and $R^{23}$ represent, independently from each occurrence, a hydrogen atom or a group selected from $C_1$-$C_2$-alkyl and ($C_1$-$C_2$-alkyl)-C(=O)-;

$R^{24}$ and $R^{25}$ represent, independently from each occurrence, a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I)*, supra,* in which:

$R^{20}$ represents a hydrogen atom or a group selected from optionally substituted $C_1$-$C_3$-alkyl, unsubstituted $C_4$-$C_6$-alkyl, prop-2-ynyl, methoxy, $C_3$-$C_6$-cycloalkyl, adamantyl, monocyclic 4- to 7-membered heterocycloalkyl, phenyl, and 5- to 10-membered heteroaryl,

wherein said $C_1$-$C_3$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_6$-cycloalkyl, adamantyl, monocyclic 4- to 7-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a methyl group,

wherein said $C_3$-$C_6$-cycloalkyl, adamantyl and monocyclic 4- to 7-membered heterocycloalkyl groups are optionally substituted one or two or three times, each substituent independently selected from a fluorine atom or a group selected from oxo, $C_1$-$C_2$-alkyl and ($C_1$-$C_2$-alkyl)-C(=O)-,

and wherein said phenyl and 5- to 10-membered heteroaryl groups are optionally substituted one, two or three times, each substituent independently selected from a fluorine atom and a chlorine atom or a group selected from cyano, $C_1$-$C_2$-alkyl, $C_1$-$C_2$-fluoroalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group, or

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-fluoroalkyl, benzyl, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$),

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ represents a hydrogen atom or a group selected from optionally substituted $C_1$-$C_3$-alkyl, unsubstituted $C_4$-$C_6$-alkyl, prop-2-ynyl, methoxy, $C_3$-$C_6$-cycloalkyl, adamantyl, monocyclic 4- to 7-membered heterocycloalkyl, phenyl, and 5- to 10-membered heteroaryl,

wherein said $C_1$-$C_3$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_6$-cycloalkyl, adamantyl, monocyclic 4- to 7-membered heterocycloalkyl, phenyl, and 5- to 10-membered heteroaryl, said phenyl and 5- to 10-membered heteroaryl substituents themselves being optionally substituted one or two times, each substituent independently selected from a fluorine atom, a chlorine atom and a methyl group,

wherein said $C_3$-$C_6$-cycloalkyl, adamantyl and monocyclic 4- to 7-membered heterocycloalkyl groups are optionally substituted one or two or three times, each substituent independently selected from a fluorine atom or a group selected from oxo, $C_1$-$C_2$-alkyl and ($C_1$-$C_2$-alkyl)-C(=O)-,

and wherein said phenyl and 5- to 10-membered heteroaryl groups are optionally substituted one, two or three times, each substituent independently selected from a fluorine atom and a chlorine atom or a group selected from cyano, $C_1$-$C_2$-alkyl, $C_1$-$C_2$-fluoroalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_2$-alkyl, $C_1$-$C_2$-fluoroalkyl, benzyl, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$),

and stereoisomers, tautomers, N-oxides, hydrates, solvates, and salts thereof, and mixtures of same.

In a further embodiment of the first aspect, the present invention covers compounds of formula (I), *supra,* in which:

$R^{22}$ and $R^{23}$ represent, independently from each occurrence, a hydrogen atom or a group selected from $C_1$-$C_2$-alkyl and ($C_1$-$C_2$-alkyl)-C(=O)-,

and stereoisomers, tautomers, N-oxides, hydrates, solvates, and salts thereof, and mixtures of same.

In a further embodiment of the first aspect, the present invention covers compounds of formula (I), *supra,* in which:

$R^{22}$ and $R^{23}$ represent, independently from each occurrence, a hydrogen atom or a group selected from methyl and ($CH_3$)-C(=O)-,

and stereoisomers, tautomers, N-oxides, hydrates, solvates, and salts thereof, and mixtures of same.

In a further embodiment of the first aspect, the present invention covers compounds of formula (I), *supra,* in

which:

$R^{24}$ and $R^{25}$ represent, independently from each occurrence, a hydrogen atom or a $C_1$-$C_2$-alkyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{24}$ and $R^{25}$ represent, independently from each occurrence, a hydrogen atom or a methyl group,

and stereoisomers, tautomers, N-oxides, hydrates, solvates, and salts thereof, and mixtures of same.

In a further embodiment of the first aspect, the present invention covers compounds of formula (I), *supra,* in which:

$R^{26}$ represents a fluorine atom, a chlorine atom, or a group selected from $C_1$-$C_2$-alkyl, $C_1$-$C_2$-fluoroalkyl, $C_1$-$C_2$-alkoxy and $C_1$-$C_2$-fluoroalkoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^{26}$ represents a fluorine atom, a chlorine atom, or a group selected from methyl, difluoromethyl, trifluoromethyl, methoxy, difluoromethoxy and trifluoromethoxy,

and stereoisomers, tautomers, N-oxides, hydrates, solvates, and salts thereof, and mixtures of same.

In a further embodiment of the first aspect, the present invention covers compounds of formula (I), *supra,* in which:

$R^{26}$ represents a fluorine atom, a chlorine atom, or a group selected from difluoromethyl, trifluoromethyl, methoxy, difluoromethoxy and trifluoromethoxy,

and stereoisomers, tautomers, N-oxides, hydrates, solvates, and salts thereof, and mixtures of same.

In a further embodiment of the first aspect, the present invention covers compounds of formula (I), *supra,* in which:

$Y^1$ **represents** -C(H)=, -C(F)=, -C(Cl)=, -C(CN)= or -N=,

and stereoisomers, tautomers, N-oxides, hydrates, solvates, and salts thereof, and mixtures of same.

In a further embodiment of the first aspect, the present invention covers compounds of formula (I), *supra,* in which:

$Y^1$ **represents** -C(H)=, -C(F)= or -N=,

and stereoisomers, tautomers, N-oxides, hydrates, solvates, and salts thereof, and mixtures of same.

In a further embodiment of the first aspect, the present invention covers compounds of formula (I), *supra,* in which:

$Y^1$ **represents** -C(H)= or -C(F)=,

and stereoisomers, tautomers, N-oxides, hydrates, solvates, and salts thereof, and mixtures of same.

In a further embodiment of the first aspect, the present invention covers compounds of formula (I), *supra,* in which:

$Y^1$ **represents** -C(H)=,

and stereoisomers, tautomers, N-oxides, hydrates, solvates, and salts thereof, and mixtures of same.

In a further embodiment of the first aspect, the present invention covers compounds of formula (I)*, supra,* in which:

$Y^1$ **represents** -C(F)=,

and stereoisomers, tautomers, N-oxides, hydrates, solvates, and salts thereof, and mixtures of same.

In a further embodiment of the first aspect, the present invention covers compounds of formula (I)*, supra,* in which:

$Y^1$ **represents** -N=,

and stereoisomers, tautomers, N-oxides, hydrates, solvates, and salts thereof, and mixtures of same.

In a particular further embodiment of the first aspect, the present invention covers combinations of two or more of the above mentioned embodiments under the heading "further embodiments of the first aspect of the present invention".

The present invention covers any sub-combination within any embodiment or aspect of the present invention of compounds of general formula (I)*, supra.*

The present invention covers the compounds of general formula (I) which are disclosed in the Example Section of this text, *infra.*

The compounds of general formula (I) of the present invention can be converted to any salt, preferably pharmaceutically acceptable salts, as described herein, by any method which is known to the person skilled in the art. Similarly, any salt of a compound of general formula (I) of the present invention can be converted into the free compound, by any method which is known to the person skilled in the art.

Compounds of general formula (I) of the present invention demonstrate a valuable pharmacological spectrum of action, which could not have been predicted. Compounds of the present invention have surprisingly been found to effectively inhibit DGKζ and it is possible therefore that said compounds be used for the treatment or prophylaxis of diseases, preferably conditions with dysregulated immune responses, particularly cancer or other disorders associated with aberrant DGKζ signaling, in mammals, including humans.

Disorders and conditions particularly suitable for treatment with an DGKζ inhibitor of the present invention are liquid and solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancers include, but are not limited to, triple negative breast cancer, invasive ductal carcinoma, invasive lobular carcinoma, ductal carcinoma *in situ,* and lobular carcinoma *in situ.*

Examples of cancers of the respiratory tract include, but are not limited to, small-cell and non-small-cell lung carcinoma, as well as bronchial adenoma and pleuropulmonary blastoma.

Examples of brain cancers include, but are not limited to, brain stem and hypophtalmic glioma, cerebellar and cerebral astrocytoma, glioblastoma, medulloblastoma, ependymoma, as well as neuroectodermal and pineal tumour.

Tumours of the male reproductive organs include, but are not limited to, prostate and testicular cancer.

Tumours of the female reproductive organs include, but are not limited to, endometrial, cervical, ovarian, vaginal, and vulvar cancer, as well as sarcoma of the uterus.

Examples of ovarian cancer include, but are not limited to serous tumour, endometrioid tumour, mucinous cystadenocarcinoma, granulosa cell tumour, Sertoli-Leydig cell tumour and arrhenoblastoma.

Examples of cervical cancer include, but are not limited to squamous cell carcinoma, adenocarcinoma, adenosquamous carcinoma, small cell carcinoma, neuroendocrine tumour, glassy cell carcinoma and villoglandular adenocarcinoma.

Tumours of the digestive tract include, but are not limited to, anal, colon, colorectal, esophageal, gallbladder, gastric, pancreatic, rectal, small-intestine, and salivary gland cancers.

Examples of esophageal cancer include, but are not limited to esophageal cell carcinomas and adenocarcinomas, as well as squamous cell carcinomas, leiomyosarcoma, malignant melanoma, rhabdomyosarcoma and lymphoma.

Examples of gastric cancer include, but are not limited to intestinal type and diffuse type gastric adenocarcinoma.

Examples of pancreatic cancer include, but are not limited to ductal adenocarcinoma, adenosquamous carcinomas and pancreatic endocrine tumours.

Tumours of the urinary tract include, but are not limited to, bladder, penile, kidney, renal pelvis, ureter, urethral and human papillary renal cancers.

Examples of kidney cancer include, but are not limited to renal cell carcinoma, urothelial cell carcinoma, juxtaglomerular cell tumour (reninoma), angiomyolipoma, renal oncocytoma, Bellini duct carcinoma, clear-cell sarcoma

of the kidney, mesoblastic nephroma and Wilms' tumour. Examples of bladder cancer include, but are not limited to transitional cell carcinoma, squamous cell carcinoma, adenocarcinoma, sarcoma and small cell carcinoma.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, squamous cell carcinoma, Kaposi's sarcoma, malignant melanoma, Merkel cell skin cancer, and non-melanoma skin cancer.

Head-and-neck cancers include, but are not limited to, squamous cell cancer of the head and neck, laryngeal, hypopharyngeal, nasopharyngeal, oropharyngeal cancer, salivary gland cancer, lip and oral cavity cancer and squamous cell.

Lymphomas include, but are not limited to, AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to, sarcoma of the soft tissue, osteosarcoma, malignant fibrous histiocytoma, lymphosarcoma, and rhabdomyosarcoma.

Leukemias include, but are not limited to, acute myeloid leukemia, acute lymphoblastic leukemia, chronic lymphocytic leukemia, chronic myelogenous leukemia, and hairy cell leukemia.

The term "treating" or "treatment" as stated throughout this document is used conventionally, for example the management or care of a subject for the purpose of combating, alleviating, reducing, relieving, improving the condition of a disease or disorder, such as a carcinoma.

The compounds of the present invention can be used in particular in therapy and prevention, i.e. prophylaxis, of tumour growth and metastases, especially in solid tumours of all indications and stages with or without pre-treatment of the tumour growth.

Generally, the use of chemotherapeutic agents and/or anti-cancer agents in combination with a compound or pharmaceutical composition of the present invention will serve to:

1. yield better efficacy in reducing the growth of a tumour or even eliminate the tumour as compared to administration of either agent alone,
2. provide for the administration of lesser amounts of the administered chemotherapeutic agents,
3. provide for a chemotherapeutic treatment that is well tolerated in the patient with fewer deleterious pharmacological complications than observed with single agent chemotherapies and certain other combined therapies,
4. provide for treating a broader spectrum of different cancer types in mammals, especially humans,
5. provide for a higher response rate among treated patients,
6. provide for a longer survival time among treated patients compared to standard chemotherapy treatments,
7. provide a longer time for tumour progression, and/or
8. yield efficacy and tolerability results at least as good as those of the agents used alone, compared to known instances where other cancer agent combinations produce antagonistic effects.

In addition, the compounds of general formula (I) of the present invention can also be used in combination with radiotherapy and/or surgical intervention.

In a further disclosure, the compounds of general formula (I) of the present invention can be used in combination with radiation: *i.e.* radiation treatment sensitizes cancers to anti-tumor immune responses by induction of tumor cell death and subsequent presentation of tumor neoantigens to tumor-reactive Tcells. As DGKζ is enhancing the antigen specific activation of T cells, the overall effect results in a much stronger cancer cell attack as compared to irradiation treatment alone.

The present disclosure also provides a method of killing a tumor, wherein conventional radiation therapy is employed previous to administering one or more of the compounds of the present invention.

The compounds of the present invention can be administered as the sole pharmaceutical agent or in combination with one or more other pharmaceutically active ingredients where the combination causes no unacceptable adverse effects. The present invention also covers such pharmaceutical combinations. For example, the compounds of the present invention can be combined with:

131I-chTNT, abarelix, abemaciclib, abiraterone, acalabrutinib, aclarubicin, adalimumab, adotrastuzumab emtansine, afatinib, aflibercept, aldesleukin, alectinib, alemtuzumab, alendronic acid, alitretinoin, alpharadin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, apalutamide, aprepitant, arcitumomab, arglabin, arsenic trioxide, asparaginase, atezolizumab, avelumab, axicabtagene ciloleucel, axitinib, azacitidine, basiliximab, belotecan, bendamustine, besilesomab, belinostat, bevacizumab, bexarotene, bicalutamide, bisantrene, bleomycin, blinatumomab, bortezomib, bosutinib, buserelin, brentuximab vedotin, brigatinib, busulfan, cabazitaxel, cabozantinib, calcitonine,

calcium folinate, calcium levofolinate, capecitabine, capromab, carbamazepine carboplatin, carboquone, carfilzomib, carmofur, carmustine, catumaxomab, celecoxib, celmoleukin, cemiplimab, ceritinib, cetuximab, chlorambucil, chlormadinone, chlormethine, cidofovir, cinacalcet, cisplatin, cladribine, clodronic acid, clofarabine, cobimetinib, copanlisib, crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, durvalumab, eculizumab, edrecolomab, elimusertib (BAY1895344), elliptinium acetate, elotuzumab, eltrombopag, enasidenib, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, inotuzumab ozogamicin, interferon alfa, interferon beta, interferon gamma, iobitridol, iobenguane (123I), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, lutetium Lu 177 dotatate, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, midostaurin, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, mvasi, nabilone, nabiximols, nafarelin, naloxone + pentazocine, naltrexone, nartograstim, necitumumab, nedaplatin, nelarabine, neratinib, neridronic acid, netupitant/palonosetron, nivolumab, pentetreotide, nilotinib, nilutamide, nimorazole, nimotuzumab, nimustine, nintedanib, niraparib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib, regorafenib, ribociclib, risedronic acid, rhenium-186 etidronate, rituximab, rolapitant, romidepsin, romiplostim, romurtide, rucaparib, samarium (153Sm) lexidronam, sargramostim, sarilumab, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tisagenlecleucel, tislelizumab, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib, valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

The compounds of the invention can further be combined with other reagents targeting the immune system, such as immune checkpoint inhibitors, e.g. aPD-1/-L1 axis antagonists. PD-1, along with its ligands PD-L1 and PD-L2, function as negative regulators of T cell activation. PD-L1 is overexpressed in many cancers and overexpression of PD-1 often occurs concomitantly in tumor infiltrating T cells. This results in attenuation of T cell activation and evasion of immune surveillance, which contributes to impaired antitumor immune responses. (M. E. Keir et al., Annu. Rev. Immunol. 2008, 26, 677-704).

In accordance with a further aspect, the present invention covers combinations comprising one or more of the compounds of general formula (I), as described herein, or stereoisomers, tautomers, N-oxides, hydrates, solvates, and salts thereof, particularly pharmaceutically acceptable salts thereof, or mixtures of same, and one or more immune checkpoint inhibitors. Preferably, the immune checkpoint inhibitor is a aPD-1/-L1 axis antagonist.

The compounds of the invention can further be combined with inhibitors of DGK$\alpha$, such as those inhibitors of DGK$\alpha$ disclosed in WO2020/006016, WO2020/006018 and WO 2021/041588. As DGK$\alpha$ in T cells operates in a similar fashion as DGK$\zeta$, a dual inhibition profoundly enhances T cell effector functions compared with cells with deletion of either

DGK isoform alone or wild-type cells. (M. J. Riese et al. Cancer Res. (2013), 73(12); p. 3566-77).

The compounds of the invention can further be combined with chimeric antigen receptor T cells (CAR-T cells), such as Axicabtagen-Ciloleucel or Tisagenlecleucel. The activity of CAR-T cells can be suppressed by the tumor micro environment (TME). Knock out of DGKs by techniques such as Crispr had been shown to enhance CAR-T cell activity in a suppressive TME (I. Y. Jung et al., Mol. Cells 2018, 41 (8), 717-723).

In accordance with a further aspect, the present invention covers combinations comprising one or more compounds of general formula (I), as described herein, or stereoisomers, tautomers, N-oxides, hydrates, solvates, and salts thereof, particularly pharmaceutically acceptable salts thereof, or mixtures of same, with chimeric antigen receptor T cells, (CAR-T cells), CAR-NKT cells or CAR-NK cells.

Preferably, the chimeric antigen receptor T cells (CAR-T cells) are Axicabtagen-Ciloleucel or Tisagenlecleucel.

The present invention further provides the use of the compounds according to the invention for expansion of T cells including CAR-T and tumor infiltrated lymphocytes *ex-vivo.*

In accordance with a further aspect, the present invention covers compounds of general formula (I), as described herein, or stereoisomers, tautomers, N-oxides, hydrates, solvates, and salts thereof, particularly pharmaceutically acceptable salts thereof, or mixtures of same, for use in the expansion of T cells including CAR-T cells, CAR-NKT cells or CAR-NK cells and tumor infiltrated lymphocytes *ex-vivo.*

Hence, the present invention also relates to the use of the compounds according to the invention for the expansion of T cells, including CAR-T cell, CAR-NKT cells or CAR-NK cells and tumor infiltrated lymphocytes, *ex-vivo.*

The present invention also comprises an *ex-vivo* method for the expansion of T cells, including CAR-T cells, CAR-NKT cells or CAR-NK cells and tumor infiltrated lymphocytes, contacting said T cells with compounds according to the invention.

Compounds of the present invention can be utilized to inhibit, block, reduce or decrease DGKζ activity resulting in the modulation of dysregulated immune responses e.g. to block immunosuppression and increase immune cell activation and infiltration in the context of cancer and cancer immunotherapy that will eventually lead to reduction of tumour growth.

The present disclosure also provides methods of treating a variety of other disorders wherein DGKζ is involved such as, but not limited to, disorders with dysregulated immune responses, inflammation, vaccination for infection & cancer, virus infections, lymphoproliferative disorders, asthma, eye diseases, and type 2 diabetes/ insulin resistance.

These disorders have been well characterized in humans, but also exist with a similar etiology in other mammals, and can be treated by administering pharmaceutical compositions of the present invention.

In accordance with a further aspect, the present invention covers compounds of general formula (I), as described *supra,* or stereoisomers, tautomers, N-oxides, hydrates, solvates, and salts thereof, particularly pharmaceutically acceptable salts thereof, or mixtures of same, for use in the treatment or prophylaxis of diseases, in particular cancer or conditions with dysregulated immune responses or other disorders associated with aberrant DGKζ signaling.

The pharmaceutical activity of the compounds according to the invention can be explained by their activity as DGKζ inhibitors.

In accordance with a further aspect, the present invention covers the compounds of general formula (I), as described *supra,* or stereoisomers, tautomers, N-oxides, hydrates, solvates, and salts thereof, particularly pharmaceutically acceptable salts thereof, or mixtures of same, for the use in the treatment or prophylaxis of diseases, in particular cancer or conditions with dysregulated immune responses or other disorders associated with aberrant DGKζ signaling, particularly liquid and solid tumours.

In accordance with a further aspect, the present invention covers use of a compound of general formula (I), as described *supra,* or stereoisomers, tautomers, N-oxides, hydrates, solvates, and salts thereof, particularly pharmaceutically acceptable salts thereof, or mixtures of same, for the preparation of a pharmaceutical composition, preferably a medicament, for the prophylaxis or treatment of diseases, in particular cancer or conditions with dysregulated immune responses or other disorders associated with aberrant DGKζ signaling, particularly liquid and solid tumours.

In accordance with a further aspect, the present invention covers pharmaceutical compositions, in particular a medicament, comprising a compound of general formula (I), as described *supra,* or a stereoisomer, a tautomer, an N-oxide, a hydrate, a solvate, a salt thereof, particularly a pharmaceutically acceptable salt, or a mixture of same, and one or more excipients), in particular one or more pharmaceutically acceptable excipient(s). Conventional procedures for preparing such pharmaceutical compositions in appropriate dosage forms can be utilized.

The present invention furthermore covers pharmaceutical compositions, in particular medicaments, which comprise at least one compound according to the invention, conventionally together with one or more pharmaceutically suitable excipients, and to their use for the above mentioned purposes.

It is possible for the compounds according to the invention to have systemic and/or local activity. For this purpose, they can be administered in a suitable manner, such as, for example, via the oral, parenteral, pulmonary, nasal, sublingual, lingual, buccal, rectal, vaginal, dermal, transdermal, conjunctival, otic route or as an implant or stent.

For these administration routes, it is possible for the compounds according to the invention to be administered in suitable administration forms.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

The compounds according to the invention can be incorporated into the stated administration forms. This can be effected in a manner known per se by mixing with pharmaceutically suitable excipients. Pharmaceutically suitable excipients include, inter alia,

- fillers and carriers (for example cellulose, microcrystalline cellulose (such as, for example, Avicel®), lactose, mannitol, starch, calcium phosphate (such as, for example, Di-Cafos®)),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette®), sorbitan fatty acid esters (such as, for example, Span®), polyoxyethylene sorbitan fatty acid esters (such as, for example, Tween®), polyoxyethylene fatty acid glycerides (such as, for example, Cremophor®), polyoxethylene fatty acid esters, polyoxyethylene fatty alcohol ethers, glycerol fatty acid esters, poloxamers (such as, for example, Pluronic®),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol®); alginates, gelatine),
- disintegrants (for example modified starch, carboxymethylcellulose-sodium, sodium starch glycolate (such as, for example, Explotab®), cross- linked polyvinylpyrrolidone, croscarmellose-sodium (such as, for example, AcDiSol®)),
- flow regulators, lubricants, glidants and mould release agents (for example magnesium stearate, stearic acid, talc, highly-disperse silicas (such as, for example, Aerosil®)),
- coating materials (for example sugar, shellac) and film formers for films or diffusion membranes which dissolve rapidly or in a modified manner (for example polyvinylpyrrolidones (such as, for example, Kollidon®), polyvinyl alcohol,

hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit®)),

- capsule materials (for example gelatine, hydroxypropylmethylcellulose),
- synthetic polymers (for example polylactides, polyglycolides, polyacrylates, polymethacrylates (such as, for example, Eudragit®), polyvinylpyrrolidones (such as, for example, Kollidon®), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition which comprise at least one compound according to the invention, conventionally together with one or more pharmaceutically suitable excipient(s), and to their use according to the present invention.

In accordance with another aspect, the present invention covers pharmaceutical combinations, in particular medicaments, comprising at least one compound of general formula (I) of the present invention and at least one or more further active ingredients, in particular for the treatment and/or prophylaxis of cancer or conditions with dysregulated immune responses or other disorders associated with aberrant DGKζ signaling, particularly liquid and solid tumours.

Particularly, the present invention covers a pharmaceutical combination, which comprises:

- one or more first active ingredients, in particular compounds of general formula (I) as defined *supra,* and
- one or more further active ingredients, in particular in particular immune checkpoint inhibitors.

The term "combination" in the present invention is used as known to persons skilled in the art, it being possible for said combination to be a fixed combination, a non-fixed combination or a kit-of-parts.

A "fixed combination" in the present invention is used as known to persons skilled in the art and is defined as a combination wherein, for example, a first active ingredient, such as one or more compounds of general formula (I) of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.

A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

Based upon standard laboratory techniques known to evaluate compounds useful for the treatment of cancer or conditions with dysregulated immune responses or other disorders associated with aberrant DGKζ signaling, by standard toxicity tests and by standard pharmacological assays for the determination of treatment of the conditions identified above in mammals, and by comparison of these results with the results of known active ingredients or medicaments that are used to treat these conditions, the effective dosage of the compounds of the present invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the

condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

**General syntheses of compounds of the present invention**

The compounds according to the invention of general formula (I) can be prepared according to the following Schemes 1a, 1b, 2, 3, 4, 5 and 6. The schemes and procedures described below illustrate synthetic routes to the compounds of general formula (I) of the invention and are not intended to be limiting. It is clear to the person skilled in the art that the order of transformations as exemplified in Schemes 1a, 1b, 2, 3, 4, 5 and 6 can be modified in various ways. The order of transformations exemplified in these schemes is therefore not intended to be limiting. In addition, modification of any of the substituents, $R^1$, $R^2$, $R^3$ or $R^4$ can be achieved before and/or after the exemplified transformations. These modifications can be such as the introduction of protecting groups, cleavage of protecting groups, reduction or oxidation of functional groups, halogenation, metallation, substitution, or coupling reactions such as amide couplings (couplings of carboxylic acids with amines) or transition metal catalysed coupling reactions (such as the well-known Suzuki coupling) known to the person skilled in the art. These transformations include those which introduce a functionality which allows for further modification of substituents. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs.

Suitable routes for the preparation of compounds of formulae (I-a) and (I-b), which are sub-sets of general formula (I), which when taken together they form, and of further sub-sets (I-c), (I-d), (I-e), (I-f) and (I-g) of general formula (I), are described in Schemes 1a, 1b, 2, 3, 4, 5 and 6.

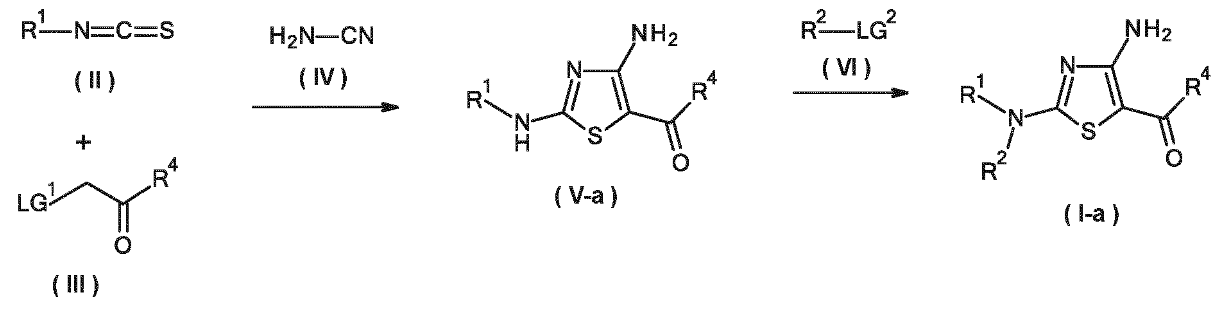

*Scheme 1a:* Route for the preparation of compounds of formula (I-a), a sub-set of general formula (I) in which $R^3$ represents an amino group, from isothiocyanates of formula (II) and ketones of formula (III).

Compounds of formula (I-a), which constitutes a sub-set of general formula (I) in which $R^3$ represents an amino group, can be prepared from isothiocyanates of formula (II), in which $R^1$ has the meaning as given for general formula (I),

and ketones of formula (III), in which $R^4$ has the meaning as given for general formula (I), and in which $LG^1$ represents a leaving group as defined herein, preferably a chlorine, bromine, or iodine atom, more preferably a bromine atom, by reacting with cyanamide (IV), in the presence of a non-nucleophilic base, preferably 1,8-diazabicyclo(5.4.0)undec-7-ene (herein also being referred to as DBU, CAS-RN 6674-22-2), in a dipolar aprotic solvent as defined herein, preferably acetonitrile, at a temperature in the range from 0°C to 50 °C, preferably 15°C to 30°C, more preferably at room temperature, for a time in the range from 1 hour to 100 hours, preferably from 1 hour to 10 hours, more preferably from 2 hours to 4 hours, to give intermediate compounds of formula (V-a). Said intermediate compounds of formula (V-a) can subsequently be reacted with compounds of formula (VI), in which $R^2$ has the meaning as given for general formula (I), and in which $LG^2$ represents a leaving group as defined herein, preferably a chlorine, bromine, or iodine atom, or a (methylsulfonyl)oxy or [(4-methylphenyl)sulfonyl]oxy group, more preferably a bromine atom, in a dipolar aprotic solvent as defined herein, preferably N,N-dimethylformamide, at a temperature in the range from 60°C to 120 °C, preferably 80°C to 100°C, more preferably 90°C, for a time in the range from 30 minutes to 24 hours, preferably from 1 hour to 4 hours, more preferably 2 hours, to give compounds of the present invention, of formula (I-a). Said conversion of intermediate compounds of formula (V-a) into compounds of the present invention of formula (I-a) by reacting with compounds of formula (VI) can also be accomplished advantageously in a dipolar aprotic solvent as defined herein, preferably N,N-dimethylformamide, at a temperature in the range from 0°C to 60 °C, preferably 10°C to 40°C, more preferably at room temperature as defined herein, for a time in the range from 6 hours to 48 hours, preferably from 12 hour to 24 hours. As indicated in the introductory paragraph of this section, substituents, e.g. those attached to $R^1$ and $R^4$, can be modified by various methods known to the person skilled in the art during this synthesis route or on the final step. Specific examples are described in the Experimental Section.

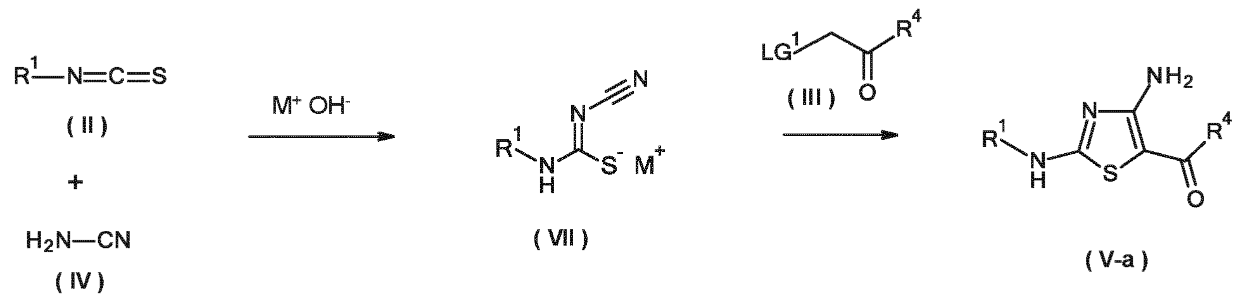

( II )
( IV )
( VII )
( III )
( V-a )

*Scheme 1b:* Alternative route for the preparation of intermediate compounds of formula (V-a), from isothiocyanates of formula (II) and ketones of formula (III).

As shown in Scheme 1b, the conversion of isothiocyanates of formula (II), ketones of formula (III) and cyanamide (IV) into intermediate compounds of formula (V-a) as specified above can also be performed in two steps, in which isothiocyanates of formula (II), in which $R^1$ has the meaning as given for general formula (I), are reacted with cyanamide (IV) in the presence of a base, such as an alkali hydroxide, preferably potassium hydroxide, in an aliphatic alcohol of the formula $C_1$-$C_4$-alkyl-OH, preferably methanol or ethanol, at a temperature in the range from 0°C to 60 °C, preferably 10°C to 40°C, more preferably at room temperature as defined herein, for a time in the range from 15 minutes to 2 hours, preferably for 30 minutes, to give the corresponding carbamimidothioate salts of formula (VII), in which $M^+$ represents an alkali metal cation, which can be reacted with ketones of formula (III), in which $R^4$ has the meaning as given for general formula (I), and in which $LG^1$ represents a leaving group as defined herein, preferably a chlorine, bromine, or iodine atom, more preferably a bromine atom, in an aliphatic alcohol of the formula $C_1$-$C_4$-alkyl-OH, preferably methanol or ethanol, at a temperature in the range from 40°C to 100 °C, preferably from 60°C to 90°C, for a time in the range from 1 minutes to 4 hours, preferably for 2 hours, to give the intermediate compounds of formula (V-a). Specific examples are described in the Experimental Section.

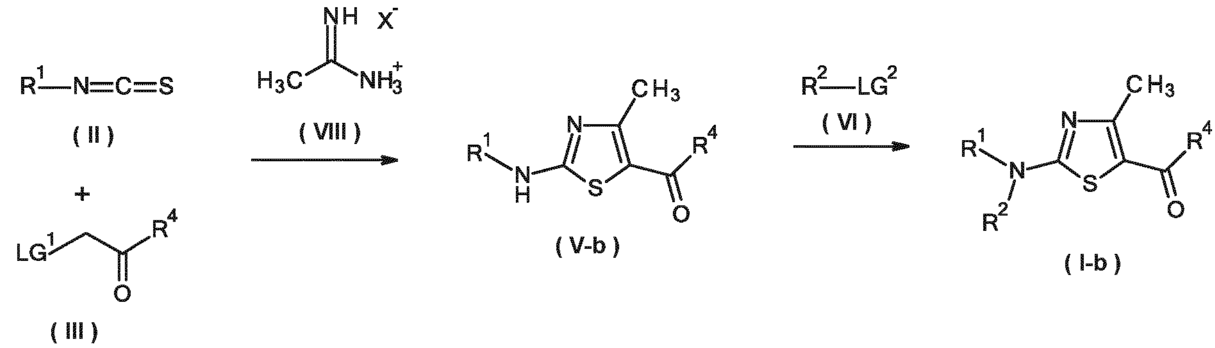

*Scheme 2:* Route for the preparation of compounds of formula (I-b), a sub-set of general formula (I) in which $R^3$ represents a methyl group, from isothiocyanates of formula (II) and ketones of formula (III).

Compounds of formula (I-b), which constitutes a sub-set of general formula (I) in which $R^3$ represents a methyl group, can be prepared from isothiocyanates of formula (II), in which $R^1$ has the meaning as given for general formula (I), and ketones of formula (III), in which $R^4$ has the meaning as given for general formula (I), and in which $LG^1$ represents a leaving group as defined herein, preferably a chlorine, bromine, or iodine atom, more preferably a bromine atom, by reacting with a salt of ethanimidamide of formula (VIII), in which $X^-$ represents a monovalent anion, preferably chloride, bromide, iodide or acetate, more preferably chloride, in the presence of a non-nucleophilic base, preferably 1,8-diazabicyclo(5.4.0)undec-7-ene (herein also being referred to as DBU, CAS-RN 6674-22-2), in a dipolar aprotic solvent as defined herein, preferably acetonitrile, at a temperature in the range from 0°C to 50 °C, preferably 15°C to 30°C, more preferably at room temperature, for a time in the range from 1 hour to 100 hours, preferably from 1 hour to 10 hours, more preferably from 2 hours to 4 hours, to give intermediate compounds of formula (V-b). Said intermediate compounds of formula (V-b) can subsequently be reacted with compounds of formula (VI), in which $R^2$ has the meaning as given for general formula (I), and in which $LG^2$ represents a leaving group as defined herein, preferably a chlorine, bromine, or iodine atom, or a (methylsulfonyl)oxy or [(4-methylphenyl)sulfonyl]oxy group, more preferably a bromine atom, in a dipolar aprotic solvent as defined herein, preferably N,N-dimethylformamide, at a temperature in the range from 60°C to 120 °C, preferably 80°C to 100°C, more preferably 90°C, for a time in the range from 30 minutes to 24 hours, preferably from 1 hour to 4 hours, more preferably 2 hours, to give compounds of the present invention, of formula (I-b).

Compounds of formulae (II), (III), (IV), (VI), and (VIII) are largely commercially available or can be prepared using methods known to the person skilled in the art, see e.g. F. Calderon et al. Journal of Medicinal Chemistry 2017, 60 (16), 6880 - 6896, for the preparation of isothiocyanates of formula (II), or Y. Xing et al. European Journal of Organic Chemistry 2017, 2017 (4), 781 - 785; A. Wu et al. Tetrahedron 2013, 69 (31), 6392 - 6398, for the preparation of ketones of formula (III); see also the synthesis protocols of Intermediates 9, 10 and 11 in the Experimental Section.

Alternatively, compounds of formulae (I-c), (I-d), (I-e), (I-f) and (I-g), which constitute various sub-sets of general formula (I) in which $R^3$ represents an amino group, can be prepared from precursors in which the $R^4$ group is not present from the start (Schemes 3, 4, and 5), or is substituted with a group not covered within the definition of $R^4$ (such as a carboxy group), which however allows for establishing diverse $R^4$ groups from a common precursor (Scheme 6)

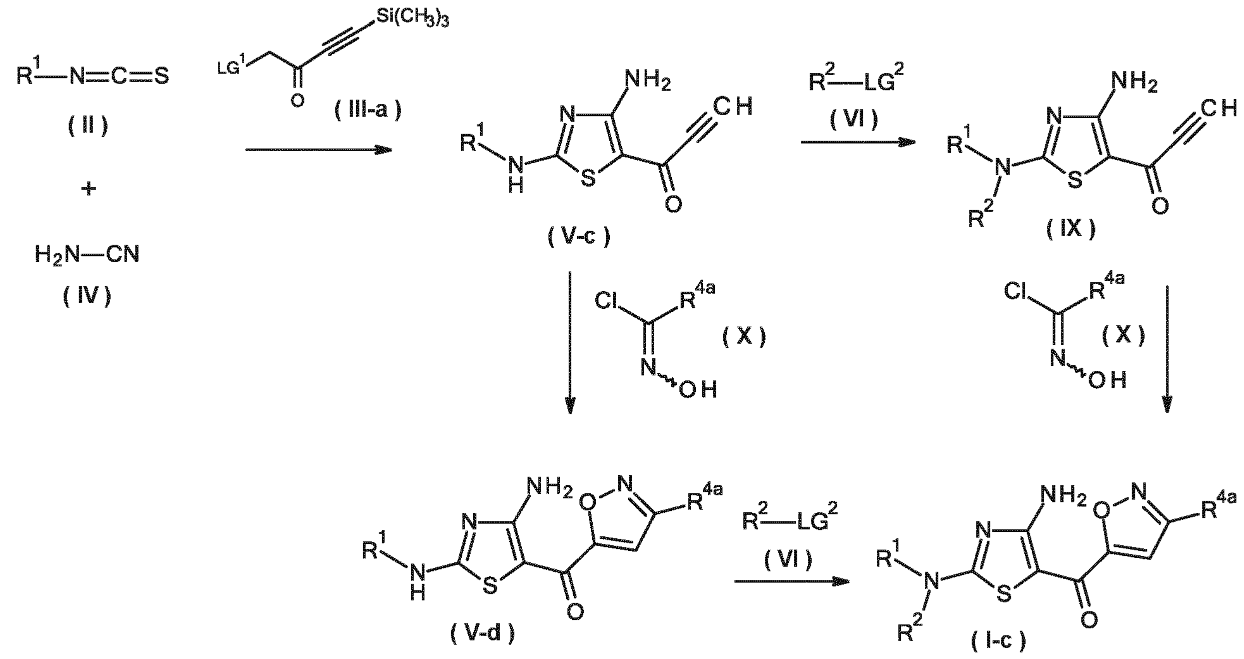


*Scheme 3:* Route for the preparation of compounds of formula (I-c), a sub-set of general formula (I) in which $R^3$ represents an amino group and in which $R^4$ represents an isoxazolyl group, from isothiocyanates of formula (II) and silyl ethynyl ketones of formula (III-a).

As shown in Scheme 3, compounds of formula (I-c), which constitutes a sub-set of general formula (I) in which $R^3$ represents an amino group and in which $R^4$ represents an isoxazolyl group, can be prepared, in some analogy to Scheme 1a, by reacting isothiocyanates of formula (II), in which $R^1$ has the meaning as given for general formula (I), with cyanamide (IV), followed by silyl ethynyl ketones of formula (III-a), in which $LG^1$ represents a leaving group as defined herein, preferably a chlorine, bromine, or iodine atom, more preferably a bromine atom, in the presence of a non-nucleophilic base, preferably 1,8-diazabicyclo(5.4.0)undec-7-ene (herein also being referred to as DBU, CAS-RN 6674-22-2), in a dipolar aprotic solvent as defined herein, preferably acetonitrile, at a temperature in the range from 0°C to 50 °C, preferably 15°C to 30°C, more preferably at room temperature, for a time in the range from 1 hour to 10 hours, preferably from 2 hours to 4 hours, to give intermediate compounds of formula (V-c), featuring a terminal alkyne. Said intermediate compounds of formula (V-c) can subsequently be reacted with compounds of formula (VI), in which $R^2$ has the meaning as given for general formula (I), and in which $LG^2$ represents a leaving group as defined herein, preferably a chlorine, bromine, or iodine atom, or a (methylsulfonyl)oxy or [(4-methylphenyl)sulfonyl]oxy group, more preferably a bromine atom, in the presence of a base such as potassium carbonate, in a dipolar aprotic solvent as defined herein, preferably N,N-dimethylformamide, at a temperature in the range from 0°C to 60 °C, preferably 10°C to 40°C, more preferably at room temperature as defined herein, for a time in the range from 6 hours to 48 hours, preferably from 12 hour to 24 hours, to give intermediate compounds of formula (IX). Said intermediate compounds of formula (IX) can be converted into compounds of formula (I-c) by reacting with N-hydroxy imidoyl chlorides of formula (X), in which $R^{4a}$ has the meaning as given for general formula (I), in a mixture of DMSO, tetrahydrofuran and tert-butanol as a solvent, in the presence of copper(II)sulfate pentahydrate, (+)-ascorbic acid and potassium carbonate, at a temperature in the range from 0°C to 50 °C, preferably 20°C to 40°C, for a time in the range from 2 hours to 3 days. Alternatively, this reaction can be achieved in the presence of triethylamine, in tetrahydrofuran as a solvent, without the addition of copper salts. Specific examples are given in the Experimental section. Upon replacement of said N-hydroxy imidoyl chlorides of formula (X) with azides, compounds of the invention in which $R^4$ represents a substituted 1,2,3-triazolyl group can be obtained. Specific examples are described in the Experimental Section.

The order of the last two steps can be reversed, that is, the alkyne group present in the intermediate compounds of formula (V-c) can be first reacted with said N-hydroxy imidoyl chorides of formula (X), to give intermediate compounds of formula (V-d), followed by the introduction of the $R^2$ group by reacting with compounds of formula (VI) as described above.

Silyl ethynyl ketones of formula (III-a) are largely commercially available or can be prepared using methods known to the person skilled in the art. Also, N-hydroxy imidoyl chorides of formula (X) are known to the person skilled in the art; for a synthesis protocol see Intermediates 41 and 72-76, in the Experimental section below.

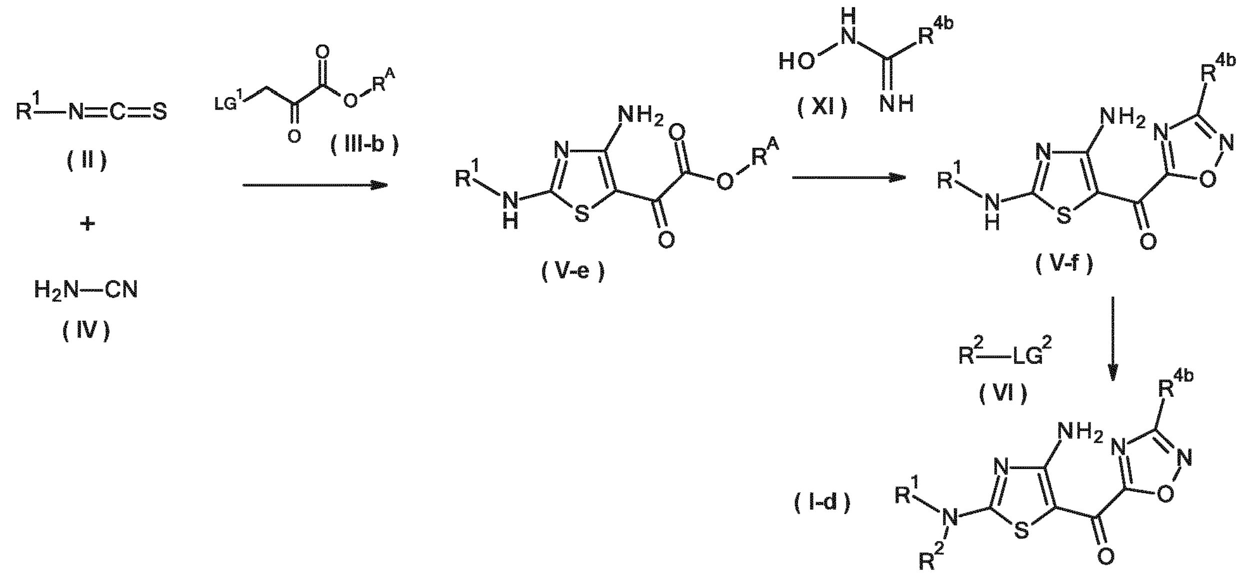

*Scheme 4:* Route for the preparation of compounds of formula (I-d), a sub-set of general formula (I) in which $R^3$ represents an amino group and in which $R^4$ represents an oxadiazolyl group, from isothiocyanates of formula (II) and pyruvate esters of formula (III-b).

As shown in Scheme 4, compounds of formula (I-d), which constitutes a sub-set of general formula (I) in which $R^3$ represents an amino group and in which $R^4$ represents an oxadiazolyl group, can be prepared, in some analogy to Scheme 1a, by reacting isothiocyanates of formula (II), in which $R^1$ has the meaning as given for general formula (I), with cyanamide (IV), followed by pyruvate esters of formula (III-b), in which $LG^1$ represents a leaving group as defined herein, preferably a chlorine, bromine, or iodine atom, more preferably a bromine atom, and in which $R^A$ represents a $C_1$-$C_4$-alkyl group, preferably methyl or ethyl, in the presence of a non-nucleophilic base, preferably 1,8-diazabicyclo(5.4.0)undec-7-ene (herein also being referred to as DBU, CAS-RN 6674-22-2), in a dipolar aprotic solvent as defined herein, preferably acetonitrile, at a temperature in the range from 0°C to 50 °C, preferably 15°C to 30°C, more preferably at room temperature, for a time in the range from 2 hours to 48 hours, preferably from 12 hours to 20 hours, to give intermediate compounds of formula (V-e), featuring a carboxylic ester group. Said carboxylic ester group can be reacted subsequently with N-hydroxy amidines of formula (XI), in which $R^{4b}$ has the meaning as given for general formula (I), to give intermediate compounds of formula (V-f). Said intermediate compounds of formula (V-f) can subsequently be reacted with compounds of formula (VI), in which $R^2$ has the meaning as given for general formula (I), and in which $LG^2$ represents a leaving group as defined herein, preferably a chlorine, bromine, or iodine atom, or a (methylsulfonyl)oxy or [(4-methylphenyl)sulfonyl]oxy group, more preferably a bromine atom, in the presence of a base such as potassium carbonate, in a dipolar aprotic solvent as defined herein, preferably N,N-dimethylformamide, at a temperature in the range from 0°C to 60 °C, preferably 10°C to 40°C, more preferably at room temperature as defined herein, for a time in the range from 6 hours to 48 hours, preferably from 12 hour to 24 hours, to give compounds of formula (I-d). Specific examples are described in the Experimental Section.

Pyruvate esters of formula (III-b) are largely commercially available or can be prepared using methods known to the person skilled in the art. Also, N-hydroxy amidines of formula (XI) are known to the person skilled in the art; for a synthesis protocol see Intermediates 64 and 67, in the Experimental section below.

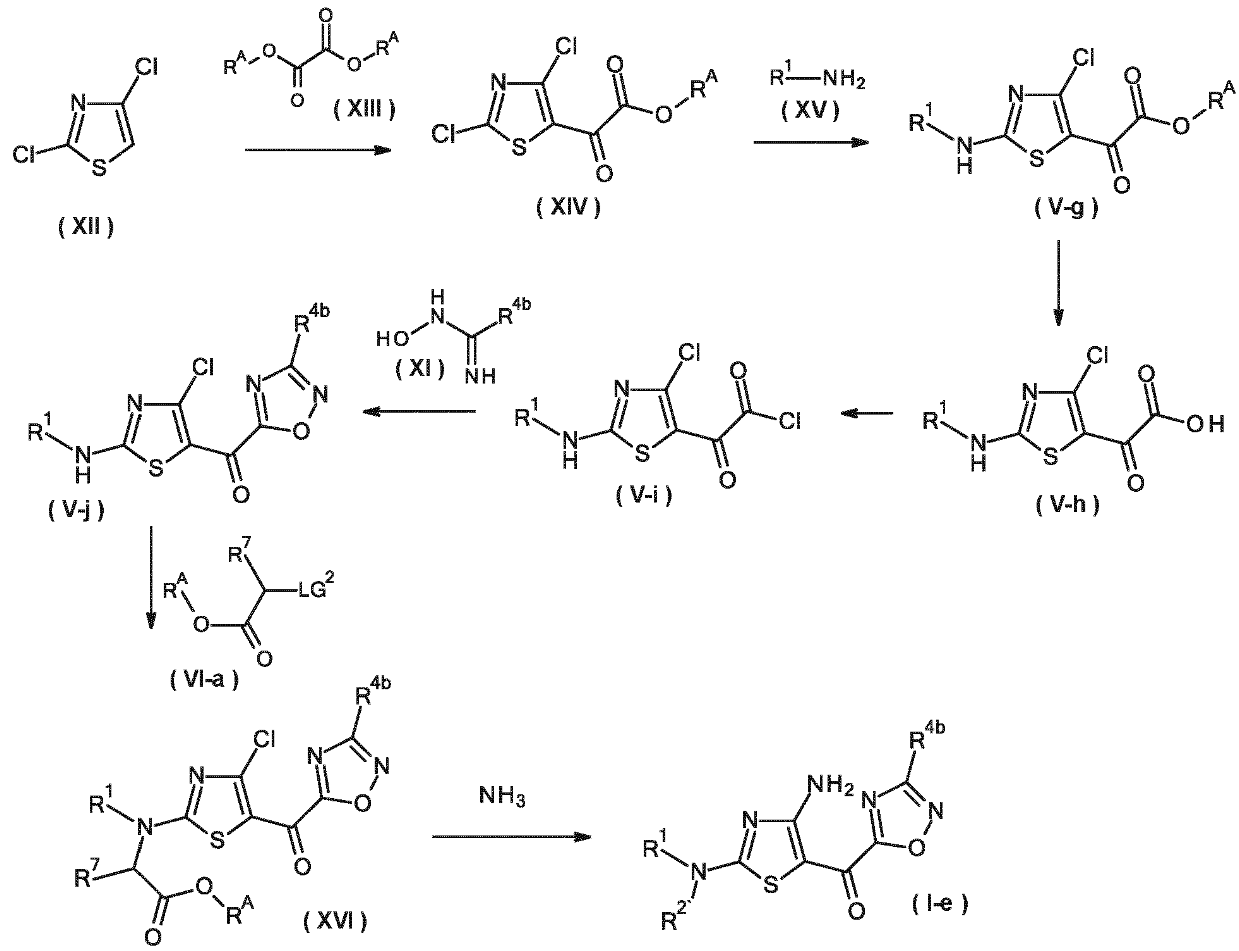


(XII) (XIII) (XIV) (XV) (V-g) (XI) (V-j) (V-i) (V-h) (VI-a) (XVI) (I-e)

*Scheme 5:* Route for the preparation of compounds of formula (I-e), a sub-set of general formula (I) in which $R^3$ represents an amino group, in which $R^4$ represents an oxadiazolyl group, and in which $R^{2'}$ has the meaning as given for $R^2$ in general formula (I) with the proviso that $R^8$ represents a group -C(=O)-$NH_2$, from 2,4-dichlorothiazole (XII).

As shown in Scheme 5, compounds of formula (I-e), which constitutes a sub-set of general formula (I) in which $R^3$ represents an amino group, in which $R^4$ represents an oxadiazolyl group, and in which $R^{2'}$ has the meaning as given for $R^2$ in general formula (I) with the proviso that $R^8$ represents a group -C(=O)-$NH_2$, can be prepared from 2,4-dichlorothiazole (XII) by reacting with a strong base, such as lithium diisopropylamide, followed by an oxalate ester of formula (XIII), in which $R^A$, independently from each other, represents a $C_1$-$C_4$-alkyl group, preferably methyl or ethyl, in a solvent such as tetrahydrofuran, at a temperature in the range from -80°C to -50 °C, preferably -70°C, to give dichlorothiazole derivatives of formula (XIV), which can in turn be reacted with amines of formula (XV), in which $R^1$ has the meaning as given for general formula (I), in a solvent such as N,N-dimethylformamide, to give ester derivatives of formula (V-g). The ester group present in said compounds of formula (V-g) can be readily converted into a carboxy group, as in compounds of formula (V-h), by well known methods, such as saponification with aqueous alkali hydroxide, such as lithium hydroxide, in a solvent such as methanol, ethanol, or tetrahydrofuran, or mixtures thereof, followed by conversion into an acyl choride, as in compounds of formula (V-i), by well-known methods such a reaction with oxalyl chloride in a solvent such as dichloromethane in the presence of N,N-dimethylformamide. Said acyl chloride functionality can be reacted subsequently with a N-hydroxy amidine of formula (XI), in which $R^{4b}$ has the meaning as given for general formula (I), in a solvent such as dichloromethane or N,N-dimethylformamide, in the presence of a base such as trimethylamine, triethylamine or N,N-diisopropylethylamine, at a temperature in the range from 0°C to 50 °C, preferably 20°C to 40°C, for a time in the range from 2 hours to 24 hours, to give intermediate compounds of formula (V-j), which can be reacted with carboxylic esters of formula (VI-a), in which $R^7$ has the meaning as given for general formula (I), in which $R^A$ represents a $C_1$-$C_4$-alkyl group, preferably methyl or ethyl, and in which $LG^2$ represents a leaving group as defined herein, preferably a chlorine, bromine, or iodine atom, or a

(methylsulfonyl)oxy or [(4-methylphenyl)sulfonyl]oxy group, more preferably a bromine atom, in a dipolar aprotic solvent as defined herein, preferably N,N-dimethylformamide, at a temperature in the range from 0°C to 50 °C, preferably at room temperature as defined herein, for a time in the range from 2 hours to 24 hours, preferably from 12 hour to 20 hours, more preferably 16 hours, to give intermediate ester compounds of formula (XVI). Said intermediate ester compounds can be reacted with ammonia, in an aliphatic alcohol $C_1$-$C_4$-alkyl-OH, preferably methanol or ethanol, as a solvent, at a temperature in the range from 0°C to 50 °C, preferably at room temperature as defined herein, more preferably at 25 °C, for a time in the range from 2 hours to 24 hours, preferably from 12 hour to 20 hours, more preferably 16 hours, to give compounds of the present invention, of formula (I-e). Specific examples are described in the Experimental Section.

Alternatively to establishing the oxadiazole moiety from the acyl cloride function present in intermediates of formula (V-i), also their precursor carboxylic acids of formula (V-h) can be reacted with a N-hydroxy amidine of formula (XI), in which $R^{4b}$ has the meaning as given for general formula (I), in a dipolar aprotic solvent such as N,N-dimethylformamide, in the presence of propane phosphonic anhydride and in the presence of a base such as triethylamine or N,N-diisopropylethylamine, at a temperature in the range from 20°C to 100 °C, preferably 50°C to 70°C, more preferably 60°C, for a time in the range from 2 hours to 24 hours, preferably 16 hours, to give intermediate compounds of formula (V-j), which can be processed further as described *supra.* Specific examples are described in the Experimental Section.

Compounds of formulae (VI-a), (XI), (XII), (XIII), and (XV) are largely commercially available or can be prepared using methods known to the person skilled in the art; for a synthesis protocol of N-hydroxy amidines of formula (XI) see Intermediates 64 and 67, in the Experimental section below.

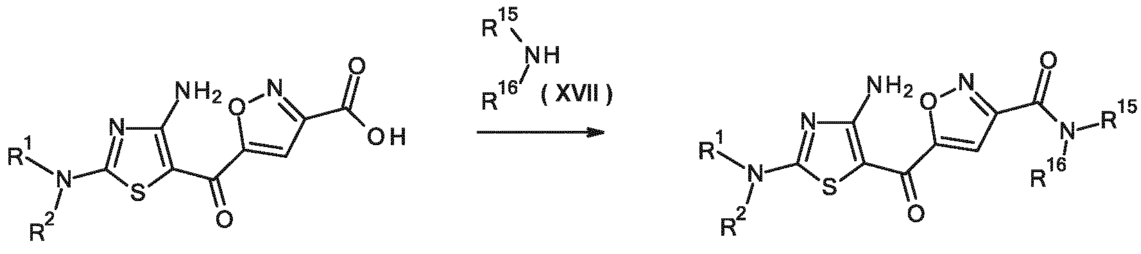

( V-k ) ( XVII ) ( I-f )

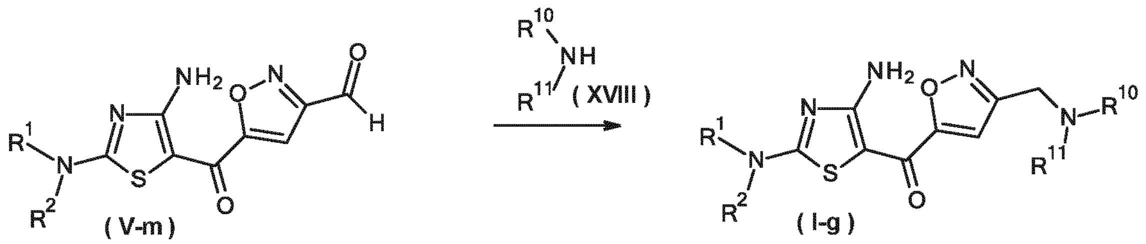

( V-m ) ( XVIII ) ( I-g )

*Scheme 6:* Route for the preparation of further compounds of the invention, of formulae (I-f) and (I-g), from advanced intermediate compounds of formulae (V-k) and (V-m).

**Scheme** 6 **exemplifies** (without any limitation to the invention) the conversions of advanced intermediates of formulae (V-k) and (V-m), which can be prepared using the methods described in the preceding Schemes, in combination with well-established methods such as the saponification of carboxylic esters or the oxidation of primary alcohols, and which are substituted with a group not covered within the definition of $R^4$, which however allows for establishing diverse $R^4$ groups from a common precursor. Thus, carboxylic acid derivatives of formula (V-m) can be reacted in well-known amide coupling reactions with amines of formula (XVII), in which $R^{15}$ and $R^{16}$ have the meanings as given for general formula (I), in the presence of a coupling reagent such as HATU and a tertiary amine such as triethylamine or N,N-diisopropylethylamine, and optionally in the presence of DMAP, in a dipolar aprotic solvent as defined herein, preferably N,N-dimethylformamide, at a temperature in the range from 0°C to 50 °C, preferably at room temperature as defined herein, for a time in the range from 2 hours to 24 hours, preferably from 12 hour to 20 hours, to give carboxamide compounds of the invention, of formula (I-f). Likewise, **a**ldehydes of formula (V-m) can be reacted in well-known reductive amination reactions with amines of formula (XVIII), in which $R^{10}$ and $R^{11}$ have the meanings as given for general formula (I), in the presence of acetic acid in a mixture of methanol, tetrahydrofuran and N,N-dimethylformamide, followed by the addition of sodium cyanoborohydride, at a temperature in the range from 0°C to 50 °C, preferably at room temperature as defined herein, for a time in the range from 2 hours to 24 hours, preferably from 12 hour to 20 hours, to give aminoalkyl compounds of the invention, of formula (I-g). Specific examples are described in the Experimental Section.

Amines of formulae (XVII) and (XVIII) are commercially available.

In accordance with a second aspect, the present invention covers methods of preparing compounds of formulae (I-a) and (I-b), which are sub-sets of general formula (I), *supra,* which when taken together they form.

In accordance with a second embodiment of the second aspect, said methods comprise the step of allowing an intermediate compound of formula (V-a) :

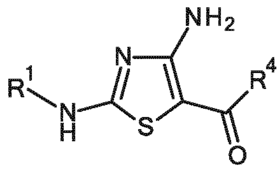

(V-a)

in which $R^1$ and $R^4$ are as defined for the compound of general formula (I) as defined *supra,* to react with a compound of formula (VI) :

$R^2$—$LG^2$

(VI),

in which $R^2$ is as defined for the compound of general formula (I) as defined *supra,* and in which $LG^2$ represents a leaving group as defined herein,
thereby giving a compound of formula (I-a) :

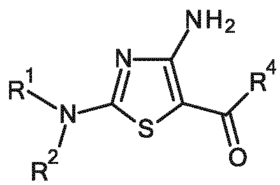

(I-a),

in which R', $R^2$ and $R^4$ are as defined *supra.*

In accordance with a third aspect, the present invention covers methods of preparing compounds of formulae (I-a) and (I-b), which are sub-sets of general formula (I), *supra,* which when taken together they form.

In accordance with a second embodiment of the third aspect, said methods comprise the step of allowing an intermediate compound of formula (V-a) :

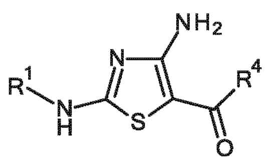

(V-a)

in which $R^1$ and $R^4$ are as defined for the compound of general formula (I) as defined *supra,* to react with a compound of formula (VI) :

$R^2$—$LG^2$

(VI),

in which $R^2$ is as defined for the compound of general formula (I) as defined *supra,* and in which $LG^2$ represents a leaving group as defined herein,
thereby giving a compound of formula (I-a) :

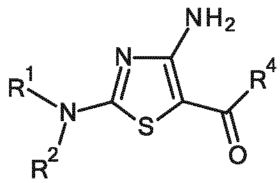

(I-a),

in which R', $R^2$ and $R^4$ are as defined *supra,*
then optionally converting said compound into solvates, salts and/or solvates of such salts using the corresponding (i) solvents and/or (ii) bases or acids.

The present invention covers methods of preparing compounds of the present invention of general formula (I), said methods comprising the steps as described in the Experimental Section herein.

In accordance with a fourth aspect, the present invention covers the use of intermediate compounds for the preparation of the compounds of formulae (I-a) and (I-b), which are sub-sets of general formula (I), *supra,* which when taken together they form.

In accordance with a second embodiment of the fourth aspect, the present invention covers the intermediate compounds of formula (V-a) :

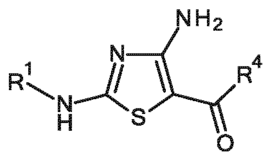

(V-a)

in which $R^1$ and $R^4$ are as defined for the compound of general formula (I) *supra,* for the preparation of a compound of formula (I-a) as defined *supra.*

The present invention covers intermediate compounds which are disclosed in the Example Section of this text, *infra.*

The present invention covers the use of intermediate compounds which are disclosed in the Example Section of this text, *infra.*

The present invention covers any sub-combination within any embodiment or aspect of the present invention of intermediate compounds of formulae (V-a), *supra.*

**Description of the Figures**

Figure 1: DGKz_hu_1 encoding human DGKζ M1 to V928 plus N-terminal Flag-Tag, as described under SEQ ID No. 1.

Figure 2: SIINFEKL amino acid sequence, as described under SEQ ID No. 2.

Figure 3: OVA-30 peptide sequence, as described under SEQ ID No. 3.

Figure 4: FLAG-Tag, as described under SEQ ID No. 4.

Figure 5: Kozak sequence for translation initiation, as described under SEQ ID No. 5.

Figure 6: 50% thermal ellipsoids of Example 3.1

## EXPERIMENTAL SECTION - GENERAL PART

NMR peak forms are stated as they appear in the spectra, possible higher order effects have not been considered. The multiplicities are stated according to the signal form which appears in the spectrum, NMR-spectroscopic effects of a higher order were not taken into consideration. Multiplicity of the NMR signals: s = singlet, d = doublet, t = triplet, q = quartet, quin = quintet, br = broad signal, m = multiplet. NMR signals: shift in [ppm]. Combinations of multiplicity could be e.g. dd = doublet from doublet.

Chemical names were generated using software programs such as the ACD/Name batch version 14.05 from ACD/Labs and BioVia Draw 2019 Version 19.1 NET, and chemical names were adapted if needed. In some cases generally accepted names of commercially available reagents were used in place of chemical names generated using abovementioned software programs.

All reagents the synthesis of which is not described in the experimental part were purchased commercially, or said reagents are known compounds or can be formed from known compounds by known methods by a person skilled in the art.

Table 1 lists the abbreviations used in this paragraph and in the Examples section as far as they are not explained within the text body. Other abbreviations have their meanings customary *per* se to the skilled person.

**Table 1: Abbreviations**

| | |
|---|---|
| $CDCl_3$ | deuterochloroform |
| DAD | diode array detector |
| SQD | single quadrupole detector |
| Azura UVD | single variable wavelength UV detector for HPLC |
| DMF | N, N-dimethylformamide |
| DMSO-d6 | deuterated dimethyl sulphoxide |
| DMSO | dimethyl sulphoxide |
| ELSD | evaporative light scattering detector |
| ESIpos | electrospray ionization positive |
| Expl. | example |
| HATU | (7-aza-1H-benzotriazol-1-yl)-1,1,3,3-tetramethyluronium hexafluorophosphate |
| HPLC | high-pressure liquid chromatography |
| LC-MS | liquid chromatography coupled with mass spectrometry |
| mL | milliliter |
| min | minute(s) |
| MTBE | methyl *tert-butyl* ether |
| RP-HPLC | reverse-phase high-pressure liquid chromatography |
| Rt | retention time |
| rt | room temperature |
| sat. | saturated |
| THF | tetrahydrofurane |
| EtOAc | ethyl acetate |
| TLC | thin layer chromatography |
| rac | racemic |
| µM | micromolar |
| M | molar |
| UPLC | Ultra high performance chromatography |
| UPLC-MS | Ultra high performance chromatography coupled with mass spectrometry |
| BEH | ethylene bridged hybrid |
| CSH | charged surface hybrid |
| UV | ultra violet |
| CAS-RN | chemical abstracts service registry number |
| NMR | nuclear magnetic resonance |
| MHz | Megahertz |

## Analytical UPLC-MS Standard Procedures

**Method** 1 / acidic
Instrument: Waters Acquity UPLC-MS SQD 3001; column: Acquity UPLC BEH C18 1.7 50×2.1mm; eluent A: water + 0.1 vol% formic acid, eluent B: acetonitrile; gradient: 0-1.6 min 1-99% B, 1.6-2.0 min 99% B; flow 0.8 mL/min; temperature: 60°C; injection: 2 μL; DAD scan: 210-400 nm; ELSD
**Method 2 /** basic
Instrument: Waters Acquity UPLC-MS SQD 3001; column: Acquity UPLC BEH C18 1.7 50×2.1mm; eluent A: water + 0.2 vol% aqueous ammonia (32%), eluent B: acetonitrile; gradient: 0-1.6 min 1-99% B, 1.6-2.0 min 99% B; flow 0.8 mL/min; temperature: 60°C; injection: 2 μL; DAD scan: 210-400 nm; ELSD
**Method 3** / basic
Instrument: Waters Acquity UPLC-MS SingleQuad; Column: Acquity UPLC CSH C18 1.7μm 50×2.1mm; eluent A: water + 0.2 vol % aqueous ammonia (32%), eluent B: acetonitrile; gradient: 0-1.6 min 1-99% B, 1.6-2.0 min 99% B; flow 0.8 mL/min; temperature: 60°C; DAD scan: 210-400 nm.
Method 4:
5-95AB, Shimadzu
Instrument: SHIMADZU LCMS-2020 SingleQuad; Column: Chromolith@Flash RP-18E 25-2 MM; eluent A: water + 0.0375 vol % trifluoroacetic acid, eluent B: acetonitrile + 0.01875 vol % trifluoroacetic acid; gradient: 0-0.8 min, 5-95% B, 0.8-1.2 min 95% B; flow 1.5 mL/min; temperature: 50 °C; PDA: 220 nm & 254 nm.

## Optical Rotation

Optical rotations were measured with a JASCO Polarimeter 2000 using the solvent and concentration stated in each case at 20°C, wavelength 589 nm, integration time 10 s, layer thickness 100 mm.

## Compound purification - General

The example compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil® or KP-NH® in combination with a Biotage autopurifier system (SP4® or Isolera Four®) and eluents such as gradients of hexane/ethyl acetate or dichloromethane/methanol. In some cases, the compounds may be purified by preparative HPLC using for example a Waters autopurifier system equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

Specific methods are described below, and in the respective protocols describing the preparations of example compounds and intermediates.

## Preparative HPLC

Instrument: pump: Labomatic HD-3000, head HDK 280, low pressure gradient module ND-B1000; manual injection valve: Rheodyne 3725i038; detector: Knauer Azura UVD 2.15; collector: Labomatic Labocol Vario-4000; column: Chromatorex RP C-18 10 μm, 125x30mm; eluent; gradient; UV-Detection. Eluent: solvent A: water + 0.1 vol% formic acid (99%; acidic) or 0.2 vol% aqueous ammonia (32%, basic) , solvent B: acetonitrile; flow 150 mL/min.

**Gradients:**

**Method A:** 0.00-0.50 min 1% B, 0.50-6.00 min 1-25% B, 6.00-6.10 min 25-100% B, 6.10-8.00 min 100% B
**Method B:** 0.00-0.50 min 10% B, 0.50-6.00 min 10-50% B, 6.00-6.10 min 50-100% B, 6.10-8.00 min 100% B
**Method C:** 0.00-0.50 min 15% B, 0.50-6.00 min 15-55% B, 6.00-6.10 min 55-100% B, 6.10-8.00 min 100% B
**Method D:** 0.00-0.50 min 30% B, 0.50-6.00 min 30-70% B, 6.00-6.10 min 70-100% B, 6.10-8.00 min 100% B
**Method E:** 0.00-0.50 min 40% B, 0.50-6.00 min 40-80% B, 6.00-6.10 min 80-100% B, 6.10-8.00 min 100% B

**Method F:**

Instrument: Waters autopurification system; column: Waters CSH C18 5µ 100x30mm; eluent A: water + 0.1 Vol-% aqueous ammonia (32%), eluent B: acetonitrile, DAD scan: 210-400 nm; MS Instrument: QDA (Waters); Collector-Trigger: DAD-MS flow rate: 60 mL/min

**Chiral HPLC and stereochemistry assignments**

Separations of stereoisomeric mixtures, such as racemic compounds, by chiral HPLC can result in the isolation of single stereoisomers without known configuration of the respective stereogenic centres in the isolated isomers. In the following, the full chemical names of all such separated isomers obtained from an isomeric mixture, including (R) and (S) configurations, are listed in alphabetical order together with all respective intermediate or example numbers, followed by the individual isomers with data on their analytics, isolation and yield, followed by descriptors such as "(enantiomer 1)", "(stereoisomer 2)", and the like. Likewise, example compounds obtained from starting materials being single stereoisomers of unknown absolute configuration are disclosed herein in an analogous fashion. The order of the full chemical names (including (R) and (S) configurations) cannot be construed as to encode for any correlation to the individual intermediate or example numbers.

**Preparative flash chromatography**

Instrument: Biotage Isolera Four; pump: Dual-Piston; flow rate: 1 to 200 mL/min; internal detector: 200-400 nm (variable UV detector); solvent inlets: 4; cartridges: Biotage SNAP Ultra™, sizes: 10 g, 25 g, 50 g, 100 g, 340 g, media: Biotage® HP-Sphere - 25 micron spherical silica, resolution: minimum 7000 N/m (plates per meter) typical 10 000 N/m; solvent A: hexane, solvent B: ethyl acetate, solvent C: dichlormethane, solvent D: ethanol; solvent E: methanol; UV collection modes: single/dual/λ-All wavelengths (variable UV ); fractionation modes: volume, threshold, threshold with volume, low slope, medium slope, custom slope or via external detection

**Method X:** gradient with solvent A and B, λ-all
**Method Y:** gradient with solvent C and D, λ-all
**Method Z:** gradient with solvent C and E, λ-all

The various aspects of the invention described in this application are illustrated by the following examples which are not meant to limit the invention in any way.

The example testing experiments described herein serve to illustrate the present invention and the invention is not limited to the examples given.

## EXPERIMENTAL SECTION - PREPARATION OF INTERMEDIATES

### Intermediate 1

[4-amino-2-(4-fluoroanilino)-1,3-thiazol-5-yl][3-(4-chlorophenyl)-1,2,4-oxadiazol-5-yl]methanone

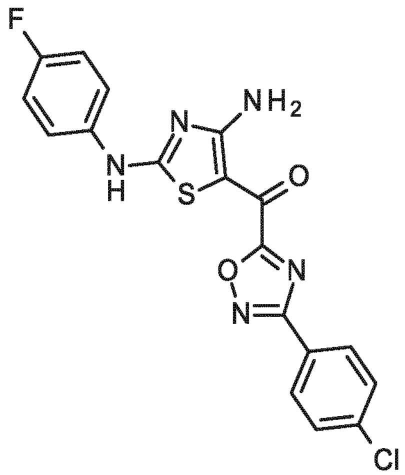

**1-fluoro-4-isothiocyanatobenzene** (230 mg, 0.76 mmol) was dissolved in acetonitrile (6 mL) followed by the addition of 1,8-diazabicyclo(5.4.0)undec-7-ene (116 mg, 0.76 mmol) and cyanamide (38.5 mg, 42 mmol). After stirring for 45 min at rt further 1,8-diazabicyclo(5.4.0)undec-7-ene (58 mg, 0.38 mmol) and 2-bromo-1-[3-(4-chlorophenyl)-1,2,4-oxadiazol-5-yl]ethanone (Intermediate 9, 230 mg, 0.76 mmol) were added. The reaction mixture was stirred at rt overnight followed by the addition of water and ethyl acetate. The phases were separated and the aqueous phase was extracted three times with ethyl acetate. The combined organic phases were washed with brine and filtered through a water repellent filter circle (MN 617 WA) and evaporated to dryness. The crude product was purified by Biotage (method X) to give 26 mg (0.06 mmol, 8 % yield) of the title compound.

$^1H$ NMR (400 MHz, DMSO-$d_6$) δ ppm = 7.24 - 7.28 (m, 2 H), 7.70 - 7.78 (m, 4 H), 8.06 (d, J=8.62 Hz, 2 H), 8.87 (br s, 1 H), 8.97 (br s, 1 H), 11.24 (s, 1 H).

LC-MS (method 2) Rt = 0.96 min; MS (ESIpos): m/z = 416.3 $[M+H]^+$

The following intermediates were prepared from commercial starting or separately prepared materials as stated in Table 2, below, using the procedure as for Intermediate 1, followed by purification by chromatography if needed. If no purification is specified, the respective title compound was isolated as crude product.

The crude product was either purified by RP-HPLC (methods A-D depending on polarity) or by preparative flash chromatography (Biotage, methods X, Y or Z depending on polarity) if necessary. In case of a missing precipitation, the reaction mixture was extracted three times with ethyl acetate. The combined organic phases were dried over sodium sulfate, filtrated and evaporated to dryness. The crude product was purified by chromatography as stated in Table 2.

**Table 2: Intermediates 2-8**

| **Interme diate number** | Chemical structure Compound name | Starting materials | Analytics/yield |
|---|---|---|---|
| **2** | 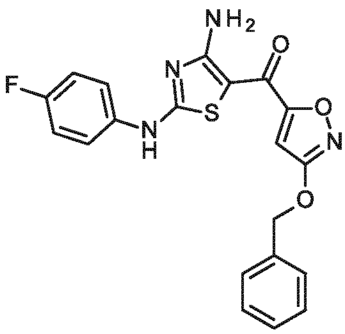 [4-amino-2-(4-fluoroanilino)thiazol-5-yl]-(3-benzyloxyisoxazol-5-yl)methanone | 1-fluoro-4-isothiocyanatobenze ne; 1-[3-(benzyloxy)-1,2-oxazol-5-yl]-2-bromoethanone | LC-MS (method 2) Rt = 0.99 min; MS (ESIpos): m/z = 411.1 $[M+H]^+$ 9 % yield |

(continued)

| Interme diate number | Chemical structure Compound name | Starting materials | Analytics/yield |
|---|---|---|---|
| 3 | 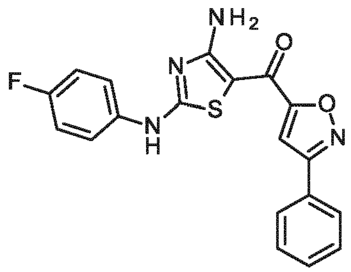 [4-amino-2-(4-fluoroanilino)thiazol-5-yl]-(3-phenylisoxazol-5-yl)methanone | 1-fluoro-4-isothiocyanatobenze ne; 2-bromo-1-(3-phenyl-1,2-oxazol-5-yl)ethanone | LC-MS (method 2) Rt = 0.99 min; MS (ESIpos): m/z = 381.2 $[M+H]^+$ 87 % yield |
| 4 | 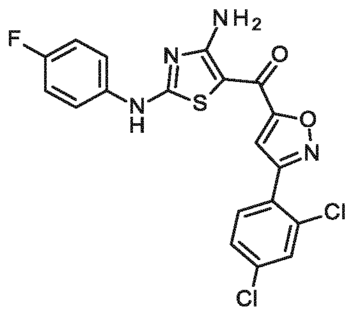 [4-amino-2-(4-fluoroanilino)thiazol-5-yl]-[3-(2,4-dichlorophenyl)isoxazol-5-yl]methanone | 1-fluoro-4-isothiocyanatobenze ne; 2-bromo-1-[3-(2,4-dichlorophenyl)-1,2-oxazol-5-yl]ethanone | LC-MS (method 2) Rt = 1.14 min; MS (ESIpos): m/z = 449.2 $[M+H]^+$ 88 % yield |
| 5 | 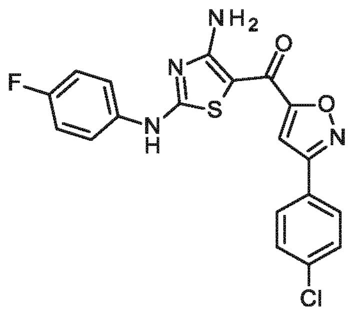 [4-amino-2-(4-fluoroanilino)thiazol-5-yl]-[3-(4-chlorophenyl)isoxazol-5-yl]methanone | 1-fluoro-4-isothiocyanatobenze ne; 2-bromo-1-[3-(4-chlorophenyl)-1,2-oxazol-5-yl] ethanone | $^1H$ NMR (400 MHz, DMSO-$d_6$) δ ppm = 7.26 (m, 2 H), 7.62 (m, 2 H), 7.64 (s, 1 H), 7.69 (m, 2 H), 8.00 (m, 2 H), 8.61 (m, 1 H), 8.76 (m, 1 H), 11.10 (br s, 1 H) LC-MS (method 2) Rt = 1.13 min; MS (ESIpos): m/z = 415.2 $[M+H]^+$ 100 % yield |
| 6 | 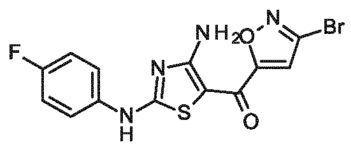 [4-amino-2-(4-fluoroanilino)-1,3-thiazol-5-yl](3-bromo-1,2-oxazol-5-yl)methanone | 1-fluoro-4-isothiocyanatobenze ne; 2-bromo-1-(3-bromoisoxazol-5-yl)ethanone | LC-MS (method 2) Rt = 0.85 min; MS (ESIpos): m/z = 383.1 $[M+H]^+$ 70 % yield |

(continued)

| Interme diate number | Chemical structure Compound name | Starting materials | Analytics/yield |
|---|---|---|---|
| **7** | 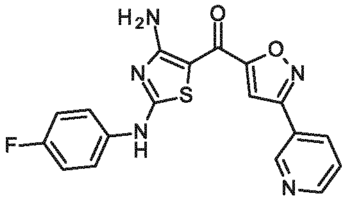 [4-amino-2-(4-fluoroanilino)-1,3-thiazol-5-yl][3-(pyridin-3-yl)-1,2-oxazol-5-yl]methanone | 1-fluoro-4-isothiocyanatobenze ne; 2-bromo-1-[3-(pyridin-3-yl)-1,2-oxazol-5-yl] ethanone (Intermediate 10) | LC-MS (method 2) Rt = 1.04 min; MS (ESIpos): m/z = 382.2 $[M+H]^+$ 5 % yield |
| **8** | 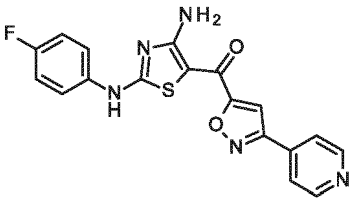 [4-amino-2-(4-fluoroanilino)thiazol-5-yl]-[3-(4-pyridyl) isoxazol-5-yl]methanone | 1-fluoro-4-isothiocyanatobenze ne; 2-bromo-1-[3-(pyridin-4-yl)-1,2-oxazol-5-yl] ethanone (Intermediate 11) | LC-MS (method 2) Rt = 0.75 min; MS (ESIpos): m/z = 382.3 $[M+H]^+$ 30 % yield |

**Intermediate** 9

2-bromo-1-[3-(4-chlorophenyl)-1,2,4-oxadiazol-5-yl]ethanone

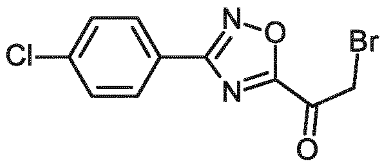

1-[3-(4-chlorophenyl)-1,2,4-oxadiazol-5-yl]ethenone (500 mg, 2.25 mmol) in THF (12.5 mL) at 0°C was treated with phenyl trimethyl ammonium tribromide (844 mg, 2.25 mmol). The reaction mixture was stirred at rt overnight, diluted with water and extracted three times with dichloromethane. The combined organic phases were washed with brine, filtrated and evaporated to dryness. The crude product was purified by Biotage (method X) to give 383 mg (1.27 mmol, 56 % yield) of the title compound.

$^1$H NMR (400 MHz, DMSO-$d_6$) $\delta$ ppm = 5.00 (s, 2 H), 7.71 (d, J=8.7 Hz, 2 H), 8.09 (d, J=8.7 Hz, 2 H).

**Intermediate** 10

2-bromo-1-[3-(pyridin-3-yl)-1,2-oxazol-5-yl]ethanone

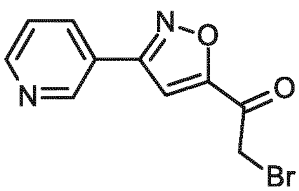

1-[3-(3-pyridyl)isoxazol-5-yl]ethanone (100 mg, 0.53 mmol) and 1-bromopyrrolidine-2,5-dione (189 mg, 1.06

mmol) were solved in acetonitrile (2.5 mL) and treated with trimethyl silyl trifluormethane sulfonate (118 mg, 0.53 mmol, TMS-OTf). The reaction mixture was stirred for 9 h at 40°C followed by the addition of further 1-bromopyrrolidine-2,5-dione (189 mg, 1.06 mmol). After stirring at 40°C overnight, water was added to the reaction mixture followed by the extraction with ethyl acetate. The combined organic phases were washed with brine, filtered through a water repellent filter circle (MN 617 WA) and evaporated to dryness. The crude product (quant.) was used without further purification.

LC-MS (method 1): Rt = 0.83 min; MS(ESIpos) m/z = 266.9 $[M+H]^+$

**Intermediate 11**

2-bromo-1-[3-(pyridin-4-yl)-1,2-oxazol-5-yl]ethanone

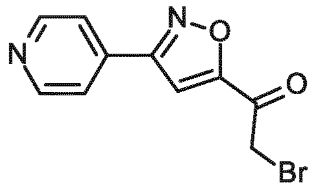

1-[3-(4-pyridyl)isoxazol-5-yl]ethanone (250 mg, 1.33 mmol) and 1-bromopyrrolidine-2,5-dione (473 mg, 2.66 mmol) were solved in acetonitrile (6 mL) and treated with trimethyl silyl trifluormethane sulfonate (295 mg, 1.33 mmol, TMS-OTf). The reaction mixture was stirred for 9 h at 40°C followed by the addition of further 1-bromopyrrolidine-2,5-dione (473 mg, 2.66 mmol). After stirring at 40°C for 4 h and rt for further 48 h, water was added to the reaction mixture followed by the extraction with ethyl acetate. The combined organic phases were washed with brine, filtered through a water repellent filter circle (MN 617 WA) and evaporated to dryness. The crude product (quant.) was used without further purification.

**Intermediate 12**

Ethyl 5-[4-amino-2-(4-fluoroanilino)thiazole-5-carbonyl]isoxazole-3-carboxylate

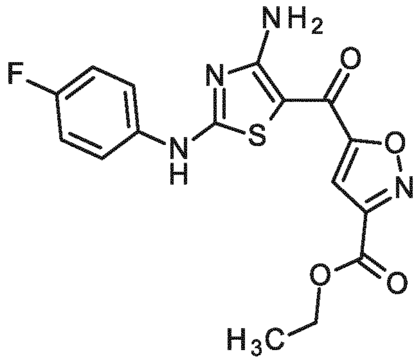

1-fluoro-4-isothiocyanatobenzene (250 mg, 1.63 mmol) was dissolved in acetonitrile (15 mL) followed by the addition of 1,8-diazabicyclo(5.4.0)undec-7-ene (248.5 mg, 1.63 mmol) and cyanamide (82.3 mg, 1.96 mmol). After stirring for 45 min at rt, further 1,8-diazabicyclo(5.4.0)undec-7-ene (96 mg, 0.62 mmol) and ethyl 5-(bromoacetyl)-1,2-oxazole-3-carboxylate (124 mg, 0.81 mmol) dissolved in acetonitrile (6 mL) were added. The reaction mixture was stirred at rt overnight. The suspension was treated with water and the precipitate was isolated by filtration, washed with water and some ethyl acetate and dried by lyophilization to give 586 mg (1.25 mmol, 76 %) of the title compound.

$^1H$ NMR (400 MHz, DMSO-$d_6$) δ ppm = 1.33 (t, J=7.10 Hz, 3 H), 4.39 (q, J=7.10 Hz, 2 H), 7.22 - 7.26 (m, 3 H), 7.67 - 7.70 (m, 2 H), 8.70 - 8.74 (m, 2 H), 11.13 (s, 1 H).

LC-MS (method 2): Rt = 0.86 min; MS(ESIpos) m/z = 377.3 $[M+H]^+$

**Intermediate 13**

5-[4-amino-2-(4-fluoroanilino)thiazole-5-carbonyl]isoxazole-3-carboxylic acid

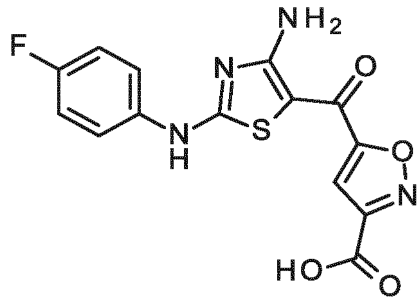

Ethyl 5-[4-amino-2-(4-fluoroanilino)thiazole-5-carbonyl]isoxazole-3-carboxylate (2.3 g, 6.1 mmol, Intermediate 12) was suspended in THF (60 mL) and treated with 1M aqueous sodium hydroxide (61 mL, 61 mmol). The reaction mixture was stirred overnight followed by the addition of 2M aqueous hydrochloric acid up to pH 4. The precipitate was filtered off, washed with water and dried to yield 1.73 g (4.96 mmol, 81%) of the title compound.

$^{1}$H NMR (400 MHz, DMSO-$d_6$) δ ppm = 4.42 (br s, 1 H), 7.23 (m, 3 H), 7.68 (m, 2 H), 8.70 (m, 2 H), 11.12 (s, 1 H).

LC-MS (method 1): Rt = 0.84 min; MS(ESIpos) m/z = 349.2 $[M+H]^+$

**Intermediate 14**

rac-5-[4-amino-2-(N-(2-amino-1-methyl-2-oxo-ethyl)-4-fluoro-anilino)thiazole-5-carbonyl]isoxazole-3-carboxylic acid

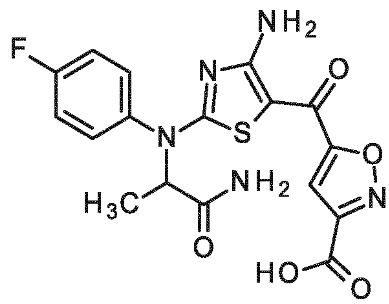

rac-Ethyl 5-[4-amino-2-(N-(2-amino-1-methyl-2-oxo-ethyl)-4-fluoro-anilino)thiazole-5-carbonyl]isoxazole-3-carboxylate (1.99 g, 4.45 mmol, Example 9) was suspended in THF (42 mL) and treated with 1M aqueous sodium hydroxide (45 mL, 45 mmol). The reaction mixture was stirred overnight followed by the addition of 2M aqueous hydrochloric acid up to pH 5. The solution was extracted three times with ethyl acetate. The combined organic phases were washed with brine, filtered through a water repellent filter circle (MN 617 WA) and evaporated to dryness to give 383 mg (21 % yield) of the title compound.

LC-MS (method 1): Rt = 0.74 min; MS(ESIpos) m/z = 420.1 $[M+H]^+$

**Intermediate 15**

5-[4-amino-2-(4-fluoroanilino)thiazole-5-carbonyl]-N-(1-methylcyclopentyl)isoxazole-3-carboxamide

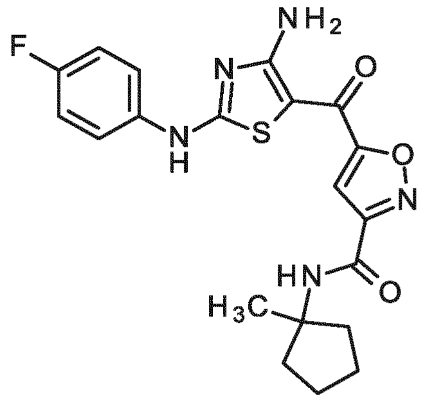

5-[4-amino-2-(4-fluoroanilino)thiazole-5-carbonyl]isoxazole-3-carboxylic acid (50 mg, 144 μmol, Intermediate 13) and 1-methylcyclopentanamine (17.1 mg, 173 μmol) were dissolved in dimethylformamide (1 mL). N,N-diisopropylethylamine (27.8 mg, 215 μmol) and HATU (65.5 mg, 173 μmol) were added. The reaction mixture was stirred at rt for 1 h, then evaporated to dryness in vacuo and triturated with isopropanol. The organic phase was filtrated and evaporated to dryness. The crude product was purified by RP-HPLC (method C, basic) to give 23 mg (37 % yield) of the title compound.

LC-MS (method 2): Rt = 0.95 min; MS(ESIpos) m/z = 430.5 $[M+H]^+$

**Intermediate 16**

rac-5-[4-amino-2-(4-fluoroanilino)thiazole-5-carbonyl]-N-(3,3-dimethylcyclopentyl)isoxazole-3-carboxamide

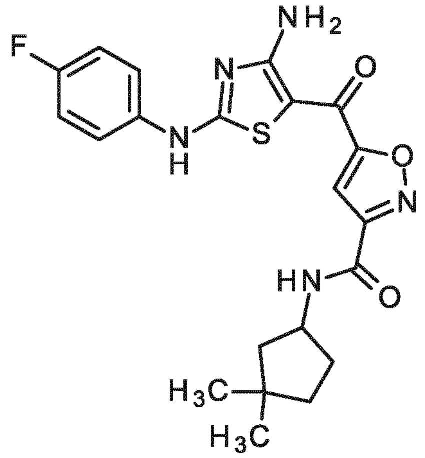

5-[4-amino-2-(4-fluoroanilino)thiazole-5-carbonyl]isoxazole-3-carboxylic acid (50 mg, 144 μmol, Intermediate 13) and rac-3,3-dimethylcyclopentanamine (19.5 mg, 173 μmol) were dissolved in dimethylformamide (1 mL). N,N-diisopropylethylamine (27.8 mg, 215 μmol) and HATU (65.5 mg, 173 μmol) were added. The reaction mixture was stirred at rt for 1 h followed by the addition of water. The precipitate was filtered off, washed with water and dried in vacuo to yield 20 mg (50 μmol, 31%) of the title compound.

$^1$H NMR (400 MHz, DMSO-$d_6$) δ ppm 0.98 (s, 3 H), 1.07 (s, 3 H), 1.44 (m, 2 H), 1.54 (m, 1 H), 1.67 (m, 1 H), 1.77 (m, 1 H), 2.03 (m, 1 H), 4.35 (m, 1 H), 7.20 (s, 1 H), 7.26 (t, J=8.87 Hz, 2 H), 7.70 (m, 2 H), 8.69 (m, 2 H), 8.85 (d, J=7.60 Hz, 1 H), 11.13 (m, 1 H).

LC-MS (method 2): Rt = 0.99 min; MS(ESIpos) m/z = 444.4 $[M+H]^+$

**Intermediate 17**

rac-5-[4-amino-2-(4-fluoroanilino)thiazole-5-carbonyl]-N-(2,2-dimethylcyclopentyl)isoxazole-3-carboxamide

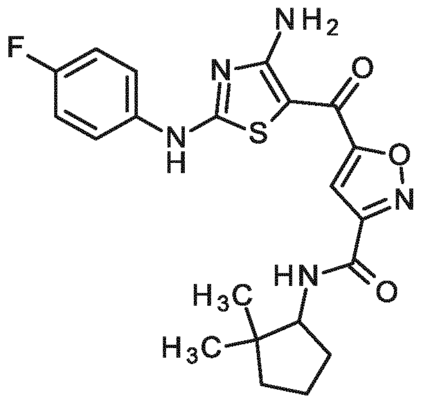

5-[4-amino-2-(4-fluoroanilino)thiazole-5-carbonyl]isoxazole-3-carboxylic acid (50 mg, 144 µmol, Intermediate 13) and rac-2,2-dimethylcyclopentanamine (19.5 mg, 173 µmol) were dissolved in dimethylformamide (1 mL). N,N-diisopropylethylamine (27.8 mg, 215 µmol) and HATU (65.5 mg, 173 µmol) were added. The reaction mixture was stirred at rt for 1 h followed by the addition of water. The solution was extracted three times with ethyl acetate. The combined organic phases were washed with saturated aqueous ammonium chloride solution, filtered through a water repellent filter circle (MN 617 WA) and evaporated to dryness. The crude product was purified by RP-HPLC (method C, basic) to give 13 mg (20 % yield) of the title compound.

LC-MS (method 2): Rt = 0.99 min; MS(ESIpos) m/z = 444.5 $[M+H]^+$

Intermediate 18

rac-5-[4-amino-2-(4-fluoroanilino)thiazole-5-carbonyl]-N-(3,3-difluorocyclopentyl)isoxazole-3-carboxamide

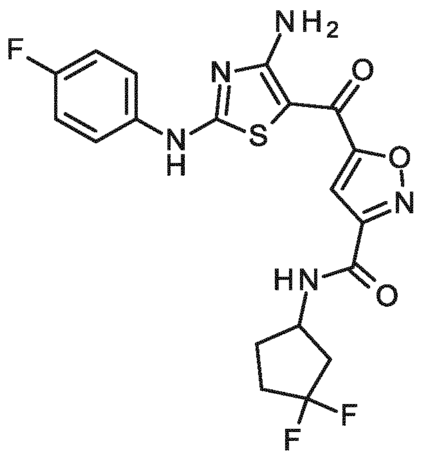

5-[4-amino-2-(4-fluoroanilino)thiazole-5-carbonyl]isoxazole-3-carboxylic acid (50 mg, 144 µmol, Intermediate 13) and rac-3,3-difluorocyclopentanamine; hydrochloride (27.2 mg, 173 µmol) were dissolved in dimethylformamide (1 mL). N,N-diisopropylethylamine (27.8 mg, 215 µmol) and HATU (65.5 mg, 173 µmol) were added. The reaction mixture was stirred at rt for 1 h followed by the addition of water. The precipitate was filtered off, washed with water and dried in vacuo to yield 34 mg (70 µmol, 52 %) of the title compound.

LC-MS (method 2): Rt = 0.83 min; MS(ESIpos) m/z = 452.3 $[M+H]^+$

**Intermediate** 19

5-[4-amino-2-(4-fluoroanilino)thiazole-5-carbonyl]-N-isopropyl-isoxazole-3-carboxamide

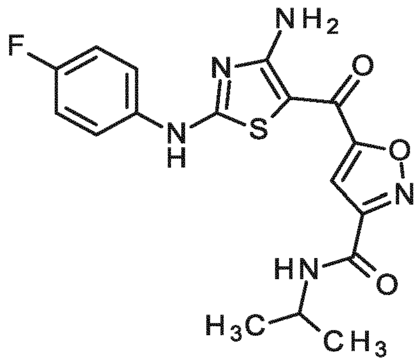

5-[4-amino-2-(4-fluoroanilino)thiazole-5-carbonyl]isoxazole-3-carboxylic acid (50 mg, 144 μmol, Intermediate 13) and propane-2-amine (10.2 mg, 173 μmol) were dissolved in dimethylformamide (1 mL). N,N-diisopropylethylamine (27.8 mg, 215 μmol) and HATU (65.5 mg, 173 μmol) were added. The reaction mixture was stirred at rt for 48 h followed by the addition of water. The precipitate was filtered off, washed with water and dried in vacuo to yield 11.9 mg (30 μmol, 21 %) of the title compound.

LC-MS (method 2): Rt = 0.76 min; MS(ESIpos) m/z = 390.3 $[M+H]^+$

**Intermediate** 20

rac-5-[4-amino-2-(4-fluoroanilino)thiazole-5-carbonyl]-N-(2-methoxy-1-methyl-ethyl)isoxazole-3-carboxamide

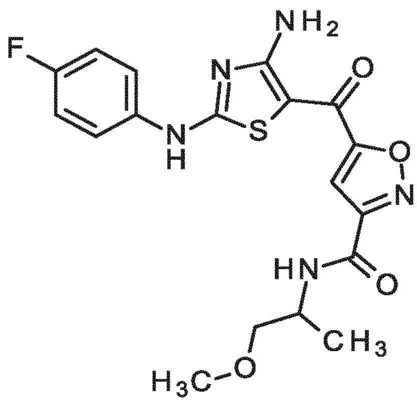

5-[4-amino-2-(4-fluoroanilino)thiazole-5-carbonyl]isoxazole-3-carboxylic acid (50 mg, 144 μmol, Intermediate 13) and (2RS)-1-methoxypropan-2-amine (15.4 mg, 173 μmol) were dissolved in dimethylformamide (1 mL). N,N-diisopropylethylamine (27.8 mg, 215 μmol) and HATU (65.5 mg, 173 μmol) were added. The reaction mixture was stirred at rt for 48 h followed by the addition of water. The solution was extracted three times with ethyl acetate. The combined organic phases were washed with saturated aqueous ammonium chloride solution, filtered through a water repellent filter circle (MN 617 WA) and evaporated to dryness to give 60 mg (quant., crude) of the title compound.

LC-MS (method 2): Rt = 0.74 min; MS(ESIpos) m/z = 420.3 $[M+H]^+$

**Intermediate 21**

5-[4-amino-2-(4-fluoroanilino)thiazole-5-carbonyl]-N-[2-fluoro-1-(fluoromethyl)ethyl]isoxazole-3-carboxamide

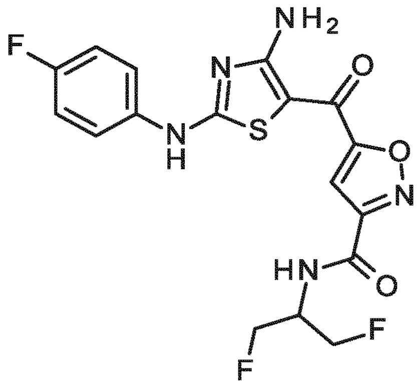

5-[4-amino-2-(4-fluoroanilino)thiazole-5-carbonyl]isoxazole-3-carboxylic acid (50 mg, 144 μmol, Intermediate 13) and 1,3-difluoropropan-2-amine (16.4 mg, 173 μmol) were dissolved in dimethylformamide (1 mL). N,N-diisopropylethylamine (27.8 mg, 215 μmol) and HATU (65.5 mg, 173 μmol) were added. The reaction mixture was stirred at rt for 48 h followed by the addition of water. The precipitate was filtered off, washed with water and dried in vacuo to yield 38.8 mg (91 μmol, 63 %) of the title compound.

LC-MS (method 2): Rt = 0.72 min; MS(ESIpos) m/z = 424.3 $[M+H]^+$

**Intermediate 22**

5-[4-amino-2-(4-fluoroanilino)thiazole-5-carbonyl]-N-(cyanomethyl)isoxazole-3-carboxamide

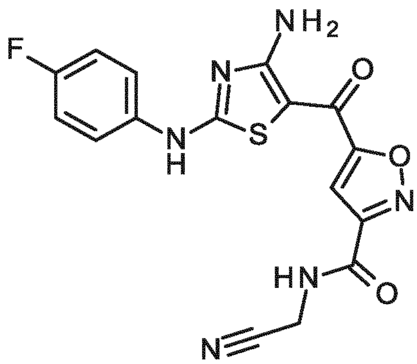

5-[4-amino-2-(4-fluoroanilino)thiazole-5-carbonyl]isoxazole-3-carboxylic acid (50 mg, 144 μmol, Intermediate 13) and aminoacetonitrile, hydrochloride (15.9 mg, 173 μmol) were dissolved in dimethylformamide (1 mL). N,N-diisopropylethylamine (27.8 mg, 215 μmol) and HATU (65.5 mg, 173 μmol) were added. The reaction mixture was stirred at rt for 48 h followed by the addition of water. The precipitate was filtered off, washed with water and dried in vacuo to yield 46.2 mg (119 μmol, 83 %) of the title compound.

LC-MS (method 2): Rt = 0.65 min; MS(ESIpos) m/z = 387.2 $[M+H]^+$

**Intermediate** 23

rac-5-[4-amino-2-(4-fluoroanilino)thiazole-5-carbonyl]-N-(2,2-difluoro-1-methyl-ethyl)isoxazole-3-carboxamide

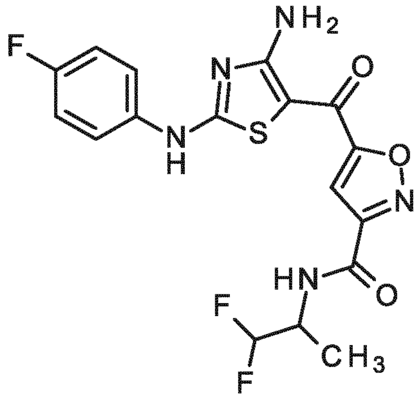

5-[4-amino-2-(4-fluoroanilino)thiazole-5-carbonyl]isoxazole-3-carboxylic acid (50 mg, 144 μmol, Intermediate 13) and (2RS)-1,1-difluoropropan-2-amine, hydrochloride (22.6 mg, 173 μmol) were dissolved in dimethylformamide (1 mL). N,N-diisopropylethylamine (27.8 mg, 215 μmol) and HATU (65.5 mg, 173 μmol) were added. The reaction mixture was stirred at rt for 2 h followed by the addition of water. The precipitate was filtered off, washed with water and dried in vacuo to yield 35.2 mg (83 μmol, 58 %) of the title compound.

LC-MS (method 2): Rt = 0.76 min; MS(ESIpos) m/z = 426.3 $[M+H]^+$

## Intermediate 24

5-[4-amino-2-(4-fluoroanilino)thiazole-5-carbonyl]-N-(cis-3-fluorocyclobutyl)isoxazole-3-carboxamide

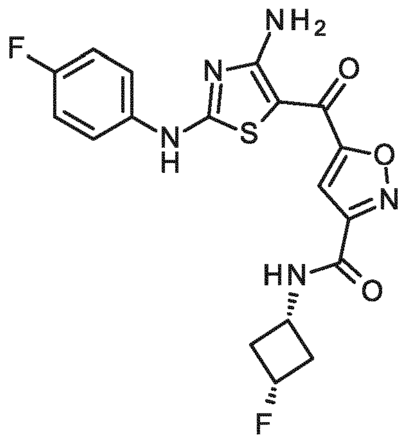

5-[4-amino-2-(4-fluoroanilino)thiazole-5-carbonyl]isoxazole-3-carboxylic acid (50 mg, 144 μmol, Intermediate 13) and cis-3-fluorocyclobutanamine (15.4 mg, 173 μmol) were dissolved in dimethylformamide (1 mL). N,N-diisopropylethylamine (27.8 mg, 215 μmol) and HATU (65.5 mg, 173 μmol) were added. The reaction mixture was stirred at rt for 2 h followed by the addition of water. The precipitate was filtered off, washed with water and dried in vacuo to yield 61.8 mg (quant.) of the title compound.

LC-MS (method 2): Rt = 0.79 min; MS(ESIpos) m/z = 420.3 $[M+H]^+$

## Intermediate 25

5-[4-amino-2-(4-fluoroanilino)thiazole-5-carbonyl]-N-(trans-3-fluorocyclobutyl)isoxazole-3-carboxamide

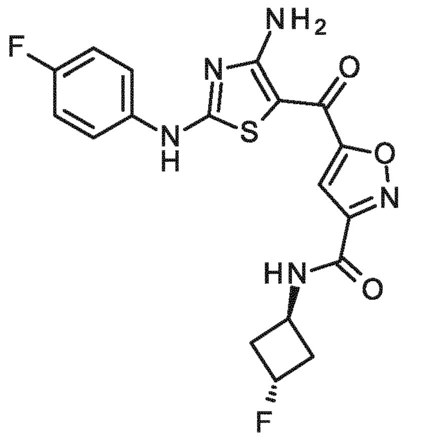

5-[4-amino-2-(4-fluoroanilino)thiazole-5-carbonyl]isoxazole-3-carboxylic acid (50 mg, 144 µmol, Intermediate 13) and trans-3-fluorocyclobutanamine (15.4 mg, 173 µmol) were dissolved in dimethylformamide (1 mL). N,N-diisopropylethylamine (27.8 mg, 215 µmol) and HATU (65.5 mg, 173 µmol) were added. The reaction mixture was stirred at rt for 2 h followed by the addition of water. The precipitate was filtered off, washed with water and dried in vacuo to yield 46.1 mg (110 µM, 77 %) of the title compound.

LC-MS (method 2): Rt = 0.79 min; MS(ESIpos) m/z = 420.3 $[M+H]^+$

## Intermediate 26

Ethyl 5-[4-amino-2-(3,4-difluoroanilino)thiazole-5-carbonyl]isoxazole-3-carboxylate

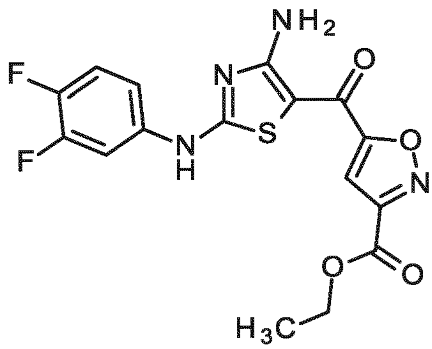

1,2-difluoro-4-isothiocyanatobenzene (3.27 g, 19 mmol) was dissolved in acetonitrile (60 mL) followed by the addition of 1,8-diazabicyclo(5.4.0)undec-7-ene (2.9 g, 19 mmol) and cyanamide (0.96 g, 22.9 mmol). After stirring for 45 min at rt, further 1,8-diazabicyclo(5.4.0)undec-7-ene (1.46 g, 9.5 mmol) and ethyl 5-(bromoacetyl)-1,2-oxazole-3-carboxylate (5 g, 19 mmol) dissolved in acetonitrile (28 mL) were added. The reaction mixture was stirred at rt overnight. The suspension was treated with water and the precipitate was isolated by filtration, washed with water and some ethyl acetate and dried by lyophilization to give 5.89 g (14.48 mmol, 76 %) of the title compound.

$^1H$ NMR (400 MHz, DMSO-$d_6$) δ ppm = 1.34 (t, J=7.1 Hz, 3 H), 4.39 (q, J=7.1 Hz, 2 H), 7.25 (s, 1 H), 7.31 (m, 1 H), 7.47 (dt, J=10.39, 9.12 Hz, 1 H), 8.02 (ddd, J=12.86, 7.29, 2.41 Hz, 1 H), 8.75 (br d, *J*=2.03 Hz, 2 H), 11.28 (s, 1 H).

LC-MS (method 2): Rt = 0.81 min; MS(ESIpos) m/z = 395.2 $[M+H]^+$

## Intermediate 27

5-[4-amino-2-(3,4-difluoroanilino)thiazole-5-carbonyl]isoxazole-3-carboxylic acid

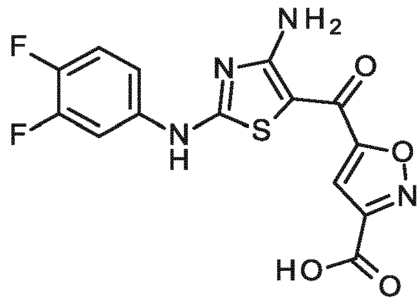

Ethyl 5-[4-amino-2-(3,4-difluoroanilino)thiazole-5-carbonyl]isoxazole-3-carboxylate (3.64 g, 9.23 mmol, Intermediate 26) was suspended in THF (90 mL) and treated with 1M aqueous sodium hydroxide (92 mL, 92 mmol). The reaction mixture was stirred at rt for 3h followed by the addition of 2M aqueous hydrochloric acid up to pH 3. The organic solvent was evaporated, and the aqueous phase was extracted three times with ethyl acetate. The combined organic phases were filtered through a water repellent filter circle (MN 617 WA) and evaporated to dryness to give 3.25 g (8.9 mmol, 96 %) of the title compound.

$^{1}H$ NMR (400 MHz, DMSO-$d_6$) δ ppm = 7.18 (s, 1 H), 7.32 (m, 1 H), 7.47 (dt, *J=10.39,* 9.12 Hz, 1 H), 8.02 (ddd, J=12.80, 7.35, 2.41 Hz, 1 H), 8.74 (br s, 2 H), 11.27 (s, 1 H), 14.33 (m, 1 H).

LC-MS (method 1): Rt = 0.47 min; MS(ESIpos) m/z = 367.2 $[M+H]^+$

## Intermediate 28

5-{[4-amino-2-(3,4-difluoroanilino)-1,3-thiazol-5-yl]carbonyl}-N-cyclopentyl-1,2-oxazole-3-carboxamide

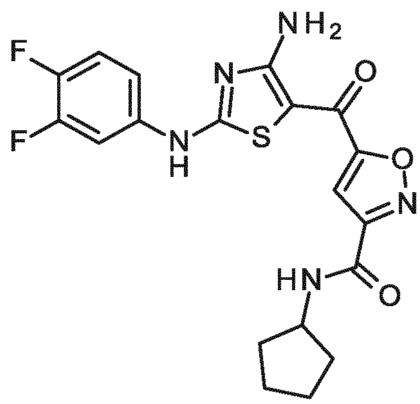

5-[4-amino-2-(3,4-difluoroanilino)thiazole-5-carbonyl]isoxazole-3-carboxylic acid (280 mg, 0.76 mmol, Intermediate 27) and cyclopentanamine (65.1 mg, 0.76 mmol) were dissolved in dimethyl sulfoxide (5.6 mL). N,N-diisopropylethylamine (148 mg, 1.15 mmol) and HATU (436 mg, 1.15 mmol) were added. The reaction mixture was stirred at rt for 2 h and then treated with water. The precipitate was filtered off, washed with water and dried in vacuo to give 228 mg (0.53 mmol, 69 % yield) of the title compound.

LC-MS (method 2): Rt = 0.88 min; MS(ESIpos) m/z = 434.3 $[M+H]^+$

## Intermediate 29

5-[4-amino-2-(3,4-difluoroanilino)thiazole-5-carbonyl]-N-(1-methylcyclobutyl)isoxazole-3-carboxamide

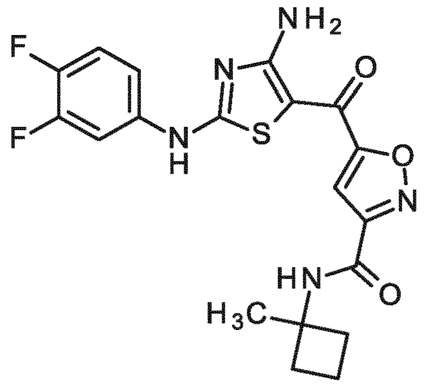

5-[4-amino-2-(3,4-difluoroanilino)thiazole-5-carbonyl]isoxazole-3-carboxylic acid (250 mg, 0.68 mmol, Intermediate 27) and 1-methylcyclobutanamine;hydrochloride (83 mg, 0.68 mmol) were dissolved in dimethyl sulfoxide (5 mL). N,N-diisopropylethylamine (176 mg, 1.36 mmol) and HATU (389 mg, 1.0 mmol) were added. The reaction mixture was stirred at rt for 2 h and then treated with water. The precipitate was filtered off, washed with water and dried in vacuo to give 228 mg (0.66 mmol, 96 % yield) of the title compound.

LC-MS (method 2): Rt = 0.88 min; MS(ESIpos) m/z = 434.3 $[M+H]^+$

## Intermediate 30

5-[4-amino-2-(3,4-difluoroanilino)thiazole-5-carbonyl]-N-(3,3-difluorocyclobutyl)isoxazole-3-carboxamide

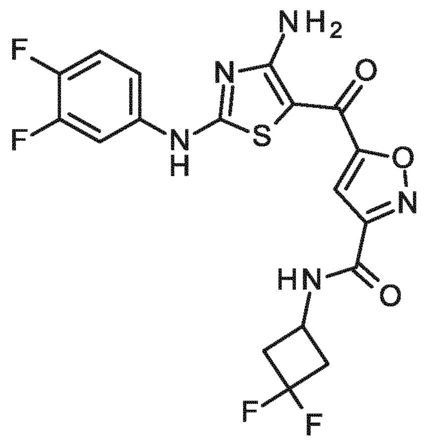

5-[4-amino-2-(3,4-difluoroanilino)thiazole-5-carbonyl]isoxazole-3-carboxylic acid (250 mg, 0.68 mmol, Intermediate 27) and 3,3-difluorocyclobutanamine, hydrochloride (98 mg, 0.68 mmol) were dissolved in dimethyl sulfoxide (5 mL). N,N-diisopropylethylamine (176 mg, 1.36 mmol) and HATU (389 mg, 1.0 mmol) were added. The reaction mixture was stirred at rt for 2 h and then treated with water. The precipitate was filtered off, washed with water and dried in vacuo to give 288 mg (0.63 mmol, 93 % yield) of the title compound.

LC-MS (method 2): Rt = 0.79 min; MS(ESIpos) m/z = 456.3 $[M+H]^+$

## Intermediate 31

Ethyl 2-[4-amino-2-(4-fluoroanilino)thiazol-5-yl]-2-oxo-acetate

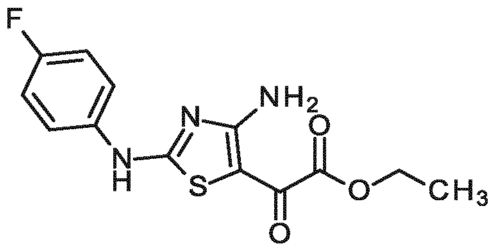

1-fluoro-4-isothiocyanatobenzene (393 mg, 2.56 mmol) was dissolved in acetonitrile (12 mL) followed by the addition of 1,8-diazabicyclo(5.4.0)undec-7-ene (390 mg, 2.57 mmol) and cyanamide (129.3 mg, 3.1 mmol). After stirring for 45 min at rt, further 1,8-diazabicyclo(5.4.0)undec-7-ene (195 mg, 1.28 mmol) and ethyl 3-bromopyruvate (500 mg, 2.56 mmol) dissolved in acetonitrile (4 mL) were added. The reaction mixture was stirred at rt overnight. The suspension was treated with water and extracted with ethyl acetate. The combined organic phases were washed with brine, dried over sodium sulfate, filtered off and evaporated to dryness. The residue was purified by column chromatography (method Y) to give 289 mg (0.93 mmol, 36 %) of the title compound.

$^1$H NMR (400 MHz, DMSO-$d_6$) δ ppm = 1.32 (m, 1 H), 4.23 (d, *J*=7.10 Hz, 2 H), 7.24 (t, *J*=8.87 Hz, 3 H), 7.67 (m, 2 H), 8.65 (s, 2 H), 11.02 (s, 1 H).

LC-MS (method 2): Rt = 0.75 min; MS(ESIpos) m/z = 310.2 $[M+H]^+$

## Intermediate 32

4-amino-2-(4-fluoroanilino)thiazol-5-yl]-(3-isopropyl-1,2,4-oxadiazol-5-yl)methanone

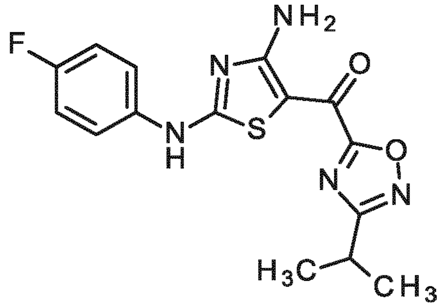

N-Hydroxyisobutyramidine (14 mg, 0.14 mmol) was solved in dry THF (1.5 mL) and treated with sodium hydride (60% in mineral oil, 6.5 mg, 0.16 mmol) for 1 h at rt followed by the addition of ethyl 2-[4-amino-2-(4-fluoroanilino)thiazol-5-yl]-2-oxo-acetate (50 mg, 0.16 mmol, Intermediate 31). The reaction mixture was stirred at 50°C for 3 h, filtrated and purified by by RP-HPLC (method B, basic) to give 19 mg (34 % yield) of the title compound.

$^1$H NMR (400 MHz, DMSO-$d_6$) δ ppm = 1.31 (d, *J*=6.84 Hz, 6 H) 3.10 - 3.21 (m, 1 H) 7.17 - 7.30 (m, 2 H) 7.63 - 7.75 (m, 2 H) 8.73 - 8.96 (m, 2 H) 11.10 - 11.20 (m, 1 H).

LC-MS (method 2): Rt = 0.84 min; MS(ESIpos) m/z = 348.4 $[M+H]^+$

## Intermediate 33

Methyl 3-(difluoromethoxy)-1,2-oxazole-5-carboxylate

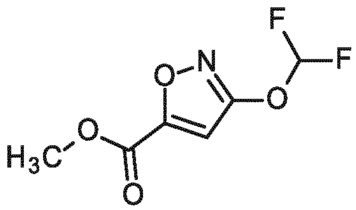

To a mixture of methyl 3-hydroxy-1,2-oxazole-5-carboxylate (2.00 g, 14.0 mmol) in N,N-dimethylformamide (30 mL) were added chloro difluoro acetic acid sodium salt (5.31 g, 34.9 mmol) and potassium carbonate (2.9 g, 21.0 mmol) at room temperature. The mixture was stirred at 80 °C for 3 hours. The reaction mixture was poured into water and extracted with ethyl acetate. The combined organic phase was washed with brine, dried over anhydrous sodium sulfate and filtered. The filtrate was concentrated under reduced pressure to give a crude product as a white solid. The crude product was purified by column chromatography on silica gel (petroleum ether: ethyl acetate = 3: 1) to give methyl 3-(difluoromethoxy)-1,2-oxazole-5-carboxylate (1.60 g, 59% yield) as a white solid.
LC-MS (method 4): Rt = 0.621 min; MS (ESIpos): m/z = 193.7 $[M+H]^+$.

## Intermediate 34

3-(difluoromethoxy)-1,2-oxazole-5-carboxylic acid

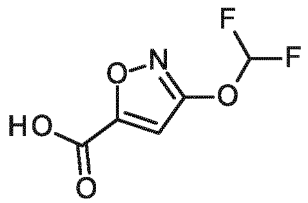

To a solution of methyl 3-(difluoromethoxy)-1,2-oxazole-5-carboxylate (1.60 g, 8.29 mmol, Intermediate 33) in methanol (24 mL) were added lithium hydroxide monohydrate (3.48 g, 82.9 mmol) and water (4.8 mL) at room temperature. The mixture was stirred at room temperature for 2 hours. Concentrated hydrochloric acid was added to the reaction to adjust the pH = 4. The mixture was concentrated in vacuo to give a residue as a white solid. The residue was diluted in water and filtered. The filter cake was dried in vacuo to give 3-(difluoromethoxy)-1,2-oxazole-5-carboxylic acid (1.40 g, 94% yield) as a white solid.

## Intermediate 35

3-(difluoromethoxy)-N-methoxy-N-methyl-1,2-oxazole-5-carboxamide

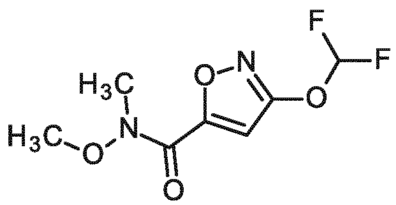

To a solution of 3-(difluoromethoxy)-1,2-oxazole-5-carboxylic acid (700 mg, 3.91 mmol, Intermediate 34), N,N-diisopropylethylamine (2.0 mL, 12 mmol) and O-(7-azabenzotriazol-1-yl)-N,N,N,N-tetrametyluronium hexafluorophosphate (1.78 g, 4.69 mmol) in N,N-dimethylformamide (14 mL) was added N-methoxymethanamine-hydrochloric acid salt (458 mg, 4.69 mmol) at room temperature. The reaction mixture was stirred at room temperature for 1 hour. The reaction mixture was diluted with water and extracted with ethyl acetate. The combined organic layers were washed with brine, dried over anhydride sodium sulfate and filtered. The filtrate was concentrated in vacuo to give a residue as a yellow solid. The residue was purified by column chromatography on silica gel (petroleum ether: ethyl acetate = 10: 1 then 3: 1) to give 3-(difluoromethoxy)-N-methoxy-N-methyl-1,2-oxazole-5-carboxamide (300 mg, 35% yield) as a yellow solid.
LC-MS (method 4): Rt = 0.686 min; MS (ESIpos): m/z = 222.8 $[M+H]^+$.

## Intermediate 36

1-[3-(difluoromethoxy)-1,2-oxazol-5-yl]ethan-1-one

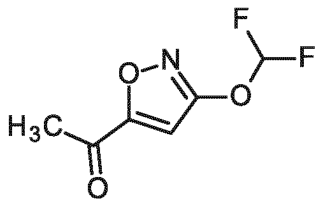

To a mixture of 3-(difluoromethoxy)-N-methoxy-N-methyl-1,2-oxazole-5-carboxamide (300 mg, 1.35 mmol, Intermediate 35) in tetrahydrofuran (8.0 mL) was added methyl magnesium bromide (1.40 mL, 4.10 mmol, 3 M in diethyl ether) at -78 °C. The mixture was stirred at -78°C for 3 hours. The reaction mixture was quenched with saturated ammonium chloride solution and extracted with ethyl acetate. The combined extracts were washed with brine, dried over anhydrous sodium sulfate and filtered. The filtrate was concentrated under vacuum to give a residue. The residue was purified by column chromatography on silica gel (300-400 mesh, petroleum ether: ethyl acetate = 10: 1) to give 1-[3-(difluoromethoxy)-1,2-oxazol-5-yl]ethanone (110 mg, 46% yield) as yellow oil.

**Intermediate 37**

Potassium N'-cyano-N-(4-fluorophenyl)carbamimidothioate

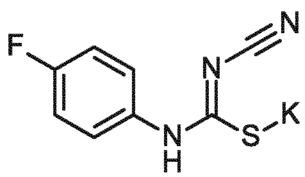

To a mixture of potassium hydroxide (1.73 g, 30.9 mmol) in ethanol (39 mL) were added cyanamide (1.30 g, 30.9 mmol) and 1-fluoro-4-isothiocyanatobenzene (4.74 g, 30.9 mmol) at room temperature. The mixture was stirred at room temperature for 0.5 hour. The reaction mixture was filtered. The filter cake was washed with ethanol (40.0 mL) and dried in vacuo to give potassium N'-cyano-N-(4-fluorophenyl)carbamimidothioate (4.90 g, 68% yield) as a white solid.
LC-MS (method 4): Rt = 0.316 min; MS (ESIpos): m/z = 195.7 $[M+H]^+$.

**Intermediate 38**

2-bromo-1-[3-(difluoromethoxy)-1,2-oxazol-5-yl]ethan-1-one

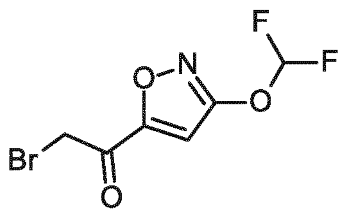

To a solution of 1-[3-(difluoromethoxy)-1,2-oxazol-5-yl]ethan-1-one (110 mg, 0.621 mmol, Intermediate 36) in dichloromethane (2.0 mL) were added bromine (0.064 mL, 1.2 mmol) and acetic acid (0.071 mL) at 0 °C. The reaction mixture was stirred at room temperature for 16 hours. The reaction mixture was concentrated in vacuo to give a residue. The residue was purified by preparative thin layer chromatography (petroleum ether: ethyl acetate = 5:1) to give 2-bromo-1-[5-bromo-4-(3-bromo-4-methoxybenzyl)-4H-1,2,4-triazol-3-yl]ethan-1-one (130 mg, 82% yield) as yellow oil.

**Intermediate 39**

[4-amino-2-(4-fluoroanilino)-1,3-thiazol-5-yl][3-(difluoromethoxy)-1,2-oxazol-5-yl]methanone

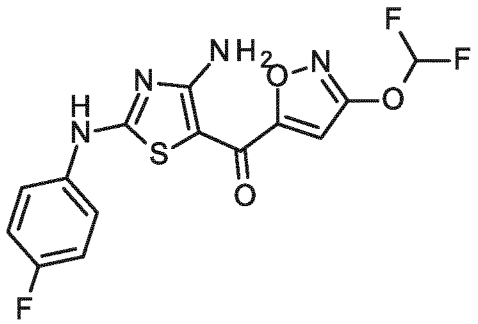

To a mixture of 2-bromo-1-[3-(difluoromethoxy)-1,2-oxazol-5-yl]ethan-1-one (130 mg, 0.508 mmol, Intermediate 38) in ethanol (2.0 mL) was added potassium N'-cyano-N-(4-fluorophenyl)carbamimidothioate (142 mg, 0.609 mmol, Intermediate 37) at room temperature. The reaction mixture was refluxed for 2 hours. The reaction mixture was concentrated in vacuo to give a residue as a brown solid. The residue was purified by preparative thin layer chromatography (petroleum ether: ethyl acetate = 1: 1) to give [4-amino-2-(4-fluoroanilino)-1,3-thiazol-5-yl][3-(difluoromethoxy)-1,2-oxazol-5-yl]methanone (60.0 mg, 32% yield) as a deep yellow solid.

LC-MS (method 4): Rt = 0.898 min; MS (ESIpos): m/z = 370.8 $[M+H]^+$.

**Intermediate 40**

N-(cyclopentylmethylidene)hydroxylamine

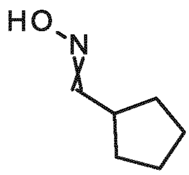

To a solution of cyclopentane carbaldehyde (3.00 g, 30.6 mmol) and hydroxylamine hydrogen chloride (1:1) (3.19 g, 45.9 mmol) in toluene (20 mL) was added potassium carbonate (3.17 g, 22.9 mmol) at 20 °C. The reaction mixture was stirred at 20 °C for 16 hours. The reaction mixture was concentrated to remove the solvent. Water was added and the mixture was extracted with ethyl acetate. The combined organic layers were washed with aqueous saturated sodium hydrogen carbonate solution and brine, dried over anhydrous sodium sulfate and filtered. The filtrate was concentrated under reduced pressure to provide a crude product as colorless oil. The crude product was purified by column chromatography on silica gel (100-200 mesh, petroleum ether: ethyl acetate = 1:0 then 10:1) to give N-(cyclopentyl methylene)hydroxylamine (3.0 g, 87% yield) as colorless oil.

**Intermediate 41**

N-hydroxy cyclopentane carboximidoyl chloride

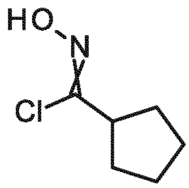

To a solution of N-(cyclopentylmethylidene)hydroxylamine (3.00 g, 26.5 mmol, Intermediate 40) in dichloromethane (40 mL) were added N-chlorosuccinimide (3.72 g, 27.8 mmol) and N,N-dimethylformamide (2.0 mL) at 20 °C. The reaction mixture was stirred at 20 °C for 3 hours. The reaction mixture was concentrated to give N-hydroxy cyclopentane carboximidoyl chloride (3.00 g, 77% yield) as yellow oil.

**Intermediate 42**

rac-1-(3-cyclopentyl-1,2-oxazol-5-yl)ethan-1-ol

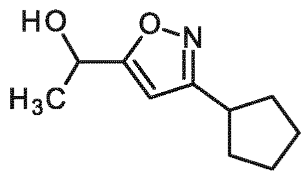

To a solution of N-hydroxy cyclopentane carboximidoyl chloride (3.00 g, 20.3 mmol, Intermediate 41) and rac-but-3-yn-2-ol (1.50 g, 21.3 mmol) in dichloromethane (40 mL) was added triethylamine (5.7 mL, 41 mmol) at 0 °C. The reaction mixture was stirred at 40 °C for 2 hours. The reaction mixture was concentrated to give a residue as a yellow solid. The residue was purified by column chromatography on silica gel (100-200 mesh, petroleum ether: ethyl acetate = 2:1) to give 1-(3-cyclopentyl-1,2-oxazol-5-yl)ethanol (2.40 g, 65% yield) as a yellow solid.
LC-MS (method 4): Rt = 0.665 min; MS (ESIpos): m/z = 182.1 $[M+H]^+$.

**Intermediate 43**

1-(3-cyclopentyl-1,2-oxazol-5-yl)ethan-1-one

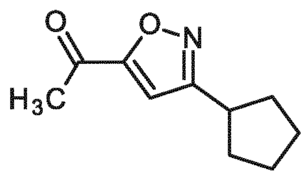

To a solution of rac-1-(3-cyclopentyl-1,2-oxazol-5-yl)ethan-1-ol (2.40 g, 13.2 mmol, Intermediate 42) in dichloromethane (30 mL) was added 1,1,1-triacetoxy-1lambda5,2-benziodoxol-3(1H)-one (11.2 g, 26.5 mmol) at 20 °C. The reaction mixture was stirred at 20 °C for 16 hours. The reaction mixture was filtered. The filtrate was concentrated under reduced pressure to give a crude product as yellow oil. The crude product was purified by column chromatography on silica gel (petroleum ether: ethyl acetate = 10:1 then 3:1) to give 1-(3-cyclopentyl-1,2-oxazol-5-yl)ethan-1-one (2.00 g, 84% yield) as yellow oil.
LC-MS (method 4): Rt = 0.74 min; MS (ESIpos): m/z = 180.1 $[M+H]^+$.

**Intermediate 44**

2-bromo-1-(3-cyclopentyl-1,2-oxazol-5-yl)ethan-1-one

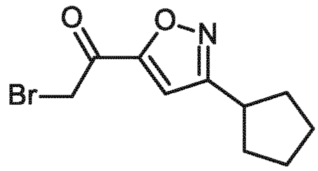

To a solution of 1-(3-cyclopentyl-1,2-oxazol-5-yl)ethan-1-one (200 mg, 1.12 mmol, Intermediate 43) in dichloromethane (2.0 mL) were added bromine (0.11 mL, 2.2 mmol) and acetic acid (0.13 mL) at 0 °C. The reaction mixture was stirred at room temperature for 16 hours. The reaction mixture was concentrated in vacuo to give 2-bromo-1-(3-cyclopentyl-1,2-oxazol-5-yl)ethan-1-one (200 mg, 69% yield) as yellow oil.

## Intermediate 45

[4-amino-2-(4-fluoroanilino)-1,3-thiazol-5-yl](3-cyclopentyl-1,2-oxazol-5-yl)methanone

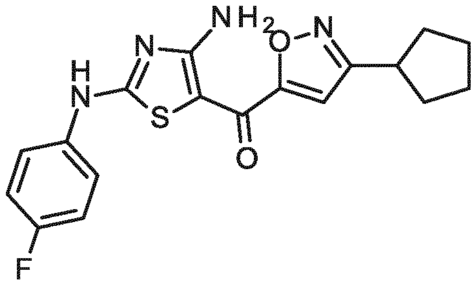

To a mixture of 2-bromo-1-(3-cyclopentyl-1,2-oxazol-5-yl)ethan-1-one (200 mg, 0.775 mmol, Intermediate 44) in ethanol (2.0 mL) was added potassium N'-cyano-N-(4-fluorophenyl) carbamimidothioate (217 mg, 0.930 mmol, Intermediate 37) at room temperature. The reaction mixture was refluxed for 2 hours. The reaction mixture was concentrated in vacuo to give a residue as a yellow solid. The residue was purified by preparative thin layer chromatography (petroleum ether: ethyl acetate = 1:1) to give [4-amino-2-(4-fluoroanilino)-1,3-thiazol-5-yl](3-cyclopentyl-1,2-oxazol-5-yl)methanone (100 mg, 35% yield) as a yellow solid.

LC-MS (method 4): Rt = 0.870 min; MS (ESIpos): m/z = 373.1 $[M+H]^+$.

## Intermediate 46

Ethyl (2,4-dichloro-1,3-thiazol-5-yl)(oxo)acetate

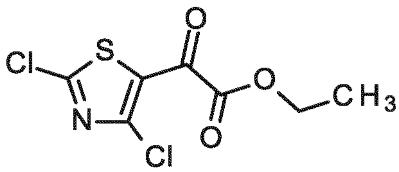

To a mixture of 2,4-dichloro-1,3-thiazole (5.00 g, 32.5 mmol) in tetrahydrofuran (100 mL) was added lithium diisopropylamide (24.0 mL, 49.0 mmol, 2 M in tetrahydrofuran-hexane solution) at -70 °C. The mixture was stirred at -70 °C for 1 hour. Diethyl ethanedioate (9.49 g, 64.9 mmol) was added to the mixture at -70 °C. The mixture was stirred at -70 °C for another 1 hour. The mixture was quenched with saturated ammonium chloride solution and extracted with ethyl acetate. The combined organic phase was dried over anhydrous sodium sulfate and filtered. The filtrate was concentrated in vacuo to give a crude product as brown oil. The crude product was purified by column chromatography on silica gel (100-200 mesh, petroleum ether: ethyl acetate = 5:1) to give ethyl (2,4-dichloro-1,3-thiazol-5-yl)(oxo)acetate (8.0 g, 97% yield) as a yellow solid.

**Intermediate 47**

Ethyl [4-chloro-2-(4-fluoroanilino)-1,3-thiazol-5-yl](oxo)acetate

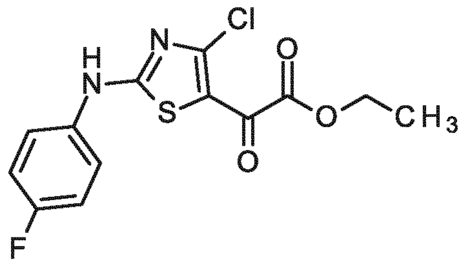

A mixture of ethyl (2,4-dichloro-1,3-thiazol-5-yl)(oxo)acetate (7.00 g, 27.5 mmol, Intermediate 46) and 4-fluoroaniline (3.37 g, 30.3 mmol) in N,N-dimethylformamide (100 mL) was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product as a yellow solid. The crude product was purified by column chromatography on silica gel (100-200 mesh, petroleum ether: ethyl acetate = 5:1 then 1:1) to give ethyl [4-chloro-2-(4-fluoroanilino)-1,3-thiazol-5-yl](oxo)acetate (6.35 g, 68% yield) as a yellow solid.

LC-MS (method 4): Rt = 0.1.162 min; MS (ESIpos): m/z = 328.7 $[M+H]^+$.

$^1$H NMR (400 MHz, DMSO-$d_6$) δ ppm = 9.48 (s, 1H), 7.44-7,39 (m, 2H), 7.18 (d, J = 8.4 Hz, 2H), 4.40 (q, J = 7.2 Hz, 2H), 1.41 (t, J = 7.2 Hz, 3H).

**Intermediate 48**

[4-chloro-2-(4-fluoroanilino)-1,3-thiazol-5-yl](oxo)acetic acid

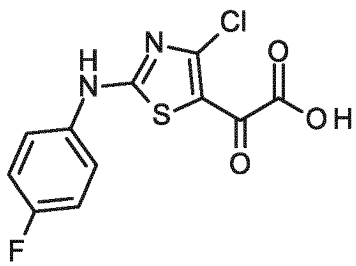

To a mixture of ethyl [4-chloro-2-(4-fluoroanilino)-1,3-thiazol-5-yl](oxo)acetate (2.00 g, 6.08 mmol, Intermediate 47) in tetrahydrofuran (30 mL) and water (10 mL) was added lithium hydroxide monohydrate (383 mg, 9.13 mmol) at 25 °C. The mixture was stirred at 25 °C for 1 hour. The reaction mixture was concentrated in vacuo to remove the tetrahydrofuran. The mixture was acidified by hydrochloric acid (2 M) to pH = 4~5. The mixture was filtered. The filter cake was washed with water and dried in vacuo to give [4-chloro-2-(4-fluoroanilino)-1,3-thiazol-5-yl](oxo)acetic acid (1.82 g, 95% yield) as a yellow solid.

LC-MS (method 4): Rt = 0.793 min; MS (ESIpos): m/z = 300.7 $[M+H]^+$.

**Intermediate 49**

[4-chloro-2-(4-fluoroanilino)-1,3-thiazol-5-yl](oxo)acetyl chloride

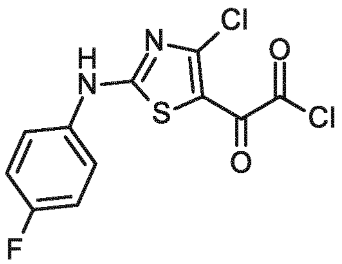

To a mixture of [4-chloro-2-(4-fluoroanilino)-1,3-thiazol-5-yl](oxo)acetic acid (1.82 g, 6.05 mmol, Intermediate 48) in dichloromethane (20 mL) were added oxalyl chloride (0.79 mL, 9.1 mmol) and N,N-dimethylformamide (0.2 mL) at 25 °C. The mixture was stirred at 25 °C for 1 hour. The mixture was concentrated in vacuo to give [4-chloro-2-(4-fluoroanilino)-1,3-thiazol-5-yl](oxo)acetyl chloride (2.00 g, 43% purity, 45% yield) as a yellow solid.

**Intermediate 50**

[4-chloro-2-(4-fluoroanilino)-1,3-thiazol-5-yl](3-phenyl-1,2,4-oxadiazol-5-yl)methanone

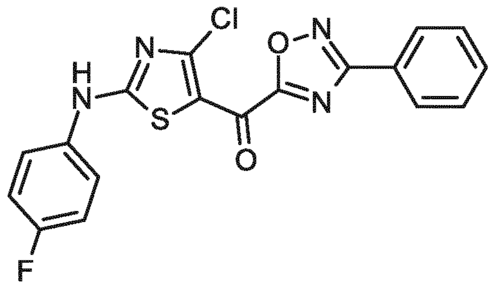

To a mixture of [4-chloro-2-(4-fluoroanilino)-1,3-thiazol-5-yl](oxo)acetyl chloride (950 mg, 43% purity, 1.28 mmol, Intermediate 49) and N-hydroxybenzenecarboximidamide (192 mg, 1.41 mmol) in dichloromethane (5.8 mL) was added trimethylamine (0.36 mL, 2.6 mmol) at 25 °C. The mixture was stirred at 40 °C for 16 hours. The mixture was poured into water and extracted with ethyl acetate. The combined organic phase was washed with water and brine, dried over anhydrous sodium sulfate and filtered. The filtrate was concentrated in vacuo to give a residue. The residue was washed with ethyl acetate (30 mL x 2) to give [4-chloro-2-(4-fluoroanilino)-1,3-thiazol-5-yl](3-phenyl-1,2,4-oxadiazol-5-yl)methanone (300 mg, 58% yield) as a orange solid.

LC-MS (method 4): Rt = 1.041 min; MS (ESIpos): m/z = 400.8 $[M+H]^+$.

$^1$H NMR (400 MHz, DMSO-$d_6$) $\delta$ ppm = 11.72 (s, 1H), 8.07 (d, J = 8.0 Hz, 2H), 7.69-7.63 (m, 5H), 7.32 (d, J = 7.2 Hz, 2H).

**Intermediate 51**

rac-Ethyl N-[4-chloro-5-(3-phenyl-1,2,4-oxadiazole-5-carbonyl)-1,3-thiazol-2-yl]-N-(4-fluorophenyl)- alaninate

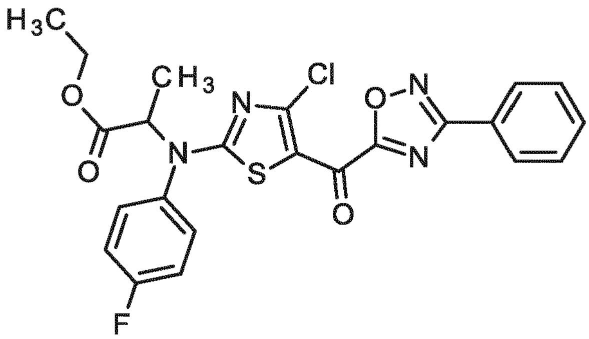

To a mixture of [4-chloro-2-(4-fluoroanilino)-1,3-thiazol-5-yl](3-phenyl-1,2,4-oxadiazol-5-yl)methanone (200 mg, 0.499 mmol, Intermediate 50) in N,N-dimethylformamide (8.0 mL) were added potassium carbonate (138 mg, 0.998 mmol) and rac-ethyl-2-bromopropanoate (181 mg, 0.998 mmol) at 25 °C. The mixture was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product. The crude product was purified by preparative thin layer chromatography (petroleum ether : ethyl acetate = 3: 1) to give rac-ethyl N-{4-chloro-5-[(3-phenyl-1,2,4-oxadiazol-5-yl)carbonyl]-1,3-thiazol-2-yl}-N-(4-fluorophenyl)alaninate (80 mg, 32% yield) as a yellow solid.
LC-MS (method 4): Rt = 0.994 min; MS (ESIpos): m/z = 501.1 $[M+H]^+$.

## Intermediate 52

[4-chloro-2-(4-fluoroanilino)-1,3-thiazol-5-yl](3-methyl-1,2,4-oxadiazol-5-yl)methanone

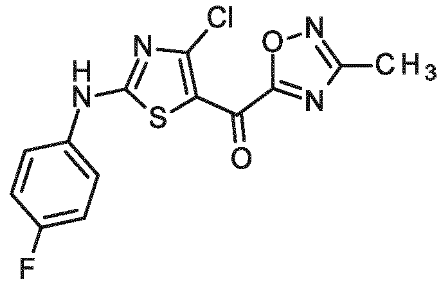

To a mixture of [4-chloro-2-(4-fluoroanilino)-1,3-thiazol-5-yl](oxo)acetic acid (1.00 g, 3.33 mmol, Intermediate 48) and N-hydroxyethanimidamide (493 mg, 6.65 mmol) in N,N-dimethylformamide (10 mL) were added propane phosphonic anhydride (10.6 g, 16.6 mmol, 50% in ethyl acetate) and triethylamine (1.39 mL, 9.98 mmol) at 25 °C. The mixture was stirred at 60 °C for 16 hours. The mixture was poured into water and extracted with ethyl acetate. The combined organic phase was dried over anhydrous sodium sulfate and filtered. The filtrate was concentrated in vacuo to give a crude product as brown oil. The crude product was purified by column chromatography on silica gel (100-200 mesh, petroleum ether: ethyl acetate = 5:1 then 3:1) to give [4-chloro-2-(4-fluoroanilino)-1,3-thiazol-5-yl](3-methyl-1,2,4-oxadiazol-5-yl)methanone (150 mg, 13% yield) as a yellow solid.
LC-MS (method 4): Rt = 0.914 min; MS (ESIpos): m/z = 338.7 $[M+H]^+$.

## Intermediate 53

rac-Ethyl 2-(N-[4-chloro-5-(3-methyl-1,2,4-oxadiazole-5-carbonyl)thiazol-2-yl]-4-fluoro-anilino)propanoate

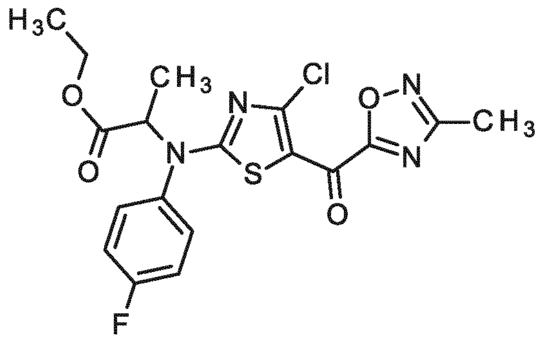

To a mixture of [4-chloro-2-(4-fluoroanilino)-1,3-thiazol-5-yl](3-methyl-1,2,4-oxadiazol-5-yl)methanone (110 mg, 0.325 mmol, Intermediate 52) and rac-ethyl-2-bromopropanoate (118 mg, 0.649 mmol) in N,N-dimethylformamide (1.0 mL) was added potassium carbonate (89.8 mg, 0.649 mmol) at 25 °C. The reaction mixture was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product as a yellow solid. The crude product was purified by preparative thin layer chromatography (petroleum ether : ethyl acetate = 3:1) to give rac-ethyl N-{4-chloro-5-[(3-methyl-1,2,4-oxadiazol-5-yl)carbonyl]-1,3-thiazol-2-yl}-N-(4-fluorophenyl)alaninate (70.0 mg, 49% yield) as a yellow solid.

LC-MS (method 4): Rt = 0.918 min; MS (ESIpos): m/z = 439.1 $[M+H]^+$.

**Intermediate 54**

(3-tert-butyl-1,2,4-oxadiazol-5-yl)[4-chloro-2-(4-fluoroanilino)-1,3-thiazol-5-yl]methanone

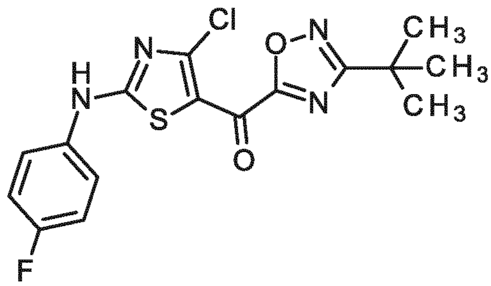

To a mixture of N-hydroxy-2,2-dimethylpropanimidamide (437 mg, 3.76 mmol) and N,N-diisopropylethylamine (2.2 mL, 12.5 mmol) in dichloromethane (9.4 mL) was added [4-chloro-2-(4-fluoroanilino)-1,3-thiazol-5-yl](oxo)acetyl chloride (1 g, 3.13 mmol, Intermediate 49) at 25 °C. The mixture was stirred at 25 °C for 16 hours. The mixture was poured into water and extracted with ethyl acetate. The combined organic phase was washed with brine, dried over anhydrous sodium sulfate and filtered. The filtrate was concentrated in vacuo to give a crude product as brown oil. The crude product was purified by column chromatography on silica gel (100-200 mesh, petroleum ether: ethyl acetate = 10:1 then 5:1) to give (3-tert-butyl-1,2,4-oxadiazol-5-yl)[4-chloro-2-(4-fluoroanilino)-1,3-thiazol-5-yl]methanone (150 mg, 13% yield) as a yellow solid.

LC-MS (method 4): Rt = 0.930 min; MS (ESIpos): m/z = 381.1 $[M+H]^+$.

**Intermediate 55**

rac-Ethyl N-[5-(3-tert-butyl-1,2,4-oxadiazole-5-carbonyl)-4-chloro-1,3-thiazol-2-yl]-N-(4-fluorophenyl)-alaninate

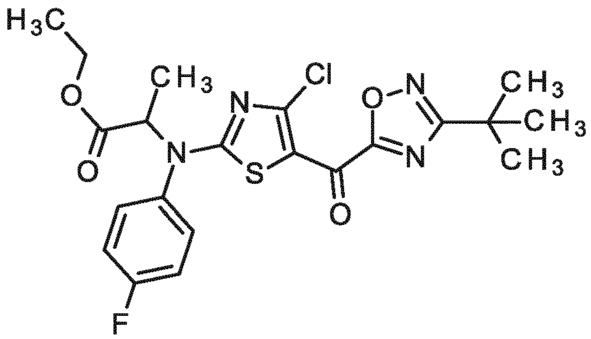

To a mixture of (3-tert-butyl-1,2,4-oxadiazol-5-yl)[4-chloro-2-(4-fluoroanilino)-1,3-thiazol-5-yl]methanone (150 mg, 0.394 mmol, Intermediate 54) in N,N-dimethylformamide (2.0 mL) were added potassium carbonate (109 mg, 0.788 mmol) and rac-ethyl-2-bromopropanoate (143 mg, 0.788 mmol) at 25 °C. The mixture was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product. The crude product was purified by preparative thin layer chromatography (petroleum ether : ethyl acetate = 3: 1) to give rac-ethyl N-{5-[(3-tert-butyl-1,2,4-oxadiazol-5-yl)carbonyl]-4-chloro-1,3-thiazol-2-yl}-N-(4-fluorophenyl)alaninate (90.0 mg, 48% yield) as a yellow solid.
LC-MS (method 4): Rt = 1.088 min; MS (ESIpos): m/z = 481.1 [M+H]$^+$.

**Intermediate 56**

[4-chloro-2-(4-fluoroanilino)-1,3-thiazol-5-yl][3-(pyridin-2-yl)-1,2,4-oxadiazol-5-yl]methanone

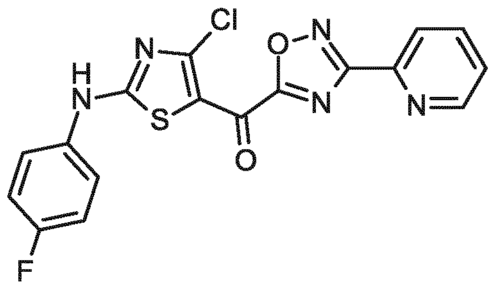

To a mixture of [4-chloro-2-(4-fluoroanilino)-1,3-thiazol-5-yl](oxo)acetic acid (1.00 g, 3.33 mmol, Intermediate 48) and N-hydroxypyridine-2-carboximidamide (547 mg, 3.99 mmol) in N,N-dimethylformamide (10 mL) were added propane phosphonic anhydride (10.6 g, 16.6 mmol, 50% purity) and triethylamine (1.4 mL, 9.98 mmol) at 25 °C. The mixture was stirred at 60 °C for 16 hours. The mixture was poured into water and extracted with ethyl acetate. The combined organic phase was washed with brine, dried over anhydrous sodium sulfate and filtered. The filtrate was concentrated in vacuo to give a residue. The residue was triturated with ethyl acetate (10.0 mL) to give [4-chloro-2-(4-fluoroanilino)-1,3-thiazol-5-yl][3-(pyridin-2-yl)-1,2,4-oxadiazol-5-yl]methanone (400 mg, 50% purity, 15% yield) as an orange solid.
LC-MS (method 4): Rt = 0.940 min; MS (ESIpos): m/z = 401.8 [M+H]$^+$.

**Intermediate 57**

rac-Ethyl N-{4-chloro-5-[3-(pyridin-2-yl)-1,2,4-oxadiazole-5-carbonyl]-1,3-thiazol-2-yl}-N-(4-fluorophenyl)-alaninate

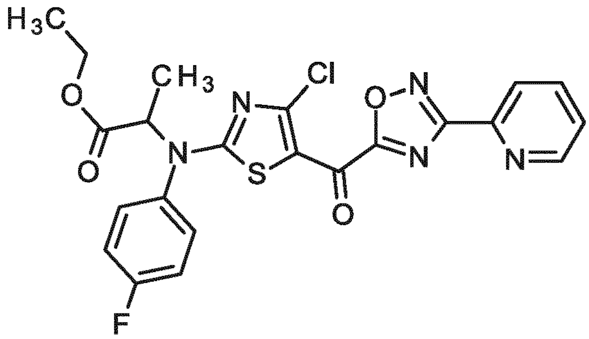

To a mixture of [4-chloro-2-(4-fluoroanilino)-1,3-thiazol-5-yl][3-(pyridin-2-yl)-1,2,4-oxadiazol-5-yl]methanone (350 mg, 50% purity, 0.436 mmol, Intermediate 56) and rac-ethyl-2-bromopropanoate (158 mg, 0.871 mmol) in N,N-dimethylformamide (3.5 mL) was added potassium carbonate (120 mg, 0.871 mmol) at 25 °C. The mixture was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product. The crude product was purified by preparative thin layer chromatography (petroleum ether : ethyl acetate = 2: 1) to give rac-ethyl N-(4-chloro-5-{[3-(pyridin-2-yl)-1,2,4-oxadiazol-5-yl]carbonyl}-1,3-thiazol-2-yl)-N-(4-fluorophenyl)alaninate (105 mg, 48% yield) as a yellow solid.

LC-MS (method 4): Rt = 1.009 min; MS (ESIpos): m/z = 501.9 $[M+H]^+$.

**Intermediate 58**

[4-chloro-2-(4-fluoroanilino)-1,3-thiazol-5-yl][3-(3-fluorophenyl)-1,2,4-oxadiazol-5-yl]methanone

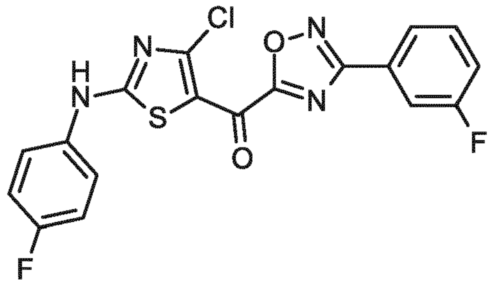

To a mixture of [4-chloro-2-(4-fluoroanilino)-1,3-thiazol-5-yl](oxo)acetyl chloride (1 g, 3.13 mmol, Intermediate 49) and triethylamine (1.75 mL, 12.5 mmol) in N,N-dimethylformamide (5.0 mL) was added 3-fluoro-N-hydroxybenzene-1-carboximidamide (580 mg, 3.76 mmol) at 0 °C. The mixture was stirred at 20 °C for 16 hours. The reaction mixture was quenched with water and extracted with ethyl acetate. The combined organic phase was washed with brine, dried over anhydrous sodium sulfate and filtered. The filtrate was concentrated in vacuo to give a crude product. The crude product was purified by flash column chromatography (petroleum ether: ethyl acetate = 3: 1) to give [4-chloro-2-(4-fluoroanilino)-1,3-thiazol-5-yl][3-(3-fluorophenyl)-1,2,4-oxadiazol-5-yl]methanone (300 mg, 23% yield) as yellow oil.

LC-MS (method 4): Rt = 0.957 min; MS (ESIpos): m/z = 419.1 $[M+H]^+$.

**Intermediate 59**

rac-Ethyl N-{4-chloro-5-[3-(3-fluorophenyl)-1,2,4-oxadiazole-5-carbonyl]-1,3-thiazol-2-yl}-N-(4-fluorophenyl)- alaninate

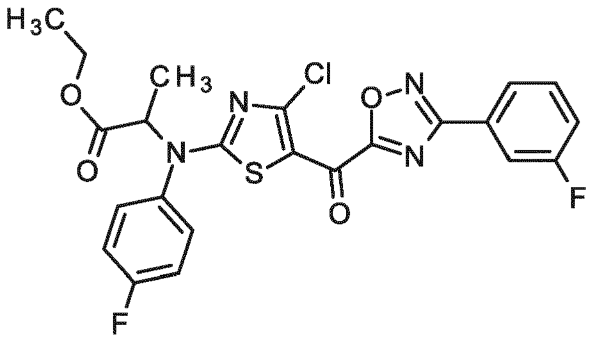

To a mixture of [4-chloro-2-(4-fluoroanilino)-1,3-thiazol-5-yl][3-(3-fluorophenyl)-1,2,4-oxadiazol-5-yl]methanone (150 mg, 0.358 mmol, Intermediate 58) in N,N-dimethylformamide (2.0 mL) were added potassium carbonate (99.0 mg, 0.716 mmol) and rac-ethyl-2-bromopropanoate (130 mg, 0.716 mmol) at 25 °C. The mixture was stirred at 25 °C for 16 hours. The reaction mixture was diluted with water and extracted with ethyl acetate. The combined organic phase was washed with brine, dried over anhydrous sodium sulfate and filtered. The filtrate was concentrated in vacuo to give a residue. The residue was purified preparative thin layer chromatography (petroleum ether: ethyl acetate = 2:1) to give rac-ethyl N-(4-chloro-5-{[3-(3-fluorophenyl)-1,2,4-oxadiazol-5-yl]carbonyl}-1,3-thiazol-2-yl)-N-(4-fluorophenyl)alaninate (80.0 mg, 43% yield) as brown oil.

LC-MS (method 4): Rt = 1.014 min; MS (ESIpos): m/z = 519.2 $[M+H]^+$.

## Intermediate 60

[3-(2,3-dimethylphenyl)-1,2,4-oxadiazol-5-yl][2-(4-fluoroanilino)-4-hydroxy-1,3-thiazol-5-yl]methanone

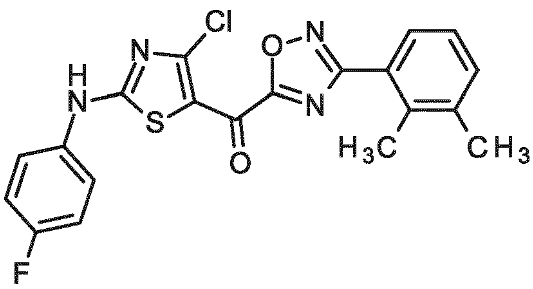

To a mixture of N-hydroxy-2,3-dimethylbenzene-1-carboximidamide (617 mg, 3.76 mmol) and N,N-diisopropylethylamine (2.18 mL, 12.5 mmol) in N,N-dimethylformamide (11 mL) was added [4-chloro-2-(4-fluoroanilino)-1,3-thiazol-5-yl](oxo)acetyl chloride (1.00 g, 3.13 mmol, Intermediate 49) at 25 °C. The mixture was stirred at 25 °C for 16 hours. The mixture was poured into water and extracted with ethyl acetate. The combined organic phase was washed with brine, dried over anhydrous sodium sulfate and filtered. The filtrate was concentrated in vacuo to give a residue. The residue was purified by preparative thin layer chromatography (petroleum ether: ethyl acetate = 2:1) to give [3-(2,3-dimethylphenyl)-1,2,4-oxadiazol-5-yl][2-(4-fluoroanilino)-4-hydroxy-1,3-thiazol-5-yl]methanone (105 mg, 8% yield) as yellow oil.

LC-MS (method 4): Rt = 0.992 min; MS (ESIpos): m/z = 411.1 $[M+H]^+$.

## Intermediate 61

rac-Ethyl N-{5-[3-(2,3-dimethylphenyl)-1,2,4-oxadiazole-5-carbonyl]-4-hydroxy-1,3-thiazol-2-yl}-N-(4-fluorophenyl)-alaninate

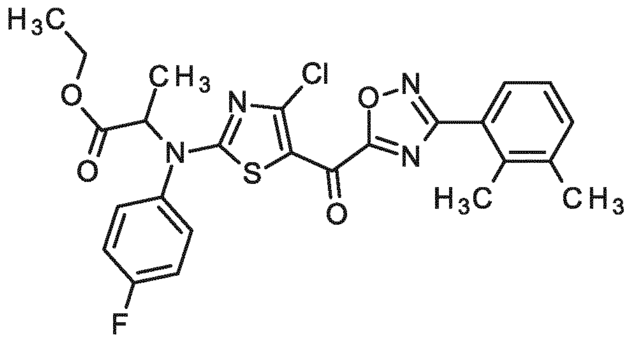

To a mixture of [3-(2,3-dimethylphenyl)-1,2,4-oxadiazol-5-yl][2-(4-fluoroanilino)-4-hydroxy-1,3-thiazol-5-yl] methanone (105 mg, 0.256 mmol, Intermediate 60) in N,N-dimethylformamide (2.0 mL) were added potassium carbonate (70.7 mg, 0.512 mmol) and rac-ethyl-2-bromopropanoate (92.6 mg, 0.512 mmol) at 25 °C. The mixture was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product. The crude product was purified by preparative thin layer chromatography (petroleum ether : ethyl acetate = 2: 1) to give rac-ethyl N-(5-{[3-(2,3-dimethylphenyl)-1,2,4-oxadiazol-5-yl]carbonyl}-4-hydroxy-1,3-thiazol-2-yl)-N-(4-fluorophenyl)alaninate (80.0 mg, 61% yield) as a yellow solid.

LC-MS (method 4): Rt = 1.14 min; MS (ESIpos): m/z = 511.1 $[M+H]^+$.

<u>**Intermediate 62**</u>

[4-chloro-2-(4-fluoroanilino)-1,3-thiazol-5-yl](3-cyclopropyl-1,2,4-oxadiazol-5-yl)methanone

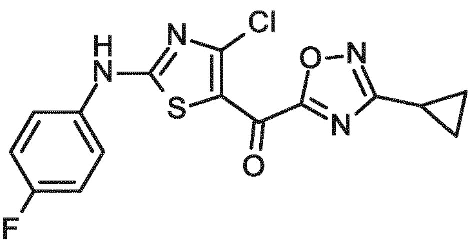

To a mixture of N-hydroxy cyclopropane carboximidamide (399 mg, 3.99 mmol) in dichloromethane (20 mL) were added N,N-diisopropylethylamine (2.89 mL, 16.6 mmol) and [4-chloro-2-(4-fluoroanilino)-1,3-thiazol-5-yl](oxo)acetyl chloride (1.06 g, 3.32 mmol, Intermediate 49) at 25 °C. The mixture was stirred at 35 °C for 16 hours. The reaction mixture was poured into water and extracted with ethyl acetate. The combined organic extracts were washed with brine, dried over anhydrous sodium sulfate and filtered. The filtrate was concentrated under reduced pressure to give a crude product. The crude product was purified by column chromatography on silica gel (petroleum ether: ethyl acetate = 10:1 then 5:1) to give [4-chloro-2-(4-fluoroanilino)-1,3-thiazol-5-yl](3-cyclopropyl-1,2,4-oxadiazol-5-yl)methanone (100 mg, 20% purity, 2% yield) as brown solid.

LC-MS (method 4): Rt = 0.987 min; MS (ESIpos): m/z = 365.0 $[M+H]^+$.

<u>**Intermediate 63**</u>

rac-[4-chloro-2-(4-fluoroanilino)-1,3-thiazol-5-yl](3-cyclopropyl-1,2,4-oxadiazol-5-yl)methanone

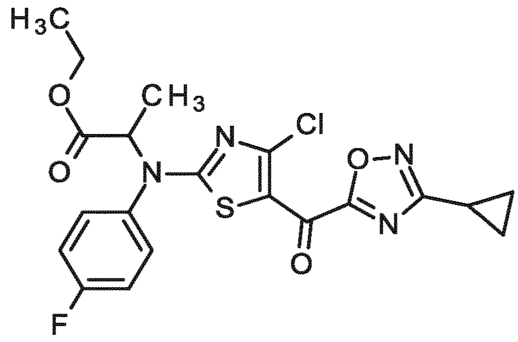

To a mixture of [4-chloro-2-(4-fluoroanilino)-1,3-thiazol-5-yl](3-cyclopropyl-1,2,4-oxadiazol-5-yl)methanone (100 mg, 20% purity, 0.0548 mmol, Intermediate 62) in N,N-dimethylformamide (2.0 mL) were added potassium carbonate (15.2 mg, 0.110 mmol) and rac-ethyl-2-bromopropanoate (19.9 mg, 0.110 mmol) at 25 °C. The mixture was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product. The crude product was purified by preparative thin layer chromatography (petroleum ether: ethyl acetate = 2:1) to give rac-ethyl N-{4-chloro-5-[(3-cyclopropyl-1,2,4-oxadiazol-5-yl)carbonyl]-1,3-thiazol-2-yl}-N-(4-fluorophenyl)alaninate (10.0 mg, 84% purity, 18% yield) as a yellow solid.

LC-MS (method 4): Rt = 0.854 min; MS (ESIpos): m/z = 464.9 $[M+H]^+$.

**Intermediate 64**

N-hydroxy-1-methyl-1H-pyrazole-4-carboximidamide

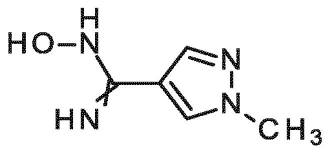

To a solution of 1-methyl-1H-pyrazole-4-carbonitrile (1.00 g, 9.34 mmol) in ethanol (10 mL) were added hydroxylamine hydrochloride (649 mg, 9.34 mmol) and sodium ethanolate (762 mg, 11.2 mmol) at 25 °C. The reaction mixture was stirred at 90 °C for 16 hours. The mixture was filtered. The filtrate was concentrated in vacuo to give N-hydroxy-1-methyl-1H-pyrazole-4-carboximidamide (1.05 g, 80% yield) as light-yellow oil.

LC-MS (method 4): Rt = 0.150 min; MS (ESIpos): m/z = 141.1 $[M+H]^+$.

**Intermediate 65**

[4-chloro-2-(4-fluoroanilino)-1,3-thiazol-5-yl][3-(1-methyl-1H-pyrazol-4-yl)-1,2,4-oxadiazol-5-yl]methanone

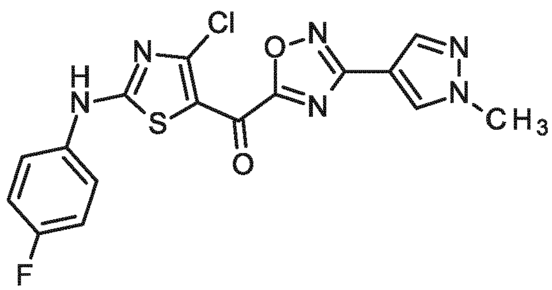

To a mixture of N-hydroxy-1-methyl-1H-pyrazole-4-carboximidamide (527 mg, 3.76 mmol, Intermediate 64) and N,N-diisopropylethylamine (4.4 mL, 13 mmol, 50% purity) in dichloromethane (10 mL) was added [4-chloro-2-(4-fluoroanilino)-1,3-thiazol-5-yl](oxo)acetyl chloride (1.00 g, 3.13 mmol, Intermediate 49) at 25 °C. The mixture was stirred at 25 °C for 16 hours. The mixture was poured into water and extracted with ethyl acetate. The combined organic phase was washed with brine, dried over anhydrous sodium sulfate and filtered. The filtrate was concentrated in vacuo to give a residue. The residue was purified by preparative thin layer chromatography (petroleum ether: ethyl acetate = 2:1) to give [4-chloro-2-(4-fluoroanilino)-1,3-thiazol-5-yl][3-(1-methyl-1H-pyrazol-4-yl)-1,2,4-oxadiazol-5-yl]methanone (100 mg, 8% yield) as yellow oil.

LC-MS (method 4): Rt = 0.934 min; MS (ESIpos): m/z = 404.8 [M+H]+.

**Intermediate 66**

rac-[4-chloro-2-(4-fluoroanilino)-1,3-thiazol-5-yl][3-(1-methyl-1H-pyrazol-4-yl)-1,2,4-oxadiazol-5-yl]methanone

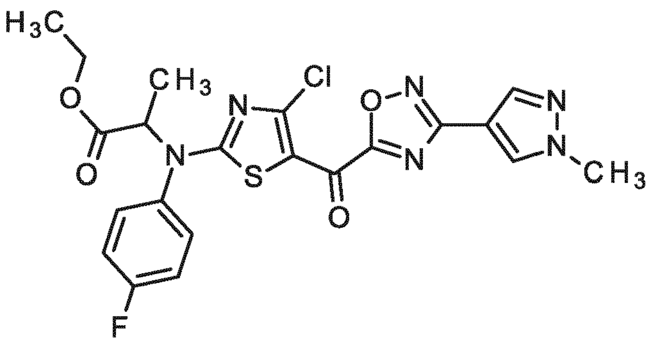

To a mixture of [4-chloro-2-(4-fluoroanilino)-1,3-thiazol-5-yl][3-(1-methyl-1H-pyrazol-4-yl)-1,2,4-oxadiazol-5-yl] methanone (100 mg, 0.247 mmol, Intermediate 65) and rac-ethyl-2-bromopropanoate (89.4 mg, 0.494 mmol) in N,N-dimethylformamide (1.5 mL) was added potassium carbonate (68.3 mg, 0.494 mmol) at 25 °C. The mixture was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product. The crude product was purified by preparative thin layer chromatography (petroleum ether : ethyl acetate = 2:1) to give rac-ethyl N-(4-chloro-5-{[3-(1-methyl-1H-pyrazol-4-yl)-1,2,4-oxadiazol-5-yl]carbonyl}-1,3-thiazol-2-yl)-N-(4-fluorophenyl)alaninate (30.0 mg, 24% yield) as a yellow solid.

LC-MS (method 4): Rt = 0.993 min; MS (ESIpos): m/z = 505.0 [M+H]+.

**Intermediate 67**

N-hydroxy cyclopentane carboximidamide

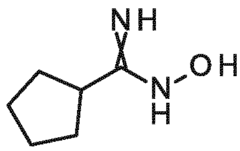

To a solution of cyclopentane carbonitrile (1.00 g, 10.5 mmol) in ethanol (10.0 mL) were added hydroxylamine hydrochloride (803 mg, 11.6 mmol) and sodium ethanolate (858 mg, 12.6 mmol) at 25 °C. The reaction mixture was stirred at 90 °C for 16 hours. The mixture was filtered, and the filtrate was concentrated in vacuo to give N-hydroxy cyclopentane carboximidamide (1.00 g, 74% yield) as light yellow oil.

LC-MS (method 4): Rt = 0.149 min; MS (ESIpos): m/z = 129.0 [M+H]+.

**Intermediate 68**

[4-chloro-2-(4-fluoroanilino)-1,3-thiazol-5-yl](3-cyclopentyl-1,2,4-oxadiazol-5-yl)methanone

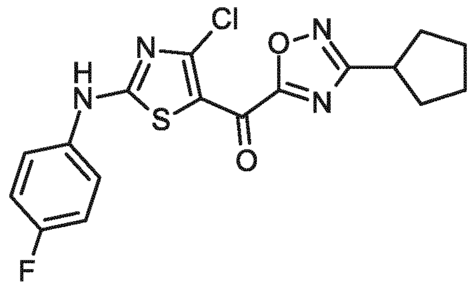

To a mixture of N-hydroxy cyclopentane carboximidamide (145 mg, 1.13 mmol, Intermediate 67) and N,N-diisopropylethylamine (0.650 mL, 3.76 mmol) in dichloromethane (3.0 mL) was added [4-chloro-2-(4-fluoroanilino)-1,3-thiazol-5-yl](oxo)acetyl chloride (300 mg, 0.940 mmol, Intermediate 49) at 25 °C. The mixture was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product. The crude product was purified by preparative thin layer chromatography (petroleum ether: ethyl acetate = 2:1) to give [4-chloro-2-(4-fluoroanilino)-1,3-thiazol-5-yl](3-cyclopentyl-1,2,4-oxadiazol-5-yl)methanone (60.0 mg, 16% yield) as a yellow solid.

LC-MS (method 4): Rt = 1.045 min; MS (ESIpos): m/z = 392.9 $[M+H]^+$.

**Intermediate 69**

rac-Ethyl N-[4-chloro-5-(3-cyclopentyl-1,2,4-oxadiazole-5-carbonyl)-1,3-thiazol-2-yl]-N-(4-fluorophenyl)-alaninate

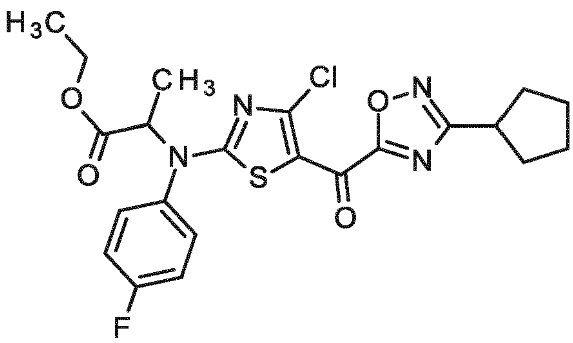

To a mixture of [4-chloro-2-(4-fluoroanilino)-1,3-thiazol-5-yl](3-cyclopentyl-1,2,4-oxadiazol-5-yl)methanone (60 mg, 0.153 mmol, Intermediate 68) and rac-ethyl-2-bromopropanoate (55.3 mg, 0.305 mmol) in N,N-dimethylformamide (1.0 mL) was added potassium carbonate (42.2 mg, 0.305 mmol) at 25 °C. The mixture was stirred at 25 °C for 16 hours. The reaction mixture was poured into water and extracted with ethyl acetate. The combined organic phase was washed with water and brine, dried over anhydrous sodium sulfate and filtered. The filtrate was concentrated in vacuo to give a crude product. The crude product was purified by preparative thin layer chromatography (petroleum ether: ethyl acetate = 2:1) to give rac-ethyl N-{4-chloro-5-[(3-cyclopentyl-1,2,4-oxadiazol-5-yl)carbonyl]-1,3-thiazol-2-yl}-N-(4-fluorophenyl) alaninate (30 mg, 40% yield) as a yellow solid.

LC-MS (method 4): Rt = 1.008 min; MS (ESIpos): m/z = 493.1 $[M+H]^+$.

**Intermediate 70**

1-[4-Amino-2-(4-fluoroanilino)thiazol-5-yl]prop-2-yn-1-one

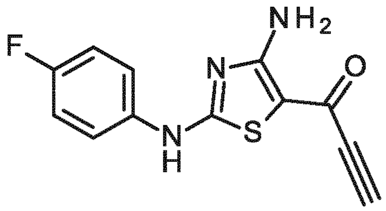

1-Fluoro-4-isothiocyanatobenzene (699 mg, 4.56 mmol) was dissolved in acetonitrile (28 mL) and cyanamide (230 mg, 5.48 mmol) was added at room temperature followed by DBU (0.68 mL, 4.56 mmol). The reaction mixture was stirred for 45 minutes then additional DBU (0.34 mL, 2.28 mmol) followed by 1-bromo-4-trimethylsilyl-but-3-yn-2-one (1.0 g, 4.56 mmol) as a solution in acetonitrile (14 mL) were added at room temperature. The reaction mixture was stirred for 3 h, then it was cooled with an ice bath and was diluted with water and a saturated aqueous solution of ammonium chloride. The precipitate was filtered off, washed with water and triturated with hexane, dried at high vacuum to give 810 mg (3.1 mmol, 68%) of the title compound which was employed in the next step without further purification.

LC-MS (method 2): Rt = 0.70 min; MS(ESIpos) m/z = 262.2 $[M+H]^+$

**Intermediate 71**

rac-2-(N-(4-Amino-5-prop-2-ynoyl-thiazol-2-yl)-4-fluoro-anilino)propanamide

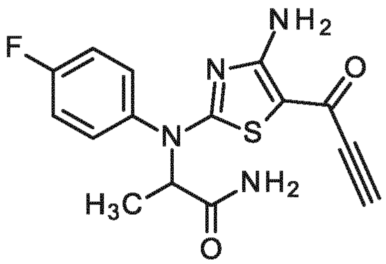

1-[4-Amino-2-(4-fluoroanilino)thiazol-5-yl]prop-2-yn-1-one (570 mg, 2.18 mmol, Intermediate 70) and potassium carbonate (904 mg, 6.55 mmol) were mixed together in DMF (15 mL), then rac-2-bromopropanamide (497 mg, 3.27 mmol) was added portion wise and the reaction was stirred at room temperature overnight. The reaction mixture was diluted with ethyl acetate and water. After separation of the phases, the aqueous phase was extracted again three times with ethyl acetate, the combined organic phases were washed with brine and dried over sodium sulfate. The solvent was removed *in vacuo* and the residue thus obtained was purified by automated flash column chromatography (method Z, methanol + 2 Vol-% aqueous ammonia (33%)) to give 135 mg (0.37 mmol, 17%) of the title compound.

$^1$H NMR (DMSO-$d_6$, 400 MHz) δ 8.0-8.3 (br s, 1H), 7.7-8.0 (br s, 1H), 7.6-7.7 (m, 2H), 7.59 (s, 1H), 7.37 (t, 2H, *J*=8.7 Hz), 7.27 (s, 1H), 5.04 (br d, 1H, *J*=6.3 Hz), 4.37 (s, 1H), 1.16 (d, 3H, *J*=7.4 Hz).

LC-MS (method 2): Rt = 0.85 min; MS(ESIpos) m/z = 333.4 $[M+H]^+$.

**Intermediate 72**

2-Fluoro-N-hydroxy-benzimidoyl chloride

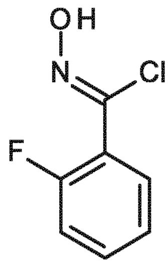

To a solution of 2-fluoro benzaldoxime (200 mg, 1.44 mmol) in DMF (1.4 mL), N-chlorosuccinimide (35 mg, 0.28 mmol) was added to it in one portion to initiate the reaction. After few minutes, the remaining N-chlorosuccinimide (176 mg, 1.3 mmol) was added portion wise over few minutes avoiding that the reaction temperature went above 40°C. The reaction mixture was stirred for three hours at room temperature, thin layer chromatography check (hexane: ethyl acetate 7:3) showed complete consumption of starting material and formation of a less polar spot. The solution was diluted with ethyl acetate and brine, the organic phase was dried over sodium sulfate and evaporated under vacuum to obtain an oily residue that was directly used in the next step without further purification.

**Intermediate 73**

N-Hydroxy-2-methoxy-benzimidoyl chloride

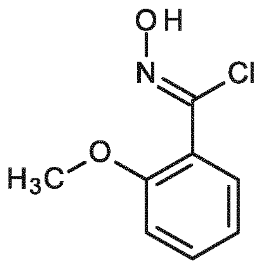

To a solution of o-anisaldehyde oxime (175 mg, 1.16 mmol) in DMF (1.1 mL), N-chlorosuccinimide (28 mg, 0.23 mmol) was added to it in one portion to initiate the reaction. After few minutes, the remaining N-chlorosuccinimide (142 mg, 1.04 mmol) was added portion wise over few minute avoiding that the reaction temperature went above 40°C. The reaction was stirred for three hours at room temperature, thin layer chromatography check (hexane: ethyl acetate 7:3) showed complete consumption of starting material and formation of a less polar spot. The solution was diluted with ethyl acetate and brine, the organic phase was dried over sodium sulfate and evaporated under vacuum to obtain an oily residue that was directly used in the next step without further purification.

**Intermediate 74**

N-Hydroxypyridine-2-carboximidoyl chloride

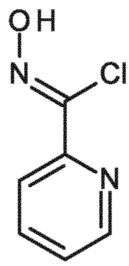

To a solution of 2-pyridinecarbaldehyde oxime (182 mg, 1.49 mmol) in DMF (1.5 mL), N-chlorosuccinimide (38 mg, 0.28 mmol) was added to it in one portion to initiate the reaction. After few minutes, the remaining N-chlorosuccinimide

(170 mg, 1.29 mmol) was added portion wise over few minute avoiding that the reaction temperature went above 40°C. The reaction was stirred for three hours at room temperature. Thin layer chromatography (hexane:ethyl acetate 1:1) showed formation of a less polar spot but still mainly starting material present therefore the reaction was heated to 38°C overnight. The solution was diluted with ethyl acetate and brine, the organic phase was dried over sodium sulfate and evaporated under vacuum to obtain an oily residue that was directly used in the next step without further purification.

**Intermediate 75**

N-Hydroxy-4-(trifluoromethoxy)benzimidoyl chloride

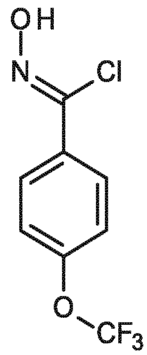

To a solution of p-trifluoromethoxybenzaldoxime (305 mg, 1.49 mmol) in DMF (1.5 mL), N-chlorosuccinimide (38 mg, 0.28 mmol) was added to it in one portion to initiate the reaction. After few minutes, the remaining N-chlorosuccinimide (170 mg, 1.29 mmol) was added portion wise over few minute avoiding that the reaction temperature went above 40°C. The reaction was stirred at 36°C overnight. The solution was diluted with ethyl acetate and brine, the organic phase was dried over sodium sulfate and evaporated under vacuum to obtain an oily residue that was directly used in the next step without further purification.

**Intermediate 76**

N-Hydroxy-6-methoxy-pyridine-3-carboximidoyl chloride

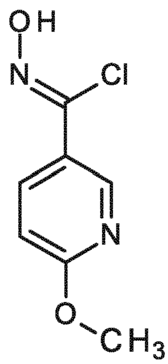

To a solution of 6-methoxypyridine-3-carbaldehyde oxime (257 mg, 1.69 mmol) in DMF (1.7 mL), N-chlorosuccinimide (43 mg, 0.32 mmol) was added to it in one portion to initiate the reaction. After few minutes, the remaining N-chlorosuccinimide (194 mg, 1.45 mmol) was added portion wise over few minute avoiding that the reaction temperature went above 40°C. The reaction was stirred at 36°C overnight. In the morning thin layer chromatography check (hexane: ethyl acetate 1:1) showed still presence of starting material, therefore further N-chlorosuccinimide (237 mg, 1.77 mmol) was added portion wise and the reaction was stirred for an additional day at 36 °C. The solution was then diluted with ethyl acetate and brine, the organic phase was dried over sodium sulfate and evaporated under vacuum to obtain an oily residue that was directly used in the next step without further purification.

**Intermediate 77**

[4-Amino-2-(4-fluoroanilino)thiazol-5-yl]-[3-(hydroxymethyl)isoxazol-5-yl]methanone

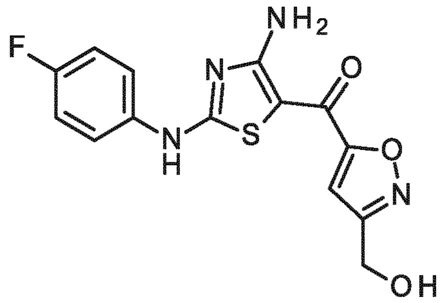

Ethyl 5-[4-amino-2-(4-fluoroanilino)thiazole-5-carbonyl]isoxazole-3-carboxylate (2.6 g, 6.9 mmol, Intermediate 12) was suspended in ethanol (44 mL) and THF (26 mL) under an inert atmosphere. The suspension was cooled with an ice bath and sodium borohydride (1.05 g, 27.6 mmol) was added portion wise. The reaction mixture was stirred for 1 h at room temperature. The reaction mixture was then quenched by addition of a saturated aqueous solution of ammonium chloride and water causing the formation of a yellow precipitate that was collected by vacuum filtration. The solid was washed with water, triturated with hexane and left at high vacuum to give 2.15 g (6.43 mmol, 93%) of the title compound that was used in the next step without further purification.

LC-MS (method 1): Rt = 0.88 min; MS(ESIpos) m/z = 335.1 $[M+H]^+$.

**Intermediate 78**

rac-2-(N-[4-amino-5-(3-formylisoxazole-5-carbonyl)thiazol-2-yl]-4-fluoro-anilino)propanamide

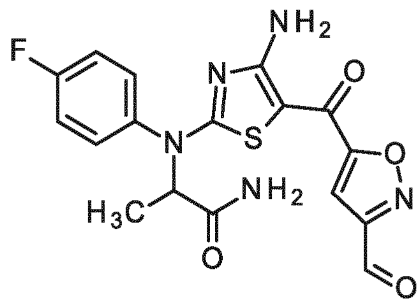

rac-2-(N-[4-amino-5-[3-(hydroxymethyl)isoxazole-5-carbonyl]thiazol-2-yl]-4-fluoro-anilino)propanamide (105 mg, 0.259 mmol, Example 65) was dissolved in DMSO (5 mL) and 2-iodoxybenzoic acid 45wt.% (322 mg, 0.517 mmol) was added portion wise to it. The reaction was stirred overnight and then was diluted with ethyl acetate, a saturated solution of sodium carbonate followed by a solution of sodium thiosulfate. The mixture was left stirring for few minutes, the organic phase was then washed with brine, dried over sodium sulfate and evaporated *in vacuo.* The residue thus obtained was used in the next step without further purification.

LC-MS (method 2): Rt = 0.80 min; MS(ESIpos) m/z = 404.4 $[M+H]^+$.

**Intermediate 79**

rac-[5-[4-amino-2-(N-(2-amino-1-methyl-2-oxo-ethyl)-4-fluoro-anilino)thiazole-5-carbonyl]isoxazol-3-yl]methyl methanesulfonate

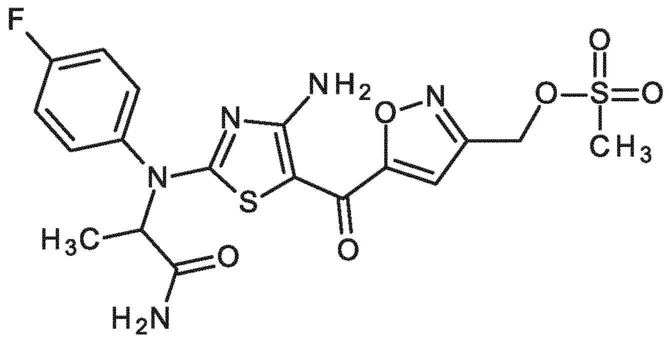

rac-2-(N-[4-amino-5-[3-(hydroxymethyl)isoxazole-5-carbonyl]thiazol-2-yl]-4-fluoro-anilino)propanamide (250 mg, 0.62 mmol, Example 65) was dissolved in pyridine/THF 1:1 (8 mL). The reaction mixture was cooled with an ice bath and methanesulfonyl chloride (57 μL, 0.74 mmol) was added to it. The reaction mixture was stirred at room temperature for 4 h and then it was diluted with ethyl acetate and HCl (0.01 N aqueous solution). The organic phase was washed with saturated aqueous sodium carbonate solution and brine, dried over sodium sulfate and reduced *in vacuo.* The residue thus obtained was used in the next step without further purification.

LC-MS (method 2): Rt = 0.95 min; MS(ESIpos) m/z = 484.5 $[M+H]^+$.

**Intermediate 80**

N-methoxy-N-methyl-3-phenyl-isothiazole-5-carboxamide

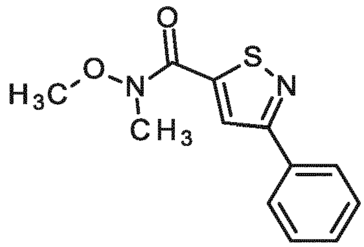

3-phenyl-1,2-thiazole-5-carboxylic acid (250 mg, 1.22 mmol), N,O-dimethylhydroxylamine hydrochloride (238 mg, 2.44 mmol) were mixed in DMF (7.4 mL), then diisopropyl ethyl amine (0.85 mL, 4.87 mmol) and HATU (926 mg, 2.44 mmol) were added and the reaction mixture was stirred for one hour. The reaction mixture was poured in water and extracted three times with ethyl acetate. The combined organic phases were washed with brine,dried over sodium sulfate, filtrated and evaporated to dryness. The residue thus obtained was purified by automated flash column chromatography (method X) to give 262 mg (1.06 mmol, 87%) of the title compound.

$^1H$ NMR (400 MHz, DMSO-$d_6$) δ ppm = 8.46 (s, 1 H) 8.04 - 8.13 (m, 2 H) 7.41 - 7.55 (m, 3 H) 3.87 (s, 3 H) 3.36 (s, 3 H).

LC-MS (method 1): Rt = 1.18 min; MS(ESIpos) m/z = 249.5 $[M+H]^+$.

**Intermediate 81**

2-bromo-1-(3-phenylisothiazol-5-yl)ethanone

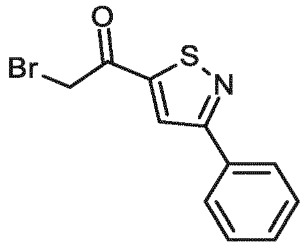

N-methoxy-N-methyl-3-phenyl-isothiazole-5-carboxamide (100 mg, 0.40 mmol, Intermediate 80) was dissolved in THF (2 mL) under an inert atmosphere, dibromomethane (0.057 mL, 0.81 mmol) was added to it and the solution was cooled to -78°C with an acetone/dry ice bath. Methyl lithium solution 1.6 M in hexane (0.5 mL, 0.81 mmol) was then added to the cooled solution. After 15 min the reaction mixture was quenched by the addition of acetic acid, diluted with ethyl acetate and saturated aqueous ammonium chloride solution. The organic phase was washed with a saturated aqueous solution of sodium hydrogen carbonate andbrine, dried over sodium sulfate, filtrated and reduced under vacuum. The residue thus obtained was directly used in the next step without further purification.
$^1$H NMR (Chloroform-d, 400 MHz) δ ppm = 8.09 (s, 1H), 7.9-8.0 (m, 2H), 7.4-7.5 (m, 3H), 4.39 (s, 2H).
LC-MS (method 1): Rt = 1.31 min; MS(ESIpos) m/z = 282.0 [M+H]$^+$.

**Intermediate 82**

[4-amino-2-(4-fluoroanilino)thiazol-5-yl]-(3-phenylisothiazol-5-yl)methanone

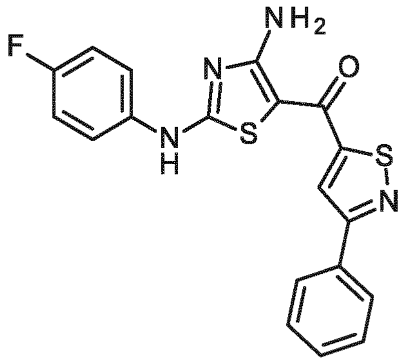

1-fluoro-4-isothiocyanatobenzene (57 mg, 0.37 mmol) was dissolved in acetonitrile (4.3 mL) and cyanamide (19 mg, 0.45 mmol) was added at room temperature followed by DBU (0.055 mL, 0.37 mmol). The reaction mixture was stirred for 45 minutes then additional DBU (0.028 mL, 0.19 mmol) followed by 2-bromo-1-(3-phenylisothiazol-5-yl)ethanone (105 mg, 0.37 mmol, Intermediate 81) as a solution in acetonitrile (1.9 mL) were added at room temperature. The reaction mixture was stirred for 2 h, then it was cooled with an ice bath and diluted with water and saturated aqueous ammonium chloride solution causing precipitation of the desired product that was collected by vacuum filtration. The solid was washed with water and triturated with hexane, dried at high vacuum to give 110 mg (0.28 mmol, 75%) of the title compound which was employed in the next step without further purification.
LC-MS (method 2): Rt = 1.02 min; MS(ESIpos) m/z = 397.2 [M+H]$^+$.

**Intermediate 83**

1-(3-allyloxyisoxazol-5-yl)-2-bromo-ethanone

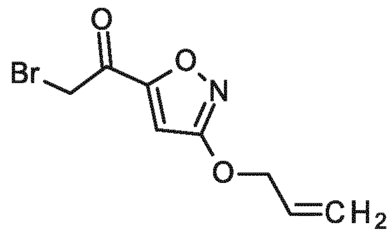

Methyl 3-allyloxyisoxazole-5-carboxylate (synthesized as reported in the Eur. J. Org. Chem. 1998, 473-479) (535 mg, 2.92 mmol) was dissolved in THF (15 mL) under an inert atmosphere. Dibromomethane (0.37 mL, 5.26 mmol) was added and the solution was cooled to -78°C with an acetone/dry ice bath. Methyl lithium solution (1.6 M in hexane, 3.29 mL, 5.26 mmol) was then added to the cooled solution and the reaction was stirred for 15 minutes. The reaction mixture was quenched by addition of acetic acid, diluted with ethyl acetate, water and brine. The aqueous phase was extracted three times with ethyl acetate and the combined organic phases were washed with a saturated solution of sodium hydrogen carbonate, brine, dried over sodium sulfate and reduced under vacuum. The residue thus obtained was directly used in the next step without further purification.

$^{1}$H NMR (400 MHz, CHLOROFORM-d) $\delta$ ppm = 6.65 (s, 1 H) 5.99 - 6.12 (m, 1 H) 5.41 - 5.50 (m, 1 H) 5.36 (dd, *J*=10.39, 1.27 Hz, 1 H) 4.81 (dt, *J*=5.77, 1.17 Hz, 2 H) 4.37 (s, 2 H).

LC-MS (method 1): Rt = 1.10 min; MS(ESIpos) m/z = 246.0 $[M+H]^+$.

**Intermediate 84**

(3-allyloxyisoxazol-5-yl)-[4-amino-2-(4-fluoroanilino)thiazol-5-yl]methanone

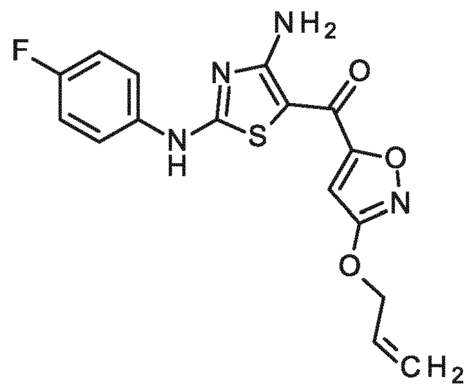

1-fluoro-4-isothiocyanatobenzene (399 mg, 2.61 mmol) was dissolved in acetonitrile (18 mL) and cyanamide (132 mg, 3.13 mmol) was added at room temperature followed by DBU (0.40 mL, 2.61 mmol). The reaction was stirred for 45 minutes, then additional DBU (0.18 mL, 1.29 mmol) followed by 1-(3-allyloxyisoxazol-5-yl)-2-bromo-ethanone (Intermediate 83, 642 mg, 2.61 mmol) as a solution in acetonitrile (9 mL) were added at room temperature. The reaction mixture was stirred for three hours, then cooled with an ice bath and diluted with water and saturated aqueous ammonium chloride solution causing precipitation of the desired product that was collected by vacuum filtration. The solid was washed with water and triturated with hexane, dried at high vacuum to give 650 mg (1.80 mmol, 65%) of the title compound as red solid that was employed in the next step without further purification.

LC-MS (method 2): Rt = 0.89 min; MS(ESIneg) m/z = 359.3 $[M-H]^-$.

**Intermediate 85**

rac-2-(N-[5-(3-allyloxyisoxazole-5-carbonyl)-4-amino-thiazol-2-yl]-4-fluoro-anilino)propanamide

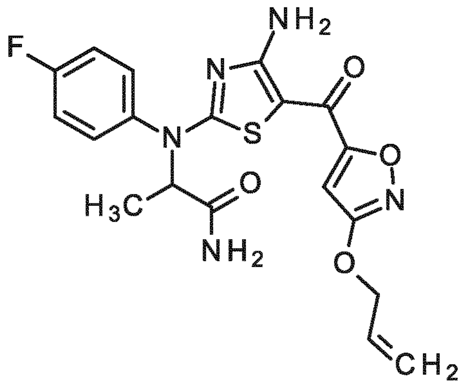

(3-allyloxyisoxazol-5-yl)-[4-amino-2-(4-fluoroanilino)thiazol-5-yl]methanone (Intermediate 84, 650 mg, 1.81 mmol) and potassium carbonate (748 mg, 5.42 mmol) were mixed together in DMF (12 mL), then rac-2-bromopropanamide (329 mg, 2.17 mmol) was added portion wise and the reaction mixture was stirred at room temperature overnight. The reaction mixture was diluted with ethyl acetate and brine and dried over sodium sulfate. The solvent was removed under vacuum distillation. The residue thus obtained was purified by automated flash column chromatography (method Z, basic, 2 vol% aqueous ammonia in MeOH) to give 250 mg (0.58 mmol, 32%) of the title compound.

$^1$H NMR (400 MHz, DMSO-$d_6$) δ ppm = 8.67 (br s, 1 H) 8.46 (br s, 1 H) 7.69 (dd, *J*=8.62, 5.07 Hz, 2 H) 7.62 (br s, 1 H) 7.39 (t, *J*=8.87 Hz, 2 H) 7.29 (s, 1 H) 6.67 (s, 1 H) 5.96 - 6.09 (m, 1 H) 5.40 (dq, *J*=17.24, 1.52 Hz, 1 H) 5.29 (dq, *J*=10.52, 1.31 Hz, 1 H) 5.10 (br s, *J*=0.76 Hz, 1 H) 4.71 (dt, *J*=5.58, 1.27 Hz, 2 H) 3.17 (d, *J*=5.07 Hz, 1 H) 1.19 (d, *J*=7.35 Hz, 3 H).

LC-MS (method 2): Rt = 1.11 min; MS(ESIpos) m/z = 432.3 [M+H]$^+$.

## EXPERIMENTAL SECTION - PREPARATION OF EXAMPLE COMPOUNDS

### Example 1

rac-2-(N-[4-amino-5-[3-(4-chlorophenyl)-1,2,4-oxadiazole-5-carbonyl]thiazol-2-yl]-4-fluoro-anilino)propanamide

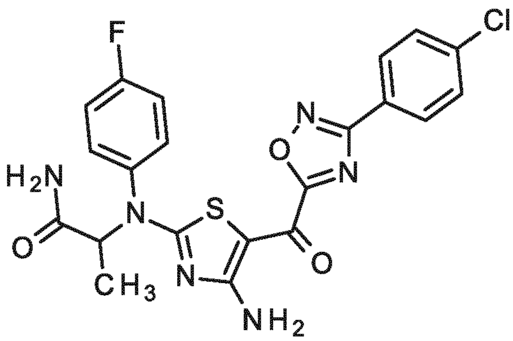

[4-amino-2-(4-fluoroanilino)-1,3-thiazol-5-yl][3-(4-chlorophenyl)-1,2,4-oxadiazol-5-yl]methanone (26 mg, 0.06 mmol; Intermediate 1) was dissolved in N,N-dimethylformamide (0.5 mL) followed by the addition of potassium carbonate (43 mg, 0.31 mmol) and rac-2-bromopropanamide (14 mg, 0.094 mmol). The reaction mixture was stirred at room temperature overnight followed by filtration and purification by RP-HPLC (method D, basic) to give 5 mg (16 % yield) of the title compound.

$^1$H NMR (400 MHz, DMSO-$d_6$) δ ppm = 1.20 (d, *J*=7.35 Hz, 3 H), 5.14 (q, *J*=7.35 Hz, 1 H), 7.30 - 7.34 (m, 1 H), 7.39 - 7.41 (m, 2 H), 7.62 - 7.67 (m, 3 H), 7.69 - 7.72 (m, 2 H), 7.85 -7.89 (m, 2 H), 8.78 - 8.02 (m, 2 H)

LC-MS (method 2) Rt = 1.28 min MS (ESIpos): m/z = 487.4 [M+H]+

The following examples were prepared from the starting materials stated in Table 3, below, using the procedure as for Example 1.

The crude product was either purified by RP-HPLC (methods A-D depending on polarity) or by preparative flash

chromatography (methods X, Y or Z depending on polarity) after precipitation, extraction or filtration of the reaction mixture if necessary.

Enantiomers were separated from their racemate by chiral HPLC using the column and solvent conditions stated.

**Table 3: Example 2-8**

| Example number | Chemical structure Compound name | Starting materials | Analytics/purification/yield |
|---|---|---|---|
| **2** | 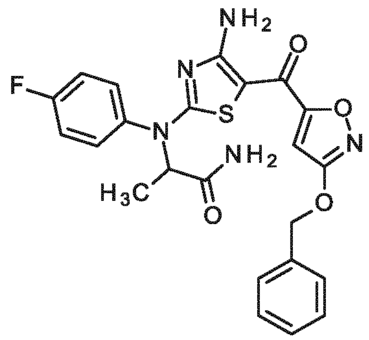<br>rac-2-(N-[4-amino-5-(3-benzyloxyisoxazole-5-carbonyl)thiazol-2-yl]-4-fluoro-anilino)propanamide | Intermediate 2, rac-2-bromopropanam ide | $^1$H NMR (400 MHz, DMSO-$d_6$) $\delta$ ppm = 1.19 (d, *J*=7.35 Hz, 3 H), 5.11 (m, 1 H), 5.24 (s, 2 H), 6.69 (s, 1 H), 7.29 (br s, 1 H), 7.40 (m, 7 H), 7.62 (br s, 1 H), 7.69 (dd, *J*=8.62, 5.07 Hz, 2 H), 8.46 (br s, 1 H), 8.67 (br s, 1 H)<br>RP-HPLC (method C basic)<br>LC-MS (method 2) Rt = 1.20 min MS (ESIpos): m/z = 482.1 [M+H]$^+$ 42 % yield |
| **3** | 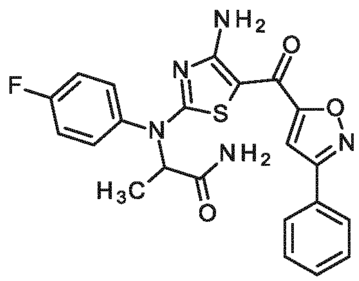<br>rac-2-(N-[4-amino-5-(3-phenylisoxazole-5-carbonyl)thiazol-2-yl]-4-fluoro-anilino)propanamide | Intermediate 3, rac-2-bromopropanam ide | $^1$H NMR (400 MHz, DMSO-$d_6$) $\delta$ ppm = 1.20 (d, *J*=7.35 Hz, 3 H), 5.14 (m, 1 H), 7.30 (s, 1 H), 7.41 (dd, *J*=8.87 Hz, 2 H), 7.52 (m, 4 H), 7.63 (br s, 1 H), 7.72 (m, 2 H), 7.91 (m, 2 H), 8.48 (m, 1 H), 8.74 (m, 1 H)<br>LC-MS (method 2) Rt = 1.20 min MS (ESIpos): m/z = 452.3 [M+H]$^+$ 52 % yield after trituration in DCM |
| **3.1** and **3.2** | (R)- 2-(N-[4-amino-5-(3-phenylisoxazole-5-carbonyl)thiazol-2-yl]-4-fluoro-anilino)propanamide and (S)-2-(N-[4-amino-5-(3-phenylisoxazole-5-carbonyl)thiazol-2-yl]-4-fluoro-anilino)propanamide | | |
| **3.1** | 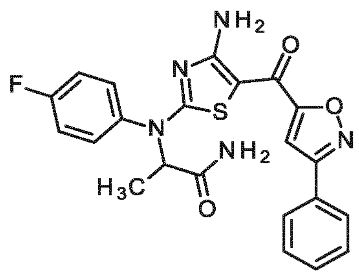<br>2-(N-[4-amino-5-(3-phenylisoxazole-5-carbonyl)thiazol-2-yl]-4-fluoro-anilino)propanamide (enantiomer 1) | Example 3 | $^1$H NMR (400 MHz, DMSO-$d_6$) $\delta$ ppm = 1.20 (d, *J*=7.35 Hz, 3 H), 5.14 (m, 1 H), 7.30 (s, 1 H), 7.41 (dd, *J*=8.87 Hz, 2 H), 7.52 (m, 4 H), 7.63 (br s, 1 H), 7.72 (m, 2 H), 7.91 (m, 2 H), 8.48 (m, 1 H), 8.74 (m, 1 H)<br>LC-MS (method 2) Rt = 1.20 min MS (ESIpos): m/z = 452.3 [M+H]$^+$ |
| Chiral HPLC Example 3.1<br>HPLC separation of rac-2-(N-[4-amino-5-(3-phenylisoxazole-5-carbonyl)thiazol-2-yl]-4-fluoro-anilino)propanamide (370 mg, 0.82 mmol, Example 3 on a chiral column gave 160 mg (43 % yield) of 2-(N-[4-amino-5-(3-phenylisoxazole-5-carbonyl)thiazol-2-yl]-4-fluoro-anilino)propanamide, enantiomer 1.<br>Preparative chiral HPLC | | | |

(continued)

| Example number | Chemical structure Compound name | Starting materials | Analytics/purification/yield |
|---|---|---|---|
| Instrument: PrepCon Labomatic HPLC-3; Column: YMC Amylose SA 10μ, 250x50; eluent A: methyl tert-butyl ether + 0.1 vol % diethylamine; eluent B: acetonitrile + 0.1 vol % diethylamine; isocratic: 80%A+20%B; flow: 100 mL/min; temperature: 25°C; UV: 254 nm<br>Analytical chiral HPLC: Rt = 1.71 min<br>Instrument: Waters Alliance 2695; Column: YMC Amylose SA 3μ, 100x4.6; eluent A: methyl tert-butyl ether + 0.1 vol % diethylamine; eluent B: acetonitrile; isocratic: 80%A+20%B; flow: 1.4 mL/min; temperature: 25°C; UV: 254 nm | | | |
| **3.1** | 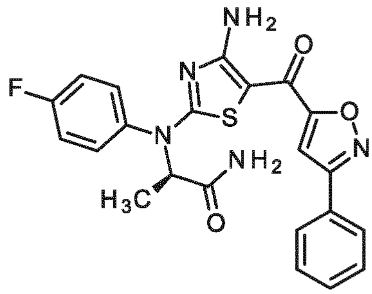 | Example 3.1 was determined to be (R)-2-(N-[4-amino-5-(3-phenylisoxazole-5-carbonyl)thiazol-2-yl]-4-fluoro-anilino)propanamide, by means of X-ray crystal structure analysis. | |
| **3.2** | 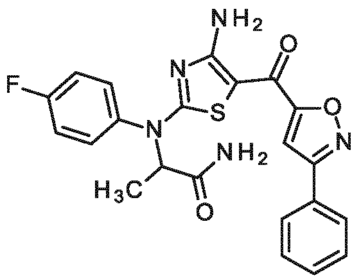<br>2-(N-[4-amino-5-(3-phenylisoxazole-5-carbonyl)thiazol-2-yl]-4-fluoro-anilino)propanamide (enantiomer 2) | Example 3 | $^1$H NMR (400 MHz, DMSO-$d_6$) δ ppm = 1.20 (d, *J*=7.35 Hz, 3 H), 5.14 (m, 1 H), 7.30 (s, 1 H), 7.41 (dd, *J*=8.87 Hz, 2 H), 7.52 (m, 4 H), 7.63 (br s, 1 H), 7.72 (m, 2 H), 7.91 (m, 2 H), 8.48 (m, 1 H), 8.74 (m, 1 H)<br>LC-MS (method 2) Rt = 1.20 min MS (ESIpos): m/z = 452.3 $[M+H]^+$ 52 % yield after trituration in DCM |
| Chiral HPLC Example 3.2<br>HPLC separation of rac-2-(N-[4-amino-5-(3-phenylisoxazole-5-carbonyl)thiazol-2-yl]-4-fluoro-anilino)propanamide (370 mg, 0.82 mmol, Example 3 on a chiral column gave 90 mg (24 % yield) of 2-(N-[4-amino-5-(3-phenylisoxazole-5-carbonyl)thiazol-2-yl]-4-fluoro-anilino)propanamide, enantiomer 2.<br>Preparative chiral HPLC<br>Instrument: PrepCon Labomatic HPLC-3; Column: YMC Amylose SA 10μ, 250x50; eluent A: methyl tert-butyl ether + 0.1 vol % diethylamine; eluent B: acetonitrile + 0.1 vol % diethylamine; isocratic: 80%A+20%B; flow: 100 mL/min; temperature: 25°C; UV: 254 nm<br>Analytical chiral HPLC: Rt = 3.01 min<br>Instrument: Waters Alliance 2695; Column: YMC Amylose SA 3μ, 100x4.6; eluent A: methyl tert-butyl ether + 0.1 vol % diethylamine; eluent B: acetonitrile; isocratic: 80%A+20%B; flow: 1.4 mL/min; temperature: 25°C; UV: 254 nm | | | |

(continued)

| Example number | Chemical structure Compound name | Starting materials | Analytics/purification/yield |
|---|---|---|---|
| **4** | 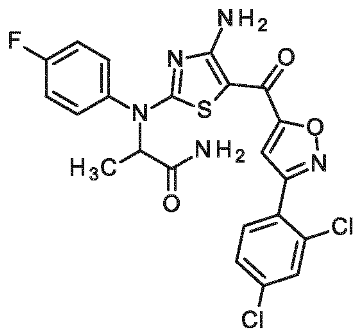 rac-2-(N-[4-amino-5-[3-(2,4-dichlorophenyl) isoxazole-5-carbonyl]thiazol-2-yl]-4-fluoro-anilino)propanamide | Intermediate 4, rac-2-bromopropanam ide | $^{1}H$ NMR (400 MHz, DMSO-$d_6$) $\delta$ ppm = 1.20 (d, *J*=7.35 Hz, 3 H), 5.13 (m, 1 H), 7.30 (s, 1 H), 7.31 (br s, 1 H), 7.40 (dd, *J*=8.74 Hz, 2 H), 7.59 (dd, *J*=8.36, 2.03 Hz, 1 H), 7.64 (br s, 1 H), 7.74 (m, 3 H), 7.86 (m, 1 H), 8.51 (m, 1 H), 8.73 (m, 1 H) LC-MS (method 2) Rt = 1.36 min MS (ESIpos): m/z = 520.2 $[M+H]^+$ 57 % yield after trituration in MTBE |
| **5** | 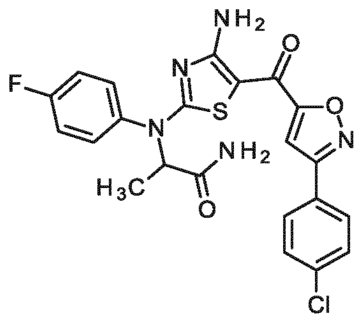 rac-2-(N-[4-amino-5-[3-(4-chlorophenyl)isoxazole-5-carbonyl]thiazol-2-yl]-4-fluoro-anilino)propanamide | Intermediate 5, rac-2-bromopropanam ide | $^{1}H$ NMR (400 MHz, DMSO-$d_6$) $\delta$ ppm = 1.20 (d, *J*=7.35 Hz, 3 H), 5.13 (q, *J*=7.35 Hz, 1 H), 7.30 (br s, 1 H), 7.41 (dd, *J*=8.74 Hz, 2 H), 7.58 (m, 3 H), 7.63 (br s, 1 H), 7.71 (dd, *J*=8.74, 4.94 Hz, 2 H), 7.94 (m, 2 H), 8.50 (m, 1 H), 8.74 (m, 1 H) LC-MS (method 2) Rt = 1.30 min MS (ESIpos): m/z = 486.3 $[M+H]^+$ 51 % yield after trituration in DCM |
| **6** | 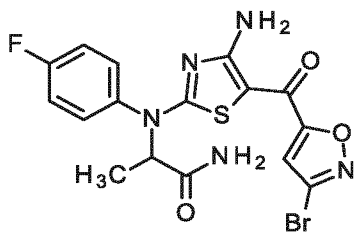 rac-2-(N-[4-amino-5-(3-bromoisoxazole-5-carbonyl)thiazol-2-yl]-4-fluoro-anilino)propanamide | Intermediate 6, rac-2-bromopropanam ide | $^{1}H$ NMR (400 MHz, DMSO-$d_6$) $\delta$ ppm = 1.18 (d, *J*=7.35 Hz, 3 H), 5.10 (m, 1 H), 7.21 (s, 1 H), 7.30 (s, 1 H), 7.39 (t, *J*=8.74 Hz, 2 H), 7.63 (br s, 1 H), 7.70 (dd, *J*=8.74, 4.94 Hz, 2 H), 8.57 (br s, 1 H), 8.70 (br s, 1 H) RP-HPLC (method C basic) LC-MS (method 2) Rt = 1.16 min MS (ESIpos): m/z = 454.2 $[M+H]^+$ 39 % yield |
| **6.1** and **6.2** | (R)-2-(N-[4-amino-5-(3-bromoisoxazole-5-carbonyl)thiazol-2-yl]-4-fluoro-anilino)propanamide and (S)-2-(N-[4-amino-5-(3-bromoisoxazole-5-carbonyl)thiazol-2-yl]-4-fluoro-anilino)propanamide | | |

(continued)

| Example number | Chemical structure Compound name | Starting materials | Analytics/purification/yield |
|---|---|---|---|
| **6.1** | 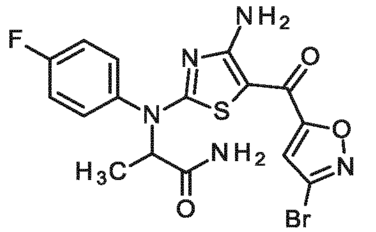 2-(N-[4-amino-5-(3-bromoisoxazole-5-carbonyl)thiazol-2-yl]-4-fluoro-anilino)propanamide (enantiomer 1) | Example 6 | $^1$H NMR (400 MHz, DMSO-$d_6$) δ ppm = 1.18 (d, $J$=7.35 Hz, 3 H), 5.10 (m, 1 H), 7.21 (s, 1 H), 7.30 (s, 1 H), 7.39 (t, $J$=8.74 Hz, 2 H), 7.63 (br s, 1 H), 7.70 (dd, $J$=8.74, 4.94 Hz, 2 H), 8.57 (br s, 1 H), 8.70 (br s, 1 H) LC-MS (method 2) Rt = 1.16 min MS (ESIpos): m/z = 454.2 [M+H]$^+$ 49 % yield |
| Chiral HPLC Example 6.1<br>HPLC separation of rac-2-(N-[4-amino-5-(3-bromoisoxazole-5-carbonyl)thiazol-2-yl]-4-fluoro-anilino)propanamide (150 mg, 0.33 mmol, Example 6) on a chiral column gave 76 mg (49 % yield) of 2-(N-[4-amino-5-(3-bromoisoxazole-5-carbonyl)thiazol-2-yl]-4-fluoro-anilino)propanamide, enantiomer 1.<br><u>Preparative chiral HPLC</u><br>Instrument: PrepCon Labomatic HPLC-1; Column: YMC Cellulose SC 5μ, 250x30; eluent A: methyl tert-butyl ether + 0.1 vol % diethylamine; eluent B: ethanol; isocratic: 95%A+5%B; flow: 50 mL/min; temperature: 25°C; UV: 280 nm<br><u>Analytical chiral HPLC:</u> Rt = 2.23 min<br>Instrument: Thermo Fisher UltiMate 3000; Column: YMC Cellulose SC 3μ, 100x4.6; eluent A: methyl tert-butyl ether + 0.1 vol % diethylamine; eluent B: ethanol; isocratic: 95%A+5%B; flow: 1.4 mL/min; temperature: 25°C; UV: 280 nm | | | |
| **6.2** | 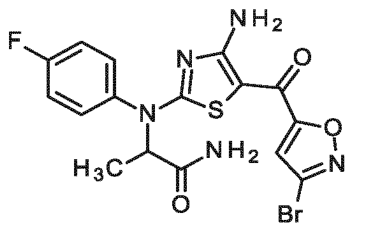 2-(N-[4-amino-5-(3-bromoisoxazole-5-carbonyl)thiazol-2-yl]-4-fluoro-anilino)propanamide (enantiomer 2) | Example 6 | $^1$H NMR (400 MHz, DMSO-$d_6$) δ ppm = 1.18 (d, $J$=7.35 Hz, 3 H), 5.10 (m, 1 H), 7.21 (s, 1 H), 7.30 (s, 1 H), 7.39 (t, $J$=8.74 Hz, 2 H), 7.63 (br s, 1 H), 7.70 (dd, $J$=8.74, 4.94 Hz, 2 H), 8.57 (br s, 1 H), 8.70 (br s, 1 H) LC-MS (method 2) Rt = 1.16 min MS (ESIpos): m/z = 454.2 [M+H]$^+$ 48 % yield |
| Chiral HPLC Example 6.2<br>HPLC separation of rac-2-(N-[4-amino-5-(3-bromoisoxazole-5-carbonyl)thiazol-2-yl]-4-fluoro-anilino)propanamide (150 mg, 0.33 mmol, Example 6) on a chiral column gave 74 mg (48 % yield) of 2-(N-[4-amino-5-(3-bromoisoxazole-5-carbonyl)thiazol-2-yl]-4-fluoro-anilino)propanamide, enantiomer 2.<br><u>Preparative chiral HPLC</u><br>Instrument: PrepCon Labomatic HPLC-1; Column: YMC Cellulose SC 5μ, 250x30; eluent A: methyl tert-butyl ether + 0.1 vol % diethylamine; eluent B: ethanol; isocratic: 95%A+5%B; flow: 50 mL/min; temperature: 25°C; UV: 280 nm<br><u>Analytical chiral HPLC:</u> Rt = 4.34 min<br>Instrument: Thermo Fisher UltiMate 3000; Column: YMC Cellulose SC 3μ, 100x4.6; eluent A: methyl tert-butyl ether + 0.1 vol % diethylamine; eluent B: ethanol; isocratic: 95%A+5%B; flow: 1.4 mL/min; temperature: 25°C; UV: 280 nm | | | |

(continued)

| Example number | Chemical structure Compound name | Starting materials | Analytics/purification/yield |
|---|---|---|---|
| **7** | 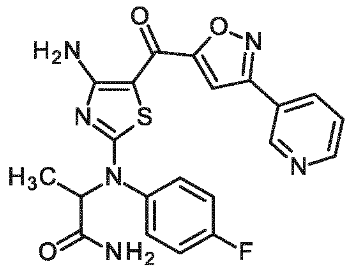 rac-2-(N-[4-amino-5-[3-(3-pyridyl)isoxazole-5-carbonyl]thiazol-2-yl]-4-fluoro-anilino) propanamide | Intermediate 7, rac-2-bromopropanam ide | $^1$H NMR (400 MHz, DMSO-$d_6$) δ ppm = 1.20 (d, *J*=7.60 Hz, 3 H), 5.06 - 5.19 (m, 1 H), 7.28 - 7.32 (m, 1 H), 7.38 - 7.45 (m, 2 H), 7.56 (ddd, *J*=7.92, 4.88, 0.89 Hz, 1 H), 7.62 - 7.66 (m, 2 H), 7.69 - 7.74 (m, 2 H), 8.27 - 8.32 (m, 1 H), 8.48 - 8.55 (m, 1 H), 8.70 (dd, *J*=4.82, 1.52 Hz, 1 H), 8.72 - 8.79 (m, 1 H), 9.11 (dd, *J*=2.28, 0.76 Hz, 1 H) RP-HPLC (method C basic) LC-MS (method 2) Rt = 0.98 min MS (ESIpos): m/z = 453.3 [M+H]+ 38 % yield |
| **8** | 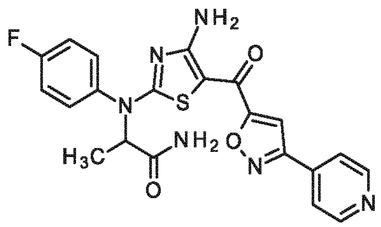 rac-2-(N-[4-amino-5-[3-(4-pyridyl)isoxazole-5-carbonyl]thiazol-2-yl]-4-fluoro-anilino) propanamide | Intermediate 8, rac-2-bromopropanam ide | $^1$H NMR (400 MHz, DMSO-$d_6$) δ ppm = 1.20 (d, *J*=7.35 Hz, 3 H), 5.06 - 5.18 (m, 1 H), 7.28 - 7.32 (m, 1 H), 7.41 (dd, *J*=8.87 Hz, 2 H), 7.60 - 7.66 (m, 1 H), 7.67 (s, 1 H), 7.69 - 7.75 (m, 2 H), 7.88 - 7.91 (m, 2 H), 8.48 - 8.56 (br s, 1H), 8.73 - 8.75 (m, 3 H) RP-HPLC (method C basic) LC-MS (method 2) Rt = 0.97 min MS (ESIpos): m/z = 453.4 [M+H]+ 2 % yield |

**Example 9** rac-ethyl 5-[4-amino-2-(N-(2-amino-1-methyl-2-oxo-ethyl)-4-fluoro-anilino)thiazole-5-carbonyl]isoxazole-3-caboxylate

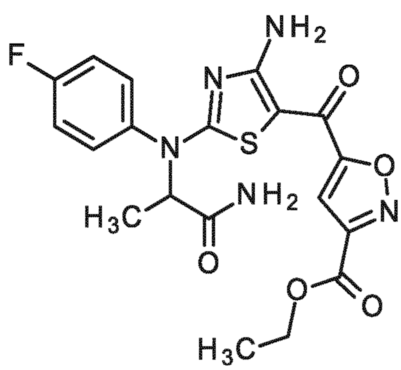

Ethyl 5-[4-amino-2-(4-fluoroanilino)thiazole-5-carbonyl]isoxazole-3-carboxylate (585 mg, 1.55 mmol, Intermediate 12) was suspended in DMF (10 mL) and treated with rac-2-bromo propionamide (283.5 mg, 1.86 mmol) and potassium carbonate (1074 mg, 7.8 mmol). The reaction mixture was stirred for 48 h and then treated with water. After 30

min the reaction mixture was extracted three times with ethyl acetate. The combined organic phases were washed with saturated aqueous ammonium hydrochloride solution, filtered through a water repellent filter circle (MN 617 WA) and evaporated to dryness. The crude product was purified by Biotage (method X) to give 43 mg (6 % yield) of the title compound.

$^1$H NMR (400 MHz, DMSO-$d_6$) δ ppm = 1.19 (d, *J*=7.35 Hz, 3 H), 1.30 (t, *J*=7.10 Hz, 3 H), 4.35 (q, *J*=7.10 Hz, 2 H), 5.16 (m, 1 H), 7.12 (s, 1 H), 7.30 (s, 1 H), 7.40 (t, *J*=8.87 Hz, 2 H), 7.63 (m, 1 H), 7.71 (dd, *J*=8.62, 5.07 Hz, 2 H), 8.59 (m, 1 H), 8.74 (m, 1 H).

LC-MS (method 1): Rt = 1.02 min; MS(ESIpos) m/z = 448.4 [M+H]$^+$

## Example 10

rac-5-[4-amino-2-(N-(2-amino-1-methyl-2-oxo-ethyl)-4-fluoro-anilino)thiazole-5-carbonyl]-N-phenyl-isoxazole-3-carboxamide

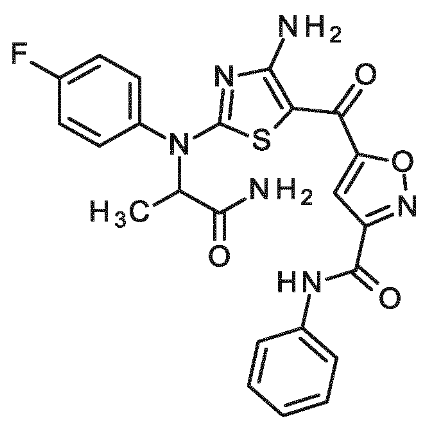

rac-5-[4-Amino-2-(N-(2-amino-1-methyl-2-oxo-ethyl)-4-fluoro-anilino)thiazole-5 carbonyl]isoxazole-3-carboxylic acid (Intermediate 14, 50 mg, 0.12 mmol) and aniline (13 mg, 0.14 mmol) were solved in 1 mL DMF and treated with HATU (54 mg, 0.14 mmol) and N,N-diisopropylethylamine (23 mg, 0.18 mmol). The reaction mixture was stirred at rt for 3.5 h, filtrated and purified by RP-HPLC (method C) to yield 24 mg (0.05 mmol, 39%) of the title compound.

$^1$H NMR (400 MHz, DMSO-$d_6$) δ ppm = 1.21 (d, *J*=7.35 Hz, 3 H), 5.12 (m, 1 H), 7.12 - 7.18 (m, 1 H), 7.22 (s, 1 H), 7.31 (br s, 1 H), 7.34 - 7.44 (m, 4 H), 7.64 (br s, 1 H), 7.68 - 7.78 (m, 4 H), 8.57 (br s, 1 H), 8.70 (br s, 1 H), 10.68 (s, 1 H). LC-MS (method 2): Rt = 1.11 min; MS(ESIpos) m/z = 495.2 [M+H]$^+$

The following examples were prepared from the starting materials stated in Table 4, below, using the procedure as for Example 10.

The crude product was either purified by RP-HPLC (methods A-D depending on polarity) or by preparative flash chromatography (methods X, Y or Z depending on polarity) after precipitation, extraction or filtration of the reaction mixture if necessary.

Enantiomers were separated from their racemate by chiral HPLC using the column and solvent conditions stated.

**Table 4: Examples 11 - 32.2**

| Example number | Chemical structure Compound name | Starting materials | Analytics/purification/yield |
|---|---|---|---|
| **11** | 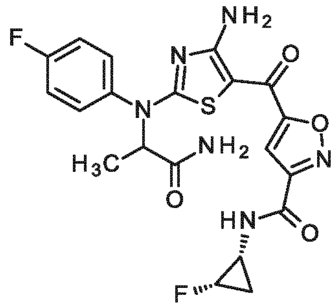 5-[4-amino-2-(N-(2-amino-1-methyl-2-oxo-ethyl)-4-fluoro-anilino)thiazole-5-carbonyl]-N-[(cis)-2-fluorocyclopropyl] isoxazol e-3-carboxamide (mixture of stereoisomers) | Intermediate 14, rac-(1 R,2S)-2-fluorocyclopropa-nam ine | $^1$H NMR (400 MHz, DMSO-$d_6$) δ ppm = 1.06 - 1.16 (m, 2H), 1.20 (d, *J*=7.60 Hz, 3 H), 2.76 - 2.85 (m, 1 H), 4.63 - 4.86 (m, 1 H), 5.03 - 5.19 (m, 1 H), 7.10 (s, 1 H), 7.31 (br s, 1 H), 7.36 - 7.43 (m, 2 H), 7.63 (br s, 1 H), 7.67 - 7.75 (m, 2 H), 8.54 (br s, 1 H), 8.67 (br s, 1 H), 8.98 (d, *J*=3.80 Hz, 1 H) RP-HPLC (method C basic)<br>LC-MS (method 2) Rt = 0.92 min MS (ESIpos): m/z = 477.4 $[M+H]^+$ 17 % yield |
| **12** | 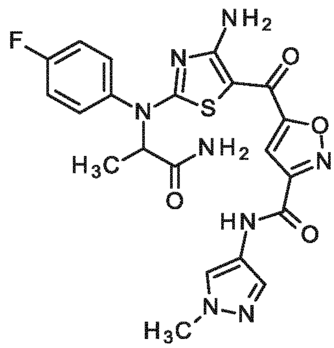 rac-5-[4-amino-2-(N-(2-amino-1-methyl-2-oxoethyl)-4-fluoro-anilino)thiazole-5-carbonyl]-N-(1-methylpyrazol-4-yl)isoxazole-3-carboxamide | Intermediate 14, 1-methyl-1H-pyrazol-4-amine | $^1$H NMR (400 MHz, DMSO-$d_6$) δ ppm = 1.20 (d, *J*=7.35 Hz, 3 H), 3.81 (s, 3 H), 5.03 - 5.20 (m, 1 H), 7.16 (s, 1 H), 7.31 (br s, 1 H), 7.40 (t, *J*=8.87 Hz, 2 H), 7.57 (s, 1 H), 7.64 (br s, 1 H), 7.71 (dd, *J*=8.62, 5.07 Hz, 2 H), 7.99 (s, 1 H), 8.56 (br s, 1 H), 8.68 (br s, 1 H), 10.92 (s, 1 H)<br>RP-HPLC (method C basic)<br>LC-MS (method 2) Rt = 0.89 min MS (ESIpos): m/z = 499.3 $[M+H]^+$ 17 % yield |
| **13** | 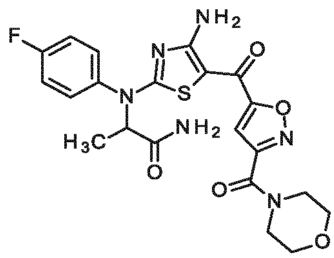 rac-2-(N-[4-amino-5-[3-(morpholine-4-carbonyl) isoxazole-5-carbonyl]thiazol-2-yl]-4-fluoro-anilino)propanamide | Intermediate 14, morpho-line | $^1$H NMR (400 MHz, DMSO-$d_6$) δ ppm = 1.19 (d, *J*=7.35 Hz, 3 H), 3.48 - 3.59 (m, 4 H), 3.59 - 3.70 (m, 4 H), 5.05 - 5.19 (m, 1 H), 7.05 (s, 1 H), 7.30 (br s, 1 H), 7.40 (br t, *J*=8.74 Hz, 2 H), 7.63 (br s, 1 H), 7.71 (br dd, *J*=8.49, 5.20 Hz, 2 H), 8.54 (br s, 1 H), 8.71 (br s, 1 H)<br>RP-HPLC (method C basic)<br>LC-MS (method 2) Rt = 0.92 min MS (ESIpos): m/z = 489.3 $[M+H]^+$ 34 % yield |

(continued)

| Example number | Chemical structure Compound name | Starting materials | Analytics/purification/yield |
|---|---|---|---|
| **14** | 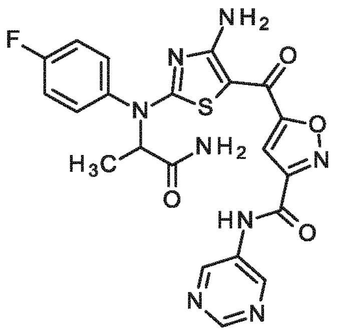 rac-5-[4-amino-2-(N-(2-amino-1-methyl-2-oxoethyl)-4-fluoro-anilino)thiazole-5-carbonyl]-N-pyrimidin-5-yl-isoxazole-3-carboxamide | Intermediate 14, pyrimidin-5-amine | $^{1}H$ NMR (400 MHz, DMSO-$d_6$) δ ppm = 1.21 (d, $J$=7.35 Hz, 3 H), 5.07 - 5.19 (m, 1 H), 7.24 (s, 1 H), 7.31 (br s, 1 H), 7.38 - 7.44 (m, 2 H), 7.64 (br s, 1 H), 7.72 (br dd, $J$=8.74, 4.94 Hz, 2 H), 8.60 (br s, 1 H), 8.69 (br s, 1 H), 8.98 (s, 1 H), 9.15 (s, 2 H), 11.18 (br s, 1 H) RP-HPLC (method C basic) LC-MS (method 2) Rt = 0.73 min MS (ESIpos): m/z = 497.3 $[M+H]^+$ 34 % yield |
| **15** | 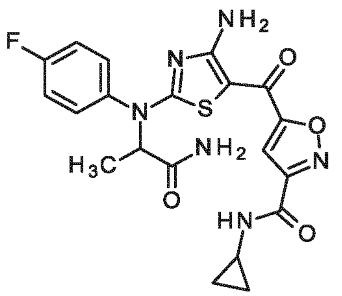 rac-5-[4-amino-2-(N-(2-amino-1-methyl-2-oxoethyl)-4-fluoro-anilino)thiazole-5-carbonyl]-N-cyclopropyl-isoxazole-3-carboxamide | Intermediate 14, cyclopropanamine | $^{1}H$ NMR (400 MHz, DMSO-$d_6$) δ ppm = 0.53 - 0.61 (m, 2 H), 0.65 - 0.73 (m, 2 H), 1.19 (d, $J$=7.35 Hz, 3 H), 2.76 - 2.86 (m, 1 H), 5.04 - 5.19 (m, 1 H), 7.05 (s, 1 H), 7.30 (br s, 1 H), 7.39 (t, $J$=8.87 Hz, 2 H), 7.63 (br s, 1 H), 7.70 (br dd, $J$=8.62, 5.07 Hz, 2 H), 8.53 (br s, 1 H), 8.66 (br s, 1 H), 8.84 (d, $J$=4.31 Hz, 1 H) RP-HPLC (method C basic) LC-MS (method 2) Rt = 0.96 min MS (ESIpos): m/z = 459.3 $[M+H]^+$ 30 % yield |
| **16** | 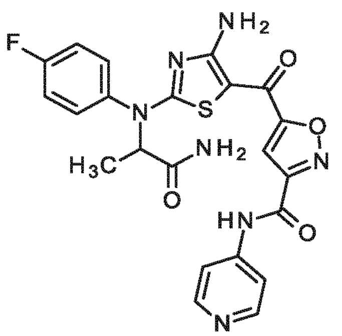 rac-5-[4-amino-2-(N-(2-amino-1-methyl-2-oxoethyl)-4-fluoro-anilino)thiazole-5-carbonyl]-N-(4-pyridyl)isoxazole-3-carboxamide | Intermediate 14, pyridin-4-amine | $^{1}H$ NMR (400 MHz, DMSO-$d_6$) δ ppm = 1.20 (d, $J$=7.35 Hz, 3 H), 5.05 - 5.19 (m, 1 H), 7.24 (s, 1 H), 7.31 (br s, 1 H), 7.37 - 7.45 (m, 2 H), 7.64 (br s, 1 H), 7.69 - 7.79 (m, 4 H), 8.47 - 8.53 (m, 2 H), 8.58 (br s, 1 H), 8.70 (br s, 1 H), 10.83 - 11.13 (m, 1 H) RP-HPLC (method B basic) LC-MS (method 2) Rt = 0.88 min MS (ESIpos): m/z = 496.2 $[M+H]^+$ 14 % yield |

(continued)

| Example number | Chemical structure Compound name | Starting materials | Analytics/purification/yield |
|---|---|---|---|
| **17** | 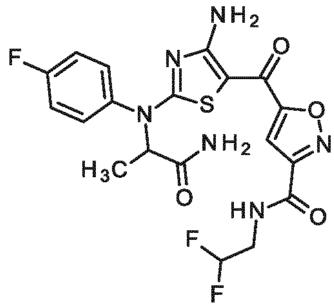 rac-5-[4-amino-2-(N-(2-amino-1-methyl-2-oxoethyl)-4-fluoro-anilino)thiazole-5-carbonyl]-N-(2,2-difluoroethyl) isoxazole-3-carboxamide | Intermediate 14, 2,2-difluoroethanamine | $^1$H NMR (400 MHz, DMSO-$d_6$) δ ppm = 1.19 (d, *J*=7.35 Hz, 3 H), 3.59 - 3.72 (m, 2 H), 5.04 - 5.19 (m, 1 H), 5.95 - 6.28 (m, 1 H), 7.11 (s, 1 H), 7.24 - 7.35 (m, 1 H), 7.40 (t, *J*=8.74 Hz, 2 H), 7.63 (br s, 1 H), 7.67 - 7.74 (m, 2 H), 8.55 (br s, 1 H), 8.69 (br s, 1 H), 9.15 - 9.21 (m, 1 H) RP-HPLC (method B basic) LC-MS (method 2) Rt = 0.95 min MS (ESIpos): m/z = 483.4 [M+H]$^+$ 21 % yield |
| **18** | 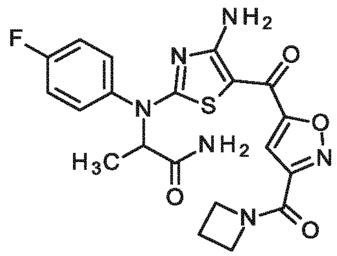 rac-2-(N-[4-amino-5-[3-(azetidine-1-carbonyl) isoxazole-5-carbonyl]thiazol-2-yl]-4-fluoro-anilino)propanamide | Intermediate 14, azetidine hydrochloride (1:1) | $^1$H NMR (400 MHz, DMSO-$d_6$) δ ppm = 1.19 (d, *J*=7.35 Hz, 3 H), 2.22 - 2.31 (m, 2 H), 4.02 - 4.08 (m, 2 H), 4.40 (t, *J*=7.60 Hz, 2 H), 5.04 - 5.18 (m, 1 H), 6.98 (s, 1 H), 7.30 (br s, 1 H), 7.37 - 7.44 (m, 2 H), 7.63 (br s, 1 H), 7.71 (br dd, *J*=8.49, 4.69 Hz, 2 H), 8.54 (br s, 1 H), 8.70 (br s, 1 H) RP-HPLC (method B basic) LC-MS (method 2) Rt = 0.96 min MS (ESIpos): m/z = 459.3 [M+H]$^+$ 27 % yield |
| **19** | 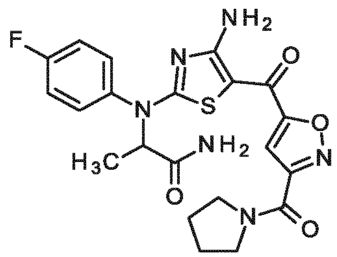 rac-2-(N-[4-amino-5-[3-(pyrrolidine-1-carbonyl) isoxazole-5-carbonyl]thiazol-2-yl]-4-fluoro-anilino)propanamide | Intermediate 14, pyrrolidine | $^1$H NMR (400 MHz, DMSO-$d_6$) δ ppm = 1.81 - 1.87 (m, 4 H), 2.83 - 2.87 (m, 4 H), 3.47 (t, *J*=6.59 Hz, 3 H), 3.61 (t, *J*=6.46 Hz, 2 H), 5.05 - 5.18 (m, 1 H), 7.01 (s, 1 H), 7.30 (br s, 1 H), 7.37 - 7.43 (m, 2 H), 7.63 (br s, 1 H), 7.71 (dd, *J*=8.87, 5.07 Hz, 2 H), 8.53 (br s, 1 H), 8.71 (br s, 1 H) RP-HPLC (method B basic) LC-MS (method 2) Rt = 1.02 min MS (ESIpos): m/z = 473.3 [M+H]$^+$ 28 % yield |

(continued)

| Example number | Chemical structure Compound name | Starting materials | Analytics/purification/yield |
|---|---|---|---|
| **20** | 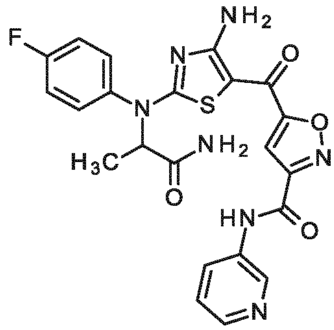 rac-5-[4-amino-2-(N-(2-amino-1-methyl-2-oxoethyl)-4-fluoro-anilino)thiazole-5-carbonyl]-N-(3-pyridyl)isoxazole-3-carboxamide | Intermediate 14, pyridin-3-amine | $^{1}$H NMR (400 MHz, DMSO-$d_6$) δ ppm = 1.21 (d, *J*=7.60 Hz, 3 H), 5.01 - 5.20 (m, 1 H), 7.18 - 7.28 (m, 1 H), 7.31 (br s, 1 H), 7.38 - 7.46 (m, 3 H), 7.64 (br s, 1 H), 7.68 - 7.75 (m, 2 H), 8.15 (ddd, *J*=8.36, 2.53, 1.52 Hz, 1 H), 8.36 (dd, *J*=4.82, 1.52 Hz, 1 H), 8.57 (br s, 1 H), 8.69 (br s, 1 H), 8.91 (d, *J*=2.03 Hz, 1 H), 10.95 (br s, 1 H)<br>RP-HPLC (method B basic)<br>LC-MS (method 2) Rt = 0.91 min MS (ESIpos): m/z = 496.3 [M+H]$^+$ 42 % yield |
| **21** | 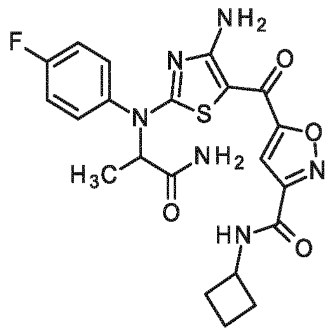 rac-5-[4-amino-2-(N-(2-amino-1-methyl-2-oxoethyl)-4-fluoro-anilino)thiazole-5-carbonyl]-N-cyclobutyl-isoxazole-3-carboxamide | Intermediate 14, cyclobutanamine | $^{1}$H NMR (400 MHz, DMSO-$d_6$) δ ppm = 1.20 (d, *J*=7.35 Hz, 3 H), 1.59 - 1.71 (m, 2 H), 1.99- 2.11 (m, 2 H), 2.12 - 2.22 (m, 2 H), 4.29 - 4.42 (m, 1 H), 5.06 - 5.17 (m, 1 H), 7.06 (s, 1 H), 7.30 (br s, 1 H), 7.40 (br t, *J*=8.74 Hz, 2 H), 7.63 (br s, 1 H), 7.70 (br dd, *J*=8.62, 5.07 Hz, 2 H), 8.54 (br s, 1 H), 8.68 (br s, 1 H), 9.02 (d, *J*=7.86 Hz, 1 H)<br>RP-HPLC (method B basic)<br>LC-MS (method 2) Rt = 1.05 min MS (ESIpos): m/z = 473.3 [M+H]$^+$ 33 % yield |
| **22** | 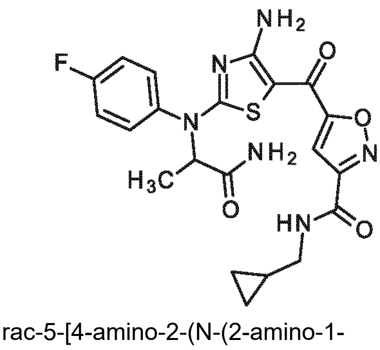 rac-5-[4-amino-2-(N-(2-amino-1-methyl-2-oxoethyl)-4-fluoro-anilino)thiazole-5-carbonyl]-N-(cyclopropylmethyl) isoxaz ole-3-carboxamide | Intermediate 14, 1-cyclopropylmethana mine | $^{1}$H NMR (400 MHz, DMSO-$d_6$) δ ppm = 0.18 - 0.24 (m, 2 H), 0.38 - 0.45 (m, 2 H), 0.96 - 1.06 (m, 1 H), 1.19 (d, *J*=7.35 Hz, 3 H), 3.10 (t, *J*=6.34 Hz, 2 H), 5.05 - 5.20 (m, 1 H), 7.07 (s, 1 H), 7.30 (s, 1 H), 7.36 - 7.44 (m, 2 H), 7.64 (br s, 1 H), 7.71 (dd, *J*=8.74, 5.20 Hz, 2 H), 8.55 (br s, 1 H), 8.67 (br s, 1 H), 8.90 (t, *J*=5.70 Hz, 1 H)<br>RP-HPLC (method C basic)<br>LC-MS (method 2) Rt = 1.03 min MS (ESIpos): m/z = 473.4 [M+H]$^+$ 15 % yield |

(continued)

| Example number | Chemical structure Compound name | Starting materials | Analytics/purification/yield |
|---|---|---|---|
| **23** | 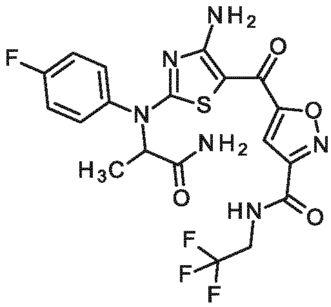 rac-5-[4-amino-2-(N-(2-amino-1-methyl-2-oxoethyl)-4-fluoro-anilino)thiazole-5-carbonyl]-N-(2,2,2-trifluoroethyl) isoxazole-3-carboxamide | Intermediate 14, 2,2,2-trifluoroethanamine | $^1H$ NMR (400 MHz, DMSO-$d_6$) δ ppm = 1.20 (d, *J*=7.35 Hz, 3 H), 3.99 - 4.12 (m, 2 H), 5.03 - 5.19 (m, 1 H), 7.14 (s, 1 H), 7.30 (br s, 1 H), 7.40 (t, *J*=8.74 Hz, 2 H), 7.63 (br s, 1 H), 7.71 (dd, *J*=8.62, 5.07 Hz, 2 H), 8.55 (br s, 1 H), 8.68 (br s, 1 H), 9.49 (br t, *J*=6.08 Hz, 1 H) RP-HPLC (method C basic) LC-MS (method 2) Rt = 1.01 min MS (ESIpos): m/z = 501.3 $[M+H]^+$ 19 % yield |
| **24** | 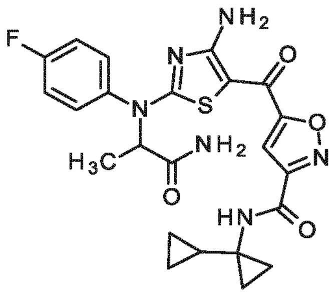 rac-5-[4-amino-2-(N-(2-amino-1-methyl-2-oxoethyl)-4-fluoro-anilino)thiazole-5-carbonyl]-N-(1-cyclopropylcyclopropyl)iso xazole-3-carboxamide | Intermediate 14, [1,1'-bi(cyclopropyl)]-1-amine | $^1H$ NMR (400 MHz, DMSO-$d_6$) δ ppm = 0.12 - 0.19 (m, 2 H), 0.30 - 0.37 (m, 2 H), 0.53 - 0.59 (m, 2 H), 0.62 - 0.68 (m, 2 H), 1.19 (d, *J*=7.35 Hz, 3 H), 1.31 - 1.40 (m, 1 H), 5.03 - 5.18 (m, 1 H), 7.04 (s, 1 H), 7.29 (br s, 1 H), 7.35 - 7.43 (m, 2 H), 7.63 (br s, 1 H), 7.67 - 7.73 (m, 2 H), 8.57 (br s, 1 H), 8.66 (br s, 1 H), 9.02 (br s, 1 H) RP-HPLC (method C basic) LC-MS (method 2) Rt = 1.10 min MS (ESIpos): m/z = 499.4 $[M+H]^+$ 8 % yield |
| **25** | 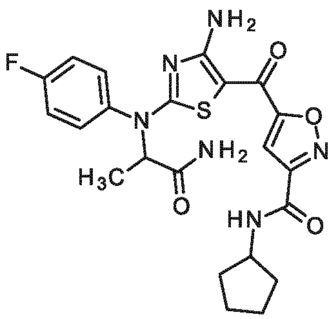 rac-5-[4-amino-2-(N-(2-amino-1-methyl-2-oxoethyl)-4-fluoro-anilino)thiazole-5-carbonyl]-N-cyclopentyl-isoxazole-3-carboxamide | Intermediate 14, cyclopentanamine | $^1H$ NMR (400 MHz, DMSO-$d_6$) δ ppm = 1.19 (d, *J*=7.60 Hz, 3 H), 1.45 - 1.57 (m, 4 H), 1.66 (br s, 2 H), 1.84 (br d, *J*=3.04 Hz, 2 H), 4.11 - 4.21 (m, 1 H), 5.04 - 5.20 (m, 1 H), 7.08 (s, 1 H), 7.24 - 7.36 (m, 1 H), 7.40 (t, *J*=8.87 Hz, 2 H), 7.63 (br s, 1 H), 7.70 (dd, *J*=8.87, 5.07 Hz, 2 H), 8.53 (br s, 1 H), 8.65 (br s, 1 H), 8.70 (d, *J*=7.35 Hz, 1 H) RP-HPLC (method C basic) LC-MS (method 2) Rt = 1.12 min MS (ESIpos): m/z = 487.4 $[M+H]^+$ 48 % yield |

(continued)

| Example number | Chemical structure Compound name | Starting materials | Analytics/purification/yield |
|---|---|---|---|
| **26** | 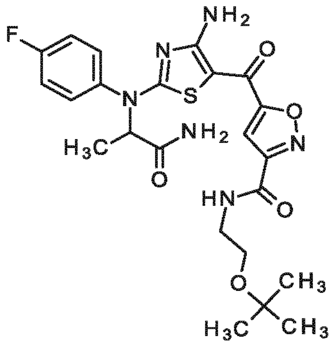 rac-5-[4-amino-2-(N-(2-amino-1-methyl-2-oxoethyl)-4-fluoro-anilino)thiazole-5-carbonyl]-N-(2-tert-butoxyethyl) isoxazole-3-carboxamide | Intermediate 14, 2-tert-butoxyethanamine hydrochloride (1:1) | $^1$H NMR (400 MHz, DMSO-$d_6$) δ ppm = 1.19 (d, *J*=7.35 Hz, 3 H), 3.27 - 3.32 (m, 2 H), 3.37 - 3.42 (m, 2 H), 5.04 - 5.21 (m, 1 H), 7.06 (s, 1 H), 7.30 (s, 1 H), 7.39 (t, *J*=8.74 Hz, 2 H), 7.63 (br s, 1 H), 7.66 - 7.74 (m, 2 H), 8.47 - 8.59 (m, 1 H), 8.62 - 8.69 (m, 1 H), 8.72 (t, *J*=5.70 Hz, 1 H) RP-HPLC (method C basic) LC-MS (method 2) Rt = 1.08 min MS (ESIpos): m/z = 519.2 $[M+H]^+$ 63 % yield |
| **27** | 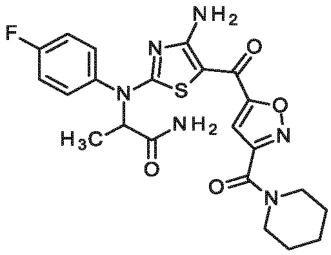 rac-2-(N-[4-amino-5-[3-(piperidine-1-carbonyl) isoxazole-5-carbonyl]thiazol-2-yl]-4-fluoro-anilino)propanamide | Intermediate 14, piperidine | $^1$H NMR (400 MHz, DMSO-$d_6$) δ ppm = 1.19 (d, *J*=7.35 Hz, 3 H), 1.44 - 1.65 (m, 6 H), 3.36 - 3.43 (m, 2 H), 3.58 (br t, *J*=5.32 Hz, 2 H), 5.05 - 5.19 (m, 1 H), 7.03 (s, 1 H), 7.29 (br s, 1 H), 7.40 (t, *J*=8.74 Hz, 2 H), 7.63 (br s, 1 H), 7.71 (br dd, *J*=8.62, 5.07 Hz, 2 H), 8.54 (br s, 1 H), 8.71 (br s, 1 H) RP-HPLC (method C basic) LC-MS (method 2) Rt = 1.07 min MS (ESIpos): m/z = 487.4 $[M+H]^+$ 27 % yield |
| **28** | 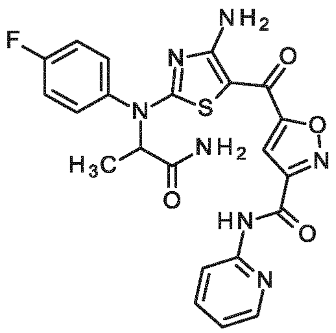 rac-5-[4-amino-2-(N-(2-amino-1-methyl-2-oxoethyl)-4-fluoro-anilino)thiazole-5-carbonyl]-N-(2-pyridyl)isoxazole-3-carboxamide | Intermediate 14, pyridin-2-amine | $^1$H NMR (400 MHz, DMSO-$d_6$) δ ppm = 1.20 (d, *J*=7.35 Hz, 3 H), 5.05 - 5.21 (m, 1 H), 7.22 (ddd, *J*=7.35, 4.82, 1.01 Hz, 1 H), 7.31 (s, 1 H), 7.34 (s, 1 H), 7.41 (t, *J*=8.74 Hz, 2 H), 7.64 (br s, 1 H), 7.72 (dd, *J*=8.74, 4.94 Hz, 2 H), 7.84 - 7.91 (m, 1 H), 8.10 (d, *J*=8.36 Hz, 1 H), 8.40 (dt, *J*=3.87, 0.98 Hz, 1 H), 8.58 (br s, 1 H), 8.72 (br s, 1 H), 10.94 (s, 1 H) RP-HPLC (method C basic) LC-MS (method 2) Rt = 1.04 min MS (ESIpos): m/z = 496.4 $[M+H]^+$ 24 % yield |

(continued)

| Example number | Chemical structure Compound name | Starting materials | Analytics/purification/yield |
|---|---|---|---|
| **29** | 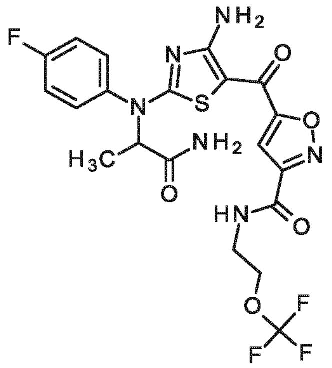 rac-5-[4-amino-2-(N-(2-amino-1-methyl-2-oxoethyl)-4-fluoro-anilino)thiazole-5-carbonyl]-N-[2-(trifluoromethoxy) ethyl]iso xazole-3-carboxamide | Intermediate 14, 2-(trifluoromethoxy)eth anamine hydrochloride (1:1) | $^1$H NMR (400 MHz, DMSO-$d_6$) δ ppm = 1.20 (d, *J*=7.35 Hz, 3 H), 3.55 (br d, *J*=5.32 Hz, 2 H), 4.18 (t, *J*=5.32 Hz, 2 H), 5.01 - 5.18 (m, 1 H), 7.07 (s, 1 H), 7.30 (s, 1 H), 7.39 (t, *J*=8.74 Hz, 2 H), 7.63 (br s, 1 H), 7.71 (dd, *J*=8.74, 5.20 Hz, 2 H), 8.54 (br s, 1 H), 8.62 - 8.72 (m, 1 H), 9.05 (t, J=5.70 Hz, 1 H) RP-HPLC (method C basic) LC-MS (method 2) Rt = 1.05 min MS (ESIpos): m/z = 531.1 [M+H]$^+$ 65 % yield |
| **30** | 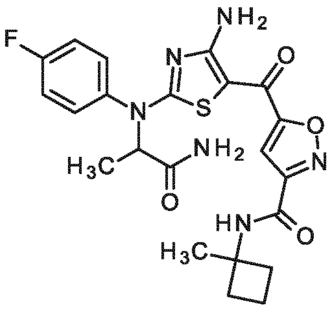 rac-5-[4-amino-2-(N-(2-amino-1-methyl-2-oxoethyl)-4-fluoro-anilino)thiazole-5-carbonyl]-N-(1-methylcyclobutyl) isoxazole -3-carboxamide | Intermediate 14, 1-methylcyclobutanam ine | $^1$H NMR (400 MHz, DMSO-$d_6$) δ ppm = 1.19 (d, *J*=7.60 Hz, 3 H), 1.43 (s, 3 H), 1.73 - 1.84 (m, 2 H), 1.90 - 2.01 (m, 2 H), 2.26 - 2.37 (m, 2 H), 5.04 - 5.19 (m, 1 H), 7.06 (s, 1 H), 7.30 (s, 1 H), 7.40 (t, *J*=8.74 Hz, 2 H), 7.63 (br s, 1 H), 7.70 (br dd, *J*=8.62, 5.07 Hz, 2 H), 8.52 (br s, 1 H), 8.66 (br s, 1 H), 8.76 (s, 1 H) RP-HPLC (method C basic) LC-MS (method 2) Rt = 1.12 min MS (ESIpos): m/z = 487.4 [M+H]$^+$ 16 % yield |
| **30.1 and 30.2** | (R)-5-[4-amino-2-(N-(2-amino-1-methyl-2-oxoethyl)-4-fluoro-anilino)thiazole-5-carbonyl]-N-(1-methylcyclobutyl) isoxazole -3-carboxamide and (S)-5-[4-amino-2-(N-(2-amino-1-methyl-2-oxo-ethyl)-4-fluoro-anilino)thiazole-5-carbonyl]-N-(1-methylcyclobutyl)isoxazole -3-carboxamide | | |
| **30.1** | 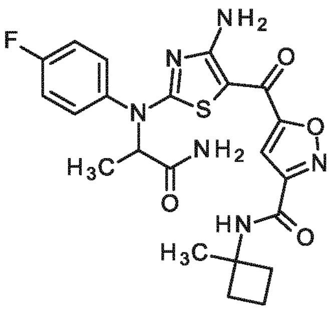 5-[4-amino-2-(N-(2-amino-1-methyl-2-oxo-ethyl)-4-fluoro-anilino)thiazole-5-carbonyl]-N-(1-methylcyclobutyl)isoxazole -3-carboxamide (enantiomer 1) | Example 30 | $^1$H NMR (400 MHz, DMSO-$d_6$) δ ppm = 1.19 (d, *J*=7.60 Hz, 3 H), 1.43 (s, 3 H), 1.73 - 1.84 (m, 2 H), 1.90 - 2.01 (m, 2 H), 2.26 - 2.37 (m, 2 H), 5.04 - 5.19 (m, 1 H), 7.06 (s, 1 H), 7.30 (s, 1 H), 7.40 (t, *J*=8.74 Hz, 2 H), 7.63 (br s, 1 H), 7.70 (br dd, *J*=8.62, 5.07 Hz, 2 H), 8.52 (br s, 1 H), 8.66 (br s, 1 H), 8.76 (s, 1 H) LC-MS (method 2) Rt = 1.12 min MS (ESIpos): m/z = 487.4 [M+H]$^+$ 41 % yield |

(continued)

| Example number | Chemical structure Compound name | Starting materials | Analytics/purification/yield |
|---|---|---|---|
| Chiral HPLC Example 30.1<br>HPLC separation of rac-5-[4-amino-2-(N-(2-amino-1-methyl-2-oxo-ethyl)-4-fluoro-anilino)thiazole-5-carbonyl]-N-(1-methylcyclobutyl)isoxazole-3-carboxamide (180 mg, 0.37 mmol, Example 30) on a chiral column gave 77 mg (41 % yield) of 5-[4-amino-2-(N-(2-amino-1-methyl-2-oxo-ethyl)-4-fluoro-anilino)thiazole-5-carbonyl]-N-(1-methylcyclobutyl)isoxazole-3-carboxamide, enantiomer 1.<br>Preparative chiral HPLC<br>Instrument: PrepCon Labomatic HPLC-3; Column: YMC Cellulose SC 5μ, 250x30; eluent A: hexane + 0.1 vol % diethylamine; eluent B: ethanol + 0.1 vol % diethylamine; isocratic: 60%A+40%B; flow: 50 mL/min; temperature: 25°C; UV: 254 nm<br>Analytical chiral HPLC: Rt = 3.56 min<br>Instrument: Thermo Fisher UltiMate 3000; Column: YMC Cellulose SC 3μ, 100x4.6; eluent A: hexane + 0.1 vol % diethylamine; eluent B: ethanol; isocratic: 60%A+40%B; flow: 1.4 mL/min; temperature: 25°C; UV: 254 nm | | | |
| **30.2** | 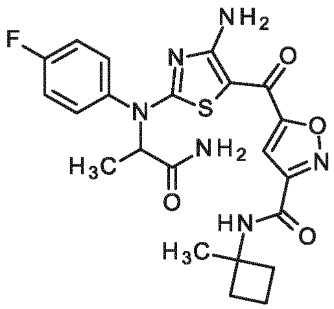<br>5-[4-amino-2-(N-(2-amino-1-methyl-2-oxo-ethyl)-4-fluoro-anilino)thiazole-5-carbonyl]-N-(1-methylcyclobutyl)isoxazole -3-carboxamide (enantiomer 2) | Example 30 | $^1$H NMR (400 MHz, DMSO-$d_6$) δ ppm = 1.19 (d, *J*=7.60 Hz, 3 H), 1.43 (s, 3 H), 1.73 - 1.84 (m, 2 H), 1.90 - 2.01 (m, 2 H), 2.26 - 2.37 (m, 2 H), 5.04 - 5.19 (m, 1 H), 7.06 (s, 1 H), 7.30 (s, 1 H), 7.40 (t, *J*=8.74 Hz, 2 H), 7.63 (br s, 1 H), 7.70 (br dd, *J*=8.62, 5.07 Hz, 2 H), 8.52 (br s, 1 H), 8.66 (br s, 1 H), 8.76 (s, 1 H)<br>LC-MS (method 2) Rt = 1.12 min MS (ESIpos): m/z = 487.4 $[M+H]^+$ 43 % yield |
| Chiral HPLC Example 30.2<br>HPLC separation of rac-5-[4-amino-2-(N-(2-amino-1-methyl-2-oxo-ethyl)-4-fluoro-anilino)thiazole-5-carbonyl]-N-(1-methylcyclobutyl)isoxazole-3-carboxamide (180 mg, 0.37 mmol, Example 30) on a chiral column gave 81 mg (43 % yield) of 5-[4-amino-2-(N-(2-amino-1-methyl-2-oxo-ethyl)-4-fluoro-anilino)thiazole-5-carbonyl]-N-(1-methylcyclobutyl)isoxazole-3-carboxamide, enantiomer 2.<br>Preparative chiral HPLC<br>Instrument: PrepCon Labomatic HPLC-3; Column: YMC Cellulose SC 5μ, 250x30; eluent A: hexane + 0.1 vol % diethylamine; eluent B: ethanol + 0.1 vol % diethylamine; isocratic: 60%A+40%B; flow: 50 mL/min; temperature: 25°C; UV: 254 nm<br>Analytical chiral HPLC: Rt = 5.02 min<br>Instrument: Thermo Fisher UltiMate 3000; Column: YMC Cellulose SC 3μ, 100x4.6; eluent A: hexane + 0.1 vol % diethylamine; eluent B: ethanol; isocratic: 60%A+40%B; flow: 1.4 mL/min; temperature: 25°C; UV: 254 nm | | | |

(continued)

| Example number | Chemical structure Compound name | Starting materials | Analytics/purification/yield |
|---|---|---|---|
| **31** | 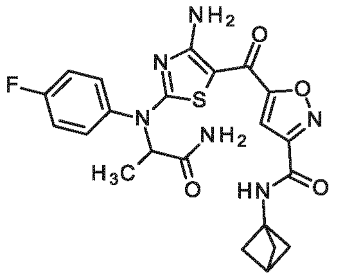<br>rac-5-[4-amino-2-(N-(2-amino-1-methyl-2-oxoethyl)-4-fluoro-anilino)thiazole-5-carbonyl]-N-(1-bicyclo[1.1.1]pentanyl)isox azole-3-carboxamide | Intermediate 14, bicyclo [1.1.1]pentan-1-amine | $^1$H NMR (400 MHz, DMSO-$d_6$) δ ppm = 1.19 (d, *J*=7.35 Hz, 3 H), 2.06 (s, 6 H), 2.45 (s, 1H), 5.03 - 5.18 (m, 1 H), 7.04 (s, 1 H), 7.30 (br s, 1 H), 7.39 (t, *J*=8.74 Hz, 2 H), 7.63 (br s, 1 H), 7.70 (br dd, *J*=8.62, 5.07 Hz, 2 H), 8.53 (br s, 1 H), 8.68 (br s, 1 H), 9.34 (s, 1 H)<br>RP-HPLC (method C basic)<br>LC-MS (method 2) Rt = 1.10 min MS (ESIpos): m/z = 485.4 $[M+H]^+$ 16 % yield |
| **32** | 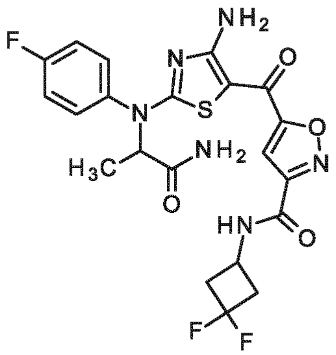<br>rac-5-[4-amino-2-(N-(2-amino-1-methyl-2-oxoethyl)-4-fluoro-anilino)thiazole-5-carbonyl]-N-(3,3-difluorocyclobutyl)isoxazol e-3-carboxamide | Intermediate 14, 3,3-difluorocyclobutana mine hydrochloride (1:1) | $^1$H NMR (400 MHz, DMSO-$d_6$) δ ppm = 1.20 (d, *J*=7.35 Hz, 3 H), 2.71 - 2.83 (m, 2 H), 2.86 - 3.00 (m, 2 H), 4.15 - 4.29 (m, 1 H), 5.04 - 5.18 (m, 1 H), 7.08 (s, 1 H), 7.30 (br s, 1 H), 7.40 (t, *J*=8.74 Hz, 2 H), 7.63 (br s, 1 H), 7.70 (dd, *J*=8.62, 5.07 Hz, 2 H), 8.55 (br s, 1 H), 8.66 (br s, 1 H), 9.29 (d, *J*=6.59 Hz, 1 H)<br>RP-HPLC (method C basic)<br>LC-MS (method 2) Rt = 1.03 min MS (ESIpos): m/z = 509.4 $[M+H]^+$ 8 % yield |
| **32.1 and 32.2** | (R)-5-[4-amino-2-(N-(2-amino-1-methyl-2-oxoethyl)-4-fluoro-anilino)thiazole-5-carbonyl]-N-(3,3-difluorocyclobutyl)isoxazol e-3-carboxamide and (S)-5-[4-amino-2-(N-(2-amino-1-methyl-2-oxo-ethyl)-4-fluoro-anilino)thiazole-5-carbonyl]-N-(3,3-difluorocyclobutyl)isoxazol e-3-carboxamide | | |
| **32.1** | 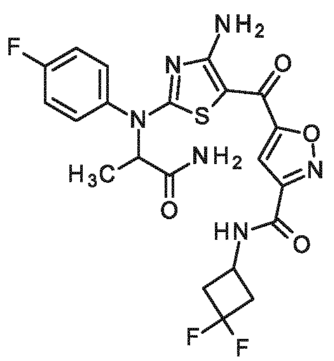<br>5-[4-amino-2-(N-(2-amino-1-methyl-2-oxo-ethyl)-4-fluoro-anilino)thiazole-5-carbonyl]-N-(3,3-difluorocyclobutyl)isoxazol e-3-carboxamide (enantiomer 1) | Example 32 | $^1$H NMR (400 MHz, DMSO-$d_6$) δ ppm = 1.20 (d, *J*=7.35 Hz, 3 H), 2.71 - 2.83 (m, 2 H), 2.86 - 3.00 (m, 2 H), 4.15 - 4.29 (m, 1 H), 5.04 - 5.18 (m, 1 H), 7.08 (s, 1 H), 7.30 (br s, 1 H), 7.40 (t, *J*=8.74 Hz, 2 H), 7.63 (br s, 1 H), 7.70 (dd, *J*=8.62, 5.07 Hz, 2 H), 8.55 (br s, 1 H), 8.66 (br s, 1 H), 9.29 (d, *J*=6.59 Hz, 1 H)<br>RP-HPLC (method C basic)<br>LC-MS (method 2) Rt = 1.03 min MS (ESIpos): m/z = 509.4 $[M+H]^+$ 47 % yield |

(continued)

| Example number | Chemical structure Compound name | Starting materials | Analytics/purification/yield |
|---|---|---|---|
| Chiral HPLC Example 32.1<br>HPLC separation of rac-5-[4-amino-2-(N-(2-amino-1-methyl-2-oxo-ethyl)-4-fluoro-anilino)thiazole-5-carbonyl]-N-(3,3-difluorocyclobutyl)isoxazole-3-carboxamide (200 mg, 0.39 mmol, Example 32) on a chiral column gave 95 mg (47 % yield) of 5-[4-amino-2-(N-(2-amino-1-methyl-2-oxo-ethyl)-4-fluoro-anilino)thiazole-5-carbonyl]-N-(3,3-difluorocyclobutyl)isoxazole-3-carboxamide, enantiomer 1.<br>Preparative chiral HPLC<br>Instrument: PrepCon Labomatic HPLC-3; Column: YMC Amylose SA 5μ, 250x30; eluent A: methyl tert-butyl ether + 0.1 vol % diethylamine; eluent B: ethanol + 0.1 vol % diethylamine; isocratic: 90%A+10%B; flow: 40 mL/min; temperature: 25°C; UV: 280 nm<br>Analytical chiral HPLC: Rt = 2.08 min<br>Instrument: Thermo Fisher UltiMate 3000; Column: YMC Amylose SA 3μ, 100x4.6; eluent A: methyl tert-butyl ether + 0.1 vol % diethylamine; eluent B: ethanol; isocratic: 90%A+10%B; flow: 1.4 mL/min; temperature: 25°C; UV: 280 nm | | | |
| **32.2** | 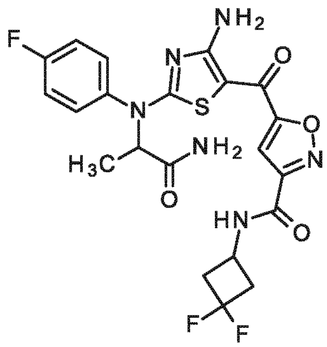<br>5-[4-amino-2-(N-(2-amino-1-methyl-2-oxo-ethyl)-4-fluoro-anilino)thiazole-5-carbonyl]-N-(3,3-difluorocyclobutyl)isoxazol e-3-carboxamide (enantiomer 2) | Example 32 | $^1$H NMR (400 MHz, DMSO-$d_6$) δ ppm = 1.20 (d, *J*=7.35 Hz, 3 H), 2.71 - 2.83 (m, 2 H), 2.86 - 3.00 (m, 2 H), 4.15 - 4.29 (m, 1 H), 5.04 - 5.18 (m, 1 H), 7.08 (s, 1 H), 7.30 (br s, 1 H), 7.40 (t, *J*=8.74 Hz, 2 H), 7.63 (br s, 1 H), 7.70 (dd, *J*=8.62, 5.07 Hz, 2 H), 8.55 (br s, 1 H), 8.66 (br s, 1 H), 9.29 (d, *J*=6.59 Hz, 1 H)<br>RP-HPLC (method C basic)<br>LC-MS (method 2) Rt = 1.03 min MS (ESIpos): m/z = 509.4 [M+H]$^+$ 42 % yield |
| Chiral HPLC Example 32.1<br>HPLC separation of rac-5-[4-amino-2-(N-(2-amino-1-methyl-2-oxo-ethyl)-4-fluoro-anilino)thiazole-5-carbonyl]-N-(3,3-difluorocyclobutyl)isoxazole-3-carboxamide (200 mg, 0.39 mmol, Example 32) on a chiral column gave 85 mg (42 % yield) of 5-[4-amino-2-(N-(2-amino-1-methyl-2-oxo-ethyl)-4-fluoro-anilino)thiazole-5-carbonyl]-N-(3,3-difluorocyclobutyl)isoxazole-3-carboxamide, enantiomer 2.<br>Preparative chiral HPLC<br>Instrument: PrepCon Labomatic HPLC-3; Column: YMC Amylose SA 5μ, 250x30; eluent A: methyl tert-butyl ether + 0.1 vol % diethylamine; eluent B: ethanol + 0.1 vol % diethylamine; isocratic: 90%A+10%B; flow: 40 mL/min; temperature: 25°C; UV: 280 nm<br>Analytical chiral HPLC: Rt = 3.03 min<br>Instrument: Thermo Fisher UltiMate 3000; Column: YMC Amylose SA 3μ, 100x4.6; eluent A: methyl tert-butyl ether + 0.1 vol % diethylamine; eluent B: ethanol; isocratic: 90%A+10%B; flow: 1.4 mL/min; temperature: 25°C; UV: 280 nm | | | |

The following examples were prepared from the starting materials stated in Table 5, below, using the procedure as for Example 1.

The crude product was either purified by RP-HPLC (methods A-D depending on polarity) or by preparative flash chromatography (methods X, Y or Z depending on polarity) after precipitation, extraction or filtration of the reaction mixture if necessary.

Enantiomers were separated from their racemate by chiral HPLC using the column and solvent conditions stated.

**Table 5: Examples 33 - 50**

| Example number | Chemical structure Compound name | Starting materials | Analytics/purification/yield |
|---|---|---|---|
| **33** | 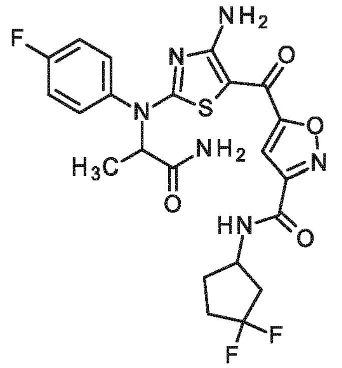 5-[4-amino-2-(N-(2-amino-1-methyl-2-oxo-ethyl)-4-fluoro-anilino)thiazole-5-carbonyl]-N-(3,3-difluorocyclopentyl)isoxaz ole-3-carboxamide (mixture of stereoisomers) | Intermediate 18, rac-2-bromopropanamide | $^1$H NMR (400 MHz, DMSO-$d_6$) δ ppm = 1.20 (d, *J*=7.35 Hz, 3 H), 1.74 - 1.86 (m, 1 H), 2.04 - 2.30 (m, 4 H), 2.39 - 2.46 (m, 1 H), 4.31 - 4.43 (m, 1 H), 5.04 - 5.19 (m, 1 H), 7.08 (s, 1 H), 7.30 (br s, 1 H), 7.40 (t, *J*=8.74 Hz, 2 H), 7.63 (br s, 1 H), 7.70 (dd, *J*=8.62, 5.07 Hz, 2 H), 8.53 (br s, 1 H), 8.68 (br s, 1 H), 9.02 (d, *J*=7.35 Hz, 1 H)<br>Biotage (method X)<br>LC-MS (method 1) Rt = 1.08 min MS (ESIpos): m/z = 523.4 [M+H]$^+$ 100 % yield |
| **34** | 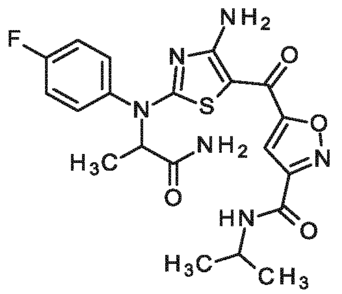 rac-5-[4-amino-2-(N-(2-amino-1-methyl-2-oxoethyl)-4-fluoro-anilino)thiazole-5-carbonyl]-N-isopropyl-isoxazole-3-carboxamide | Intermediate 19, rac-2-bromopropanamide | $^1$H NMR (400 MHz, DMSO-$d_6$) δ ppm = 1.13 (d, *J*=6.59 Hz, 6 H), 1.20 (d, *J*=7.35 Hz, 3 H), 3.98 - 4.10 (m, 1 H), 5.04 - 5.17 (m, 1 H), 7.07 (s, 1 H), 7.30 (s, 1 H), 7.36 - 7.43 (m, 2 H), 7.63 (br s, 1 H), 7.68 - 7.74 (m, 2 H), 8.45 - 8.57 (m, 1 H), 8.61 - 8.64 (m, 1 H), 8.64 - 8.71 (m, 1 H)<br>RP-HPLC (method C)<br>LC-MS (method 1) Rt = 1.03 min MS (ESIpos): m/z = 461.4 [M+H]$^+$ 74 % yield |
| **35** | 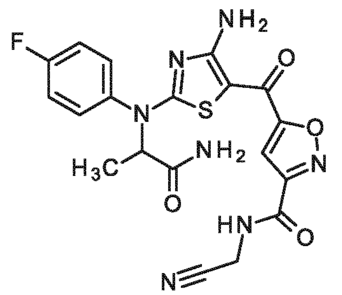 rac-5-[4-amino-2-(N-(2-amino-1-methyl-2-oxoethyl)-4-fluoro-anilino)thiazole-5-carbonyl]-N-(cyanomethyl)isoxazole-3-carboxamide | Intermediate 22, rac-2-bromopropanamide | $^1$H NMR (400 MHz, DMSO-$d_6$) δ ppm = 1.19 (d, *J*=7.60 Hz, 3 H), 4.32 (d, *J*=5.58 Hz, 2 H), 4.99 - 5.19 (m, 1 H), 7.13 (s, 1 H), 7.30 (s, 1 H), 7.40 (t, *J*=8.74 Hz, 2 H), 7.63 (br s, 1 H), 7.71 (dd, J=8.87, 5.07 Hz, 2 H), 8.58 (br s, 1 H), 8.69 (br s, 1 H), 9.56 (t, *J*=5.58 Hz, 1 H)<br>RP-HPLC (method C)<br>LC-MS (method 2) Rt = 0.88 min MS (ESIpos): m/z = 458.3 [M+H]$^+$ 23 % yield |

(continued)

| Example number | Chemical structure Compound name | Starting materials | Analytics/purification/yield |
|---|---|---|---|
| **36** | 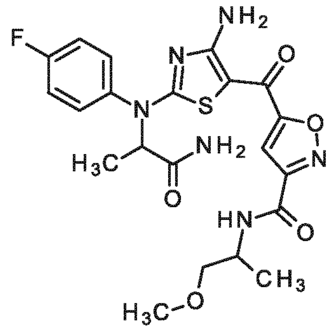 5-[4-amino-2-(N-(2-amino-1-methyl-2-oxo-ethyl)-4-fluoro-anilino)thiazole-5-carbonyl]-N-(2-methoxy-1-methyl-ethyl)isoxazole-3-carboxamide (mixture of stereoisomers) | Intermediate 20, rac-2-bromopropanamide | $^1$H NMR (400 MHz, DMSO-$d_6$) δ ppm = 1.10 (d, *J*=6.84 Hz, 3 H), 1.20 (d, *J*=7.35 Hz, 3 H), 3.24 (s, 3 H), 3.25 - 3.29 (m, 1 H), 3.35 - 3.39 (m, 1 H), 4.08 - 4.20 (m, 1 H), 5.05 - 5.18 (m, 1 H), 7.07 (s, 1 H), 7.30 (s, 1 H), 7.40 (dd, *J*=8.74 Hz, 2 H), 7.63 (br s, 1 H), 7.70 (dd, *J*=8.87, 5.07 Hz, 2 H), 8.54 (br s, 1 H), 8.60 (d, *J*=8.36 Hz, 1 H), 8.68 (br s, 1 H)<br><br>RP-HPLC (method C)<br>LC-MS (method 1) Rt = 1.00 min MS (ESIpos): m/z = 491.4 [M+H]$^+$ 39 % yield |
| **37** | 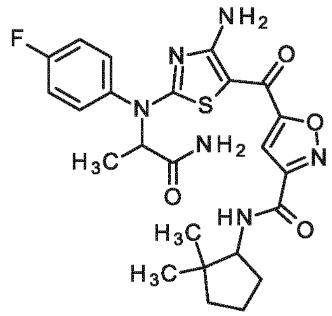 5-[4-amino-2-(N-(2-amino-1-methyl-2-oxo-ethyl)-4-fluoro-anilino)thiazole-5-carbonyl]-N-(2,2-dimethylcyclopentyl)isoxazole-3-carboxamide (mixture of stereoisomers) | Intermediate 17, rac-2-bromopropanamide | $^1$H NMR (400 MHz, DMSO-$d_6$) δ ppm = 0.83 (s, 3 H), 0.96 (s, 3 H), 1.20 (d, *J*=7.35 Hz, 3 H), 1.38 - 1.59 (m, 3 H), 1.61 - 1.73 (m, 2 H), 1.83 - 1.97 (m, 1 H), 4.00 (q, *J*=8.28 Hz, 1 H), 5.04 - 5.18 (m, 1 H), 7.10 (s, 1 H), 7.30 (s, 1 H),<br><br>7.40 (t, *J*=8.87 Hz, 2 H), 7.63 (br s, 1 H), 7.71 (dd, *J*=8.74, 5.20 Hz, 2 H), 8.37 (d, *J*=9.12 Hz, 1 H), 8.53 (br s, 1 H), 8.67 (br s, 1 H) Biotage (method X)<br>LC-MS (method 1) Rt = 1.21 min MS (ESIpos): m/z = 515.4 [M+H]$^+$ 65 % yield |
| **38** | 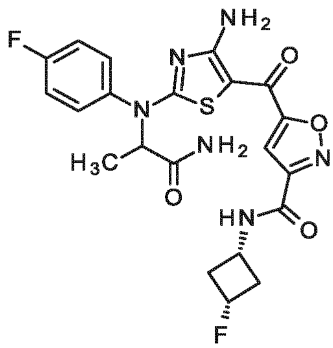 5-[4-amino-2-(N-(2-amino-1-methyl-2-oxo-ethyl)-4-fluoro-anilino)thiazole-5-carbonyl]-N-(cis-3-fluorocyclobutyl) isoxazole-3-carboxamide (mixture of stereoisomers) | Intermediate 24, rac-2-bromopropanamide | $^1$H NMR (400 MHz, DMSO-$d_6$) δ ppm = 0.72 - 0.88 (m, 2H), 1.20 (d, *J*=7.35 Hz, 3 H), 2.22 - 2.31 (m, 1 H), 2.68 - 2.73 (m, 1 H), 3.87 - 4.00 (m, 1 H), 4.72 - 4.95 (m, 1 H), 5.05 - 5.19 (m, 1 H), 7.07 (s, 1 H), 7.30 (s, 1 H), 7.40 (t, *J*=8.74 Hz, 2 H), 7.63 (br s, 1 H), 7.70 (dd, *J*=8.74, 5.20 Hz, 2 H), 8.54 (br s, 1 H), 8.67 (br s, 1 H), 9.13 (d, *J*=7.60 Hz, 1 H) RP-HPLC (method C)<br><br>LC-MS (method 1) Rt = 1.00 min MS (ESIpos): m/z = 491.4 [M+H]$^+$ 13 % yield |

(continued)

| Example number | Chemical structure Compound name | Starting materials | Analytics/purification/yield |
|---|---|---|---|
| **39** | 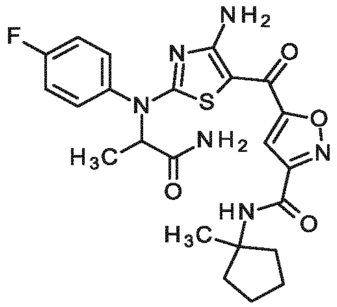 rac-5-[4-amino-2-(N-(2-amino-1-methyl-2-oxoethyl)-4-fluoro-anilino)thiazole-5-carbonyl]-N-(1-methylcyclopentyl)isoxazol e-3-carboxamide | Intermediate 15, rac-2-bromopropanamide | $^1$H NMR (400 MHz, DMSO-$d_6$) δ ppm = 1.19 (d, *J*=7.35 Hz, 3 H), 1.38 (s, 3 H), 1.50 - 1.69 (m, 6 H), 2.03 - 2.12 (m, 2 H), 5.05 - 5.17 (m, 1 H), 7.06 (s, 1 H), 7.30 (br s, 1 H), 7.40 (t, *J*=8.74 Hz, 2 H), 7.63 (br s, 1 H), 7.70 (dd, *J*=8.62, 5.07 Hz, 2 H), 8.23 (s, 1 H), 8.52 (br s, 1 H), 8.68 (br s, 1 H) Biotage (method X)<br><br>LC-MS (method 1) Rt = 1.16 min MS (ESIpos): m/z = 501.4 [M+H]$^+$ 28 % yield |
| **40** | 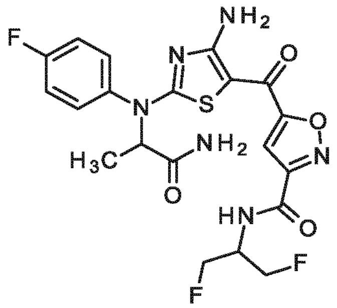 rac-5-[4-amino-2-(N-(2-amino-1-methyl-2-oxoethyl)-4-fluoro-anilino)thiazole-5-carbonyl]-N-[2-fluoro-1-(fluoromethyl)ethyl]isoxazo le-3-carboxamide | Intermediate 21, rac-2-bromopropanamide | $^1$H NMR (400 MHz, DMSO-$d_6$) δ ppm = 1.20 (d, *J*=7.35 Hz, 3 H), 4.40 - 4.57 (m, 3 H), 4.59 - 4.65 (m, 2 H), 5.05 - 5.18 (m, 1 H), 7.12 (s, 1 H), 7.30 (s, 1 H), 7.40 (t, *J*=8.74 Hz, 2 H), 7.63 (br s, 1 H), 7.71 (dd, *J*=8.87, 5.07 Hz, 2 H), 8.55 (br s, 1 H), 8.65 (br s, 1 H), 9.11 (d, *J*=7.86 Hz, 1 H) RP-HPLC (method C basic) LC-MS (method 2) Rt = 0.95 min MS (ESIpos): m/z = 497.4 [M+H]$^+$ 36 % yield |
| **41** | 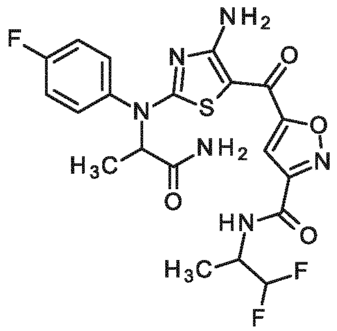 5-[4-amino-2-(N-(2-amino-1-methyl-2-oxo-ethyl)-4-fluoro-anilino)thiazole-5-carbonyl]-N-(2,2-difluoro-1-methyl-ethyl)isoxazole-3-carboxamide (mixture of stereoisomers) | Intermediate 23, rac-2-bromopropanamide | $^1$H NMR (400 MHz, DMSO-$d_6$) δ ppm = 1.20 (m, *J*=7.22, 1.39 Hz, 6 H), 4.26 - 4.41 (m, 1 H), 5.05 - 5.18 (m, 1 H), 5.96 (td, *J*=1.00 Hz, 1 H), 7.12 (s, 1 H), 7.28 - 7.32 (m, 1 H), 7.36 - 7.43 (m, 2 H), 7.63 (br s, 1 H), 7.71 (dd, *J*=8.87, 5.07 Hz, 2 H), 8.55 (br s, 1 H), 8.67 (br s, 1 H), 9.00 - 9.06 (m, 1 H)<br><br>RP-HPLC (method C basic) LC-MS (method 2) Rt = 1.00 min MS (ESIpos): m/z = 497.4 [M+H]$^+$ 74 % yield |

(continued)

| Example number | Chemical structure Compound name | Starting materials | Analytics/purification/yield |
|---|---|---|---|
| **42** | 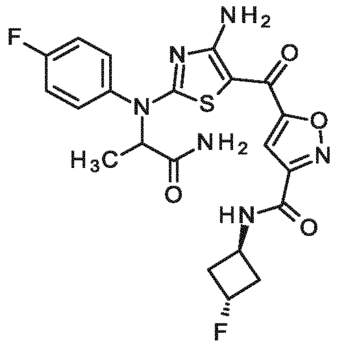 5-[4-amino-2-(N-(2-amino-1-methyl-2-oxo-ethyl)-4-fluoro-anilino)thiazole-5-carbonyl]-N-(trans-3-fluorocyclobutyl) isoxazole-3-carboxamide (mixture of stereoisomers) | Intermediate 25, rac-2-bromopropanamide | $^1H$ NMR (400 MHz, DMSO-$d_6$) δ ppm = 1.20 (d, $J$=7.35 Hz, 3 H), 2.39 - 2.47 (m, 2 H), 4.45 - 4.55 (m, 1 H), 5.04 - 5.14 (m, 1 H), 5.15 - 5.36 (m, 1 H), 7.06 (s, 1 H), 7.30 (s, 1 H), 7.40 (t, $J$=8.87 Hz, 2 H), 7.63 (br s, 1 H), 7.70 (dd, $J$=8.74, 5.20 Hz, 2 H), 8.53 (br s, 1 H), 8.67 (br s, 1 H), 9.16 (d, $J$=7.10 Hz, 1 H) RP-HPLC (method C basic) LC-MS (method 2) Rt = 1.01 min MS (ESI-pos): m/z = 491.4 $[M+H]^+$ 13 % yield |
| **43** | 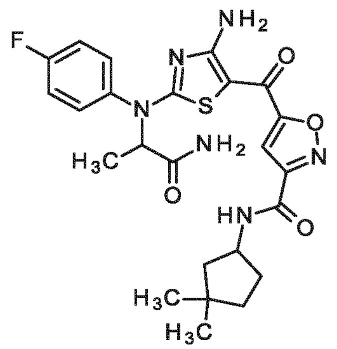 5-[4-amino-2-(N-(2-amino-1-methyl-2-oxo-ethyl)-4-fluoro-anilino)thiazole-5-carbonyl]-N-(3,3-dimethylcyclopentyl)isoxaz ole-3-carboxamide (mixture of stereoisomers) | Intermediate 16, rac-2-bromopropanamide | $^1H$ NMR (400 MHz, DMSO-$d_6$) δ ppm = 0.96 (s, 3 H), 1.04 (s, 3 H), 1.20 (d, $J$=7.60 Hz, 3 H), 1.31 - 1.44 (m, 2 H), 1.47 - 1.56 (m, 1 H), 1.58 - 1.69 (m, 1 H), 1.70 - 1.78 (m, 1 H), 1.94 - 2.05 (m, 1 H), 4.23 - 4.36 (m, 1 H), 5.04 - 5.19 (m, 1 H), 7.07 (s, 1 H), 7.30 (s, 1 H), 7.40 (t, $J$=8.74 Hz, 2 H), 7.63 (br s, 1 H), 7.70 (dd, J=8.62, 5.07 Hz, 2 H), 8.54 (br s, 1 H), 8.66 (br s, 1 H), 8.74 (d, $J$=7.60 Hz, 1 H) Biotage (method X) LC-MS (method 2) Rt = 1.01 min MS (ESIpos): m/z = 515.4 $[M+H]^+$ 100 % yield |
| **44** | 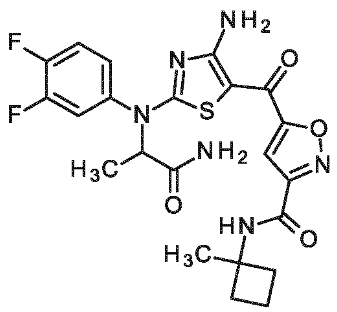 rac-5-[4-amino-2-(N-(2-amino-1-methyl-2-oxoethyl)-3,4-difluoro-anilino)thiazole-5-carbonyl]-N-(1-methylcyclobutyl)isoxazole -3-carboxamide | Intermediate 29, rac-2-bromopropanamide | $^1H$ NMR (400 MHz, DMSO-$d_6$) δ ppm = 1.22 (d, $J$=7.35 Hz, 3 H), 1.44 (s, 3 H), 1.73 - 1.85 (m, 2 H), 1.90 - 2.01 (m, 2 H), 2.27 - 2.38 (m, 2 H), 5.02 - 5.11 (m, 1 H), 7.09 (s, 1 H), 7.34 (s, 1 H), 7.51 - 7.71 (m, 3 H), 7.81 - 7.88 (m, 1 H), 8.57 (br s, 1 H), 8.64 (br s, 1 H), 8.77 (s, 1 H) Biotage (method X) LC-MS (method 2) Rt = 1.13 min MS (ESIpos): m/z = 505.4 $[M+H]^+$ 31 % yield |

(continued)

| Example number | Chemical structure Compound name | Starting materials | Analytics/purification/yield |
|---|---|---|---|
| **44.1** and **44.2** | (R)-5-[4-amino-2-(N-(2-amino-1-methyl-2-oxoethyl)-3,4-difluoro-anilino)thiazole-5-carbonyl]-N-(1-methylcyclobutyl)isoxazole -3-carboxamide and (S)-5-[4-amino-2-(N-(2-amino-1-methyl-2-oxo-ethyl)-3,4-difluoro-anilino)thiazole-5-carbonyl]-N-(1-methylcyclobutyl)isoxazole -3-carboxamide | | |
| **44.1** | 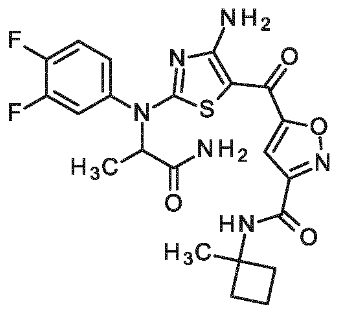 5-[4-amino-2-(N-(2-amino-1-methyl-2-oxo-ethyl)-3,4-difluoro-anilino)thiazole-5-carbonyl]-N-(1-methylcyclobutyl)isoxazole -3-carboxamide (enantiomer 1) | Example 44 | $^1$H NMR (400 MHz, DMSO-$d_6$) δ ppm = 1.22 (d, *J*=7.35 Hz, 3 H), 1.44 (s, 3 H), 1.73 - 1.85 (m, 2 H), 1.90 - 2.01 (m, 2 H), 2.27 - 2.38 (m, 2 H), 5.02 - 5.11 (m, 1 H), 7.09 (s, 1 H), 7.34 (s, 1 H), 7.51 - 7.71 (m, 3 H), 7.81 - 7.88 (m, 1 H), 8.57 (br s, 1 H), 8.64 (br s, 1 H), 8.77 (s, 1 H)<br><br>LC-MS (method 2) Rt = 1.13 min MS (ESIpos): m/z = 505.4 [M+H]$^+$ 34 % yield |

Chiral HPLC Example 44.1

HPLC separation of rac-5-[4-amino-2-(N-(2-amino-1-methyl-2-oxo-ethyl)-3,4-difluoro-anilino)thiazole-5-carbonyl]-N-(1-methylcyclobutyl)isoxazole-3-carboxamide (200 mg, 0.39 mmol, Example 44) on a chiral column gave 98 mg (34 % yield) of 5-[4-amino-2-(N-(2-amino-1-methyl-2-oxo-ethyl)-3,4-difluoro-anilino)thiazole-5-carbonyl]-N-(1-methylcyclobutyl)isoxazole-3-carboxamide, enantiomer 1.

Preparative chiral HPLC

Instrument: PrepCon Labomatic HPLC-3; Column: YMC Cellulose SC 5μ, 250x30; eluent A: hexane + 0.1 vol % diethylamine; eluent B: ethanol + 0.1 vol % diethylamine; isocratic: 60%A+40%B; flow: 50 mL/min; temperature: 25°C; UV: 254 nm

Analytical chiral HPLC: Rt = 2.81 min

Instrument: Thermo Fisher UltiMate 3000; Column: YMC Cellulose SC 3μ, 100x4.6; eluent A: hexane + 0.1 vol % diethylamine; eluent B: ethanol; isocratic: 60%A+40%B; flow: 1.4 mL/min; temperature: 25°C; UV: 254 nm

(continued)

| Example number | Chemical structure Compound name | Starting materials | Analytics/purification/yield |
|---|---|---|---|
| **44.2** | 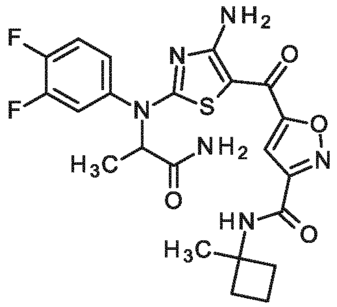 5-[4-amino-2-(N-(2-amino-1-methyl-2-oxo-ethyl)-3,4-difluoro-anilino)thiazole-5-carbonyl]-N-(1-methylcyclobutyl)isoxazole -3-carboxamide (enantiomer 2) | Example 44 | $^1$H NMR (400 MHz, DMSO-$d_6$) δ ppm = 1.22 (d, *J*=7.35 Hz, 3 H), 1.44 (s, 3 H), 1.73 - 1.85 (m, 2 H), 1.90 - 2.01 (m, 2 H), 2.27 - 2.38 (m, 2 H), 5.02 - 5.11 (m, 1 H), 7.09 (s, 1 H), 7.34 (s, 1 H), 7.51 - 7.71 (m, 3 H), 7.81 - 7.88 (m, 1 H), 8.57 (br s, 1 H), 8.64 (br s, 1 H), 8.77 (s, 1 H) LC-MS (method 2) Rt = 1.13 min MS (ESIpos): m/z = 505.4 [M+H]$^+$ 33 % yield |
| Chiral HPLC Example 44.2 HPLC separation of rac-5-[4-amino-2-(N-(2-amino-1-methyl-2-oxo-ethyl)-3,4-difluoro-anilino)thiazole-5-carbonyl]-N-(1-methylcyclobutyl)isoxazole-3-carboxamide (200 mg, 0.39 mmol, Example 44) on a chiral column gave 98 mg (33 % yield) of 5-[4-amino-2-(N-(2-amino-1-methyl-2-oxo-ethyl)-3,4-difluoro-anilino)thiazole-5-carbonyl]-N-(1-methylcyclobutyl)isoxazole-3-carboxamide, enantiomer 2. <br> Preparative chiral HPLC <br> Instrument: PrepCon Labomatic HPLC-3; Column: YMC Cellulose SC 5μ, 250x30; eluent A: hexane + 0.1 vol % diethylamine; eluent B: ethanol + 0.1 vol % diethylamine; isocratic: 60%A+40%B; flow: 50 mL/min; temperature: 25°C; UV: 254 nm <br> Analytical chiral HPLC: Rt = 3.81 min <br> Instrument: Thermo Fisher UltiMate 3000; Column: YMC Cellulose SC 3μ, 100x4.6; eluent A: hexane + 0.1 vol % diethylamine; eluent B: ethanol; isocratic: 60%A+40%B; flow: 1.4 mL/min; temperature: 25°C; UV: 254 nm | | | |
| **45** | 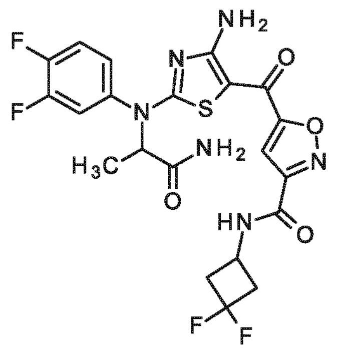 rac-5-[4-amino-2-(N-(2-amino-1-methyl-2-oxoethyl)-3,4-difluoro-anilino)thiazole-5-carbonyl]-N-(3,3-difluorocyclobutyl) isoxazol e-3-carboxamide | Intermediate 30, rac-2-bromopropanamide | $^1$H NMR (400 MHz, DMSO-$d_6$) δ ppm = 1.22 (d, *J*=7.35 Hz, 3 H), 2.70 - 2.83 (m, 2 H), 2.87 - 3.00 (m, 2 H), 4.17 - 4.28 (m, 1 H), 5.01 - 5.14 (m, 1 H), 7.11 (s, 1 H), 7.34 (s, 1 H), 7.54 - 7.60 (m, 1 H), 7.61 - 7.70 (m, 2 H), 7.80 - 7.89 (m, 1 H), 8.57 (br s, 1 H), 8.66 (br s, 1 H), 9.30 (d, *J*=6.84 Hz, 1 H) Biotage (method X) LC-MS (method 2) Rt = 1.05 min MS (ESIpos): m/z = 527.3 [M+H]$^+$ 52 % yield |

(continued)

| Example number | Chemical structure Compound name | Starting materials | Analytics/purification/yield |
|---|---|---|---|
| **45.1** and **45.2** | (R)-5-[4-amino-2-(N-(2-amino-1-methyl-2-oxoethyl)-3,4-difluoro-anilino)thiazole-5-carbonyl]-N-(3,3-difluorocyclobutyl) isoxazol e-3-carboxamide and (S)-5-[4-amino-2-(N-(2-amino-1-methyl-2-oxo-ethyl)-3,4-difluoro-anilino)thiazole-5-carbonyl]-N-(3,3-difluorocyclobutyl) isoxazol e-3-carboxamide | | |
| **45.1** | 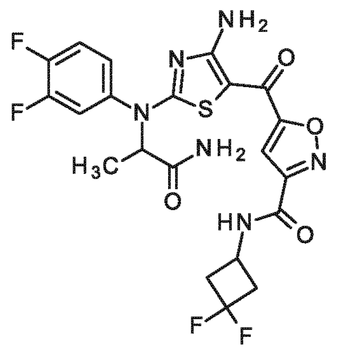 5-[4-amino-2-(N-(2-amino-1-methyl-2-oxo-ethyl)-3,4-difluoro-anilino)thiazole-5-carbonyl]-N-(3,3-difluorocyclobutyl) isoxazol e-3-carboxamide (enantiomer 1) | Example 45 | $^1$H NMR (400 MHz, DMSO-$d_6$) δ ppm = 1.22 (d, *J*=7.35 Hz, 3 H), 2.70 - 2.83 (m, 2 H), 2.87 - 3.00 (m, 2 H), 4.17 - 4.28 (m, 1 H), 5.01 - 5.14 (m, 1 H), 7.11 (s, 1 H), 7.34 (s, 1 H), 7.54 - 7.60 (m, 1 H), 7.61 - 7.70 (m, 2 H), 7.80 - 7.89 (m, 1 H), 8.57 (br s, 1 H), 8.66 (br s, 1 H), 9.30 (d, J=6.84 Hz, 1 H) LC-MS (method 2) Rt = 1.05 min MS (ESIpos): m/z = 527.3 $[M+H]^+$ 42 % yield |

Chiral HPLC Example 45.1

HPLC separation of rac-5-[4-amino-2-(N-(2-amino-1-methyl-2-oxo-ethyl)-3,4-difluoro-anilino)thiazole-5-carbonyl]-N-(3,3-difluorocyclobutyl)isoxazole-3-carboxamide (137 mg, 0.26 mmol, Example 45) on a chiral column gave 59 mg (42 % yield) of 5-[4-amino-2-(N-(2-amino-1-methyl-2-oxo-ethyl)-3,4-difluoro-anilino)thiazole-5-carbonyl]-N-(3,3-difluorocyclobutyl)isoxazole-3-carboxamide, enantiomer 1.

Preparative chiral HPLC

Instrument: PrepCon Labomatic HPLC-3; Column: YMC Amylose SA 5μ, 250x30; eluent A: methyl tert-butyl ether + 0.1 vol % diethylamine; eluent B: ethanol + 0.1 vol % diethylamine; isocratic: 95%A+5%B; flow: 50 mL/min; temperature: 25°C; UV: 280 nm

Analytical chiral HPLC: Rt = 3.17 min

Instrument: Thermo Fisher UltiMate 3000; Column: YMC Amylose SA 3μ, 100x4.6; eluent A: methyl tert-butyl ether + 0.1 vol % diethylamine; eluent B: ethanol; isocratic: 95%A+5%B; flow: 1.4 mL/min; temperature: 25°C; UV: 280 nm

(continued)

| Example number | Chemical structure Compound name | Starting materials | Analytics/purification/yield |
|---|---|---|---|
| **45.2** | 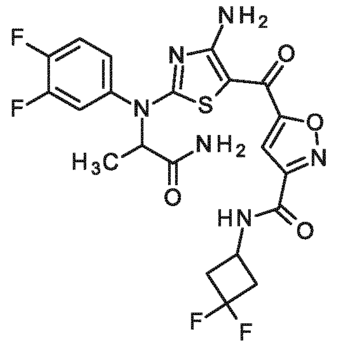<br>5-[4-amino-2-(N-(2-amino-1-methyl-2-oxo-ethyl)-3,4-difluoro-anilino)thiazole-5-carbonyl]-N-(3,3-difluorocyclobutyl) isoxazol e-3-carboxamide (enantiomer 2) | Example 45 | $^1$H NMR (400 MHz, DMSO-$d_6$) δ ppm = 1.22 (d, *J*=7.35 Hz, 3 H), 2.70 - 2.83 (m, 2 H), 2.87 - 3.00 (m, 2 H), 4.17 - 4.28 (m, 1 H), 5.01 - 5.14 (m, 1 H), 7.11 (s, 1 H), 7.34 (s, 1 H), 7.54 - 7.60 (m, 1 H), 7.61 - 7.70 (m, 2 H), 7.80 - 7.89 (m, 1 H), 8.57 (br s, 1 H), 8.66 (br s, 1 H), 9.30 (d, *J*=6.84 Hz, 1 H) LC-MS (method 2) Rt = 1.05 min MS (ESIpos): m/z = 527.3 [M+H]$^+$ 52 % yield |
| Chiral HPLC Example 45.2<br>HPLC separation of rac-5-[4-amino-2-(N-(2-amino-1-methyl-2-oxo-ethyl)-3,4-difluoro-anilino)thiazole-5-carbonyl]-N-(3,3-difluorocyclobutyl)isoxazole-3-carboxamide (137 mg, 0.26 mmol, Example 45) on a chiral column gave 63 mg (46 % yield) of 5-[4-amino-2-(N-(2-amino-1-methyl-2-oxo-ethyl)-3,4-difluoro-anilino)thiazole-5-carbonyl]-N-(3,3-difluorocyclobutyl)isoxazole-3-carboxamide, enantiomer 2.<br><u>Preparative chiral HPLC</u><br>Instrument: PrepCon Labomatic HPLC-3; Column: YMC Amylose SA 5μ, 250x30; eluent A: methyl tert-butyl ether + 0.1 vol % diethylamine; eluent B: ethanol + 0.1 vol % diethylamine; isocratic: 95%A+5%B; flow: 50 mL/min; temperature: 25°C; UV: 280 nm<br><u>Analytical chiral HPLC</u>: Rt = 4.60 min<br>Instrument: Thermo Fisher UltiMate 3000; Column: YMC Amylose SA 3μ, 100x4.6; eluent A: methyl tert-butyl ether + 0.1 vol % diethylamine; eluent B: ethanol; isocratic: 95%A+5%B; flow: 1.4 mL/min; temperature: 25°C; UV: 280 nm | | | |
| **46** | 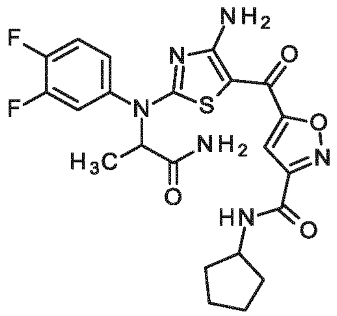<br>rac-5-[4-amino-2-(N-(2-amino-1-methyl-2-oxoethyl)-3,4-difluoro-anilino)thiazole-5-carbonyl]-N-cyclopentyl-isoxazole-3-carboxamide | Intermediate 28, rac-2-bromopropanamide | $^1$H NMR (400 MHz, DMSO-$d_6$) δ ppm = 1.19 - 1.24 (m, 3 H), 1.46 - 1.57 (m, 4 H), 1.62 - 1.70 (m, 2 H), 1.80 - 1.92 (m, 2 H), 4.12 - 4.23 (m, 1 H), 5.00 - 5.13 (m, 1 H), 7.11 (s, 1 H), 7.30 - 7.38 (m, 1 H), 7.54 - 7.60 (m, 1 H), 7.62 - 7.71 (m, 2 H), 7.79 - 7.88 (m, 1 H), 8.56 (br s, 1 H), 8.64 (br s, 1 H), 8.71 (d, *J*=7.35 Hz, 1 H) Biotage (method X)<br>LC-MS (method 2) Rt = 1.13 min MS (ESIpos): m/z = 505.4 [M+H]$^+$ 36 % yield |

(continued)

| Example number | Chemical structure Compound name | Starting materials | Analytics/purification/yield |
|---|---|---|---|
| **46.1** and **46.2** | (R)-5-[4-amino-2-(N-(2-amino-1-methyl-2-oxoethyl)-3,4-difluoro-anilino)thiazole-5-carbonyl]-N-cyclopentyl-isoxazole-3-carboxamide and (S)- 5-[4-amino-2-(N-(2-amino-1-methyl-2-oxoethyl)-3,4-difluoro-anilino)thiazole-5-carbonyl]-N-cyclopentyl-isoxazole-3-carboxamide | | |
| **46.1** | 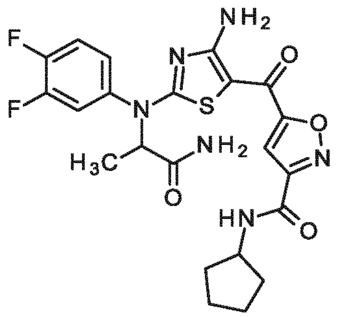 5-[4-amino-2-(N-(2-amino-1-methyl-2-oxo-ethyl)-3,4-difluoro-anilino)thiazole-5-carbonyl]-N-cyclopentyl-isoxazole-3-carboxamide (enantiomer 1) | Example 46 | $^1$H NMR (400 MHz, DMSO-$d_6$) δ ppm = 1.19 - 1.24 (m, 3 H), 1.46 - 1.57 (m, 4 H), 1.62 - 1.70 (m, 2 H), 1.80 - 1.92 (m, 2 H), 4.12 - 4.23 (m, 1 H), 5.00 - 5.13 (m, 1 H), 7.11 (s, 1 H), 7.30 - 7.38 (m, 1 H), 7.54 - 7.60 (m, 1 H), 7.62 - 7.71 (m, 2 H), 7.79 - 7.88 (m, 1 H), 8.56 (br s, 1 H), 8.64 (br s, 1 H), 8.71 (d, *J*=7.35 Hz, 1 H) LC-MS (method 2) Rt = 1.13 min MS (ESIpos): m/z = 505.4 $[M+H]^+$ 45 % yield |
| Chiral HPLC Example 46.1<br>HPLC separation of rac-5-[4-amino-2-(N-(2-amino-1-methyl-2-oxo-ethyl)-3,4-difluoro-anilino)thiazole-5-carbonyl]-N-cyclopentyl-isoxazole-3-carboxamide (48 mg, 0.10 mmol, Example 46) on a chiral column gave 22 mg (45 % yield) of 5-[4-amino-2-(N-(2-amino-1-methyl-2-oxoethyl)-3,4-difluoro-anilino)thiazole-5-carbonyl]-N-cyclopentyl-isoxazole-3-carboxamide, enantiomer 1.<br>Preparative chiral HPLC<br>Instrument: PrepCon Labomatic HPLC-4; Column: YMC Amylose SA 10μ, 250x50; eluent A: methyl tert-butyl ether + 0.1 vol % diethylamine; eluent B: acetonitrile; isocratic: 90%A+10%B; flow: 100 mL/min; temperature: 25°C; UV: 254 nm<br>Analytical chiral HPLC: Rt = 2.61 min<br>Instrument: Thermo Fisher UltiMate 3000; Column: YMC Amylose SA 3μ, 100x4.6; eluent A: methyl tert-butyl ether + 0.1 vol % diethylamine; eluent B: acetonitrile; isocratic: 90%A+10%B; flow: 1.4 mL/min; temperature: 25°C; UV: 254 nm | | | |
| **46.2** | 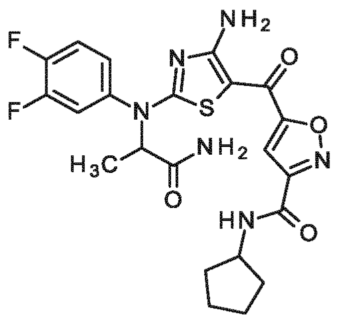 | Example 46 | $^1$H NMR (400 MHz, DMSO-$d_6$) δ ppm = 1.19 - 1.24 (m, 3 H), 1.46 - 1.57 (m, 4 H), 1.62 - 1.70 (m, 2 H), 1.80 - 1.92 (m, 2 H), 4.12 - 4.23 (m, 1 H), 5.00 - 5.13 (m, 1 H), 7.11 (s, 1 H), 7.30 - 7.38 (m, 1 H), 7.54 - 7.60 (m, 1 H), 7.62 - |

(continued)

| Example number | Chemical structure Compound name | Starting materials | Analytics/purification/yield |
|---|---|---|---|
| | 5-[4-amino-2-(N-(2-amino-1-methyl-2-oxo-ethyl)-3,4-difluoro-anilino)thiazole-5-carbonyl]-N-cyclopentyl-isoxazole-3-carboxamide (enantiomer 2) | | 7.71 (m, 2 H), 7.79 - 7.88 (m, 1 H), 8.56 (br s, 1 H), 8.64 (br s, 1 H), 8.71 (d, *J*=7.35 Hz, 1 H) LC-MS (method 2) Rt = 1.13 min MS (ESIpos): m/z = 505.4 $[M+H]^+$ 45 % yield |
| Chiral HPLC Example 46.2<br>HPLC separation of rac-5-[4-amino-2-(N-(2-amino-1-methyl-2-oxo-ethyl)-3,4-difluoro-anilino)thiazole-5-carbonyl]-N-cyclopentyl-isoxazole-3-carboxamide (48 mg, 0.1 mmol, Example 46) on a chiral column gave 22 mg (45 % yield) of 5-[4-amino-2-(N-(2-amino-1-methyl-2-oxo-ethyl)-3,4-difluoro-anilino)thiazole-5-carbonyl]-N-cyclopentyl-isoxazole-3-carboxamide, enantiomer 2.<br>Preparative chiral HPLC<br>Instrument: PrepCon Labomatic HPLC-4; Column: YMC Amylose SA 10μ, 250x50; eluent A: methyl tert-butyl ether + 0.1 vol % diethylamine; eluent B: acetonitrile; isocratic: 90%A+10%B; flow: 100 mL/min; temperature: 25°C; UV: 254 nm<br>Analytical chiral HPLC: Rt = 3.49 min<br>Instrument: Thermo Fisher UltiMate 3000; Column: YMC Amylose SA 3μ, 100x4.6; eluent A: methyl tert-butyl ether + 0.1 vol % diethylamine; eluent B: acetonitrile; isocratic: 90%A+10%B; flow: 1.4 mL/min; temperature: 25°C; UV: 254 nm | | | |
| **47** | 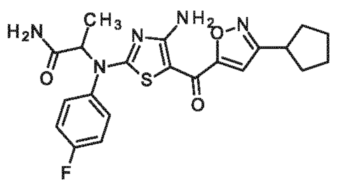<br>rac-2-(N-[4-amino-5-(3-cyclopentylisoxazole-5-carbonyl)thiazol-2-yl]-4-fluoro-anilino)propanamide | Intermediate 45, rac-2-bromopropanamide | $^1H$ NMR (400 MHz, DMSO-$d_6$) δ ppm = 8.76-8.39 (m, 2H), 7.73-7.68 (m, 2H), 7.46 (s, 1H), 7.40 (t, J = 8.8 Hz, 2H), 7.28 (s, 1 H), 6.85 (s, 1H), 5.15-5.05 (m, 1H), 3.12-3.07 (m, 1H), 2.00-1.91 (m, 2H), 1.70-1.55 (m, 6H), 1.19 (d, J = 7.6 Hz, 3H)<br>RP-HPLC [Instrument: ACSWH-GX-k; Column: Phenomenex Gemini-NX C18 75* 30 mm* 3 μm); eluent A: water (0.225% formic acid v/v), eluent B: acetonitrile; gradient: 0-10 minutes 52-62% B; flow 25 mL/minute; temperature: RT; Detector: UV 220/254 nm LC-MS (method 4): Rt=0.936 min MS (ESIpos): m/z = 444.1 [M+H]+. 67% yield |
| **48** | 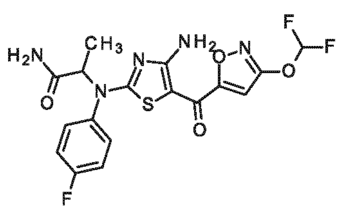<br>rac-2-(N-[4-amino-5-[3-(difluoromethoxy)isoxazole -5-carbonyl] thiazol-2-yl]-4-fluoro-anilino)propanamide | Intermediate 39, rac-2-bromopropanamide | $^1H$ NMR (400 MHz, DMSO-$d_6$) δ ppm = 8.71-8.50 (m, 2H), 7.73-7.46 (m, 4H), 7.41-7.32 (m, 2H), 7.29 (s, 1H), 6.98 (s, 1H), 5.15-5.05 (m, 1H), 1.19 (d, J = 7.6 Hz, 3H)<br>LC-MS (method 4): Rt = 0.865 min; MS (ESIpos): m/z = 441.8 [M+H]+<br>RP-HPLC (Instrument: ACSWH-GX-G; Column: Unisil 3-100 C18 Ultra 150* 50 mm* 3 μm; eluent A: water (0.225% formic acid in water), eluent B: acetonitrile; gradient: 0-10 minutes 30-60% B; flow 25 mL/minute; temperature: RT; Detector: UV 220/254 nm 48 % yield |

(continued)

| Example number | Chemical structure Compound name | Starting materials | Analytics/purification/yield |
|---|---|---|---|
| **49** | 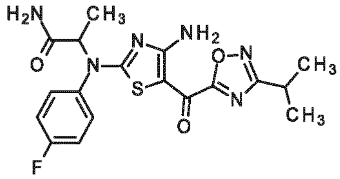 rac-2-(N-[4-amino-5-(3-isopropyl-1,2,4-oxadiazole-5-carbonyl)thiazol-2-yl]-4-fluoro-anilino)propanamide | Intermediate 32, rac-2-bromopropanamide | $^{1}H$ NMR (400 MHz, DMSO-$d_6$) δ ppm = 1.13 - 1.25 (m, 9 H), 2.96 - 3.10 (m, 1 H), 5.01 - 5.21 (m, 1 H), 7.27 - 7.32 (m, 1 H), 7.39 (t, *J*=8.74 Hz, 2 H), 7.63 (br s, 1 H), 7.69 (br dd, *J*=8.49, 4.94 Hz, 2 H), 8.68 - 8.79 (m, 2 H) RP-HPLC (method C basic) LC-MS (method 2) Rt = 1.08 min MS (ESI-pos): m/z = 419.4 $[M+H]^+$ 55 % yield |
| **50** | 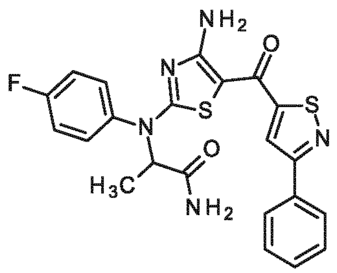 rac-2-(N-[4-amino-5-(3-phenylisothiazole-5-carbonyl)thiazol-2-yl]-4-fluoro-anilino)propanamide | Intermediate 82, rac-2-bromopropanamide | $^{1}H$ NMR (500 MHz, DMSO-$d_6$) δ ppm = 8.57 (br s, 1 H) 8.42 (br s, 1 H) 7.90 - 7.99 (m, 3 H) 7.69 (dd, *J*=8.67, 5.20 Hz, 2 H) 7.64 (br s, 1 H) 7.43 - 7.52 (m, 3 H) 7.37 (t, *J*=8.67 Hz, 2 H) 7.31 (br s, 1 H) 5.07 - 5.11 (br s, 1 H) 1.19 (d, *J*=7.25 Hz, 3 H). Biotage (method Y) LC-MS (method 2): Rt = 1.27 min; MS(ESIpos) m/z = 468.5 $[M+H]^+$. 19 % yield |

## Example 51

rac-2-(N-[4-amino-5-(3-phenyl-1,2,4-oxadiazole-5-carbonyl)thiazol-2-yl]-4-fluoro-anilino) propanamide

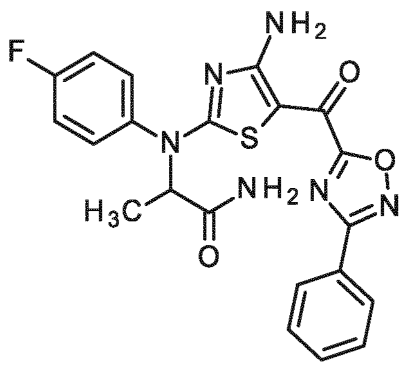

To a mixture of ammonia in methanol (1.0 mL, ammonia gas was bubbled into methanol at -78 °C for 10 minutes) was added ethyl 2-(N-[4-chloro-5-(3-phenyl-1,2,4-oxadiazole-5-carbonyl)thiazol-2-yl]-4-fluoro-anilino)propanoate (Intermediate 51, 80.0 mg, 0.160 mmol) at 25 °C. The mixture was stirred at 25 °C for 16 hours. The mixture was concentrated under vacuum to give a residue. The residue was purified by preparative HPLC [Instrument: GX-c; Column: Phenomenex luna C18 150* 25 mm* 10 μm); eluent A: water (0.225% formic acid v/v), eluent B: acetonitrile; gradient: 0-10 minutes 38~68% B; flow 25 mL/minute; temperature: RT; Detector: UV 220/254 nm] and preparative thin layer chromatography (dichloromethane: methanol = 20: 1) to give the title compound (3.70 mg, 5% yield) as a yellow solid.

LC-MS (method 4): Rt = 0.823 min; MS (ESIpos): m/z = 453.2 $[M+H]^+$.

$^{1}H$ NMR (400 MHz, DMSO-$d_6$) δ ppm = 8.81 (s, 2H), 7.92-7,82 (m, 2H), 7.75-7.69 (m, 2H), 7.63-7.58 (m, 1H), 7.56-7.52 (m, 2H), 7.42 (t, J = 8.0 Hz, 2H), 7.31 (s, 1H), 5.21-5.04 (m, 1H), 1.21 (d, J = 7.6 Hz, 3H).

**Example 52**

rac-2-(N-[4-amino-5-(3-methyl-1,2,4-oxadiazole-5-carbonyl)thiazol-2-yl]-4-fluoro-anilino)propanamide

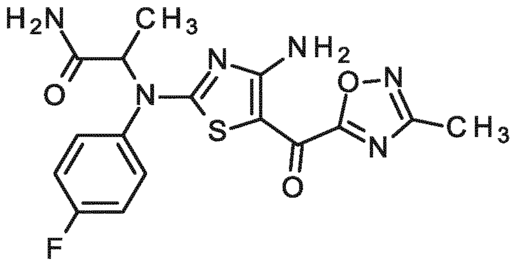

To a mixture of ammonia in methanol (2.0 mL, ammonia gas bubbled into methanol at -78 °C for 10 minutes) was added ethyl 2-(N-[4-chloro-5-(3-methyl-1,2,4-oxadiazole-5-carbonyl)thiazol-2-yl]-4-fluoro-anilino)propanoate (Intermediate 53, 70.0 mg, 0.160 mmol) at 25 °C. The mixture was stirred at 25 °C for 16 hours. The mixture was concentrated under vacuum to give a residue. The residue was purified by preparative HPLC [Instrument: ACS-WH-GX-L; Column: Unisil 3-100 C18 Ultra 150* 50 mm* 3 μm); eluent A: water (0.225% formic acid v/v), eluent B: acetonitrile; gradient: 0-10 minutes 24~54% B; flow 25 mL/minute; temperature: RT; Detector: UV 220/254 nm] to give the title compound (16.0 mg, 25% yield) as a yellow solid.

LC-MS (method 4): Rt = 0.837 min; MS (ESIpos): m/z = 391.0 $[M+H]^+$.

$^1H$ NMR (400 MHz, DMSO-$d_6$): δ ppm = 8.85-8.72 (m, 2H), 7.71-7.65 (m, 2H), 7.61 (s, 1H), 7.40 (t, *J* = 8.8 Hz, 2H), 7.28 (s, 1H), 5.14-5.11 (m, 1H), 2.07 (s, 3H), 1.17 (d, *J* = 7.6 Hz, 3H).

The following examples were prepared from the starting materials stated in Table 6, below, using the procedure as for Example 52.

**Table 6: Examples 53 - 59**

| Example number | Chemical structure Compound name | Starting materials | Analytics/purification/yield |
|---|---|---|---|
| **53** | 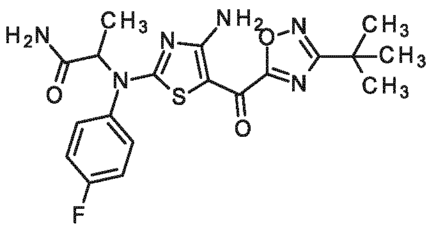 rac-2-(N-[4-amino-5-(3-tert-butyl-1,2,4-oxadiazole-5-carbonyl)thiazol-2-yl]-4-fluoro-anilino)propanamide | Intermediate 55, ammonia in methanol | $^1H$ NMR (400 MHz, DMSO-$d_6$): δ ppm = 8.71 (s, 2H), 7.75-7.55 (m, 3H), 7.40 (t, *J* = 8.4 Hz, 2H), 7.29 (s, 1H), 5.21-5.05 (m, 1H), 1.39-1.15 (m, 12H). LC-MS (method 4): Rt = 0.823 min; MS (ESIpos): m/z = 433.2 $[M+H]^+$ 25 % yield |
| **54** | 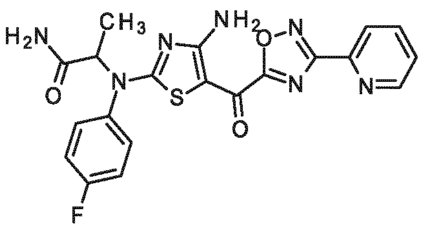 rac-2-(N-[4-amino-5-[3-(2-pyridyl)-1,2,4-oxadiazole-5-carbonyl]thiazol-2-yl]-4-fluoro-anilino)propanamide | Intermediate 57, ammonia in methanol | $^1H$ NMR (400 MHz, DMSO-$d_6$): δ ppm = 8.88-8.65 (m, 3H), 8.05-7.95 (m, 1H), 7.93-7.86 (m, 1H), 7.75-7.59 (m, 4H), 7.41 (t, *J* = 8.4 Hz, 2H), 7.31 (s, 1H), 5.21-5.08 (m, 1H), 1.19 (d, *J* = 7.2 Hz, 3H). LC-MS (method 4): Rt = 0.843 min; MS (ESIpos): m/z = 453.9 $[M+H]^+$ 3 % yield |

(continued)

| Example number | Chemical structure Compound name | Starting materials | Analytics/purification/yield |
|---|---|---|---|
| **55** | 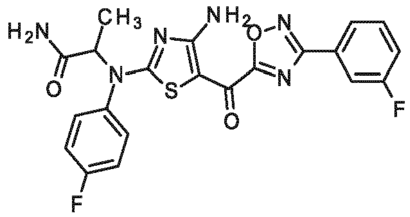 rac-2-(N-[4-amino-5-[3-(3-fluorophenyl)-1,2,4-oxadiazole-5-carbonyl]thiazol-2-yl]-4-fluoro-anilino)propanamide | Intermediate 59, ammonia in methanol | $^1$H NMR (400 MHz, DMSO-$d_6$): δ ppm = 8.80 (s, 2H), 7.78-7.71 (m, 3H), 7.70-7.45 (m, 4H), 7.43 (t, $J$ = 8.0 Hz, 2H), 7.32 (s, 1H), 5.19-5.10 (m, 1H), 1.22 (d, $J$ = 7.2 Hz, 3H). LC-MS (method 4): $R_t$ = 0.844 min; MS (ESIpos): m/z = 471.1 $[M+H]^+$ 4% yield |
| **56** | 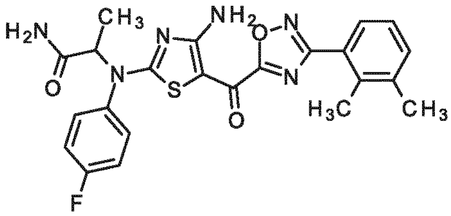 rac-2-(N-[4-amino-5-[3-(2,3-dimethylphenyl)-1,2,4-oxadiazole-5-carbonyl]thiazol-2-yl]-4-fluoro-anilino)propanamide | Intermediate 61, ammonia in methanol | $^1$H NMR (400 MHz, DMSO-$d_6$): δ ppm = 8.78-8.71 (m, 2H), 7.71-7.59 (m, 3H), 7.51-7.47 (m, 1H), 7.45-7.35 (m, 3H) 7.27-7.23 (m, 1H), 7.21 (t, $J$ = 7.6 Hz, 1H), 5.23-5.04 (m, 1H), 2.31 (s, 3H), 2.24 (s, 3H), 1.21 (d, $J$ = 7.2 Hz, 3H) LC-MS (method 4): $R_t$ = 0.873 min; MS (ESIpos): m/z = 481.2 $[M+H]^+$ 3 % yield |
| **57** | 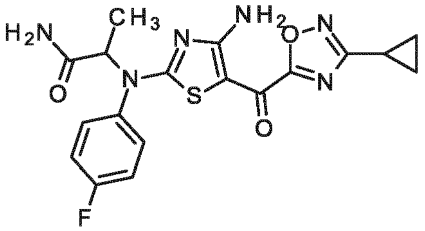 rac-2-(N-[4-amino-5-(3-cyclopropyl-1,2,4-oxadiazole-5-carbonyl)thiazol-2-yl]-4-fluoro-anilino)propanamide | Intermediate 63, ammonia in methanol | $^1$H NMR (400 MHz, DMSO-$d_6$): δ ppm = 8.68-8.65 (m, 2H), 7.71-7.66 (m, 2H), 7.59 (s, 1H), 7.39 (t, $J$ = 8.4 Hz, 2H), 7.24 (s, 1H), 5.11 (s, 1H), 2.13-2.05 (m, 1H), 1.19 (d, $J$ = 7.6 Hz, 3H), 1.07-1.03 (m, 2H), 0.77-0.65 (m, 2H). LC-MS (method 4): $R_t$ = 0.679 min; MS (ESIpos): m/z = 416.9 $[M+H]^+$. 9 % yield |
| **58** | 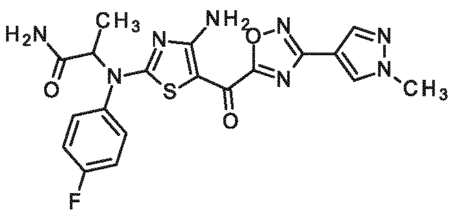 rac-2-(N-[4-amino-5-[3-(1-methylpyrazol-4-yl)-1,2,4-oxadiazole-5-carbonyl]thiazol-2-yl]-4-fluoro-anilino)propanamide | Intermediate 66, ammonia in methanol | $^1$H NMR (400 MHz, DMSO-$d_6$) δ ppm = 8.80-8.71 (m, 2H), 8.27 (s, 1H), 7.79-7,62 (m, 4H), 7.41 (t, $J$ = 8.4 Hz, 2H), 7.30 (s, 1H), 5.20-5.08 (m, 1H), 3.91 (s, 3H), 1.20 (d, $J$ = 7.2 Hz, 3H). LC-MS (method 4): $R_t$ = 0.736 min; MS (ESIpos): m/z = 457.2 $[M+H]^+$. 6 % yield |
| **59** | 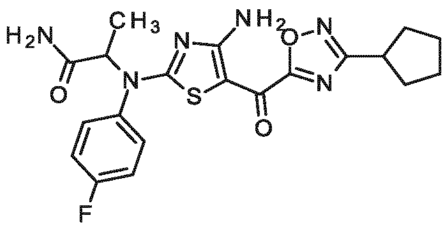 rac-2-(N-[4-amino-5-(3-cyclopentyl-1,2,4-oxadiazole-5-carbonyl)thiazol-2-yl]-4-fluoro-anilino)propanamide | Intermediate 69, ammonia in methanol | $^1$H NMR (400 MHz, DMSO-$d_6$): δ ppm = 8.70 (s, 2H), 7.75-7.65 (m, 2H), 7.62 (s, 1H), 7.40 (t, $J$ = 8.8 Hz, 2H), 7.28 (s, 1H), 5.20-5.05 (m, 1H), 3.26-3.20 (m, 1H), 1.95-1.85 (m, 2H), 1.65-1.57 (m, 6H), 1.20 (d, $J$ = 7.2 Hz, 3H). LC-MS (method 4): $R_t$ = 0.918 min; MS (ESIpos): m/z = 444.9 $[M+H]^+$. 28 % yield |

**Example 60**

rac-2-(N-[4-amino-5-[3-[[cyclopentyl(methyl)amino]methyl]isoxazole-5-carbonyl]thiazol-2-yl]-4-fluoro-anilino)propanamide

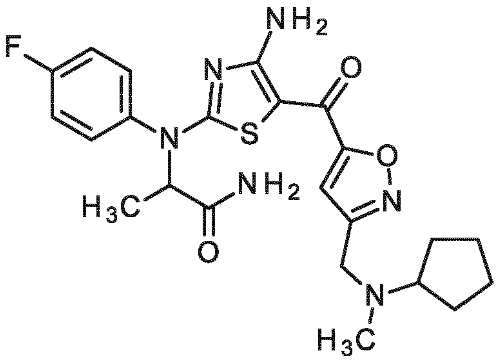

rac-2-(N-[4-amino-5-(3-formylisoxazole-5-carbonyl)thiazol-2-yl]-4-fluoro-anilino)propanamide (Intermediate 78, 97 mg, 0.24 mmol) was dissolved in a mixture of MeOH (2.5 mL), THF (0.4 mL) and DMF (0.4 mL). Cyclopentyl-methyl-amine (57 µL, 0.48 mmol) was added followed by the addition of acetic acid (14 µL, 0.24 mmol).The reaction mixture was stirred for 15 minutes at room temperature followed by the addition of sodium cyanoborohydride (23 mg, 0.36 mmol).The reaction was stirred overnight and then diluted with ethyl acetate and a saturated aqueous solution of sodium carbonate and brine. The organic phase was dried over sodium sulfate, filtered off and the solvent was evaporated *in vacuo.* The residue obtained was purified by automated flash column chromatography (method Z, methanol + 2 Vol-% aqueous ammonia (33%)) to give 14 mg (0.029 mmol, 12%) of the title compound as a yellow solid.

$^1H$ NMR (DMSO-$d_6$, 400 MHz) δ ppm = 8.67 (br s, 1H), 8.42 (br s, 1H, J=1.5 Hz), 7.72-7.62 (m, 2H), 7.62 (br s, 1H), 7.39 (t, 2H, J=8.9 Hz), 7.29 (s, 1H), 6.75 (s, 1H), 5.11 (br s, 1H, J=2.5, 5.6 Hz), 3.60 (s, 2H), 2.6-2.7 (m, 1H), 2.09 (s, 3H), 1.7-1.8 (m, 2H), 1.5-1.7 (m, 2H), 1.4-1.5 (m, 2H), 1.3-1.4 (m, 2H), 1.19 (d, 3H, J=7.4 Hz).

LC-MS (method 2): Rt = 1.17 min; MS(ESIpos) m/z = 487.4 $[M+H]^+$.

**Example 61**

rac-2-(N-[4-amino-5-[3-[[(1-methylcyclopentyl)amino]methyl]isoxazole-5-carbonyl]thiazol-2-yl]-4-fluoro-anilino)propanamide

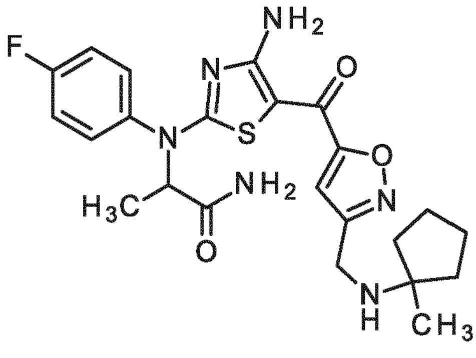

rac-2-(N-[4-amino-5-(3-formylisoxazole-5-carbonyl)thiazol-2-yl]-4-fluoro-anilino)propanamide (Intermediate 78, 20 mg, 50 µmol) was dissolved in a mixture of MeOH (0.5 mL), THF (0.1 mL) and DMF (0.1 mL). 1-methylcyclopentanamine (12 µL, 99 µmol) was added followed by the addition of acetic acid (3 µL, 50 µmol) and the reaction mixture was stirred for 15 minutes at room temperature followed by the addition of sodium cyanoborohydride (5 mg, 74 µmol). The reaction mixture was stirred overnight at room temperature and 5 h at 50°C. The reaction mixture was diluted with DMSO and acetonitrile and purified by preparative RP-HPLC (method C, basic) to obtain 4.4 mg (9 µmol, 18%) of the

title compound .

$^1$H NMR (DMSO-$d_6$, 400 MHz) δ ppm = 8.65 (br s, 1H), 8.40 (br s, 1H), 7.69 (dd, 2H, *J*=5.1, 8.9 Hz), 7.62 (br s, 1H), 7.39 (t, 2H, *J*=8.9 Hz), 7.28 (br s, 1H), 6.82 (s, 1H), 5.10 (br s, 1H), 3.66 (d, 2H, *J*=7.6 Hz), 1.6-1.7 (m, 2H), 1.5-1.6 (m, 4H), 1.3-1.4 (m, 2H), 1.18 (d, 3H, *J*=7.4 Hz), 1.09 (s, 3H).

LC-MS (method 2): Rt = 1.14 min; MS(ESIneg) m/z = 485.4 [M-H]$^-$.

**Example 62**

rac-2-(N-[4-amino-5-[3-[(cyclopentylamino)methyl]isoxazole-5-carbonyl]thiazol-2-yl]-4-fluoro-anilino)propanamide

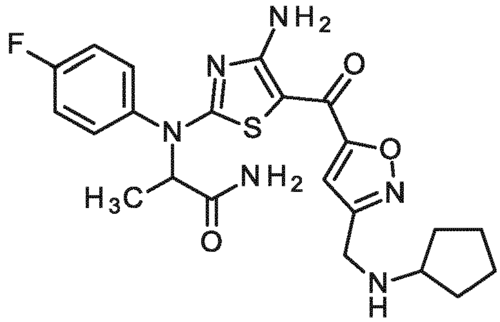

rac-2-(N-[4-amino-5-(3-formylisoxazole-5-carbonyl)thiazol-2-yl]-4-fluoro-anilino)propanamide (Intermediate 78, 17 mg, 43 μmol) was dissolved in a mixture of MeOH (0.5 mL) and DMF (0.1 mL). Cyclopentanamine (10 μL, 85 μmol) was added followed by the addition of acetic acid (2.4 μL, 43 μmol). The reaction mixture was stirred for 15 minutes at room temperature and then sodium cyanoborohydride (4 mg, 64 μmol) was added. The reaction mixture was stirred for 4 h at room temperature and then was heated to 50°C overnight. The solvent was partially reduced with a stream of nitrogen and the residue was diluted with DMSO and purified by preparative RP-HPLC (method C, basic) to obtain 6.0 mg (13 μmol, 30%) of the title compound.

$^1$H NMR (DMSO-$d_6$, 500 MHz) δ ppm = 8.67 (br s, 1H, J=1.9 Hz), 8.40 (br s, 1H), 7.70 (dd, 2H, *J*=5.2, 8.7 Hz), 7.62 (br s, 1H), 7.39 (t, 2H, *J*=8.8 Hz), 7.28 (br s, 1H), 6.86 (s, 1H), 5.0-5.2 (m, 1H), 3.67 (d, 2H, *J*=6.0 Hz), 2.91-2.85 (m, 1H), 2.30-2.20 (m, 1H), 1.5-1.7 (m, 4H), 1.3-1.5 (m, 2H), 1.2-1.3 (m, 2H), 1.18 (d, 3H, *J*=7.6 Hz).

LC-MS (method 2): Rt = 1.10 min; MS(ESIpos) m/z = 473.6 [M+H]$^+$.

**Example 63**

rac-2-(N-[4-amino-5-[3-(1-piperidylmethyl)isoxazole-5-carbonyl]thiazol-2-yl]-4-fluoro-anilino)propanamide

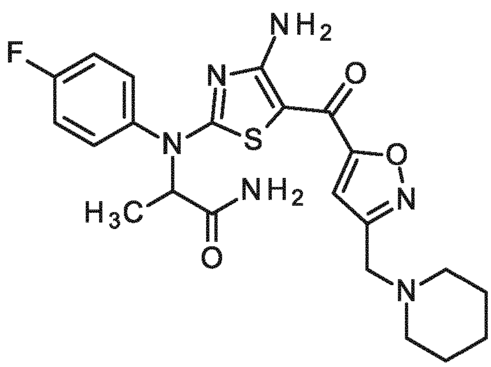

rac-2-(N-[4-amino-5-(3-formylisoxazole-5-carbonyl)thiazol-2-yl]-4-fluoro-anilino)propanamide (Intermediate 78, 14 mg, 35 μmol) was dissolved in MeOH (0.5 mL), piperidine (5 μL, 52 μmol) was added followed by the addition of acetic acid (2.0 μL, 35 μmol). The reaction mixture was stirred for 15 minutes at room temperature followed by the addition of sodium cyanoborohydride (2.6 mg, 42 μmol). The reaction mixture was stirred for two days at room

temperature. The reaction mixture was diluted with DMSO and purified by preparative RP-HPLC (method C, basic) to obtain 3.2 mg (7 μmol, 20%) of the title compound.

$^1$H NMR (DMSO-$d_6$, 400 MHz) δ ppm = 8.66 (br s, 1H, *J*=1.5 Hz), 8.43 (br s, 1H), 7.70 (dd, 2H, *J*=5.1, 8.9 Hz), 7.62 (br s, 1H), 7.39 (t, 2H, *J*=8.9 Hz), 7.29 (br s, 1H), 6.74 (s, 1H), 5.11 (br s, 1H, *J*=3.3 Hz), 3.50 (s, 2H), 2.31 (m, 4H), 1.45 (m, 4H), 1.34 (m, 2H), 1.19 (d, 3H, *J*=7.4 Hz).

LC-MS (method 2): Rt = 1.12 min; MS(ESIneg) m/z = 471.5 $[M-H]^-$.

**Example 64**

rac-2-(N-[4-amino-5-[3-[(4-cyanophenoxy)methyl]isoxazole-5-carbonyl]thiazol-2-yl]-4-fluoro-anilino)propanamide

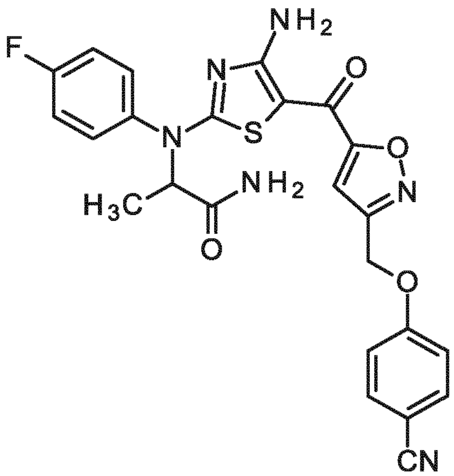

rac-2-(N-[4-amino-5-[3-(hydroxymethyl)isoxazole-5-carbonyl]thiazol-2-yl]-4-fluoro-anilino)propanamide (Example 65, 50 mg, 0.123 mmol), triphenylphosphine (65 mg, 0.247 mmol) and 4-cyanophenol (29 mg, 0.247 mmol) were dissolved in THF (1 mL) and DMF (0.5 mL). To this solution diisopropyl azodicarboxylate (46 μL, 0.234 mmol) was added dropwise and the reaction mixture was stirred at room temperature overnight. The reaction mixture was diluted with ethyl acetate and a saturated aqueous solution of ammonium chloride. The phases were separated and the organic phase was washed with a saturated aqueous solution of sodium carbonate and brine, dried over sodium sulfate, filtered off and evaporated *in vacuo*. The residue obtained was purified by automated flash column chromatography (method Z, methanol + 2 Vol-% aqueous ammonia (33%)) to give 30 mg (0.058 mmol, 47%) of the title compound.

$^1$H NMR (DMSO-$d_6$, 400 MHz) δ ppm = 8.69 (br s, 1H), 8.49 (br s, 1H), 7.8-7.8 (m, 2H), 7.70 (dd, 2H, *J*=4.9, 8.7 Hz), 7.62 (br s, 1H), 7.39 (t, 2H, *J*=8.7 Hz), 7.29 (br s, 1H), 7.2-7.2 (m, 2H), 6.98 (s, 1H), 5.33 (s, 2H), 5.1-5.2 (m, 1H), 1.18 (d, 3H, *J*=7.4 Hz).

LC-MS (method 2): Rt = 1.09 min; MS(ESIneg) m/z = 505.4 $[M-H]^-$.

**Example 65**

rac-2-(N-[4-amino-5-[3-(hydroxymethyl)isoxazole-5-carbonyl]thiazol-2-yl]-4-fluoro-anilino)propanamide

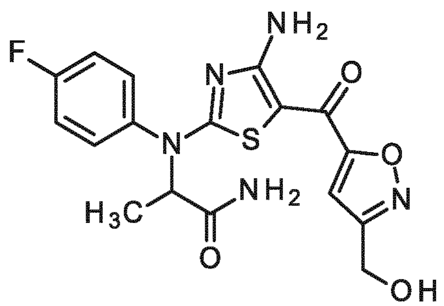

[4-amino-2-(4-fluoroanilino)thiazol-5-yl]-[3-(hydroxymethyl)isoxazol-5-yl]methanone (700 mg, 2.09 mmol, Intermediate 77) and potassium carbonate (868 mg, 6.28 mmol) were mixed together in DMF (14 mL), then rac-2-bromopropanamide (382 mg, 2.51 mmol) was added portion wise and the reaction was stirred at room temperature overnight. The reaction mixture was diluted with ethyl acetate and a saturated aqueous solution of ammonium chloride. The phases were separated and the organic phase was washed with brine and dried over sodium sulfate. The solvent was removed under vacuo distillation, the residue thus obtained was purified by automated flash column chromatography (method Z, methanol + 2 Vol-% aqueous ammonia (33%)) to give 535 mg (1.32 mmol, 63%) of the title compound.

$^1H$ NMR (400 MHz, DMSO-$d_6$) δ ppm = 8.67 (br s, 1 H) 8.43 (br s, 1 H) 7.70 (dd, *J*=8.87, 5.07 Hz, 2 H) 7.62 (br s, 1 H) 7.39 (t, *J*=8.74 Hz, 2 H) 7.29 (br s, 1 H) 6.79 (s, 1 H) 5.53 (t, *J*=6.08 Hz, 1 H) 5.11 (m, 1 H) 4.49 (d, *J*=6.08 Hz, 2 H), 1.18 (d, *J*=7.60 Hz, 3 H).

LC-MS (method 1): Rt = 0.82 min; MS(ESIpos) m/z = 406.0 $[M+H]^+$.

**Example 66**

rac-[5-[4-amino-2-(N-(2-amino-1-methyl-2-oxo-ethyl)-4-fluoro-anilino)thiazole-5-carbonyl]isoxazol-3-yl]methyl N-phenylcarbamate

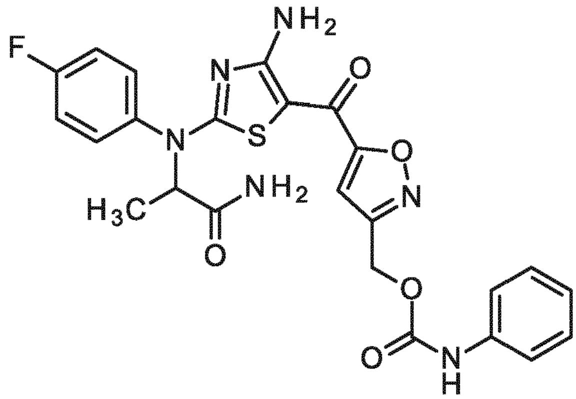

rac-2-(N-[4-amino-5-[3-(hydroxymethyl)isoxazole-5-carbonyl]thiazol-2-yl]-4-fluoro-anilino)propanamide (Example 65, 25 mg, 62 μmol) was dissolved in DMF (0.3 mL), diisopropylethylamine (32 μL, 0.185 mmol) was added followed by the addition of 4-nitrophenyl-chloroformiate (15 mg, 0.074 mmol) as solution in THF (0.3 mL). The reaction mixture was stirred for three hours then further diisopropylethylamine (32 μL, 0.185 mmol) followed by aniline (11 μL, 0.123 mmol) were added. The reaction was stirred for two days then it was diluted with ethyl acetate and a saturated aqueous solution of ammonium chloride.The phases were separated and the organic phase was washed with a saturated aqueous solution of sodium carbonate and brine, dried over sodium sulfate and evaporated *in vacuo.* The residue was purified by preparative RP-HPLC (method B, basic) to obtain 2.4 mg (5 μmol, 7%) of the title compound.

$^1H$ NMR (DMSO-$d_6$, 400 MHz) δ ppm = 9.88 (s, 1H), 8.68 (br s, 1H, *J*=0.8 Hz), 8.4-8.5 (m, 1H), 7.69 (dd, 2H, *J*=5.1, 8.6 Hz), 7.62 (br s, 1H), 7.3-7.5 (m, 4H), 7.2-7.3 (m, 3H), 7.0-7.1 (m, 1H), 6.92 (s, 1H), 5.21 (s, 2H), 5.0-5.2 (m, 1H), 1.18 (d, 3H, *J*=7.4 Hz).

LC-MS (method 2): Rt = 1.11 min; MS(ESIpos) m/z = 525.4 $[M+H]^+$.

**Example 67**

rac-2-(N-[4-amino-5-[3-(2-fluorophenyl)isoxazole-5-carbonyl]thiazol-2-yl]-4-fluoro-anilino)propanamide

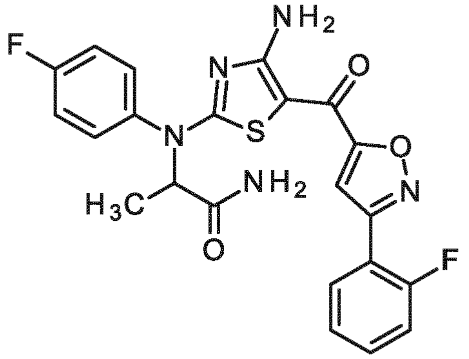

rac-2-(N-(4-amino-5-prop-2-ynoyl-thiazol-2-yl)-4-fluoro-anilino)propanamide (Intermediate 71, 18.5 mg, 56 μmol) was dissolved in DMSO (0.5 mL) and water (0.1 mL). 2-fluoro-N-hydroxy-benzimidoyl chloride (15 mg, 83 μmol, Intermediate 72) was added as a solution in t-BuOH (0.25 mL) directly followed by the addition of premixed copper(II) sulfate pentahydrate (2.8 mg, 11 μmol) and L-(+)-ascorbic acid (2.5 mg, 14 μmol) in water (0.15 mL) and finally potassium hydrogen carbonate (25 mg, 250 μmol) was added at once. The reaction mixture was stirred for 3 h and then it was diluted with ethyl acetate and water. The phases were separated and the organic phase was washed with brine, dried over sodium sulfate and evaporated under reduced pressure. The residue obtained was purified by preparative RP-HPLC (method D, basic) to obtain 3.9 mg (8 μmol, 15%) of the title compound.

$^1$H NMR (acetone-$d_6$, 400 MHz) δ ppm = 8.8-8.9 (br s, 1H), 7.95 (dt, 1H, *J*=1.9, 7.7 Hz), 7.7-7.8 (m, 2H), 7.5-7.6 (m, 1H), 7.50 (br s, 1H), 7.3-7.4 (m, 4H), 7.21 (d, 1H, *J*=3.3 Hz), 7.1-7.2 (m, 1H), 6.64 (br s, 1H), 5.32 (q, 1H, *J*=7.4 Hz), 1.31 (d, 3H, *J*=7.4 Hz).

LC-MS (method 2): Rt = 1.19 min; MS(ESIpos) m/z = 470.3 $[M+H]^+$.

## Example 68

rac-2-(N-[4-amino-5-[3-(2-pyridyl)isoxazole-5-carbonyl]thiazol-2-yl]-4-fluoro-anilino)propanamide

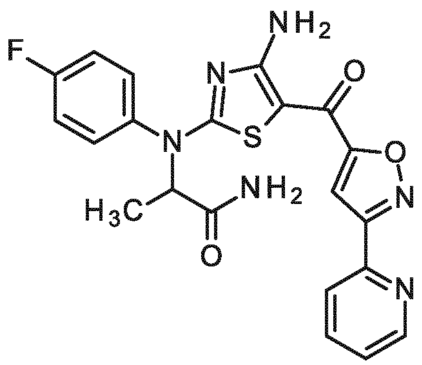

rac-2-(N-(4-amino-5-prop-2-ynoyl-thiazol-2-yl)-4-fluoro-anilino)propanamide (Intermediate 71, 20 mg, 60 μmol) was dissolved in DMSO (0.5 mL) and t-BuOH (0.25 mL), then N-hydroxypyridine-2-carboximidoyl chloride (12 mg, 75 μmol, Intermediate 74) was added as a solution in THF (0.1 mL) directly followed by the addition of premixed copper(II) sulfate pentahydrate (3 mg, 12 μmol) and L-(+)-ascorbic acid (2.7 mg, 15 μmol) in water (0.15 mL) and finally potassium hydrogen carbonate (27 mg, 271 μmol) as a solution in water (0.1 mL). The reaction mixture was stirred for three hours and then diluted with ethyl acetate and water. The phases were separated and the organic phase was washed with brine, dried over sodium sulfate and evaporated under reduced pressure. The residue obtained was purified by preparative RP-HPLC (method C, basic) to obtain 2.5 mg (5 μmol, 18%) of the title compound.

$^1$H NMR (DMSO-$d_6$, 400 MHz) δ ppm = 8.7-8.8 (m, 2H), 8.51 (br s, 1H), 7.9-8.0 (m, 2H), 7.72 (dd, 2H, *J*=5.1, 8.6 Hz), 7.64 (br s, 1H), 7.54 (ddd, 1H, *J*=1.5, 4.9, 7.3 Hz), 7.41 (t, 2H, *J*=8.7 Hz), 7.30 (br s, 1H), 7.28 (s, 1H), 5.13 (br s, 1H), 1.20 (d, 3H, *J*=7.4 Hz).

LC-MS (method 2): Rt = 1.03 min; MS(ESIpos) m/z = 453.4 $[M+H]^+$.

**Example 69**

rac-2-(N-[4-amino-5-[3-[4-(trifluoromethoxy)phenyl]isoxazole-5-carbonyl]thiazol-2-yl]-4-fluoro-anilino)propanamide

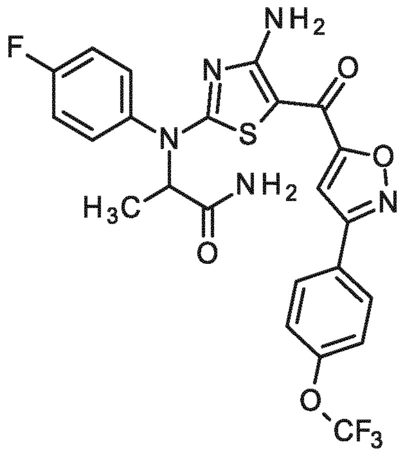

rac-2-(N-(4-amino-5-prop-2-ynoyl-thiazol-2-yl)-4-fluoro-anilino)propanamide (Intermediate 71, 15 mg, 45 μmol) was dissolved in DMSO (0.3 mL) and t-BuOH (0.1 mL), then N-hydroxy-4-(trifluoromethoxy)benzimidoyl chloride (Intermediate 75, 29 mg, 122 μmol) was added as a solution in THF (0.1 mL) directly followed by premixed copper(II) sulfate pentahydrate (1.4 mg, 9 μmol) and L-(+)-ascorbic acid (2.0 mg, 11 μmol) in water (0.15 mL) and finally potassium hydrogen carbonate (23 mg, 226 μmol). The reaction mixture was stirred at 38°C overnight and then diluted with ethyl acetate and water. The phases were separated, and the organic phase was washed with brine, dried over sodium sulfate and evaporated under reduced pressure. The obtained residue was purified by preparative RP-HPLC (method E, basic) to obtain 7.5 mg (14 μmol, 31%) of the title compound.

$^1$H NMR (400 MHz, methanol-*d*$_4$) δ ppm = 7.92 - 7.98 (m, 2 H) 7.62 - 7.69 (m, 2 H) 7.36 - 7.43 (m, 2 H) 7.29 (t, *J*=8.74 Hz, 2 H) 7.25 (s, 1 H) 5.28 (q, *J*=7.35 Hz, 1 H) 1.29 (d, *J*=7.35 Hz, 3 H).

LC-MS (method 2): Rt = 1.33 min; MS(ESIpos) m/z = 436.3 $[M+H]^+$.

**Example 70**

rac-2-(N-[4-amino-5-[3-(2-methoxyphenyl)isoxazole-5-carbonyl]thiazol-2-yl]-4-fluoro-anilino)propanamide

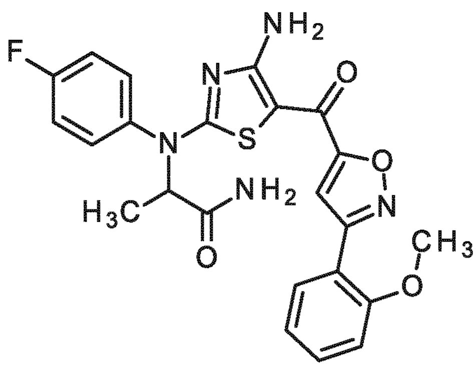

rac-2-(N-(4-amino-5-prop-2-ynoyl-thiazol-2-yl)-4-fluoro-anilino)propanamide (9.5 mg, 29 μmol, Intermediate 71) was dissolved in DMSO (0.3 mL) and *t*-BuOH (0.1 mL), then N-hydroxy-2-methoxy-benzimidoyl chloride (11 mg, 60 μmol, Intermediate 73) was added as a solution in THF (0.1 mL) directly followed by premixed copper(II) sulfate (0.9 mg, 6 μmol) and L-(+)-ascorbic acid (1.3 mg, 7 μmol) in water (0.15 mL) and finally potassium hydrogen carbonate (13 mg, 129 μmol) was added at once. The reaction mixture was stirred at 37°C for 2 h and at room temperature for two days. After dilution with ethyl acetate and water, the phases were separated and the organic phase was washed with brine, dried over

sodium sulfate and evaporated under reduced pressure. The residue obtained was purified by preparative RP-HPLC (method D, basic) to obtain 2.5 mg (5 μmol, 18%) of the title compound.

$^1$H NMR (DMSO-$d_6$, 400 MHz) δ ppm = 8.69 (br s, 1H), 8.47 (br s, 1H), 7.7-7.8 (m, 3H), 7.64 (br s, 1H), 7.50 (ddd, 1H, *J*=1.8, 7.2, 8.6 Hz), 7.41 (t, 2H, *J*=8.7 Hz), 7.30 (br s, 1H), 7.2-7.2 (m, 2H), 7.06 (dt, 1H, *J*=0.9, 7.5 Hz), 5.0-5.2 (m, 1H), 3.85 (s, 3H), 1.20 (d, 3H, *J*=7.6 Hz)

**Example 71**

rac-2-(N-[4-amino-5-[3-(6-methoxy-3-pyridyl)isoxazole-5-carbonyl]thiazol-2-yl]-4-fluoro-anilino)propanamide

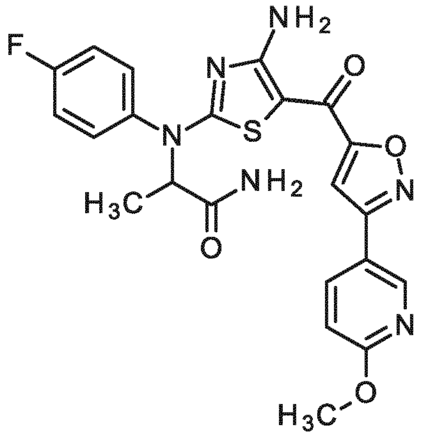

rac-2-(N-(4-Amino-5-prop-2-ynoyl-thiazol-2-yl)-4-fluoro-anilino)propanamide (19 mg, 57 μmol, Intermediate 71) was dissolved in THF (0.3 mL) then N-hydroxy-6-methoxy-pyridine-3-carboximidoyl chloride (17 mg, 91 μmol, Intermediate 76) as a solution in THF (0.3 mL) was added to it followed by triethylamine (32 μL, 229 mmol). The reaction mixture was heated to 37°C overnight. The solvent was then removed with a stream of nitrogen and the residue thus obtained was purified by preparative RP-HPLC (method D, basic) to obtain 4.5 mg (9 μmol, 16%) of the title compound.

$^1$H NMR (DMSO-$d_6$, 400 MHz) δ ppm = 8.74 (d, 2H, *J*=1.8 Hz), 8.49 (br s, 1H), 8.18 (dd, 1H, *J*=2.4, 8.7 Hz), 7.71 (dd, 2H, *J*=5.2, 8.7 Hz), 7.64 (br s, 1H), 7.57 (s, 1H), 7.41 (t, 2H, *J*=8.7 Hz), 7.30 (s, 1H), 6.9-7.0 (m, 1H), 5.0-5.2 (m, 1H), 3.91 (s, 3H), 1.20 (d, 3H, *J*=7.4 Hz).

LC-MS (method 2): Rt = 1.15 min; MS(ESIpos) m/z = 483.6 [M+H]$^+$.

**Example 72**

rac-2-(N-[4-amino-5-(1-phenyltriazole-4-carbonyl)thiazol-2-yl]-4-fluoro-anilino)propanamide

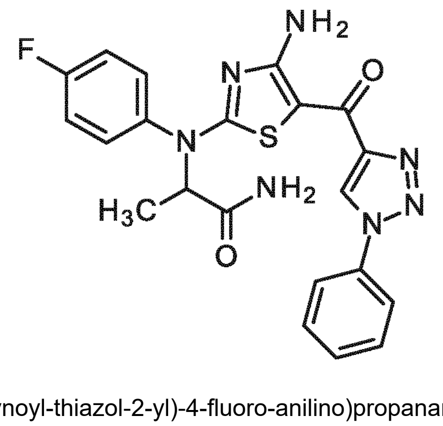

rac-2-(N-(4-amino-5-prop-2-ynoyl-thiazol-2-yl)-4-fluoro-anilino)propanamide (Intermediate 71, 27 mg, 81 μmol) and a 0.5 M solution of phenyl azide in 2-methyltetrahydrofuran (0.24 mL, 122 μmol) were dissolved in a mixture of DMSO

(0.5 mL), t-BuOH (0.25 mL) and water (0.1 mL). To this solution a premixed solution of copper(II) sulfate (2.59 mg, 16 μmol) and L-(+)-ascorbic acid (3.58 mg, 20 μmol) in water (0.15 mL) were added and the reaction mixture was stirred at room temperature for 1.5 hours. The reaction mixture was diluted with ethyl acetate and brine, the organic phase was dried over sodium sulfate and evaporated under reduced pressure. The residue obtained was purified by preparative RP-HPLC (method C, basic) to obtain 7.5 mg (17 μmol, 37%) of the title compound.

$^{1}$H NMR (DMSO-$d_6$, 400 MHz) δ ppm = 9.24 (s, 1H), 8.65 (br s, 1H), 8.05 (br s, 1H), 7.9-8.0 (m, 2H), 7.6-7.7 (m, 2H), 7.6-7.6 (m, 3H), 7.5-7.5 (m, 1H), 7.40 (t, 2H, *J*=8.7 Hz), 7.26 (br s, 1H), 5.0-5.2 (m, 1H), 1.19 (d, 3H, *J*=7.4 Hz).

LC-MS (method 2): Rt = 1.07 min; MS(ESIpos) m/z = 452.3 [M+H]$^+$.

**Example 73**

rac-2-(N-[4-amino-5-[3-[(3-fluoroazetidin-1-yl)methyl]isoxazole-5-carbonyl]thiazol-2-yl]-4-fluoro-anilino)propanamide

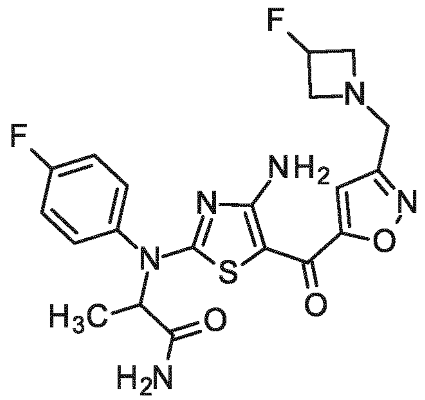

rac-[5-[4-amino-2-(N-(2-amino-1-methyl-2-oxo-ethyl)-4-fluoro-anilino)thiazole-5-carbonyl]isoxazol-3-yl]methyl methanesulfonate (Intermediate 79, 200 mg, 0.41 mmol) was dissolved in THF (4 mL). Diisopropyl ethyl amine (0.36 mL, 2.1 mmol) and 3-fluoroazetidine hydrochloride (92 mg, 0.83 mmol) were added and the reaction mixture was stirred overnight at room temperature. The reaction mixture was diluted with ethyl acetate and brine, the phases separated, and the organic phase was dried over sodium sulfate and reduced *in vacuo*. The residue obtained was purified by automated flash column chromatography (method Z, basic, 2 vol% aqueous ammonia in MeOH) to give 11 mg (0.02 mmol, 6 %) of the title compound as yellow solid.

$^{1}$H NMR (400 MHz, DMSO-$d_6$) δ ppm = 8.68 (br s, 1 H) 8.44 (br s, 1 H) 7.70 (dd, *J*=8.87, 5.07 Hz, 2 H) 7.62 (s, 1 H) 7.39 (t, *J*=8.74 Hz, 2 H) 7.29 (s, 1 H) 6.77 (s, 1 H) 5.16 - 5.26 (m, 1 H) 5.00 - 5.15 (m, 2 H) 3.68 (s, 2 H) 3.48 - 3.63 (m, 2 H) 3.12 - 3.26 (m, 2 H) 1.18 (d, *J*=7.35 Hz, 3 H).

LC-MS (method 2): Rt = 0.97 min; MS(ESIpos) m/z = 463.6 [M+H]$^+$.

**Example 74**

rac-2-(N-[4-amino-5-[3-[(3,3-difluoroazetidin-1-yl)methyl]isoxazole-5-carbonyl]thiazol-2-yl]-4-fluoro-anilino)propanamide

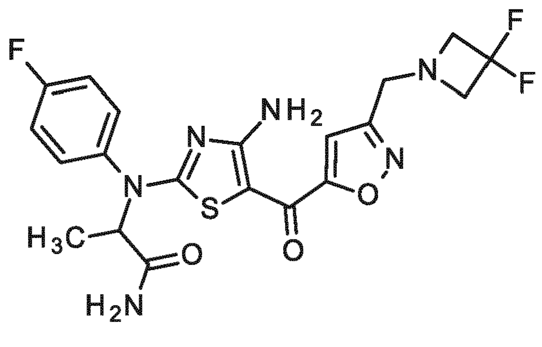

rac-[5-[4-amino-2-(N-(2-amino-1-methyl-2-oxo-ethyl)-4-fluoro-anilino)thiazole-5-carbonyl]isoxazol-3-yl]methyl methanesulfonate (Intermediate 79, 200 mg, 0.41 mmol) was dissolved in THF (4 mL). Diisopropyl ethyl amine (0.36 mL, 2.1 mmol) and 3,3-difluoroazetidine hydrochloride (107 mg, 0.83 mmol) were added and the reaction mixture was stirred overnight at room temperature. The reaction mixture was diluted with ethyl acetate and brine, the phases were separated, and the organic phase was dried over sodium sulfate and reduced *in vacuo.* The residue obtained was purified by automated flash column chromatography (method Z, basic, 2 vol% aqueous ammonia in MeOH) to give 52 mg (0.11 mmol, 26 %) of the title compound as yellow solid.

$^1$H NMR (400 MHz, DMSO-$d_6$) δ ppm = 8.68 (br s, 1 H) 8.45 (br s, 1 H) 7.70 (dd, *J*=8.74, 5.20 Hz, 2 H) 7.62 (br s, 1 H) 7.39 (t, *J*=8.74 Hz, 2 H) 7.29 (s, 1 H) 6.82 (s, 1 H) 5.10 (br s, 1 H) 3.80 (s, 2 H) 3.66 (t, *J*=12.55 Hz, 4 H) 1.18 (d, *J*=7.35 Hz, 3 H).

LC-MS (method 2): Rt = 1.03 min; MS(ESIpos) m/z = 481.5 $[M+H]^+$.

## Example 75

rac-2-(N-[4-amino-5-[3-[(4,4-difluoro-1-piperidyl)methyl]isoxazole-5-carbonyl]thiazol-2-yl]-4-fluoro-anilino)propanamide

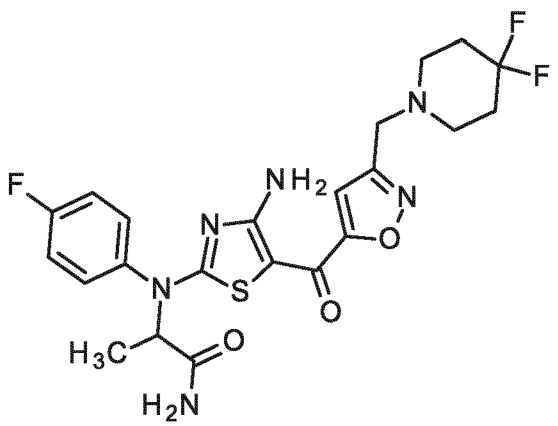

rac-[5-[4-amino-2-(N-(2-amino-1-methyl-2-oxo-ethyl)-4-fluoro-anilino)thiazole-5-carbonyl]isoxazol-3-yl]methyl methanesulfonate (Intermediate 79, 200 mg, 0.41 mmol) was dissolved in THF (4 mL). Diisopropyl ethyl amine (0.36 mL, 2.1 mmol) and 4,4-difluoropiperidine (100 mg, 0.83 mmol) were added and the reaction mixture was stirred overnight at room temperature. The reaction mixture was diluted with ethyl acetate and brine, the phases were separated, and the organic phase was dried over sodium sulfate and reduced *in vacuo.* The residue obtained was purified by automated flash column chromatography (method Z, basic, 2 vol% aqueous ammonia in MeOH) to give 61 mg (0.12 mmol, 29 %) of the title compound as yellow solid.

$^1$H NMR (400 MHz, DMSO-$d_6$) δ ppm = 8.67 (br s, 1 H) 8.44 (br s, 1 H) 7.70 (dd, *J*=8.74, 5.20 Hz, 2 H) 7.62 (s, 1 H) 7.39 (t, J=8.74 Hz, 2 H) 7.29 (s, 1 H) 6.81 (s, 1 H) 5.10 (br s, 1 H) 3.65 (s, 2 H) 1.82 - 2.07 (m, 4 H) 1.19 (d, J=7.35 Hz, 3 H).

LC-MS (method 2): Rt = 1.11 min; MS(ESIpos) m/z = 509.4 $[M+H]^+$.

## Example 76

2-(N-[4-amino-5-[3-[(4,4-difluoro-3-methyl-1-piperidyl)methyl]isoxazole-5-carbonyl]thiazol-2-yl]-4-fluoro-anilino)propanamide (mixture of stereoisomers)

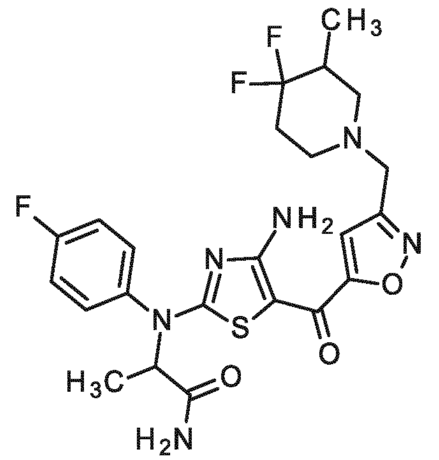

rac-[5-[4-amino-2-(N-(2-amino-1-methyl-2-oxo-ethyl)-4-fluoro-anilino)thiazole-5-carbonyl]isoxazol-3-yl]methyl methanesulfonate (Intermediate 79, 190 mg, 0.39 mmol) was dissolved in THF (4 mL). Diisopropyl ethyl amine (0.21 mL, 1.18 mmol) and rac-4,4-difluoro-3-methylpiperidine (106 mg, 0.79 mmol) were added and the reaction mixture was stirred overnight at room temperature. The reaction mixture was diluted with ethyl acetate and brine, the phases were separated, and the organic phase was dried over sodium sulfate and reduced *in vacuo.* The residue obtained was purified by automated flash column chromatography (method Z, basic, 2 vol% aqueous ammonia in MeOH) to give 52 mg (0.1 mmol, 25 %) of the title compound as yellow solid.

$^{1}$H NMR (400 MHz, DMSO-$d_6$) δ ppm = 8.67 (br s, 1 H) 8.44 (br s, 1 H) 7.70 (dd, *J*=8.74, 5.20 Hz, 2 H) 7.62 (br s, 1 H) 7.39 (t, *J*=8.74 Hz, 2 H) 7.29 (s, 1 H) 6.80 (s, 1 H) 5.10 (br s 1 H) 3.63 (s, 2 H) 2.65 - 2.78 (m, 2 H) 2.22 - 2.31 (m, 1 H) 1.83 - 2.13 (m, 4 H) 1.19 (d, *J*=7.35 Hz, 3 H) 0.89 (d, *J*=6.59 Hz, 3 H).

LC-MS (method 2): Rt = 1.18 min; MS(ESIpos) m/z = 523.4 $[M+H]^+$.

## Example 77

2-(N-[4-amino-5-[3-[(3-fluoropyrrolidin-1-yl)methyl]isoxazole-5-carbonyl]thiazol-2-yl]-4-fluoro-anilino)propanamide (mixture of stereoisomers)

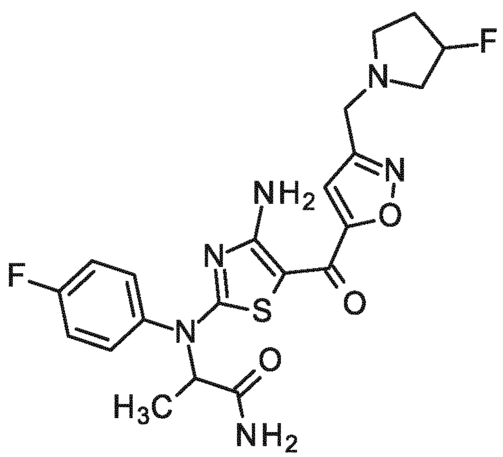

rac-[5-[4-amino-2-(N-(2-amino-1-methyl-2-oxo-ethyl)-4-fluoro-anilino)thiazole-5-carbonyl]isoxazol-3-yl]methyl methanesulfonate (Intermediate 79, 190 mg, 0.39 mmol) was dissolved in THF (4 mL). Diisopropyl ethyl amine (0.21 mL, 1.18 mmol) and rac-3-fluoropyrrolidine (70 mg, 0.79 mmol) were added and the reaction mixture was stirred overnight at room temperature. The reaction mixture was diluted with ethyl acetate and brine, the phases were separated, and the organic phase was dried over sodium sulfate and reduced *in vacuo.* The residue obtained was purified by automated flash column chromatography (method Z, basic, 2 vol% aqueous ammonia in MeOH) to give 48 mg (0.1 mmol, 25 %) of the title compound as yellow solid.

$^{1}$H NMR (400 MHz, DMSO-$d_6$) δ ppm = 8.68 (br s, 1 H) 8.44 (br s, 1 H) 7.70 (dd, *J*=8.87, 5.07 Hz, 2 H) 7.62 (br s, 1

H) 7.39 (t, *J*=8.74 Hz, 2 H) 7.29 (br s, 1 H) 6.79 (s, 1 H) 5.03 - 5.28 (m, 2 H) 3.69 (s, 2 H) 2.56 - 2.89 (m, 3 H) 2.29 - 2.41 (m, 1 H) 2.00 - 2.24 (m, 1 H) 1.73 - 1.95 (m, 1 H) 1.19 (d, *J*=7.35 Hz, 3 H).

LC-MS (method 2): Rt = 0.99 min; MS(ESIpos) m/z = 477.4 $[M+H]^+$.

## Example 78

rac-2-(N-[4-Amino-5-(3-hydroxyisoxazole-5-carbonyl)thiazol-2-yl]-4-fluoro-anilino)propanamide

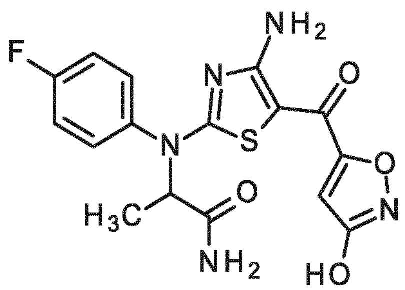

rac-2-(N-[5-(3-allyloxyisoxazole-5-carbonyl)-4-amino-thiazol-2-yl]-4-fluoro-anilino)propanamide (Intermediate 85, 1.95 g, 4.52 mmol) was dissolved in MeOH (135 mL) and THF (55 mL). Tetrakis(triphenylphosphine)palladium(0) (521 mg, 0.45 mmol) was added and the reaction was stirred for 10 minutes followed by the addition of potassium carbonate (1.25 g, 9.03 mmol) and stirring was prolonged for one hour. Few drops of acetic acid were added and the solvent was evaporated under vacuum. The residue was purified by automated flash column chromatography (method Z, acidic, 1 vol% AcOH) to give 1.64 g (4.09 mmol, 91 %) of the title compound as yellow solid.

$^1$H NMR (400 MHz, DMSO-$d_6$) δ ppm = 11.78 (br s, 1 H) 8.66 (br s, 1 H) 8.38 (br s, 1 H) 7.69 (dd, *J*=8.74, 4.94 Hz, 2 H) 7.61 (br s, 1 H) 7.39 (t, *J*=8.87 Hz, 2 H) 7.28 (br s, 1 H) 6.34 (s, 1 H) 5.10 (br s, 1 H) 1.18 (d, *J*=7.35 Hz, 3 H).

LC-MS (method 2): Rt = 0.47 min; MS(ESIneg) m/z = 390.3 $[M-H]^-$.

## Example 79

rac-2-(N-[4-amino-5-[3-(cyclobutoxy)isoxazole-5-carbonyl]thiazol-2-yl]-4-fluoro-anilino)propanamide

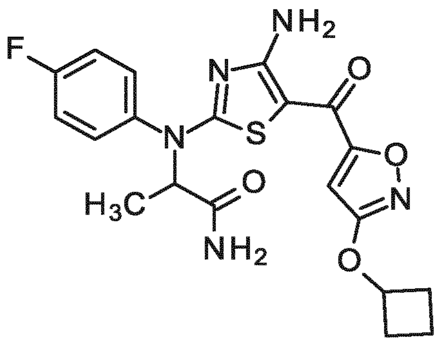

rac-2-(N-[4-Amino-5-(3-hydroxyisoxazole-5-carbonyl)thiazol-2-yl]-4-fluoro-anilino)propanamide (Example 78, 162 mg, 0.41 mmol) was dissolved in DMF (1 mL) and potassium carbonate (200 mg, 1.45 mmol) followed by cyclobutyl bromide (52 μL, 0.50 mmol) were added. The reaction mixture was heated to 70°C overnight then diluted with ethyl acetate and water. The phases were separated, and the organic phase was washed with brine, dried over sodium sulfate, and reduced *in vacuo*. The residue obtained was purified by automated flash column chromatography (method Z, basic, 2 vol% aqueous ammonia in MeOH) to give 68 mg (0.15 mmol, 37%) of the title compound.

$^1$H NMR (400 MHz, DMSO-$d_6$) δ ppm = 8.66 (br s, 1 H) 8.39 - 8.45 (br s, 1 H) 7.69 (dd, *J*=8.74, 4.94 Hz, 2 H) 7.62 (br s, 1 H) 7.39 (t, *J*=8.74 Hz, 2 H) 7.28 (br s, 1 H) 6.60 (s, 1 H) 5.10 (br s, 1 H) 4.83 (quin, *J*=7.22 Hz, 1 H) 2.28 - 2.41 (m, 2 H) 1.98 - 2.16 (m, 2 H) 1.68 - 1.85 (m, 1 H) 1.49 - 1.65 (m, 1 H) 1.18 (d, *J*=7.35 Hz, 3 H).

LC-MS (method 1): Rt = 1.21 min; MS(ESIpos) m/z = 446.4 $[M+H]^+$.

**Example 80**

rac-2-(N-[4-amino-5-[3-(cyclobutylmethoxy)isoxazole-5-carbonyl]thiazol-2-yl]-4-fluoro-anilino)propanamide

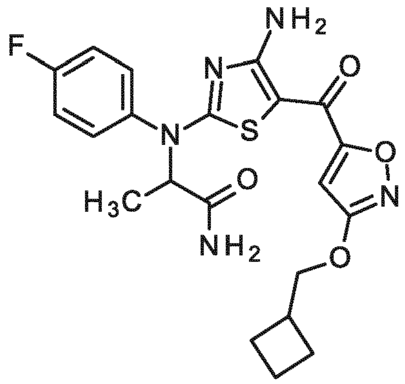

2-(N-[4-amino-5-(3-hydroxyisoxazole-5-carbonyl)thiazol-2-yl]-4-fluoro-anilino)propanamide (Example 78, 104 mg, 0.27 mmol) was dissolved in DMF (2 mL) and potassium carbonate (147 mg, 1.06 mmol) was added. The reaction mixture was heated to 70°C for one hour and cooled to room temperature followed by the addition of bromomethyl cyclobutane (36 μL, 0.32 mmol). The reaction mixture was heated to 70°C overnight. The reaction was then diluted with ethyl acetate and water, the organic phase was washed with brine and dried over sodium sulfate and reduced *in vacuo.* The residue obtained was purified by automated flash column chromatography (method Z, basic, 2 vol% aqueous ammonia in MeOH) and again by preparative RP-HPLC (method E, basic) to obtain 39 mg (84 μmol, 32%) of the title compound.

$^1$H NMR (400 MHz, DMSO-$d_6$) δ ppm = 8.66 (br s, 1 H) 8.45 (br s, 1 H) 7.69 (dd, *J*=8.74, 5.20 Hz, 2 H) 7.62 (br s, 1 H) 7.39 (t, *J*=8.87 Hz, 2 H) 7.29 (br s, 1 H) 6.63 (s, 1 H) 5.10 (br s, 1 H) 4.14 (d, *J*=6.84 Hz, 2 H) 2.61 - 2.81 (m, 1 H) 1.95 - 2.10 (m, 2 H) 1.68 - 1.97 (m, 4 H) 1.18 (d, *J*=7.60 Hz, 3 H).

LC-MS (method 2): Rt = 1.28 min; MS(ESIpos) m/z = 460.3 [M+H]$^+$.

**Example 81**

rac-2-(N-[4-Amino-5-[3-(4,4-difluorocyclohexoxy)isoxazole-5-carbonyl]thiazol-2-yl]-4-fluoro-anilino)propanamide

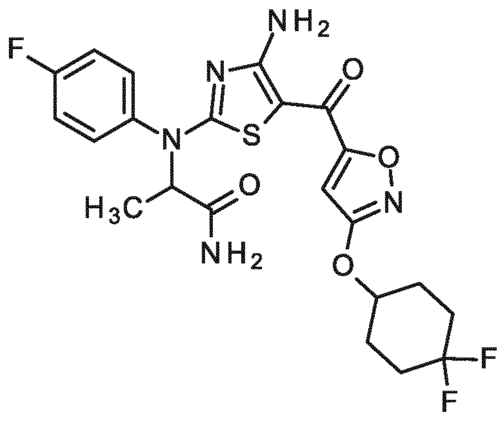

rac-2-(N-[4-Amino-5-(3-hydroxyisoxazole-5-carbonyl)thiazol-2-yl]-4-fluoro-anilino)propanamide (Example 78, 112 mg, 0.29 mmol) was dissolved in DMF (2 mL) and potassium carbonate (159 mg, 1.15 mmol) was added. The reaction mixture was heated to 70°C for one hour and cooled to room temperature followed by the addition of 4-bromo-1,1-difluorocyclohexane (69 mg, 0.34 mmol). The reaction mixture was heated to 70°C for 72 hours, then cooled to room temperature and further 4-bromo-1,1-difluorocyclohexane (69 mg, 0.34 mmol) was added and the reaction mixture was heated to 70°C for additional two hours. The reaction mixture was then diluted with ethyl acetate and water, the organic

phase was washed with brine and dried over sodium sulfate and reduced *in vacuo.* The residue obtained was purified by automated flash column chromatography (method Z, basic, 2 vol% aqueous ammonia in MeOH) followed by suspension of the residue in DMSO and a mixture of acetonitrile/water (1:1) causing a dense precipitate. The solid was collected by vacuum filtration and dried at high vacuum to afford 19 mg (38 μmol, 13%) of the title compound.

$^1H$ NMR (400 MHz, DMSO-$d_6$) δ ppm = 8.65 (br s, 1 H) 8.46 (br s, 1 H) 7.69 (dd, *J*=8.74, 5.20 Hz, 2 H) 7.62 (br s, 1 H) 7.39 (t, *J*=8.74 Hz, 2 H) 7.29 (br s, 1 H) 6.67 (s, 1 H) 5.10 (br s, 1 H) 4.74 (br m, 1 H) 1.77 - 2.05 (m, 8 H) 1.18 (d, *J*=7.35 Hz, 3 H).

LC-MS (method 2): Rt = 1.24 min; MS(ESIpos) m/z = 510.3 $[M+H]^+$.

## Example 82

rac-2-(N-[4-amino-5-(3-isopropoxyisoxazole-5-carbonyl)thiazol-2-yl]-4-fluoro-anilino)propanamide

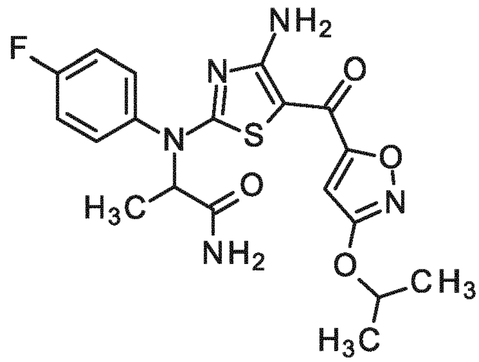

rac-2-(N-[4-amino-5-(3-hydroxyisoxazole-5-carbonyl)thiazol-2-yl]-4-fluoro-anilino)propanamide (Example 78, 100 mg, 0.26 mmol) was dissolved in DMF (2 mL) and potassium carbonate (142 mg, 1.03 mmol) followed by 2-bromopropane (29 μL, 0.31 mmol) were added to the reaction mixture and the reaction was heated to 70°C overnight. The reaction mixture was then diluted with ethyl acetate and water, the organic phase was washed with brine and dried over sodium sulfate and reduced *in vacuo.* The residue obtained was purified by automated flash column chromatography (method Z, basic, 2 vol% aqueous ammonia in MeOH) and again by preparative RP-HPLC (method E, basic) to yield 44 mg (102 μmol, 40%) of the title compound.

$^1H$ NMR (400 MHz, DMSO-$d_6$) δ ppm = 8.66 (br s, *J*=4.31 Hz, 1 H) 8.44 (br s, *J*=2.28 Hz, 1 H) 7.69 (dd, *J*=8.74, 4.94 Hz, 2 H) 7.62 (br s, 1 H) 7.39 (t, *J*=8.74 Hz, 2 H) 7.29 (br s, 1 H) 6.60 (s, 1 H) 5.10 (br s, 1 H) 4.76 (quin, *J*=6.08 Hz, 1 H) 1.30 (d, *J*=6.08 Hz, 6 H) 1.18 (d, *J*=7.35 Hz, 3 H).

## EXPERIMENTAL SECTION - DETERMINATION OF ABSOLUTE STEREOCHEMISTRY BY MEANS OF X-RAY-ANALYSIS

### Determination of the absolute configuration of Example 3.1

(R)-2-(N-[4-amino-5-(3-phenylisoxazole-5-carbonyl)thiazol-2-yl]-4-fluoro-anilino)propanamide

The crystallographic data of Example 3.1 as well as a figure depicting the thermal ellipsoids and numbering of the structure, are shown in Table 7 and Figure 6. Colorless crystals of Example 3.1 were obtained by slow evaporation from a propanol solution. A single crystal was mounted on a cryoloop using a protective oil. Single-crystal X-ray diffraction data were collected at 100 K on a Rigaku XtaLAB Synergy S system with a kappa goniometer and a HyPix-6000HE hybrid photon detector using Cu X-ray radiation (CuKα, = 1.54178 Å). Data were integrated using the program CrysAlis$^{PRO}$. SHELXM was used for structure solution and SHELXL was used for full-matrix least-squares refinement on $F^2$. In the asymmetric unit one molecule of Example 3.1 is present. In addition, a propanol molecule (half occupied) and one water molecule are present. The two solvent molecules are highly disordered and could not be refined properly, so the only a best model was included. These atoms were not refined anisotropically, hydrogens atoms were not added. All non-hydrogen atoms of example 3.1 were refined anisotropically and all hydrogen atoms were added in calculated positions and refined riding on their resident atoms. The isotropic temperature factors of the hydrogen atoms were refined as 1.2 and 1.5 times the size of the temperature factors of the corresponding heavy atoms, respectively. The absolute stereochemistry (R-configuration) could be assigned unambiguously with a Flack Parameter of -0.03(9). The program XP was used for molecular representations.

**Table 7: Crystal data and structure refinement for Example 3.1**

| | | |
|---|---|---|
| Identification code | 3.1 | |
| Empirical formula | C22 H23 F N5 O3 S + 0.5 C2 H6 O + H2 O | |
| Formula weight | 456.51 + 20.01 + 18.02 | |
| Temperature | 100(2) K | |
| Wavelength | 1.54178 Å | |
| Crystal system | Hexagonal | |
| Space group | P61 | |
| Unit cell dimensions | a = 26.728(4) Å | a= 90°. |
| | b = 26.728(4) Å | b= 90°. |
| | c = 6.876(3) Å | g = 120°. |
| Volume | 4254(2) $Å^3$ | |
| Z | 6 | |
| Density (calculated) | 1.154 $Mg/m^3$ | |
| Absorption coefficient | 1.380 $mm^{-1}$ | |
| F(000) | 1542 | |
| Crystal size | 0.100 × 0.050 × 0.050 $mm^3$ | |
| Theta range for data collection | 3.307 to 54.283°. | |
| Reflections collected | 2749 | |
| Independent reflections | 18122 [R(int) = 11.96] | |
| Completeness to theta = 54.283° | 97.7 % | |
| Refinement method | Full-matrix least-squares on $F^2$ | |
| Data / restraints / parameters | 2749 / 3 / 305 | |
| Goodness-of-fit on F2 | 0.997 | |
| Final R indices [I>2sigma(I)] | R1 = 0.0931, wR2 = 0.2238 | |
| R indices (all data) | R1 = 0.1765, wR2 = 0.2702 | |
| Absolute structure parameter | -0.03(9) | |
| Extinction coefficient | n/a | |
| Largest diff. peak and hole | 0.662 and -0.322 $e.Å^{-3}$ | |

**EXPERIMENTAL SECTION - BIOLOGICAL ASSAYS AND BIOLOGICAL DATA**

Table 8, below, lists the abbreviations used in this paragraph and in the Assays section as far as they are not explained within the text body. Other abbreviations have their meanings customary *per se* to the skilled person.

**Table 8: Abbreviations**

| | |
|---|---|
| nL | nanoliter |
| µL | microliter |
| mL | milliliter |
| nM | nanomolar |
| µM | micromolar |
| mM | millimolar |
| min | minute(s) |
| s | second(s) |
| kDa | kilodalton |
| MW | molecular weight |
| cAMP | cyclic adenosine monophosphat |
| ADP | adenosine diphosphate |
| ATP | adenosine triphosphate |

(continued)

| | |
|---|---|
| FCS | fetal calf serum |
| FBS | fetal bovine serum |
| PBS | phosphate buffered saline |
| RPMI | Roswell Park Memorial Institute |
| ACK lysing buffer | ammonium-chloride-potassium lysis buffer |
| DMEM | Dulbecco's Modified Eagle's Medium |
| HEPES | 2-[4-(2-hydroxyethyl)piperazin-1-yl]ethanesulfonic acid |
| MOPS | 3-(N-morpholino)propanesulfonic acid |
| Pen/Strep | penicillin and streptomycin |
| HBS-P+ | buffer containing 0.1 M HEPES, 1.5 M NaCl and 0.5% v/v Surfactant P20 |
| DTT | DL-Dithiothreitol |
| BGG | bovine gamma globulin |
| PBMC | peripheral blood mononuclear cells |
| APC | antigen presenting cells |
| CD | cluster of differentiation |
| IgG | immunoglobulin G |
| OKT3 | CD3 monoclonal antibody |
| FLAG-Tag | amino acid sequence DYKDDDDK |
| DNA | deoxyribonucleic acid |
| CFSE | carboxyfluorescein succinimidyl ester |
| OVA | ovalbumin antigen |
| FACS | fluorescence-activated cell sorting |
| s.c. | subcutaneous |
| i.v. | intravenous |
| i.p. | intraperitoneal |

Examples were tested in selected biological assays one or more times. When tested more than once, data are reported as either average values or as median values, wherein

- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

The *in vitro* activity of the compounds of the present invention can be demonstrated in the following assays:

**Human DGKζ kinase activity inhibition assay.**

Human diacylglycerol kinase zeta (DGKζ) inhibitory activity of compounds of the present invention was quantified employing the human DGKζ kinase activity assay as described in the following paragraphs. In essence, the enzyme activity was measured by quantification of the adenosine-di-phosphate (ADP) generated as a co-product of the enzyme reaction via the "ADP-Glo™ Kinase Assay" kit from the company Promega. This detection system works as follows: In a first step the adenosine-tri-phosphate (ATP) not consumed in the kinase reaction is quantitatively converted to cyclic adenosine-mono-phosphate (cAMP) employing an adenylate cyclase ("ADP-Glo-reagent"), then the adenylate cyclase is stopped and the ADP generated in the kinase reaction is converted to ATP, which subsequently generates in a luciferase-based reaction a glow-luminescence signal ("Kinase Detection Reagent").

C-terminally FLAG-tagged, recombinant full-length human DGKζ (inhouse expressed in baculovirus infected insect cells, purified using anti-Flag pulldown and size exclusion chromatography) was used as enzyme. As an alternative, commercially available enzyme by Carnabio can be used. As substrate for the kinase, 1,2-dioleoyl-sn-glycerol, reconstituted in octyl-β-D-glucopyranoside micelles, was used. For the preparation of the micelles, 1 volume of a 16.1 mM solution of 1,2-dioleoyl-sn-glycerol (Avanti, Cat. # O8001-25G) in chloroform was slowly evaporated using a nitrogen stream. Subsequently, 22.55 volumes of a 510 mM solution of octyl-β-D-glucopyranoside (Sigma-Aldrich, Cat. # O8001-10G) in 50 mM MOPS buffer (pH 7.4) were added, and the mixture was sonicated in an ultrasonic bath for 20 s. Then 35 volumes of 50 mM MOPS buffer (pH 7.4) were added to yield a solution of 0.28 mM 1,2 dioleoyl-sn-glycerol and 200 mM octyl-β-D-glucopyranoside, which was aliquoted, flash-frozen in liquid nitrogen, and stored at -20°C until use. For each experiment, a fresh aliquot was quickly thawed and diluted 24-fold with aqueous assay buffer (described below) containing 95.7 μM adenosine triphosphate (Promega) to yield a 1.67-fold concentrated substrate solution.

For the assay 50 nl of a 100-fold concentrated solution of the test compound in dimethyl sulfoxide (DMSO, Sigma) was pipetted into either a white 1536-well or a white low-volume 384-well microtiter plate (both Greiner Bio-One, Frickenhausen, Germany). Subsequently, 2 μl of a solution of human DGKζ in aqueous assay buffer [50 mM (3-(N-morpholino)propanesulfonic acid (MOPS, pH 7.4, Sigma-Aldrich), 1 mM dithiothreitol (DTT, Sigma-Aldrich), 100 mM NaCl (Sigma-Aldrich), 10 mM $MgCl_2$ (Sigma-Aldrich), 0.1 % (w/v) bovine gamma globulin (BGG, Sigma-Aldrich), 1 μM $CaCl_2$ (Sigma-Aldrich)] were added to the wells, and the mixture was incubated for 15 min at 22°C to allow pre-binding of the test compounds to the enzyme. The reaction was initiated by the addition of 3 μL of substrate solution [preparation described above; 11.7 μM 1,2-dioleoyl-sn-glycerol (=> final conc. in the 5 μL assay volume is 7 μM), 8.33 mM octyl-β-D-glucopyranoside (=> final conc. in 5 μL assay volume is 5 mM), and 91.67 μM adenosine triphosphate (=> final conc. in 5 μL assay volume is 55 μM) in assay buffer] and the resulting mixture was incubated for a reaction time of 20 min at 22°C. The concentration of DGKζ was adjusted depending of the activity of the enzyme lot and was chosen appropriate to have the assay in the linear range, a typical concentration is about 0.1 nM. The reaction was stopped by the addition of 2.5 μL of "ADP-Glo-reagent" (1 to 1.5 diluted with water) and the resulting mixture was incubated at 22°C for 1 h to convert the ATP not consumed in the kinase reaction completely to cAMP. Subsequently 2.5 μl of the "kinase detection reagent" (1.2fold more concentrated than recommended by the producer) were added, the resulting mixture was incubated at 22°C for 1 h and then the luminescence measured with a suitable measurement instrument (e.g. Viewlux™ from Perkin-Elmer). The amount of emitted light was taken as a measure for the amount of ADP generated and thereby for the activity of the DGKζ.

The data were normalised (enzyme reaction without inhibitor = 0 % inhibition, all other assay components but no enzyme = 100 % inhibition). Usually the test compounds were tested on the same microtiterplate in 11 different concentrations in the range of 20 μM to 0.07 nM (20 μM, 5.7 μM, 1.6 μM, 0.47 μM, 0.13 μM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and $IC_{50}$ values were calculated using Genedata Screener™ software.

**Table 9: $IC_{50}$ values of examples in *in vitro* human DGKζ kinase activity inhibition assays**

| Example number | $IC_{50}$ [nM] |
|---|---|
| 1 | 9 |
| 2 | 27 |
| 3 | 104 |
| 3.1 | 61 |
| 3.2 | 1752 |
| 4 | 37 |
| 5 | 67 |
| 6 | 123 |
| 6.1 | 64 |
| 6.2 | 3486 |
| 7 | 407 |
| 8 | 728 |
| 9 | 189 |
| 10 | 23 |
| 11 | 2358 |
| 12 | 938 |

(continued)

| Example number | $IC_{50}$ [nM] |
|---|---|
| 13 | 977 |
| 14 | 999 |
| 15 | 565 |
| 16 | 420 |
| 17 | 699 |
| 18 | 492 |
| 19 | 315 |
| 20 | 300 |
| 21 | 293 |
| 22 | 182 |
| 23 | 212 |
| 24 | 120 |
| 25 | 134 |
| 26 | 138 |
| 27 | 148 |
| 28 | 90 |
| 29 | 97 |
| 30 | 88 |
| 30.1 | 29 |
| 30.2 | 13437 |
| 31 | 193 |
| 32 | 93 |
| 32.1 | 31 |
| 32.2 | 897 |
| 33 | 48 |
| 34 | 223 |
| 35 | 566 |
| 36 | 446 |
| 37 | 43 |
| 38 | 344 |
| 39 | 53 |
| 40 | 204 |
| 41 | 134 |
| 42 | 146 |
| 43 | 94 |
| 44 | 59 |
| 44.1 | 19 |
| 44.2 | 4511 |
| 45 | 53 |

(continued)

| Example number | $IC_{50}$ [nM] |
|---|---|
| 45.1 | 20 |
| 45.2 | 1275 |
| 46 | 77 |
| 46.1 | 27 |
| 46.2 | 2279 |
| 47 | 42 |
| 48 | 73 |
| 49 | 28 |
| 50 | 409 |
| 51 | 17 |
| 52 | 412 |
| 53 | 25 |
| 54 | 306 |
| 55 | 17 |
| 56 | 14 |
| 57 | 32 |
| 58 | 325 |
| 59 | 12 |
| 60 | 58 |
| 61 | 98 |
| 62 | 194 |
| 63 | 88 |
| 64 | 32 |
| 65 | 751 |
| 66 | 29 |
| 67 | 109 |
| 68 | 402 |
| 69 | 117 |
| 70 | 27 |
| 71 | 96 |
| 72 | 999 |
| 73 | 325 |
| 74 | 113 |
| 75 | 26 |
| 76 | 10 |
| 77 | 242 |
| 78 | n.d. |
| 79 | 88 |
| 80 | 31 |

(continued)

| Example number | $IC_{50}$ [nM] |
|---|---|
| 81 | 17 |
| 82 | 49 |

**Table 10: $IC_{50}$ values of intermediates in *in vitro* human DGKζ kinase activity inhibition assays.**

| Intermediate number | $IC_{50}$ [nM] |
|---|---|
| 3 | > 5710 |
| 5 | > 5710 |
| 19 | > 20000 |
| 24 | > 20000 |
| 28 | 19450 |
| 30 | 11950 |

**Transactivation Assay in Jurkat IL2-reporter cell line**

Transactivation assays were carried out in Jurkat cells purchased from Promega (Promega, #CS187001) stably transfected with a firefly luciferase reporter gene construct under the control of the IL2-promoter. Cells were cultured as specified by the manufacturer. Bulk cells were harvested at a culture density of approx. 1E+06 cells/mL, suspended in cryo-storage medium (70%RPMI/20%FCS/10%DMSO), frozen at controlled rate of -1°/min in 1.8 mL cryo-vials with cell densities of 1E+07 to 1E+08 cells per vial, and stored at -150°C or below until further use. Frozen cells were thawed and cultured in medium at a starting density of 3.5E+05 cells/mL for 6 days. On day 6 cells were centrifuged for 5 min at 300 × g, medium was decanted and cell concentration was adjusted to 5.0E+06 cells/mL with fresh assay medium (500 mL RPMI (Gibco, # 22400) + 5 mL L-Glutamin (Sigma, #G7513) + 5 mL Penicillin / Streptomycin (Sigma #P0781) + 5 mL Non-essential amino acids (Invitrogen, #11140) + 5 mL sodium-pyruvate (Gibco #1136088), 5 mL FBS (Biochrom, #S0615)). Cell working stock was split in two parts: neutral control and compounds with EC30 stimulation, high control with EC100 stimulation.

An antibody premix was prepared by diluting anti-CD3 (BD Pharmingen, #555329), anti-CD28 (BD Pharmingen, #555725) and goat anti mouse anti-IgG (ThermoFisher, #31160) antibodies at 1/1/4 ratio in assay medium at 2-fold of final concentration (final concentrations depend on cell batch, typically for neutral control 0.055/0.055/0.22 μg/mL, for high control 0.5/0.5/2 mg/mL). The premix solutions were added to the cells in 1+1 volume prior use.

Fifty nL of a 100-fold concentrated solution of the test compounds in DMSO were transferred into a white microtiter test plate (384, Greiner Bio-One, Germany). For this, either a Hummingbird liquid handler (Digilab, USA) or an Echo acoustic system (Labcyte, USA) was used. Five μL of the freshly prepared cell suspension was added to the wells of a test plate and incubated at 37°C in a 5% $CO_2$ atmosphere. After completion of the incubation for 4 hours, 3 μl of Bio-Glo Luciferase assay reagent (Promega, #G7941, prepared as recommended by the supplier) were added to all wells. The test plate was incubated at 20°C for 10 min before measurement of the luminescence in a microplate reader (typically Pherastar by BMG, Germany, or ViewLux by Perkin-Elmer, USA). Data were normalized (neutral control = 0% effect, high control = 100% effect). Compounds were tested in duplicates at up to 11 concentrations (typically 20 μM, 5,7 μM, 1,6 μM, 0,47 μM, 0,13 μM, 38 nM, 11 nM, 3,1 nM, 0,89 nM, 0,25 nM and 0,073 nM). Dilution series were made prior to the assay in a 100-fold concentrated form by serial dilution. $EC_{50}$ values were calculated by 4-Parameter fitting using a commercial software package (Genedata Analyzer, Switzerland).

**Polyclonal activation of human PBMCs**

To test the effect of DGKζ inhibitors of the present invention on IL-2 and IFN-γ secretion of human Peripheral Blood Mononuclear Cells (PBMCs) a 24h human PBMC assay was performed as screening assay. For this, a 96 well flat bottom plate was coated with a suboptimal stimulation condition (EC 10-30) of human aCD3 (Invitrogen, clone OKT3) antibody in 50 μL PBS/well at 4°C overnight. PBMCs isolated and frozen at liquid $N_2$ from leucapherese samples was thawed and resuspended in culture medium (X-Vivo-20). $4 \times 10^5$ cells/well were plated. Wells were treated with the DGKζ inhibitors of the present invention at the respective concentrations (5-fold dilution steps from 10 μM to 3 nM) and the final DMSO concentration per well is 0.1%. Medium+ DMSO (0.1%) was used as baseline value. As positive controls 1000 ng/mL aCD3 + aCD28 (1 μg/mL) and a DGKζ reference inhibitor was used. After 24 h the medium was collected and hIL-2

or hIFN-γ ELISA were performed. The following parameters were calculated: $EC_{50}$ value, concentration at 50% increase; max increase in % and respective concentration and maximum effect normalized to max concentration (10 μM) of a selected DGKζ reference inhibitor.

### *In vitro* activation of mouse OT-I antigen-specific T-cells

To test the effect of DGKζ inhibitors of the present invention in murine antigen-specific T-cells, spleens and lymph nodes of OT-I mice were collected and mashed through a 40 μm cell strainer and incubated for 1 min in 1 mL ACK lysing buffer (Gibco)/spleen. 4x106 cells/mL were incubated in medium containing 0.05 ng/mL SIINFEKL (figure 2) in a 50 mL falcon at 37°C for 30 min. Afterwards cells were centrifuged and 4x106 cells/mL were resuspended in fresh medium (DMEM; 10% FCS, 1% Pen/Strep, 0.1% β-mercaptoethanol, 1% HEPES). 4x105 cells were plated per well in a 96-well round bottom plate. Wells were treated with DGKζ inhibitors of the present invention at the respective concentrations (5-fold dilution steps from 10 μM to 3 nM) in a final DMSO concentration of 0.1%. Medium + DMSO (0.1%) was used as baseline value. As positive controls cells incubated with the 4x SIINFEKL concentration (0.2ng/ml) and a DGKζ reference inhibitor were used. The plates were centrifuged to reduce the distance between T-cells and APCs before incubation. After 24 h the medium was collected and mIL-2 or mIFN-γ ELISAs were performed. The following parameters were calculated: $EC_{50}$ value, concentration at 50% increase; max increase in % and respective concentration and maximum effect normalized to max concentration (10 μM) of a selected DGKζ reference inhibitor.

### DGKζ Surface Plasmon Resonance Interaction Assay

The ability of the compounds described in this invention to bind to DGKζ were determined using surface plasmon resonance (SPR). This allows for the quantification of binding in terms of the equilibrium dissociation constant ($K_D$ [M]), as well as association and dissociation rate constants ($k_{on}$ [1/Ms] and $k_{off}$ [1/s], respectively). The measurements were performed using Biacore® T200, Biacore® S200 or Biacore® 8K (GE Healthcare).

All buffers described in this section were prepared with 10 × HBS-P+ Buffer (GE Healthcare, #BR100671) supplemented with additional buffer components as indicated below, dithiothreitol (DTT from Sigma, #D0632-25G), Adenosine 5'-triphosphate (ATP from Sigma, #A26209-10G), $MgCl_2$ (Sigma, #M1028-100ML), dimethyl sulfoxide (DMSO from Biomol, #54686.500).

For SPR measurements, recombinant and biotinylated human DGKζ (obtained from Carna Biosciences, Product number: 12-410-20N) was immobilized via the streptavidin-biotin interaction onto a Series S Sensor Chip SA (GE Healthcare, # BR-1005-31). Briefly, DGKζ was diluted to a concentration of 10 μg/mL in Immobilization Buffer (10 mM HEPES, 150 mM NaCl, 0.05% v/v Surfactant P20, 2 mM $MgCl_2$, 1 mM DTT, pH 7.4) and captured on the SA Chip surface using a flow rate of 10 μL/min for 500 seconds at a temperature of 10°C. Immobilization levels of approximately 6000 RU were typically achieved. The reference surface consisted of a streptavidin surface without immobilized protein. Compounds were diluted from 10 mM DMSO stock solution into Running Buffer (10 mM HEPES, 150 mM NaCl, 0.05% v/v Surfactant P20, 2 mM $MgCl_2$, 1 mM DTT, 0.2 mM ATP and 1% v/v DMSO, pH 7.4). For SPR-binding measurements serial dilutions (typically 1:3 dilutions resulting in 8 concentrations up to 2 μM or 20 μM) were injected over immobilized protein. Binding affinity and kinetics were measured at 18°C and at a flow rate of 100 μL/min.

A variation of the assay with an additional regeneration step was performed by injection of Regeneration Buffer without ATP (10 mM HEPES, 150 mM NaCl, 0.05% v/v Surfactant P20, 1 mM DTT and 1% v/v DMSO, pH 7.4) for 200 s at a flow rate of 30 μL/min

The double-referenced sensorgrams were fit to a simple reversible Langmuir 1:1 reaction mechanism as implemented in the Biacore® T200, S200 and 8K evaluation software (Biacore T200 Evaluation Software version 2.0, Biacore S200 Evaluation Software version 1.0, Biacore 8K Evaluation Software, GE Healthcare).

### Expression of DGKζ in insect cells using the Baculovirus system

Expression constructs:

The cDNA encoding the full length sequence of human DGKζ (Uniprot **Q13574-2)** was optimized for expression in eukaryotic cells and synthesized by the GeneArt Technology at Life Technologies.

The DNA sequence encoded the following sequence:

Construct DGKζ_hu amino acid M1 to V928

Additionally the expression construct encoded: a Kozak DNA sequence for translation initiation (GCCACC), a translational start codon for methionine followed by amino acid glycine, a Flag (DYKDDDDK) sequence at the N-terminus of DGKζ, and at the C-terminus of DGKζ two stop codons and moreover 5' and 3' att-DNA sequences for Gateway Cloning.

The DGKζ construct was subcloned using the Gateway Technology into the Destination vector pD-INS. The

vector pD-INS is a Baculovirus transfer vector (based on vector pVL1393, Pharmingen) which enables the expression of the Flag-DGKζ protein. The respective protein was named DGKz_hu_1.

Generation of recombinant Baculovirus

The DGKζ transfer vector was co-transfected in Sf9 cells with Baculovirus DNA (Flashbac Gold DNA, Oxford Expression Technologies) using Fugene HD (Roche). After 5 days the supernatant of the transfected cells containing the recombinant Baculovirus encoding the various DGK proteins was used for further infection of Sf9 cells for virus amplification whereby the virus titer was monitored using qPCR.

DGKζ expression in Sf9 cells using bioreactor

Sf9 cells cultured (Insect-xpress medium, Lonza, 27 °C) in a Wave-bioreactor with a disposable culture bag were infected at a cell density of $10^6$ cells/mL with one of the recombinant baculovirus stocks at a multiplicity of infection of 1 and incubated for 72. Subsequently, the cells were harvested by centrifugation (800 xg) and the cell pellet frozen at -80 °C.

Purification of the DGKz_hu_1 protein:

Purification of the DGKz_hu_1 protein was achieved by a two-step chromatography procedure as follows.

The pelleted cells (from 8 L cell culture) were resuspended in Lysis-Buffer (25 mM Tris HCl 8.0; 500 mM NaCl; 250 mM Sucrose, 1 mM DTT; 0.1 % Triton X-100; Complete Protease Inhibitor Cocktail-(Roche)) and lysed by a freeze-thaw cycle followed by an incubation on ice for 60 min in the presence of Benzonase (25 U/mL). The lysate was centrifuged at 63.000 xg for 30 min at 4 °C. The soluble supernatant was than incubated with 40 mL anti-Flag M2 Agarose (Sigma) in a plastic flask rotating for 16 h at 4 °C for binding of the tagged DGKζ proteins, subsequently rinsed with 5 × 50 mL Wash-Buffer (25 mM Tris HCl 8.0; 500 mM NaCl; 250 mM Sucrose; 1 mM DTT) and finally the bound protein was eluted using Elution-Buffer (Wash-Buffer with 250 μg/mL FLAG-Peptide, incubated 30 min. at 4 °C with 3 x25 mL).

The elution fractions from the affinity chromatography were concentrated (using Amicon Ultra 15, Centrifugal Filters, 30 kDa MW cut-off; Millipore #UFC903024) to 25 mL and applied to a size exclusion chromatography column (S200 prep grade 26/60, GE Healthcare) and the resulting monomeric peak fraction was collected, pooled and again concentrated. Wash-buffer with 300 mM NaCl was used for size exclusion chromatography and the final concentrated sample. The final protein sample concentration was 5 to 10 mg/mL and the yield was 5 mg final protein per L cell culture.

The *in vivo* activity of the compounds of the present invention can be demonstrated in the following assays:

### *In vivo* activation of murine antigen specific OT-I T cells

Oral Administration of compounds enhances antigen-specific T cell activation *in vivo.*

Direct detection of antigen-specific T cell proliferation *in vivo* is technically challenging, since it requires the presence of T cells specific for a cognate antigen and also a specific measurement procedure for cell proliferation. Both these requirements are fulfilled in the OT-I transfer model, which utilizes the direct transfer of CD8 T cells transgenic for a T cell receptor recognizing an Ovalbumin-derived peptide as antigen.

Before transfer, the OT-I T cells were labeled with the fluorescent dye CFSE, which was diluted by every cell division and therefore allowed detection of cell proliferation. After transfer of the CFSE-labeled T cells, mice were vaccinated with the Ovalbumin antigen OVA-30 (figure 3). Only transferred OT-I cells were able to recognize the OVA-antigen presented by APC and only these transferred T cells then got activated. Flow cytometric analysis of CFSE-levels in the OT-I cells can be combined with measurement of multiple activation markers like CD69, CD25 and PD1. In particular, Wild type C57Bl6 mice received 2x10x6 CFSE-labeled OT-I T cells and were vaccinated one day later by intravenous application of 2.5 μg OVA-30. Mice were then divided into groups which received vehicle only, DGKζ inhibitors of the present invention alone or in combination with other immune modulating agents. Mice were treated for 2 to 20 days and T cell composition (incl. transferred OT-1 cells) of spleen, blood and lymph nodes were analysed by FACS.

### *In vivo* syngeneic tumor models

Mice were assigned to a study at the age of 6-8 weeks. Animal husbandry, feeding and health conditions were according to animal welfare guidelines. Syngeneic tumor cell lines were cultivated with appropriate medium and split at least 3 times before inoculation. Female mice were inoculated with appropriate amount of tumor cells in medium or a medium /matrigel mixture s.c, i.v., or i.p, depending on the model. After 4-10 days the mice were randomized and therapeutic treatment started when tumors had reached a size of approx. 40-70mm$^2$.

Tumor size was measured using calipers determining length (a) and width (b). Tumor volume was calculated

according to:

$$v=(a \times b^2)/2$$

Significance of monotherapies and combination treatment was calculated *versus* control group as determined by 2-Way ANOVA analysis.

## Claims

1. A compound of general formula (I):

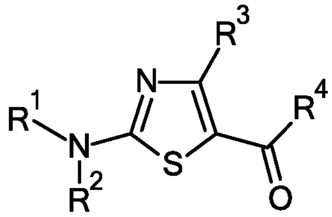

(I)

in which:

$R^1$ represents a phenyl or 6-membered heteroaryl group optionally substituted, one, two, or three times, each substituent independently selected from a halogen atom or a group selected from hydroxy, cyano, nitro, $C_1$-$C_6$-alkyl, (phenyl)-($C_1$-$C_3$-alkyl)-, $C_1$-$C_6$-haloalkyl, $C_1$-$C_6$-alkoxy, (phenyl)-($C_1$-$C_3$-alkoxy)-, $C_1$-$C_6$-haloalkoxy, -N($R^5$)($R^6$),

wherein the phenyl groups in said (phenyl)-($C_1$-$C_3$-alkyl)- and (phenyl)-($C_1$-$C_3$-alkoxy)-groups are optionally substituted one or two times, each substituent independently selected from a halogen atom or a group selected from cyano, methyl, ethyl, trifluoromethyl, methoxy, ethoxy, dimethylamino and trifluoromethoxy, or two substituents attached to adjacent carbon atoms of said phenyl or 6-membered heteroaryl group together form a bivalent group selected from $-(CH_2)_3-$, $-(CH_2)_4-$, - $(CH_2)_2$-O-, $-(CH_2)_3$-O-, $-CH_2$-O-$CH_2$-, $-(CH_2)_2$-O-$CH_2$-, -O-$CH_2$-O-, -O-$CH_2$-$CH_2$-O-, -O-$CF_2$-O-, -O-$CH_2$-$CF_2$-O-, and -O-$CF_2$-$CF_2$-O-,

or

$R^1$ represents a 5-membered heteroaryl group optionally substituted one or two times, each substituent independently selected from a halogen atom or a group selected from cyano, $C_1$-$C_3$-alkyl, and $C_1$-$C_3$-alkoxy;

$R^2$ represents a group

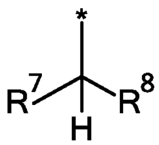

,

wherein "*" indicates the point of attachment to the nitrogen atom to which $R^2$ is attached;

$R^3$ represents a group selected from methyl and $-NH_2$;

$R^4$ represents a 5-membered heteroaryl group optionally substituted one or two times, each substituent independently selected from a halogen atom or a group selected from $C_1$-$C_6$-alkyl, (($R^9$)O)-($C_1$-$C_3$-alkyl)-, (($R^{10}$)($R^{11}$)N)-($C_1$-$C_3$-alkyl)-, ($C_3$-$C_7$-cycloalkyl)-($C_4$-$C_3$-alkyl)-, $C_3$-$C_7$-cycloalkyl, -O$R^9$, -N($R^{10}$)($R^{11}$), -C(=O)-N($R^{15}$)($R^{16}$), -C(=O)-O$R^{17}$, phenyl and a 5- or 6-membered heteroaryl group,

wherein the phenyl group and the 5- or 6-membered heteroaryl group are optionally substituted one or two times, each substituent independently selected from a halogen atom or a group selected from cyano, $C_1$-$C_3$-alkyl, $C_1$-$C_3$-haloalkyl, (($R^{22}$)($R^{23}$)N)-$C_1$-$C_3$-alkyl, -O$R^9$, -N($R^{10}$)($R^{11}$) and -C(=O)-N($R^{15}$)($R^{16}$);

$R^5$ and $R^6$ represent, independently from each occurrence, a hydrogen atom or a group selected from $C_1$-$C_4$-alkyl, ($C_1$-$C_4$-alkyl)-C(=O)-, $C_3$-$C_4$-cycloalkyl and (phenyl)-($C_1$-$C_3$-alkyl)-, or

$R^5$ and $R^6$, together with the nitrogen atom to which they are attached, represent a monocyclic nitrogen containing 4- to 7-membered heterocycloalkyl group which is optionally substituted one, two or three times, each substituent independently selected from a halogen atom or a group selected from oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy;
$R^7$ represents a hydrogen atom or a $C_1$-$C_2$-alkyl group;
$R^8$ represents a group selected from -C(=O)-$NH_2$ and -S(=O)$_2$-$NH_2$;
$R^9$ represents a hydrogen atom or a group selected from $C_1$-$C_6$-alkyl, ($C_3$-$C_7$-cycloalkyl)-($C_1$-$C_3$-alkyl)-, (phenyl)-($C_1$-$C_3$-alkyl)-, (5- or 6-membered heteroaryl)-($C_1$-$C_3$-alkyl)-, $C_1$-$C_6$-haloalkyl, $C_2$-$C_4$-hydroxyalkyl, ($C_1$-$C_3$-alkoxy)-$C_2$-$C_3$-alkyl-, (($C_1$-$C_3$-alkyl)-C(=O)-O)-$C_2$-$C_3$-alkyl-, -C($R^{18}$)($R^{19}$)-C(=O)-O$R^{17}$, -C($R^{18}$)($R^{19}$)-C(=O)-N($R^{20}$)($R^{21}$), -C(=O)-N($R^{20}$)($R^{21}$), $C_3$-$C_7$-cycloalkyl, phenyl and a 5- or 6-membered heteroaryl group,

> wherein $C_3$-$C_7$-cycloalkyl, and the $C_3$-$C_7$-cycloalkyl within said ($C_3$-$C_7$-cycloalkyl)-($C_1$-$C_3$-alkyl)- group is optionally substituted one or two times, each substituent independently selected from a fluorine atom or a group selected from oxo, cyano, $C_1$-$C_2$-alkyl and $C_1$-$C_2$-haloalkyl,
> and wherein the phenyl group within said (phenyl)-($C_1$-$C_3$-alkyl)- group and said phenyl group itself, and the 5- or 6-membered heteroaryl group within said (5- or 6-membered heteroaryl)-($C_1$-$C_3$-alkyl)- group and said 5- or 6-membered heteroaryl group itself, are optionally substituted one or two times, each substituent independently selected from a halogen atom or a group selected from cyano, methyl, ethyl, trifluoromethyl, methoxy, ethoxy, dimethylamino and trifluoromethoxy;

$R^{10}$ and $R^{11}$ represent, independently from each occurrence, a hydrogen atom or a group selected from $C_1$-$C_4$-alkyl, $C_1$-$C_4$-haloalkyl, $C_2$-$C_4$-hydroxyalkyl, ($C_1$-$C_3$-alkoxy)-$C_2$-$C_3$-alkyl-, (($R^{22}$)($R^{23}$)N)-$C_2$-$C_3$-alkyl, ($C_3$-$C_7$-cycloalkyl)-($C_1$-$C_3$-alkyl)-, ($C_1$-$C_4$-alkyl)-C(=O)-, $C_3$-$C_7$-cycloalkyl, ($C_3$-$C_7$-cycloalkyl)-C(=O)-, (phenyl)-($C_1$-$C_3$-alkyl)-, (phenyl)-($C_1$-$C_3$-alkyl)-C(=O)-, (phenyl)-($C_1$-$C_3$-alkyl)-O-C(=O)-, phenyl and a 5- or 6-membered heteroaryl group,

> wherein $C_3$-$C_7$-cycloalkyl, and the $C_3$-$C_7$-cycloalkyl within said ($C_3$-$C_7$-cycloalkyl)-($C_1$-$C_3$-alkyl)- and ($C_3$-$C_7$-cycloalkyl)-C(=O)- groups are optionally substituted one or two times, each substituent independently selected from a fluorine atom or a group selected from cyano, $C_1$-$C_2$-alkyl and $C_1$-$C_2$-haloalkyl,
> nd wherein said phenyl and said 5- or 6-membered heteroaryl group, and the phenyl groups within said (phenyl)-($C_1$-$C_3$-alkyl)-, (phenyl)-($C_1$-$C_3$-alkyl)-C(=O)- and (phenyl)-($C_1$-$C_3$-alkyl)-O-C(=O)- groups, are optionally substituted one or two times, each substituent independently selected from a halogen atom or a group selected from cyano, methyl, ethyl, trifluoromethyl, methoxy, ethoxy, dimethylamino and trifluoromethoxy,

a or
$R^{10}$ and $R^{11}$, together with the nitrogen atom to which they are attached, represent a monocyclic nitrogen containing 4- to 7-membered heterocycloalkyl group, or a bicyclic nitrogen containing 5- to 11-membered heterocycloalkyl group, which are optionally substituted one, two or three times, each substituent independently selected from a halogen atom or a group selected from cyano, oxo, hydroxy, $C_1$-$C_4$-alkyl, $C_1$-$C_4$-haloalkyl, ($C_1$-$C_4$-alkyl)-C(=O)-, $C_3$-$C_7$-cycloalkyl, $C_1$-$C_4$-alkoxy, -N($R^{22}$)($R^{23}$), and a monocyclic 4- to 7-membered heterocycloalkyl group;
$R^{15}$ and $R^{16}$ represent, independently from each occurrence, a hydrogen atom or a group selected from $C_1$-$C_4$-alkyl, $C_1$-$C_4$-haloalkyl, $C_1$-$C_4$-hydroxyalkyl, (cyano)-$C_1$-$C_4$-alkyl-, ($C_1$-$C_4$-alkoxy)-$C_2$-$C_3$-alkyl-, ($C_1$-$C_4$-haloalkoxy)-$C_2$-$C_3$-alkyl-, (phenoxy)-$C_2$-$C_3$-alkyl-, ($C_3$-$C_7$-cycloalkyl)-($C_1$-$C_3$-alkyl)-, $C_3$-$C_7$-cycloalkyl, bicyclic $C_5$-$C_{11}$-cycloalkyl, monocyclic 4- to 7-membered heterocycloalkyl, (phenyl)-($C_1$-$C_3$-alkyl)-, phenyl and a 5- or 6-membered heteroaryl group, wherein $C_3$-$C_7$-cycloalkyl, the $C_3$-$C_7$-cycloalkyl within said ($C_3$-$C_7$-cycloalkyl)-($C_1$-$C_3$-alkyl)- group, the bicyclic $C_5$-$C_{11}$-cycloalkyl and the monocyclic 4- to 7-membered heterocycloalkyl groups are optionally substituted one, two or three times, each substituent independently selected from a halogen atom or a group selected from oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy, and wherein the phenyl and the 5- or 6-membered heteroaryl groups, including the phenyl groups within said (phenoxy)-$C_2$-$C_3$-alkyl- and (phenyl)-($C_1$-$C_3$-alkyl)- groups, are optionally substituted one or two times, each substituent independently selected from a halogen atom or a group selected from cyano, methyl, ethyl, trifluoromethyl, methoxy, ethoxy, dimethylamino and trifluoromethoxy, or
$R^{15}$ and $R^{16}$, together with the nitrogen atom to which they are attached, represent a monocyclic nitrogen containing 4- to 7-membered heterocycloalkyl group which is optionally substituted one, two or three times, each substituent independently selected from a halogen atom or a group selected from oxo, hydroxy, $C_1$-$C_4$-alkyl,

($C_1$-$C_4$-alkyl)-C(=O)-, $C_3$-$C_4$-cycloalkyl and $C_1$-$C_4$-alkoxy;
$R^{17}$ represents a $C_1$-$C_4$-alkyl group;
$R^{18}$ and $R^{19}$ represent, independently from each occurrence, a hydrogen atom or a $C_1$-$C_4$-alkyl group;
$R^{20}$ represents a hydrogen atom or a group selected from $C_1$-$C_6$-alkyl, $C_3$-$C_4$-alkenyl, $C_3$-$C_4$-alkynyl, $C_1$-$C_3$-alkoxy, $C_3$-$C_7$-cycloalkyl, bicyclic $C_5$-$C_{11}$-cycloalkyl, adamantyl, monocyclic 4- to 7-membered heterocycloalkyl, bicyclic 5- to 11-membered heterocycloalkyl, phenyl, naphthyl, and 5- to 10-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, bicyclic $C_5$-$C_{11}$-cycloalkyl, adamantyl, monocyclic 4- to 7-membered heterocycloalkyl, bicyclic 5- to 11-membered heterocycloalkyl, phenyl, and 5- to 10-membered heteroaryl, said phenyl and 5- to 10-membered heteroaryl substituents themselves being optionally substituted one or two times, each substituent independently selected from a halogen atom or a group selected from cyano, methyl, ethyl, trifluoromethyl, methoxy, ethoxy, dimethylamino and trifluoromethoxy,
and wherein $C_3$-$C_7$-cycloalkyl, bicyclic $C_5$-$C_{11}$-cycloalkyl, adamantyl, monocyclic 4-to 7-membered heterocycloalkyl and bicyclic 5- to 11-membered heterocycloalkyl are optionally substituted one, two or three times, each substituent independently selected from a halogen atom or a group selected from cyano, oxo, hydroxy, $C_1$-$C_4$-alkyl, ($C_1$-$C_4$-alkyl)-C(=O)-, $C_3$-$C_4$-cycloalkyl and $C_1$-$C_4$-alkoxy,
and wherein said phenyl, naphthyl and 5- to 10-membered heteroaryl groups are optionally substituted one, two or three times, each substituent independently selected from a halogen atom or a group selected from cyano, $C_1$-$C_4$-alkyl, $C_1$-$C_4$-haloalkyl, $C_1$-$C_4$-alkoxy, $C_1$-$C_4$-haloalkoxy, -N($R^{22}$)($R^{23}$) and -C(=O)-N($R^{24}$)($R^{25}$),

$R^{21}$ represents a hydrogen atom or a $C_1$-$C_4$-alkyl group, or
$R^{20}$ and $R^{21}$, together with the nitrogen atom to which they are attached, represent a monocyclic nitrogen containing 4- to 7-membered heterocycloalkyl group which is optionally benzocondensed, and which is optionally substituted one, two or three times, each substituent independently selected from a halogen atom or a group selected from cyano, oxo, hydroxy, $C_1$-$C_4$-alkyl, $C_1$-$C_4$-haloalkyl, (phenyl)-($C_1$-$C_3$-alkyl)-, ($C_1$-$C_4$-alkyl)-C(=O)-, $C_3$-$C_4$-cycloalkyl, $C_1$-$C_4$-alkoxy, $C_1$-$C_3$-haloalkoxy, -N($R^{22}$)($R^{23}$) and -C(=O)-N($R^{24}$)($R^{25}$);
$R^{22}$ and $R^{23}$ represent, independently from each occurrence, a hydrogen atom or a group selected from $C_1$-$C_2$-alkyl and ($C_1$-$C_2$-alkyl)-C(=O)-, and
$R^{24}$ and $R^{25}$ represent, independently from each occurrence, a hydrogen atom or a $C_1$-$C_4$-alkyl group,
or a stereoisomer, a tautomer, an N-oxide, a hydrate, a solvate, or a salt thereof, or a mixture of same.

**2.** The compound according to claim 1, wherein:

$R^1$ represents a phenyl or pyridinyl group optionally substituted, one, two, or three times, each substituent independently selected from a halogen atom or a group selected from hydroxy, cyano, nitro, $C_1$-$C_4$-alkyl, (phenyl)-($C_1$-$C_2$-alkyl)-, $C_1$-$C_4$-haloalkyl, $C_1$-$C_4$-alkoxy, (phenyl)-($C_1$-$C_2$-alkoxy)-, $C_1$-$C_4$-haloalkoxy, -N($R^5$)($R^6$), wherein the phenyl groups in said (phenyl)-($C_1$-$C_2$-alkyl)- and (phenyl)-($C_1$-$C_2$-alkoxy)-groups are optionally substituted one or two times, each substituent independently selected from a fluorine atom, a chlorine atom and a bromine atom, or a group selected from methyl, trifluoromethyl and methoxy, or two substituents attached to adjacent carbon atoms of said phenyl or pyridinyl group together form a bivalent group selected from $-(CH_2)_3-$, $-(CH_2)_4-$, $-(CH_2)_2-O-$, $-(CH_2)_3-O-$, $-CH_2-O-CH_2-$, $-O-CH_2-O-$, $-O-CH_2-CH_2-O-$ and $-O-CF_2-O-$, or
$R^1$ represents a pyrazolyl group optionally substituted one or two times, each substituent independently selected from a halogen atom or a group selected from cyano, $C_1$-$C_2$-alkyl, and $C_1$-$C_2$-alkoxy;
$R^2$ represents a group

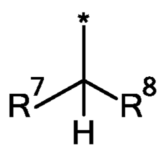

,

wherein "*" indicates the point of attachment to the nitrogen atom to which $R^2$ is attached;
$R^3$ represents a group selected from methyl and $-NH_2$;
$R^4$ represents a 5-membered heteroaryl group selected from oxazolyl, isoxazolyl, thiazolyl, isothiazolyl and

oxadiazolyl, optionally substituted one or two times, each substituent independently selected from a fluorine atom, a chlorine atom and a bromine atom, or a group selected from $C_1$-$C_4$-alkyl, (($R^9$)O)-($C_1$-$C_3$-alkyl)-, (($R^{10}$)($R^{11}$)N)-($C_1$-$C_3$-alkyl)-, ($C_3$-$C_7$-cycloalkyl)-($C_1$-$C_3$-alkyl)-, $C_3$-$C_7$-cycloalkyl, -$OR^9$, -N($R^{10}$)($R^{11}$), -C(=O)-N($R^{15}$)($R^{16}$), - C(=O)-$OR^{17}$, phenyl and a 5- or 6-membered heteroaryl group, wherein the phenyl group and the 5- or 6-membered heteroaryl group are optionally substituted one or two times, each substituent independently selected from a fluorine atom, a chlorine atom and a bromine atom, or a group selected from cyano, $C_1$-$C_2$-alkyl, $C_1$-$C_2$-fluoroalkyl, -$OR^9$, -N($R^{10}$)($R^{11}$) and -C(=O)-N($R^{15}$)($R^{16}$);

$R^5$ and $R^6$ represent, independently from each occurrence, a hydrogen atom or a group selected from $C_1$-$C_2$-alkyl and ($C_1$-$C_2$-alkyl)-C(=O)-, or

$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom, a chlorine atom and a bromine atom, or a group selected from oxo, hydroxy, $C_1$-$C_2$-alkyl and ($C_1$-$C_2$-alkyl)-C(=O)-;

$R^7$ represents a hydrogen atom or a $C_1$-$C_2$-alkyl group;

$R^8$ represents a -C(=O)-$NH_2$ group;

$R^9$ represents a hydrogen atom or a group selected from $C_1$-$C_4$-alkyl, ($C_3$-$C_7$-cycloalkyl)-($C_1$-$C_2$-alkyl)-, (phenyl)-($C_1$-$C_2$-alkyl)-, $C_1$-$C_4$-haloalkyl, $C_2$-$C_3$-hydroxyalkyl, ($C_1$-$C_2$-alkoxy)-$C_2$-alkyl-, (($C_1$-$C_2$-alkyl)-C(=O)-O)-$C_2$-alkyl-, -C(=O)-N($R^{20}$)($R^{21}$), $C_3$-$C_7$cycloalkyl and phenyl, wherein $C_3$-$C_7$-cycloalkyl, and the $C_3$-$C_7$-cycloalkyl within said ($C_3$-$C_7$-cycloalkyl)-($C_1$-$C_2$-alkyl)- group is optionally substituted one or two times, each substituent independently selected from a fluorine atom or a group selected from oxo and methyl, and wherein the phenyl group within said (phenyl)-($C_1$-$C_2$-alkyl)- group and said phenyl group itself are optionally substituted one or two times, each substituent independently selected from a fluorine atom, a chlorine atom and a bromine atom, or a group selected from cyano, methyl, trifluoromethyl and methoxy;

$R^{10}$ and $R^{11}$ represent, independently from each occurrence, a hydrogen atom or a group selected from $C_1$-$C_2$-alkyl, $C_1$-$C_2$-haloalkyl, $C_2$-$C_3$-hydroxyalkyl, ($C_1$-$C_2$-alkoxy)-$C_2$-alkyl-, (($R^{22}$)($R^{23}$)N)-$C_2$-alkyl, ($C_3$-$C_7$-cycloalkyl)-($C_1$-$C_2$-alkyl)-, ($C_1$-$C_2$-alkyl)-C(=O)-, $C_3$-$C_7$-cycloalkyl, ($C_3$-$C_7$-cycloalkyl)-C(=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,

> wherein $C_3$-$C_7$-cycloalkyl, and the $C_3$-$C_7$-cycloalkyl within said ($C_3$-$C_7$-cycloalkyl)-($C_1$-$C_2$-alkyl)- and ($C_3$-$C_7$-cycloalkyl)-C(=O)- groups are optionally substituted one or two times, each substituent independently selected from a fluorine atom or a group selected from cyano, $C_1$-$C_2$-alkyl and $C_1$-$C_2$-haloalkyl,
> and wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a bromine atom, or a group selected from methyl, trifluoromethyl and methoxy,

or

$R^{10}$ and $R^{11}$, together with the nitrogen atom to which they are attached, represent a monocyclic nitrogen containing 4- to 7-membered heterocycloalkyl group, or a bicyclic nitrogen containing 5- to 10-membered heterocycloalkyl group, which are optionally substituted one, two or three times, each substituent independently selected from a halogen atom or a group selected from cyano, oxo, hydroxy, $C_1$-$C_2$-alkyl, $C_1$-$C_2$-haloalkyl, ($C_1$-$C_2$-alkyl)-C(=O)-, $C_1$-$C_2$-alkoxy, -N($R^{22}$)($R^{23}$), and a monocyclic 4- to 7-membered heterocycloalkyl group;

$R^{15}$ and $R^{16}$ represent, independently from each occurrence, a hydrogen atom or a group selected from $C_1$-$C_4$-alkyl, $C_1$-$C_4$-haloalkyl, $C_1$-$C_4$-hydroxyalkyl, (cyano)-$C_1$-$C_4$-alkyl-, ($C_1$-$C_4$-alkoxy)-$C_2$-$C_3$-alkyl-, ($C_1$-$C_4$-haloalkoxy)-$C_2$-$C_3$-alkyl-, ($C_3$-$C_7$-cycloalkyl)-($C_1$-$C_3$-alkyl)-, $C_3$-$C_7$-cycloalkyl, bicyclic $C_5$-$C_{11}$-cycloalkyl, monocyclic 4- to 7-membered heterocycloalkyl, (phenyl)-($C_1$-$C_3$-alkyl)-, phenyl and a 5- or 6-membered heteroaryl group, wherein $C_3$-$C_7$-cycloalkyl, the $C_3$-$C_7$-cycloalkyl within said ($C_3$-$C_7$cycloalkyl)-($C_1$-$C_3$-alkyl)- group, the bicyclic $C_5$-$C_{11}$-cycloalkyl and the monocyclic 4- to 7-membered heterocycloalkyl groups are optionally substituted one or two times, each substituent independently selected from a halogen atom or a group selected from oxo, $C_1$-$C_2$-alkyl, ($C_1$-$C_2$-alkyl)-C(=O)- and cyclopropyl, and wherein the phenyl and the 5- or 6-membered heteroaryl groups, including the phenyl group within said (phenyl)-($C_1$-$C_3$-alkyl)- group, are optionally substituted one or two times, each substituent independently selected from a fluorine atom, a chlorine atom and a bromine atom, or a group selected from methyl, trifluoromethyl and methoxy, or

$R^{15}$ and $R^{16}$, together with the nitrogen atom to which they are attached, represent a monocyclic nitrogen containing 4- to 7-membered heterocycloalkyl group which is optionally substituted one or two times, each substituent independently selected from a halogen atom or a group selected from oxo, $C_1$-$C_2$-alkyl and ($C_1$-$C_2$-alkyl)-C(=O)-;

$R^{17}$ represents a $C_1$-$C_4$-alkyl group;

$R^{20}$ represents a hydrogen atom or a group selected from $C_1$-$C_6$-alkyl, $C_3$-$C_7$-cycloalkyl, monocyclic 4- to 7-membered heterocycloalkyl, phenyl, and 5- to 6-membered heteroaryl,

wherein said $C_1$-$C_6$-alkyl group is optionally substituted one, two or three times, each substituent independently selected from a halogen atom or a group selected from hydroxy, cyano, $C_1$-$C_3$-alkoxy, -N($R^{22}$)($R^{23}$), $C_3$-$C_7$-cycloalkyl, monocyclic 4- to 7-membered heterocycloalkyl, phenyl, and 5- to 6-membered heteroaryl, said phenyl and 5- to 6-membered heteroaryl substituents themselves being optionally substituted one or two times, each substituent independently selected from a fluorine atom, a chlorine atom and a bromine atom, or a group selected from methyl, trifluoromethyl and methoxy,
and wherein $C_3$-$C_7$-cycloalkyl and monocyclic 4- to 7-membered heterocycloalkyl are optionally substituted one or two or three times, each substituent independently selected from a halogen atom or a group selected from cyano, oxo, hydroxy, $C_1$-$C_2$-alkyl and ($C_1$-$C_2$-alkyl)-C(=O)-,
and wherein said phenyl and 5- to 6-membered heteroaryl groups are optionally substituted one, two or three times, each substituent independently selected from a halogen atom or a group selected from cyano, $C_1$-$C_2$-alkyl, $C_1$-$C_2$-haloalkyl, $C_1$-$C_2$-alkoxy, $C_1$-$C_2$-haloalkoxy, -N($R^{22}$)($R^{23}$) and -C(=O)-N($R^{24}$)($R^{25}$),

$R^{21}$ represents a hydrogen atom or a $C_1$-$C_2$-alkyl group, or
$R^{20}$ and $R^{21}$, together with the nitrogen atom to which they are attached, represent a monocyclic nitrogen containing 4- to 7-membered heterocycloalkyl group which is substituted one, two or three times, each substituent independently selected from a halogen atom or a group selected from cyano, oxo, hydroxy, $C_1$-$C_2$-alkyl, $C_1$-$C_2$-haloalkyl, (phenyl)-($C_1$-$C_2$-alkyl)-, ($C_1$-$C_2$-alkyl)-C(=O)-, $C_3$-$C_4$-cycloalkyl, $C_1$-$C_2$-alkoxy, $C_1$-$C_2$-haloalkoxy, -N($R^{22}$)($R^{23}$) and -C(=O)-N($R^{24}$)($R^{25}$);
$R^{22}$ and $R^{23}$ represent, independently from each occurrence, a hydrogen atom or a group selected from $C_1$-$C_2$-alkyl and ($C_1$-$C_2$-alkyl)-C(=O)-, and
$R^{24}$ and $R^{25}$ represent, independently from each occurrence, a hydrogen atom or a $C_1$-$C_2$-alkyl group,
or a stereoisomer, a tautomer, an N-oxide, a hydrate, a solvate, or a salt thereof, or a mixture of same.

3. The compound according to claim 1 or 2, wherein:

$R^1$ represents a phenyl or pyridinyl group optionally substituted, one, two, or three times, each substituent independently selected from a fluorine atom, a chlorine atom and a bromine atom, or a group selected from hydroxy, cyano, $C_1$-$C_4$-alkyl, $C_1$-$C_2$-fluoroalkyl, $C_1$-$C_2$-alkoxy, (phenyl)-($C_1$-$C_2$-alkoxy)-, $C_1$-$C_2$-fluoroalkoxy and -N($R^5$)($R^6$),
or two substituents attached to adjacent carbon atoms of said phenyl or pyridinyl group together form a bivalent group selected from $-(CH_2)_3-$, $-O-CH_2-O-$ and $-O-CF_2-O-$, or
$R^1$ represents a pyrazolyl group optionally substituted with one methyl group,
$R^2$ represents a group

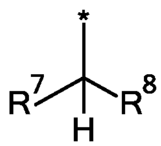

,

wherein "*" indicates the point of attachment to the nitrogen atom to which $R^2$ is attached;
$R^3$ represents a group selected from methyl and $-NH_2$;
$R^4$ represents a group selected from

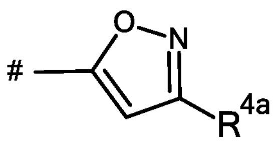 and 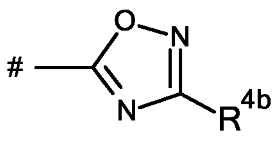

,

wherein "#" indicates the point of attachment to the carbonyl group to which $R^4$ is attached, and wherein
$R^{4a}$ and $R^{4b}$ represent, independently from each other, a fluorine atom, a chlorine atom or a bromine atom, or a group selected from $C_1$-$C_4$-alkyl, (($R^9$)O)-($C_1$-$C_2$-alkyl)-, (($R^{10}$)($R^{11}$)N)-($C_1$-$C_2$-alkyl)-, ($C_3$-$C_7$-cycloalkyl)-($C_1$-$C_2$-alkyl)-, $C_3$-$C_7$-cycloalkyl, $-OR^9$, -N($R^{10}$)($R^{11}$), - C(=O)-N($R^{15}$)($R^{16}$), $-C(=O)-OR^{17}$, phenyl and a 5-

or 6-membered heteroaryl group selected from oxazolyl, isoxazolyl, thiazolyl, isothiazolyl, pyrazolyl, pyridinyl, pyrazinyl, pyrimidinyl and pyridazinyl,
wherein the phenyl group and the 5- or 6-membered heteroaryl group selected from oxazolyl, isoxazolyl, thiazolyl, isothiazolyl, pyrazolyl, pyridinyl, pyrazinyl, pyrimidinyl and pyridazinyl are optionally substituted one or two times, each substituent independently selected from a fluorine atom and a chlorine atom, or a group selected from cyano, $C_1$-$C_2$-alkyl, -$OR^9$, -$N(R^{10})(R^{11})$ and -C(=O)-$N(R^{15})(R^{16})$;
$R^5$ and $R^6$ represent, independently from each occurrence, a hydrogen atom or a $C_1$-$C_2$-alkyl group,
or
$R^5$ and $R^6$, together with the nitrogen atom to which they are attached, represent a monocyclic nitrogen containing 4- to 7-membered heterocycloalkyl group which is optionally substituted one or two times, each substituent independently selected from a fluorine atom or a group selected from hydroxy and $C_1$-$C_2$-alkyl;
$R^7$ represents a hydrogen atom or a $C_1$-$C_2$-alkyl group;
$R^8$ represents a -C(=O)-$NH_2$ group;
$R^9$ represents a hydrogen atom or a group selected from $C_1$-$C_2$-alkyl, benzyl, $C_1$-$C_2$-fluoroalkyl, $C_2$-hydroxyalkyl, ($C_1$-$C_2$-alkoxy)-$C_2$-alkyl-, (($C_1$-$C_2$-alkyl)-C(=O)-O)-$C_2$-alkyl-, - C(=O)-$N(R^{20})(R^{21})$ and phenyl,
wherein the phenyl group within said benzyl group and said phenyl group itself are optionally substituted one or two times, each substituent independently selected from a fluorine atom and a chlorine atom, or a group selected from cyano and methyl;
$R^{10}$ and $R^{11}$ represent, independently from each occurrence, a hydrogen atom or a group selected from $C_1$-$C_2$-alkyl, $C_1$-$C_2$-fluoroalkyl, ($C_3$-$C_5$-cycloalkyl)-($C_1$-$C_2$-alkyl)- ($C_1$-$C_2$-alkyl)-C(=O)-, $C_3$-$C_7$-cycloalkyl, $C_3$-$C_7$-cycloalkyl-(C=O)-, (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)-,

wherein $C_3$-$C_7$-cycloalkyl, and the $C_3$-$C_5$-cycloalkyl within said ($C_3$-$C_5$-cycloalkyl)-($C_1$-$C_2$-alkyl)- and the $C_3$-$C_7$-cycloalkyl within the $C_3$-$C_7$-cycloalkyl-(C=O)- groups are optionally substituted one or two times, each substituent independently selected from a fluorine atom or a group selected from cyano, $C_1$-$C_2$-alkyl and $C_1$-$C_2$-fluoroalkyl,
and wherein the phenyl groups within said (phenyl)-($C_1$-$C_2$-alkyl)-, (phenyl)-($C_1$-$C_2$-alkyl)-C(=O)- and (phenyl)-($C_1$-$C_2$-alkyl)-O-C(=O)- groups are optionally substituted one or two times, each substituent independently selected from a fluorine atom, a chlorine atom and a methyl group,

or
$R^{10}$ and $R^{11}$, together with the nitrogen atom to which they are attached, represent a monocyclic nitrogen containing 4- to 7-membered heterocycloalkyl group which is optionally substituted one, two or three times, each substituent independently selected from a fluorine atom or a group selected from cyano, oxo, $C_1$-$C_2$-alkyl, $C_1$-$C_2$-fluoroalkyl and ($C_1$-$C_2$-alkyl)-C(=O)-;
$R^{15}$ and $R^{16}$ represent, independently from each occurrence, a hydrogen atom or a group selected from $C_1$-$C_4$-alkyl, $C_1$-$C_3$-fluoroalkyl, (cyano)-$C_1$-$C_2$-alkyl-, ($C_1$-$C_4$-alkoxy)-$C_2$-$C_3$-alkyl-, ($C_1$-$C_2$-fluoroalkoxy)-$C_2$-$C_3$-alkyl-, ($C_3$-$C_5$-cycloalkyl)-($C_1$-$C_2$-alkyl)-, $C_3$-$C_5$-cycloalkyl, phenyl and a 5- or 6-membered heteroaryl group selected from pyrazolyl, pyridinyl and pyrimidinyl,

wherein $C_3$-$C_5$-cycloalkyl, and the $C_3$-$C_5$-cycloalkyl within said ($C_3$-$C_5$-cycloalkyl)-($C_1$-$C_2$-alkyl)- group are optionally substituted one or two times, each substituent independently selected from a fluorine atom, or a group selected from methyl and cyclopropyl,
and wherein the phenyl and the 5- or 6-membered heteroaryl groups selected from pyrazolyl, pyridinyl and pyrimidinyl are optionally substituted one or two times, each substituent independently selected from a fluorine atom and a chlorine atom, or a group selected from methyl, trifluoromethyl and methoxy,

or
$R^{15}$ and $R^{16}$, together with the nitrogen atom to which they are attached, represent a monocyclic nitrogen containing 4- to 7-membered heterocycloalkyl group which is optionally substituted once with a fluorine atom or a group selected from oxo, $C_1$-$C_2$-alkyl and $CH_3$-C(=O)-;
$R^{17}$ represents a $C_1$-$C_2$-alkyl group;
$R^{20}$ represents a hydrogen atom or a group selected from $C_1$-$C_3$-alkyl and phenyl, wherein said $C_1$-$C_3$-alkyl group is optionally substituted one or two times, each substituent independently selected from a fluorine atom or a group selected from hydroxy, $C_1$-$C_3$-alkoxy and phenyl, said phenyl itself being optionally substituted one or two times, each substituent independently selected from a fluorine atom, a chlorine atom and a methyl group, and wherein said phenyl group is optionally substituted one, two or three times, each substituent independently selected from a fluorine atom and a chlorine atom or a group selected from methyl, trifluoromethyl, methoxy and trifluoro-

methoxy;
$R^{21}$ represents a hydrogen atom or a $C_1$-$C_2$-alkyl group;
or
$R^{20}$ and $R^{21}$, together with the nitrogen atom to which they are attached, represent a monocyclic nitrogen containing 4- to 7-membered heterocycloalkyl group which is optionally substituted one or two times, each substituent independently selected from a halogen atom or a group selected from cyano, oxo, hydroxy, $C_1$-$C_2$-alkyl, $C_1$-$C_2$-fluoroalkyl, benzyl, ($C_1$-$C_2$-alkyl)-C(=O)- and $C_3$-$C_4$-cycloalkyl,

or a stereoisomer, a tautomer, an N-oxide, a hydrate, a solvate, or a salt thereof, or a mixture of same.

**4.** The compound according to claim 1, 2 or 3, wherein:

$R^1$ represents a phenyl or pyridinyl group optionally substituted, one or two times, each substituent independently selected from a fluorine atom, a chlorine atom and a bromine atom, or a group selected from methyl, difluoromethyl, trifluoromethyl, methoxy, benzyloxy, difluoromethoxy and trifluoromethoxy,
or two substituents attached to adjacent carbon atoms of said phenyl or pyridinyl group together form a bivalent group -O-$CF_2$-O-;
$R^2$ represents a group

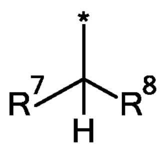

,

wherein "*" indicates the point of attachment to the nitrogen atom to which $R^2$ is attached;
$R^3$ represents a group selected from methyl and -$NH_2$;
$R^4$ represents a group

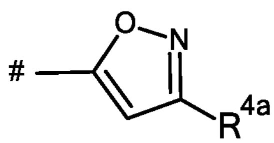

,

wherein "#" indicates the point of attachment to the carbonyl group to which $R^4$ is attached,
and wherein
$R^{4a}$ represents a chlorine atom or a bromine atom, or a group selected from (($R^9$)O)-($C_1$-$C_2$-alkyl)-, (($R^{10}$)($R^{11}$)N)-($C_1$-$C_2$-alkyl)-, ($C_3$-$C_5$-cycloalkyl)-($C_1$-$C_2$-alkyl)-, $C_3$-$C_5$-cycloalkyl, - $OR^9$, -C(=O)-N($R^{15}$)($R^{16}$) and phenyl, wherein the phenyl group is optionally substituted one or two times, each substituent independently selected from a fluorine atom and a chlorine atom and a methyl group,
or
$R^4$ represents a group

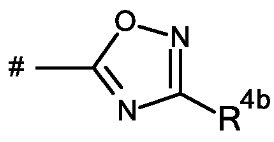

,

wherein "#" indicates the point of attachment to the carbonyl group to which $R^4$ is attached, and wherein
$R^{4b}$ represents a group selected from $C_1$-$C_4$-alkyl, $C_3$-$C_5$-cycloalkyl, -C(=O)-N($R^{15}$)($R^{16}$) and phenyl,
wherein the phenyl group is optionally substituted one or two times, each substituent independently selected from a fluorine atom and a chlorine atom, or a group selected from cyano, $C_1$-$C_2$-alkyl and -$OR^9$;
$R^7$ represents a hydrogen atom or a $C_1$-$C_2$-alkyl group;
$R^8$ represents a -C(=O)-$NH_2$ group;
$R^9$ represents a hydrogen atom or a group selected from $C_1$-$C_2$-alkyl, benzyl, $C_1$-$C_2$-fluoroalkyl, ($C_1$-$C_2$-alkoxy)-$C_2$-alkyl-, (($C_1$-$C_2$-alkyl)-C(=O)-O)-$C_2$-alkyl-, -C(=O)-N($R^{20}$)($R^{21}$) and phenyl,

wherein the phenyl group within said benzyl group and said phenyl group itself are optionally substituted one or two times, each substituent independently selected from a fluorine atom and a chlorine atom, or a group selected from cyano and methyl;

$R^{10}$ and $R^{11}$ represent, independently from each occurrence, a hydrogen atom or a group selected from $C_1$-$C_2$-alkyl, ($C_3$-$C_5$-cycloalkyl)-($C_1$-$C_2$-alkyl)-, $C_3$-$C_7$-cycloalkyl and (phenyl)-($C_1$-$C_2$-alkyl)-O-C(=O)-,

wherein $C_3$-$C_7$-cycloalkyl, and the $C_3$-$C_5$-cycloalkyl within said ($C_3$-$C_5$-cycloalkyl)-($C_1$-$C_2$-alkyl)- group are optionally substituted one or two times, each substituent independently selected from a fluorine atom or a group selected from cyano, methyl and $C_1$-fluoroalkyl,
and wherein the phenyl group within said (phenyl)-($C_1$-$C_2$-alkyl)-O-C(=O)- group is optionally substituted one or two times, each substituent independently selected from a fluorine atom, a chlorine atom and a methyl group,

or

$R^{10}$ and $R^{11}$, together with the nitrogen atom to which they are attached, represent a monocyclic nitrogen containing 4- to 7-membered heterocycloalkyl group which is optionally substituted one, two or three times, each substituent independently selected from a fluorine atom or a group selected from cyano, methyl and $C_1$-fluoroalkyl;

$R^{15}$ and $R^{16}$ represent, independently from each occurrence, a hydrogen atom or a group selected from $C_1$-$C_4$-alkyl, $C_1$-$C_2$-fluoroalkyl, ($C_1$-$C_4$-alkoxy)-$C_2$-$C_3$-alkyl-, ($C_1$-$C_2$-fluoroalkoxy)-$C_2$-$C_3$-alkyl-, ($C_3$-$C_5$-cycloalkyl)-($C_1$-$C_2$-alkyl)-, $C_3$-$C_5$-cycloalkyl, phenyl and pyridinyl,

wherein $C_3$-$C_5$-cycloalkyl, and the $C_3$-$C_5$-cycloalkyl within said ($C_3$-$C_5$-cycloalkyl)-($C_1$-$C_2$-alkyl)- group are optionally substituted one or two times, each substituent independently selected from a fluorine atom and a methyl group,
and wherein the phenyl and pyridinyl groups are optionally substituted one or two times, each substituent independently selected from a fluorine atom and a chlorine atom, or a group selected from methyl, trifluoromethyl and methoxy;

$R^{20}$ represents a group selected from benzyl and phenyl,
wherein said phenyl group, and the phenyl group within said benzyl group, is optionally substituted one or two times, each substituent independently selected from a fluorine atom, a chlorine atom and a methyl group, and
$R^{21}$ represents a hydrogen atom or a methyl group,

or a stereoisomer, a tautomer, an N-oxide, a hydrate, a solvate, or a salt thereof, or a mixture of same.

**5.** The compound according to claims 1, 2 or 3, wherein:

$R^1$ represents a group

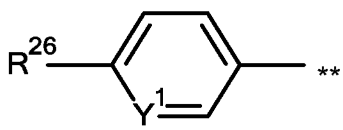

,

wherein "**" indicates the point of attachment to the nitrogen atom to which $R^1$ is attached;
$R^2$ represents a group

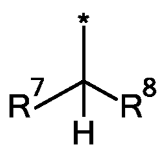

,

wherein "*" indicates the point of attachment to the nitrogen atom to which $R^2$ is attached;
$R^3$ represents a group selected from methyl and $-NH_2$;

$R^4$ represents a group

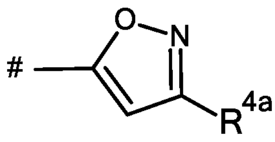

,

wherein "#" indicates the point of attachment to the carbonyl group to which $R^4$ is attached, and wherein $R^{4a}$ represents a chlorine atom or a bromine atom, or a group selected from (($R^9$)O)-($C_1$-alkyl)-, (($R^{10}$)($R^{11}$)N)-($C_1$-alkyl)-, ($C_3$-$C_5$-cycloalkyl)-($C_1$-alkyl)-, $C_3$-$C_5$-cycloalkyl, -O$R^9$, - C(=O)-N($R^{15}$)($R^{16}$) and phenyl, wherein the phenyl group is optionally substituted one or two times, each substituent independently selected from a fluorine atom and a chlorine atom and a methyl group, or
$R^4$ represents a group

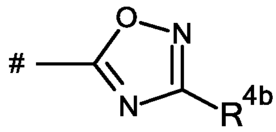

,

wherein "#" indicates the point of attachment to the carbonyl group to which $R^4$ is attached, and wherein $R^{4b}$ represents a group selected from $C_1$-$C_4$-alkyl, $C_3$-$C_5$-cycloalkyl and phenyl,
wherein the phenyl group is optionally substituted one or two times, each substituent independently selected from a fluorine atom and a chlorine atom, or a group selected from cyano, $C_1$-$C_2$-alkyl and -O$R^9$;
$R^7$ represents a hydrogen atom or a $C_1$-$C_2$-alkyl group;
$R^8$ represents a -C(=O)-$NH_2$ group;
$R^9$ represents a hydrogen atom or a group selected from $C_1$-$C_2$-alkyl, benzyl, $C_1$-$C_2$-fluoroalkyl, ($C_1$-$C_2$-alkoxy)-$C_2$-alkyl-, (($C_1$-$C_2$-alkyl)-C(=O)-O)-$C_2$-alkyl- and phenyl,
wherein the phenyl group within said benzyl group and said phenyl group itself are optionally substituted one or two times, each substituent independently selected from a fluorine atom and a chlorine atom, or a group selected from cyano and methyl;
$R^{10}$ and $R^{11}$ represent, independently from each occurrence, a hydrogen atom or a group selected from $C_1$-$C_2$-alkyl, $C_3$-$C_7$-cycloalkyl and (benzyl)-O-C(=O)-,

wherein $C_3$-$C_7$-cycloalkyl is optionally substituted one or two times, each substituent independently selected from a fluorine atom or a group selected from methyl and trifluoromethyl,
and wherein the phenyl group within said (benzyl)-O-C(=O)- group is optionally substituted one or two times, each substituent independently selected from a fluorine atom, a chlorine atom and a methyl group,

or
$R^{10}$ and $R^{11}$, together with the nitrogen atom to which they are attached, represent a monocyclic nitrogen containing 4- to 7-membered heterocycloalkyl group which is optionally substituted one, two or three times, each substituent independently selected from a fluorine atom or a group selected from cyano, methyl and trifluoromethyl;
$R^{15}$ and $R^{16}$ represent, independently from each occurrence, a hydrogen atom or a group selected from $C_1$-$C_4$-alkyl, $C_1$-$C_2$-fluoroalkyl, ($C_1$-$C_4$-alkoxy)-$C_2$-alkyl-, ($C_1$-$C_2$-fluoroalkoxy)-$C_2$-alkyl-, $C_3$-$C_5$-cycloalkyl, phenyl and pyridinyl,
wherein $C_3$-$C_5$-cycloalkyl is optionally substituted one or two times, each substituent independently selected from a fluorine atom and a methyl group;
$Y^1$ represents -C(H)=, -C(F)=, -C(Cl)=, -C(CN)= or -N=, and
$R^{26}$ represents a fluorine atom, a chlorine atom or a bromine atom, or a group selected from methyl, difluoromethyl, trifluoromethyl, methoxy, benzyloxy, difluoromethoxy and trifluoromethoxy,
or a stereoisomer, a tautomer, an N-oxide, a hydrate, a solvate, or a salt thereof, or a mixture of same.

**6.** The compound according to claim 1, 2, 3, 4 or 5, wherein:

$R^1$ represents a group

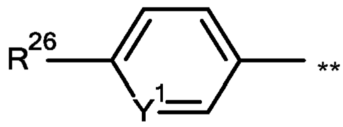

,

wherein "**" indicates the point of attachment to the nitrogen atom to which $R^1$ is attached;
$R^2$ represents a group

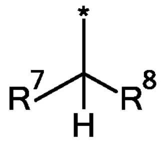

,

wherein "*" indicates the point of attachment to the nitrogen atom to which $R^2$ is attached;
$R^3$ represents a group selected from methyl and $-NH_2$;
$R^4$ represents a group

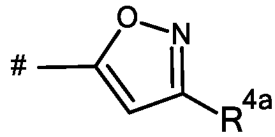

,

wherein "#" indicates the point of attachment to the carbonyl group to which $R^4$ is attached, and wherein
$R^{4a}$ represents a chlorine atom or a bromine atom, or a group selected from $((R^9)O)-(C_1\text{-alkyl})-$, $C_3$-$C_5$-cycloalkyl, $-OR^9$ and $-C(=O)-N(R^{15})(R^{16})$, or
$R^4$ represents a group

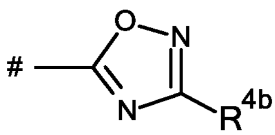

,

wherein "#" indicates the point of attachment to the carbonyl group to which $R^4$ is attached, and wherein
$R^{4b}$ represents a group selected from $C_1$-$C_4$-alkyl, $C_3$-$C_5$-cycloalkyl and phenyl, wherein the phenyl group is optionally substituted one or two times, each substituent independently selected from a fluorine atom and a chlorine atom and a methyl group;
$R^7$ represents a hydrogen atom or a $C_1$-$C_2$-alkyl group;
$R^8$ represents a $-C(=O)-NH_2$ group;
$R^9$ represents a hydrogen atom or a group selected from $C_1$-$C_2$-alkyl, benzyl, $C_1$-$C_2$-fluoroalkyl and phenyl, wherein the phenyl group within said benzyl group and said phenyl group itself are optionally substituted one or two times, each substituent independently selected from a fluorine atom and a chlorine atom, or a group selected from cyano and methyl;
$R^{15}$ and $R^{16}$ represent, independently from each occurrence, a hydrogen atom or a group selected from $C_1$-$C_2$-alkyl, $C_3$-$C_5$-cycloalkyl, phenyl and pyridinyl, wherein $C_3$-$C_5$-cycloalkyl is optionally substituted one or two times, each substituent independently selected from a fluorine atom and a methyl group;
$Y^1$ represents -C(H)=, -C(F)=, -C(Cl)= or -N=, and
$R^{26}$ represents a fluorine atom, a chlorine atom or a bromine atom, or a group selected from difluoromethyl, methoxy, benzyloxy, difluoromethoxy and trifluoromethoxy,
or a stereoisomer, a tautomer, an N-oxide, a hydrate, a solvate, or a salt thereof, or a mixture of same.

**7.** The compound according to claim 1, 2, 3, 4, 5 or 6, wherein:

$R^1$ represents a group

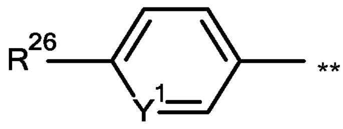

,

wherein "**" indicates the point of attachment to the nitrogen atom to which R' is attached;
$R^2$ represents a group

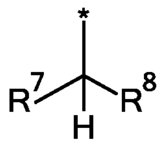

,

wherein "*" indicates the point of attachment to the nitrogen atom to which $R^2$ is attached;
$R^3$ represents a group $-NH_2$;
$R^4$ represents a group

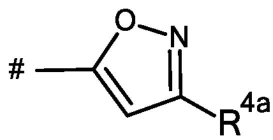

,

wherein "#" indicates the point of attachment to the carbonyl group to which $R^4$ is attached, and wherein $R^{4a}$ represents a chlorine atom or a bromine atom, or a group selected from $((R^9)O)-(C_1\text{-alkyl})-$, $C_3$-$C_5$-cycloalkyl, $-OR^9$ and $-C(=O)-N(R^{15})(R^{16})$, or
$R^4$ represents a group

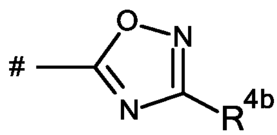

,

wherein "#" indicates the point of attachment to the carbonyl group to which $R^4$ is attached, and wherein $R^{4b}$ represents a group selected from $C_1$-$C_4$-alkyl, $C_3$-$C_5$-cycloalkyl and phenyl, wherein the phenyl group is optionally substituted one or two times, each substituent independently selected from a fluorine atom and a chlorine atom and a methyl group;
$R^7$ represents a methyl group;
$R^8$ represents a $-C(=O)-NH_2$ group;
$R^9$ represents a hydrogen atom or a group selected from $C_1$-$C_2$-alkyl, benzyl, $C_1$-$C_2$-fluoroalkyl and phenyl, wherein the phenyl group within said benzyl group and said phenyl group itself are optionally substituted one or two times, each substituent independently selected from a fluorine atom and a chlorine atom, or a group selected from cyano and methyl;
$R^{15}$ and $R^{16}$ represent, independently from each occurrence, a hydrogen atom or a group selected from $C_1$-$C_2$-alkyl, $C_3$-$C_5$-cycloalkyl, phenyl and pyridinyl, wherein $C_3$-$C_5$-cycloalkyl is optionally substituted one or two times, each substituent independently selected from a fluorine atom and a methyl group;
$Y^1$ represents -C(H)=, -C(F)=, -C(Cl)= or -N=, and
$R^{26}$ represents a fluorine atom, a chlorine atom or a bromine atom, or a group selected from difluoromethyl, methoxy, benzyloxy, difluoromethoxy and trifluoromethoxy,
or a stereoisomer, a tautomer, an N-oxide, a hydrate, a solvate, or a salt thereof, or a mixture of same.

**8.** The compound according to claim 1, which is selected from the group consisting of:

rac-2-(N-[4-amino-5-[3-(4-chlorophenyl)-1,2,4-oxadiazole-5-carbonyl]thiazol-2-yl]-4-fluoro-anilino)propanamide ,

rac-2-(N-[4-amino-5-(3-benzyloxyisoxazole-5-carbonyl)thiazol-2-yl]-4-fluoro-anilino)propanamide ,
rac-2-(N-[4-amino-5-(3-phenylisoxazole-5-carbonyl)thiazol-2-yl]-4-fluoro-anilino)propanamide ,
(R)- 2-(N-[4-amino-5-(3-phenylisoxazole-5-carbonyl)thiazol-2-yl]-4-fluoro-anilino)propanamide ,
(S)-2-(N-[4-amino-5-(3-phenylisoxazole-5-carbonyl)thiazol-2-yl]-4-fluoro-anilino)propanamide ,
rac-2-(N-[4-amino-5-[3-(2,4-dichlorophenyl)isoxazole-5-carbonyl]thiazol-2-yl]-4-fluoro-anilino)propanamide ,
rac-2-(N-[4-amino-5-[3-(4-chlorophenyl)isoxazole-5-carbonyl]thiazol-2-yl]-4-fluoro-anilino)propanamide ,
rac-2-(N-[4-amino-5-(3-bromoisoxazole-5-carbonyl)thiazol-2-yl]-4-fluoro-anilino)propanamide ,
(R)-2-(N-[4-amino-5-(3-bromoisoxazole-5-carbonyl)thiazol-2-yl]-4-fluoro-anilino)propanamide ,
(S)-2-(N-[4-amino-5-(3-bromoisoxazole-5-carbonyl)thiazol-2-yl]-4-fluoro-anilino)propanamide ,
rac-2-(N-[4-amino-5-[3-(3-pyridyl)isoxazole-5-carbonyl]thiazol-2-yl]-4-fluoro-anilino)propanamide ,
rac-2-(N-[4-amino-5-[3-(4-pyridyl)isoxazole-5-carbonyl]thiazol-2-yl]-4-fluoro-anilino)propanamide ,
rac-ethyl 5-[4-amino-2-(N-(2-amino-1-methyl-2-oxo-ethyl)-4-fluoro-anilino)thiazole-5-carbonyl]isoxazole-3-carboxylate ,
rac-5-[4-amino-2-(N-(2-amino-1-methyl-2-oxo-ethyl)-4-fluoro-anilino)thiazole-5-carbonyl]-N-phenyl-isoxazole-3-carboxamide ,
5-[4-amino-2-(N-(2-amino-1-methyl-2-oxo-ethyl)-4-fluoro-anilino)thiazole-5-carbonyl]-N-[(cis)-2-fluorocyclopropyl]isoxazole-3-carboxamide (mixture of stereoisomers),
rac-5-[4-amino-2-(N-(2-amino-1-methyl-2-oxo-ethyl)-4-fluoro-anilino)thiazole-5-carbonyl]-N-(1-methylpyrazol-4-yl)isoxazole-3-carboxamide,
rac-2-(N-[4-amino-5-[3-(morpholine-4-carbonyl)isoxazole-5-carbonyl]thiazol-2-yl]-4-fluoro-anilino)propanamide ,
rac-5-[4-amino-2-(N-(2-amino-1-methyl-2-oxo-ethyl)-4-fluoro-anilino)thiazole-5-carbonyl]-N-pyrimidin-5-yl-isoxazole-3-carboxamide ,
rac-5-[4-amino-2-(N-(2-amino-1-methyl-2-oxo-ethyl)-4-fluoro-anilino)thiazole-5-carbonyl]-N-cyclopropyl-isoxazole-3-carboxamide ,
rac-5-[4-amino-2-(N-(2-amino-1-methyl-2-oxo-ethyl)-4-fluoro-anilino)thiazole-5-carbonyl]-N-(4-pyridyl)isoxazole-3-carboxamide ,
rac-5-[4-amino-2-(N-(2-amino-1-methyl-2-oxo-ethyl)-4-fluoro-anilino)thiazole-5-carbonyl]-N-(2,2-difluoroethyl) isoxazole-3-carboxamide ,
rac-2-(N-[4-amino-5-[3-(azetidine-1-carbonyl)isoxazole-5-carbonyl]thiazol-2-yl]-4-fluoro-anilino)propanamide ,
rac-2-(N-[4-amino-5-[3-(pyrrolidine-1-carbonyl)isoxazole-5-carbonyl]thiazol-2-yl]-4-fluoro-anilino)propanamide ,
rac-5-[4-amino-2-(N-(2-amino-1-methyl-2-oxo-ethyl)-4-fluoro-anilino)thiazole-5-carbonyl]-N-(3-pyridyl)isoxazole-3-carboxamide ,
rac-5-[4-amino-2-(N-(2-amino-1-methyl-2-oxo-ethyl)-4-fluoro-anilino)thiazole-5-carbonyl]-N-cyclobutyl-isoxazole-3-carboxamide ,
rac-5-[4-amino-2-(N-(2-amino-1-methyl-2-oxo-ethyl)-4-fluoro-anilino)thiazole-5-carbonyl]-N-(cyclopropylmethyl)isoxazole-3-carboxamide ,
rac-5-[4-amino-2-(N-(2-amino-1-methyl-2-oxo-ethyl)-4-fluoro-anilino)thiazole-5-carbonyl]-N-(2,2,2-trifluoroethyl)isoxazole-3-carboxamide ,
rac-5-[4-amino-2-(N-(2-amino-1-methyl-2-oxo-ethyl)-4-fluoro-anilino)thiazole-5-carbonyl]-N-(1-cyclopropylcyclopropyl)isoxazole-3-carboxamide ,
rac-5-[4-amino-2-(N-(2-amino-1-methyl-2-oxo-ethyl)-4-fluoro-anilino)thiazole-5-carbonyl]-N-cyclopentyl-isoxazole-3-carboxamide ,
rac-5-[4-amino-2-(N-(2-amino-1-methyl-2-oxo-ethyl)-4-fluoro-anilino)thiazole-5-carbonyl]-N-(2-tert-butoxyethyl)isoxazole-3-carboxamide ,
rac-2-(N-[4-amino-5-[3-(piperidine-1-carbonyl)isoxazole-5-carbonyl]thiazol-2-yl]-4-fluoro-anilino)propanamide ,
rac-5-[4-amino-2-(N-(2-amino-1-methyl-2-oxo-ethyl)-4-fluoro-anilino)thiazole-5-carbonyl]-N-(2-pyridyl)isoxazole-3-carboxamide ,
rac-5-[4-amino-2-(N-(2-amino-1-methyl-2-oxo-ethyl)-4-fluoro-anilino)thiazole-5-carbonyl]-N-[2-(trifluoromethoxy)ethyl]isoxazole-3-carboxamide ,
rac-5-[4-amino-2-(N-(2-amino-1-methyl-2-oxo-ethyl)-4-fluoro-anilino)thiazole-5-carbonyl]-N-(1-methylcyclobutyl)isoxazole-3-carboxamide ,
(R)-5-[4-amino-2-(N-(2-amino-1-methyl-2-oxo-ethyl)-4-fluoro-anilino)thiazole-5-carbonyl]-N-(1-methylcyclobutyl)isoxazole-3-carboxamide ,
(S)-5-[4-amino-2-(N-(2-amino-1-methyl-2-oxo-ethyl)-4-fluoro-anilino)thiazole-5-carbonyl]-N-(1-methylcyclobutyl)isoxazole-3-carboxamide ,

rac-5-[4-amino-2-(N-(2-amino-1-methyl-2-oxo-ethyl)-4-fluoro-anilino)thiazole-5-carbonyl]-N-(1-bicyclo[1.1.1]pentanyl)isoxazole-3-carboxamide ,
rac-5-[4-amino-2-(N-(2-amino-1-methyl-2-oxo-ethyl)-4-fluoro-anilino)thiazole-5-carbonyl]-N-(3,3-difluorocyclobutyl)isoxazole-3-carboxamide ,
(R)-5-[4-amino-2-(N-(2-amino-1-methyl-2-oxo-ethyl)-4-fluoro-anilino)thiazole-5-carbonyl]-N-(3,3-difluorocyclobutyl)isoxazole-3-carboxamide ,
(S)-5-[4-amino-2-(N-(2-amino-1-methyl-2-oxo-ethyl)-4-fluoro-anilino)thiazole-5-carbonyl]-N-(3,3-difluorocyclobutyl)isoxazole-3-carboxamide ,
5-[4-amino-2-(N-(2-amino-1-methyl-2-oxo-ethyl)-4-fluoro-anilino)thiazole-5-carbonyl]-N-(3,3-difluorocyclopentyl)isoxazole-3-carboxamide (mixture of stereoisomers),
rac-5-[4-amino-2-(N-(2-amino-1-methyl-2-oxo-ethyl)-4-fluoro-anilino)thiazole-5-carbonyl]-N-isopropyl-isoxazole-3-carboxamide ,
rac-5-[4-amino-2-(N-(2-amino-1-methyl-2-oxo-ethyl)-4-fluoro-anilino)thiazole-5-carbonyl]-N-(cyanomethyl)isoxazole-3-carboxamide ,
5-[4-amino-2-(N-(2-amino-1-methyl-2-oxo-ethyl)-4-fluoro-anilino)thiazole-5-carbonyl]-N-(2-methoxy-1-methyl-ethyl)isoxazole-3-carboxamide (mixture of stereoisomers),
5-[4-amino-2-(N-(2-amino-1-methyl-2-oxo-ethyl)-4-fluoro-anilino)thiazole-5-carbonyl]-N-(2,2-dimethylcyclopentyl)isoxazole-3-carboxamide (mixture of stereoisomers),
5-[4-amino-2-(N-(2-amino-1-methyl-2-oxo-ethyl)-4-fluoro-anilino)thiazole-5-carbonyl]-N-(cis-3-fluorocyclobutyl)isoxazole-3-carboxamide (mixture of stereoisomers),
rac-5-[4-amino-2-(N-(2-amino-1-methyl-2-oxo-ethyl)-4-fluoro-anilino)thiazole-5-carbonyl]-N-(1-methylcyclopentyl)isoxazole-3-carboxamide,
rac-5-[4-amino-2-(N-(2-amino-1-methyl-2-oxo-ethyl)-4-fluoro-anilino)thiazole-5-carbonyl]-N-[2-fluoro-1-(fluoromethyl)ethyl]isoxazole-3-carboxamide ,
5-[4-amino-2-(N-(2-amino-1-methyl-2-oxo-ethyl)-4-fluoro-anilino)thiazole-5-carbonyl]-N-(2,2-difluoro-1-methyl-ethyl)isoxazole-3-carboxamide (mixture of stereoisomers),
5-[4-amino-2-(N-(2-amino-1-methyl-2-oxo-ethyl)-4-fluoro-anilino)thiazole-5-carbonyl]-N-(trans-3-fluorocyclobutyl)isoxazole-3-carboxamide (mixture of stereoisomers),
5-[4-amino-2-(N-(2-amino-1-methyl-2-oxo-ethyl)-4-fluoro-anilino)thiazole-5-carbonyl]-N-(3,3-dimethylcyclopentyl)isoxazole-3-carboxamide (mixture of stereoisomers),
rac-5-[4-amino-2-(N-(2-amino-1-methyl-2-oxo-ethyl)-3,4-difluoro-anilino)thiazole-5-carbonyl]-N-(1-methylcyclobutyl)isoxazole-3-carboxamide ,
(R)-5-[4-amino-2-(N-(2-amino-1-methyl-2-oxo-ethyl)-3,4-difluoro-anilino)thiazole-5-carbonyl]-N-(1-methylcyclobutyl)isoxazole-3-carboxamide ,
(S)-5-[4-amino-2-(N-(2-amino-1-methyl-2-oxo-ethyl)-3,4-difluoro-anilino)thiazole-5-carbonyl]-N-(1-methylcyclobutyl)isoxazole-3-carboxamide ,
rac-5-[4-amino-2-(N-(2-amino-1-methyl-2-oxo-ethyl)-3,4-difluoro-anilino)thiazole-5-carbonyl]-N-(3,3-difluorocyclobutyl)isoxazole-3-carboxamide ,
(R)-5-[4-amino-2-(N-(2-amino-1-methyl-2-oxo-ethyl)-3,4-difluoro-anilino)thiazole-5-carbonyl]-N-(3,3-difluorocyclobutyl)isoxazole-3-carboxamide ,
(S)-5-[4-amino-2-(N-(2-amino-1-methyl-2-oxo-ethyl)-3,4-difluoro-anilino)thiazole-5-carbonyl]-N-(3,3-difluorocyclobutyl)isoxazole-3-carboxamide ,
rac-5-[4-amino-2-(N-(2-amino-1-methyl-2-oxo-ethyl)-3,4-difluoro-anilino)thiazole-5-carbonyl]-N-cyclopentylisoxazole-3-carboxamide ,
(R)-5-[4-amino-2-(N-(2-amino-1-methyl-2-oxo-ethyl)-3,4-difluoro-anilino)thiazole-5-carbonyl]-N-cyclopentylisoxazole-3-carboxamide ,
(S)- 5-[4-amino-2-(N-(2-amino-1-methyl-2-oxo-ethyl)-3,4-difluoro-anilino)thiazole-5-carbonyl]-N-cyclopentylisoxazole-3-carboxamide ,
rac-2-(N-[4-amino-5-(3-cyclopentylisoxazole-5-carbonyl)thiazol-2-yl]-4-fluoro-anilino)propanamide ,
rac-2-(N-[4-amino-5-[3-(difluoromethoxy)isoxazole-5-carbonyl]thiazol-2-yl]-4-fluoro-anilino)propanamide ,
rac-2-(N-[4-amino-5-(3-isopropyl-1,2,4-oxadiazole-5-carbonyl)thiazol-2-yl]-4-fluoro-anilino)propanamide ,
rac-2-(N-[4-amino-5-(3-phenylisothiazole-5-carbonyl)thiazol-2-yl]-4-fluoro-anilino)propanamide,
rac-2-(N-[4-amino-5-(3-phenyl-1,2,4-oxadiazole-5-carbonyl)thiazol-2-yl]-4-fluoro-anilino) propanamide ,
rac-2-(N-[4-amino-5-(3-methyl-1,2,4-oxadiazole-5-carbonyl)thiazol-2-yl]-4-fluoro-anilino)propanamide ,
rac-2-(N-[4-amino-5-(3-tert-butyl-1,2,4-oxadiazole-5-carbonyl)thiazol-2-yl]-4-fluoro-anilino)propanamide ,
rac-2-(N-[4-amino-5-[3-(2-pyridyl)-1,2,4-oxadiazole-5-carbonyl]thiazol-2-yl]-4-fluoro-anilino)propanamide ,
rac-2-(N-[4-amino-5-[3-(3-fluorophenyl)-1,2,4-oxadiazole-5-carbonyl]thiazol-2-yl]-4-fluoro-anilino)propanamide ,

rac-2-(N-[4-amino-5-[3-(2,3-dimethylphenyl)-1,2,4-oxadiazole-5-carbonyl]thiazol-2-yl]-4-fluoro-anilino)propanamide ,
rac-2-(N-[4-amino-5-(3-cyclopropyl-1,2,4-oxadiazole-5-carbonyl)thiazol-2-yl]-4-fluoro-anilino)propanamide ,
rac-2-(N-[4-amino-5-[3-(1-methylpyrazol-4-yl)-1,2,4-oxadiazole-5-carbonyl]thiazol-2-yl]-4-fluoro-anilino)propanamide ,
rac-2-(N-[4-amino-5-(3-cyclopentyl-1,2,4-oxadiazole-5-carbonyl)thiazol-2-yl]-4-fluoro-anilino)propanamide ,
rac-2-(N-[4-amino-5-[3-[[cyclopentyl(methyl)amino]methyl]isoxazole-5-carbonyl]thiazol-2-yl]-4-fluoro-anilino) propanamide ,
rac-2-(N-[4-amino-5-[3-[[(1-methylcyclopentyl)amino]methyl]isoxazole-5-carbonyl]thiazol-2-yl]-4-fluoro-anilino)propanamide ,
rac-2-(N-[4-amino-5-[3-[(cyclopentylamino)methyl]isoxazole-5-carbonyl]thiazol-2-yl]-4-fluoro-anilino)propanamide ,
rac-2-(N-[4-amino-5-[3-(1-piperidylmethyl)isoxazole-5-carbonyl]thiazol-2-yl]-4-fluoro-anilino)propanamide ,
rac-2-(N-[4-amino-5-[3-[(4-cyanophenoxy)methyl]isoxazole-5-carbonyl]thiazol-2-yl]-4-fluoro-anilino)propanamide ,
rac-2-(N-[4-amino-5-[3-(hydroxymethyl)isoxazole-5-carbonyl]thiazol-2-yl]-4-fluoro-anilino)propanamide ,
rac-[5-[4-amino-2-(N-(2-amino-1-methyl-2-oxo-ethyl)-4-fluoro-anilino)thiazole-5-carbonyl]isoxazol-3-yl]methyl N-phenylcarbamate ,
rac-2-(N-[4-amino-5-[3-(2-fluorophenyl)isoxazole-5-carbonyl]thiazol-2-yl]-4-fluoro-anilino)propanamide ,
rac-2-(N-[4-amino-5-[3-(2-pyridyl)isoxazole-5-carbonyl]thiazol-2-yl]-4-fluoro-anilino)propanamide ,
rac-2-(N-[4-amino-5-[3-[4-(trifluoromethoxy)phenyl]isoxazole-5-carbonyl]thiazol-2-yl]-4-fluoro-anilino)propanamide ,
rac-2-(N-[4-amino-5-[3-(2-methoxyphenyl)isoxazole-5-carbonyl]thiazol-2-yl]-4-fluoro-anilino)propanamide ,
rac-2-(N-[4-amino-5-[3-(6-methoxy-3-pyridyl)isoxazole-5-carbonyl]thiazol-2-yl]-4-fluoro-anilino)propanamide,
rac-2-(N-[4-amino-5-(1-phenyltriazole-4-carbonyl)thiazol-2-yl]-4-fluoro-anilino)propanamide ,
rac-2-(N-[4-amino-5-[3-[(3-fluoroazetidin-1-yl)methyl]isoxazole-5-carbonyl]thiazol-2-yl]-4-fluoro-anilino)propanamide ,
rac-2-(N-[4-amino-5-[3-[(3,3-difluoroazetidin-1-yl)methyl]isoxazole-5-carbonyl]thiazol-2-yl]-4-fluoro-anilino) propanamide ,
rac-2-(N-[4-amino-5-[3-[(4,4-difluoro-1-piperidyl)methyl]isoxazole-5-carbonyl]thiazol-2-yl]-4-fluoro-anilino)propanamide ,
2-(N-[4-amino-5-[3-[(4,4-difluoro-3-methyl-1-piperidyl)methyl]isoxazole-5-carbonyl]thiazol-2-yl]-4-fluoro-anilino)propanamide (mixture of stereoisomers),
2-(N-[4-amino-5-[3-[(3-fluoropyrrolidin-1-yl)methyl]isoxazole-5-carbonyl]thiazol-2-yl]-4-fluoro-anilino)propanamide (mixture of stereoisomers),
rac-2-(N-[4-Amino-5-(3-hydroxyisoxazole-5-carbonyl)thiazol-2-yl]-4-fluoro-anilino)propanamide,
rac-2-(N-[4-amino-5-[3-(cyclobutoxy)isoxazole-5-carbonyl]thiazol-2-yl]-4-fluoro-anilino)propanamide ,
rac-2-(N-[4-amino-5-[3-(cyclobutylmethoxy)isoxazole-5-carbonyl]thiazol-2-yl]-4-fluoro-anilino)propanamide ,
rac-2-(N-[4-Amino-5-[3-(4,4-difluorocyclohexoxy)isoxazole-5-carbonyl]thiazol-2-yl]-4-fluoro-anilino)propanamide , and
rac-2-(N-[4-amino-5-(3-isopropoxyisoxazole-5-carbonyl)thiazol-2-yl]-4-fluoro-anilino)propanamide ,
or a stereoisomer, a tautomer, an N-oxide, a hydrate, a solvate, or a salt thereof, or a mixture of same.

**9.** A compound of general formula (I) according to any one of claims 1 to 8 for use in the treatment or prophylaxis of a disease.

**10.** A pharmaceutical composition comprising a compound of general formula (I) according to any one of claims 1 to 8 and one or more pharmaceutically acceptable excipients.

**11.** A pharmaceutical combination comprising:

- one or more compounds of general formula (I) according to any one of claims 1 to 8, and
- one or more immune checkpoint inhibitors.

**12.** A pharmaceutical combination according to claim 11, wherein the immune checkpoint inhibitor is an aPD-1/-L1 axis antagonist.

**13.** A pharmaceutical combination according to claim 11, wherein the immune checkpoint inhibitor is an inhibitor of DGK$\alpha$.

**14.** A compound for use according to claim 9, wherein the disease is cancer or a condition with dysregulated immune response or a disorder associated with aberrant DGKζ signalling.

## Patentansprüche

**1.** Verbindung der allgemeinen Formel (I):

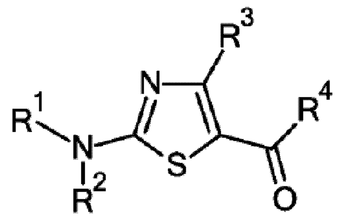

(I)

in der:

$R^1$ für eine Phenyl- oder 6-gliedrige Heteroarylgruppe steht, die gegebenenfalls ein-, zwei- oder dreifach substituiert ist, wobei jeder Substituent unabhängig aus einem Halogenatom oder einer Gruppe ausgewählt aus Hydroxy, Cyano, Nitro, $C_1$-$C_6$-Alkyl, (Phenyl)-($C_1$-$C_3$-alkyl) -, $C_1$-$C_6$-Halogenalkyl, $C_1$-$C_6$-Alkoxy, (Phenyl) -($C_1$-$C_3$-alkoxy) -, $C_1$-$C_6$-Halogenalkoxy und - N($R^5$) ($R^6$) ausgewählt ist,

wobei die Phenylgruppen in den genannten (Phenyl)-($C_1$-$C_3$-alkyl)- und (Phenyl) -($C_1$-$C_3$-alkoxy)-Gruppen gegebenenfalls ein- oder zweifach substituiert sind, wobei jeder Substituent unabhängig aus einem Halogenatom oder einer Gruppe ausgewählt aus Cyano, Methyl, Ethyl, Trifluormethyl, Methoxy, Ethoxy, Dimethylamino und Trifluormethoxy ausgewählt ist,
oder zwei Substituenten, die an benachbarte Kohlenstoffatome der genannten Phenyl- oder 6-gliedrigen Heteroarylgruppe gebunden sind, zusammen eine zweiwertige Gruppe bilden, die aus $-(CH_2)_3-$, - $(CH_2)_4-$, $-(CH_2)_2-O-$, - $(CH_2)_3-O-$, $-CH_2-O-CH_2-$, - $(CH_2)_2-O-CH_2-$, $-O-CH_2-O-$, $-O-CH_2-CH_2-O-$, - $O-CF_2-O-$, $-O-CH_2-CF_2-O-$ und $-O-CF_2-CF_2-O-$ausgewählt ist,

oder
$R^1$ für eine 5-gliedrige Heteroarylgruppe steht, die gegebenenfalls ein- oder zweifach substituiert ist, wobei jeder Substituent unabhängig aus einem Halogenatom oder einer Gruppe ausgewählt aus Cyano, $C_1$-$C_3$-Alkyl und $C_1$-$C_3$-Alkoxy ausgewählt ist;
$R^2$ für eine Gruppe steht,

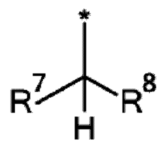

wobei "*" den Verknüpfungspunkt mit dem Stickstoffatom, an das $R^2$ gebunden ist, kennzeichnet;
$R^3$ für eine Gruppe steht, die aus Methyl und $-NH_2$ ausgewählt ist;
$R^4$ für eine 5-gliedrige Heteroarylgruppe steht, die gegebenenfalls ein- oder zweifach substituiert ist, wobei jeder Substituent unabhängig aus einem Halogenatom oder einer Gruppe ausgewählt aus $C_1$-$C_6$-Alkyl, (($R^9$) O)-($C_1$-$C_3$-Alkyl)-, (($R^{10}$)($R^{11}$)N)-($C_1$-$C_3$-Alkyl) -, ($C_3$-$C_7$-Cycloalkyl)- ($C_1$-$C_3$-alkyl) -, $C_3$-$C_7$-Cycloalkyl, -O$R^9$, -N ($R^{10}$)($R^{11}$) , -C(=O)-N($R^{15}$)($R^{16}$), - C (=O)-O$R^{17}$, Phenyl und einer 5- oder 6-gliedrigen Heteroarylgruppe ausgewählt ist,
wobei die Phenylgruppe und die 5- oder 6-gliedrige Heteroarylgruppe gegebenenfalls ein- oder zweifach substituiert sind, wobei jeder Substituent unabhängig aus einem Halogenatom oder einer Gruppe ausgewählt aus Cyano, $C_1$-$C_3$-Alkyl, $C_1$-$C_3$-Halogenalkyl, (($R^{22}$)($R^{23}$)N) -$C_1$-$C_3$-Alkyl, - O$R^9$, -N($R^{10}$)($R^{11}$) und -C(=O)-N($R^{15}$)($R^{16}$) ausgewählt ist;
$R^5$ und $R^6$ unabhängig von jedem Auftreten für ein Wasserstoffatom oder eine Gruppe ausgewählt aus $C_1$-$C_4$-Alkyl, ($C_1$-$C_4$-Alkyl)-C(=O)-, $C_3$-$C_4$-Cycloalkyl und (Phenyl)-($C_1$-$C_3$-Alkyl)- stehen, oder

$R^5$ und $R^6$ zusammen mit dem Stickstoffatom, an das sie gebunden sind, für eine monocyclische stickstoffhaltige 4- bis 7-gliedrige Heterocycloalkylgruppe stehen, die gegebenenfalls ein-, zwei- oder dreifach substituiert ist, wobei jeder Substituent unabhängig aus einem Halogenatom oder einer Gruppe ausgewählt aus Oxo, Hydroxy, $C_1$-$C_4$-Alkyl, ($C_1$-$C_4$-Alkyl)-C(=O)-, $C_3$-$C_4$-Cycloalkyl und $C_1$-$C_4$-Alkoxy ausgewählt ist;
$R^7$ für ein Wasserstoffatom oder eine $C_1$-$C_2$-Alkylgruppe steht;
$R^8$ für eine Gruppe steht, die aus -C(=O)-$NH_2$ und - S(=O)$_2$-$NH_2$ ausgewählt ist;
$R^9$ für ein Wasserstoffatom oder eine Gruppe ausgewählt aus $C_1$-$C_6$-Alkyl, ($C_3$-$C_7$-Cycloalkyl)- ($C_1$-$C_3$-alkyl) -, (Phenyl) -($C_1$-$C_3$-alkyl)-, (5- oder 6-gliedriges Heteroaryl) - ($C_1$-$C_3$-alkyl) -, $C_1$-$C_6$-Halogenalkyl, $C_2$-$C_4$-Hydroxyalkyl, ($C_1$-$C_3$-Alkoxy)-$C_2$-$C_3$-alkyl-, (($C_1$-$C_3$-Alkyl)-C(=O)-O)-$C_2$-$C_3$-alkyl-, -C ($R^{18}$)($R^{19}$)-C (=O)-O$R^{17}$, -C($R^{10}$)($R^{19}$)-C(=O)-N($R^{20}$)($R^{21}$), -C(=O)-N($R^{20}$)($R^{21}$), $C_3$-$C_7$-Cycloalkyl, Phenyl und einer 5- oder 6-gliedrigen Heteroarylgruppe steht, wobei $C_3$-$C_7$-Cycloalkyl und das $C_3$-$C_7$-Cycloalkyl in der genannten ($C_3$-$C_7$-Cycloalkyl)-($C_1$-$C_3$-alkyl)-Gruppe gegebenenfalls ein- oder zweifach substituiert ist, wobei jeder Substituent unabhängig aus einem Fluoratom oder einer Gruppe ausgewählt aus Oxo, Cyano, $C_1$-$C_2$-Alkyl und $C_1$-$C_2$-Halogenalkyl ausgewählt ist, und wobei die Phenylgruppe in der genannten (Phenyl)-($C_1$-$C_3$-alkyl)-Gruppe und die genannte Phenylgruppe selbst und die 5- oder 6-gliedrige Heteroarylgruppe in der genannten (5- oder 6-gliedriges Heteroaryl)-($C_1$-$C_3$-alkyl)-Gruppe und die genannte 5- oder 6-gliedrige Heteroarylgruppe selbst gegebenenfalls ein- oder zweifach substituiert sind, wobei jeder Substituent unabhängig aus einem Halogenatom oder einer Gruppe ausgewählt aus Cyano, Methyl, Ethyl, Trifluormethyl, Methoxy, Ethoxy, Dimethylamino und Trifluormethoxy ausgewählt ist;
$R^{10}$ und $R^{11}$ unabhängig von jedem Auftreten für ein Wasserstoffatom oder eine Gruppe ausgewählt aus $C_1$-$C_4$-Alkyl, $C_1$-$C_4$-Halogenalkyl, $C_2$-$C_4$-Hydroxyalkyl, ($C_1$-$C_3$-Alkoxy)-$C_2$-$C_3$-alkyl-, (($R^{22}$)($R^{23}$)N)-$C_2$-$C_3$-Alkyl, ($C_3$-$C_7$-Cycloalkyl)- ($C_1$-$C_3$-alkyl) -, ($C_1$-$C_4$-Alkyl) -C (=O)-, $C_3$-$C_7$-Cycloalkyl, ($C_3$-$C_7$-Cycloalkyl)-C(=O)-, (Phenyl) - ($C_1$-$C_3$-alkyl) -, (Phenyl) - ($C_1$-$C_3$-alkyl) -C (=O)-, (Phenyl) - ($C_1$-$C_3$-alkyl) -O-C (=O)-, Phenyl und einer 5- oder 6-gliedrigen Heteroarylgruppe stehen,

wobei $C_3$-$C_7$-Cycloalkyl und das $C_3$-$C_7$-Cycloalkyl in den genannten ($C_3$-$C_7$-Cycloalkyl)-($C_1$-$C_3$-alkyl)- und ($C_3$-$C_7$-Cycloalkyl)-C (=O)-Gruppen gegebenenfalls ein- oder zweifach substituiert sind, wobei jeder Substituent unabhängig aus einem Fluoratom oder einer Gruppe ausgewählt aus Cyano, $C_1$-$C_2$-Alkyl und $C_1$-$C_2$-Halogenalkyl ausgewählt ist,
und wobei das genannte Phenyl und die genannte 5- oder 6-gliedrige Heteroarylgruppe und die Phenylgruppen in den genannten (Phenyl) - ($C_1$-$C_3$-alkyl) -, (Phenyl) - ($C_1$-$C_3$-alkyl) -C (=O)- und (Phenyl) - ($C_1$-$C_3$-alkyl) -O-C (=O)-Gruppen gegebenenfalls ein- oder zweifach substituiert sind, wobei jeder Substituent unabhängig aus einem Halogenatom oder einer Gruppe ausgewählt aus Cyano, Methyl, Ethyl, Trifluormethyl, Methoxy, Ethoxy, Dimethylamino und Trifluormethoxy ausgewählt ist,

oder
$R^{10}$ und $R^{11}$ zusammen mit dem Stickstoffatom, an das sie gebunden sind, für eine monocyclische stickstoffhaltige 4- bis 7-gliedrige Heterocycloalkylgruppe oder eine bicyclische stickstoffhaltige 5- bis 11-gliedrige Heterocycloalkylgruppe stehen, wobei diese Gruppen gegebenenfalls ein-, zwei- oder dreifach substituiert sind, wobei jeder Substituent unabhängig aus einem Halogenatom oder einer Gruppe ausgewählt aus Cyano, Oxo, Hydroxy, $C_1$-$C_4$-Alkyl, $C_1$-$C_4$-Halogenalkyl, ($C_1$-$C_4$-Alkyl)-C (=O)-, $C_3$-$C_7$-Cycloalkyl, $C_1$-$C_4$-Alkoxy, -N ($R^{22}$)($R^{23}$)und einer monocyclischen 4- bis 7-gliedrigen Heterocycloalkylgruppe ausgewählt ist;
$R^{15}$ und $R^{16}$ unabhängig von jedem Auftreten für ein Wasserstoffatom oder eine Gruppe ausgewählt aus $C_1$-$C_4$-Alkyl, $C_1$-$C_4$-Halogenalkyl, $C_1$-$C_4$-Hydroxyalkyl, (Cyano) -$C_1$-$C_4$-alkyl-, ($C_1$-$C_4$-Alkoxy)-$C_2$-$C_3$-alkyl-, ($C_1$-$C_4$-Halogenalkoxy)-$C_2$-$C_3$-alkyl-, (Phenoxy) -$C_2$-$C_3$-alkyl-, ($C_3$-$C_7$-Cycloalkyl)- ($C_1$-$C_3$-alkyl) -, $C_3$-$C_7$-Cycloalkyl, bicyclischem $C_5$-$C_{11}$-Cycloalkyl, monocyclischem 4- bis 7-gliedrigem Heterocycloalkyl, (Phenyl) - ($C_1$-$C_3$-alkyl) -, Phenyl und einer 5- oder 6-gliedrigen Heteroarylgruppe stehen,

wobei $C_3$-$C_7$-Cycloalkyl, das $C_3$-$C_7$-Cycloalkyl in der genannten ($C_3$-$C_7$-Cycloalkyl)-($C_1$-$C_3$-alkyl) - Gruppe, das bicyclische $C_5$-$C_{11}$-Cycloalkyl und die monocyclischen 4- bis 7-gliedrigen Heterocycloalkylgruppen gegebenenfalls ein-, zwei- oder dreifach substituiert sind, wobei jeder Substituent unabhängig aus einem Halogenatom oder einer Gruppe ausgewählt aus Oxo, Hydroxy, $C_1$-$C_4$-Alkyl, ($C_1$-$C_4$-Alkyl)-C (=O)-, $C_3$-$C_4$-Cycloalkyl and $C_1$-$C_4$-Alkoxy ausgewählt ist,
nd wobei das Phenyl und die 5- oder 6-gliedrigen Heteroarylgruppen, einschließlich der Phenylgruppen in den genannten (Phenoxy)-$C_2$-$C_3$-alkyl- und (Phenyl) - ($C_1$-$C_3$-alkyl) -Gruppen, gegebenenfalls ein- oder zweifach substituiert sind, wobei jeder Substituent unabhängig aus einem Halogenatom oder einer Gruppe ausgewählt aus Cyano, Methyl, Ethyl, Trifluormethyl, Methoxy, Ethoxy, Dimethylamino und Trifluormethoxy ausgewählt ist,

u oder

$R^{15}$ und $R^{16}$ zusammen mit dem Stickstoffatom, an das sie gebunden sind, für eine monocyclische stickstoffhaltige 4- bis 7-gliedrige Heterocycloalkylgruppe stehen, die gegebenenfalls ein-, zwei- oder dreifach substituiert ist, wobei jeder Substituent unabhängig aus einem Halogenatom oder einer Gruppe ausgewählt aus Oxo, Hydroxy, $C_1$-$C_4$-Alkyl, ($C_1$-$C_4$-Alkyl)-C (=O)-, $C_3$-$C_4$-Cycloalkyl und $C_1$-$C_4$-Alkoxy ausgewählt ist;

$R^{17}$ für eine $C_1$-$C_4$-Alkylgruppe steht;

$R^{18}$ und $R^{19}$ unabhängig von jedem Auftreten für ein Wasserstoffatom oder eine $C_1$-$C_4$-Alkylgruppe stehen;

$R^{20}$ für ein Wasserstoffatom oder eine Gruppe ausgewählt aus $C_1$-$C_6$-Alkyl, $C_3$-$C_4$-Alkenyl, $C_3$-$C_4$-Alkinyl, $C_1$-$C_3$-Alkoxy, $C_3$-$C_7$-Cycloalkyl, bicyclischem $C_5$-$C_{11}$-Cycloalkyl, Adamantyl, monocyclischem 4- bis 7-gliedrigem Heterocycloalkyl, bicyclischem 5- bis 11- gliedrigem Heterocycloalkyl, Phenyl, Naphthyl und 5- bis 10-gliedrigem Heteroaryl steht, wobei die genannte $C_1$-$C_6$-Alkylgruppe gegebenenfalls ein-, zwei- oder dreifach substituiert ist, wobei jeder Substituent unabhängig aus einem Halogenatom oder einer Gruppe ausgewählt aus Hydroxy, Cyano, $C_1$-$C_3$-Alkoxy, -N ($R^{22}$)($R^{23}$), $C_3$-$C_7$-Cycloalkyl, bicyclischem $C_5$-$C_{11}$-Cycloalkyl, Adamantyl, monocyclischem 4- bis 7-gliedrigem Heterocycloalkyl, bicyclischem 5- bis 11-gliedrigem Heterocycloalkyl, Phenyl und 5- bis 10-gliedrigem Heteroaryl ausgewählt ist, wobei die genannten Phenyl- und 5- bis 10-gliedrigen Heteroarylsubstituenten selbst gegebenenfalls ein- oder zweifach substituiert sind, wobei jeder Substituent unabhängig aus einem Halogenatom oder einer Gruppe ausgewählt aus Cyano, Methyl, Ethyl, Trifluormethyl, Methoxy, Ethoxy, Dimethylamino und Trifluormethoxy ausgewählt ist, und wobei $C_3$-$C_7$-Cycloalkyl, bicyclisches $C_5$-$C_{11}$-Cycloalkyl, Adamantyl, monocyclisches 4- bis 7-gliedriges Heterocycloalkyl und bicyclisches 5-bis 11-gliedriges Heterocycloalkyl gegebenenfalls ein-, zwei- oder dreifach substituiert sind, wobei jeder Substituent unabhängig aus einem Halogenatom oder einer Gruppe ausgewählt aus Cyano, Oxo, Hydroxy, $C_1$-$C_4$-Alkyl, ($C_1$-$C_4$-Alkyl)-C(=O)-, $C_3$-$C_4$-Cycloalkyl und $C_1$-$C_4$-Alkoxy ausgewählt ist, und wobei die genannten Phenyl-, Naphthyl- und 5- bis 10-gliedrigen Heteroarylgruppen gegebenenfalls ein-, zwei- oder dreifach substituiert sind, wobei jeder Substituent unabhängig aus einem Halogenatom oder einer Gruppe ausgewählt aus Cyano, $C_1$-$C_4$-Alkyl, $C_1$-$C_4$-Halogenalkyl, $C_1$-$C_4$-Alkoxy, $C_1$-$C_4$-Halogenalkoxy, -N($R^{22}$)($R^{23}$)und -C(=O)-N($R^{24}$)($R^{25}$) ausgewählt ist,

$R^{21}$ für ein Wasserstoffatom oder eine $C_1$-$C_4$-Alkylgruppe steht,

oder

$R^{20}$ und $R^{21}$ zusammen mit dem Stickstoffatom, an das sie gebunden sind, für eine monocyclische stickstoffhaltige 4- bis 7-gliedrige Heterocycloalkylgruppe stehen, die gegebenenfalls benzokondensiert ist und die gegebenenfalls ein-, zwei- oder dreifach substituiert ist, wobei jeder Substituent unabhängig aus einem Halogenatom oder einer Gruppe ausgewählt aus Cyano, Oxo, Hydroxy, $C_1$-$C_4$-Alkyl, $C_1$-$C_4$-Halogenalkyl, (Phenyl)-($C_1$-$C_3$-alkyl) -, ($C_1$-$C_4$-Alkyl)-C (=O)-, $C_3$-$C_4$-Cycloalkyl, $C_1$-$C_4$-Alkoxy, $C_1$-$C_3$-Halogenalkoxy, -N ($R^{22}$)($R^{23}$) und - C(=O)-N($R^{24}$)($R^{25}$) ausgewählt ist;

$R^{22}$ und $R^{23}$ unabhängig von jedem Auftreten für ein Wasserstoffatom oder eine Gruppe, die aus $C_1$-$C_2$-Alkyl und ($C_1$-$C_2$-Alkyl)-C(=O)- ausgewählt ist, stehen, und

$R^{24}$ und $R^{25}$ unabhängig von jedem Auftreten für ein Wasserstoffatom oder eine $C_1$-$C_4$-Alkylgruppe stehen,

oder ein Stereoisomer, ein Tautomer, ein N-Oxid, ein Hydrat, ein Solvat, oder ein Salz davon, oder ein Gemisch davon.

**2.** Verbindung nach Anspruch 1, wobei:

$R^1$ für eine Phenyl- oder Pyridinylgruppe steht, die gegebenenfalls ein-, zwei- oder dreifach substituiert ist, wobei jeder Substituent unabhängig aus einem Halogenatom oder einer Gruppe ausgewählt aus Hydroxy, Cyano, Nitro, $C_1$-$C_4$-Alkyl, (Phenyl)-($C_1$-$C_2$-alkyl)-, $C_1$-$C_4$-Halogenalkyl, $C_1$-$C_4$-Alkoxy, (Phenyl) - ($C_1$-$C_2$-alkoxy)-, $C_1$-$C_4$-Halogenalkoxy und - N($R^5$)($R^6$) ausgewählt ist,

wobei die Phenylgruppen in den genannten (Phenyl) - ($C_1$-$C_2$-alkyl) - und (Phenyl) - ($C_1$-$C_2$-alkoxy)-Gruppen gegebenenfalls ein- oder zweifach substituiert sind, wobei jeder Substituent unabhängig aus einem Fluoratom, einem Chloratom und einem Bromatom oder einer Gruppe ausgewählt aus Methyl, Trifluormethyl und Methoxy ausgewählt ist,

oder zwei Substituenten, die an benachbarte Kohlenstoffatome der genannten Phenyl- oder Pyridinylgruppe gebunden sind, zusammen eine bivalente Gruppe bilden, die aus $-(CH_2)_3-$, - $(CH_2)_4-$, $-(CH_2)2-O-$, $-(CH_2)_3-O-$, $-CH_2-O-CH_2-$, $-O-CH_2-O-$, $-O-CH_2-CH_2-O-$ und $-O-CF_2-O-$ ausgewählt ist,

oder

$R^1$ für eine Pyrazolylgruppe steht, die gegebenenfalls ein- oder zweifach substituiert ist, wobei jeder Substituent unabhängig aus einem Halogenatom oder einer Gruppe ausgewählt aus Cyano, $C_1$-$C_2$-Alkyl und $C_1$-$C_2$-Alkoxy

ausgewählt ist;
$R^2$ für eine Gruppe

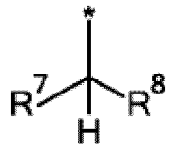

steht,
wobei "*" den Verknüpfungspunkt mit dem Stickstoffatom, an das $R^2$ gebunden ist, kennzeichnet;
$R^3$ für eine Gruppe steht, die aus Methyl und $-NH_2$ ausgewählt ist;
$R^4$ für eine aus Oxazolyl, Isoxazolyl, Thiazolyl, Isothiazolyl und Oxadiazolyl ausgewählte 5-gliedrige Heteroarylgruppe steht, die gegebenenfalls ein- oder zweifach substituiert ist, wobei jeder Substituent unabhängig aus einem Fluoratom, einem Chloratom und einem Bromatom oder einer Gruppe ausgewählt aus $C_1$-$C_4$-Alkyl, (($R^9$)O)-($C_1$-$C_3$-Alkyl)-, (($R^{10}$)($R^{11}$)N)- ($C_1$-$C_3$-Alkyl)-, ($C_3$-$C_7$-Cycloalkyl) - ($C_1$-$C_3$-alkyl) -, $C_3$-$C_7$-Cycloalkyl, -O$R^9$, -N($R^{10}$)($R^{11}$) , -C(=O)-N($R^{15}$)($R^{16}$), -C(=O)-O$R^{17}$, Phenyl und einer 5- oder 6-gliedrigen Heteroarylgruppe ausgewählt ist,
wobei die Phenylgruppe und die 5- oder 6-gliedrige Heteroarylgruppe gegebenenfalls ein- oder zweifach substituiert sind, wobei jeder Substituent unabhängig aus einem Fluoratom, einem Chloratom und einem Bromatom oder einer Gruppe ausgewählt aus Cyano, $C_1$-$C_2$-Alkyl, $C_1$-$C_2$-Fluoralkyl, -O$R^9$, -N($R^{10}$)($R^{11}$) und -C(=O)-N($R^{15}$)-N($R^{16}$)ausgewählt ist;
$R^5$ und $R^6$ unabhängig von jedem Auftreten für ein Wasserstoffatom oder eine Gruppe, die aus $C_1$-$C_2$-Alkyl und ($C_1$-$C_2$-Alkyl)-C (=O)- ausgewählt ist, stehen, oder
$R^5$ und $R^6$ zusammen mit dem Stickstoffatom, an das sie gebunden sind, für eine monocyclische stickstoffhaltige 4- bis 7-gliedrige Heterocycloalkylgruppe stehen, die gegebenenfalls ein- oder zweifach substituiert ist, wobei jeder Substituent unabhängig aus einem Fluoratom, einem Chloratom und einem Bromatom oder einer Gruppe ausgewählt aus Oxo, Hydroxy, $C_1$-$C_2$-Alkyl und ($C_1$-$C_2$-Alkyl)-C(=O)- ausgewählt ist;
$R^7$ für ein Wasserstoffatom oder eine $C_1$-$C_2$-Alkylgruppe steht;
$R^8$ für eine -C(=O)-$NH_2$-Gruppe steht;
$R^9$ für ein Wasserstoffatom oder eine Gruppe steht, die aus $C_1$-$C_4$-Alkyl, ($C_3$-$C_7$-Cycloalkyl) - ($C_1$-$C_2$-alkyl)-, (Phenyl) - ($C_1$-$C_2$-alkyl)-, $C_1$-$C_4$-Halogenalkyl, $C_2$-$C_3$-Hydroxyalkyl, ($C_1$-$C_2$-Alkoxy)-$C_2$-alkyl-, (($C_1$-$C_2$-Alkyl)-C(=O)-O)-$C_2$-alkyl-, -C(=O)-N($R^{20}$)($R^{21}$), $C_3$-$C_7$-Cycloalkyl und Phenyl ausgewählt ist, wobei $C_3$-$C_7$-Cycloalkyl und das $C_3$-$C_7$-Cycloalkyl in der genannten ($C_3$-$C_7$-Cycloalkyl)- ($C_1$-$C_2$-alkyl)-Gruppe gegebenenfalls ein- oder zweifach substituiert sind, wobei jeder Substituent unabhängig aus einem Fluoratom oder einer Gruppe ausgewählt aus Oxo und Methyl ausgewählt ist, und wobei die Phenylgruppe in der genannten (Phenyl) - ($C_1$-$C_2$-alkyl)-Gruppe und die genannte Phenylgruppe selbst gegebenenfalls ein- oder zweifach substituiert sind, wobei jeder Substituent unabhängig aus einem Fluoratom, einem Chloratom und einem Bromatom oder einer Gruppe ausgewählt aus Cyano, Methyl, Trifluormethyl und Methoxy ausgewählt ist;
$R^{10}$ und $R^{11}$ unabhängig von jedem Auftreten für ein Wasserstoffatom oder eine Gruppe ausgewählt aus $C_1$-$C_2$-Alkyl, $C_1$-$C_2$-Halogenalkyl, $C_2$-$C_3$-Hydroxyalkyl, ($C_1$-$C_2$-Alkoxy)-$C_2$-alkyl-, (($R^{22}$)($R^{23}$)N)-$C_2$-Alkyl, ($C_3$-$C_7$-Cycloalkyl)-($C_1$-$C_2$-alkyl)-, ($C_1$-$C_2$-Alkyl)-C(=O)-, $C_3$-$C_7$-Cycloalkyl, ($C_3$-$C_7$-Cycloalkyl)-C (=O)-, (Phenyl)-($C_1$-$C_2$-alkyl)-, (Phenyl)-($C_1$-$C_2$-alkyl) - C(=O)- und (Phenyl) - ($C_1$-$C_2$-alkyl)-O-C (=O) - stehen,
wobei $C_3$-$C_7$-Cycloalkyl und das $C_3$-$C_7$-Cycloalkyl in den genannten ($C_3$-$C_7$-Cycloalkyl)- ($C_1$-$C_2$-alkyl)- und ($C_3$-$C_7$-Cycloalkyl)-C (=O)-Gruppen gegebenenfalls ein- oder zweifach substituiert sind, wobei jeder Substituent unabhängig aus einem Fluoratom oder einer Gruppe ausgewählt aus Cyano, $C_1$-$C_2$-Alkyl und $C_1$-$C_2$-Halogenalkyl ausgewählt ist, und wobei die Phenylgruppen in den genannten (Phenyl)-($C_1$-$C_2$-alkyl)-, (Phenyl)-($C_1$-$C_2$-alkyl)-C(=O)- und (Phenyl)-($C_1$-$C_2$-alkyl)-O-C(=O)-Gruppen gegebenenfalls ein- oder zweifach substituiert sind, wobei jeder Substituent unabhängig aus einem Fluoratom, einem Chloratom und einem Bromatom oder einer Gruppe ausgewählt aus Methyl, Trifluormethyl und Methoxy ausgewählt ist, oder
$R^{10}$ und $R^{11}$ zusammen mit dem Stickstoffatom, an das sie gebunden sind, für eine monocyclische stickstoffhaltige 4- bis 7-gliedrige Heterocycloalkylgruppe oder eine bicyclische stickstoffhaltige 5- bis 10-gliedrige Heterocycloalkylgruppe stehen, wobei diese Gruppen gegebenenfalls ein-, zwei- oder dreifach substituiert sind, wobei jeder Substituent unabhängig aus einem Halogenatom oder einer Gruppe ausgewählt aus Cyano, Oxo, Hydroxy, $C_1$-$C_2$-Alkyl, $C_1$-$C_2$-Halogenalkyl, ($C_1$-$C_2$-Alkyl)-C(=O)-, $C_1$-$C_2$-Alkoxy, -N($R^{22}$)($R^{23}$)und einer monocyclischen 4- bis 7-gliedrigen Heterocycloalkylgruppe ausgewählt ist;
$R^{15}$ und $R^{16}$ unabhängig von jedem Auftreten für ein Wasserstoffatom oder eine Gruppe ausgewählt aus $C_1$-$C_4$-Alkyl, $C_1$-$C_4$-Halogenalkyl, $C_1$-$C_4$-Hydroxyalkyl, (Cyano) -$C_1$-$C_4$-alkyl-, ($C_1$-$C_4$-Alkoxy)-$C_2$-$C_3$-alkyl-,

$(C_1-C_4$-Halogenalkoxy)-$C_2-C_3$-alkyl-, ($C_3-C_7$-Cycloalkyl) - ($C_1-C_3$-alkyl) -, $C_3-C_7$-Cycloalkyl, bicyclischem $C_5-C_{11}$-Cycloalkyl, monocyclischem 4-bis 7-gliedrigem Heterocycloalkyl, (Phenyl) - ($C_1-C_3$-Alkyl und einer 5- oder 6-gliedrigen Heteroarylgruppe stehen,

wobei $C_3-C_7$-Cycloalkyl, das $C_3-C_7$-Cycloalkyl in der genannten ($C_3-C_7$-Cycloalkyl)-($C_1-C_3$-alkyl) - Gruppe, die bicyclischen $C_5-C_{11}$-Cycloalkyl- und die monocyclischen 4- bis 7-gliedrigen Heterocycloalkylgruppen gegebenenfalls ein- oder zweifach substituiert sind, wobei jeder Substituent unabhängig aus einem Halogenatom oder einer Gruppe ausgewählt aus Oxo, $C_1-C_2$-Alkyl, ($C_1-C_2$-Alkyl)-C(=O)- und Cyclopropyl ausgewählt ist, und wobei das Phenyl und die 5- oder 6-gliedrigen Heteroarylgruppen einschließlich der Phenylgruppe in der genannten (Phenyl)-($C_1-C_3$-alkyl)-Gruppe gegebenenfalls ein- oder zweifach substituiert sind, wobei jeder Substituent unabhängig aus einem Fluoratom, einem Chloratom und einem Bromatom oder einer Gruppe ausgewählt aus Methyl, Trifluormethyl und Methoxy ausgewählt ist, oder

$R^{15}$ und $R^{16}$ zusammen mit dem Stickstoffatom, an das sie gebunden sind, für eine monocyclische stickstoffhaltige 4- bis 7-gliedrige Heterocycloalkylgruppe stehen, die gegebenenfalls ein- oder zweifach substituiert ist, wobei jeder Substituent unabhängig aus einem Halogenatom oder einer Gruppe ausgewählt aus Oxo, $C_1-C_2$-Alkyl und ($C_1-C_2$-Alkyl)-C(=O)- ausgewählt ist;

$R^{17}$ für eine $C_1-C_4$-Alkylgruppe steht;

$R^{20}$ für ein Wasserstoffatom oder eine Gruppe ausgewählt aus $C_1-C_6$-Alkyl, $C_3-C_7$-Cycloalkyl, monocyclischem 4-bis 7-gliedrigem Heterocycloalkyl, Phenyl und 5- bis 6-gliedrigem Heteroaryl steht, wobei die genannte $C_1-C_6$-Alkylgruppe gegebenenfalls ein-, zwei- oder dreifach substituiert ist, wobei jeder Substituent unabhängig aus einem Halogenatom oder einer Gruppe ausgewählt aus Hydroxy, Cyano, $C_1-C_3$-Alkoxy, -N ($R^{22}$)($R^{23}$), $C_3-C_7$-Cycloalkyl, monocyclischem 4- bis 7-gliedrigem Heterocycloalkyl, Phenyl und 5- bis 6-gliedrigem Heteroaryl ausgewählt ist, wobei die genannten Phenyl- und 5- bis 6-gliedrigen Heteroarylsubstituenten selbst gegebenenfalls ein- oder zweifach substituiert sind, wobei jeder Substituent unabhängig aus einem Fluoratom, einem Chloratom und einem Bromatom oder einer Gruppe ausgewählt aus Methyl, Trifluormethyl und Methoxy ausgewählt ist, und wobei $C_3-C_7$-Cycloalkyl und monocyclisches 4-bis 7-gliedriges Heterocycloalkyl gegebenenfalls ein-, zwei- oder dreifach substituiert sind, wobei jeder Substituent unabhängig aus einem Halogenatom oder einer Gruppe ausgewählt aus Cyano, Oxo, Hydroxy, $C_1-C_2$-Alkyl und ($C_1-C_2$-Alkyl)-C(=O)- ausgewählt ist, und wobei die genannten Phenyl- und 5- bis 6-gliedrigen Heteroarylgruppen gegebenenfalls ein-, zwei- oder dreifach substituiert sind, wobei jeder Substituent unabhängig aus einem Halogenatom oder einer Gruppe ausgewählt aus Cyano, $C_1-C_2$-Alkyl, $C_1-C_2$-Halogenalkyl, $C_1-C_2$-Alkoxy, $C_1-C_2$-Halogenalkoxy, -N($R^{22}$)($R^{23}$) und - C(=O)-N($R^{24}$)($R^{25}$) ausgewählt ist,

$R^{21}$ für ein Wasserstoffatom oder eine $C_1-C_2$-Alkylgruppe steht, oder

$R^{20}$ und $R^{21}$ zusammen mit dem Stickstoffatom, an das sie gebunden sind, für eine monocyclische stickstoffhaltige 4- bis 7-gliedrige Heterocycloalkylgruppe stehen, die ein-, zwei- oder dreifach substituiert ist, wobei jeder Substituent unabhängig aus einem Halogenatom oder einer Gruppe ausgewählt aus Cyano, Oxo, Hydroxy, $C_1-C_2$-Alkyl, $C_1-C_2$-Halogenalkyl, (Phenyl) - ($C_1-C_2$-alkyl)-, ($C_1-C_2$-Alkyl)-C(=O)-, $C_3-C_4$-Cycloalkyl, $C_1-C_2$-Alkoxy, $C_1-C_2$-Halogenalkoxy, -N($R^{22}$)($R^{23}$) und -C(=O)-N($R^{24}$)($R^{25}$) ausgewählt ist;

$R^{22}$ und $R^{23}$ unabhängig von jedem Auftreten für ein Wasserstoffatom oder eine Gruppe, die aus $C_1-C_2$-Alkyl und ($C_1-C_2$-Alkyl)-C(=O)- ausgewählt ist, stehen, und

$R^{24}$ und $R^{25}$ unabhängig von jedem Auftreten für ein Wasserstoffatom oder eine $C_1-C_2$-Alkylgruppe stehen,

oder ein Stereoisomer, ein Tautomer, ein N-Oxid, ein Hydrat, ein Solvat, oder ein Salz davon, oder ein Gemisch davon.

**3.** Verbindung nach Anspruch 1 oder 2, wobei:

$R^1$ für eine Phenyl- oder Pyridinylgruppe steht, die gegebenenfalls ein-, zwei- oder dreifach substituiert ist, wobei jeder Substituent unabhängig aus einem Fluoratom, einem Chloratom und einem Bromatom oder einer Gruppe ausgewählt aus Hydroxy, Cyano, $C_1-C_4$-Alkyl, $C_1-C_2$-Fluoralkyl, $C_1-C_2$-Alkoxy, (Phenyl) - ($C_1-C_2$-alkoxy) -, $C_1-C_2$-Fluoralkoxy und - N($R^5$)($R^6$) ausgewählt ist, oder zwei Substituenten, die an benachbarte Kohlenstoffatome der genannten Phenyl- oder Pyridinylgruppe gebunden sind, zusammen eine zweiwertige Gruppe bilden, die aus $-(CH_2)_3-$, $-O-CH_2-O-$ und $-O-CF_2-O-$ ausgewählt ist, oder

$R^1$ für eine Pyrazolylgruppe steht, die gegebenenfalls durch eine Methylgruppe substituiert ist;

$R^2$ für eine Gruppe

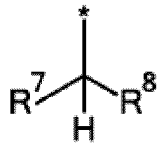

steht, wobei "*" den Verknüpfungspunkt mit dem Stickstoffatom, an das $R^2$ gebunden ist, kennzeichnet;
$R^3$ für eine Gruppe steht, die aus Methyl und $-NH_2$ ausgewählt ist;
$R^4$ für eine Gruppe steht, die aus

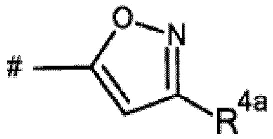

und

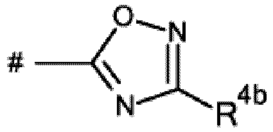

ausgewählt ist,
wobei "#" den Verknüpfungspunkt mit der Carbonylgruppe, an die $R^4$ gebunden ist, kennzeichnet, und wobei $R^{4a}$ und $R^{4b}$ unabhängig voneinander für ein Fluoratom, ein Chloratom oder ein Bromatom oder eine Gruppe ausgewählt aus $C_1$-$C_4$-Alkyl, (($R^9$)O)-($C_1$-$C_2$-Alkyl) -, (($R^{10}$)($R^{11}$)N)-($C_1$-$C_2$-Alkyl) -, ($C_3$-$C_7$-Cycloalkyl) - ($C_1$-$C_2$-alkyl) -, $C_3$-$C_7$-Cycloalkyl, -$OR^9$, -N($R^{10}$)($R^{11}$), - C(=O)-N($R^{15}$)($R^{16}$), -C(=O)-$OR^{17}$, Phenyl und einer 5- oder 6-gliedrigen Heteroarylgruppe, die aus Oxazolyl, Isoxazolyl, Thiazolyl, Isothiazolyl, Pyrazolyl, Pyridinyl, Pyrazinyl, Pyrimidinyl und Pyridazinyl ausgewählt ist, stehen, wobei die Phenylgruppe und die 5- oder 6-gliedrige Heteroarylgruppe, die aus Oxazolyl, Isoxazolyl, Thiazolyl, Isothiazolyl, Pyrazolyl, Pyridinyl, Pyrazinyl, Pyrimidinyl und Pyridazinyl ausgewählt ist, gegebenenfalls ein- oder zweifach substituiert sind, wobei jeder Substituent unabhängig aus einem Fluoratom und einem Chloratom oder einer Gruppe ausgewählt aus Cyano, $C_1$-$C_2$-Alkyl, -$OR^9$, -N($R^{10}$)($R^{11}$) und -C(=O)-N($R^{15}$)($R^{16}$) ausgewählt ist;
$R^5$ und $R^6$ unabhängig von jedem Auftreten für ein Wasserstoffatom oder eine $C_1$-$C_2$-Alkylgruppe stehen, oder $R^5$ und $R^6$ zusammen mit dem Stickstoffatom, an das sie gebunden sind, für eine monocyclische stickstoffhaltige 4- bis 7-gliedrige Heterocycloalkylgruppe stehen, die gegebenenfalls ein- oder zweifach substituiert ist, wobei jeder Substituent unabhängig aus einem Fluoratom oder einer Gruppe ausgewählt aus Hydroxy und $C_1$-$C_2$-Alkyl ausgewählt ist;
$R^7$ für ein Wasserstoffatom oder eine $C_1$-$C_2$-Alkylgruppe steht;
$R^8$ für eine -C(=O)-$NH_2$-Gruppe steht;
$R^9$ für ein Wasserstoffatom oder eine Gruppe ausgewählt aus $C_1$-$C_2$-Alkyl, Benzyl, $C_1$-$C_2$-Fluoralkyl, $C_2$-Hydroxyalkyl, ($C_1$-$C_2$-Alkoxy) -$C_2$-alkyl-, (($C_1$-$C_2$-Alkyl)-C(=O)-O)-$C_2$-alkyl-, -C(=O)-N($R^{20}$)($R^{21}$) und Phenyl steht, wobei die Phenylgruppe in der genannten Benzylgruppe und die genannte Phenylgruppe selbst gegebenenfalls ein- oder zweifach substituiert sind, wobei jeder Substituent unabhängig aus einem Fluoratom und einem Chloratom oder einer Gruppe ausgewählt aus Cyano und Methyl ausgewählt ist;
$R^{10}$ und $R^{11}$ unabhängig von jedem Auftreten für ein Wasserstoffatom oder eine Gruppe ausgewählt aus $C_1$-$C_2$-Alkyl, $C_1$-$C_2$-Fluoralkyl, ($C_3$-$C_5$-Cycloalkyl) - ($C_1$-$C_2$-alkyl) - ($C_1$-$C_2$-alkyl)-C(=O)-, $C_3$-$C_7$-Cycloalkyl, $C_3$-$C_7$-Cycloalkyl- (C=O) -, (Phenyl) -($C_1$-$C_2$-alkyl) -, (Phenyl)-($C_1$-$C_2$-alkyl)-C(=O)- und (Phenyl)-($C_1$-$C_2$-alkyl)-O-C(=O)- stehen, wobei $C_3$-$C_7$-Cycloalkyl und das $C_3$-$C_5$-Cycloalkyl in dem genannten ($C_3$-$C_5$-Cycloalkyl) - ($C_1$-$C_2$-alkyl) - und das $C_3$-$C_7$-Cycloalkyl in den $C_3$-$C_7$-Cycloalkyl-(C=O)-Gruppen gegebenenfalls ein- oder zweifach substituiert sind, wobei jeder Substituent unabhängig aus einem Fluoratom oder einer Gruppe ausgewählt aus Cyano, $C_1$-$C_2$-Alkyl und $C_1$-$C_2$-Fluoralkyl ausgewählt ist, und wobei die Phenylgruppen in den genannten (Phenyl)-($C_1$-$C_2$-alkyl)-, (Phenyl)-($C_1$-$C_2$-alkyl)-C(=O)- und (Phenyl) - ($C_1$-$C_2$-alkyl)-O-C(=O)-Gruppen gegebenenfalls ein- oder zweifach substituiert sind, wobei jeder Substituent unabhängig aus einem Fluoratom, einem Chloratom und einer Methylgruppe ausgewählt ist, oder
$R^{10}$ und $R^{11}$ zusammen mit dem Stickstoffatom, an das sie gebunden sind, für eine monocyclische stickstoffhaltige 4- bis 7-gliedrige Heterocycloalkylgruppe stehen, die gegebenenfalls ein-, zwei- oder dreifach substituiert

ist, wobei jeder Substituent unabhängig aus einem Fluoratom oder einer Gruppe ausgewählt aus Cyano, Oxo, $C_1$-$C_2$-Alkyl, $C_1$-$C_2$-Fluoralkyl und ($C_1$-$C_2$-Alkyl)-C(=O)-ausgewählt ist;

$R^{15}$ und $R^{16}$ unabhängig von jedem Auftreten für ein Wasserstoffatom oder eine Gruppe ausgewählt aus $C_1$-$C_4$-Alkyl, $C_1$-$C_3$-Fluoralkyl, (Cyano) -$C_1$-$C_2$-alkyl-, ($C_1$-$C_4$-Alkoxy)-$C_2$-$C_3$-alkyl-, ($C_1$-$C_2$-Fluoralkoxy)-$C_2$-$C_3$-alkyl-, ($C_3$-$C_5$-Cycloalkyl) - ($C_1$-$C_2$-alkyl)-, $C_3$-$C_5$-Cycloalkyl, Phenyl und einer 5- oder 6-gliedrigen Heteroarylgruppe, die aus Pyrazolyl, Pyridinyl und Pyrimidinyl ausgewählt ist, stehen,

wobei $C_3$-$C_5$-Cycloalkyl und das $C_3$-$C_5$-Cycloalkyl in der genannten ($C_3$-$C_5$-Cycloalkyl) - ($C_1$-$C_2$-alkyl) - Gruppe gegebenenfalls ein- oder zweifach substituiert sind, wobei jeder Substituent unabhängig aus einem Fluoratom oder einer Gruppe ausgewählt aus Methyl und Cyclopropyl ausgewählt ist,

und wobei das Phenyl und die 5- oder 6-gliedrigen Heteroarylgruppen, die aus Pyrazolyl, Pyridinyl und Pyrimidinyl ausgewählt sind, gegebenenfalls ein- oder zweifach substituiert sind, wobei jeder Substituent unabhängig aus einem Fluoratom und einem Chloratom oder einer Gruppe ausgewählt aus Methyl, Trifluormethyl und Methoxy ausgewählt ist,

oder

$R^{15}$ und $R^{16}$ zusammen mit dem Stickstoffatom, an das sie gebunden sind, für eine monocyclische stickstoffhaltige 4- bis 7-gliedrige Heterocycloalkylgruppe stehen, die gegebenenfalls einfach mit einem Fluoratom oder einer Gruppe ausgewählt aus Oxo, $C_1$-$C_2$-Alkyl und $CH_3$-C(=O)-ausgewählt ist, substituiert ist;

$R^{17}$ für eine $C_1$-$C_2$-Alkylgruppe steht;

$R^{20}$ für ein Wasserstoffatom oder eine Gruppe ausgewählt aus $C_1$-$C_3$-Alkyl und Phenyl steht, wobei die genannte $C_1$-$C_3$-Alkylgruppe gegebenenfalls ein- oder zweifach substituiert ist, wobei jeder Substituent unabhängig aus einem Fluoratom oder einer Gruppe ausgewählt aus Hydroxy, $C_1$-$C_3$-Alkoxy und Phenyl ausgewählt ist, wobei das Phenyl selbst gegebenenfalls ein- oder zweifach substituiert ist, wobei jeder Substituent unabhängig aus einem Fluoratom, einem Chloratom und einer Methylgruppe ausgewählt ist, und wobei die genannte Phenylgruppe gegebenenfalls ein-, zwei- oder dreifach substituiert ist, wobei jeder Substituent unabhängig aus einem Fluoratom und einem Chloratom oder einer Gruppe ausgewählt aus Methyl, Trifluormethyl, Methoxy und Trifluormethoxy ausgewählt ist;

$R^{21}$ für ein Wasserstoffatom oder eine $C_1$-$C_2$-Alkylgruppe steht; oder

$R^{20}$ und $R^{21}$ zusammen mit dem Stickstoffatom, an das sie gebunden sind, für eine monocyclische stickstoffhaltige 4- bis 7-gliedrige Heterocycloalkylgruppe stehen, die gegebenenfalls ein- oder zweifach substituiert ist, wobei jeder Substituent unabhängig aus einem Halogenatom oder einer Gruppe ausgewählt aus Cyano, Oxo, Hydroxy, $C_1$-$C_2$-Alkyl, $C_1$-$C_2$-Fluoralkyl, Benzyl, ($C_1$-$C_2$-Alkyl) - C(=O)- und $C_3$-$C_4$-Cycloalkyl ausgewählt ist;

oder ein Stereoisomer, ein Tautomer, ein N-Oxid, ein Hydrat, ein Solvat, oder ein Salz davon, oder ein Gemisch davon.

**4.** Verbindung nach Anspruch 1, 2 oder 3, wobei:

$R^1$ für eine Phenyl- oder Pyridinylgruppe steht, die gegebenenfalls ein- oder zweifach substituiert ist, wobei jeder Substituent unabhängig aus einem Fluoratom, einem Chloratom und einem Bromatom oder einer Gruppe ausgewählt aus Methyl, Difluormethyl, Trifluormethyl, Methoxy, benzyloxy, Difluormethoxy und Trifluormethoxy ausgewählt ist, oder zwei Substituenten, die an benachbarte Kohlenstoffatome der genannten Phenyl- oder Pyridinylgruppe gebunden sind, zusammen eine zweiwertige Gruppe -O-$CF_2$-O- bilden;

$R^2$ für eine Gruppe

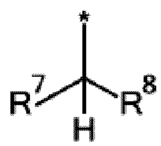

steht,

$R^3$ wobei "*" den Verknüpfungspunkt mit dem Stickstoffatom, an das $R^2$ gebunden ist, kennzeichnet; für eine Gruppe steht, die aus Methyl und -$NH_2$ ausgewählt ist;

$R^4$ für eine Gruppe

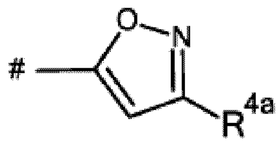

steht,
wobei "#" den Verknüpfungspunkt mit der Carbonylgruppe, an die $R^4$ gebunden ist, kennzeichnet, und wobei $R^{4a}$ für ein Chloratom oder ein Bromatom oder eine Gruppe ausgewählt aus $((R^9)O)-(C_1-C_2\text{-Alkyl})-$, $((R^{10})(R^{11})N)-(C_1-C_2\text{-Alkyl})-$, $(C_3-C_5\text{-Cycloalkyl})-(C_1-C_2\text{-alkyl})-$, $C_3-C_5$-Cycloalkyl, $-OR^9$, $-C(=O)-N(R^{15})(R^{16})$ und Phenyl steht, wobei die Phenylgruppe gegebenenfalls ein- oder zweifach substituiert ist, wobei jeder Substituent unabhängig aus einem Fluoratom und einem Chloratom und einer Methylgruppe ausgewählt ist, oder
$R^4$ für eine Gruppe

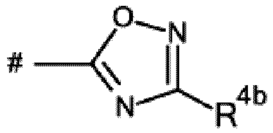

steht,
wobei "#" den Verknüpfungspunkt mit der Carbonylgruppe, an die $R^4$ gebunden ist, kennzeichnet, und wobei $R^{4b}$ für eine Gruppe steht, die aus $C_1-C_4$-Alkyl, $C_3-C_5$-Cycloalkyl, $-C(=O)-N(R^{15})(R^{16})$ und Phenyl ausgewählt ist, wobei die Phenylgruppe gegebenenfalls ein- oder zweifach substituiert ist, wobei jeder Substituent unabhängig aus einem Fluoratom und einem Chloratom oder einer Gruppe ausgewählt aus Cyano, $C_1-C_2$-Alkyl und $-OR^9$ ausgewählt ist;
$R^7$ für ein Wasserstoffatom oder eine $C_1-C_2$-Alkylgruppe steht;
$R^8$ für eine $-C(=O)-NH_2$-Gruppe steht;
$R^9$ für ein Wasserstoffatom oder eine Gruppe ausgewählt aus $C_1-C_2$-Alkyl, Benzyl, $C_1-C_2$-Fluoralkyl, $(C_1-C_2\text{-Alkoxy})-C_2\text{-alkyl-}$, $((C_1-C_2\text{-Alkyl})-C(=O)-O)-C_2\text{-alkyl-}$, $-C(=O)-N(R^{20})(R^{21})$ und Phenyl steht, wobei die Phenylgruppe in der genannten Benzylgruppe und die genannte Phenylgruppe selbst gegebenenfalls ein- oder zweifach substituiert sind, wobei jeder Substituent unabhängig aus einem Fluoratom und einem Chloratom oder einer Gruppe ausgewählt aus Cyano und Methyl ausgewählt ist;
$R^{10}$ und $R^{11}$ unabhängig von jedem Auftreten für ein Wasserstoffatom oder eine Gruppe ausgewählt aus $C_1-C_2$-Alkyl, $(C_3-C_5\text{-Cycloalkyl})-(C_1-C_2\text{-alkyl})-$, $C_3-C_7$-Cycloalkyl und $(\text{Phenyl})-(C_1-C_2\text{-alkyl})-O-C(=O)-$ stehen, wobei $C_3-C_7$-Cycloalkyl und das $C_3-C_5$-Cycloalkyl in der genannten $(C_3-C_5\text{-Cycloalkyl})-(C_1-C_2\text{-alkyl})-$Gruppe gegebenenfalls ein- oder zweifach substituiert sind, wobei jeder Substituent unabhängig aus einem Fluoratom oder einer Gruppe ausgewählt aus Cyano, Methyl und $C_1$-Fluoralkyl ausgewählt ist,
und wobei die Phenylgruppe in der genannten $(\text{Phenyl})-(C_1-C_2\text{-alkyl})-O-C(=O)$-Gruppe gegebenenfalls ein- oder zweifach substituiert ist, wobei jeder Substituent unabhängig aus einem Fluoratom, einem Chloratom und einer Methylgruppe ausgewählt ist, oder
$R^{10}$ und $R^{11}$ zusammen mit dem Stickstoffatom, an das sie gebunden sind, für eine monocyclische stickstoffhaltige 4- bis 7-gliedrige Heterocycloalkylgruppe stehen, die gegebenenfalls ein-, zwei- oder dreifach substituiert ist, wobei jeder Substituent unabhängig aus einem Fluoratom oder einer Gruppe ausgewählt aus Cyano, Methyl und $C_1$-Fluoralkyl ausgewählt ist;
$R^{15}$ und $R^{16}$ unabhängig von jedem Auftreten für ein Wasserstoffatom oder eine Gruppe ausgewählt aus $C_1-C_4$-Alkyl, $C_1-C_2$-Fluoroalkyl, $(C_1-C_4\text{-Alkoxy})-C_2-C_3\text{-alkyl-}$, $(C_1-C_2\text{-Fluoralkoxy})-C_2-C_3\text{-alkyl-}$, $(C_3-C_5\text{-Cycloalkyl})-(C_1-C_2\text{-alkyl})-$, $C_3-C_5$-Cycloalkyl, Phenyl und Pyridinyl stehen, wobei $C_3-C_5$-Cycloalkyl und das $C_3-C_5$-Cycloalkyl in der genannten $(C_3-C_5\text{-Cycloalkyl})-(C_1-C_2\text{-alkyl})-$Gruppe gegebenenfalls ein- oder zweifach substituiert sind, wobei jeder Substituent unabhängig aus einem Fluoratom und einer Methylgruppe ausgewählt ist, und wobei die Phenyl- und Pyridinylgruppen gegebenenfalls ein- oder zweifach substituiert sind, wobei jeder Substituent unabhängig aus einem Fluoratom und einem Chloratom oder einer Gruppe ausgewählt aus Methyl, Trifluormethyl und Methoxy ausgewählt ist;
$R^{20}$ für eine Gruppe steht, die aus Benzyl und Phenyl ausgewählt ist, wobei die genannte Phenylgruppe und die Phenylgruppe in der genannten Benzylgruppe gegebenenfalls ein- oder zweifach substituiert ist, wobei jeder Substituent unabhängig aus einem Fluoratom, einem Chloratom und einer Methylgruppe ausgewählt ist, und
$R^{21}$ für ein Wasserstoffatom oder eine Methylgruppe steht,

oder ein Stereoisomer, ein Tautomer, ein N-Oxid, ein Hydrat, ein Solvat, oder ein Salz davon, oder ein Gemisch davon.

**5.** Verbindung nach Anspruch 1, 2 oder 3, wobei:

$R^1$ für eine Gruppe

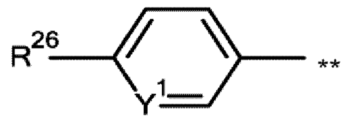

steht,
wobei "**" den Verknüpfungspunkt mit dem Stickstoffatom, an das $R^1$ gebunden ist, kennzeichnet;
$R^2$ für eine Gruppe

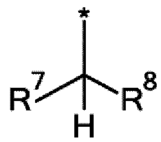

steht,
wobei "*" den Verknüpfungspunkt mit dem Stickstoffatom, an das $R^2$ gebunden ist, kennzeichnet;
$R^3$ für eine Gruppe steht, die aus Methyl und $-NH_2$ ausgewählt ist;
$R^4$ für eine Gruppe

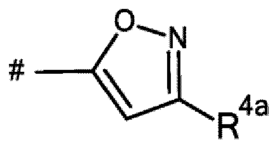

steht,
wobei "#" den Verknüpfungspunkt mit der Carbonylgruppe, an die $R^4$ gebunden ist, kennzeichnet, und wobei $R^{4a}$ für ein Chloratom oder ein Bromatom oder eine Gruppe ausgewählt aus $((R^9)O)-(C_1\text{-Alkyl})-$, $((R^{10})(R^{11})N)-(C_1\text{-Alkyl})$ -, $(C_3\text{-}C_5\text{-Cycloalkyl})-(C_1\text{-alkyl})-$, $C_3\text{-}C_5$-Cycloalkyl, $-OR^9$, $-C(=O)-N(R^{15})(R^{16})$ und Phenyl steht, wobei die Phenylgruppe gegebenenfalls ein- oder zweifach substituiert ist, wobei jeder Substituent unabhängig aus einem Fluoratom und einem Chloratom und einer Methylgruppe ausgewählt ist, oder
$R^4$ für eine Gruppe

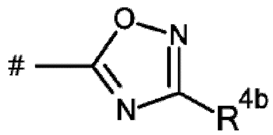

steht,
wobei "#" den Verknüpfungspunkt mit der Carbonylgruppe, an die $R^4$ gebunden ist, kennzeichnet, und wobei $R^{4b}$ für eine Gruppe steht, die aus $C_1\text{-}C_4$-Alkyl, $C_3\text{-}C_5$-Cycloalkyl und Phenyl ausgewählt ist, wobei die Phenylgruppe gegebenenfalls ein- oder zweifach substituiert ist, wobei jeder Substituent unabhängig aus einem Fluoratom und einem Chloratom oder einer Gruppe ausgewählt aus Cyano, $C_1\text{-}C_2$-Alkyl und $-OR^9$ ausgewählt ist;
$R^7$ für ein Wasserstoffatom oder eine $C_1\text{-}C_2$-Alkylgruppe steht;
$R^8$ für eine $-C(=O)-NH_2$-Gruppe steht;
$R^9$ für ein Wasserstoffatom oder eine Gruppe ausgewählt aus $C_1\text{-}C_2$-Alkyl, Benzyl, $C_1\text{-}C_2$-Fluoralkyl, $(C_1\text{-}C_2\text{-Alkoxy})\text{-}C_2\text{-alkyl-}$, $((C_1\text{-}C_2\text{-Alkyl})-C(=O)-O)-C_2\text{-alkyl-}$ und Phenyl steht,
wobei die Phenylgruppe in der genannten Benzylgruppe und die genannte Phenylgruppe selbst gegebenenfalls ein- oder zweifach substituiert sind, wobei jeder Substituent unabhängig aus einem Fluoratom und einem Chloratom oder einer Gruppe ausgewählt aus Cyano und Methyl ausgewählt ist;
$R^{10}$ und $R^{11}$ unabhängig von jedem Auftreten für ein Wasserstoffatom oder eine Gruppe ausgewählt aus $C_1\text{-}C_2$-Alkyl, $C_3\text{-}C_7$-Cycloalkyl und (Benzyl)-O-C(=O)-stehen,

wobei $C_3$-$C_7$-Cycloalkyl gegebenenfalls ein- oder zweifach substituiert ist, wobei jeder Substituent unabhängig aus einem Fluoratom oder einer Gruppe ausgewählt aus Methyl und Trifluormethyl ausgewählt ist, und wobei die Phenylgruppe in der genannten (Benzyl)-O-C(=O)-Gruppe gegebenenfalls ein- oder zweifach substituiert ist, wobei jeder Substituent unabhängig aus einem Fluoratom, einem Chloratom und einer Methylgruppe ausgewählt ist, oder

$R^{10}$ und $R^{11}$ zusammen mit dem Stickstoffatom, an das sie gebunden sind, für eine monocyclische stickstoffhaltige 4- bis 7-gliedrige Heterocycloalkylgruppe stehen, die gegebenenfalls ein-, zwei- oder dreifach substituiert ist, wobei jeder Substituent unabhängig aus einem Fluoratom oder einer Gruppe ausgewählt aus Cyano, Methyl und Trifluormethyl ausgewählt ist;

$R^{15}$ und $R^{16}$ unabhängig von jedem Auftreten für ein Wasserstoffatom oder eine Gruppe ausgewählt aus $C_1$-$C_4$-Alkyl, $C_1$-$C_2$-Fluoralkyl, ($C_1$-$C_4$-Alkoxy)-$C_2$-alkyl-, ($C_1$-$C_2$-Fluoralkoxy)-$C_2$-alkyl-, $C_3$-$C_5$-Cycloalkyl, Phenyl und Pyridinyl stehen,

wobei $C_3$-$C_5$-Cycloalkyl gegebenenfalls ein- oder zweifach substituiert ist, wobei jeder Substituent unabhängig aus einem Fluoratom und einer Methylgruppe ausgewählt ist;

$Y^1$ für -C(H)=, -C(F)=, -C(Cl)=, -C(CN)= oder -N= steht, und

$R^{26}$ für ein Fluoratom, ein Chloratom oder ein Bromatom oder eine Gruppe ausgewählt aus Methyl, Difluormethyl, Trifluormethyl, Methoxy, Benzyloxy, Difluormethoxy und Trifluormethoxy steht,

oder ein Stereoisomer, ein Tautomer, ein N-Oxid, ein Hydrat, ein Solvat, oder ein Salz davon, oder ein Gemisch davon.

**6.** Verbindung nach Anspruch 1, 2, 3, 4 oder 5, wobei:

$R^1$ für eine Gruppe

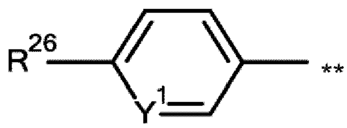

steht,

wobei "**" den Verknüpfungspunkt mit dem Stickstoffatom, an das $R^1$ gebunden ist, kennzeichnet;

$R^2$ für eine Gruppe

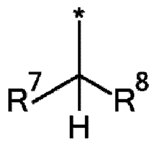

steht,

wobei "*" den Verknüpfungspunkt mit dem Stickstoffatom, an das $R^2$ gebunden ist, kennzeichnet;

$R^3$ für eine Gruppe steht, die aus Methyl und $-NH_2$ ausgewählt ist;

$R^4$ für eine Gruppe

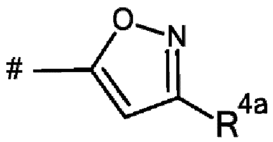

steht,

wobei "#" den Verknüpfungspunkt mit der Carbonylgruppe, an die $R^4$ gebunden ist, kennzeichnet, und wobei $R^{4a}$ für ein Chloratom oder ein Bromatom oder eine Gruppe ausgewählt aus (($R^9$)O)-($C_1$-Alkyl)-, $C_3$-$C_5$-Cycloalkyl, $-OR^9$ und -C(=O)-N($R^{15}$)($R^{16}$) steht, oder

$R^4$ für eine Gruppe

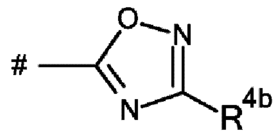

steht,
wobei "#" den Verknüpfungspunkt mit der Carbonylgruppe, an die $R^4$ gebunden ist, kennzeichnet, und wobei $R^{4b}$ für eine Gruppe steht, die aus $C_1$-$C_4$-Alkyl, $C_3$-$C_5$-Cycloalkyl und Phenyl ausgewählt ist, wobei die Phenylgruppe gegebenenfalls ein- oder zweifach substituiert ist, wobei jeder Substituent unabhängig aus einem Fluoratom und einem Chloratom und einer Methylgruppe ausgewählt ist;
$R^7$ für ein Wasserstoffatom oder eine $C_1$-$C_2$-Alkylgruppe steht;
$R^8$ für eine -C(=O)-$NH_2$-Gruppe steht;
$R^9$ für ein Wasserstoffatom oder eine Gruppe ausgewählt aus $C_1$-$C_2$-Alkyl, Benzyl, $C_1$-$C_2$-Fluoralkyl und Phenyl steht,
wobei die Phenylgruppe in der genannten Benzylgruppe und die genannte Phenylgruppe selbst gegebenenfalls ein- oder zweifach substituiert sind, wobei jeder Substituent unabhängig aus einem Fluoratom und einem Chloratom oder einer Gruppe ausgewählt aus Cyano und Methyl ausgewählt ist;
$R^{15}$ und $R^{16}$ unabhängig von jedem Auftreten für ein Wasserstoffatom oder eine Gruppe ausgewählt aus $C_1$-$C_2$-Alkyl, $C_3$-$C_5$-Cycloalkyl, Phenyl und Pyridinyl stehen, wobei $C_3$-$C_5$-Cycloalkyl gegebenenfalls ein- oder zweifach substituiert ist, wobei jeder Substituent unabhängig aus einem Fluoratom und einer Methylgruppe ausgewählt ist;
$Y^1$ für -C(H)=, -C(F)=, -C(Cl)= oder -N= steht, und
$R^{26}$ für ein Fluoratom, ein Chloratom oder ein Bromatom oder eine Gruppe ausgewählt aus Difluormethyl, Methoxy, Benzyloxy, Difluormethoxy und Trifluormethoxy steht,
oder ein Stereoisomer, ein Tautomer, ein N-Oxid, ein Hydrat, ein Solvat, oder ein Salz davon, oder ein Gemisch davon.

**7.** Verbindung nach Anspruch 1, 2, 3, 4, 5 oder 6, wobei:

$R^1$ für eine Gruppe

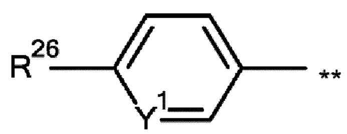

steht,
wobei "**" den Verknüpfungspunkt mit dem Stickstoffatom, an das $R^1$ gebunden ist, kennzeichnet;
$R^2$ für eine Gruppe

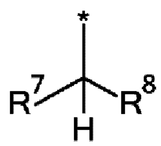

steht,
wobei "*" den Verknüpfungspunkt mit dem Stickstoffatom, an das $R^2$ gebunden ist, kennzeichnet;
$R^3$ für eine Gruppe -$NH_2$ steht;
$R^4$ für eine Gruppe

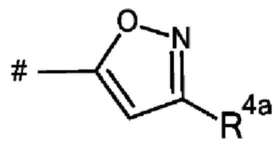

steht,

wobei "#" den Verknüpfungspunkt mit der Carbonylgruppe, an die $R^4$ gebunden ist, kennzeichnet, und wobei $R^{4a}$ für ein Chloratom oder ein Bromatom oder eine Gruppe ausgewählt aus $((R^9)O)-(C_1\text{-Alkyl})-$, $C_3$-$C_5$-Cycloalkyl, $-OR^9$ und $-C(=O)-N(R^{15})(R^{16})$ steht, oder
$R^4$ für eine Gruppe

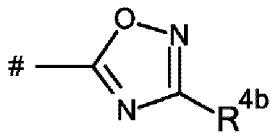

steht,
wobei "#" den Verknüpfungspunkt mit der Carbonylgruppe, an die $R^4$ gebunden ist, kennzeichnet, und wobei $R^{4b}$ für eine Gruppe steht, die aus $C_1$-$C_4$-Alkyl, $C_3$-$C_5$-Cycloalkyl und Phenyl ausgewählt ist, wobei die Phenylgruppe gegebenenfalls ein- oder zweifach substituiert ist, wobei jeder Substituent unabhängig aus einem Fluoratom und einem Chloratom und einer Methylgruppe ausgewählt ist;
$R^7$ für eine Methylgruppe steht;
$R^8$ für eine $-C(=O)-NH_2$-Gruppe steht;
$R^9$ für ein Wasserstoffatom oder eine Gruppe ausgewählt aus $C_1$-$C_2$-Alkyl, Benzyl, $C_1$-$C_2$-Fluoralkyl und Phenyl steht, wobei die Phenylgruppe in der genannten Benzylgruppe und die genannte Phenylgruppe selbst gegebenenfalls ein- oder zweifach substituiert sind, wobei jeder Substituent unabhängig aus einem Fluoratom und einem Chloratom oder einer Gruppe ausgewählt aus Cyano und Methyl ausgewählt ist;
$R^{15}$ und $R^{16}$ unabhängig von jedem Auftreten für ein Wasserstoffatom oder eine Gruppe ausgewählt aus $C_1$-$C_2$-Alkyl, $C_3$-$C_5$-Cycloalkyl, Phenyl und Pyridinyl stehen, wobei $C_3$-$C_5$-Cycloalkyl gegebenenfalls ein- oder zweifach substituiert ist, wobei jeder Substituent unabhängig aus einem Fluoratom und einer Methylgruppe ausgewählt ist;
$Y^1$ für -C(H)=, -C(F)=, -C(Cl)= oder -N= steht, und
$R^{26}$ für ein Fluoratom, ein Chloratom oder ein Bromatom oder eine Gruppe ausgewählt aus Difluormethyl, Methoxy, Benzyloxy, Difluormethoxy und Trifluormethoxy steht,
oder ein Stereoisomer, ein Tautomer, ein N-Oxid, ein Hydrat, ein Solvat, oder ein Salz davon, oder ein Gemisch davon.

**8.** Verbindung nach Anspruch 1, die ausgewählt ist aus der Gruppe bestehend aus:

rac-2-(N-[4-Amino-5-[3-(4-chlorphenyl)-1,2,4-oxadiazol-5-carbonyl]thiazol-2-yl]-4-fluoranilino)propanamid,
rac-2-(N-[4-Amino-5-(3-benzyloxyisoxazol-5-carbonyl)thiazol-2-yl]-4-fluoranilino)propanamid,
rac-2-(N-[4-Amino-5-(3-phenylisoxazol-5-carbonyl)thiazol-2-yl]-4-fluoranilino)propanamid,
(R)- 2-(N-[4-Amino-5-(3-phenylisoxazol-5-carbonyl)thiazol-2-yl]-4-fluoranilino)propanamid,
(S)-2-(N-[4-Amino-5-(3-phenylisoxazol-5-carbonyl)thiazol-2-yl]-4-fluoranilino)propanamid,
rac-2-(N-[4-Amino-5-[3-(2,4-dichlorphenyl)isoxazol-5-carbonyl]thiazol-2-yl]-4-fluoranilino)propanamid,
rac-2-(N-[4-Amino-5-[3-(4-chlorphenyl)isoxazol-5-carbonyl]thiazol-2-yl]-4-fluoranilino)propanamid,
rac-2-(N-[4-Amino-5-(3-bromisoxazol-5-carbonyl)thiazol-2-yl]-4-fluoranilino)propanamid,
(R)-2-(N-[4-Amino-5-(3-bromisoxazol-5-carbonyl)thiazol-2-yl]-4-fluoranilino)propanamid,
(S)-2-(N-[4-Amino-5-(3-bromisoxazol-5-carbonyl)thiazol-2-yl]-4-fluoranilino)propanamid,
rac-2-(N-[4-Amino-5-[3-(3-pyridyl)isoxazol-5-carbonyl]thiazol-2-yl]-4-fluoranilino)propanamid,
rac-2-(N-[4-Amino-5-[3-(4-pyridyl)isoxazol-5-carbonyl]thiazol-2-yl]-4-fluoranilino)propanamid,
rac-5-[4-Amino-2-(N-(2-amino-1-methyl-2-oxoethyl)-4-fluoranilino)thiazol-5-carbonyl]isoxazol-3-carbonsäureethylester,
rac-5-[4-Amino-2-(N-(2-amino-1-methyl-2-oxoethyl)-4-fluoranilino)thiazol-5-carbonyl]-N-phenyl-isoxazol-3-carboxamid,
5-[4-Amino-2-(N-(2-amino-1-methyl-2-oxoethyl)-4-fluoranilino)thiazol-5-carbonyl]-N-[(cis)-2-fluorcyclopropyl]isoxazol-3-carboxamid (Gemisch von Stereoisomeren),
rac-5-[4-Amino-2-(N-(2-amino-1-methyl-2-oxoethyl)-4-fluoranilino)thiazol-5-carbonyl]-N-(1-methylpyrazol-4-yl)isoxazol-3-carboxamid,
rac-2-(N-[4-Amino-5-[3-(morpholin-4-carbonyl)isoxazol-5-carbonyl]thiazol-2-yl]-4-fluoranilino)propanamid,
rac-5-[4-Amino-2-(N-(2-amino-1-methyl-2-oxoethyl)-4-fluoranilino)thiazol-5-carbonyl]-N-pyrimidin-5-yl-isoxazol-3-carboxamid,
rac-5-[4-Amino-2-(N-(2-amino-1-methyl-2-oxoethyl)-4-fluoranilino)thiazol-5-carbonyl]-N-cyclopropylisoxazol-3-carboxamid,

rac-5-[4-Amino-2-(N-(2-amino-1-methyl-2-oxoethyl)-4-fluoranilino)thiazol-5-carbonyl]-N-(4-pyridyl)isoxazol-3-carboxamid,
rac-5-[4-Amino-2-(N-(2-amino-1-methyl-2-oxoethyl)-4-fluoranilino)thiazol-5-carbonyl]-N-(2,2-difluorethyl)isoxazol-3-carboxamid,
rac-2-(N-[4-Amino-5-[3-(azetidin-1-carbonyl)isoxazol-5-carbonyl]thiazol-2-yl]-4-fluoranilino)propanamid,
rac-2-(N-[4-Amino-5-[3-(pyrrolidin-1-carbonyl)isoxazol-5-carbonyl]thiazol-2-yl]-4-fluoranilino)propanamid,
rac-5-[4-Amino-2-(N-(2-amino-1-methyl-2-oxoethyl)-4-fluoranilino)thiazol-5-carbonyl]-N-(3-pyridyl)isoxazol-3-carboxamid,
rac-5-[4-Amino-2-(N-(2-amino-1-methyl-2-oxoethyl)-4-fluoranilino)thiazol-5-carbonyl]-N-cyclobutyl-isoxazol-3-carboxamid,
rac-5-[4-Amino-2-(N-(2-amino-1-methyl-2-oxoethyl)-4-fluoranilino)thiazol-5-carbonyl]-N-(cyclopropylmethyl) isoxazol-3-carboxamid,
rac-5-[4-Amino-2-(N-(2-amino-1-methyl-2-oxoethyl)-4-fluoranilino)thiazol-5-carbonyl]-N-(2,2,2-trifluorethyl) isoxazol-3-carboxamid,
rac-5-[4-Amino-2-(N-(2-amino-1-methyl-2-oxoethyl)-4-fluoranilino)thiazol-5-carbonyl]-N-(1-cyclopropylcyclopropyl)isoxazol-3-carboxamid,
rac-5-[4-Amino-2-(N-(2-amino-1-methyl-2-oxoethyl)-4-fluoranilino)thiazol-5-carbonyl]-N-cyclopentyl-isoxazol-3-carboxamid,
rac-5-[4-Amino-2-(N-(2-amino-1-methyl-2-oxoethyl)-4-fluoranilino)thiazol-5-carbonyl]-N-(2-tert-butoxyethyl) isoxazol-3-carboxamid,
rac-2-(N-[4-Amino-5-[3-(piperidin-1-carbonyl)isoxazol-5-carbonyl]thiazol-2-yl]-4-fluoranilino)propanamid,
rac-5-[4-Amino-2-(N-(2-amino-1-methyl-2-oxoethyl)-4-fluoranilino)thiazol-5-carbonyl]-N-(2-pyridyl)isoxazol-3-carboxamid,
rac-5-[4-Amino-2-(N-(2-amino-1-methyl-2-oxoethyl)-4-fluoranilino)thiazol-5-carbonyl]-N-[2-(trifluormethoxy) ethyl]isoxazol-3-carboxamid,
rac-5-[4-Amino-2-(N-(2-amino-1-methyl-2-oxoethyl)-4-fluoranilino)thiazol-5-carbonyl]-N-(1-methylcyclobutyl) isoxazol-3-carboxamid,
(R)-5-[4-Amino-2-(N-(2-amino-1-methyl-2-oxoethyl)-4-fluoranilino)thiazol-5-carbonyl]-N-(1-methylcyclobutyl) isoxazol-3-carboxamid,
(S)-5-[4-Amino-2-(N-(2-amino-1-methyl-2-oxoethyl)-4-fluoranilino)thiazol-5-carbonyl]-N-(1-methylcyclobutyl) isoxazol-3-carboxamid,
rac-5-[4-Amino-2-(N-(2-amino-1-methyl-2-oxoethyl)-4-fluoranilino)thiazol-5-carbonyl]-N-(1-bicyclo[1.1.1]-pentanyl)isoxazol-3-carboxamid,
rac-5-[4-Amino-2-(N-(2-amino-1-methyl-2-oxoethyl)-4-fluoranilino)thiazol-5-carbonyl]-N-(3,3-difluorcyclobutyl) isoxazol-3-carboxamid,
(R)-5-[4-Amino-2-(N-(2-amino-1-methyl-2-oxoethyl)-4-fluoranilino)thiazol-5-carbonyl]-N-(3,3-difluorcyclobutyl) isoxazol-3-carboxamid,
(S)-5-[4-Amino-2-(N-(2-amino-1-methyl-2-oxoethyl)-4-fluoranilino)thiazol-5-carbonyl]-N-(3,3-difluorcyclobutyl) isoxazol-3-carboxamid,
5-[4-Amino-2-(N-(2-amino-1-methyl-2-oxoethyl)-4-fluoranilino)thiazol-5-carbonyl]-N-(3,3-difluorcyclopentyl) isoxazol-3-carboxamid (Gemisch von Stereoisomeren),
rac-5-[4-Amino-2-(N-(2-amino-1-methyl-2-oxoethyl)-4-fluoranilino)thiazol-5-carbonyl]-N-isopropyl-isoxazol-3-carboxamid,
rac-5-[4-Amino-2-(N-(2-amino-1-methyl-2-oxoethyl)-4-fluoranilino)thiazol-5-carbonyl]-N-(cyanomethyl)isoxazol-3-carboxamid,
5-[4-Amino-2-(N-(2-amino-1-methyl-2-oxoethyl)-4-fluoranilino)thiazol-5-carbonyl]-N-(2-methoxy-1-methylethyl)isoxazol-3-carboxamid (Gemisch von Stereoisomeren),
5-[4-Amino-2-(N-(2-amino-1-methyl-2-oxoethyl)-4-fluoranilino)thiazol-5-carbonyl]-N-(2,2-dimethylcyclopentyl) isoxazol-3-carboxamid (Gemisch von Stereoisomeren),
5-[4-Amino-2-(N-(2-amino-1-methyl-2-oxoethyl)-4-fluoranilino)thiazol-5-carbonyl]-N-(cis-3-fluorcyclobutyl)isoxazol-3-carboxamid (Gemisch von Stereoisomeren),
rac-5-[4-Amino-2-(N-(2-amino-1-methyl-2-oxoethyl)-4-fluoranilino)thiazol-5-carbonyl]-N-(1-methylcyclopentyl) isoxazol-3-carboxamid,
rac-5-[4-Amino-2-(N-(2-amino-1-methyl-2-oxoethyl)-4-fluoranilino)thiazol-5-carbonyl]-N-[2-Fluor-1-(fluormethyl)ethyl]isoxazol-3-carboxamid,
5-[4-Amino-2-(N-(2-amino-1-methyl-2-oxoethyl)-4-fluoranilino)thiazol-5-carbonyl]-N-(2,2-difluor-1-methylethyl)isoxazol-3-carboxamid (Gemisch von Stereoisomeren),
5-[4-Amino-2-(N-(2-amino-1-methyl-2-oxoethyl)-4-fluoranilino)thiazol-5-carbonyl]-N-(trans-3-fluorcyclobutyl)isoxazol-3-carboxamid (Gemisch von Stereoisomeren),
5-[4-Amino-2-(N-(2-amino-1-methyl-2-oxoethyl)-4-fluoranilino)thiazol-5-carbonyl]-N-(3,3-dimethylcyclopentyl) isoxazol-3-carboxamid (Gemisch von Stereoisomeren),
rac-5-[4-Amino-2-(N-(2-amino-1-methyl-2-oxoethyl)-3,4-difluor-anilino)thiazol-5-carbonyl]-N-(1-methylcyclobutyl)isoxazol-3-carboxamid,
(R)-5-[4-Amino-2-(N-(2-amino-1-methyl-2-oxoethyl)-3,4-difluor-anilino)thiazol-5-carbonyl]-N-(1-methylcyclobutyl)isoxazol-3-carboxamid,
(S)-5-[4-Amino-2-(N-(2-amino-1-methyl-2-oxoethyl)-3,4-difluor-anilino)thiazol-5-carbonyl]-N-(1-methylcyclobutyl)isoxazol-3-carboxamid,
rac-5-[4-Amino-2-(N-(2-amino-1-methyl-2-oxoethyl)-3,4-difluor-anilino)thiazol-5-carbonyl]-N-(3,3-difluorcyclobutyl)isoxazol-3-carboxamid,
(R)-5-[4-Amino-2-(N-(2-amino-1-methyl-2-oxoethyl)-3,4-difluor-anilino)thiazol-5-carbonyl]-N-(3,3-difluorcyclobutyl)isoxazol-3-carboxamid,
(S)-5-[4-Amino-2-(N-(2-amino-1-methyl-2-oxoethyl)-3,4-difluor-anilino)thiazol-5-carbonyl]-N-(3,3-difluorcyclobutyl)isoxazol-3-carboxamid,
rac-5-[4-Amino-2-(N-(2-amino-1-methyl-2-oxoethyl)-3,4-difluor-anilino)thiazol-5-carbonyl]-N-cyclopentyl-isoxazol-3-carboxamid,
(R)-5-[4-Amino-2-(N-(2-amino-1-methyl-2-oxoethyl)-3,4-difluor-anilino)thiazol-5-carbonyl]-N-cyclopentyl-isoxazol-3-carboxamid,
(S)-5-[4-Amino-2-(N-(2-amino-1-methyl-2-oxoethyl)-3,4-difluor-anilino)thiazol-5-carbonyl]-N-cyclopentyl-isoxazol-3-carboxamid,
rac-2-(N-[4-Amino-5-(3-cyclopentylisoxazol-5-carbonyl)thiazol-2-yl]-4-fluoranilino)propanamid,
rac-2-(N-[4-Amino-5-[3-(difluormethoxy)isoxazol-5-carbonyl]thiazol-2-yl]-4-fluoranilino)propanamid,
rac-2-(N-[4-Amino-5-(3-isopropyl-1,2,4-oxadiazol-5-carbonyl)thiazol-2-yl]-4-fluoranilino)propanamid,
rac-2-(N-[4-Amino-5-(3-phenylisothiazol-5-carbonyl)thiazol-2-yl]-4-fluoranilino)propanamid,
rac-2-(N-[4-Amino-5-(3-phenyl-1,2,4-oxadiazol-5-carbonyl)thiazol-2-yl]-4-fluoranilino)propanamid,
rac-2-(N-[4-Amino-5-(3-methyl-1,2,4-oxadiazol-5-carbonyl)thiazol-2-yl]-4-fluoranilino)propanamid,
rac-2-(N-[4-Amino-5-(3-tert-butyl-1,2,4-oxadiazol-5-carbonyl)thiazol-2-yl]-4-fluoranilino)propanamid,
rac-2-(N-[4-Amino-5-[3-(2-pyridyl)-1,2,4-oxadiazol-5-carbonyl]thiazol-2-yl]-4-fluoranilino)propanamid,
rac-2-(N-[4-Amino-5-[3-(3-fluorphenyl)-1,2,4-oxadiazol-5-carbonyl]thiazol-2-yl]-4-fluoranilino)propanamid,
rac-2-(N-[4-Amino-5-[3-(2,3-dimethylphenyl)-1,2,4-oxadiazol-5-carbonyl]thiazol-2-yl]-4-fluoranilino)propanamid,
rac-2-(N-[4-Amino-5-(3-cyclopropyl-1,2,4-oxadiazol-5-carbonyl)thiazol-2-yl]-4-fluoranilino)propanamid,
rac-2-(N-[4-Amino-5-[3-(1-methylpyrazol-4-yl)-1,2,4-oxadiazol-5-carbonyl]thiazol-2-yl]-4-fluoranilino)propanamid,
rac-2-(N-[4-Amino-5-(3-cyclopentyl-1,2,4-oxadiazol-5-carbonyl)thiazol-2-yl]-4-fluoranilino)propanamid,
rac-2-(N-[4-Amino-5-[3-[[cyclopentyl(methyl)amino]methyl]isoxazol-5-carbonyl]thiazol-2-yl]-4-fluoranilino)propanamid,
rac-2-(N-[4-Amino-5-[3-[[(1-methylcyclopentyl)amino]methyl]isoxazol-5-carbonyl]thiazol-2-yl]-4-fluoranilino) propanamid,
rac-2-(N-[4-Amino-5-[3-[(cyclopentylamino)methyl]isoxazol-5-carbonyl]thiazol-2-yl]-4-fluoranilino)propanamid,
rac-2-(N-[4-Amino-5-[3-(1-piperidylmethyl)isoxazol-5-carbonyl]thiazol-2-yl]-4-fluoranilino)propanamid,
rac-2-(N-[4-Amino-5-[3-[(4-cyanophenoxy)methyl]isoxazol-5-carbonyl]thiazol-2-yl]-4-fluoranilino)propanamid,
rac-2-(N-[4-Amino-5-[3-(hydroxymethyl)isoxazol-5-carbonyl]thiazol-2-yl]-4-fluoranilino)propanamid,
rac-[5-[4-Amino-2-(N-(2-amino-1-methyl-2-oxoethyl)-4-fluoranilino)thiazol-5-carbonyl]isoxazol-3-yl]methyl-N-phenylcarbamat,
rac-2-(N-[4-Amino-5-[3-(2-fluorphenyl)isoxazol-5-carbonyl]thiazol-2-yl]-4-fluoranilino)propanamid,
rac-2-(N-[4-Amino-5-[3-(2-pyridyl)isoxazol-5-carbonyl]thiazol-2-yl]-4-fluoranilino)propanamid,
rac-2-(N-[4-Amino-5-[3-[4-(trifluormethoxy)phenyl]isoxazol-5-carbonyl]thiazol-2-yl]-4-fluoranilino)propanamid,
rac-2-(N-[4-Amino-5-[3-(2-methoxyphenyl)isoxazol-5-carbonyl]thiazol-2-yl]-4-fluoranilino)propanamid,
rac-2-(N-[4-Amino-5-[3-(6-methoxy-3-pyridyl)isoxazol-5-carbonyl]thiazol-2-yl]-4-fluoranilino)propanamid,
rac-2-(N-[4-Amino-5-(1-phenyltriazol-4-carbonyl)thiazol-2-yl]-4-fluoranilino)propanamid,
rac-2-(N-[4-Amino-5-[3-[(3-fluorazetidin-1-yl)methyl]isoxazol-5-carbonyl]thiazol-2-yl]-4-fluoranilino)propanamid,
rac-2-(N-[4-Amino-5-[3-[(3,3-difluorazetidin-1-yl)methyl]isoxazol-5-carbonyl]thiazol-2-yl]-4-fluoranilino)propanamid,
rac-2-(N-[4-Amino-5-[3-[(4,4-difluor-1-piperidyl)methyl]isoxazol-5-carbonyl]thiazol-2-yl]-4-fluoranilino)propanamid,

2-(N-[4-Amino-5-[3-[(4,4-difluor-3-methyl-1-piperidyl)methyl]isoxazol-5-carbonyl]thiazol-2-yl]-4-fluoranilino) propanamid (Gemisch von Stereoisomeren),
2-(N-[4-Amino-5-[3-[(3-fluorpyrrolidin-1-yl)methyl]isoxazol-5-carbonyl]thiazol-2-yl]-4-fluoranilino)propanamid (Gemisch von Stereoisomeren),
rac-2-(N-[4-Amino-5-(3-hydroxyisoxazol-5-carbonyl)thiazol-2-yl]-4-fluoranilino)propanamid,
rac-2-(N-[4-Amino-5-[3-(cyclobutoxy)isoxazol-5-carbonyl]thiazol-2-yl]-4-fluoranilino)propanamid,
rac-2-(N-[4-Amino-5-[3-(cyclobutylmethoxy)isoxazol-5-carbonyl]thiazol-2-yl]-4-fluoranilino)propanamid,
rac-2-(N-[4-Amino-5-[3-(4,4-difluorcyclohexoxy)isoxazol-5-carbonyl]thiazol-2-yl]-4-fluoranilino)propanamid und
rac-2-(N-[4-Amino-5-(3-isopropoxyisoxazol-5-carbonyl)thiazol-2-yl]-4-fluoranilino)propanamid,
oder ein Stereoisomer, ein Tautomer, ein N-Oxid, ein Hydrat, ein Solvat, oder ein Salz davon, oder ein Gemisch davon.

**9.** Verbindung der allgemeinen Formel (I) nach einem der Ansprüche 1 bis 8 zur Verwendung bei der Behandlung oder Prophylaxe einer Erkrankung.

**10.** Pharmazeutische Zusammensetzung, umfassend eine Verbindung der allgemeinen Formel (I) nach einem der Ansprüche 1 bis 8 und einen oder mehrere pharmazeutisch unbedenkliche Hilfsstoffe.

**11.** Pharmazeutische Kombination, umfassend:

- eine oder mehrere Verbindungen der allgemeinen Formel (I) nach einem der Ansprüche 1 bis 8 und
- einen oder mehrere Immuncheckpoint-Inhibitoren.

**12.** Pharmazeutische Kombination nach Anspruch 11, wobei es sich bei dem Immuncheckpoint-Inhibitor um einen Antagonisten der aPD-1/-L1-Achse handelt.

**13.** Pharmazeutische Kombination nach Anspruch 11, wobei es sich bei dem Immuncheckpoint-Inhibitor um einen Inhibitor von DGK$\alpha$ handelt.

**14.** Verbindung zur Verwendung nach Anspruch 9, wobei es sich bei der Erkrankung um Krebs oder ein Leiden mit dysregulierter Immunantwort oder eine mit aberranter DGK$\zeta$-Signalgebung assoziierte Störung handelt.

## Revendications

**1.** Composé de formule générale (I) :

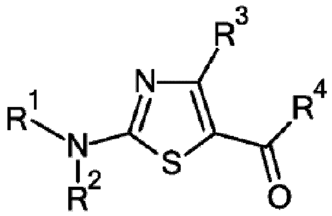
(I)

dans laquelle :

$R^1$ représente un groupe phényle ou hétéroaryle à 6 chaînons éventuellement substitué, une, deux ou trois fois, chaque substituant étant indépendamment choisi parmi un atome d'halogène ou un groupe choisi parmi hydroxy, cyano, nitro, $C_1$-$C_6$-alkyle, (phényl)-($C_1$-$C_3$-alkyle) -, $C_1$-$C_6$-halogénoalkyle, $C_1$-$C_6$-alcoxy, (phényl) - ($C_1$-$C_3$-alcoxy)-, $C_1$-$C_6$-halogénoalcoxy, - N($R^5$)($R^6$),

dans laquelle les groupes phényle dans lesdits groupes (phényl) - ($C_1$-$C_3$-alkyle) - et (phényl) - ($C_1$-$C_3$-alcoxy)- sont éventuellement substitués une ou deux fois, chaque substituant étant indépendamment choisi parmi un atome d'halogène ou un groupe choisi parmi cyano, méthyle, éthyle, trifluorométhyle, méthoxy, éthoxy, diméthylamino et trifluorométhoxy,

ou deux substituants fixés à des atomes de carbone adjacents dudit groupe phényle ou hétéroaryle à 6 chaînons formant un groupe bivalent choisi parmi - $(CH_2)_3$-, -$(CH_2)_4$-, -$(CH_2)_2$-O-, -$(CH_2)_3$-O-, -$CH_2$-O-$CH_2$-, -$(CH_2)_2$-O-$CH_2$-, -O-$CH_2$-O-, -O-$CH_2$-$CH_2$-O-, -O-$CF_2$-O-, -O-$CH_2$-$CF_2$-O-, et -O-$CF_2$-$CF_2$-O-,

ou
$R^1$ représente un groupe hétéroaryle à 5 chaînons éventuellement substitué une ou deux fois, chaque substituant étant indépendamment choisi parmi un atome d'halogène ou un groupe choisi parmi cyano, $C_1$-$C_3$-alkyle, et $C_1$-$C_3$-alcoxy ;
$R^2$ représente un groupe

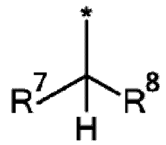

,

dans lequel « * » indique le point de fixation à l'atome d'azote auquel $R^2$ est fixé ;
$R^3$ représente un groupe choisi parmi méthyle et -$NH_2$ ;
$R^4$ représente un groupe hétéroaryle à 5 chaînons éventuellement substitué une ou deux fois, chaque substituant étant indépendamment choisi parmi un atome d'halogène ou un groupe choisi parmi $C_1$-$C_6$-alkyle, (($R^9$)O)-($C_1$-$C_3$-alkyle)-, (($R^{10}$)($R^{11}$)N) -($C_1$-$C_3$-alkyle) -, ($C_3$-$C_7$-cycloalkyl)-($C_1$-$C_3$-alkyle)-, $C_3$-$C_7$-cycloalkyle, -O$R^9$, -N($R^{10}$)($R^{11}$), -C(=O)-N($R^{15}$)($R^{16}$), - C(=O)-O$R^{17}$, phényle et un groupe hétéroaryle à 5 ou 6 chaînons, dans laquelle le groupe phényle et le groupe hétéroaryle à 5 ou 6 chaînons sont éventuellement substitués une ou deux fois, chaque substituant étant indépendamment choisi parmi un atome d'halogène ou un groupe choisi parmi cyano, $C_1$-$C_3$-alkyle, $C_1$-$C_3$-halogénoalkyle, (($R^{22}$)($R^{23}$)N)-$C_1$-$C_3$-alkyle, -O$R^9$, -N($R^{10}$)($R^{11}$)et -C(=O)-N($R^{15}$)($R^{16}$);
$R^5$ et $R^6$ représentent, indépendamment de chaque occurrence, un atome d'hydrogène ou un groupe choisi parmi $C_1$-$C_4$-alkyle, ($C_1$-$C_4$-alkyl)-C(=O)-, $C_3$-$C_4$-cycloalkyle et (phényl)-($C_1$-$C_3$-alkyle)-, ou
$R^5$ et $R^6$, conjointement avec l'atome d'azote auquel ils sont fixés, représentent un groupe hétérocycloalkyle contenant de l'azote monocyclique à 4 à 7 chaînons qui est éventuellement substitué une, deux ou trois fois, chaque substituant étant indépendamment choisi parmi un atome d'halogène ou un groupe choisi parmi oxo, hydroxy, $C_1$-$C_4$-alkyle, ($C_1$-$C_4$-alkyl)-C(=O)-, $C_3$-$C_4$-cycloalkyle et $C_1$-$C_4$-alcoxy ;
$R^7$ représente un atome d'hydrogène ou un groupe $C_1$-$C_2$-alkyle ;
$R^8$ représente un groupe choisi parmi -C(=O)-$NH_2$ et - S(=O)$_2$-$NH_2$ ;
$R^9$ représente un atome d'hydrogène ou un groupe choisi parmi $C_1$-$C_6$-alkyle, ($C_3$-$C_7$-cycloalkyl) - ($C_1$-$C_3$-alkyle) -, (phényl)-($C_1$-$C_3$-alkyle)-, (hétéroaryle à 5 ou 6 chaînons) -($C_1$-$C_3$-alkyle)-, $C_1$-$C_6$-halogénoalkyle, $C_2$-$C_4$-hydroxyalkyle, ($C_1$-$C_3$-alcoxy)-$C_2$-$C_3$-alkyle-, (($C_1$-$C_3$-alkyl)-C(=O)-O)-$C_2$-$C_3$-alkyle-, -C($R^{18}$)($R^{19}$)-C(=O)-O$R^{17}$, -C($R^{18}$)($R^{19}$)-C(=O)-N($R^{20}$)($R^{21}$), -C (=O) -N($R^{20}$)($R^{21}$), $C_3$-$C_7$-cycloalkyle, phényle et un groupe hétéroaryle à 5 ou 6 chaînons,
dans laquelle $C_3$-$C_7$-cycloalkyle, et le $C_3$-$C_7$-cycloalkyle dans ledit groupe ($C_3$-$C_7$-cycloalkyl)-($C_1$-$C_3$-alkyle)- sont éventuellement substitués une ou deux fois, chaque substituant étant indépendamment choisi parmi un atome de fluor ou un groupe choisi parmi oxo, cyano, $C_1$-$C_2$-alkyle et $C_1$-$C_2$-halogénoalkyle, et dans laquelle le groupe phényle dans ledit groupe (phényl)-($C_1$-$C_3$-alkyle)- et ledit groupe phényle lui-même, et le groupe hétéroaryle à 5 ou 6 chaînons dans ledit groupe (hétéroaryle à 5 ou 6 chaînons) -($C_1$-$C_3$-alkyle)- et ledit groupe hétéroaryle à 5 ou 6 chaînons lui-même, sont éventuellement substitués une ou deux fois, chaque substituant étant indépendamment choisi parmi un atome d'halogène ou un groupe choisi parmi cyano, méthyle, éthyle, trifluorométhyle, méthoxy, éthoxy, diméthylamino et trifluorométhoxy ;
$R^{10}$ et $R^{11}$ représentent, indépendamment de chaque occurrence, un atome d'hydrogène ou un groupe choisi parmi $C_1$-$C_4$-alkyle, $C_1$-$C_4$-halogénoalkyle, $C_2$-$C_4$-hydroxyalkyle, ($C_1$-$C_3$-alcoxy)-$C_2$-$C_3$-alkyle-, (($R^{22}$)($R^{23}$)N)-$C_2$-$C_3$-alkyle, ($C_3$-$C_7$-cycloalkyl)-($C_1$-$C_3$-alkyle) -, ($C_1$-$C_4$-alkyl) -C (=O) -, $C_3$-$C_7$-cycloalkyle, ($C_3$-$C_7$-cycloalkyl) -C (=O) -, (phényl)-($C_1$-$C_3$-alkyle)-, (phényl)-($C_1$-$C_3$-alkyl)-C(=O)-, (phényl) - ($C_1$-$C_3$- alkyl)-O-C(=O)-, phényle et un groupe hétéroaryle à 5 ou 6 chaînons,

dans laquelle $C_3$-$C_7$-cycloalkyle, et le $C_3$-$C_7$-cycloalkyle dans lesdits groupes ($C_3$- $C_7$ -cycloalkyl)-($C_1$-$C_3$-alkyle)- et ($C_3$-$C_7$-cycloalkyl)-C(=O)- sont éventuellement substitués une ou deux fois, chaque substituant étant indépendamment choisi parmi un atome de fluor ou un groupe choisi parmi cyano, $C_1$-$C_2$-alkyle et $C_1$-$C_2$-halogénoalkyle,

et dans laquelle ledit phényle et ledit groupe hétéroaryle à 5 ou 6 chaînons, et les groupes phényle dans lesdits groupes (phényl)-($C_1$-$C_3$-alkyle) -, (phényl)-($C_1$-$C_3$-alkyl)-C(=O)- et (phényl) -($C_1$-$C_3$-alkyl)-O-C(=O)-, sont éventuellement substitués une ou deux fois, chaque substituant étant indépendamment choisi parmi un atome d'halogène ou un groupe choisi parmi cyano, méthyle, éthyle, trifluorométhyle, méthoxy, éthoxy, diméthylamino et trifluorométhoxy,

ou

$R^{10}$ et $R^{11}$, conjointement avec l'atome d'azote auquel ils sont fixés, représentent un groupe hétérocycloalkyle contenant de l'azote monocyclique à 4 à 7 chaînons, ou un groupe hétérocycloalkyle contenant de l'azote bicyclique à 5 à 11 chaînons, qui sont éventuellement substitués une, deux ou trois fois, chaque substituant étant indépendamment choisi parmi un atome d'halogène ou un groupe choisi parmi cyano, oxo, hydroxy, $C_1$-$C_4$-alkyle, $C_1$-$C_4$-halogénoalkyle, ($C_1$-$C_4$-alkyl)-C(=O)-, $C_3$-$C_7$-cycloalkyle, $C_1$-$C_4$-alcoxy, -N($R^{22}$)($R^{23}$), et un groupe monocyclique hétérocycloalkyle à 4 à 7 chaînons ;

$R^{15}$ et $R^{16}$ représentent, indépendamment de chaque occurrence, un atome d'hydrogène ou un groupe choisi parmi $C_1$-$C_4$-alkyle, $C_1$-$C_4$-halogénoalkyle, $C_1$-$C_4$-hydroxyalkyle, (cyano) -$C_1$-$C_4$-alkyle-, ($C_1$-$C_4$-alcoxy)-$C_2$-$C_3$-alkyle-, ($C_1$-$C_4$-halogénoalcoxy)-$C_2$-$C_3$-alkyle-, (phénoxy)-$C_2$-$C_3$-alkyle-, ($C_3$-$C_7$-cycloalkyl)-($C_1$-$C_3$-alkyle) -, $C_3$-$C_7$-cycloalkyle, $C_5$-$C_{11}$-cycloalkyle bicyclique, hétérocycloalkyle monocyclique à 4 à 7 chaînons, (phényl) -($C_1$-$C_3$-alkyle)-, phényle et un groupe hétéroaryle à 5 ou 6 chaînons,

dans laquelle $C_3$-$C_7$-cycloalkyle, le $C_3$-$C_7$-cycloalkyle dans ledit groupe ($C_3$-$C_7$-cycloalkyl)-($C_1$-$C_3$-alkyl)-, le $C_5$-$C_{11}$-cycloalkyle bicyclique et les groupes hétérocycloalkyle monocycliques à 4 à 7 chaînons sont éventuellement substitués une, deux ou trois fois, chaque substituant étant indépendamment choisi parmi un atome d'halogène ou un groupe choisi parmi oxo, hydroxy, $C_1$-$C_4$-alkyle, ($C_1$-$C_4$-alkyl)-C(=O)-, $C_3$-$C_4$-cycloalkyle et $C_1$-$C_4$-alcoxy,
et dans laquelle le phényle et les groupes hétéroaryle à 5 ou 6 chaînons, y compris les groupes phényle dans lesdits groupes (phénoxy)-$C_2$-$C_3$-alkyle- et (phényl)-($C_1$-$C_3$-alkyle)-, sont éventuellement substitués une ou deux fois, chaque substituant étant indépendamment choisi parmi un atome d'halogène ou un groupe choisi parmi cyano, méthyle, éthyle, trifluorométhyle, méthoxy, éthoxy, diméthylamino et trifluorométhoxy,
ou

$R^{15}$ et $R^{16}$, conjointement avec l'atome d'azote auquel ils sont fixés, représentent un groupe hétérocycloalkyle contenant de l'azote monocyclique à 4 à 7 chaînons qui est éventuellement substitué une, deux ou trois fois, chaque substituant étant indépendamment choisi parmi un atome d'halogène ou un groupe choisi parmi oxo, hydroxy, $C_1$-$C_4$-alkyle, ($C_1$-$C_4$-alkyl)-C(=O)-, $C_3$-$C_4$-cycloalkyle et $C_1$-$C_4$-alcoxy ;
$R^{17}$ représente un groupe $C_1$-$C_4$-alkyle ;
$R^{18}$ et $R^{19}$ représentent, indépendamment de chaque occurrence, un atome d'hydrogène ou un groupe $C_1$-$C_4$-alkyle ;
$R^{20}$ représente un atome d'hydrogène ou un groupe choisi parmi $C_1$-$C_6$-alkyle, $C_3$-$C_4$-alcényle, $C_3$-$C_4$-alcynyle, $C_1$-$C_3$-alcoxy, $C_3$-$C_7$-cycloalkyle, $C_5$-$C_{11}$-cycloalkyle bicyclique, adamantyle, hétérocycloalkyle monocyclique à 4 à 7 chaînons, hétérocycloalkyle bicyclique à 5 à 11 chaînons, phényle, naphtyle, et hétéroaryle à 5 à 10 chaînons,

dans laquelle ledit groupe $C_1$-$C_6$-alkyle est éventuellement substitué une, deux ou trois fois, chaque substituant étant indépendamment choisi parmi un atome d'halogène ou un groupe choisi parmi hydroxy, cyano, $C_1$-$C_3$-alcoxy, -N($R^{22}$)($R^{23}$), $C_3$-$C_7$-cycloalkyle, $C_5$-$C_{11}$-cycloalkyle bicyclique, adamantyle, hétérocycloalkyle monocyclique à 4 à 7 chaînons, hétérocycloalkyle bicyclique à 5 à 11 chaînons, phényle, et hétéroaryle à 5 à 10 chaînons, lesdits substituants phényle et hétéroaryle à 5 à 10 chaînons eux-mêmes étant éventuellement substitués une ou deux fois, chaque substituant étant indépendamment choisi parmi un atome d'halogène ou un groupe choisi parmi cyano, méthyle, éthyle, trifluorométhyle, méthoxy, éthoxy, diméthylamino et trifluorométhoxy,
et dans laquelle $C_3$-$C_7$-cycloalkyle, $C_5$-$C_{11}$-cycloalkyle bicyclique, adamantyle, hétérocycloalkyle monocyclique à 4 à 7 chaînons et hétérocycloalkyle bicyclique à 5 à 11 chaînons sont éventuellement substitués une, deux ou trois fois, chaque substituant étant indépendamment choisi parmi un atome d'halogène ou un groupe choisi parmi cyano, oxo, hydroxy, $C_1$-$C_4$-alkyle, ($C_1$-$C_4$-alkyl)-C(=O)-, $C_3$-$C_4$-cycloalkyle et $C_1$-$C_4$-alcoxy,
et dans laquelle lesdits groupes phényle, naphtyle et hétéroaryle à 5 à 10 chaînons sont éventuellement substitués une, deux ou trois fois, chaque substituant étant indépendamment choisi parmi un atome

d'halogène ou un groupe choisi parmi cyano, $C_1$-$C_4$-alkyle, $C_1$-$C_4$-halogénoalkyle, $C_1$-$C_4$-alcoxy, $C_1$-$C_4$-halogénoalcoxy, -N($R^{22}$)($R^{23}$) et -C(=O)-N($R^{24}$)($R^{25}$),

$R^{21}$ représente un atome d'hydrogène ou un groupe $C_1$-$C_4$-alkyle, ou
$R^{20}$ et $R^{21}$, conjointement avec l'atome d'azote auquel ils sont fixés, représentent un groupe hétérocycloalkyle contenant de l'azote monocyclique à 4 à 7 chaînons qui est éventuellement benzocondensé, et qui est éventuellement substitué une, deux ou trois fois, chaque substituant étant indépendamment choisi parmi un atome d'halogène ou un groupe choisi parmi cyano, oxo, hydroxy, $C_1$-$C_4$-alkyle, $C_1$-$C_4$-halogénoalkyle, (phényl) - ($C_1$-$C_3$-alkyle) -, ($C_1$-$C_4$-alkyl) -C(=O)-, $C_3$-$C_4$-cycloalkyle, $C_1$-$C_4$-alcoxy, $C_1$-$C_3$-halogénoalcoxy, -N($R^{22}$)($R^{23}$) et -C (=O) -N($R^{24}$)($R^{25}$);

$R^{22}$ et $R^{23}$ représentent, indépendamment de chaque occurrence, un atome d'hydrogène ou un groupe choisi parmi $C_1$-$C_2$-alkyle et ($C_1$-$C_2$-alkyl)-C(=O)-, et
$R^{24}$ et $R^{25}$ représentent, indépendamment de chaque occurrence, un atome d'hydrogène ou un groupe $C_1$-$C_4$-alkyle,
ou stéréoisomère, forme tautomère, N-oxyde, hydrate, solvate, ou sel correspondant, ou mélange de ceux-ci.

**2.** Composé selon la revendication 1, dans lequel :

$R^1$ représente un groupe phényle ou pyridinyle éventuellement substitué une, deux ou trois fois, chaque substituant étant indépendamment choisi parmi un atome d'halogène ou un groupe choisi parmi hydroxy, cyano, nitro, $C_1$-$C_4$-alkyle, (phényl) - ($C_1$-$C_2$-alkyle) -, $C_1$-$C_4$-halogénoalkyle, $C_1$-$C_4$-alcoxy, (phényl) - ($C_1$-$C_2$-alcoxy) -, $C_1$-$C_4$-halogénoalcoxy, - N($R^5$)($R^6$),

dans lequel les groupes phényle dans lesdits groupes (phényl) - ($C_1$-$C_2$-alkyle) - et (phényl) -($C_1$-$C_2$-alcoxy)- sont éventuellement substitués une ou deux fois, chaque substituant étant indépendamment choisi parmi un atome de fluor, un atome de chlore et un atome de brome, ou un groupe choisi parmi méthyle, trifluorométhyle et méthoxy,
ou deux substituants fixés à des atomes de carbone adjacents dudit groupe phényle ou pyridinyle formant ensemble un groupe bivalent choisi parmi $-(CH_2)_3-$, $-(CH_2)_4-$, $-(CH_2)$2-O-, - $(CH_2)_3$-O-, $-CH_2-O-CH_2-$, $-O-CH_2-O-$, $-O-CH_2-CH_2-O-$ et $-O-CF_2-O-$ ,

ou
$R^1$ représente un groupe pyrazolyle éventuellement substitué une ou deux fois, chaque substituant étant indépendamment choisi parmi un atome d'halogène ou un groupe choisi parmi cyano, $C_1$-$C_2$-alkyle, et $C_1$-$C_2$-alcoxy ;
$R^2$ représente un groupe

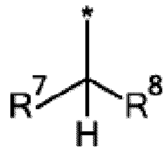

,

dans lequel « * » indique le point de fixation à l'atome d'azote auquel $R^2$ est fixé ;
$R^3$ représente un groupe choisi parmi méthyle et $-NH_2$ ;
$R^4$ représente un groupe hétéroaryle à 5 chaînons choisi parmi oxazolyle, isoxazolyle, thiazolyle, isothiazolyle et oxadiazolyle, éventuellement substitué une ou deux fois, chaque substituant étant indépendamment choisi parmi un atome de fluor, un atome de chlore et un atome de brome, ou un groupe choisi parmi $C_1$-$C_4$-alkyle, (($R^9$) O)-($C_1$-$C_3$-alkyle) -, (($R^{10}$)($R^{11}$)N) - ($C_1$-$C_3$-alkyle) -, ($C_3$-$C_7$-cycloalkyl) - ($C_1$-$C_3$-alkyle) -, $C_3$-$C_7$-cycloalkyle, -O$R^9$, -N($R^{10}$)($R^{11}$), - C(=O)-N($R^{15}$)($R^{16}$), -C(=O)-O$R^{17}$, phényle et un groupe hétéroaryle à 5 ou 6 chaînons, dans lequel le groupe phényle et le groupe hétéroaryle à 5 ou 6 chaînons sont éventuellement substitués une ou deux fois, chaque substituant étant indépendamment choisi parmi un atome de fluor, un atome de chlore et un atome de brome, ou un groupe choisi parmi cyano, $C_1$-$C_2$-alkyle, $C_1$-$C_2$-fluoroalkyle, -O$R^9$, -N($R^{10}$)($R^{11}$) et -C(=O)-N($R^{15}$)($R^{16}$);
$R^5$ et $R^6$ représentent, indépendamment de chaque occurrence, un atome d'hydrogène ou un groupe choisi parmi $C_1$-$C_2$-alkyle et ($C_1$-$C_2$-alkyl)-C(=O)-, ou

$R^5$ et $R^6$, conjointement avec l'atome d'azote auquel ils sont fixés, représentent un groupe hétérocycloalkyle contenant de l'azote monocyclique à 4 à 7 chaînons qui est éventuellement substitué une ou deux fois, chaque substituant étant indépendamment choisi parmi un atome de fluor, un atome de chlore et un atome de brome, ou un groupe choisi parmi oxo, hydroxy, $C_1$-$C_2$-alkyle et ($C_1$-$C_2$-alkyl) -C (=O) - ;
$R^7$ représente un atome d'hydrogène ou un groupe $C_1$-$C_2$-alkyle ;
$R^8$ représente un groupe -C(=O)-$NH_2$ ;
$R^9$ représente un atome d'hydrogène ou un groupe choisi parmi $C_1$-$C_4$-alkyle, ($C_3$-$C_7$-cycloalkyl) - ($C_1$-$C_2$-alkyle) -, (phényl) - ($C_1$-$C_2$-alkyle) -, $C_1$-$C_4$-halogénoalkyle, $C_2$-$C_3$-hydroxyalkyle, ($C_1$-$C_2$-alcoxy) - $C_2$-alkyle-, (($C_1$-$C_2$-alkyl)-C(=O)-O)-$C_2$-alkyle-, - C(=O)-N($R^{20}$)($R^{21}$), $C_3$-$C_7$-cycloalkyle et phényle,
dans lequel $C_3$-$C_7$-cycloalkyle, et le $C_3$-$C_7$-cycloalkyle dans ledit groupe ($C_3$-$C_7$-cycloalkyl)-($C_1$-$C_2$-alkyle) -, sont éventuellement substitués une ou deux fois, chaque substituant étant indépendamment choisi parmi un atome de fluor ou un groupe choisi parmi oxo et méthyle, et dans lequel le groupe phényle dans ledit groupe (phényl) - ($C_1$-$C_2$-alkyle) - et ledit groupe phényle lui-même sont éventuellement substitués une ou deux fois, chaque substituant étant indépendamment choisi parmi un atome de fluor, un atome de chlore et un atome de brome, ou un groupe choisi parmi cyano, méthyle, trifluorométhyle et méthoxy ;
$R^{10}$ et $R^{11}$ représentent, indépendamment de chaque occurrence, un atome d'hydrogène ou un groupe choisi parmi $C_1$-$C_2$-alkyle, $C_1$-$C_2$-halogénoalkyle, $C_2$-$C_3$-hydroxyalkyle, ($C_1$-$C_2$-alcoxy) -$C_2$-alkyle-, (($R^{22}$)($R^{23}$)N)-$C_2$-alkyle, ($C_3$-$C_7$-cycloalkyl)- ($C_1$-$C_2$-alkyle) -, ($C_1$-$C_2$-alkyl)-C(=O)-, $C_3$-$C_7$-cycloalkyle, ($C_3$-$C_7$-cycloalkyl) -C(=O)-, (phényl) - ($C_1$-$C_2$-alkyle) -, (phényl)-($C_1$-$C_2$-alkyl)-C(=O)- et (phényl) - ($C_1$-$C_2$-alkyl)-O-C(=O)-, dans lequel $C_3$-$C_7$-cycloalkyle, et le $C_3$-$C_7$-cycloalkyle dans lesdits groupes ($C_3$- $C_7$ -cycloalkyl) - ($C_1$-$C_2$-alkyle) - et ($C_3$-$C_7$-cycloalkyl)-C(=O)-, sont éventuellement substitués une ou deux fois, chaque substituant étant indépendamment choisi parmi un atome de fluor ou un groupe choisi parmi cyano, $C_1$-$C_2$-alkyle et $C_1$-$C_2$-halogénoalkyle, et dans lequel les groupes phényle dans lesdits groupes (phényl) - ($C_1$-$C_2$-alkyle)-, (phényl) - ($C_1$-$C_2$-alkyl)-C(=O)- et (phényl) - ($C_1$-$C_2$-alkyl)-OC(=O)- sont éventuellement substitués une ou deux fois, chaque substituant étant indépendamment choisi parmi un atome de fluor, un atome de chlore et un atome de brome, ou un groupe choisi parmi méthyle, trifluorométhyle et méthoxy, ou
$R^{10}$ et $R^{11}$, conjointement avec l'atome d'azote auquel ils sont fixés, représentent un groupe hétérocycloalkyle contenant de l'azote monocyclique à 4 à 7 chaînons, ou un groupe hétérocycloalkyle contenant de l'azote bicyclique à 5 à 10 chaînons, qui sont éventuellement substitués une, deux ou trois fois, chaque substituant étant indépendamment choisi parmi un atome d'halogène ou un groupe choisi parmi cyano, oxo, hydroxy, $C_1$-$C_2$-alkyle, $C_1$-$C_2$-halogénoalkyle, ($C_1$-$C_2$-alkyl)-C(=O)-, $C_1$-$C_2$-alcoxy, -N($R^{22}$)($R^{23}$), et un groupe monocyclique hétérocycloalkyle à 4 à 7 chaînons ;
$R^{15}$ et $R^{16}$ représentent, indépendamment de chaque occurrence, un atome d'hydrogène ou un groupe choisi parmi $C_1$-$C_4$-alkyle, $C_1$-$C_4$-halogénoalkyle, $C_1$-$C_4$-hydroxyalkyle, (cyano) -$C_1$-$C_4$-alkyle-, ($C_1$-$C_4$-alcoxy) -$C_2$-$C_3$-alkyle-, ($C_1$-$C_4$-halogénoalcoxy) -$C_2$-$C_3$-alkyle-, ($C_3$-$C_7$-cycloalkyl) - ($C_1$-$C_3$-alkyle) -, $C_3$-$C_7$-cycloalkyle, $C_5$-$C_{11}$-cycloalkyle bicyclique, hétérocycloalkyle monocyclique à 4 à 7 chaînons, (phényl) - ($C_1$-$C_3$-alkyle) -, phényle et un groupe hétéroaryle à 5 ou 6 chaînons,

dans lequel $C_3$-$C_7$-cycloalkyle, le $C_3$-$C_7$-cycloalkyle dans ledit groupe ($C_3$-$C_7$-cycloalkyl)-($C_1$-$C_3$-alkyle)-, le $C_5$-$C_{11}$-cycloalkyle bicyclique et les groupes hétérocycloalkyle monocycliques à 4 à 7 chaînons sont éventuellement substitués une ou deux fois, chaque substituant étant indépendamment choisi parmi un atome d'halogène ou un groupe choisi parmi oxo, $C_1$-$C_2$-alkyle, ($C_1$-$C_2$-alkyl)-C(=O)- et cyclopropyle,
et dans lequel le phényle et les groupes hétéroaryle à 5 ou 6 chaînons, y compris le groupe phényle dans ledit groupe (phényl)-($C_1$-$C_3$-alkyle)-, sont éventuellement substitués une ou deux fois, chaque substituant étant indépendamment choisi parmi un atome de fluor, un atome de chlore et un atome de brome, ou un groupe choisi parmi méthyle, trifluorométhyle et méthoxy,

ou
$R^{15}$ et $R^{16}$, conjointement avec l'atome d'azote auquel ils sont fixés, représentent un groupe hétérocycloalkyle contenant de l'azote monocyclique à 4 à 7 chaînons qui est éventuellement substitué une ou deux fois, chaque substituant étant indépendamment choisi parmi un atome d'halogène ou un groupe choisi parmi oxo, $C_1$-$C_2$-alkyle et ($C_1$-$C_2$-alkyl) -C(=O)- ;
$R^{17}$ représente un groupe $C_1$-$C_4$-alkyle ;
$R^{20}$ représente un atome d'hydrogène ou un groupe choisi parmi $C_1$-$C_6$-alkyle, $C_3$-$C_7$-cycloalkyle, hétérocycloalkyle monocyclique à 4 à 7 chaînons, phényle, et hétéroaryle à 5 à 6 chaînons,

dans lequel ledit groupe $C_1$-$C_6$-alkyle est éventuellement substitué une, deux ou trois fois, chaque substituant étant indépendamment choisi parmi un atome d'halogène ou un groupe choisi parmi hydroxy, cyano,

$C_1$-$C_3$-alcoxy, -N($R^{22}$)($R^{23}$), $C_3$-$C_7$-cycloalkyle, hétérocycloalkyle monocyclique à 4 à 7 chaînons, phényle, et hétéroaryle à 5 à 6 chaînons, lesdits substituants phényle et hétéroaryle à 5 à 6 chaînons eux-mêmes étant éventuellement substitués une ou deux fois, chaque substituant étant indépendamment choisi parmi un atome de fluor, un atome de chlore et un atome de brome, ou un groupe choisi parmi méthyle, trifluorométhyle et méthoxy,

et dans lequel $C_3$-$C_7$-cycloalkyle et hétérocycloalkyle monocyclique à 4 à 7 chaînons sont éventuellement substitués une, deux ou trois fois, chaque substituant étant indépendamment choisi parmi un atome d'halogène ou un groupe choisi parmi cyano, oxo, hydroxy, $C_1$-$C_2$-alkyle et ($C_1$-$C_2$-alkyl) -C (=O) -,

et dans lequel lesdits groupes phényle et hétéroaryle à 5 à 6 chaînons sont éventuellement substitués une, deux ou trois fois, chaque substituant étant indépendamment choisi parmi un atome d'halogène ou un groupe choisi parmi cyano, $C_1$-$C_2$-alkyle, $C_1$-$C_2$-halogénoalkyle, $C_1$-$C_2$-alcoxy, $C_1$-$C_2$-halogénoalcoxy, -N ($R^{22}$)($R^{23}$)et -C(=O)-N($R^{24}$)($R^{25}$),

$R^{21}$ représente un atome d'hydrogène ou un groupe $C_1$-$C_2$-alkyle, ou

$R^{20}$ et $R^{21}$, conjointement avec l'atome d'azote auquel ils sont fixés, représentent un groupe hétérocycloalkyle contenant de l'azote monocyclique à 4 à 7 chaînons qui est substitué une, deux ou trois fois, chaque substituant étant indépendamment choisi parmi un atome d'halogène ou un groupe choisi parmi cyano, oxo, hydroxy, $C_1$-$C_2$-alkyle, $C_1$-$C_2$-halogénoalkyle, (phényl)-($C_1$-$C_2$-alkyle)-, ($C_1$-$C_2$-alkyl) -C(=O)-, $C_3$-$C_4$-cycloalkyle, $C_1$-$C_2$-alcoxy, $C_1$-$C_2$-halogénoalcoxy, - N($R^{22}$)($R^{23}$)et -C(=O)-N($R^{24}$)($R^{25}$) ;

$R^{22}$ et $R^{23}$ représentent, indépendamment de chaque occurrence, un atome d'hydrogène ou un groupe choisi parmi $C_1$-$C_2$-alkyle et ($C_1$-$C_2$-alkyl) -C (=O) -, et

$R^{24}$ et $R^{25}$ représentent, indépendamment de chaque occurrence, un atome d'hydrogène ou un groupe $C_1$-$C_2$-alkyle,

ou stéréoisomère, forme tautomère, N-oxyde, hydrate, solvate, ou sel correspondant, ou mélange de ceux-ci.

**3.** Composé selon la revendication 1 ou 2, dans lequel :

$R^1$ représente un groupe phényle ou pyridinyle éventuellement substitué une, deux ou trois fois, chaque substituant étant indépendamment choisi parmi un atome de fluor, un atome de chlore et un atome de brome, ou un groupe choisi parmi hydroxy, cyano, $C_1$-$C_4$-alkyle, $C_1$-$C_2$-fluoroalkyle, $C_1$-$C_2$-alcoxy, (phényl) - ($C_1$-$C_2$-alcoxy) -, $C_1$-$C_2$-fluoroalcoxy et - N($R^5$)($R^6$),

ou deux substituants fixés à des atomes de carbone adjacents dudit groupe phényle ou pyridinyle formant ensemble un groupe bivalent choisi parmi $-(CH_2)_3-$, $-O-CH_2-O-$ et $-O-CF_2-O-$,

ou

$R^1$ représente un groupe pyrazolyle éventuellement substitué par un groupe méthyle ;

$R^2$ représente un groupe

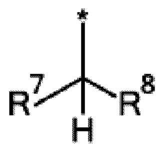

,

dans lequel « * » indique le point de fixation à l'atome d'azote auquel $R^2$ est fixé ;

$R^3$ représente un groupe choisi parmi méthyle et $-NH_2$ ;

$R^4$ représente un groupe choisi parmi

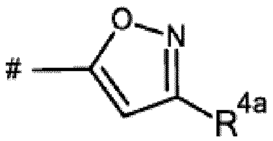

et

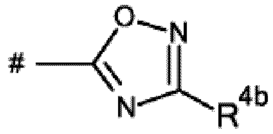

,

dans lequel « # » indique le point de fixation au groupe carbonyle auquel $R^4$ est fixé, et dans lequel

$R^{4a}$ et $R^{4b}$ représentent, indépendamment l'un de l'autre, un atome de fluor, un atome de chlore ou un atome de brome, ou un groupe choisi parmi $C_1$-$C_4$-alkyle, $((R^9)O)$- ($C_1$-$C_2$-alkyle) -, $((R^{10})(R^{11})N)$-($C_1$-$C_2$-alkyle)-, ($C_3$-$C_7$-cycloalkyl)-($C_1$-$C_2$-alkyle)-, $C_3$-$C_7$-cycloalkyle, -$OR^9$, -$N(R^{10})(R^{11})$, -C(=O)-$N(R^{15})(R^{16})$, - C(=O)-$OR^{17}$, phényle et un groupe hétéroaryle à 5 ou 6 chaînons choisi parmi oxazolyle, isoxazolyle, thiazolyle, isothiazolyle, pyrazolyle, pyridinyle, pyrazinyle, pyrimidinyle et pyridazinyle,

dans lequel le groupe phényle et le groupe hétéroaryle à 5 ou 6 chaînons choisi parmi oxazolyle, isoxazolyle, thiazolyle, isothiazolyle, pyrazolyle, pyridinyle, pyrazinyle, pyrimidinyle et pyridazinyle sont éventuellement substitués une ou deux fois, chaque substituant étant indépendamment choisi parmi un atome de fluor et un atome de chlore, ou un groupe choisi parmi cyano, $C_1$-$C_2$-alkyle, - $OR^9$, -$N(R^{10})(R^{11})$ et -C(=O)-$N(R^{15})(R^{16})$;

$R^5$ et $R^6$ représentent, indépendamment de chaque occurrence, un atome d'hydrogène ou un groupe $C_1$-$C_2$-alkyle, ou

$R^5$ et $R^6$, conjointement avec l'atome d'azote auquel ils sont fixés, représentent un groupe hétérocycloalkyle contenant de l'azote monocyclique à 4 à 7 chaînons qui est éventuellement substitué une ou deux fois, chaque substituant étant indépendamment choisi parmi un atome de fluor ou un groupe choisi parmi hydroxy et $C_1$-$C_2$-alkyle ;

$R^7$ représente un atome d'hydrogène ou un groupe $C_1$-$C_2$-alkyle ;

$R^8$ représente un groupe -C(=O)-$NH_2$ ;

$R^9$ représente un atome d'hydrogène ou un groupe choisi parmi $C_1$-$C_2$-alkyle, benzyle, $C_1$-$C_2$-fluoroalkyle, $C_2$-hydroxyalkyle, ($C_1$-$C_2$-alcoxy) -$C_2$-alkyle-, (($C_1$-$C_2$-alkyl) -C(=O)-O)-$C_2$-alkyle-, -C(=O) -$N(R^{20})(R^{21})$ et phényle, dans lequel le groupe phényle dans ledit groupe benzyle et ledit groupe phényle lui-même sont éventuellement substitués une ou deux fois, chaque substituant étant indépendamment choisi parmi un atome de fluor et un atome de chlore, ou un groupe choisi parmi cyano et méthyle ;

$R^{10}$ et $R^{11}$ représentent, indépendamment de chaque occurrence, un atome d'hydrogène ou un groupe choisi parmi $C_1$-$C_2$-alkyle, $C_1$-$C_2$-fluoroalkyle, ($C_3$-$C_5$-cycloalkyl) - ($C_1$-$C_2$-alkyle) - ($C_1$-$C_2$-alkyl) -C (=O)-, $C_3$-$C_7$-cycloalkyle, $C_3$-$C_7$-cycloalkyl- (C=O)-, (phényl) - ($C_1$-$C_2$-alkyle) -, (phényl) - ($C_1$-$C_2$-alkyl) - C (=O) -et (phényl) - ($C_1$-$C_2$-alkyl) -O-C (=O) -, dans lequel $C_3$-$C_7$-cycloalkyle, et le $C_3$-$C_5$-cycloalkyle dans ledit ($C_3$-$C_5$-cycloalkyl) -($C_1$-$C_2$-alkyle) - et le $C_3$-$C_7$-cycloalkyle dans lesdits groupes $C_3$-$C_7$-cycloalkyl-(C=O)- sont éventuellement substitués une ou deux fois, chaque substituant étant indépendamment choisi parmi un atome de fluor ou un groupe choisi parmi cyano, $C_1$-$C_2$-alkyle et $C_1$-$C_2$-fluoroalkyle, et dans lequel les groupes phényle dans lesdits groupes (phényl) - ($C_1$-$C_2$-alkyle) -, (phényl)-($C_1$-$C_2$-alkyl)-C(=O)- et (phényl) - ($C_1$-$C_2$-alkyl) -OC(=O)- sont éventuellement substitués une ou deux fois, chaque substituant étant indépendamment choisi parmi un atome de fluor, un atome de chlore et un groupe méthyle, ou

$R^{10}$ et $R^{11}$, conjointement avec l'atome d'azote auquel ils sont fixés, représentent un groupe hétérocycloalkyle contenant de l'azote monocyclique à 4 à 7 chaînons qui est éventuellement substitué une, deux ou trois fois, chaque substituant étant indépendamment choisi parmi un atome de fluor ou un groupe choisi parmi cyano, oxo, $C_1$-$C_2$-alkyle, $C_1$-$C_2$-fluoroalkyle et ($C_1$-$C_2$-alkyl)-C(=O)- ;

$R^{15}$ et $R^{16}$ représentent, indépendamment de chaque occurrence, un atome d'hydrogène ou un groupe choisi parmi $C_1$-$C_4$-alkyle, $C_1$-$C_3$-fluoroalkyle, (cyano) -$C_1$-$C_2$-alkyle-, ($C_1$-$C_4$-alcoxy) -$C_2$-$C_3$-alkyle-, ($C_1$-$C_2$-fluoroalcoxy) -$C_2$-$C_3$-alkyle-, ($C_3$-$C_5$-cycloalkyl) -($C_1$-$C_2$-alkyle)-, $C_3$-$C_5$-cycloalkyle, phényle et un groupe hétéroaryle à 5 ou 6 chaînons choisi parmi pyrazolyle, pyridinyle et pyrimidinyle,

dans lequel $C_3$-$C_5$-cycloalkyle, et le $C_3$-$C_5$-cycloalkyle dans ledit groupe ($C_3$-$C_5$-cycloalkyl)-($C_1$-$C_2$-alkyle)- sont éventuellement substitués une ou deux fois, chaque substituant étant indépendamment choisi parmi un atome de fluor, ou un groupe choisi parmi méthyle et cyclopropyle,

et dans lequel le phényle et les groupes hétéroaryle à 5 ou 6 chaînons choisis parmi pyrazolyle, pyridinyle et pyrimidinyle sont éventuellement substitués une ou deux fois, chaque substituant étant indépendamment choisi parmi un atome de fluor et un atome de chlore, ou un groupe choisi parmi méthyle, trifluorométhyle et méthoxy,

ou

$R^{15}$ et $R^{16}$, conjointement avec l'atome d'azote auquel ils sont fixés, représentent un groupe hétérocycloalkyle

contenant de l'azote monocyclique à 4 à 7 chaînons qui est éventuellement substitué une fois par un atome de fluor ou un groupe choisi parmi oxo, $C_1$-$C_2$-alkyle et $CH_3$-C(=O)- ;
$R^{17}$ représente un groupe $C_1$-$C_2$-alkyle ;
$R^{20}$ représente un atome d'hydrogène ou un groupe choisi parmi $C_1$-$C_3$-alkyle et phényle,

dans lequel ledit groupe $C_1$-$C_3$-alkyle est éventuellement substitué une ou deux fois, chaque substituant étant indépendamment choisi parmi un atome de fluor ou un groupe choisi parmi hydroxy, $C_1$-$C_3$-alcoxy et phényle, ledit phényle lui-même étant éventuellement substitué une ou deux fois, chaque substituant étant indépendamment choisi parmi un atome de fluor, un atome de chlore et un groupe méthyle,
et dans lequel ledit groupe phényle est éventuellement substitué une, deux ou trois fois, chaque substituant étant indépendamment choisi parmi un atome de fluor et un atome de chlore ou un groupe choisi parmi méthyle, trifluorométhyle, méthoxy et trifluorométhoxy ;

$R^{21}$ représente un atome d'hydrogène ou un groupe $C_1$-$C_2$-alkyle ; ou
$R^{20}$ et $R^{21}$, conjointement avec l'atome d'azote auquel ils sont fixés, représentent un groupe hétérocycloalkyle contenant de l'azote monocyclique à 4 à 7 chaînons qui est éventuellement substitué une ou deux fois, chaque substituant étant indépendamment choisi parmi un atome d'halogène ou un groupe choisi parmi cyano, oxo, hydroxy, $C_1$-$C_2$-alkyle, $C_1$-$C_2$-fluoroalkyle, benzyle, ($C_1$-$C_2$-alkyl)-C(=O)- et $C_3$-$C_4$-cycloalkyle,

ou stéréoisomère, forme tautomère, N-oxyde, hydrate, solvate, ou sel correspondant, ou mélange de ceux-ci.

**4.** Composé selon la revendication 1, 2 ou 3, dans lequel :

$R^1$ représente un groupe phényle ou pyridinyle éventuellement substitué une ou deux fois, chaque substituant étant indépendamment choisi parmi un atome de fluor, un atome de chlore et un atome de brome, ou un groupe choisi parmi méthyle, difluorométhyle, trifluorométhyle, méthoxy, benzyloxy, difluorométhoxy et trifluorométhoxy, ou deux substituants fixés à des atomes de carbone adjacents dudit groupe phényle ou pyridinyle formant ensemble un groupe bivalent -O-$CF_2$-O- ;
$R^2$ représente un groupe

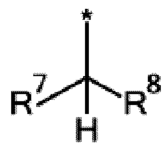

,

dans lequel « * » indique le point de fixation à l'atome d'azote auquel $R^2$ est fixé ;
$R^3$ représente un groupe choisi parmi méthyle et -$NH_2$ ;
$R^4$ représente un groupe

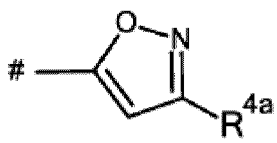

,

dans lequel « # » indique le point de fixation au groupe carbonyle auquel $R^4$ est fixé,
et dans lequel
$R^{4a}$ représente un atome de chlore ou un atome de brome, ou un groupe choisi parmi (($R^9$)O)-($C_1$-$C_2$-alkyle)-, (($R^{10}$)($R^{11}$)N) - ($C_1$-$C_2$-alkyle) -, ($C_3$-$C_5$-cycloalkyl) - ($C_1$-$C_2$-alkyle) -, $C_3$-$C_5$-cycloalkyle, -O$R^9$, -C(=O)-N($R^{15}$)($R^{16}$) et phényle,
dans lequel le groupe phényle est éventuellement substitué une ou deux fois, chaque substituant étant indépendamment choisi parmi un atome de fluor et un atome de chlore et un groupe méthyle, ou
$R^4$ représente un groupe

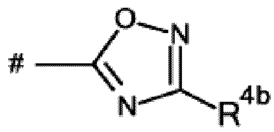

,

dans lequel « # » indique le point de fixation au groupe carbonyle auquel $R^4$ est fixé,
et dans lequel
$R^{4b}$ représente un groupe choisi parmi $C_1$-$C_4$-alkyle, $C_3$-$C_5$-cycloalkyle, -C(=O)-N($R^{15}$)($R^{16}$) et phényle, dans lequel le groupe phényle est éventuellement substitué une ou deux fois, chaque substituant étant indépendamment choisi parmi un atome de fluor et un atome de chlore, ou un groupe choisi parmi cyano, $C_1$-$C_2$-alkyle et -$OR^9$ ;
$R^7$ représente un atome d'hydrogène ou un groupe $C_1$-$C_2$-alkyle ;
$R^8$ représente un groupe -C(=O)-$NH_2$ ;
$R^9$ représente un atome d'hydrogène ou un groupe choisi parmi $C_1$-$C_2$-alkyle, benzyle, $C_1$-$C_2$-fluoroalkyle, ($C_1$-$C_2$-alcoxy)-$C_2$-alkyle-, (($C_1$-$C_2$-alkyl) -C(=O)-O)-$C_2$-alkyle-, -C(=O)-N($R^{20}$)($R^{21}$) et phényle, dans lequel le groupe phényle dans ledit groupe benzyle et ledit groupe phényle lui-même sont éventuellement substitués une ou deux fois, chaque substituant étant indépendamment choisi parmi un atome de fluor et un atome de chlore, ou un groupe choisi parmi cyano et méthyle ;
$R^{10}$ et $R^{11}$ représentent, indépendamment de chaque occurrence, un atome d'hydrogène ou un groupe choisi parmi $C_1$-$C_2$-alkyle, ($C_3$-$C_5$-cycloalkyl) - ($C_1$-$C_2$-alkyle)-, $C_3$-$C_7$-cycloalkyle et (phényl) - ($C_1$-$C_2$-alkyl)-O-C(=O)-,

dans lequel $C_3$-$C_7$-cycloalkyle, et le $C_3$-$C_5$-cycloalkyle dans ledit groupe ($C_3$-$C_5$-cycloalkyl)-($C_1$-$C_2$-alkyle)- sont éventuellement substitués une ou deux fois, chaque substituant étant indépendamment choisi parmi un atome de fluor ou un groupe choisi parmi cyano, méthyle et $C_1$-fluoroalkyle,
et dans lequel le groupe phényle dans ledit groupe (phényl) - ($C_1$-$C_2$-alkyl) -O-C (=O) - est éventuellement substitué une ou deux fois, chaque substituant étant indépendamment choisi parmi un atome de fluor, un atome de chlore et un groupe méthyle,

ou
$R^{10}$ et $R^{11}$, conjointement avec l'atome d'azote auquel ils sont fixés, représentent un groupe hétérocycloalkyle contenant de l'azote monocyclique à 4 à 7 chaînons qui est éventuellement substitué une, deux ou trois fois, chaque substituant étant indépendamment choisi parmi un atome de fluor ou un groupe choisi parmi cyano, méthyle et $C_1$-fluoroalkyle ;
$R^{15}$ et $R^{16}$ représentent, indépendamment de chaque occurrence, un atome d'hydrogène ou un groupe choisi parmi $C_1$-$C_4$-alkyle, $C_1$-$C_2$-fluoroalkyle, ($C_1$-$C_4$-alcoxy) -$C_2$-$C_3$-alkyle-, ($C_1$-$C_2$-fluoroalcoxy) -$C_2$-$C_3$-alkyle-, ($C_3$-$C_5$-cycloalkyl) - ($C_1$-$C_2$-alkyle) -, $C_3$-$C_5$-cycloalkyle, phényle et pyridinyle,

dans lequel $C_3$-$C_5$-cycloalkyle, et le $C_3$-$C_5$-cycloalkyle dans ledit groupe ($C_3$-$C_5$-cycloalkyl) - ($C_1$-$C_2$-alkyle)-, sont éventuellement substitués une ou deux fois, chaque substituant étant indépendamment choisi parmi un atome de fluor et un groupe méthyle,
et dans lequel les groupes phényle et pyridinyle sont éventuellement substitués une ou deux fois, chaque substituant étant indépendamment choisi parmi un atome de fluor et un atome de chlore, ou un groupe choisi parmi méthyle, trifluorométhyle et méthoxy ;

$R^{20}$ représente un groupe choisi parmi benzyle et phényle,
dans lequel ledit groupe phényle, et le groupe phényle dans ledit groupe benzyle, sont éventuellement substitués une ou deux fois, chaque substituant étant indépendamment choisi parmi un atome de fluor, un atome de chlore et un groupe méthyle, et
$R^{21}$ représente un atome d'hydrogène ou un groupe méthyle,

ou stéréoisomère, forme tautomère, N-oxyde, hydrate, solvate, ou sel correspondant, ou mélange de ceux-ci.

**5.** Composé selon les revendications 1, 2 ou 3, dans lequel :

$R^1$ représente un groupe

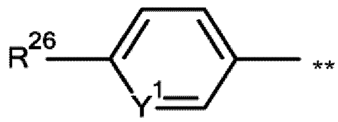

,

dans lequel « ** » indique le point de fixation à l'atome d'azote auquel $R^1$ est fixé ;
$R^2$ représente un groupe

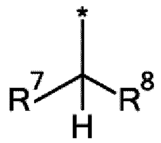

,

dans lequel « * » indique le point de fixation à l'atome d'azote auquel $R^2$ est fixé ;
$R^3$ représente un groupe choisi parmi méthyle et $-NH_2$ ;
$R^4$ représente un groupe

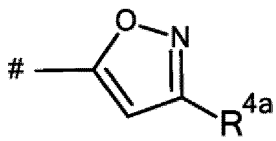

,

dans lequel « # » indique le point de fixation au groupe carbonyle auquel $R^4$ est fixé, et dans lequel
$R^{4a}$ représente un atome de chlore ou un atome de brome, ou un groupe choisi parmi $((R^9)O)-(C_1\text{-alkyle})-$, $((R^{10})(R^{11})N)-(C_1\text{-alkyle})-$, $(C_3\text{-}C_5\text{-cycloalkyl})-(C_1\text{-alkyle})-$, $C_3\text{-}C_5$-cycloalkyle, $-OR^9$, $-C(=O)-N(R^{15})(R^{16})$ et phényle, dans lequel le groupe phényle est éventuellement substitué une ou deux fois, chaque substituant étant indépendamment choisi parmi un atome de fluor et un atome de chlore et un groupe méthyle, ou
$R^4$ représente un groupe

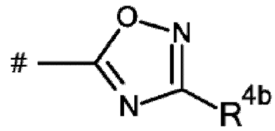

,

dans lequel « # » indique le point de fixation au groupe carbonyle auquel $R^4$ est fixé, et dans lequel
$R^{4b}$ représente un groupe choisi parmi $C_1\text{-}C_4$-alkyle, $C_3\text{-}C_5$-cycloalkyle et phényle,
dans lequel le groupe phényle est éventuellement substitué une ou deux fois, chaque substituant étant indépendamment choisi parmi un atome de fluor et un atome de chlore, ou un groupe choisi parmi cyano, $C_1\text{-}C_2$-alkyle et $-OR^9$ ;
$R^7$ représente un atome d'hydrogène ou un groupe $C_1\text{-}C_2$-alkyle ;
$R^8$ représente un groupe $-C(=O)-NH_2$ ;
$R^9$ représente un atome d'hydrogène ou un groupe choisi parmi $C_1\text{-}C_2$-alkyle, benzyle, $C_1\text{-}C_2$-fluoroalkyle, $(C_1\text{-}C_2\text{-alcoxy})-C_2\text{-alkyle-}$, $((C_1\text{-}C_2\text{-alkyl})-C(=O)-O)-C_2\text{-alkyle-}$ et phényle, dans lequel le groupe phényle dans ledit groupe benzyle et ledit groupe phényle lui-même sont éventuellement substitués une ou deux fois, chaque substituant étant indépendamment choisi parmi un atome de fluor et un atome de chlore, ou un groupe choisi parmi cyano et méthyle ;
$R^{10}$ et $R^{11}$ représentent, indépendamment de chaque occurrence, un atome d'hydrogène ou un groupe choisi parmi $C_1\text{-}C_2$-alkyle, $C_3\text{-}C_7$-cycloalkyle et (benzyl)-OC(=O)-,
dans lequel $C_3\text{-}C_7$-cycloalkyle est éventuellement substitué une ou deux fois, chaque substituant étant indépendamment choisi parmi un atome de fluor ou un groupe choisi parmi méthyle et trifluorométhyle, et dans lequel le groupe phényle dans ledit groupe (benzyl)-O-C(=O)- est éventuellement substitué une ou deux fois, chaque substituant étant indépendamment choisi parmi un atome de fluor, un atome de chlore et un groupe méthyle, ou
$R^{10}$ et $R^{11}$, conjointement avec l'atome d'azote auquel ils sont fixés, représentent un groupe hétérocycloalkyle

contenant de l’azote monocyclique à 4 à 7 chaînons qui est éventuellement substitué une, deux ou trois fois, chaque substituant étant indépendamment choisi parmi un atome de fluor ou un groupe choisi parmi cyano, méthyle et trifluorométhyle ;

$R^{15}$ et $R^{16}$ représentent, indépendamment de chaque occurrence, un atome d’hydrogène ou un groupe choisi parmi $C_1$-$C_4$-alkyle, $C_1$-$C_2$-fluoroalkyle, ($C_1$-$C_4$-alcoxy)-$C_2$-alkyle-, ($C_1$-$C_2$-fluoroalcoxy) -$C_2$-alkyle-, $C_3$-$C_5$-cycloalkyle, phényle et pyridinyle,

dans lequel $C_3$-$C_5$-cycloalkyle est éventuellement substitué une ou deux fois, chaque substituant étant indépendamment choisi parmi un atome de fluor et un groupe méthyle ;

$Y^1$ représente -C(H)=, -C(F)=, -C(Cl)=, -C(CN)= ou -N=, et

$R^{26}$ représente un atome de fluor, un atome de chlore ou un atome de brome, ou un groupe choisi parmi méthyle, difluorométhyle, trifluorométhyle, méthoxy, benzyloxy, difluorométhoxy et trifluorométhoxy,

ou stéréoisomère, forme tautomère, N-oxyde, hydrate, solvate, ou sel correspondant, ou mélange de ceux-ci.

**6.** Composé selon la revendication 1, 2, 3, 4 ou 5, dans lequel :

$R^1$ représente un groupe

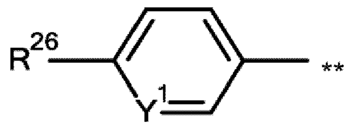

,

dans lequel « ** » indique le point de fixation à l’atome d’azote auquel $R^1$ est fixé ;

$R^2$ représente un groupe

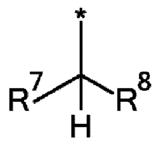

,

dans lequel « * » indique le point de fixation à l’atome d’azote auquel $R^2$ est fixé ;

$R^3$ représente un groupe choisi parmi méthyle et $-NH_2$ ;

$R^4$ représente un groupe

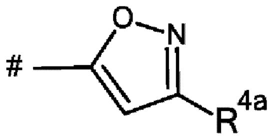

,

dans lequel « # » indique le point de fixation au groupe carbonyle auquel $R^4$ est fixé, et dans lequel

$R^{4a}$ représente un atome de chlore ou un atome de brome, ou un groupe choisi parmi (($R^9$)O)-($C_1$-alkyle)-, $C_3$-$C_5$-cycloalkyle, $-OR^9$ et -C(=O)-N($R^{15}$)($R^{16}$), Ou

$R^4$ représente un groupe

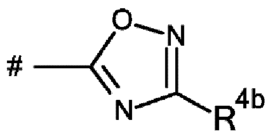

,

dans lequel « # » indique le point de fixation au groupe carbonyle auquel $R^4$ est fixé, et dans lequel

$R^{4b}$ représente un groupe choisi parmi $C_1$-$C_4$-alkyle, $C_3$-$C_5$-cycloalkyle et phényle,

dans lequel le groupe phényle est éventuellement substitué une ou deux fois, chaque substituant étant

indépendamment choisi parmi un atome de fluor et un atome de chlore et un groupe méthyle ;
$R^7$ représente un atome d'hydrogène ou un groupe $C_1$-$C_2$-alkyle ;
$R^8$ représente un groupe -C(=O)-$NH_2$ ;
$R^9$ représente un atome d'hydrogène ou un groupe choisi parmi $C_1$-$C_2$-alkyle, benzyle, $C_1$-$C_2$-fluoroalkyle et phényle,
dans lequel le groupe phényle dans ledit groupe benzyle et ledit groupe phényle lui-même sont éventuellement substitués une ou deux fois, chaque substituant étant indépendamment choisi parmi un atome de fluor et un atome de chlore, ou un groupe choisi parmi cyano et méthyle ;
$R^{15}$ et $R^{16}$ représentent, indépendamment de chaque occurrence, un atome d'hydrogène ou un groupe choisi parmi $C_1$-$C_2$-alkyle, $C_3$-$C_5$-cycloalkyle, phényle et pyridinyle,
dans lequel $C_3$-$C_5$-cycloalkyle est éventuellement substitué une ou deux fois, chaque substituant étant indépendamment choisi parmi un atome de fluor et un groupe méthyle ;
$Y^1$ représente -C(H)=, -C(F)=, -C(Cl)= ou -N=, et
$R^{26}$ représente un atome de fluor, un atome de chlore ou un atome de brome, ou un groupe choisi parmi difluorométhyle, méthoxy, benzyloxy, difluorométhoxy et trifluorométhoxy,
ou stéréoisomère, forme tautomère, N-oxyde, hydrate, solvate, ou sel correspondant, ou mélange de ceux-ci.

**7.** Composé selon la revendication 1, 2, 3, 4, 5 ou 6, dans lequel :

$R^1$ représente un groupe

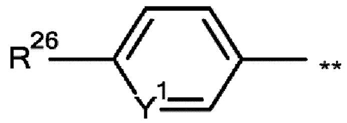

,

dans lequel « ** » indique le point de fixation à l'atome d'azote auquel $R^1$ est fixé ;
$R^2$ représente un groupe

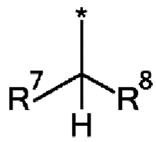

,

dans lequel « * » indique le point de fixation à l'atome d'azote auquel $R^2$ est fixé ;
$R^3$ représente un groupe -$NH_2$ ;
$R^4$ représente un groupe

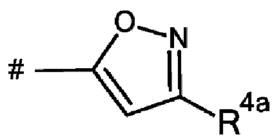

,

dans lequel « # » indique le point de fixation au groupe carbonyle auquel $R^4$ est fixé, et dans lequel
$R^{4a}$ représente un atome de chlore ou un atome de brome, ou un groupe choisi parmi (($R^9$)O)-($C_1$-alkyle)-, $C_3$-$C_5$-cycloalkyle, -$OR^9$ et -C(=O)-N($R^{15}$)($R^{16}$),
Ou
$R^4$ représente un groupe

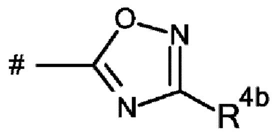

,

dans lequel « # » indique le point de fixation au groupe carbonyle auquel $R^4$ est fixé, et dans lequel

$R^{4b}$ représente un groupe choisi parmi $C_1$-$C_4$-alkyle, $C_3$-$C_5$-cycloalkyle et phényle, dans lequel le groupe phényle est éventuellement substitué une ou deux fois, chaque substituant étant indépendamment choisi parmi un atome de fluor et un atome de chlore et un groupe méthyle ;

$R^7$ représente un groupe méthyle ;

$R^8$ représente un groupe -C(=O)-$NH_2$ ;

$R^9$ représente un atome d'hydrogène ou un groupe choisi parmi $C_1$-$C_2$-alkyle, benzyle, $C_1$-$C_2$-fluoroalkyl et phényle,

dans lequel le groupe phényle dans ledit groupe benzyle et ledit groupe phényle lui-même sont éventuellement substitués une ou deux fois, chaque substituant étant indépendamment choisi parmi un atome de fluor et un atome de chlore, ou un groupe choisi parmi cyano et méthyle ;

$R^{15}$ et $R^{16}$ représentent, indépendamment de chaque occurrence, un atome d'hydrogène ou un groupe choisi parmi $C_1$-$C_2$-alkyle, $C_3$-$C_5$-cycloalkyle, phényle et pyridinyle,

dans lequel $C_3$-$C_5$-cycloalkyle est éventuellement substitué une ou deux fois, chaque substituant étant indépendamment choisi parmi un atome de fluor et un groupe méthyle ;

$Y^1$ représente -C(H)=, -C(F)=, -C(Cl)= ou -N=, et

$R^{26}$ représente un atome de fluor, un atome de chlore ou un atome de brome, ou un groupe choisi parmi difluorométhyle, méthoxy, benzyloxy, difluorométhoxy et trifluorométhoxy,

ou stéréoisomère, forme tautomère, N-oxyde, hydrate, solvate, ou sel correspondant, ou mélange de ceux-ci.

**8.** Composé selon la revendication 1, qui est choisi dans le groupe constitué par :

rac-2-(N-[4-amino-5-[3-(4-chlorophényl)-1,2,4-oxadiazole-5-carbonyl]thiazol-2-yl]-4-fluoroanilino)propanamide ,
rac-2-(N-[4-amino-5-(3-benzyloxyisoxazole-5-carbonyl)thiazol-2-yl]-4-fluoro-anilino)propanamide ,
rac-2-(N-[4-amino-5-(3-phénylisoxazole-5-carbonyl)thiazol-2-yl]-4-fluoro-anilino)propanamide ,
(R)- 2-(N-[4-amino-5-(3-phénylisoxazole-5-carbonyl)thiazol-2-yl]-4-fluoro-anilino)propanamide ,
(S)-2-(N-[4-amino-5-(3-phénylisoxazole-5-carbonyl)thiazol-2-yl]-4-fluoro-anilino)propanamide ,
rac-2-(N-[4-amino-5-[3-(2,4-dichlorophényl)isoxazole-5-carbonyl]thiazol-2-yl]-4-fluoro-anilino)propanamide ,
rac-2-(N-[4-amino-5-[3-(4-chlorophényl)isoxazole-5-carbonyl]thiazol-2-yl]-4-fluoro-anilino)propanamide ,
rac-2-(N-[4-amino-5-(3-bromoisoxazole-5-carbonyl)thiazol-2-yl]-4-fluoro-anilino)propanamide ,
(R)-2-(N-[4-amino-5-(3-bromoisoxazole-5-carbonyl)thiazol-2-yl]-4-fluoro-anilino)propanamide ,
(S)-2-(N-[4-amino-5-(3-bromoisoxazole-5-carbonyl)thiazol-2-yl]-4-fluoro-anilino)propanamide ,
rac-2-(N-[4-amino-5-[3-(3-pyridinyl)isoxazole-5-carbonyl]thiazol-2-yl]-4-fluoro-anilino)propanamide ,
rac-2-(N-[4-amino-5-[3-(4-pyridinyl)isoxazole-5-carbonyl]thiazol-2-yl]-4-fluoro-anilino)propanamide ,
rac-5-[4-amino-2-(N-(2-amino-1-méthyl-2-oxo-éthyl)-4-fluoro-anilino)thiazole-5-carbonyl]isoxazole-3-carboxylate d'éthyle,
rac-5-[4-amino-2-(N-(2-amino-1-méthyl-2-oxo-éthyl)-4-fluoro-anilino)thiazole-5-carbonyl]-N-phényl-isoxazole-3-carboxamide ,
5-[4-amino-2-(N-(2-amino-1-méthyl-2-oxo-éthyl)-4-fluoro-anilino)thiazole-5-carbonyl]-N-[(cis)-2-fluorocyclopropyl]isoxazole-3-carboxamide (mélange de stéréoisomères),
rac-5-[4-amino-2-(N-(2-amino-1-méthyl-2-oxo-éthyl)-4-fluoro-anilino)thiazole-5-carbonyl]-N-(1-méthylpyrazol-4-yl)isoxazole-3-carboxamide ,
rac-2-(N-[4-amino-5-[3-(morpholine-4-carbonyl)isoxazole-5-carbonyl]thiazol-2-yl]-4-fluoroanilino)propanamide ,
rac-5-[4-amino-2-(N-(2-amino-1-méthyl-2-oxo-éthyl)-4-fluoro-anilino)thiazole-5-carbonyl]-N-pyrimidin-5-yl-isoxazole-3-carboxamide ,
rac-5-[4-amino-2-(N-(2-amino-1-méthyl-2-oxo-éthyl)-4-fluoro-anilino)thiazole-5-carbonyl]-N-cyclopropyl-isoxazole-3-carboxamide ,
rac-5-[4-amino-2-(N-(2-amino-1-méthyl-2-oxo-éthyl)-4-fluoro-anilino)thiazole-5-carbonyl]-N-(4-pyridinyl)isoxazole-3-carboxamide ,
rac-5-[4-amino-2-(N-(2-amino-1-méthyl-2-oxo-éthyl)-4-fluoro-anilino)thiazole-5-carbonyl]-N-(2,2-difluoroéthyl)isoxazole-3-carboxamide ,
rac-2-(N-[4-amino-5-[3-(azétidine-1-carbonyl)isoxazole-5-carbonyl]thiazol-2-yl]-4-fluoro-anilino)propanamide ,
rac-2-(N-[4-amino-5-[3-(pyrrolidine-1-carbonyl)isoxazole-5-carbonyl]thiazol-2-yl]-4-fluoro-anilino)propanamide ,
rac-5-[4-amino-2-(N-(2-amino-1-méthyl-2-oxo-éthyl)-4-fluoro-anilino)thiazole-5-carbonyl]-N-(3-pyridinyl)isoxazole-3-carboxamide ,
rac-5-[4-amino-2-(N-(2-amino-1-méthyl-2-oxo-éthyl)-4-fluoro-anilino)thiazole-5-carbonyl]-N-cyclobutyl-isoxazole-3-carboxamide ,
rac-5-[4-amino-2-(N-(2-amino-1-méthyl-2-oxo-éthyl)-4-fluoro-anilino)thiazole-5-carbonyl]-N-(cyclopropylméthyl)isoxazole-3-carboxamide ,
rac-5-[4-amino-2-(N-(2-amino-1-méthyl-2-oxo-éthyl)-4-fluoro-anilino)thiazole-5-carbonyl]-N-(2,2,2-trifluoroéthyl)isoxazole-3-carboxamide ,
rac-5-[4-amino-2-(N-(2-amino-1-méthyl-2-oxo-éthyl)-4-fluoro-anilino)thiazole-5-carbonyl]-N-(1-cyclopropylcyclopropyl)isoxazole-3-carboxamide ,
rac-5-[4-amino-2-(N-(2-amino-1-méthyl-2-oxo-éthyl)-4-fluoro-anilino)thiazole-5-carbonyl]-N-cyclopentyl-isoxazole-3-carboxamide ,
rac-5-[4-amino-2-(N-(2-amino-1-méthyl-2-oxo-éthyl)-4-fluoro-anilino)thiazole-5-carbonyl]-N-(2-tert-butoxyéthyl)isoxazole-3-carboxamide ,
rac-2-(N-[4-amino-5-[3-(pipéridine-1-carbonyl)isoxazole-5-carbonyl]thiazol-2-yl]-4-fluoro-anilino)propanamide ,
rac-5-[4-amino-2-(N-(2-amino-1-méthyl-2-oxo-éthyl)-4-fluoro-anilino)thiazole-5-carbonyl]-N-(2-pyridinyl)isoxazole-3-carboxamide ,
rac-5-[4-amino-2-(N-(2-amino-1-méthyl-2-oxo-éthyl)-4-fluoro-anilino)thiazole-5-carbonyl]-N-[2-(trifluorométhoxy)éthyl]isoxazole-3-carboxamide ,
rac-5-[4-amino-2-(N-(2-amino-1-méthyl-2-oxo-éthyl)-4-fluoro-anilino)thiazole-5-carbonyl]-N-(1-méthylcyclobutyl)isoxazole-3-carboxamide ,
(R)-5-[4-amino-2-(N-(2-amino-1-méthyl-2-oxo-éthyl)-4-fluoro-anilino)thiazole-5-carbonyl]-N-(1-méthylcyclobutyl)isoxazole-3-carboxamide ,
(S)-5-[4-amino-2-(N-(2-amino-1-méthyl-2-oxo-éthyl)-4-fluoro-anilino)thiazole-5-carbonyl]-N-(1-méthylcyclobutyl)isoxazole-3-carboxamide ,
rac-5-[4-amino-2-(N-(2-amino-1-méthyl-2-oxo-éthyl)-4-fluoro-anilino)thiazole-5-carbonyl]-N-(1-bicyclo[1.1.1]pentanyl)isoxazole-3-carboxamide ,
rac-5-[4-amino-2-(N-(2-amino-1-méthyl-2-oxo-éthyl)-4-fluoro-anilino)thiazole-5-carbonyl]-N-(3,3-difluorocyclobutyl)isoxazole-3-carboxamide ,
(R)-5-[4-amino-2-(N-(2-amino-1-méthyl-2-oxo-éthyl)-4-fluoro-anilino)thiazole-5-carbonyl]-N-(3,3-difluorocyclobutyl)isoxazole-3-carboxamide ,
(S)-5-[4-amino-2-(N-(2-amino-1-méthyl-2-oxo-éthyl)-4-fluoro-anilino)thiazole-5-carbonyl]-N-(3,3-difluorocyclobutyl)isoxazole-3-carboxamide ,
5-[4-amino-2-(N-(2-amino-1-méthyl-2-oxo-éthyl)-4-fluoro-anilino)thiazole-5-carbonyl]-N-(3,3-difluorocyclopentyl)isoxazole-3-carboxamide (mélange de stéréoisomères),
rac-5-[4-amino-2-(N-(2-amino-1-méthyl-2-oxo-éthyl)-4-fluoro-anilino)thiazole-5-carbonyl]-N-isopropyl-isoxazole-3-carboxamide ,
rac-5-[4-amino-2-(N-(2-amino-1-méthyl-2-oxo-éthyl)-4-fluoro-anilino)thiazole-5-carbonyl]-N-(cyanométhyl)isoxazole-3-carboxamide ,
5-[4-amino-2-(N-(2-amino-1-méthyl-2-oxo-éthyl)-4-fluoro-anilino)thiazole-5-carbonyl]-N-(2-méthoxy-1-méthyléthyl)isoxazole-3-carboxamide (mélange de stéréoisomères),
5-[4-amino-2-(N-(2-amino-1-méthyl-2-oxo-éthyl)-4-fluoro-anilino)thiazole-5-carbonyl]-N-(2,2-diméthylcyclopentyl)isoxazole-3-carboxamide (mélange de stéréoisomères),
5-[4-amino-2-(N-(2-amino-1-méthyl-2-oxo-éthyl)-4-fluoro-anilino)thiazole-5-carbonyl]-N-(cis-3-fluorocyclobutyl)isoxazole-3-carboxamide (mélange de stéréoisomères),
rac-5-[4-amino-2-(N-(2-amino-1-méthyl-2-oxo-éthyl)-4-fluoro-anilino)thiazole-5-carbonyl]-N-(1-méthylcyclopentyl)isoxazole-3-carboxamide ,
rac-5-[4-amino-2-(N-(2-amino-1-méthyl-2-oxo-éthyl)-4-fluoro-anilino)thiazole-5-carbonyl]-N-[2-fluoro-1-(fluorométhyl)éthyl]isoxazole-3-carboxamide ,
5-[4-amino-2-(N-(2-amino-1-méthyl-2-oxo-éthyl)-4-fluoro-anilino)thiazole-5-carbonyl]-N-(2,2-difluoro-1-méthyl-éthyl)isoxazole-3-carboxamide (mélange de stéréoisomères),
5-[4-amino-2-(N-(2-amino-1-méthyl-2-oxo-éthyl)-4-fluoro-anilino)thiazole-5-carbonyl]-N-(trans-3-fluorocyclobutyl)isoxazole-3-carboxamide (mélange de stéréoisomères),

5-[4-amino-2-(N-(2-amino-1-méthyl-2-oxo-éthyl)-4-fluoro-anilino)thiazole-5-carbonyl]-N-(3,3-diméthylcyclopentyl)isoxazole-3-carboxamide (mélange de stéréoisomères),
rac-5-[4-amino-2-(N-(2-amino-1-méthyl-2-oxo-éthyl)-3,4-difluoro-anilino)thiazole-5-carbonyl]-N-(1-méthylcyclobutyl)isoxazole-3-carboxamide ,
(R)-5-[4-amino-2-(N-(2-amino-1-méthyl-2-oxo-éthyl)-3,4-difluoro-anilino)thiazole-5-carbonyl]-N-(1-méthylcyclobutyl)isoxazole-3-carboxamide ,
(S)-5-[4-amino-2-(N-(2-amino-1-méthyl-2-oxo-éthyl)-3,4-difluoro-anilino)thiazole-5-carbonyl]-N-(1-méthylcyclobutyl)isoxazole-3-carboxamide ,
rac-5-[4-amino-2-(N-(2-amino-1-méthyl-2-oxo-éthyl)-3,4-difluoro-anilino)thiazole-5-carbonyl]-N-(3,3-difluorocyclobutyl)isoxazole-3-carboxamide ,
(R)-5-[4-amino-2-(N-(2-amino-1-méthyl-2-oxo-éthyl)-3,4-difluoro-anilino)thiazole-5-carbonyl]-N-(3,3-difluorocyclobutyl)isoxazole-3-carboxamide ,
(S)-5-[4-amino-2-(N-(2-amino-1-méthyl-2-oxo-éthyl)-3,4-difluoro-anilino)thiazole-5-carbonyl]-N-(3,3-difluorocyclobutyl)isoxazole-3-carboxamide , rac-5-[4-amino-2-(N-(2-amino-1-méthyl-2-oxo-éthyl)-3,4-difluoro-anilino)thiazole-5-carbonyl]-N-cyclopentyl-isoxazole-3-carboxamide ,
(R)-5-[4-amino-2-(N-(2-amino-1-méthyl-2-oxo-éthyl)-3,4-difluoro-anilino)thiazole-5-carbonyl]-N-cyclopentyl-isoxazole-3-carboxamide ,
(S)-5-[4-amino-2-(N-(2-amino-1-méthyl-2-oxo-éthyl)-3,4-difluoro-anilino)thiazole-5-carbonyl]-N-cyclopentyl-isoxazole-3-carboxamide ,
rac-2-(N-[4-amino-5-(3-cyclopentylisoxazole-5-carbonyl)thiazol-2-yl]-4-fluoro-anilino)propanamide ,
rac-2-(N-[4-amino-5-[3-(difluorométhoxy)isoxazole-5-carbonyl]thiazol-2-yl]-4-fluoro-anilino)propanamide ,
rac-2-(N-[4-amino-5-(3-isopropyl-1,2,4-oxadiazole-5-carbonyl)thiazol-2-yl]-4-fluoro-anilino)propanamide ,
rac-2-(N-[4-amino-5-(3-phénylisothiazole-5-carbonyl)thiazol-2-yl]-4-fluoro-anilino)propanamide,
rac-2-(N-[4-amino-5-(3-phényl-1,2,4-oxadiazole-5-carbonyl)thiazol-2-yl]-4-fluoro-anilino)propanamide ,
rac-2-(N-[4-amino-5-(3-méthyl-1,2,4-oxadiazole-5-carbonyl)thiazol-2-yl]-4-fluoro-anilino)propanamide ,
rac-2-(N-[4-amino-5-(3-tert-butyl-1,2,4-oxadiazole-5-carbonyl)thiazol-2-yl]-4-fluoro-anilino)propanamide ,
rac-2-(N-[4-amino-5-[3-(2-pyridinyl)-1,2,4-oxadiazole-5-carbonyl]thiazol-2-yl]-4-fluoro-anilino)propanamide ,
rac-2-(N-[4-amino-5-[3-(3-fluorophényl)-1,2,4-oxadiazole-5-carbonyl]thiazol-2-yl]-4-fluoro-anilino)propanamide ,
rac-2-(N-[4-amino-5-[3-(2,3-diméthylphényl)-1,2,4-oxadiazole-5-carbonyl]thiazol-2-yl]-4-fluoro-anilino)propanamide ,
rac-2-(N-[4-amino-5-(3-cyclopropyl-1,2,4-oxadiazole-5-carbonyl)thiazol-2-yl]-4-fluoro-anilino)propanamide ,
rac-2-(N-[4-amino-5-[3-(1-méthylpyrazol-4-yl)-1,2,4-oxadiazole-5-carbonyl]thiazol-2-yl]-4-fluoro-anilino)propanamide ,
rac-2-(N-[4-amino-5-(3-cyclopentyl-1,2,4-oxadiazole-5-carbonyl)thiazol-2-yl]-4-fluoro-anilino)propanamide ,
rac-2-(N-[4-amino-5-[3-[[cyclopentyl(méthyl)amino]méthyl]isoxazole-5-carbonyl]thiazol-2-yl]-4-fluoro-anilino)propanamide ,
rac-2-(N-[4-amino-5-[3-[[(1-méthylcyclopentyl)amino]méthyl]isoxazole-5-carbonyl]thiazol-2-yl]-4-fluoro-anilino)propanamide ,
rac-2-(N-[4-amino-5-[3-[(cyclopentylamino)méthyl]isoxazole-5-carbonyl]thiazol-2-yl]-4-fluoro-anilino)propanamide ,
rac-2-(N-[4-amino-5-[3-(1-pipéridylméthyl)isoxazole-5-carbonyl]thiazol-2-yl]-4-fluoro-anilino)propanamide ,
rac-2-(N-[4-amino-5-[3-[(4-cyanophénoxy)méthyl]isoxazole-5-carbonyl]thiazol-2-yl]-4-fluoro-anilino)propanamide ,
rac-2-(N-[4-amino-5-[3-(hydroxyméthyl)isoxazole-5-carbonyl]thiazol-2-yl]-4-fluoro-anilino)propanamide ,
rac-carbamate de N-phényle et de [5-[4-amino-2-(N-(2-amino-1-méthyl-2-oxo-éthyl)-4-fluoro-anilino)thiazole-5-carbonyl]isoxazol-3-yl]méthyle,
rac-2-(N-[4-amino-5-[3-(2-fluorophényl)isoxazole-5-carbonyl]thiazol-2-yl]-4-fluoro-anilino)propanamide ,
rac-2-(N-[4-amino-5-[3-(2-pyridinyl)isoxazole-5-carbonyl]thiazol-2-yl]-4-fluoro-anilino)propanamide ,
rac-2-(N-[4-amino-5-[3-[4-(trifluorométhoxy)phényl]isoxazole-5-carbonyl]thiazol-2-yl]-4-fluoro-anilino)propanamide ,
rac-2-(N-[4-amino-5-[3-(2-méthoxyphényl)isoxazole-5-carbonyl]thiazol-2-yl]-4-fluoro-anilino)propanamide ,
rac-2-(N-[4-amino-5-[3-(6-méthoxy-3-pyridinyl)isoxazole-5-carbonyl]thiazol-2-yl]-4-fluoro-anilino)propanamide,
rac-2-(N-[4-amino-5-(1-phényltriazole-4-carbonyl)thiazol-2-yl]-4-fluoro-anilino)propanamide ,
rac-2-(N-[4-amino-5-[3-[(3-fluoroazétidin-1-yl)méthyl]isoxazole-5-carbonyl]thiazol-2-yl]-4-fluoro-anilino)propanamide ,
rac-2-(N-[4-amino-5-[3-[(3,3-difluoroazétidin-1-yl)méthyl]isoxazole-5-carbonyl]thiazol-2-yl]-4-fluoro-anilino)

propanamide ,
rac-2-(N-[4-amino-5-[3-[(4,4-difluoro-1-pipéridyl)méthyl]isoxazole-5-carbonyl]thiazol-2-yl]-4-fluoro-anilino)propanamide ,
2-(N-[4-amino-5-[3-[(4,4-difluoro-3-méthyl-1-pipéridyl)méthyl]isoxazole-5-carbonyl]thiazol-2-yl]-4-fluoro-anilino)propanamide (mélange de stéréoisomères),
2-(N-[4-amino-5-[3-[(3-fluoropyrrolidin-1-yl)méthyl]isoxazole-5-carbonyl]thiazol-2-yl]-4-fluoro-anilino)propanamide (mélange de stéréoisomères),
rac-2-(N-[4-amino-5-(3-hydroxyisoxazole-5-carbonyl)thiazol-2-yl]-4-fluoro-anilino)propanamide,
rac-2-(N-[4-amino-5-[3-(cyclobutoxy)isoxazole-5-carbonyl]thiazol-2-yl]-4-fluoro-anilino)propanamide ,
rac-2-(N-[4-amino-5-[3-(cyclobutylméthoxy)isoxazole-5-carbonyl]thiazol-2-yl]-4-fluoro-anilino)propanamide ,
rac-2-(N-[4-amino-5-[3-(4,4-difluorocyclohexoxy)isoxazole-5-carbonyl]thiazol-2-yl]-4-fluoro-anilino)propanamide , et
rac-2-(N-[4-amino-5-(3-isopropoxyisoxazole-5-carbonyl)thiazol-2-yl]-4-fluoro-anilino)propanamide ,
ou stéréoisomère, forme tautomère, N-oxyde, hydrate, solvate, ou sel correspondant, ou mélange de ceux-ci.

**9.** Composé de formule générale (I) selon l'une quelconque des revendications 1 à 8 pour une utilisation dans le traitement ou la prophylaxie d'une maladie.

**10.** Composition pharmaceutique comprenant un composé de formule générale (I) selon l'une quelconque des revendications 1 à 8 et un ou plusieurs excipients pharmaceutiquement acceptables.

**11.** Combinaison pharmaceutique comprenant :

- un ou plusieurs composés de formule générale (I) selon l'une quelconque des revendications 1 à 8, et
- un ou plusieurs inhibiteurs de point de contrôle immunitaire.

**12.** Combinaison pharmaceutique selon la revendication 11, dans laquelle l'inhibiteur de point de contrôle immunitaire est un antagoniste de l'axe aPD-1/-L1.

**13.** Combinaison pharmaceutique selon la revendication 11, dans laquelle l'inhibiteur de point de contrôle immunitaire est un inhibiteur de DGK$\alpha$.

**14.** Composé pour une utilisation selon la revendication 9, dans lequel la maladie est un cancer ou une affection comportant une réponse immunitaire dérégulée ou un trouble associé à une signalisation de DGK$\zeta$ aberrante.

Figure 1

```
MGDYKDDDDK MEPRDGSPEA RSSDSESASA SSSGSERDAG PEPDKAPRRL NKRRFPGLRL FGHRKAITKS GLQHLAPPPP TPGAPCSESE
RQIRSTVDWS ESATYGEHIW FETNVSGDFC YVGEQYCVAR MLKSVSRRKC AACKIVVHTP CIEQLEKINF RCKPSFRESG SRNVREPTFV
RHHWVHRRRQ DGKCRHCGKG FQQKFTFHSK EIVAISCSWC KQAYHSKVSC FMLQQIEEPC SLGVHAAVVI PPTWILRARR PQNTLKASKK
KKRASFKRKS SKKGPEEGRW RPFIIRPTPS PLMKPLLVFV NPKSGGNQGA KIIQSFLWYL NPRQVFDLSQ GGPKEALEMY RKVHNLRILA
CGGDGTVGWI LSTLDQLRLK PPPPVAILPL GTGNDLARTL NWGGGYTDEP VSKILSHVEE GNVVQLDRWD LHAEPNPEAG PEDRDEGATD
RLPLDVFNNY FSLGFDAHVT LEFHESREAN PEKFNSRFRN KMFYAGTAFS DFLMGSSKDL AKHIRVVCDG MDLTPKIQDL KPQCVVFLNI
PRYCAGTMPW GHPGEHHDFE PQRHDDGYLE VIGFTMTSLA ALQVGGHGER LTQCREVVLT TSKAIPVQVD GEPCKLAASR IRIALRNQAT
MVQKAKRRSA APLHSDQQPV PEQLRIQVSR VSMHDYEALH YDKEQLKEAS VPLGTVVVPG DSDLELCRAH IERLQQEPDG AGAKSPTCQK
LSPKWCFLDA TTASRFYRID RAQEHLNYVT EIAQDEIYIL DPELLGASAR PDLPTPTSPL PTSPCSPTPR SLQGDAAPPQ GEELIEAAKR
NDFCKLQELH RAGGDLMHRD EQSRTLLHHA VSTGSKDVVR YLLDHAPPEI LDAVEENGET CLHQAAALGQ RTICHYIVEA GASLMKTDQQ
GDTPRQRAEK AQDTELAAYL ENRQHYQMIQ REDQETAV
```

Figure 2

```
Ser Ile Ile Asn Phe Glu Lys Leu
1               5
```

Figure 3

```
Ser Met Leu Val Leu Leu Pro Asp Glu Val Ser Gly Leu Glu Gln Leu
1               5                   10                  15
Glu Ser Ile Ile Asn Phe Glu Lys Leu Thr Glu Trp Thr Ser
            20                  25                  30
```

Figure 4

```
Asp Tyr Lys Asp Asp Asp Asp Lys
1               5
```

Figure 5

```
Gly Cys Cys Ala Cys Cys
1               5
```

.

Figure 6

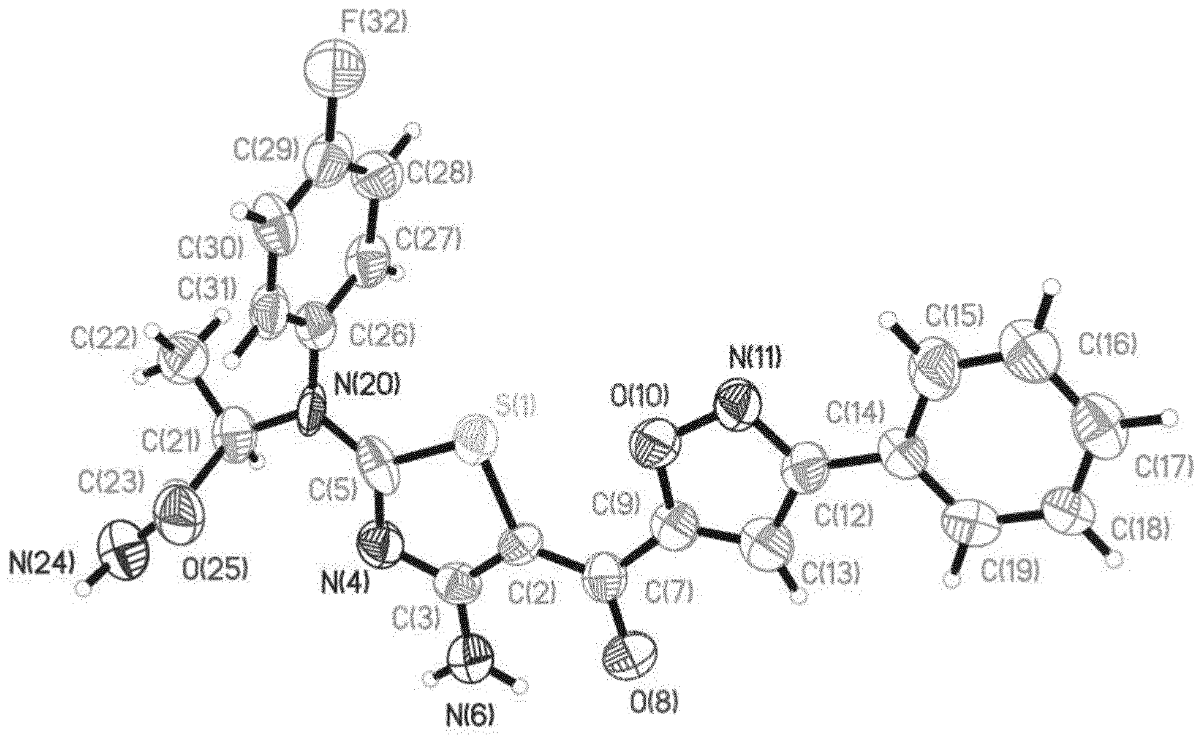

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020006016 A
- WO 2020006018 A
- WO 2021041588 A
- WO 2014181287 A
- WO 2014173904 A
- WO 2009149054 A
- WO 2007130075 A
- WO 2012064715 A
- WO 2005103022 A
- CN 106109467
- WO 2015199206 A
- WO 2015046193 A
- CN 103159695
- WO 2013056684 A
- WO 2013033037 A
- WO 2012075393 A
- JP 2011032254 B
- WO 2009041790 A
- WO 2008090382 A
- WO 2007022415 A
- WO 2006122011 A
- WO 2006078287 A
- WO 2005102318 A
- WO 2005102325 A
- WO 2005102326 A
- WO 2005102346 A
- WO 2005102455 A
- WO 2005112920 A
- WO 2005115304 A
- WO 2005115385 A
- EP 1543824 A
- US 20050137239 A
- WO 2004014884 A
- WO 2019133445 A
- WO 2021043966 A
- WO 2012112363 A, K. Kassahun


### Non-patent literature cited in the description


- **T.O. EICHMANN** ; **A. LASS**. *Cell. Mol. Life Sci.*, 2015, vol. 72, 3931-3952
- **S. KRISHNA** ; **X.-P. ZHONG**. *Front. Immunol.*, 2013, vol. 4, 178
- **E. J. QUANN et al.** *Nat. Immunol.*, 2011, vol. 12 (7), 647-654
- **JOSHI** ; **G. A. KORETZKY**. *Int. J. Mol. Sci.*, 2013, vol. 14 (4), 6649-6673
- **X.-P.-ZHONG et al.** *Nat. Immunol.*, 2003, vol. 4, 882-890
- **B. A. OLENCHOCK et al.** *Nat. Immunol.*, 2006, vol. 7 (11), 1174-1181
- **M. J. RIESE et al.** *J. Biol. Chem.*, 2011, vol. 286, 5254-5265
- **E. M. WESLEY et al.** *ImmunoHorizons*, 2018, vol. 2 (4), 107-118
- **X.-P. ZHONG et al.** *Nat. Immunol.*, 2003, vol. 4, 882-890
- **E. M. RIESE et al.** *J. Biol. Chem.*, 2011, vol. 286, 5254-5264
- **W. JING et al.** *Cancer Res.*, 2017, vol. 77 (20), 5676-5686
- **S. WEE et al.** Proceedings of the American Association for Cancer Research Annual Meeting 2019. *Cancer Res.*, 2019, vol. 79 (13)
- **M. J. RIESE et al.** *Cancer Res.*, 2013, vol. 73 (12), 3566-3577
- **I.-Y. JUNG et al.** *Cancer Res.*, 2018, vol. 78 (16), 4692-4703
- **E. YANG et al.** *J. Immunol.*, 2016, vol. 197 (3), 934-41
- **W. YU et al.** *Front. Oncol.*, 2019, vol. 8, 655
- **K. CAI et al.** *BMC Cancer*, 2014, vol. 14, 208
- **J. DIAO et al.** *Mol. Neurobiol.*, 2016, vol. 53, 5425-35
- **H. LI et al.** *Pharmazie*, 2019, vol. 74 (7), 418-422
- **B. A. SINGH et al.** *Sci. Signal.*, 2019, vol. 12, 3332
- **E. RUFFO et al.** *Sci. Transl. Med.*, 2016, vol. 8 (321), 321-7
- **S. VELNATI et al.** *Eur. J. Med. Chem.*, 2019, vol. 164, 378-390
- **L. YANG et al.** *J. Mol. Neurosci.*, 2015, vol. 56, 78-88
- **B. BENZIANE et al.** *J. Lipid Res.*, 2017, vol. 58 (12), 2324-2333
- **D. KIKELJ** ; **U. URLEB**. *Science of Synthesis*, 2002, vol. 11, 627-833
- **S. TITUS et al.** *Tetrahedron Lett.*, 2014, vol. 55, 5465-5467
- **T. N. BIRKINSHAW et al.** *J. Chem. Soc. Perkin Trans.*, 1988, vol. 1, 2209-2212

- **R. A. FUNNELL et al.** *J. Chem. Soc. Perkin Trans.*, 1987, vol. 1, 2311-2315
- **J. C. BRINDLEY et al.** *J. Chem. Soc. Perkin Trans.*, 1987, vol. 1, 1153-1158
- **G. D. MEAKINS et al.** *J. Chem. Soc. Chem. Comm.*, 1984, 837-838
- **K. AKIBA et al.** *Tetrahedron Lett.*, 1975, vol. 7, 459-462
- *CHEMICAL ABSTRACTS*, 1349635-19-3
- Isotopic Compositions of the Elements 1997. *Pure Appl. Chem.*, 1998, vol. 70 (1), 217-235
- **C. L. PERRIN et al.** *J. Am. Chem. Soc.*, 2007, vol. 129, 4490
- **C. L. PERRIN et al.** *J. Am. Chem. Soc.*, 2005, vol. 127 (9641)
- **B. TESTA et al.** *Int. J. Pharm.*, 1984, vol. 19 (3), 271
- **A. E. MUTLIB et al.** *Toxicol. Appl. Pharmacol.*, 2000, vol. 169, 102
- **NEVIRAPINE: A. M. SHARMA et al.** *Chem. Res. Toxicol.*, 2013, vol. 26, 410
- **EFAVIRENZ: A. E. MUTLIB et al.** *Toxicol. Appl. Pharmacol.*, 2000, vol. 169, 102
- **C. J. WENTHUR et al.** *J. Med. Chem.*, 2013, vol. 56, 5208
- **ROFECOXIB: F. SCHNEIDER et al.** *Arzneim. Forsch. / Drug. Res.*, 2006, vol. 56, 295
- **TELAPREVIR: F. MALTAIS et al.** *J. Med. Chem.*, 2009, vol. 52, 7993
- *Pure Appl Chem*, 1976, vol. 45, 11-30
- **S. M. BERGE et al.** Pharmaceutical Salts. *J. Pharm. Sci.*, 1977, vol. 66, 1-19
- **M. E. KEIR et al.** *Annu. Rev. Immunol.*, 2008, vol. 26, 677-704
- **M. J. RIESE et al.** *Cancer Res.*, 2013, vol. 73 (12), 3566-77
- **I. Y. JUNG et al.** *Mol. Cells*, 2018, vol. 41 (8), 717-723
- **T.W. GREENE** ; **P.G.M. WUTS**. Protective Groups in Organic Synthesis. Wiley, 1999
- *CHEMICAL ABSTRACTS*, 6674-22-2
- **F. CALDERON et al.** *Journal of Medicinal Chemistry*, 2017, vol. 60 (16), 6880-6896
- **Y. XING et al.** *European Journal of Organic Chemistry*, 2017, vol. 2017 (4), 781-785
- **A. WU et al.** *Tetrahedron*, 2013, vol. 69 (31), 6392-6398
- *Eur. J. Org. Chem.*, 1998, 473-479